(19) Europäisches Patentamt
European Patent Office
Office européen des brevets

(11) EP 3 736 277 A1

(12) EUROPEAN PATENT APPLICATION

(43) Date of publication:
11.11.2020 Bulletin 2020/46

(21) Application number: 20182522.1

(22) Date of filing: 28.07.2017

(51) Int Cl.:
*C07D 513/04* (2006.01) *A61K 31/429* (2006.01)
*A61P 35/00* (2006.01) *A61P 37/00* (2006.01)
*A61P 17/00* (2006.01) *A61P 19/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.07.2016 IN 201621026107**
**22.12.2016 IN 201621043859**
**17.03.2017 IN 201721009450**

(62) Document number(s) of the earlier application(s) in accordance with Art. 76 EPC:
**17758952.0 / 3 490 995**

(71) Applicant: **Lupin Limited**
**Mumbai 400 055 (IN)**

(72) Inventors:
- **KUKREJA, Gagan**
**412115 Pune Maharashtra (IN)**
- **IRLAPATI, Nageswara Rao**
**412115 Pune Maharashtra (IN)**
- **JAGDALE, Arun Rangnath**
**412115 Pune Maharashtra (IN)**
- **DESHMUKH, Gokul Keruji**
**412115 Pune Maharashtra (IN)**
- **VYAVAHARE, Vinod Popatrao**
**412115 Pune Maharashtra (IN)**
- **KULKARNI, Kiran Chandrashekhar**
**412115 Pune Maharashtra (IN)**
- **SINHA, Neelima**
**412115 Pune Maharashtra (IN)**
- **PALLE, Venkata P.**
**412115 Pune Maharashtra (IN)**
- **KAMBOJ, Rajender Kumar**
**412115 Pune Maharashtra (IN)**

(74) Representative: **Schlich, George et al**
**Schlich**
**9 St Catherine's Road**
**Littlehampton, West Sussex BN17 5HS (GB)**

Remarks:
This application was filed on 26-06-2020 as a divisional application to the application mentioned under INID code 62.

# (54) SUBSTITUTED THIAZOLO-PYRIDINE COMPOUNDS AS MALT1 INHIBITORS

(57) Disclosed are compounds of the general formula (I),

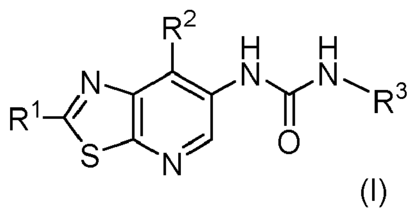

(I)

wherein $R^1$-$R^3$ are as defined herein, for use as MALT1 inhibitors in the treatment of autoimmune and inflammatory diseases or disorders. Methods of synthesizing the compounds are also disclosed. Also disclosed are pharmaceutical compositions containing a compound of the invention and a method of treating a patient for an autoimmune or an inflammatory disease or disorder, for example, a cancer, by administering a compound of the invention.

EP 3 736 277 A1

## Description

FIELD OF THE INVENTION

The present invention is related to a compound of the general formula (I),

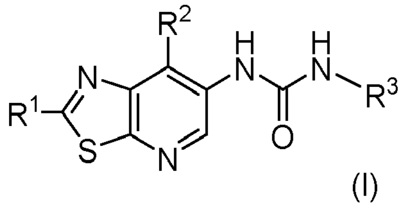

(I)

its tautomeric form, its stereoisomer, its pharmaceutically acceptable salt, polymorph, solvate, its combination with suitable medicament, its pharmaceutical composition, method of making of the compound, its use as MALT1 inhibitor, and its therapeutic utility in various pathological conditions.

CROSS-REFERENCE TO RELATED APPLICATIONS

The present application claims the benefit of Indian Provisional Patent Application Nos. 201621026107, filed on 29th July 2016, 201621043859, filed on 22nd December 2016, and 201721009450 filed on 17th March 2017, the disclosures of which are incorporated herein by reference in their entirety for all purposes.

BACKGROUND OF THE INVENTION

The present invention relates to MALT1 (Mucosa Associated Lymphoid tissue lymphoma translocation protein-1) inhibitors. MALT1 is a crucial immunomodulatory protein. Studies in Bcl-10 (Ruland et al, Cell, 104, 33-42, 2001) and MALT1-deficient (Ruland et al., Immunity, 19, 749-58, 2003; Ruefli-Brassi et al., Science, 302, 1581-84, 2003) mice revealed the importance of MALT1 for transducing antigen receptor signals to the transcription factor NF- κB (WO 2009/065897). Additionally, identification of several chromosomal translocations that leads to the generation of constitutively active MALT1 (as in the case of ABC - DLBCL) or the identification of MALT1 fusion protein API-MALT1 / IgH-MALT1 that leads to NF- κB activation independent of upstream stimulation (as in case of Malt type lymphomas) further highlight the importance of this protein in cancer.

MALT1 and its partner Bc1-10 bind to different members of CARD (caspase recruitment domain) containing CARMA (CARD containing Membrane associated guanylate kinase) family of proteins, depending on the cell lineage. The signalosome formed upon antigen receptor stimulation (via TCR or BCR pathway) in the lymphocytes involves CARMA1/CARD11, whereas CARD9 interacts with MALT1 downstream of Toll like or C-type lectin receptors. MALT1-Bc1-10 signalosome involving CARD10 links signalling via GPCR and NF- κB activation in non-immune cells (McAllister-Lucas et al., PNAS, 104, 139-44, 2007). CARD14 interacts with MALT1 (and Bcl-10) in the keratinocytes. Thus, MALT1 acts as a central protein that is involved in many diseases directly or indirectly involving the inflammatory transcription factor, NF- κB.

It has been reported that inhibitors of MALT1 proteolytic activity have antiproliferative activity against ABC type DLBCL lymphomas (Fontan et al., Cancer Cell, 22, 812-24, 2012; Nagel et al., Cancer Cell, 22, 825-37, 2012; Fontan et al., Clin Cancer Res, 19, 6662-68, 2013). Further, MALT1 has been reported to be involved in several disease pathologies, e.g., different types of oncological disorders such as lung adenocarcinoma (Jiang et al., Cancer Research, 71, 2183-92, 2011; Pan et al., Oncogene, 1-10, 2015), breast cancer (Pan et al., Mol Cancer Res, 14, 93-102, 2016), mantle cell lymphoma (Penas et al., Blood, 115, 2214-19, 2010; Rahal et al., Nature Medicine, 20, 87-95, 2014), marginal zone lymphoma (Remstein et al., Am J Pathol, 156, 1183-88, 2000; Baens et al., Cancer Res, 66, 5270-77, 2006; Ganapathi et al., Oncotarget, 1-10, 2016; Bennett et al., Am J of Surgical Pathology, 1-7, 2016), cutaneous T cell lymphomas such as Sezary syndrome (Qin et al., Blood, 98, 2778-83, 2001; Doebbeling et al., J of Exp and Clin Cancer Res, 29, 1-5, 2010), primary effusion lymphoma (Bonsignore et al., Leukemia, 31, 614-24, 2017), pancreatic cancer (Patent WO2016193339A1), certain types of chronic lymphocytic leukemia with CARD11 mutation and also certain subtypes of GCB-DLBCL type of lymphomas that involve MALT1. Moreover, targeting an immunomodulatory protein can have direct and indirect benefits in a variety of inflammatory disorders of multiple organs,for example, in treating psoriasis (Lowes et al, Ann Review Immunology, 32, 227-55, 2014; Afonina et al., EMBO Reports, 1-14, 2016; Howes et al., Biochem J, 1-23, 2016), multiple sclerosis (Jabara et al., J Allergy Clin Immunology, 132, 151-58, 2013; McGuire et al., J of Neuroinflammation, 11, 1-12, 2014), rheumatoid arthritis, Sjogren’s syndrome (Streubel et al., Clin Cancer

Research, 10, 476-80, 2004; Sagaert et al., Modern Pathology, 19, 225-32, 2006), ulcerative collitis (Liu et al., Oncotarget, 1-14, 2016), MALT lymphomas of different organs (Suzuki et al., Blood, 94, 3270-71, 1999; Akagi et al., Oncogene, 18, 5785-94, 1999) and different types of allergic disorders resulting from chronic inflammation.

In addition, several patent applications realted to MALT1 are published which are as follows: WO 2008 146259, WO 2009 065897, WO 2013 017637, WO 2013 053765, WO 2014 074815, WO 2014 086478, WO 2014 207067, WO 2015 110406, WO 2015 181747, WO 2016 193339, WO 2017 040304, WO 2017 057695, and WO 2017 081641.

The foregoing shows that there exists an unmet need for MALT1 inhibitory compounds for treating diseases or disorders involving MALT1 activation, particularly cancers as well as inflammatory disorders that are dependent on the MALT1-NF-κB axis.

BRIEF SUMMARY OF THE INVENTION

The present invention provides compounds of the general formula (I), their pharmaceutically acceptable salts, tautomeric forms, stereoisomers, polymorphs, solvates, combinations with suitable other medicament or medicaments and pharmaceutical compositions thereof and use thereof in treating various diseases or disorders including cancers.

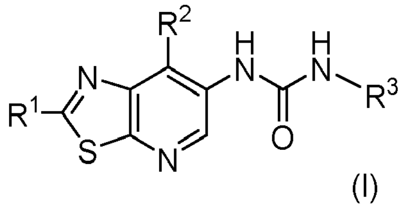

(I)

wherein, $R^1$-$R^3$ are described in detail below.The compounds of the present invention are potent inhibitors of MALT1.

According to one aspect of the present invention, there is provided a compound represented by the general formula (I), its tautomeric form, its stereoisomer, its polymorph, its solvate, its pharmaceutically acceptable salt, its combinations with suitable medicament and its pharmaceutical compositions, wherein, $R^1$-$R^3$ are described in detail below.

The present invention provides a pharmaceutical composition, containing the compound of the general formula (I) as defined herein, its tautomeric form, and its stereoisomer, its polymorph, its solvate, its pharmaceutically acceptable salt in combination with the usual pharmaceutically employed carriers, diluents, and the like are useful for the treatment of a disease or disorder mediated through MALT1.

The present invention provides a pharmaceutical composition, containing the compound of the general formula (I) as defined herein, its tautomeric form, its stereoisomer, its polymorph, its solvate, and its pharmaceutically acceptable salt in combination with the usual pharmaceutically employed carriers, diluents, and the like are useful for the treatment of a disease or disorder such as cancer, inflammation or inflammatory disease or disorder, or allergic or autoimmune disease or disorder.

The present invention provides a pharmaceutical composition, containing the compound of the general formula (I) as defined herein, its tautomeric form, its stereoisomer, its polymorph, its solvate, and its pharmaceutically acceptable salt in combination with the usual pharmaceutically employed carriers, diluents, and the like are useful for the treatment of a disease or disorder such as ABC-DLBCL type of lymphomas, a subset of GCB-DLBCL type of lymphomas involving MALT1, MALT lymphomas, mantle cell lymphoma, marginal zone lymphoma, cutaneous T cell lymphomas, primary effusion lymphoma, pancreatic cancer, chronic lymphocytic leukemia with CARD11 mutation, Hodgkin's and Non-Hodgkin's lymphomas, or a subset of acute myelogenous leukemia involving MALT1, germ cell tumors and neoplasm involving plasma cell, brain tumors including glioblastoma, hepatic adenomas, medulloblastoma, mesothelioma, different types of melanomas and multiple myeloma, clear cell carcinoma, or adenocarcinoma of lung, breast, bladder, skin, brain, colon, stomach, cervix, ovary, uterus, prostate, liver, and kidney, psoriasis, multiple sclerosis, systemic lupus erythematosus, BENTA disease, ulcerative colitis, pancreatitis, rheumatic fever, or rheumatoid arthritis, ankylosing spondylitis, inflammatory bowel disease, Crohn's disease, gastritis, celiac disease, gout, organ or transplant rejection, chronic allograft rejection, acute or chronic graft-versus-host disease, Behcet's disease, uveitis, dermatitis including atopic dermatitis, dermatomyositis, inflammation of skeletal muscles leading to polymyositis, myasthenia gravis, Grave's disease, Hashimoto thyroiditis, blistering disorders, vasculitis syndromes, Hennoch-Schonlein Purpura, or immune-complex vasculitides, Sjoren's syndrome, asthma, bronchitis, or chronic obstructive pulmonary disease, cystic fibrosis, and respiratory diseases involving lungs leading to respiratory distress and failure.

DETAILED DESCRIPTION OF THE INVENTION

The present invention is related to a compound of the general formula (I), its tautomeric form, its stereoisomer, its pharmaceutically acceptable salt, its polymorph, its solvate, its combination with suitable one or more other medicaments, its pharmaceutical composition, method of making of the compound, its use as MALT1 inhibitor, and its therapeutic utility in treating, or ameliorating various pathological conditions. The compounds are of formula (I) below:

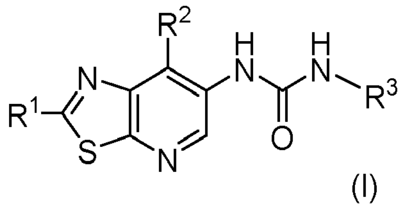

(I)

wherein,

$R^1$ is selected from hydrogen, halogen, cyano, substituted or unsubstituted alkyl, and cycloalkyl;

$R^2$ is selected from -

a) alkyl or alkyl substituted with 1 to 4 substituents independently selected from oxo (=O), halogen, cyano, cycloalkyl, substituted or unsubstituted aryl, heteroaryl, substituted or unsubstituted heterocyclyl, $-OR^4$, -C(=O)OH, $-SO_2$(alkyl), - C(=O)O(alkyl), $-NR^5R^{5a}$, $-NR^5C(=O)R^6$, $-C(=O)R^6$, and $-C(=O)NR^5R^{5a}$,

b) cycloalkyl or cycloalkyl substituted with 1 to 4 substituents independently selected from halogen, cyano, substituted or unsubstituted alkyl, $-OR^4$, -C(=O)OH, - C(=O)O(alkyl), $-C(=O)R^6$, and $-C(=O)NR^5R^{5a}$,

c) cycloalkenyl,

d) cyano,

e) substituted or unsubstituted aryl,

f) substituted or unsubstituted heteroaryl,

g) heterocyclyl or heterocyclyl substituted on either ring carbon atom or a ring nitrogen atom and when it is substituted on ring carbon atom it is substituted with 1 to 4 substituents independently selected from oxo (=O), halogen, cyano, substituted or unsubstituted alkyl, cycloalkyl, $-OR^4$, -C(=O)OH, -C(=O)O-alkyl, $-C(=O)NR^5N^{5a}$, - N(H)C(=O)(alkyl), $-N(H)R^5$, and $-N(alkyl)_2$, and when the heterocycle group is substituted on a ring nitrogen, it is substituted with substituents independently selected from alkyl, cycloalkyl, aryl, heteroaryl, $-SO_2$(alkyl), $-C(=O)R^6$, C(=O)O(alkyl), $-C(=O)N(H)R^5$, and $-C(=O)N(alkyl)R^5$, and

h) $-NR^aR^b$, wherein, $R^a$ and $R^b$ are independent selected from hydrogen, cycloalkyl, and alkyl or alkyl substituted with 1 to 4 substituents independently selected from oxo (=O), halogen, cycloalkyl, $-OR^4$, and substituted or unsubstituted aryl;

$R^3$ is selected from -

a) heteroaryl or heteroaryl substituted with 1 to 4 substituents selected from halogen, cyano, $-COOR^{4b}$, $-OR^{4a}$, substituted or unsubstituted heteroaryl, substituted or unsubstituted alkyl, substituted or unsubstituted heterocyclyl, substituted or unsubstituted aryl, nitro, $-SO_2$alkyl, $-SO_2$NH(alkyl), $-SO_2NH_2$, - $SO_2NH(CF_3)$, $-SO_2N(alkyl)_2$, $-NHSO_2$(alkyl), $-COR^6$, -CON(H)OH, $-CONR^5R^{5a}$, - $N(R^5)COR^{5a}$, and $-NR^5R^{5a}$,

b) aryl or aryl substituted with 1 to 4 substituents selected from halogen, cyano, - $COOR^{4b}$, $-OR^{4a}$, substituted or unsubstituted alkyl, substituted or unsubstituted heterocyclyl, substituted or unsubstituted aryl, nitro, $-SO_2$alkyl, $-SO_2$NH(alkyl), - $SO_2NH_2$, $-SO_2NH(CF_3)$, $-SO_2N(alkyl)_2$, $-NHSO_2$(alkyl), $-COR^6$, $-CONR^5R^{5a}$, -CO(NH)OH, $-N(R^5)COR^{5a}$, $-NR^5R^{5a}$, and heteroaryl or heteroaryl substituted with 1 to 4 substituents selected

from substituted or unsubstituted alkyl,

c) heterocyclyl or heterocyclyl substituted with 1 to 4 substituents selected from oxo (=O) and substituted or unsubstituted alkyl, and

d)

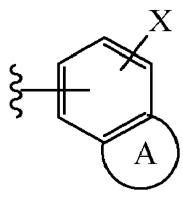

,

wherein, X is halogen and ring A is a heterocyclic ring containing heteroatom(s) selected from S, O, and N, which is optionally substituted with an oxo (=O) group;

$R^4$ is selected from hydrogen, cycloalkyl, and substituted or unsubstituted alkyl;

$R^{4a}$ is selected from

a) hydrogen, alkyl, and cycloalkyl, and

b) alkyl substituted with 1 to 4 substituents independently selected from halogen, - O-alkyl, $-NR^5R^{5a}$, and substituted or unsubstituted heterocyclyl;

$R^{4b}$ is selected from hydrogen and alkyl;

$R^5$ and $R^{5a}$ are each independently selected from

a) hydrogen, alkyl, and cycloalkyl,

b) alkyl substituted with -O-alkyl, $-NH_2$, and $-CONH_2$,

c) heteroaryl, and

d) heterocyclyl substituted with alkyl; and

$R^6$ is selected from alkyl, heterocyclyl, and cycloalkyl;

when an alkyl group is substituted, it is substituted with 1 to 4 substituents independently selected from oxo (=O), halogen, cyano, cycloalkyl, aryl, heteroaryl, heterocyclyl, $-OR^7$, -C(=O)OH, -C(=O)O(alkyl), $-NR^8R^{8a}$, $-NR^8C(=O)R^9$, and - $C(=O)NR^8R^{8a}$;

when the aryl group is substituted, it is substituted with 1 to 4 substituents independently selected from halogen, nitro, cyano, alkyl, perhaloalkyl, cycloalkyl, heterocyclyl, heteroaryl, $-OR^7$, $-NR^8R^{8a}$, $-NR^8C(=O)R^9$, $-C(=O)R^9$, $-C(=O)NR^8R^{8a}$, $-SO_2$-alkyl, -C(=O)OH, -C(=O)O-alkyl, and haloalkyl;

when the heteroaryl group is substituted, it is substituted with 1 to 4 substituents independently selected from halogen, nitro, cyano, alkyl, haloalkyl, perhaloalkyl, cycloalkyl, heterocyclyl, aryl, heteroaryl, $-OR^7$, $-NR^8R^{8a}$, $-NR^7C(=O)R^9$, $-C(=O)R^9$, - $C(=O)NR^8R^{8a}$, $-SO_2$-alkyl, -C(=O)OH, and -C(=O)O-alkyl;

when the heterocycle group is substituted, it is substituted either on a ring carbon atom or on a ring hetero atom, and when it is substituted on a ring carbon atom, it is substituted with 1 to 4 substituents independently selected from oxo (=O), halogen, cyano, alkyl, cycloalkyl, perhaloalkyl, $-OR^7$, $-C(=O)NR^8R^{8a}$, -C(=O)OH, -C(=O)O-alkyl, -N(H)C(=O)(alkyl), $-N(H)R^8$, and $-N(alkyl)_2$; and when the heterocycle group is substituted on a ring nitrogen, it is substituted with substituents independently selected from alkyl, cycloalkyl, aryl, heteroaryl, $-SO_2(alkyl)$, $-C(=O)R^9$,

and - C(=O)O(alkyl); when the heterocycle group is substituted on a ring sulfur, it is substituted with 1 or 2 oxo (=O) group(s);

$R^7$ is selected from hydrogen, alkyl, perhaloalkyl, and cycloalkyl;

$R^8$ and $R^{8a}$ are each independently selected from hydrogen, alkyl, and cycloalkyl; and $R^9$ is selected from alkyl and cycloalkyl.

In accordance with an embodiment of the invention, $R^1$ is selected from hydrogen and substituted or unsubstituted alkyl.

In certain embodiments, $R^1$ is selected from hydrogen, methyl, ethyl, and $-CF_3$.

In any of the above embodiments, $R^2$ is selected from

a) alkyl or alkyl substituted with 1 to 4 substituents independently selected from halogen, cycloalkyl, substituted or unsubstituted heterocyclyl, $-OR^4$, $-NR^5R^{5a}$, and substituted or unsubstituted aryl,

b) cycloalkyl or cycloalkyl substituted with substituted or unsubstituted alkyl,

c) cycloalkenyl,

d) substituted or unsubstituted aryl,

e) substituted or unsubstituted heteroaryl,

f) heterocyclyl or heterocyclyl substituted on ring carbon atom with 1 to 2 substituents independently selected from halogen, $-OR^4$, and substituted or unsubstituted alkyl, and

g) $-NR^aR^b$, wherein $R^a$ and $R^b$ are independent selected from cycloalkyl and alkyl or alkyl substituted with 1 to 2 substituents independently selected from cycloalkyl, $OR^4$, and substituted or unsubstituted aryl.

In certain embodiments, $R^2$ is selected from

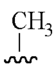 , 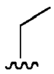 , 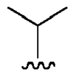 , 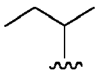 , 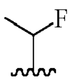 , 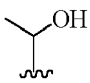 , 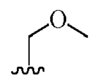 , 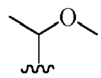 , 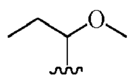 ,

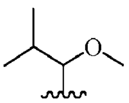 , 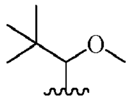 , 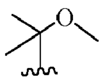 , 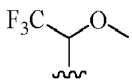 , 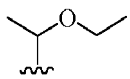 , 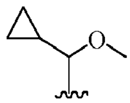 , 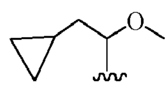 ,

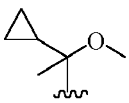 , 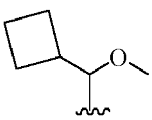 , 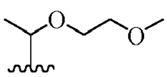 , 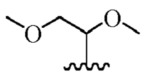 , 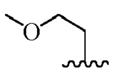 , 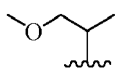 ,

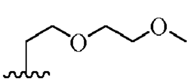 , 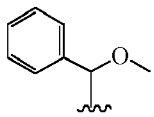 , 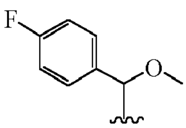 , 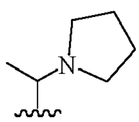 , 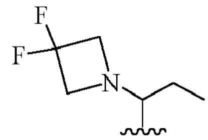 ,

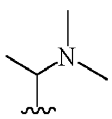 , 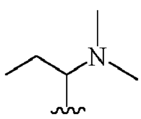 , 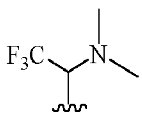 , 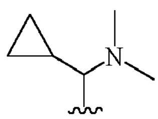 , 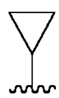 , 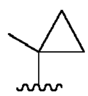 ,

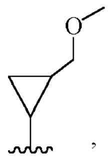, 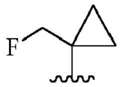, 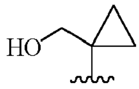, 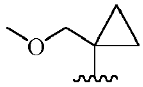, 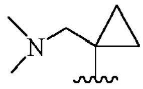, 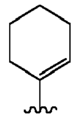, 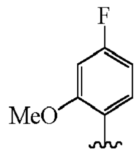,

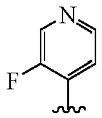, 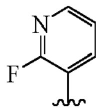, 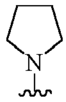, 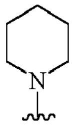, 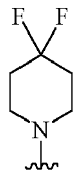, 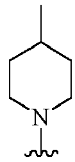, 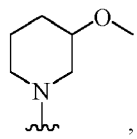, 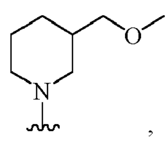,

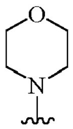, 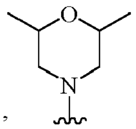, 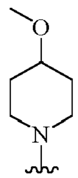, 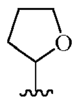, 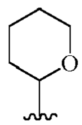, 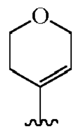, 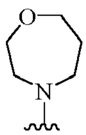, 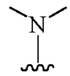, 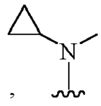, 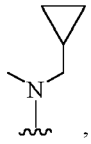,

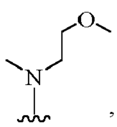, 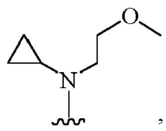, 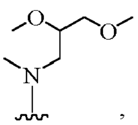, 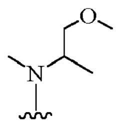, 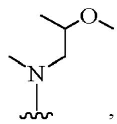, 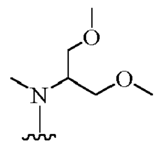, and 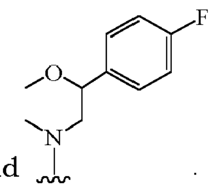.

In any of the above embodiments, $R^3$ is selected from

a) heteroaryl substituted with 1 to 3 substitutents selected from halogen, cyano, - $OR^{4a}$, substituted or unsubstituted heteroaryl, substituted or unsubstituted alkyl, and substituted or unsubstituted heterocyclyl,

b) aryl substituted with 1 to 3 substituents selected from halogen, cyano, -$OR^{4a}$, $COOR^{4b}$, substituted or unsubstituted alkyl, and heteroaryl or heteroaryl substituted with 1 to 4 substituents selected from substituted or unsubstituted alkyl,

c) heterocyclyl substituted with 1 to 3 substituents selected from oxo (=O) and substituted or unsubstituted alkyl, and

d)

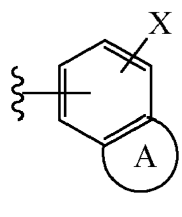,

wherein, X is chlorine and ring A is a heterocyclic ring containing N, which is optionally substituted with an oxo (=O) group.

In certain embodiments, $R^3$ is selected from

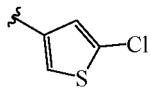, 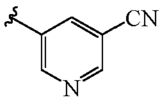, 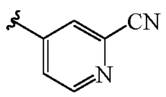, 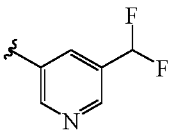, 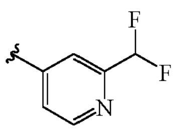, 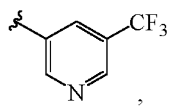,

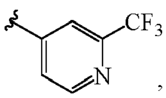, 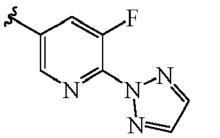, 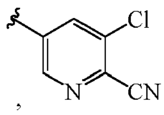, 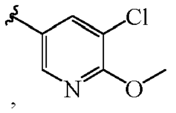, 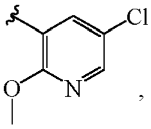, 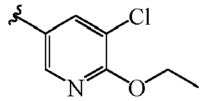,

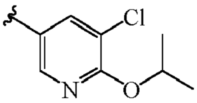, 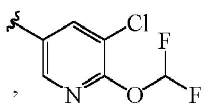, 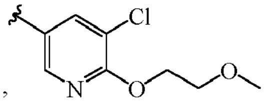, 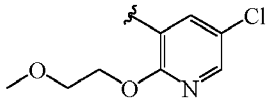,

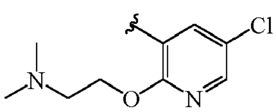, 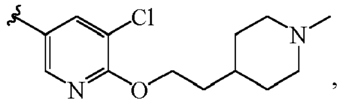, 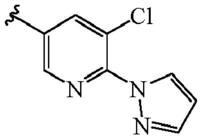, 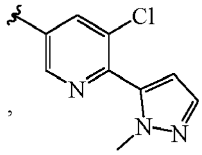,

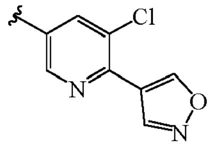, 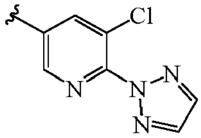, 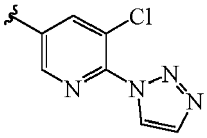, 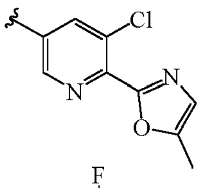, 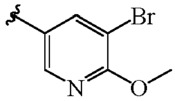,

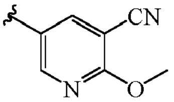, 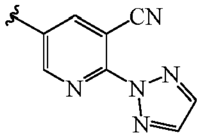, 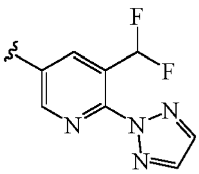, 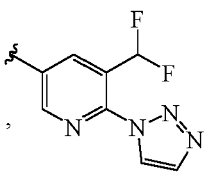, 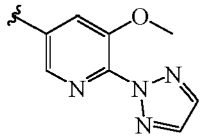,

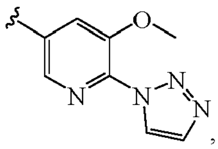, 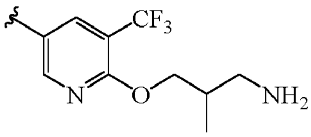, 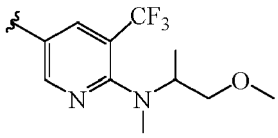, 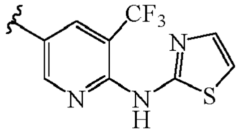,

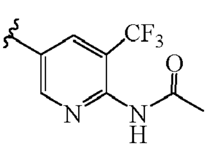, 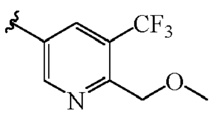, 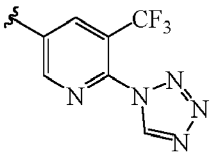,

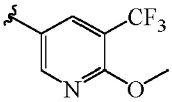, 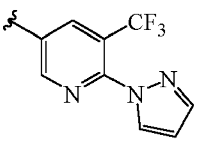, 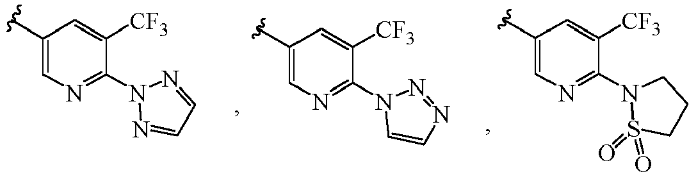, , ,

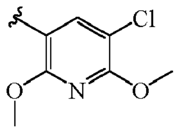, 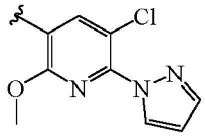, 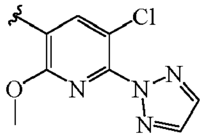, 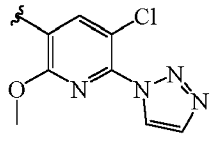, 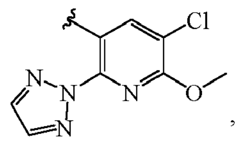,

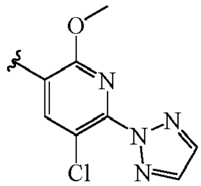, 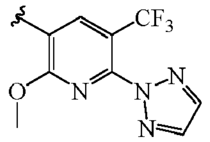, 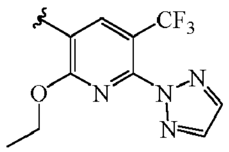, 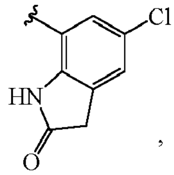, 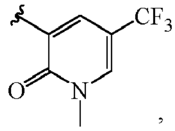,

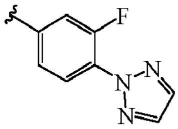, 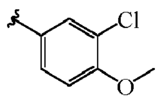, 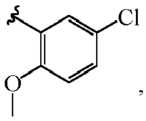, 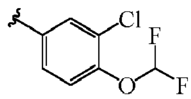, 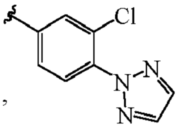, 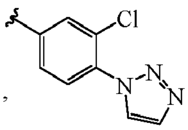,

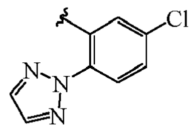, 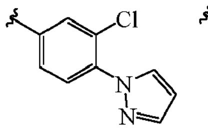, 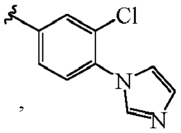, 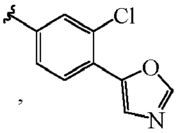, 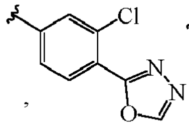, 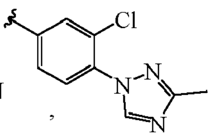,

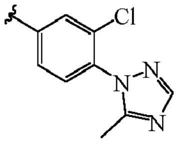, 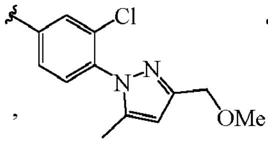, 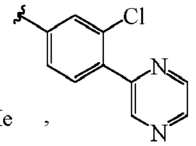, 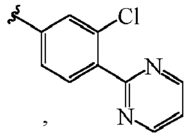, 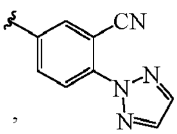, 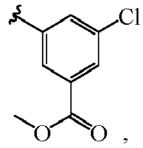,

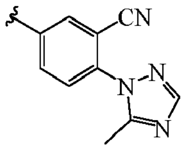, 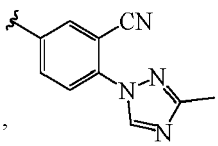, 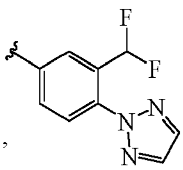, 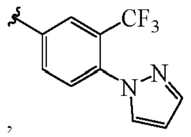, 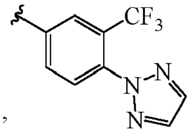, 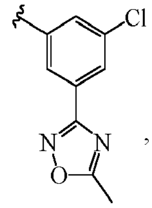,

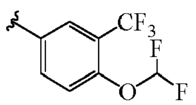, 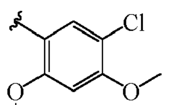, 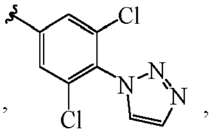, 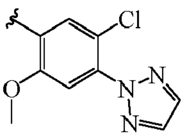, 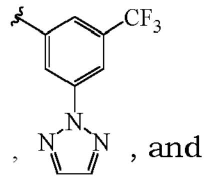, and 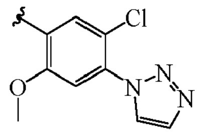.

In any of the above embodiments, $R^4$ is selected from hydrogen and substituted or unsubstituted alkyl.

In any of the above embodiments, $R^{4a}$ is selected from alkyl or alkyl substituetd with 1 to 2 substituents independently selected from halogen, -O-alkyl, $-NR^5R^{5a}$, and substituted or unsubstituted heterocyclyl.

In any of the above embodiments, $R^{4b}$ is alkyl.

In any of the above embodiments, $R^5$ and $R^{5a}$ are each independently selected from alkyl.

Whenever a range of the number of atoms in a structure is indicated (e.g., a $C_1$ to $C_{20}$ alkyl etc.), it is specifically contemplated that any sub-range or individual number of carbon atoms falling within the indicated range also can be used. Thus, for instance, the recitation of a range of 1-6 carbon atoms (e.g., $C_1$ to $C_6$), 2-6 carbon atoms (e.g., $C_2$ to $C_6$), 3-6 carbon atoms (e.g., $C_3$ to $C_6$), as used with respect to any chemical group (e.g., alkyl etc.) referenced herein encompasses and specifically describes 1, 2, 3, 4, 5, and/or 6 carbon atoms, as appropriate, as well as any sub-range thereof (e.g., 1-2 carbon atoms, 1-3 carbon atoms, 1-4 carbon atoms, 1-5 carbon atoms, 1-6 carbon atoms, 2-3 carbon atoms, 2-4 carbon atoms, 2-5 carbon atoms, 2-6 carbon atoms, 3-4 carbon atoms, 3-5 carbon atoms, 3-6 carbon atoms, 4-5 carbon atoms, 4-6 carbon atoms, as appropriate).

General terms used in formula can be defined as follows; however, the meaning stated should not be interpreted as limiting the scope of the term *per se*.

The term 'alkyl', as used herein, means a straight chain or branched hydrocarbon containing from 1 to 20 carbon atoms. Preferably, the alkyl chain may contain 1 to 10 carbon atoms. More preferably, alkyl chain may contain up to 6 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, and n-hexyl.

The term 'haloalkyl', as used herein means an alkyl group as defined hereinabove wherein at least one of the hydrogen atoms of the said alkyl group is substituted with halogen. The haloalkyl group is exemplified by chloromethyl, 1-chloroethyl, and the like.

The term 'perhaloalkyl', as used herein, means an alkyl group as defined hereinabove wherein all the hydrogen atoms of the said alkyl group are substituted with halogen. The perhaloalkyl group is exemplified by trifluoromethyl, pentafluoroethyl, and the like.

The term 'cycloalkyl' as used herein, means a monocyclic, bicyclic, or tricyclic nonaromatic ring system containing from 3 to 14 carbon atoms, preferably monocyclic cycloalkyl ring containing 3 to 6 carbon atoms. Examples of monocyclic ring systems include cyclopropyl, cyclobutyl, cyclopentyl, cyclohexyl, cycloheptyl, and cyclooctyl. Bicyclic ring systems include monocyclic ring system fused across a bond with another cyclic system which may be an alicyclic ring or an aromatic ring. Bicyclic rings also include spirocyclic systems wherein the second ring gets annulated on a single carbon atom. Bicyclic ring systems are also exemplified by a bridged monocyclic ring system in which two non-adjacent carbon atoms of the monocyclic ring are linked by an alkylene bridge. Representative examples of bicyclic ring systems include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo [4.2.1]nonane, bicyclo[3.3.2]decane, bicyclo[3.1.0]hexane, bicyclo[4.1.0]heptane, bicyclo[3.2.0]heptanes, octahydro-1H-indene, spiro[2.5]octane, spiro[4.5]decane, spiro[bicyclo[4.1.0]heptane-2,1'-cyclopentane], hexahydro-2'H-spiro[cyclopropane-1,1'-pentalene]. Tricyclic ring systems are the systems wherein the bicyclic systems as described above are further annulated with third ring, which may be an alicyclic ring or aromatic ring. Tricyclic ring systems are also exemplified by a bicyclic ring system in which two non-adjacent carbon atoms of the bicyclic ring are linked by a bond or an alkylene bridge. Representative examples of tricyclic-ring systems include, but are not limited to, tricyclo[3.3.1.$0^{3.7}$]nonane, and tricyclo[3.3.1.$1^{3.7}$]decane (adamantane).

The term 'cycloalkenyl' as used herein, means a cycloalkyl group as defined above containing at least one double bond.

The term 'aryl', as used herein, refers to a monovalent monocyclic, bicyclic or tricyclic aromatic hydrocarbon ring system. Examples of aryl groups include phenyl, naphthyl, anthracenyl, fluorenyl, indenyl, azulenyl, and the like. Aryl group also include partially saturated bicyclic and tricyclic aromatic hydrocarbons, e.g. tetrahydro-naphthalene. Aryl group also include bicyclic systems like 2,3-dihydro-indene-5-yl, and 2,3-dihydro-l-indenone-5-yl.

The term 'heteroaryl', as used herein, refers to a 5-14 membered monocyclic, bicyclic, or tricyclic ring system having 1-4 ring heteroatoms selected from O, N, or S, and the remainder ring atoms being carbon (with appropriate hydrogen atoms unless otherwise indicated), wherein at least one ring in the ring system is aromatic. The term 'heteroaryl' as used herein, also include partially saturated bicyclic and tricyclic aromatic ring system, e.g. 2,3-dihydro-isobenzofuran-5-yl, 2,3-dihydro-1-isobenzofuranone-5-yl, 2,3-dihydro-1H-indol-4-yl, 2,3-dihydro-1H-indol-6-yl, and 2,3-dihydro-1-isoindolinone-5-yl. Heteroaryl groups may be optionally substituted with one or more substituents. In one embodiment, 0, 1, 2, 3, or 4 atoms of each ring of a heteroaryl group may be substituted by a substituent. Examples of heteroaryl groups include, but not limited to, 1*H*-1,2,3-triazolyl, 2*H*-1,2,3-triazolyl , pyridyl, 1-oxo-pyridyl, furanyl, thienyl, pyrrolyl, oxazolyl, oxadiazolyl, imidazolyl, thiazolyl, isoxazolyl, quinolinyl, pyrazolyl, isothiazolyl, pyridazinyl, pyrimidinyl, pyrazinyl, triazinyl, triazolyl, thiadiazolyl, isoquinolinyl, benzoxazolyl, benzofuranyl, indolizinyl, imidazopyridyl, imidazolyl, tetrazolyl, benzimidazolyl, benzothiazolyl, benzothiadiazolyl, benzoxadiazolyl, indolyl, azaindolyl, imidazopyridyl, quinazolinyl, purinyl, pyrrolo[2,3]pyrimidinyl, pyrazolo[3,4]pyrimidinyl, and benzo(b)thienyl, 2,3-thiadiazolyl, 1H-pyrazolo[5,1-c]-1,2,4-triazolyl, pyrrolo[3,4-d]-1,2,3-triazolyl, cyclopentatriazolyl, 3H-pyrrolo[3,4-c] isoxazolyl, 2,3-dihydro-benzo[1,4]dioxin-6-yl, 2,3-dihydro-benzo[1,4]dioxin-5-yl, 2,3-dihydro-benzofuran-5-yl, 2,3-dihydrobenzofuran-4-yl, 2,3-dihydro-benzofuran-6-yl, 2,3-dihydro-benzofuran-6-yl, 2,3-dihydro-isobenzofuran-5-yl, 2,3-dihydro-1-isobenzofuranone-5-yl, 2,3-dihydro-1H-indol-5-yl, 2,3-dihydro-1H-indol-4-yl, 2,3-dihydro-1H-indol-6-yl, 2,3-dihydro-1H-indol-7-yl, 2,3-dihydro-1-isoindolinone-5-yl, benzo[1,3]dioxol-4-yl, benzo[1,3]dioxol-5-yl, 1,2,3,4-tetrahydroquinolinyl, 1,2,3,4-tetrahydroisoquinolinyl, 2,3-dihydrobenzothien-4-yl, 2-oxoindolin-5-yl and the like.

The term 'heterocycle' or 'heterocyclic' or 'heterocyclyl' as used herein, means a 'cycloalkyl' or 'cycloalkenyl' group wherein one or more of the carbon atoms are replaced by heteroatoms/groups selected from N, S, $SO_2$ and O. The heterocycle may be connected to the parent molecular moiety through any carbon atom or any nitrogen atom contained within the heterocycle. Representative examples of monocyclic heterocycle include, but are not limited to, azetidinyl, azepanyl, aziridinyl, diazepanyl, 1,3-dioxanyl, 1,3-dioxolanyl, 1,3-dithiolanyl, 1,3-dithianyl, imidazolinyl, imidazolidinyl, isothiazolinyl, isothiazolidinyl, isoxazolinyl, isoxazolidinyl, morpholinyl, oxadiazolinyl, oxadiazolidinyl, oxazolinyl, oxazolidinyl, piperazinyl, piperidinyl, pyranyl, pyrazolinyl, pyrazolidinyl, pyrrolinyl, pyrrolidinyl, tetrahydrofuranyl,

tetrahydrothienyl, thiadiazolinyl, thiadiazolidinyl, thiazolinyl, thiazolidinyl, thiomorpholinyl, 1.1-dioxidothiomorpholinyl (thiomorpholine sulfone), thiopyranyl, and trithianyl. Representative examples of bicyclic heterocycle include, but are not limited to, 1,2,3,4-tetrahydroisoquinolin-2-yl, 1,2,3,4-tetrahydroquinolin-1-yl, 1,3-benzodioxolyl, 1,3-benzodithiolyl, 2,3-dihydro-1,4-benzodioxinyl, 2,3-dihydro-1-benzofuranyl, 2,3-dihydro-1-benzothienyl, 2,3-dihydro-1H-indolyl, and 1,2,3,4-tetrahydroquinolinyl. The term heterocycle also includes bridged and spiro heterocyclic systems such as azabicyclo[3.2.1]octane, azabicyclo[3.3.1]nonane, 8-oxa-3-azabicyclo[3.2.1]octan-3-yl, 3-oxa-8-azabicyclo[3.2.1]octan-8-yl, 6-oxa-3-azabicyclo[3.1.1]heptan-3-yl, 8-azabicyclo[3.2.1]octan-8-yl, 3-azabicyclo[3.2.1]octan-3-yl, 3-azabicyclo[3.1.0]hexan-3-yl, 6-azaspiro[2.5]octan-6-yl, 5-azaspiro[2.5]octan-5-yl, 4-azaspiro[2.4]heptan-4-yl, and the like.

The 'halogen' means fluorine, chlorine, bromine, or iodine. The halogen group is exemplified by fluorine, chlorine, and bromine.

The term 'oxo' means a divalent oxygen (=O) attached to the parent group. For example, oxo attached to carbon forms a carbonyl, oxo substituted on cyclohexane forms a cyclohexanone, and the like.

The term 'annulated' means the ring system under consideration is either annulated with another ring at a carbon atom of the cyclic system or across a bond of the cyclic system as in the case of fused or spiro ring systems.

The term 'bridged' means the ring system under consideration contain an alkylene bridge having 1 to 4 methylene units joining two non-adjacent ring atoms.

A compound, its tautomeric form, its stereoisomer, its pharmaceutically acceptable salt, its polymorph, its solvate, its combination with suitable medicament, its pharmaceutical composition thereof as described hereinabove wherein the compound of general formula (I), is selected from the group consisting of:

1. 1-(5-Chloro-6-methoxypyridin-3-yl)-3-(7-cyclopropyl-2-methylthiazolo [5,4-b]pyridin-6-yl)urea (Compound 1);

2. 1-(3-Chloro-4-methoxyphenyl)-3-(7-cyclopropyl-2-methylthiazolo[5,4-b]pyridin-6-yl)urea (Compound 2);

3. 1-(7-Cyclopropyl-2-methylthiazolo[5,4-b]pyridin-6-yl)-3-(2-(trifluoromethyl)pyridin-4-yl)urea (Compound 3);

4. 1-(5-Chloro-6-ethoxypyridin-3-yl)-3-(7-cyclopropyl-2-methylthiazolo[5,4-b]pyridin-6-yl)urea (Compound 4);

5. 1-(7-Cyclopropyl-2-methylthiazolo[5,4-b]pyridin-6-yl)-3-(1-methyl-2-oxo-5-(trifluoromethyl)-1,2-dihydropyridin-3-yl)urea (Compound 5);

6. 1-(5-Chloro-6-isopropoxypyridin-3-yl)-3-(7-cyclopropyl-2 methylthiazolo[5,4-b]pyridin-6-yl)urea (Compound 6);

7. 1-(7-Cyclopropyl-2-methylthiazolo[5,4-b]pyridin-6-yl)-3-(5-(trifluoromethyl)pyridin-3-yl)urea (Compound 7);

8. 1-(7-Cyclopropyl-2-methylthiazolo[5,4-b]pyridin-6-yl)-3-(6-methoxy-5-(trifluoromethyl)pyridin-3-yl)urea (Compound 8);

9. 1-(5-Chloro-6-(2H-1,2,3-triazol-2-yl)pyridin-3-yl)-3-(7-cyclopropyl-2-methylthiazolo[5,4-b]pyridin-6-yl)urea (Compound 9);

10. 1-(5-Cyanopyridin-3-yl)-3-(7-cyclopropyl-2-methylthiazolo[5,4-b]pyridin-6-yl)urea (Compound 10);

11. 1-(7-Cyclopropyl-2-methylthiazolo[5,4-b]pyridin-6-yl)-3-(5-(difluoromethyl)pyridin-3-yl)urea (Compound 11);

12. 1-(2-Cyanopyridin-4-yl)-3-(7-cyclopropyl-2-methylthiazolo[5,4-b]pyridin-6-yl)urea (Compound 12);

13. 1-(5-Chloro-6-methoxypyridin-3-yl)-3-(2,7-dimethylthiazolo[5,4-b]pyridin-6-yl)urea (Compound 13);

14. 1-(3-Chloro-4-methoxyphenyl)-3-(2,7- dimethylthiazolo[5,4-b]pyridin-6-yl)urea (Compound 14);

15. 1-(5-Chloro-6-methoxypyridin-3-yl)-3-(7-(4-fluoro-2-methoxyphenyl)-2-methylthiazolo[5,4-b]pyridin-6-yl)urea (Compound 15);

16. 1-(5-Chloro-6-methoxypyridin-3-yl)-3-(7-(2-fluoropyridin-3-yl)-2-methylthiazolo[5,4-b]pyridin-6-yl)urea (Compound 16);

17. 1-(3-Chloro-4-methoxyphenyl)-3-(7-(2-fluoropyridin-3-yl)-2-methylthiazolo[5,4-b]pyridin-6-yl)urea (Compound

17);

18. 1-(5-Chloro-6-methoxypyridin-3-yl)-3-(7-(3-fluoropyridin-4-yl)-2-methylthiazolo[5,4-b]pyridin-6-yl)urea (Compound 18);

19. 1-(6-(2H-1,2,3-Triazol-2-yl)-5-(trifluoromethyl)pyridin-3-yl)-3-(7-cyclopropyl-2-methylthiazolo[5,4-b]pyridin-6-yl)urea (Compound 19);

20. 1-(5-Chloro-6-(difluoromethoxy)pyridin-3-yl)-3-(7-cyclopropyl-2-methylthiazolo[5,4-b]pyridin-6-yl)urea (Compound 20);

21. 1-(5-Chloro-2-oxoindolin-7-yl)-3-(7-cyclopropyl-2-methylthiazolo[5,4-b]pyridin-6-yl)urea (Compound 21);

22. 1-(7-Cyclopropyl-2-methylthiazolo[5,4-b]pyridin-6-yl)-3-(5-methoxy-6-(2H-1,2,3-triazol-2-yl)pyridin-3-yl)urea (Compound 22);

23. 1-(7-Cyclopropyl-2-methylthiazolo[5,4-b]pyridin-6-yl)-3-(6-(1,1-dioxidoisothiazolidin-2-yl)-5-(trifluoromethyl)pyridin-3-yl)urea (Compound 23);

24. 1-(7-Cyclopropyl-2-methylthiazolo[5,4-b]pyridin-6-yl)-3-(5-methoxy-6-(1H-1,2,3-triazol-1-yl)pyridin-3-yl)urea (Compound 24);

25. 1-(3-Chloro-4-methoxyphenyl)-3-(7-ethyl-2-methylthiazolo[5,4-b]pyridin-6-yl)urea (Compound 25);

26. 1-(5-Chloro-6-methoxypyridin-3-yl)-3-(7-ethyl-2-methylthiazolo[5,4-b]pyridin-6-yl)urea (Compound 26);

27. 1-(5-Chloro-6-(2H-1,2,3-triazol-2-yl)pyridin-3-yl)-3-(7-(4,4-difluoropiperidin-1-yl)-2-methylthiazolo[5,4-b]pyridin-6-yl)urea (Compound 27);

28. 1-(5-Chloro-6-(2H-1,2,3-triazol-2-yl)pyridin-3-yl)-3-(2-methyl-7-morpholinothiazolo[5,4-b]pyridin-6-yl)urea (Compound 28);

29. 1-(5-Chloro-6-(2H-1,2,3-triazol-2-yl)pyridin-3-yl)-3-(7-(4-methoxypiperidin-1-yl)-2-methylthiazolo[5,4-b]pyridin-6-yl)urea (Compound 29);

30. 1-(5-Chloro-6-methoxypyridin-3-yl)-3-(7-(4-methoxypiperidin-1-yl)-2-methylthiazolo[5,4-b]pyridin-6-yl)urea (Compound 30);

31. 1-(5-Chloro-6-(2*H*-1,2,3-triazol-2-yl)pyridin-3-yl)-3-(7-cyclopropyl-2-ethylthiazolo[5,4-b]pyridin-6-yl)urea (Compound 31);

32. 1-(5-Chloro-6-(2H-1,2,3-triazol-2-yl)pyridin-3-yl)-3-(7-(2-methoxyethyl)-2-methylthiazolo[5,4-b]pyridin-6-yl)urea (Compound 32);

33. 1-(5-Chloro-6-methoxypyridin-3-yl)-3-(7-(2-methoxyethyl)-2-methylthiazolo[5,4-b]pyridin-6-yl)urea (Compound 33);

34. 1-(5-Chloro-6-methoxypyridin-3-yl)-3-(7-(1,2-dimethoxyethyl)-2-methylthiazolo[5,4-b]pyridin-6-yl)urea (Compound 34);

35. 1-(5-Chloro-6-(2H-1,2,3-triazol-2-yl)pyridin-3-yl)-3-(7-cyclopropylthiazolo[5,4-b]pyridin-6-yl)urea (Compound 35);

36. 1-(5-Chloro-6-methoxypyridin-3-yl)-3-(7-cyclopropylthiazolo[5,4-b]pyridin-6-yl)urea (Compound 36);

37. 1-(5-Chloro-6-(2H-1,2,3-triazol-2-yl)pyridin-3-yl)-3-(2-methyl-7-(4-methylpiperidin-1-yl)thiazolo[5,4-b]pyridin-6-yl)urea (Compound 37);

38. 1-(5-Chloro-6-methoxypyridin-3-yl)-3-(7-(2,6-dimethylmorpholino)-2-methylthiazolo[5,4-b]pyridin-6-yl)urea (Compound 38);

39. 1-(5-Chloro-6-(2H-1,2,3-triazol-2-yl)pyridin-3-yl)-3-(7-(2,6-dimethylmorpholino)-2-methylthiazolo[5,4-b]pyridin-6-yl)urea (Compound 39);

40. 1-(5-Chloro-6-(2H-1,2,3-triazol-2-yl)pyridin-3-yl)-3-(2-methyl-7-(piperidin-1-yl)thiazolo[5,4-b]pyridin-6-yl)urea (Compound 40);

41. 1-(5-Chloro-6-methoxypyridin-3-yl)-3-(7-((cyclopropylmethyl)(methyl)amino)-2-methylthiazolo[5,4-b]pyridin-6-yl)urea (Compound 41);

42. 1-(5-Chloro-6-(2H-1,2,3-triazol-2-yl)pyridin-3-yl)-3-(7-((cyclopropylmethyl)(methyl)amino)-2-methylthiazolo[5,4-b]pyridin-6-yl)urea (Compound 42);

43. 1-(5-Chloro-6-(2H-1,2,3-triazol-2-yl)pyridin-3-yl)-3-(7-((2,3-dimethoxypropyl)(methyl)amino)-2-methylthiazolo[5,4-b]pyridin-6-yl)urea (Compound 43);

44. 1-(5-Chloro-6-(2H-1,2,3-triazol-2-yl)pyridin-3-yl)-3-(7-((2-methoxyethyl)(methyl)amino)-2-methylthiazolo[5,4-b]pyridin-6-yl)urea (Compound 44);

45. 1-(5-Chloro-6-methoxypyridin-3-yl)-3-(7-((2-methoxyethyl)(methyl)amino)-2-methylthiazolo[5,4-b]pyridin-6-yl)urea (Compound 45);

46. 1-(5-Chloro-6-(2H-1,2,3-triazol-2-yl)pyridin-3-yl)-3-(7-((1,3-dimethoxypropan-2-yl)(methyl)amino)-2-methylthiazolo[5,4-b]pyridin-6-yl)urea (Compound 46);

47. 1-(5-Chloro-6-(2H-1,2,3-triazol-2-yl)pyridin-3-yl)-3-(7-((2-(4-fluorophenyl)-2-methoxyethyl)(methyl)amino)-2-methylthiazolo[5,4-b]pyridin-6-yl)urea (Compound 47);

48. 1-(5-Chloro-6-methoxypyridin-3-yl)-3-(7-(cyclopropyl(2-methoxyethyl)amino)-2-methylthiazolo[5,4-b]pyridin-6-yl)urea (Compound 48);

49. 1-(5-Chloro-6-(2H-1,2,3-triazol-2-yl)pyridin-3-yl)-3-(7-(cyclopropyl(2-methoxyethyl)amino)-2-methylthiazolo[5,4-b]pyridin-6-yl)urea (Compound 49);

50. 1-(5-Chloro-6-(2H-1,2,3-triazol-2-yl)pyridin-3-yl)-3-(7-(3-(methoxymethyl)piperidin-1-yl)-2-methylthiazolo[5,4-b]pyridin-6-yl)urea (Compound 50);

51. 1-(5-Chloro-6-methoxypyridin-3-yl)-3-(7-(3-(methoxymethyl)piperidin-1-yl)-2-methylthiazolo[5,4-b]pyridin-6-yl)urea (Compound 51);

52. 1-(5-Chloro-6-(2H-1,2,3-triazol-2-yl)pyridin-3-yl)-3-(7-(3-methoxypiperidin-1-yl)-2-methylthiazolo[5,4-b]pyridin-6-yl)urea (Compound 52);

53. 1-(5-Chloro-6-(2H-1,2,3-triazol-2-yl)pyridin-3-yl)-3-(7-((1-methoxypropan-2-yl)(methyl)amino)-2-methylthiazolo[5,4-b]pyridin-6-yl)urea (Compound 53);

54. 1-(5-Chloro-6-methoxypyridin-3-yl)-3-(7-((1-methoxypropan-2-yl)(methyl)amino)-2-methylthiazolo[5,4-b]pyridin-6-yl)urea (Compound 54);

55. 1-(5-Chloro-6-methoxypyridin-3-yl)-3-(7-((2-methoxypropyl)(methyl)amino)-2-methylthiazolo[5,4-b]pyridin-6-yl)urea (Compound 55);

56. 1-(5-Chloro-6-(2H-1,2,3-triazol-2-yl)pyridin-3-yl)-3-(7-(1-methoxy-2,2-dimethylpropyl)-2-methylthiazolo[5,4-b]pyridin-6-yl)urea (Compound 56);

57. 1-(6-(2H-1,2,3-Triazol-2-yl)-5-(trifluoromethyl)pyridin-3-yl)-3-(7-(1-methoxy-2,2-dimethylpropyl)-2-methylthiazolo[5,4-b]pyridin-6-yl)urea (Compound 57);

58. 1-(5-Chloro-6-methoxypyridin-3-yl)-3-(7-(3,6-dihydro-2H-pyran-4-yl)-2-methylthiazolo[5,4-b]pyridin-6-yl)urea (Compound 58);

59. 1-(5-Chloro-6-(2H-1,2,3-triazol-2-yl)pyridin-3-yl)-3-(7-(cyclohex-1-en-1-yl)-2-methylthiazolo[5,4-b]pyridin-6-yl)urea (Compound 59);

60. 1-(5-Chloro-6-cyanopyridin-3-yl)-3-(7-cyclopropyl-2-methylthiazolo[5,4-b]pyridin-6-yl)urea (Compound 60);

61. 1-(5-Chloro-6-(1H-1,2,3-triazol-1-yl)pyridin-3-yl)-3-(7-cyclopropyl-2-methylthiazolo[5,4-b]pyridin-6-yl)urea (Compound 61);

62. 1-(5-Cyano-6-methoxypyridin-3-yl)-3-(7-cyclopropyl-2-methylthiazolo[5,4-b]pyridin-6-yl)urea (Compound 62);

63. 1-(3-Chloro-4-(1,3,4-oxadiazol-2-yl)phenyl)-3-(7-cyclopropyl-2-methylthiazolo[5,4-b]pyridin-6-yl)urea (Compound 63);

64. 1-(3-Chloro-4-(2H-1,2,3-triazol-2-yl)phenyl)-3-(7-(1-(methoxymethyl)cyclopropyl)-2-methylthiazolo[5,4-b]pyridin-6-yl)urea (Compound 64);

65. 1-(5-Chloro-6-methoxypyridin-3-yl)-3-(7-(1-(methoxymethyl)cyclopropyl)-2-methylthiazolo[5,4-b]pyridin-6-yl)urea (Compound 65);

66. 1-(5-Chloro-6-methoxypyridin-3-yl)-3-(2-methyl-7-(1,4-oxazepan-4-yl)thiazolo[5,4-b]pyridin-6-yl)urea (Compound 66);

67. 1-(5-Chloro-6-(2H-1,2,3-triazol-2-yl)pyridin-3-yl)-3-(2-methyl-7-(1,4-oxazepan-4-yl)thiazolo[5,4-b]pyridin-6-yl)urea (Compound 67);

68. 1-(3-Chloro-4-(2H-1,2,3-triazol-2-yl)phenyl)-3-(7-(cyclopropyl(2-methoxyethyl)amino)-2-methylthiazolo[5,4-b]pyridin-6-yl)urea (Compound 68);

69. 1-(6-(2H-1,2,3-Triazol-2-yl)-5-(trifluoromethyl)pyridin-3-yl)-3-(7-(cyclopropyl(2-methoxyethyl)amino)-2-methylthiazolo[5,4-b]pyridin-6-yl)urea (Compound 69);

70. 1-(4-(2H-1,2,3-Triazol-2-yl)-3-(trifluoromethyl)phenyl)-3-(7-(cyclopropyl(2-methoxyethyl)amino)-2-methylthiazolo[5,4-b]pyridin-6-yl)urea (Compound 70);

71. 1-(6-(2H-1,2,3-Triazol-2-yl)-5-(trifluoromethyl)pyridin-3-yl)-3-(7-(1-hydroxyethyl)-2-methylthiazolo[5,4-b]pyridin-6-yl)urea (Compound 71);

72. 1-(6-(2H-1,2,3-Triazol-2-yl)-5-(trifluoromethyl)pyridin-3-yl)-3-(7-(1-fluoroethyl)-2-methylthiazolo[5,4-b]pyridin-6-yl)urea (Compound 72);

73. 1-(5-Chloro-6-(2-(1-methylpiperidin-4-yl)ethoxy)pyridin-3-yl)-3-(7-cyclopropyl-2-methylthiazolo[5,4-b]pyridin-6-yl)urea (Compound 73);

74. 1-(6-(2H-1,2,3-Triazol-2-yl)-5-(trifluoromethyl)pyridin-3-yl)-3-(7-(1-(dimethylamino)ethyl)-2-methylthiazolo[5,4-b]pyridin-6-yl)urea (Compound 74);

75. 1-(6-(2H-1,2,3-Triazol-2-yl)-5-(trifluoromethyl)pyridin-3-yl)-3-(7-(1-(dimethylamino)propyl)-2-methylthiazolo[5,4-b]pyridin-6-yl)urea (Compound 75);

76. 1-(6-(2H-1,2,3-Triazol-2-yl)-5-(trifluoromethyl)pyridin-3-yl)-3-(7-(cyclopropyl(dimethylamino)methyl)-2-methylthiazolo[5,4-b]pyridin-6-yl)urea (Compound 76);

77. 1-(3-Chloro-4-(2H-1,2,3-triazol-2-yl)phenyl)-3-(2-methyl-7-(1-(pyrrolidin-1-yl)ethyl)thiazolo[5,4-b]pyridin-6-yl)urea (Compound 77);

78. 1-(6-(2H-1,2,3-Triazol-2-yl)-5-(trifluoromethyl)pyridin-3-yl)-3-(7-(1-methoxy-2-methylpropyl)-2-methylthiazolo[5,4-b]pyridin-6-yl)urea (Compound 78);

79. 1-(5-Chloro-6-(2H-1,2,3-triazol-2-yl)pyridin-3-yl)-3-(7-(1-methoxy-2-methylpropyl)-2-methylthiazolo[5,4-b]pyridin-6-yl)urea (Compound 79);

80. 1-(7-(1-Methoxy-2-methylpropyl)-2-methylthiazolo[5,4-b]pyridin-6-yl)-3-(2-methoxy-6-(2H-1,2,3-triazol-2-yl)-5-(trifluoromethyl)pyridin-3-yl)urea (Compound 80);

81. 1-(5-Chloro-2-methoxy-6-(2H-1,2,3-triazol-2-yl)pyridin-3-yl)-3-(7-(1-methoxy-2-methylpropyl)-2-methylthiazolo[5,4-b]pyridin-6-yl)urea (Compound 81);

82. 1-(3-Chloro-4-(2H-1,2,3-triazol-2-yl)phenyl)-3-(7-(methoxy(phenyl)methyl)-2-methylthiazolo[5,4-b]pyridin-6-yl)urea (Compound 82);

83. 1-(6-(2H-1,2,3-Triazol-2-yl)-5-(trifluoromethyl)pyridin-3-yl)-3-(7-(methoxy(phenyl)methyl)-2-methylthiazolo[5,4-b]pyridin-6-yl)urea (Compound 83);

84. 1-(5-Chloro-6-(2H-1,2,3-triazol-2-yl)pyridin-3-yl)-3-(7-(methoxy(phenyl)methyl)-2-methylthiazolo[5,4-b]pyridin-6-yl)urea (Compound 84);

85. 1-(6-(2H-1,2,3-Triazol-2-yl)-5-(trifluoromethyl)pyridin-3-yl)-3-(7-((4-fluorophenyl)(methoxy)methyl)-2-methylthiazolo[5,4-b]pyridin-6-yl)urea (Compound 85);

86. 1-(4-(2H-1,2,3-Triazol-2-yl)-3-(trifluoromethyl)phenyl)-3-(7-((4-fluorophenyl)(methoxy)methyl)-2-methylthiazolo[5,4-b]pyridin-6-yl)urea (Compound 86);

87. 1-(3-Chloro-4-(2H-1,2,3-triazol-2-yl)phenyl)-3-(7-((4-fluorophenyl)(methoxy)methyl)-2-methylthiazolo[5,4-b]pyridin-6-yl)urea (Compound 87);

88. 1-(2-Methoxy-6-(2H-1,2,3-triazol-2-yl)-5-(trifluoromethyl)pyridin-3-yl)-3-(7-(1-methoxyethyl)-2-methylthiazolo[5,4-b]pyridin-6-yl)urea (Compound 88);

89. 1-(5-chloro-2-(2H-1,2,3-triazol-2-yl)phenyl)-3-(7-(1-methoxyethyl)-2-methylthiazolo[5,4-b]pyridin-6-yl)urea (Compound 89);

90. 1-(5-Chloro-2-methoxy-6-(2H-1,2,3-triazol-2-yl)pyridin-3-yl)-3-(7-(1-methoxyethyl)-2-methylthiazolo[5,4-b]pyridin-6-yl)urea (Compound 90);

91. 1-(5-Chloro-2-methoxy-6-(1H-1,2,3-triazol-1-yl)pyridin-3-yl)-3-(7-(1-methoxyethyl)-2-methylthiazolo[5,4-b]pyridin-6-yl)urea (Compound 91);

92. 1-(5-Chloro-6-methoxy-2-(2H-1,2,3-triazol-2-yl)pyridin-3-yl)-3-(7-(1-methoxyethyl)-2-methylthiazolo[5,4-b]pyridin-6-yl)urea (Compound 92);

93. 1-(6-(2H-1,2,3-Triazol-2-yl)-5-(trifluoromethyl)pyridin-3-yl)-3-(7-(1-cyclopropyl-1-methoxyethyl)-2-methylthiazolo[5,4-b]pyridin-6-yl)urea (Compound 93);

94. 1-(4-(2H-1,2,3-Triazol-2-yl)-3-(trifluoromethyl)phenyl)-3-(7-(1-cyclopropyl-1-methoxyethyl)-2-methylthiazolo[5,4-b]pyridin-6-yl)urea (Compound 94);

95. 1-(6-(2H-1,2,3-Triazol-2-yl)-5-(trifluoromethyl)pyridin-3-yl)-3-(2-methyl-7-(2,2,2-trifluoro-1-methoxyethyl)thiazolo[5,4-b]pyridin-6-yl)urea (Compound 95);

96. 1-(6-(2H-1,2,3-Triazol-2-yl)-5-(trifluoromethyl)pyridin-3-yl)-3-(7-(2-methoxypropan-2-yl)-2-methylthiazolo[5,4-b]pyridin-6-yl)urea (Compound 96);

97. 1-(4-(2H-1,2,3-Triazol-2-yl)-3-(trifluoromethyl)phenyl)-3-(7-(2-methoxypropan-2-yl)-2-methylthiazolo[5,4-b]pyridin-6-yl)urea (Compound 97);

98. 1-(3-Chloro-4-(2H-1,2,3-triazol-2-yl)phenyl)-3-(7-(2-methoxypropan-2-yl)-2-methylthiazolo[5,4-b]pyridin-6-yl)urea (Compound 98);

99. 1-(4-(2H-1,2,3-Triazol-2-yl)-3-(trifluoromethyl)phenyl)-3-(7-(cyclopropyl(methyl)amino)-2-methylthiazolo[5,4-b]pyridin-6-yl)urea (Compound 99);

100. 1-(6-(2H-1,2,3-Triazol-2-yl)-5-(trifluoromethyl)pyridin-3-yl)-3-(7-(cyclopropyl(methyl)amino)-2-methylthiazolo[5,4-b]pyridin-6-yl)urea (Compound 100);

101. 1-(5-Chloro-6-(2H-1,2,3-triazol-2-yl)pyridin-3-yl)-3-(7-(1-(methoxymethyl)cyclopropyl)-2-methylthiazolo[5,4-b]pyridin-6-yl)urea (Compound 101);

102. 1-(6-(2H-1,2,3-Triazol-2-yl)-5-(trifluoromethyl)pyridin-3-yl)-3-(7-(1-(methoxymethyl)cyclopropyl)-2-methylthiazolo[5,4-b]pyridin-6-yl)urea (Compound 102);

103. 1-(5-Chloro-6-methoxypyridin-3-yl)-3-(7-cyclopropyl-2-(trifluoromethyl)thiazolo[5,4-b]pyridin-6-yl)urea (Compound 103);

104. 1-(5-Chloro-6-(2H-1,2,3-triazol-2-yl)pyridin-3-yl)-3-(7-cyclopropyl-2-(trifluoromethyl)thiazolo[5,4-b]pyridin-6-yl)urea (Compound 104);

105. 1-(5-Chloro-6-(2H-1,2,3-triazol-2-yl)pyridin-3-yl)-3-(7-(1-(hydroxymethyl)cyclopropyl)-2-methylthiazolo[5,4-b]pyridin-6-yl)urea (Compound 105);

106. 1-(3-Chloro-4-(2H-1,2,3-triazol-2-yl)phenyl)-3-(7-(1-(hydroxymethyl)cyclopropyl)-2-methylthiazolo[5,4-b]pyridin-6-yl)urea (Compound 106);

107. 1-(3-Chloro-4-(2H-1,2,3-triazol-2-yl)phenyl)-3-(7-(1-(fluoromethyl)cyclopropyl)-2-methylthiazolo[5,4-b]pyridin-6-yl)urea (Compound 107);

108. 1-(5-Chloro-6-(2H-1,2,3-triazol-2-yl)pyridin-3-yl)-3-(7-(1-((dimethylamino)methyl)cyclopropyl)-2-methylthiazolo[5,4-b]pyridin-6-yl)urea (Compound 108);

109. 1-(5-chloro-2,4-dimethoxyphenyl)-3-(7-(1-methoxyethyl)-2-methylthiazolo[5,4-b]pyridin-6-yl)urea (Compound 109);

110. 1-(5-Chloro-6-methoxypyridin-3-yl)-3-(7-(dimethylamino)-2-methylthiazolo[5,4-b]pyridin-6-yl)urea (Compound 110);

111. 1-(5-Chloro-6-methoxypyridin-3-yl)-3-(2-methyl-7-(pyrrolidin-1-yl)thiazolo[5,4-b]pyridin-6-yl)urea (Compound 111);

112. 1-(5-Chloro-6-methoxypyridin-3-yl)-3-(2-methyl-7-morpholinothiazolo[5,4-b]pyridin-6-yl)urea (Compound 112);

113. 1-(5-Chloro-6-methoxypyridin-3-yl)-3-(7-(4,4-difluoropiperidin-1-yl)-2-methylthiazolo[5,4-b]pyridin-6-yl)urea (Compound 113);

114. 1-(7-Cyclopropyl-2-methylthiazolo[5,4-b]pyridin-6-yl)-3-(2-(difluoromethyl)pyridin-4-yl)urea (Compound 114);

115. 1-(5-Chloro-6-(2H-1,2,3-triazol-2-yl)pyridin-3-yl)-3-(7-isopropyl-2-methylthiazolo[5,4-b]pyridin-6-yl)urea (Compound 115);

116. 1-(5-Chloro-6-methoxypyridin-3-yl)-3-(7-cyclopropyl-2-ethylthiazolo[5,4-b]pyridin-6-yl)urea (Compound 116);

117. 1-(5-Chloro-6-methoxypyridin-3-yl)-3-(2-methyl-7-(1-methylcyclopropyl)thiazolo[5,4-b]pyridin-6-yl)urea (Compound 117);

118. 1-(3-chloro-4-methoxyphenyl)-3-(7-(1-methoxyethyl)-2-methylthiazolo[5,4-b]pyridin-6-yl)urea (Compound 118);

119. 1-(5-Chloro-6-methoxypyridin-3-yl)-3-(7-isopropyl-2-methylthiazolo[5,4-b]pyridin-6-yl)urea (Compound 119);

120. 1-(5-Chloro-2-methoxypyridin-3-yl)-3-(7-(1-methoxyethyl)-2-methylthiazolo[5,4-b]pyridin-6-yl)urea (Compound 120);

121. 1-(5-Chloro-6-methoxypyridin-3-yl)-3-(7-(1-(2-methoxyethoxy)ethyl)-2-methylthiazolo[5,4-b]pyridin-6-yl)urea (Compound 121);

122. 1-(5-Chloro-2,6-dimethoxypyridin-3-yl)-3-(7-(1-methoxyethyl)-2-methylthiazolo[5,4-b]pyridin-6-yl)urea (Compound 122);

123. 1-(5-Chloro-6-(1H-1,2,3-triazol-1-yl)pyridin-3-yl)-3-(7-(1-methoxyethyl)-2-methylthiazolo[5,4-b]pyridin-6-yl)urea (Compound 123);

124. 1-(5-Chloro-6-methoxypyridin-3-yl)-3-(7-(1-methoxy-2-methylpropyl)-2-methylthiazolo[5,4-b]pyridin-6-yl)urea (Compound 124);

125. 1-(3-Chloro-4-(2H-1,2,3-triazol-2-yl)phenyl)-3-(7-(1-methoxy-2-methylpropyl)-2-methylthiazolo[5,4-b]pyridin-6-yl)urea (Compound 125);

126. 1-(3-Chloro-4-(2H-1,2,3-triazol-2-yl)phenyl)-3-(7-(1-methoxypropyl)-2-methylthiazolo[5,4-b]pyridin-6-yl)urea (Compound 126);

127. 1-(5-Chloro-6-methoxypyridin-3-yl)-3-(7-(1-methoxypropyl)-2-methylthiazolo[5,4-b]pyridin-6-yl)urea (Compound 127);

128. 1-(5-Chloro-2-methoxyphenyl)-3-(7-(1-methoxyethyl)-2-methylthiazolo[5,4-b]pyridin-6-yl)urea (Compound 128);

129. 1-(5-Cyanopyridin-3-yl)-3-(7-(1-methoxyethyl)-2-methylthiazolo[5,4-b]pyridin-6-yl)urea (Compound 129);

130. 1-(7-(1-Methoxyethyl)-2-methylthiazolo[5,4-b]pyridin-6-yl)-3-(2-(trifluoromethyl)pyridin-4-yl)urea (Compound 130);

131. 1-(5-Chloro-2-methoxy-4-(2H-1,2,3-triazol-2-yl)phenyl)-3-(7-(1-methoxyethyl)-2-methylthiazolo[5,4-b]pyridin-6-yl)urea (Compound 131);

132. 1-(5-Chloro-2-methoxy-4-(1H-1,2,3-triazol-1-yl)phenyl)-3-(7-(1-methoxyethyl)-2-methylthiazolo[5,4-b]pyridin-6-yl)urea (Compound 132);

133. 1-(7-(1-Methoxyethyl)-2-methylthiazolo[5,4-b]pyridin-6-yl)-3-(1-methyl-2-oxo-5-(trifluoromethyl)-1,2-dihydropyridin-3-yl)urea (Compound 133);

134. 1-(3-Chloro-4-(2H-1,2,3-triazol-2-yl)phenyl)-3-(7-(1-methoxypropan-2-yl)-2-methylthiazolo[5,4-b]pyridin-6-yl)urea (Compound 134);

135. 1-(5-Chloro-6-methoxypyridin-3-yl)-3-(2-methyl-7-(tetrahydrofuran-2-yl)thiazolo[5,4-b]pyridin-6-yl)urea (Compound 135);

136. 1-(5-Chloro-6-(2H-1,2,3-triazol-2-yl)pyridin-3-yl)-3-(2-methyl-7-(tetrahydrofuran-2-yl)thiazolo[5,4-b]pyridin-6-yl)urea (Compound 136);

137. 1-(3-Chloro-4-(2H-1,2,3-triazol-2-yl)phenyl)-3-(2-methyl-7-(tetrahydrofuran-2-yl)thiazolo[5,4-b]pyridin-6-yl)urea (Compound 137);

138. 1-(3-Chloro-4-(2H-1,2,3-triazol-2-yl)phenyl)-3-(2-methyl-7-(tetrahydro-2H-pyran-2-yl)thiazolo[5,4-b]pyridin-6-yl)urea (Compound 138);

139. 1-(5-Chloro-6-methoxypyridin-3-yl)-3-(2-methyl-7-(tetrahydro-2H-pyran-2-yl)thiazolo[5,4-b]pyridin-6-yl)urea (Compound 139);

140. 1-(5-Chloro-6-(2H-1,2,3-triazol-2-yl)pyridin-3-yl)-3-(2-methyl-7-(tetrahydro-2H-pyran-2-yl)thiazolo[5,4-b]pyridin-6-yl)urea (Compound 140);

141. 1-(6-(1H-1,2,3-Triazol-l-yl)-5-(trifluoromethyl)pyridin-3-yl)-3-(7-(1-methoxyethyl)-2-methylthiazolo[5,4-b]pyridin-6-yl)urea (Compound 141);

142. 1-(6-(2H-1,2,3-Triazol-2-yl)-5-(trifluoromethyl)pyridin-3-yl)-3-(7-(1-methoxyethyl)-2-methylthiazolo[5,4-b]pyridin-6-yl)urea (Compound 142);

143. 1-(4-(2H-1,2,3-Triazol-2-yl)-3-(trifluoromethyl)phenyl)-3-(7-(1-methoxyethyl)-2-methylthiazolo[5,4-b]pyridin-6-yl)urea (Compound 143);

144. 1-(3-Chloro-4-(2H-1,2,3-triazol-2-yl)phenyl)-3-(7-(cyclopropyl(methoxy)methyl)-2-methylthiazolo[5,4-b]pyridin-6-yl)urea (Compound 144);

145. 1-(5-Chloro-6-(2H-1,2,3-triazol-2-yl)pyridin-3-yl)-3-(7-(1-methoxypropyl)-2-methylthiazolo[5,4-b]pyridin-6-yl)urea (Compound 145);

146. 1-(6-(2H-1,2,3-Triazol-2-yl)-5-(trifluoromethyl)pyridin-3-yl)-3-(7-(1-methoxypropyl)-2-methylthiazolo[5,4-b]pyridin-6-yl)urea (Compound 146);

147. 1-(4-(2H-1,2,3-Triazol-2-yl)-3-(trifluoromethyl)phenyl)-3-(7-(1-methoxypropyl)-2-methylthiazolo[5,4-b]pyridin-6-yl)urea (Compound 147);

148. 1-(5-Chloro-6-(5-methyloxazol-2-yl)pyridin-3-yl)-3-(7-(1-methoxyethyl)-2-methylthiazolo[5,4-b]pyridin-6-yl)urea (Compound 148);

149. 1-(5-Chloro-6-(difluoromethoxy)pyridin-3-yl)-3-(7-(1-methoxyethyl)-2-methylthiazolo[5,4-b]pyridin-6-yl)urea (Compound 149);

150. 1-(5-Chloro-6-methoxypyridin-3-yl)-3-(7-(cyclopropyl(methoxy)methyl)-2-methylthiazolo[5,4-b]pyridin-6-yl)urea (Compound 150);

151. 1-(6-(2H-1,2,3-Triazol-2-yl)-5-(trifluoromethyl)pyridin-3-yl)-3-(7-(cyclopropyl(methoxy)methyl)-2-methylthiazolo[5,4-b]pyridin-6-yl)urea (Compound 151);

152. Methyl 3-chloro-5-(3-(7-(1-methoxyethyl)-2-methylthiazolo[5,4-b]pyridin-6-yl)ureido)benzoate (Compound 152);

153. 1-(4-(2H-1,2,3-Triazol-2-yl)-3-(trifluoromethyl)phenyl)-3-(7-(cyclopropyl(methoxy)methyl)-2-methylthiazolo[5,4-b]pyridin-6-yl)urea (Compound 153);

154. 1-(5-Chloro-6-(2H-1,2,3-triazol-2-yl)pyridin-3-yl)-3-(7-(cyclopropyl(methoxy)methyl)-2-methylthiazolo[5,4-b]pyridin-6-yl)urea (Compound 154);

155. 1-(7-(Cyclopropyl(methoxy)methyl)-2-methylthiazolo[5,4-b]pyridin-6-yl)-3-(2-methoxy-6-(2H-1,2,3-triazol-2-yl)-5-(trifluoromethyl)pyridin-3-yl)urea (Compound 155);

156. 1-(5-Chloro-2-methoxy-6-(2H-1,2,3-triazol-2-yl)pyridin-3-yl)-3-(7-(cyclopropyl(methoxy)methyl)-2-methylthiazolo[5,4-b]pyridin-6-yl)urea (Compound 156);

157. 1-(7-(sec-Butyl)-2-methylthiazolo[5,4-b]pyridin-6-yl)-3-(5-chloro-6-(2H-1,2,3-triazol-2-yl)pyridin-3-yl)urea (Compound 157);

158. 1-(3-Chloro-4-(2H-1,2,3-triazol-2-yl)phenyl)-3-(7-(1-methoxyethyl)-2-methylthiazolo[5,4-b]pyridin-6-yl)urea (Compound 158);

159. 1-(5-Chloro-6-methoxypyridin-3-yl)-3-(7-(1-methoxyethyl)-2-methylthiazolo[5,4-b]pyridin-6-yl)urea (Compound 159);

160. 1-(5-Chloro-6-(2*H*-1,2,3-triazol-2-yl)pyridin-3-yl)-3-(7-(1-methoxyethyl)-2-methylthiazolo[5,4-b]pyridin-6-yl)urea (Compound 160);

161. 1-(3-Chloro-4-(2H-1,2,3-triazol-2-yl)phenyl)-3-(7-(1-ethoxyethyl)-2-methylthiazolo[5,4-b]pyridin-6-yl)urea (Compound 161);

162. 1-(5-Chloro-6-methoxypyridin-3-yl)-3-(7-(1-ethoxyethyl)-2-methylthiazolo[5,4-b]pyridin-6-yl)urea (Compound 162);

163. 1-(3-Chloro-4-methoxyphenyl)-3-(7-(1-ethoxyethyl)-2-methylthiazolo[5,4-b]pyridin-6-yl)urea (Compound 163);

164. 1-(5-Chloro-6-(2H-1,2,3-triazol-2-yl)pyridin-3-yl)-3-(7-(1-ethoxyethyl)-2-methylthiazolo[5,4-b]pyridin-6-yl)urea (Compound 164);

165. 1-(6-(2H-1,2,3-Triazol-2-yl)-5-(trifluoromethyl)pyridin-3-yl)-3-(7-(1-ethoxyethyl)-2-methylthiazolo[5,4-b]pyridin-6-yl)urea (Compound 165);

166. 1-(5-Chloro-2-methoxy-6-(2H-1,2,3-triazol-2-yl)pyridin-3-yl)-3-(7-(1-ethoxyethyl)-2-methylthiazolo[5,4-b]pyridin-6-yl)urea (Compound 166);

167. 1-(5-Chloro-2-methoxypyridin-3-yl)-3-(7-(1-ethoxyethyl)-2-methylthiazolo[5,4-b]pyridin-6-yl)urea (Compound 167);

168. 1-(3-Chloro-4-(2H-1,2,3-triazol-2-yl)phenyl)-3-(7-cyclopropyl-2-methylthiazolo[5,4-b]pyridin-6-yl)urea (Compound 168);

169. 1-(3-Chloro-4-(1H-1,2,3-triazol-1-yl)phenyl)-3-(7-cyclopropyl-2-methylthiazolo[5,4-b]pyridin-6-yl)urea (Compound 169);

170. 1-(7-Cyclopropyl-2-methylthiazolo[5,4-b]pyridin-6-yl)-3-(3,5-dichloro-4-(1H-1,2,3-triazol-1-yl)phenyl)urea (Compound 170);

171. 1-(3-Cyano-4-(3-methyl-1H-1,2,4-triazol-1-yl)phenyl)-3-(7-cyclopropyl-2-methylthiazolo[5,4-b]pyridin-6-yl)urea (Compound 171);

172. 1-(3-Cyano-4-(5-methyl-1H-1,2,4-triazol-1-yl)phenyl)-3-(7-cyclopropyl-2-methylthiazolo[5,4-b]pyridin-6-yl)urea (Compound 172);

173. 1-(3-Chloro-4-(3-methyl-1H-1,2,4-triazol-1-yl)phenyl)-3-(7-cyclopropyl-2-methylthiazolo[5,4-b]pyridin-6-yl)urea (Compound 173);

174. 1-(3-Chloro-4-(5-methyl-1H-1,2,4-triazol-1-yl)phenyl)-3-(7-cyclopropyl-2-methylthiazolo[5,4-b]pyridin-6-yl)urea (Compound 174);

175. 1-(5-Bromo-6-methoxypyridin-3-yl)-3-(7-cyclopropyl-2-methylthiazolo[5,4-b]pyridin-6-yl)urea (Compound 175);

176. 1-(5-Chloro-6-(2H-1,2,3-triazol-2-yl)pyridin-3-yl)-3-(7-(methoxymethyl)-2-methylthiazolo[5,4-b]pyridin-6-yl)urea (Compound 176);

177. 1-(6-(2H-1,2,3-triazol-2-yl)-5-(trifluoromethyl)pyridin-3-yl)-3-(2-methyl-7-(1-methylcyclopropyl)thiazolo[5,4-b]pyridin-6-yl)urea (Compound 177);

178. 1-(5-Chloro-2-methoxy-6-(2H-1,2,3-triazol-2-yl)pyridin-3-yl)-3-(2-methyl-7-(1-methylcyclopropyl)thiazolo[5,4-b]pyridin-6-yl)urea (Compound 178);

179. 1-(3-Chloro-4-(2H-1,2,3-triazol-2-yl)phenyl)-3-(2-methyl-7-(1-methylcyclopropyl)thiazolo[5,4-b]pyridin-6-yl)urea (Compound 179);

180. 1-(2-Methoxy-6-(2H-1,2,3-triazol-2-yl)-5-(trifluoromethyl)pyridin-3-yl)-3-(2-methyl-7-(l-methylcyclopropyl)thiazolo[5,4-b]pyridin-6-yl)urea (Compound 180);

181. 1-(5-Chloro-6-(1H-pyrazol-1-yl)pyridin-3-yl)-3-(7-cyclopropyl-2-methylthiazolo[5,4-b]pyridin-6-yl)urea (Compound 181);

182. 1-(3-Chloro-4-(1H-pyrazol-1-yl)phenyl)-3-(7-cyclopropyl-2-methylthiazolo[5,4-b]pyridin-6-yl)urea (Compound 182);

183. 1-(3-Chloro-4-(3-(methoxymethyl)-5-methyl-1H-pyrazol-1-yl)phenyl)-3-(7-cyclopropyl-2-methylthiazolo[5,4-b]pyridin-6-yl)urea (Compound 183);

184. 1-(5-Chloro-2-methoxy-6-(2H-1,2,3-triazol-2-yl)pyridin-3-yl)-3-(7-isopropyl-2-methylthiazolo[5,4-b]pyridin-6-yl)urea (Compound 184);

185. 1-(5-Chloro-6-(2H-1,2,3-triazol-2-yl)pyridin-3-yl)-3-(7-(2-(2-methoxyethoxy)ethyl)-2-methylthiazolo[5,4-b]pyridin-6-yl)urea (Compound 185);

186. 1-(5-Chloro-2,6-dimethoxypyridin-3-yl)-3-(7-isopropyl-2-methylthiazolo[5,4-b]pyridin-6-yl)urea (Compound 186);

187. 1-(5-Chloro-2-methoxypyridin-3-yl)-3-(7-isopropyl-2-methylthiazolo[5,4-b]pyridin-6-yl)urea (Compound 187);

188. 1-(3-Chloro-4-(2H-1,2,3-triazol-2-yl)phenyl)-3-(7-isopropyl-2-methylthiazolo[5,4-b]pyridin-6-yl)urea (Compound 188);

189. 1-(5-Chlorothiophen-3-yl)-3-(7-isopropyl-2-methylthiazolo[5,4-b]pyridin-6-yl)urea (Compound 189);

190. 1-(5-Chlorothiophen-3-yl)-3-(7-cyclopropyl-2-methylthiazolo[5,4-b]pyridin-6-yl)urea (Compound 190);

191. 1-(6-(2H-1,2,3-triazol-2-yl)-5-(trifluoromethyl)pyridin-3-yl)-3-(7-isopropyl-2-methylthiazolo[5,4-b]pyridin-6-yl)urea (Compound 191);

192. 1-(5-Chloro-2-methoxypyridin-3-yl)-3-(7-cyclopropyl-2-methylthiazolo[5,4-b]pyridin-6-yl)urea (Compound 192);

193. 1-(3-Chloro-4-(difluoromethoxy)phenyl)-3-(7-cyclopropyl-2-methylthiazolo[5,4-b]pyridin-6-yl)urea (Compound 193);

194. 1-(5-Chloro-6-(1-methyl-1H-pyrazol-5-yl)pyridin-3-yl)-3-(7-cyclopropyl-2-methylthiazolo[5,4-b]pyridin-6-yl)urea (Compound 194);

195. 1-(5-Chloro-2-(2-(dimethylamino)ethoxy)pyridin-3-yl)-3-(7-cyclopropyl-2-methylthiazolo[5,4-b]pyridin-6-yl)urea (Compound 195);

196. 1-(5-Chloro-6-(1H-pyrazol-1-yl)pyridin-3-yl)-3-(7-(1-methoxyethyl)-2-methylthiazolo[5,4-b]pyridin-6-yl)urea

(Compound 196);

197. 1-(5-Chloro-6-(isoxazol-4-yl)pyridin-3-yl)-3-(7-(1-methoxyethyl)-2-methylthiazolo[5,4-b]pyridin-6-yl)urea (Compound 197);

198. 1-(3-Chloro-4-(1H-1,2,3-triazol-1-yl)phenyl)-3-(7-(1-methoxyethyl)-2-methylthiazolo[5,4-b]pyridin-6-yl)urea (Compound 198);

199. 1-(3-Chloro-4-(pyrazin-2-yl)phenyl)-3-(7-(1-methoxyethyl)-2-methylthiazolo[5,4-b]pyridin-6-yl)urea (Compound 199);

200. 1-(5-Cyano-6-(2H-1,2,3-triazol-2-yl)pyridin-3-yl)-3-(7-(1-methoxyethyl)-2-methylthiazolo[5,4-b]pyridin-6-yl)urea (Compound 200);

201. 1-(3-Chloro-4-(1H-pyrazol-1-yl)phenyl)-3-(7-(1-methoxyethyl)-2-methylthiazolo[5,4-b]pyridin-6-yl)urea (Compound 201);

202. 1-(3-Chloro-4-(pyrimidin-2-yl)phenyl)-3-(7-(1-methoxyethyl)-2-methylthiazolo[5,4-b]pyridin-6-yl)urea (Compound 202);

203. 1-(3-Chloro-4-(1,3,4-oxadiazol-2-yl)phenyl)-3-(7-(1-methoxyethyl)-2-methylthiazolo[5,4-b]pyridin-6-yl)urea (Compound 203);

204. 1-(3-Chloro-4-(oxazol-5-yl)phenyl)-3-(7-(1-methoxyethyl)-2-methylthiazolo[5,4-b]pyridin-6-yl)urea (Compound 204);

205. 1-(5-(Difluoromethyl)-6-(2H-1,2,3-triazol-2-yl)pyridin-3-yl)-3-(7-(1-methoxyethyl)-2-methylthiazolo[5,4-b]pyridin-6-yl)urea (Compound 205);

206. 1-(5-(Difluoromethyl)-6-(1H-1,2,3-triazol-1-yl)pyridin-3-yl)-3-(7-(1-methoxyethyl)-2-methylthiazolo[5,4-b]pyridin-6-yl)urea (Compound 206);

207. 1-(3-(Difluoromethyl)-4-(2H-1,2,3-triazol-2-yl)phenyl)-3-(7-(1-methoxyethyl)-2-methylthiazolo[5,4-b]pyridin-6-yl)urea (Compound 207);

208. 1-(3-Cyano-4-(2H-1,2,3-triazol-2-yl)phenyl)-3-(7-(1-methoxyethyl)-2-methylthiazolo[5,4-b]pyridin-6-yl)urea (Compound 208);

209. 1-(5-Chloro-2-methoxy-6-(1H-pyrazol-1-yl)pyridin-3-yl)-3-(7-(1-methoxyethyl)-2-methylthiazolo[5,4-b]pyridin-6-yl)urea (Compound 209);

210. 1-(4-(1H-Pyrazol-1-yl)-3-(trifluoromethyl)phenyl)-3-(7-(1-methoxyethyl)-2-methylthiazolo[5,4-b]pyridin-6-yl)urea (Compound 210);

211. 1-(3-Fluoro-4-(2H-1,2,3-triazol-2-yl)phenyl)-3-(7-(1-methoxyethyl)-2-methylthiazolo[5,4-b]pyridin-6-yl)urea (Compound 211);

212. 1-(5-Fluoro-6-(2H-1,2,3-triazol-2-yl)pyridin-3-yl)-3-(7-(1-methoxyethyl)-2-methylthiazolo[5,4-b]pyridin-6-yl)urea (Compound 212);

213. 1-(6-(1H-Pyrazol-1-yl)-5-(trifluoromethyl)pyridin-3-yl)-3-(7-(1-methoxyethyl)-2-methylthiazolo[5,4-b]pyridin-6-yl)urea (Compound 213);

214. 1-(4-(Difluoromethoxy)-3-(trifluoromethyl)phenyl)-3-(7-(1-methoxyethyl)-2-methylthiazolo[5,4-b]pyridin-6-yl)urea (Compound 214);

215. 1-(3-Chloro-4-(1H-imidazol-1-yl)phenyl)-3-(7-(1-methoxyethyl)-2-methylthiazolo[5,4-b]pyridin-6-yl)urea (Compound 215);

216. 1-(3-Chloro-5-(5-methyl-1,2,4-oxadiazol-3-yl)phenyl)-3-(7-(1-methoxyethyl)-2-methylthiazolo[5,4-b]pyridin-6-yl)urea (Compound 216);

217. 1-(3-(2H-1,2,3-Triazol-2-yl)-5-(trifluoromethyl)phenyl)-3-(7-(1-methoxyethyl)-2-methylthiazolo[5,4-b]pyridin-6-yl)urea (Compound 217);

218. 1-(5-Chloro-6-(2-methoxyethoxy)pyridin-3-yl)-3-(7-(1-methoxyethyl)-2-methylthiazolo[5,4-b]pyridin-6-yl)urea (Compound 218);

219. 1-(5-Chloro-2-(2-methoxyethoxy)pyridin-3-yl)-3-(7-(1-methoxyethyl)-2-methylthiazolo[5,4-b]pyridin-6-yl)urea (Compound 219);

220. 1-(5-chloro-6-(2H-1,2,3-triazol-2-yl)pyridin-3-yl)-3-(7-(2-(methoxymethyl)cyclopropyl)-2-methylthiazolo[5,4-b]pyridin-6-yl)urea (Compound 220);

221. 1-(2-Ethoxy-6-(2H-1,2,3-triazol-2-yl)-5-(trifluoromethyl)pyridin-3-yl)-3-(7-(1-methoxyethyl)-2-methylthiazolo[5,4-b]pyridin-6-yl)urea (Compound 221);

222. 1-(7-(1-Methoxyethyl)-2-methylthiazolo[5,4-b]pyridin-6-yl)-3-(5-(trifluoromethyl)pyridin-3-yl)urea (Compound 222);

223. 1-(6-(2H-1,2,3-Triazol-2-yl)-5-(trifluoromethyl)pyridin-3-yl)-3-(7-(1-(hydroxymethyl)cyclopropyl)-2-methylthiazolo[5,4-b]pyridin-6-yl)urea (Compound 223);

224. 1-(6-(2H-1,2,3-Triazol-2-yl)-5-(trifluoromethyl)pyridin-3-yl)-3-(7-(cyclobutyl(methoxy)methyl)-2-methylthiazolo[5,4-b]pyridin-6-yl)urea (Compound 224);

225. 1-(5-Chloro-6-(2H-1,2,3-triazol-2-yl)pyridin-3-yl)-3-(7-(cyclobutyl(methoxy)methyl)-2-methylthiazolo[5,4-b]pyridin-6-yl)urea (Compound 225);

226. 1-(7-(Cyclopropyl(methoxy)methyl)-2-methylthiazolo[5,4-b]pyridin-6-yl)-3-(2-ethoxy-6-(2H-1,2,3-triazol-2-yl)-5-(trifluoromethyl)pyridin-3-yl)urea (Compound 226);

227. 1-(5-Chloro-6-(2H-1,2,3-triazol-2-yl)pyridin-3-yl)-3-(7-(1-(dimethylamino)propyl)-2-methylthiazolo[5,4-b]pyridin-6-yl)urea (Compound 227);

228. 1-(5-Chloro-6-(2H-1,2,3-triazol-2-yl)pyridin-3-yl)-3-(7-(cyclopropyl(dimethylamino)methyl)-2-methylthiazolo[5,4-b]pyridin-6-yl)urea (Compound 228);

229. 1-(6-(2H-1,2,3-triazol-2-yl)-5-(trifluoromethyl)pyridin-3-yl)-3-(7-(1-(3,3-difluoroazetidin-1-yl)propyl)-2-methylthiazolo[5,4-b]pyridin-6-yl)urea (compound 229);

230. 1-(6-(2H-1,2,3-triazol-2-yl)-5-(trifluoromethyl)pyrdin-3-yl)-3-(7-(1-(dimethylamino)-2,2,2-trifluoroethyl)-2-methylthiazolo[5,4-b]pyridin-6-yl)urea (Compound 230);

231. 1-(5-Chloro-6-(2H-1,2,3-triazol-2-yl)pyridin-3-yl)-3-(7-(1-(dimethylamino)-2,2,2-trifluoroethyl)-2-methylthiazolo[5,4-b]pyridin-6-yl)urea (Compound 231);

232. 1-(6-(2H-1,2,3-Triazol-2-yl)-5-(trifluoromethyl)pyridin-3-yl)-3-(2-ethyl-7-(1-methoxyethyl)thiazolo[5,4-b]pyridin-6-yl)urea (Compound 232);

233. 1-(3-Chloro-4-(2H-1,2,3-triazol-2-yl)phenyl)-3-(2-ethyl-7-(1-methoxyethyl)thiazolo[5,4-b]pyridin-6-yl)urea (Compound 233);

234. 1-(6-((S)-2-Aminopropoxy)-5-(trifluoromethyl)pyridin-3-yl)-3-(7-(1-methoxyethyl)-2-methylthiazolo[5,4-b]pyridin-6-yl)urea hydrochloride (Compound 234);

235. 1-(7-(1-Methoxyethyl)-2-methylthiazolo[5,4-b]pyridin-6-yl)-3-(6-(((R)-1-methoxypropan-2-yl)(methyl)amino)-5-(trifluoromethyl)pyridin-3-yl)urea (Compound 235);

236. 1-(7-(1-Methoxyethyl)-2-methylthiazolo[5,4-b]pyridin-6-yl)-3-(6-(thiazol-2-ylamino)-5-(trifluoromethyl)pyridin-3-yl)urea (Compound 236);

237. N-(5-(3-(7-(1-Methoxyethyl)-2-methylthiazolo[5,4-b]pyridin-6-yl)ureido)-3-(trifluoromethyl)pyridin-2-yl)acetamide (Compound 237);

238. 1-(7-(1-methoxyethyl)-2-methylthiazolo[5,4-b]pyridin-6-yl)-3-(6-(methoxymethyl)-5-(trifluoromethyl)pyridin-3-yl)urea (Compound 238);

239. 1-(6-(1H-Tetrazol-l-yl)-5-(trifluoromethyl)pyridin-3-yl)-3-(7-(1-methoxyethyl)-2-methylthiazolo[5,4-b]pyridin-6-yl)urea (Compound 239); and

240. 1-(6-(2H-1,2,3-Triazol-2-yl)-5-(trifluoromethyl)pyridin-3-yl)-3-(7-(2-cyclopropyl-1-methoxyethyl)-2-methylthiazolo[5,4-b]pyridin-6-yl)urea (Compound 240).

According to a feature of the present invention, the compounds of general formula (I) where all the symbols are as defined earlier, can be prepared by methods illustrated in the schemes and examples provided herein below. However, the disclosure should not be construed to limit the scope of the invention arriving at compound of formula (I) as disclosed hereinabove.

Scheme 1

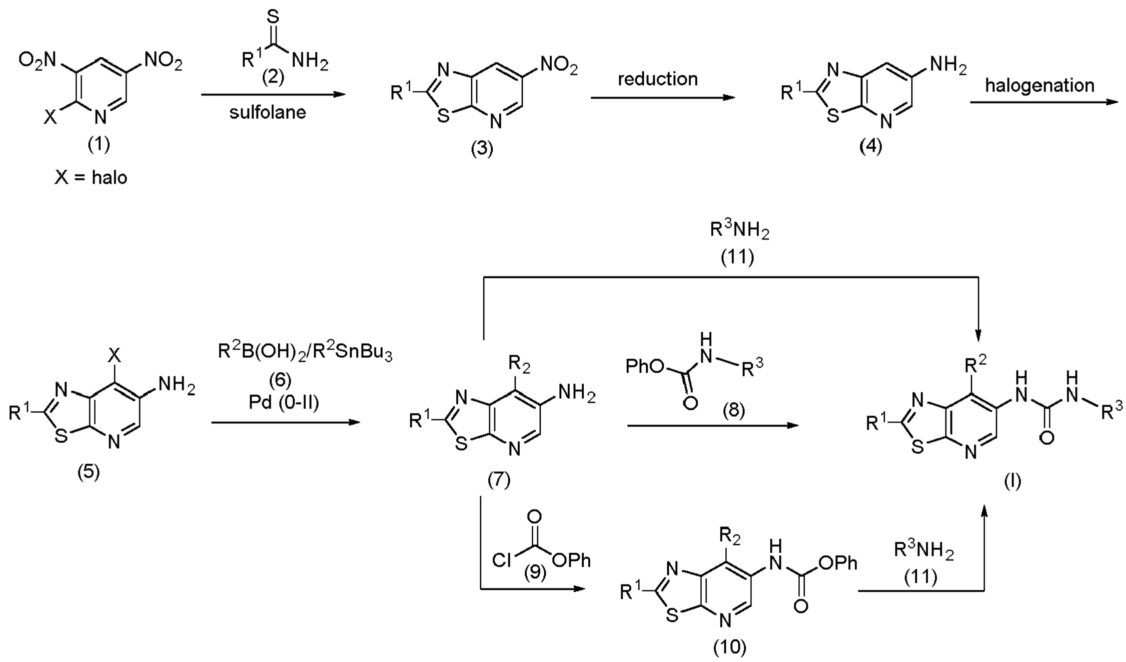


The compounds of formula (I), wherein $R^1$, $R^2$, $R^3$ are as defined herein above, can be prepared as depicted in Scheme 1. The compounds of formula (3) can be prepared by the reaction of compounds of formula (1) with thioamides of formula (2) followed by cyclisation in sulfolane. The compounds of formula (3) can be reduced to the corresponding amines of formula (4) with reducing agents known in the art. Although not limited, such reducing agents include hydrogenation with palladium on carbon, metal reductions like iron, tin or tin chloride and the like. Such reduction of the compounds of formula (3) can be carried out in one or more solvents, e.g., ethers such as THF, 1,4-dioxane and the like; alcohols such as methanol, ethanol and the like; under acidic conditions involving ammonium chloride, acetic acid, hydrochloric acid and the like or mixture(s) thereof.

The compounds of formula (4) can be converted to the compounds of formula (5) via halogenation by methods known in the art. Preferably, compounds of formula (4) are treated with N-halosuccinamides such as NBS, NIS and the like; or with bromine or any other halogenating agent known in the art. Halogenation reactions can be carried out in one

or more solvents, e.g., ether solvents such as THF and the like; chlorinated solvents such as DCM, chloroform and the like; acids such as acetic acid and the like; amides such as DMF and the like or mixture(s) thereof.

The compounds of formula (7) can be prepared by the reaction of compounds of formula (5) with boronic acid/stannane derivatives of formula (6). The same transformation may also be carried out by other suitable coupling methods known in the art. The above reaction can be mediated by a suitable catalyst known in the art such as, e.g., $Pd(PPh_3)_2Cl_2$, $Pd_2dba_3$, $Pd(PPh_3)_4$, $Pd(OAc)_2$ or mixture(s) thereof; a suitable ligand known in the art such as BINAP, xantphos, triphenylphosphine or mixture(s) thereof; in the presence of a suitable base, preferably inorganic bases such as alkali metal carbonates, e.g., sodium carbonate and cesium carbonate, and phosphates like potassium phosphate, or mixture(s) thereof. As also known from the art, such reactions are effected in solvents, e.g., ethers such as tetrahydrofuran, dioxane, and the like; hydrocarbons, e.g., toluene; amides such as DMA, DMF and the like; sulfoxides, e.g., dimethylsulfoxide; halogenated hydrocarbons, e.g., DCM or mixture(s) thereof.

The compounds of formula (8) can be prepared from the corresponding amines by reacting with phenyl chloroformate by following methods known in the art.

The compounds of formula (7) can be subsequently converted to the compounds of the formula (I) by reacting with carbamates of the formula (8). The same transformation may also be carried out by other methods known in the art. The above reaction can be carried out in the presence of an organic base such as triethyl amine, ethyldiisopropyl amine, pyridine and the like. Also known from the art, such reactions are effected in solvents like ethers such as THF, dioxane and the like; hydrocarbons such as toluene and the like; halogenated hydrocarbons like DCM; sulfoxides like DMSO or mixture(s) thereof.

Compounds of formula (7) can also be transformed into the compounds of formula (I) by treating with chloroformates such as phenyl chloroformate of formula (9) to provide carbamates of the formula (10) by following methods known in the art, followed by treatment with amines of formula (11) by following the methods known in the art or as described for the conversion of compounds of formula (7) to (I). Compounds of formula (11) are either commercially available or can be prepared by following the methods known in the art or as described in the synthetic schemes herein.

Alternatively, compounds of formula (7) can be transformed to compounds of the present invention of formula (I) by treating with amine of formula (11) by using coupling reagents, although not limited to, such as triphosgene, carbonyl diimidazole, dicyclohexyl carbodiimide, diethyl carbonate and the like; in one or more solvents like DCM, THF, toluene, DMF, DMA or mixture(s) thereof.

Scheme 2

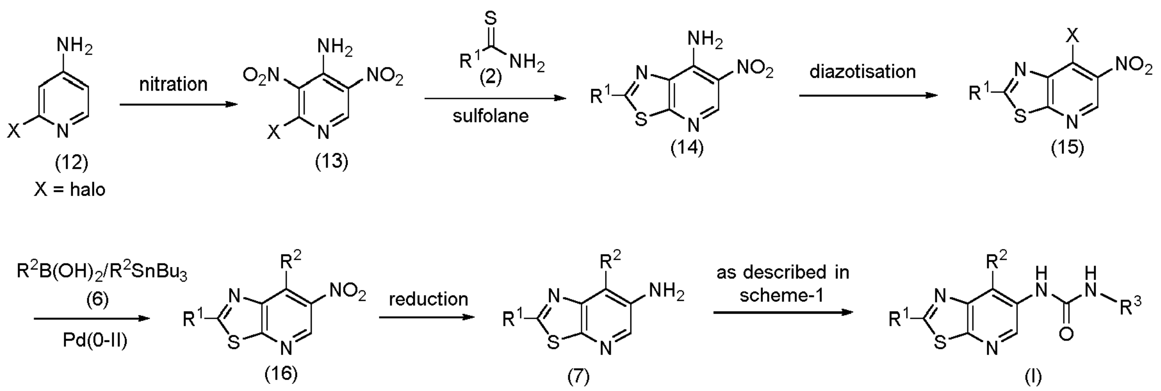


Alternatively, the compounds of the formula (I) can also be prepared by following the methods as described in Scheme 2. Nitration of the compounds of formula (12) with nitrating agents such as nitric acid, potassium nitrate and the like in acids such as sulfuric acid, trifluoroacetic acid, acetic acid and the like; anhydrides like acetic anhydride, trifluoroacetic anhydride and the like; or mixture(s) thereof provides the compounds of formula (13) or by the methods known in the art. Reaction of the compounds of formula (13) with thioamides of formula (2) followed by cyclization in sulfolane by following the methods described in the art provides the compounds of formula (14). Treatment of the compounds of formula (14) under Sandmeyer reaction conditions can provide the compounds of formula (15). The above reaction can be carried out with nitrites such as sodium nitrite, tri-butyl nitrite and the like; copper halides like copper chloride, copper bromide, copper iodide and the like. The solvents used for the above transformation are, e.g., acetonitrile and the transformation is carried out in an acidic media, e.g., hydrochloric acid.

Compounds of formula (15) can be converted to the compounds of formula (16) by following ethods known in the art or as described in the synthetic Scheme 1 for the transformation of compounds of formulas (5) to (7).

Reduction of the nitro group of the compounds of formula (16) to produce the compounds of formula (7) can

be carried out either using hydrogenation over Palladium on carbon, or metals like iron, tin or tin chloride in acidic media, e.g., hydrochloric acid or in the presence of protic solvents like methanol, ethanol or mixture(s) thereof. The compounds of formula (7) can be converted to the compounds of the present invention of formula (I) by following methods as described in general Scheme 1

Scheme-3

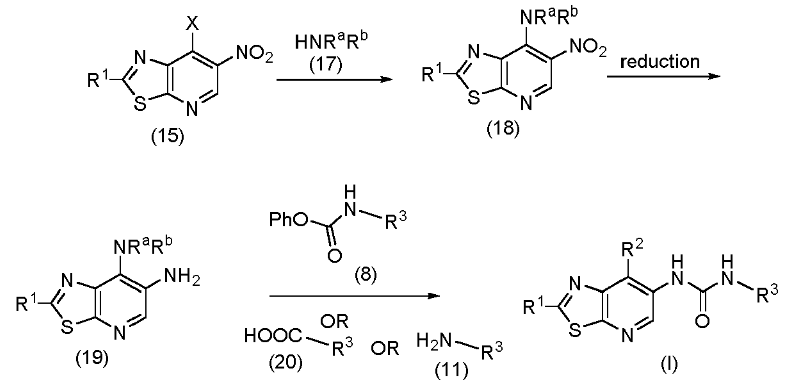


In another embodiment, the compounds of the present invention of the formula (I) can be prepared as described in Scheme-3. Compounds of formula (15) can be reacted with amines of formula (17) to provide the compounds of formula (18). The above reaction can be carried out in the presence of a suitable base such as a metal hydride, e.g., sodium hydride and the like; an organic base such as triethyl amine, ethyldiisopropyl amine, and the like; or an inorganic base such as sodium carbonate, potassium carbonate, cesium carbonate, and the like. Such amination reactions can be carried out in one or more solvents such as ethers, e.g., THF, dioxane, and the like; alcohols such as methanol, ethanol, isopropanol and the like; hydrocarbons such as toluene and the like; or amides such as DMF, DMA and the like or mixture(s) thereof.

Compounds of formula (18) can be reduced to the amines of formula (19) with a reducing agent known in the art. Although not limited, such reducing agents include hydrogenation with palladium on carbon, metal reductions like iron, tin or tin chloride and the like. Reduction of the compounds of formula (18) can be carried out in one or more solvents like ethers such as THF, dioxane and the like; alcohols such as methanol, ethanol and the like; acids such as acetic acid and the like; or mixture(s) thereof.

Compounds of formula (19) can be converted to the compounds of the present invention of the formula (I) by reacting with the compounds of the formula (8) by following the methods described in Scheme-1 for the reaction of compounds of formula (7) to (I). Alternatively, the same transformation can also be carried out by reacting with compounds of formula (20). The coupling agents used for such transformation are DPPA, sodium azide, or any other agents known in the art. The bases used for the said reaction are organic bases such as triethyl amine, diisopropylethyl amine and the like. The coupling reaction can be carried out in solvents like ethers such as dioxane, THF and the like; hydrocarbons like toluene and the like; amides such as DMF, DMA and the like; nitriles such as acetonitrile and the like or mixture(s) thereof. Compounds of formula (19) can be converted to the compounds of the present invention of formula (I) by treating with amines of formula (11) by following methods known in the art or as described for the conversion of compounds of formula (7) to (I), depicted in Scheme 1.

Scheme-4

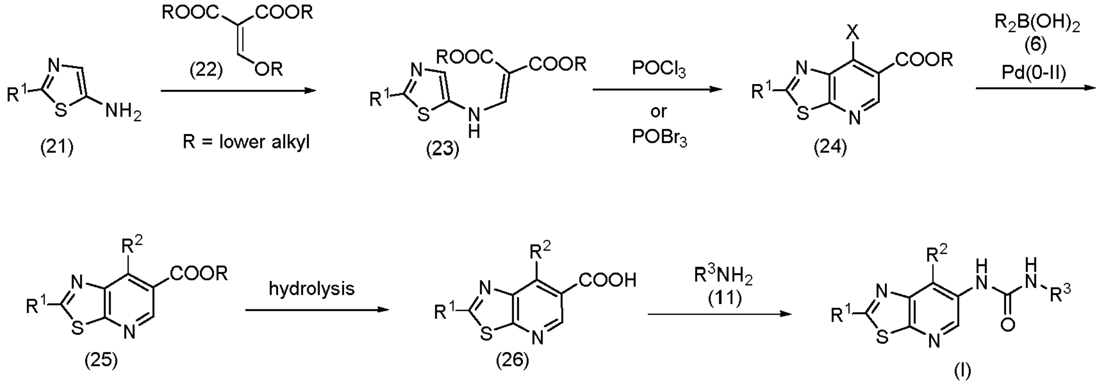


Scheme 4 depicts a method of preparation of the compounds of formula (I) starting from the amine derivatives of the formula (21), which undergoes Michael substitution reaction with dialkyl 2-(alkoxymethylene)malonate (22) to afford the compounds of formula (23). Such reactions can be carried out either neat or in alcoholic solvents such as methanol, ethanol and the like; or by methods known in the art. Treatment of the compounds of formula (23) with halogenating reagents such as $POCl_3$ or $POBr_3$ causes ring cyclisation followed by halogenation in one pot and leads to the compounds of formula (24). Such reactions can be carried out either neat or in presence of hydrocarbons such as toluene, xylene and the like or mixture(s) thereof.

Compounds of formula (24) can be converted to the compounds of formula (25) by reacting with boronic acid derivatives of the formula (6) by following the methods known in the art or as described for the preparation of compounds of the formula (7) in Scheme-1. Hydrolysis of the compounds of the formula (25) by using a base such as sodium hydroxide, potassium hydroxide, lithium hydroxide and the like; in a solvent such as THF, water, methanol, ethanol or a mixture(s) thereof to afford the corresponding acids of the formula (26).

Carboxylic acids of formula (26) can be transformed by treatment with DPPA and a tertiary amine base to generate acyl azides which undergoes rearrangement (Curtius rearrangement) upon heating to form intermediate isocyanates which can be intercepted by appropriate amines of formula (11) to afford urea derivatives of formula (I).

Scheme-5

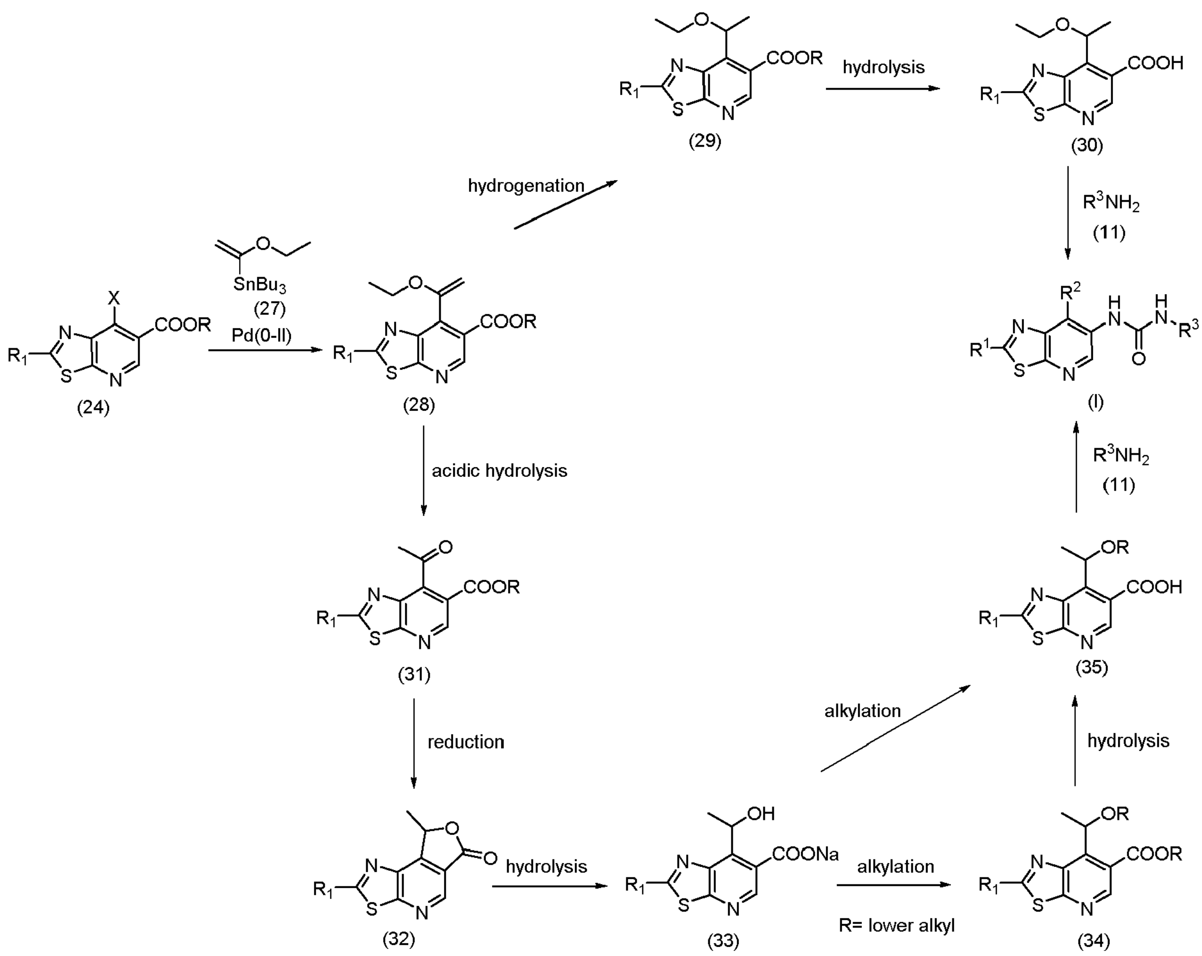


In another embodiment, as described in Scheme 5, compounds of the formula (24) can be converted to the compounds of the formula (28) by reacting with stannane derivatives of the formula (27) by following methods known in the art. The same transformation may also be carried out by other suitable coupling methods known in the art. The above reaction can be mediated by a suitable catalyst known in the art such as $Pd(PPh_3)_2Cl_2$, $Pd_2dba_3$, $Pd(PPh_3)_4$, $Pd(OAc)_2$ or mixture(s) thereof; a suitable ligand known in the art such as BINAP, xanthophos, triphenylphosphine or mixture(s) thereof. As also known from the art, such reactions are effected in the solvents like ethers such as tetrahydrofuran, 1,4-dioxane, and the like; hydrocarbons like toluene; amides such as DMA, DMF and the like or mixture(s) thereof.

Hydrogenation of the compounds of the formula (28) can provide compounds of the formula (29). The said reaction can be carried out although not limited, in presence of a catalyst such as palladium on carbon, palladium hydroxide and the like in presence of hydrogen atmosphere; in one or more solvents like ethers such as THF, 1,4-dioxane and the like; alcohols such as methanol, ethanol and the like; or mixture(s) thereof.

Hydrolysis of the compounds of the formula (29) using the base(s) such as sodium hydroxide, potassium hydroxide, lithium hydroxide and the like; in solvents like THF, water, methanol, ethanol or a mixture(s) thereof to afford the corresponding acids of the formula (30).

Carboxylic acids of formula (30) can be transformed by treatment with DPPA and a tertiary amine base to acyl azides which undergoes rearrangement (Curtius rearrangement) upon heating to form intermediate isocyanates which can be intercepted by appropriate amines of formula (11) to afford urea derivatives of formula (I).

Compounds of the formula (28) can be subjected to acidic hydrolysis by using acids such as hydrochloric acid and the like; in one or more solvents like 1,4-dioxane, THF or a mixture(s) thereof to provide compounds of the formula (31).

Reduction of the ketone compounds of the formula (31) undergoes in situ lactonisation to provide compounds of the formula (32) by treating with a reducing agent, although not limited, such as sodium borohydride, nickel boride, cobalt boride, diisobutyl aluminium hydride, and the like, in one or more solvents, for example, methanol, ethanol, THF or mixture(s) thereof.

Compounds of the formula (33) can be prepared by hydrolysis of the compounds of the formula (32). Such transformation is carried out by using the base(s) such as sodium hydroxide, potassium hydroxide, lithium hydroxide

and the like; in solvents like THF, water, methanol, ethanol or a mixture(s) thereof.

Alkylation of the compounds of the formula (33) with alkyl halides such as methyl iodide, ethyl iodide, propyl bromide; by using a basesuch as sodium hydride, lithium hexamethyldisilazine, cesium carbonate, potassium carbonate, sodium carbonate and the like, in one or more solvents such as DMF, DMA, THF, toluene or mixture(s) thereof to provide compounds of the formula (34).

Upon hydrolysis, compounds of the formula (34) can be converted to the compounds of the formula (35) by following methods known in the art or as described for compounds of formula (29).

Compounds of the formula (33) can also be converted into compounds of formula (35) by treating with alkyl halides such as methyl iodide, ethyl iodide, propyl bromide; by using bases such as sodium hydride, potassium tert-butoxide, sodium tert-butoxide and the like; in one or more solvents such as DMF, DMA, THF or mixture(s) thereof.

Compounds of formula (35) can be converted to the compounds of the present invention of the formula (I) by reacting with amine of formula (11) by following the methods described for compounds of formula (30).

Scheme-6

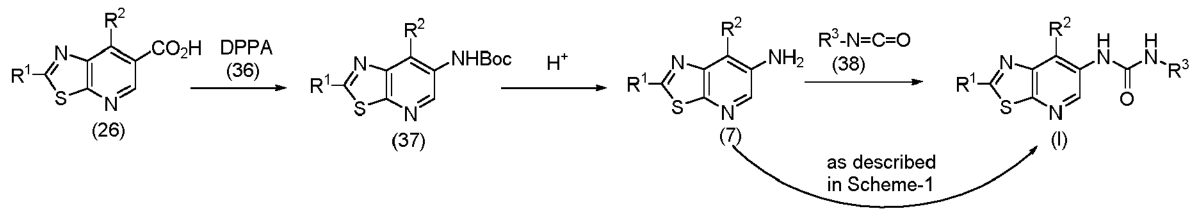


Scheme 6 depicts the alternative method of preparation of compounds of formula (I). Carboxylic acid of formula (26) undergoes Curtius rearrangement in presence of diphenyl phosphoryl azide (36) and a tertiary amine base to afford corresponding isocyanate intermediate which can be intercepted by *tert*-butanol to afford t-butoxycarbonyl protected amino compounds of formula (37). Deprotection of compounds of formula (37) can be carried out under acidic conditions using HCl or TFA to afford corresponding amines of formula (7). Amines of formula (7) can be transformed into the compounds of formula (I) by reacting with isocyantes of formula (38) in presence of tertiary amine bases; in solvents like THF, DCM or 1,4-dioxane to afford the compounds of formula (I) or as described in synthetic scheme-1. Compounds of formula (38) are either commercially available or can be prepared by following the methods known in the art or as described in the synthetic schemes

Scheme 7

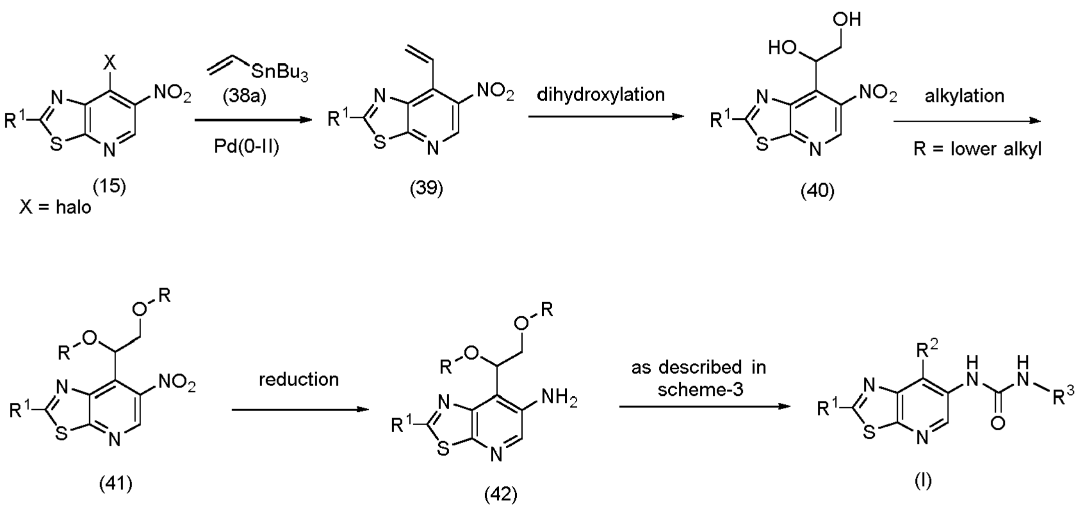


In another embodiment, as described in Scheme 7, compounds of formula (15) can be converted to the compounds of the formula (39) by reacting with stannane derivatives of the formula (38a) by following methods known in

the art or as described in Scheme 1 for the transformation of compounds of the formula (5) to compounds of the formula (7).

Dihydroxylation of the compounds of the formula (39) by following the methods known in the art can provide compounds of the formula (40). The above reaction can be carried out by using oxidants like $KMnO_4$, $OsO_4$, $RuO_4$ and the like or under the conditions of Sharpless dihydroxylation as known in the art in one or more solvents like water, THF, 1,4-dioxane and the like; alcohols such as methanol, ethanol tert-butanol and the like; or mixture(s) thereof.

Alkylation of the compounds of the formula (40) by using bases like, sodium hydride, lithium hexamethyldisilazine, cesium carbonate, potassium carbonate, sodium carbonate and the like; and alkylating reagents like trimethyloxonium tetrafluoroborate; alkyl halides such as methyl iodide, ethyl iodide, propyl bromide in one or more solvents such as DCM, DMF, DMA, THF, toluene or mixture(s) thereof to provide compounds of formula (41).

Reduction of the nitro group of the compounds of the formula (41) to produce the compounds of the formula (42) can be carried out by using reducing agents known in the art or described in Scheme 1 for the transformation of compounds of the formula (3) to the compounds of the formula (4).

Compounds of the formula (42) can be converted to the compounds of the present invention of the formula (I) by following the methods known in the art or as described in Scheme 3 for the transformation of compounds of formula (19) to compounds of formula (I).

Scheme 8

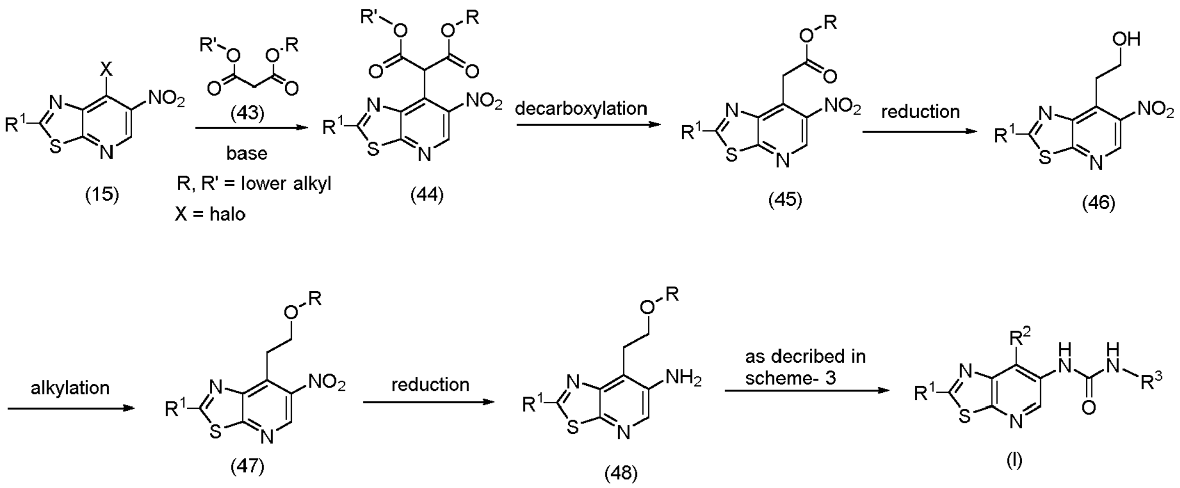


Scheme 8 depicts the method of preparation of compounds of formula (I). Compounds of formula (15) can be treated with mixed malonate derivatives of formula (43) under basic conditions to provide the compounds of the formula (44). The above reaction can be carried out in presence of a suitable base such as LDA, LiHMDS, NaHMDS, n-BuLi, metal hydrides like sodium hydride and the like; Such coupling reactions are carried out in one or more solvents such as ethers such as THF, 1,4-dioxane and the like; amides such as DMF, DMA and the like or mixture(s) thereof.

Symmetrical and unsymmetrical dialkyl malonate derivatives of formula (44) can be decarboxylated to ester derivatives of the formula (45) under acidic conditions known in the art. The above reaction can be carried out using acids like TFA, AcOH, HCl, PTSA and the like, or in basic conditions such as sodium hydroxide, potassium hydroxide and the like; in the presence of salts such as lithium chloride, sodium chloride and the like. Such a transformation can also be achieved under hydrogenation condition using palladium catalyst in suitable solvents like THF, 1,4-dioxane, toluene, methanol, ethanol, and the like.

Chemoselective reduction of ester group in compounds of formula (45) can afford the compounds of the formula (46). The reduction can be carried out using DIBAL-H, $LiBH_4$ in solvents like ethers such as THF, 1,4-dioxane and the like; hydrocarbons such as toluene and the like; halogenated hydrocarbons like DCM and alcohols like methanol, ethanol or mixture(s) thereof.

The compounds of the formula (46) can be alkylated by treating with alkyl halides such as methyl iodide, ethyl iodide, or propyl bromide, by using bases such as sodium hydride, potassium tert-butoxide, sodium tert-butoxide and the like, in one or more solvents such as DMF, DMA, THF or mixture(s) thereof to afford the compounds of the formula (47). Reduction of the compounds of the formula (47) to give compounds of the formula (48) by using methods known in the art or as described in Sheme 1 depicting the transformation of compounds of the formula (3) to the compounds of the formula (4).

The compounds of formula (48) can subsequently be converted to the compounds of the present invention of the formula (I) by following methods known in the art or as described in Scheme 3 depicting the transformation of

compounds of the formula (19) to compounds of the formula (I).

Scheme 9

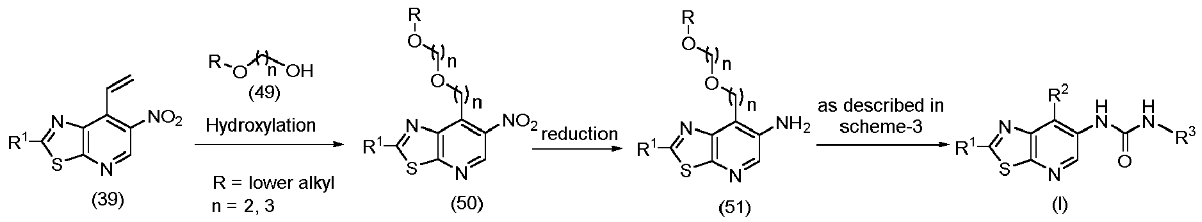


The compounds of the formula (I) can also be prepared by following the methods as described in Scheme 9. Hydroxylation of compounds of formula (39) with alcohol derivatives of formula (49) can be effected in presence of iron sources such as $FeCl_3$, $FeCl_3.6H_2O$, $Fe_2(SO_4)_3$, and $FeBr_3$ employing a suitable acids such as TfOH, HOAc, TsOH and $HClO_4$ As also known from the art, such reactions can be effected in the ethereal solvents like diethyl ether, tetrahydrofuran, 1,4-dioxane, DME, and the like; hydrocarbons like toluene, halogenated hydrocarbons like DCM, chlorobenzene or mixture(s) thereof.

Reduction of the nitro group of the compounds of the formula (50) to produce the compounds of the formula (51) can be carried out by using reducing agents known in the art or described in scheme 1 for the transformation of compounds of the formula (3) to the compounds of the formula (4).

Compounds of the formula (51) can be converted to the compounds of the present invention of the formula (I) by following methods known in the art or as described in Scheme 3 for the transformation of compounds of formula (19) to compounds of formula (I).

Scheme 10

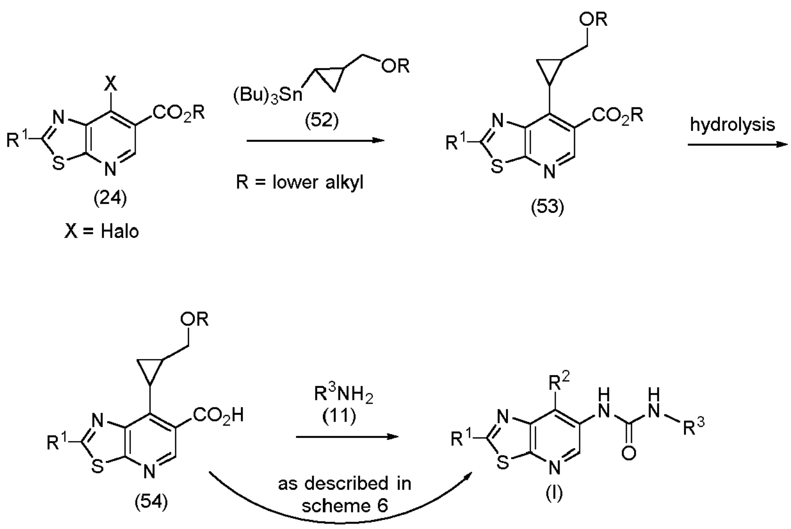


The compounds of the formula (I) can also be prepared by following the methods as described in synthetic Scheme 10. The compounds of formula (53) can be prepared by coupling compounds of formula (24) with a cis or trans isomer of tributyl-(2-alkoxymethyl-cyclopropyl)-stannane derivatives of formula (52) by following the methods known in the art or as described in Scheme 1 for the transformation of compounds of the formula (5) to compounds of the formula (7).

Hydrolysis of the compounds of the formula (53) to give compounds of the formula (54) followed by Curtius rearrangement can afford the compounds of the present invention of the formula (I) by following methods known in the art or as described in Scheme 4 for intermediate of formula (26) to (I) or as described in Scheme-6.

Scheme 11

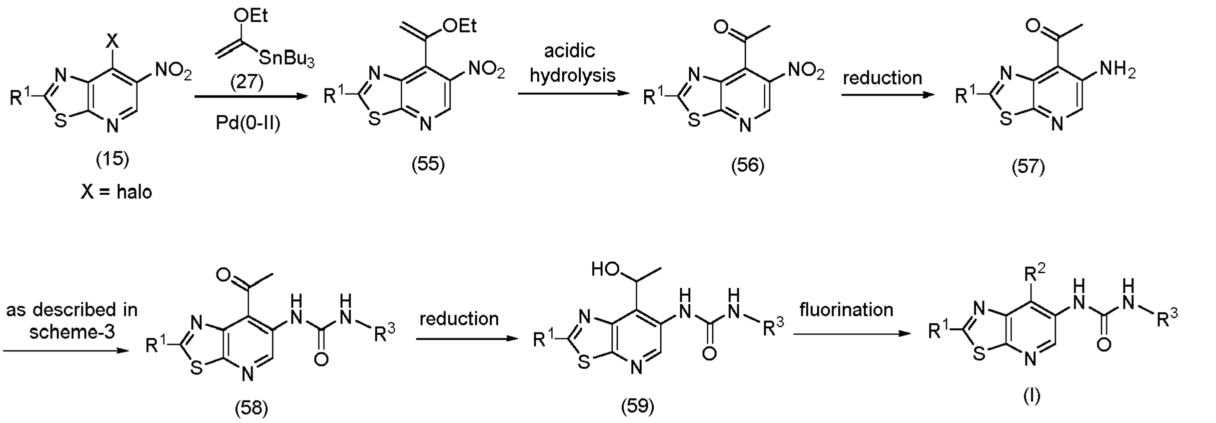

(15)
X = halo
(55)
(56)
(57)
(58)
(59)
(I)

In another embodiment, as described in Scheme 11, compounds of formula (15) can be converted into compounds of formula (56) as depicted for compounds of formula (24) to compounds of formula (31) described in scheme 5

Reduction of the nitro group of the compounds of the formula (56) to produce the compounds of the formula (57) can be carried out using reducing agents known in the art or described in scheme 1 for the transformation of compounds of the formula (3) to the compounds of the formula (4).

Amines of formula (57) can be converted to urea derivatives of formula (58) by following the methods known in the art or as described in Scheme 3 for the transformation of compounds of formula (19) to compounds of formula (I).

Reduction of the keto group in the compounds of the formula (58) to afford the compounds of the formula (59) can be carried out using reducing agents known in the art. Although not limited, such reducing agents include metal hydrides such as $NaBH_4$, $LiBH_4$, $LiAlH_4$ and the like, $BH_3$.DMS and the like. Such reduction of the compounds of formula (58) can be carried out in ethereal solvents like diethyl ether, tetrahydrofuran, 1,4-dioxane and the like; alcohols such as methanol, ethanol and the like; hydrocarbons such as toluene and the like, such as acetic acid and the like; or mixture(s) thereof.

Fluorination of the compounds of the formula (59) using fluorinating agents such as DAST, Selectfluor, $SF_4$ and the like in one or more solvents such as dichloromethane, DMF, DMA, THF, toluene or mixture(s) thereof can provide compounds of the present invention of formula (I).

## Scheme 12

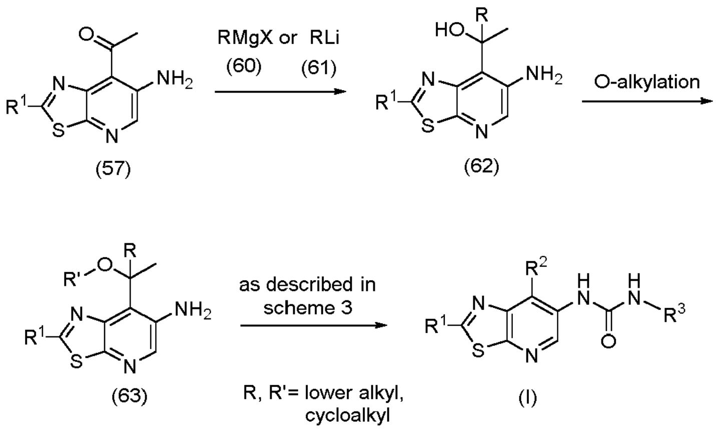

(57)
(62)
(63)
(I)

Scheme 12 depicts the method of preparation of compound of general formula (I). Compounds of the formula (57) can be transformed to compounds of the formula (59) by treating with alkyl magnesium halides of formula (60) or alkyl lithium of formula (61), following the methods known in the art. As also known from the art, such reactions are effected in ethereal solvents such as diethyl ether, tetrahydrofuran, dioxane, and the like; hydrocarbons such as toluene, hexane and the like or mixture(s) thereof.

Selective O-alkylation of the compounds of the formula (62) by using bases such as 1,8-bis(dimethylamino)naphthalene, sodium hydride, lithium hexamethyldisilazine, cesium carbonate, potassium carbonate, sodium carbonate and the like; and alkylating reagents such as trimethyloxonium tetrafluoroborate; alkyl halides such as methyl iodide, ethyl iodide, propyl bromide in one or more solvents such as dichloromethane, DMF, DMA, THF, toluene or mixture(s) thereof can provide compounds of the formula (63).

Compounds of the formula (63) can be converted to the compounds of the present invention of the formula (I) by following the methods known in the art or as described in Scheme 3 for the transformation of compounds of formula (19) to compounds of formula (I).

Scheme 13

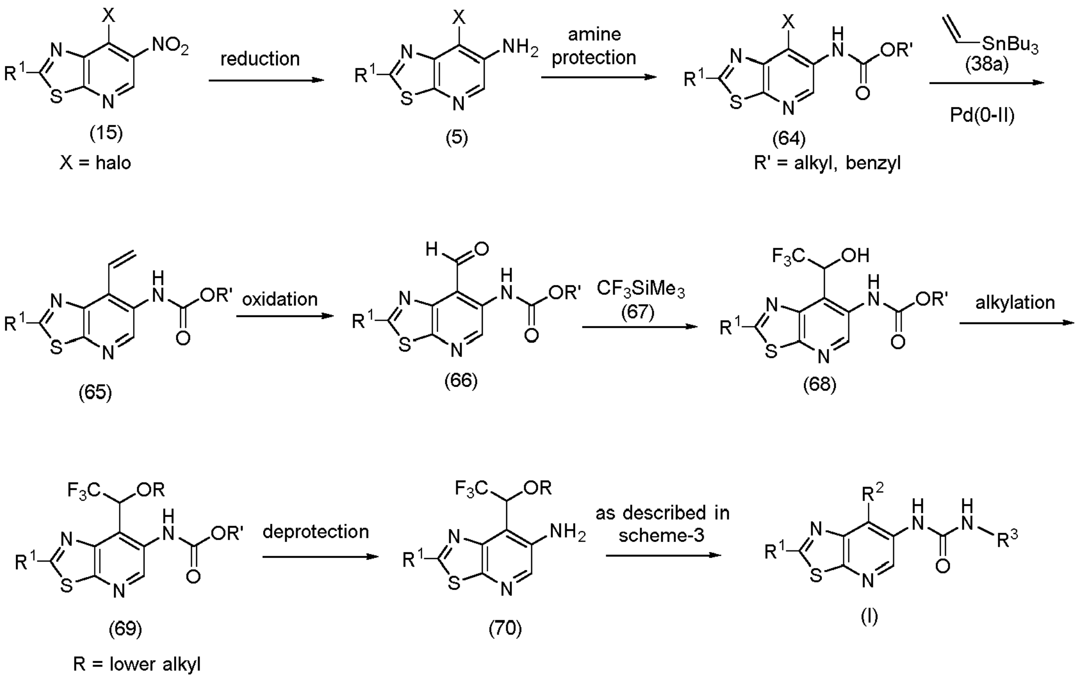

(15) (5) (64) (65) (66) (68) (69) (70) (I)

In another embodiment, the compounds of the present invention of the formula (I) can be prepared as described in Scheme 13.

Reduction of the nitro group of the compounds of the formula (15) to produce the compounds of the formula (5) can be carried out using reducing agents known in the art or described in Scheme 1 for the transformation of compounds of the formula (3) to the compounds of the formula (4).

Amine functionality in the compounds of the formula (5) can be protected as t-butyl carbamate, benzyl carbamate and the like as described in "Protecting Groups in Organic Synthesis" 3rd edition by Theodora W. Greene & Peter G.M Wuts to afford the compounds of formula (64).

Compounds of the formula (64) are converted to the compounds of the formula (65) by coupling with stannane derivatives of the formula (38a), by following methods known in the art or as described in Scheme 1 for the transformation of compounds of the formula (5) to compounds of the formula (7).

The terminal olefin in compounds of the formula (65) can be converted to aldehyde in compounds of the formula (66) by Lemieux-Johnson oxidation using osmium tetroxide dihydroxylation followed by oxidative cleavage of diol using sodium periodate. The same transformation can also be carried out by ozonolysis or osomium tetroxide along with oxidizing agents such as periodic acid ($HIO_4$), lead tetra-acetate, potassium permanganate and the like; in one or more solvents such as t-butanol, 1,4-dioxane, THF, ACN, Water, methanol, ethanol, and the like or mixture(s) thereof.

Compounds of the formula (66) can be converted to the compounds of the formula (68) by nucleophilic trifluoromethylation with Ruppert's reagent, i.e., trifluoromethyltrimethylsilane (67) using carbonate salts such as potassium carbonate, cesium carbonate, lithium carbonate, or sodium acetate and phosphate salts, such as $K_3PO_4$, $K_2HPO_4.3H_2O$, or $KH_2PO_4$; and other nucleophilic initiators such as cesium fluoride, tetrabutyl ammonium fluoride, tetramethylammonium fluoride and the like; in one or more solvents such as DMF, THF, DMSO, DCM and the like or mixture(s) thereof.

Alkylation of the compounds of the formula (68) can be carried out with alkyl halides such as methyl iodide, ethyl iodide, or propyl bromide; in the presence of a base such as sodium hydride, lithium hexamethyldisilazine, cesium carbonate, potassium carbonate, sodium carbonate, and the like; in one or more solvents such as DMF, DMA, THF,

toluene or mixture(s) thereof to provide compounds of the formula (69).

De-protection of suitably protected amino group such as t-butyl carbamate, benzyl carbamate in compounds of formula (69) can be carried out under acidic conditions using HCl, TFA, formic acid, acetic acid and lewis acids like Zinc bromide, stannic chloride and the like; in one or more solvents such as DCM, THF, methanol, water, toluene, 1, 4-dioxane or mixture(s) thereof to provide compounds of the formula (70).

The compounds of formula (70) can be converted to compounds of the present invention of the formula (I) by following methods known in the art or as described in Scheme 3 for the transformation of compounds of formula (19) to compounds of formula (I).

Scheme 14

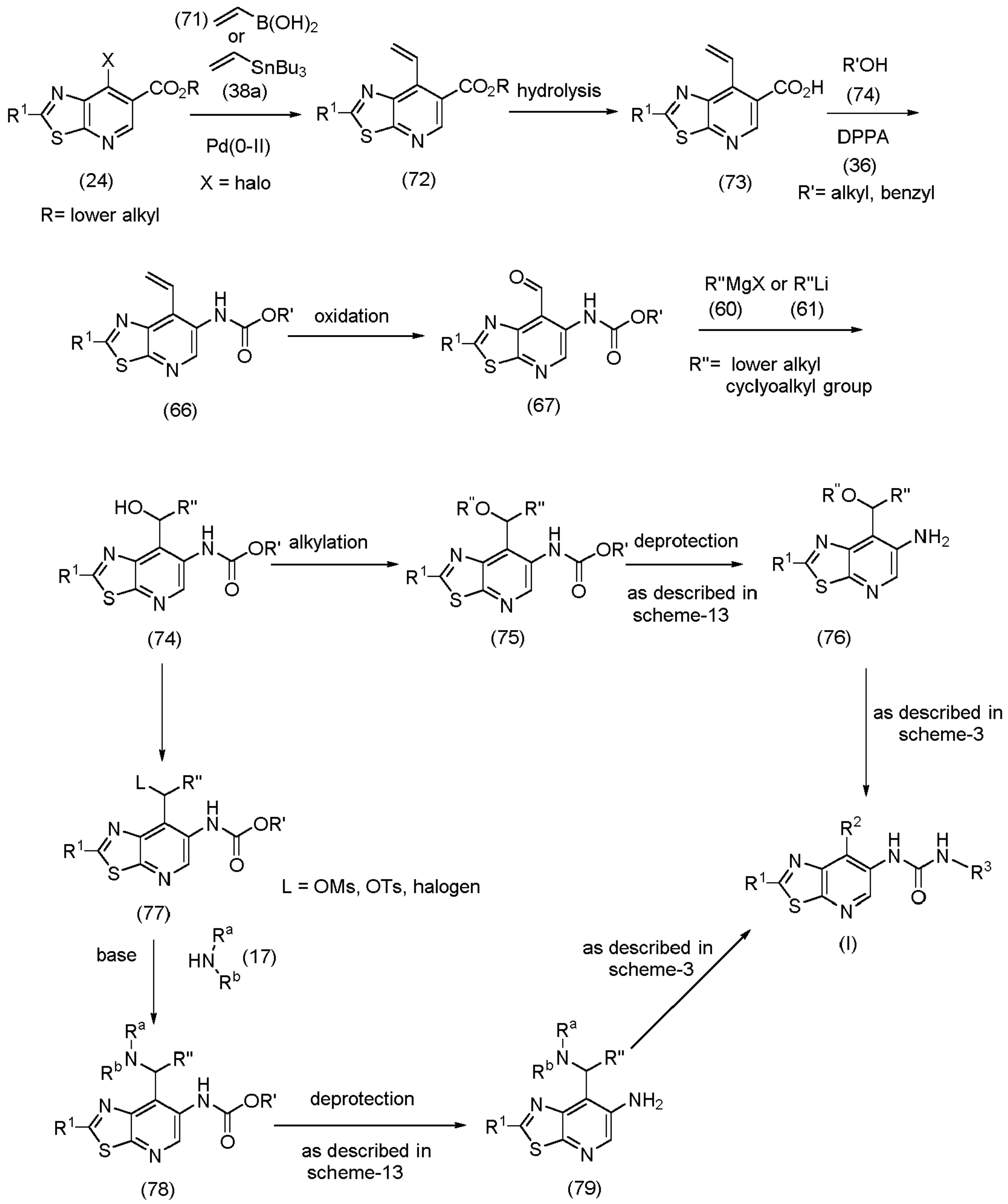


Scheme 14 depicts the method of preparation of compounds of formula (I). Compounds of the formula (24) can be converted to the compounds of the formula (72) by reacting with boronic acid/stannane derivatives of formula (71) or (38a) by following the methods known in the art or as described in Scheme 1 for the transformation of compounds

of the formula (5) to compounds of the formula (7).

Hydrolysis of the ester compounds of the formula (72) by following the methods known in the art can convert to the compounds of the formula (73). The above reaction can be carried out by following the methods known in the art or as described in Scheme 5 for the transformation of compounds of the formula (29) to compounds of the formula (30).

Carboxylic acids of formula (73) can be transformed to the carbamate derivatives (64) under the Curtius rearrangement condition by treatment of the carboxylic acids of formula (64) with DPPA (36) and a tertiary amine base to generate acyl azides which undergo rearrangement (Curtius rearrangement) upon heating to form intermediate isocyanates which can be intercepted by appropriate alcohol of the formula (74) to afford carbamate derivatives of formula (66). The above reaction can be carried out in a stepwise manner; for example, the acid can be converted to the corresponding acid chloride, followed by reaction with sodium azide to afford acyl azide which on heating with appropriate alcohol provides the carbamate derivatives of formula (66).

The terminal olefin in compounds of the formula (66) can be converted to aldehyde in compounds of the formula (67) by following the general method described in Scheme 13.

Compounds of the formula (67) can be transformed to compounds of the formula (74) by reacting with alkyl magnesium halides of the formula (60) or alkyl lithium of formula (61) by following methods known in the art or as described in Scheme 12 for the transformation of compounds of the formula (57) to compounds of the formula (62).

Alkylation of the compounds of the formula (74) to compounds of formula (75) can be carried out by following methods known in the art or as described in Scheme 7 for the transformation of compounds of the formula (40) to compounds of the formula (41).

In another embodiment, compounds of the formula (74) can also be converted to the compounds of the formula (77) wherein the alcohol functionality can be turned into the good leaving group viz. mesylate, tosylates, triflate or halo by following methods known in the art. The above transformation can be carried out by reacting alcohol derivatives of formula (72) with MsCl, TsCl or the like, in the presence of tertiary amines such $Et_3N$, DMAP, DBU, pyridine, and the like. Also known from the art, such reactions can be effected in ether solvents, e.g., diethyl ether, THF, 1.4-dioxane, and the like; hydrocarbons such as toluene and the like; halogenated hydrocarbons, e.g., dichloromethane; or mixture(s) thereof. The above transformation can also be carried out by reacting alcohol derivatives of formula (74) with thionyl chloride, carbon tetrabromide, and the like; to provide the corresponding halides.

Compounds of the formula (77) can be converted to the compounds of the formula (78) following nucleophilic substitution of the leaving group with amine derivatives viz. small dialkyl, monoalkyl, symmetrical, unsymmetrical, cyclic and acyclic amines of formula (17) by following the methods known in the art. The said transformation is carried out in the presence of tertiary amines such as $Et_3N$, DMAP, pyridine and the like or inorganic bases such $K_2CO_3$, $Na_2CO_3$ and the like; and in the presence of NaI, KI and the like. The coupling reaction can be carried out in in the etheral solvents like diethyl ether, 1,4-dioxane, THF and the like; hydrocarbons like toluene and the like; amides such as DMF, DMA and the like; nitriles such as acetonitrile and the like or mixture(s) thereof.

Subsequently compounds of formula (75) and (78) can be converted to compounds of general formula (I) by carrying out the steps as described for the compounds of formula (69) to compounds of formula (I) following Scheme 13.

Scheme 15

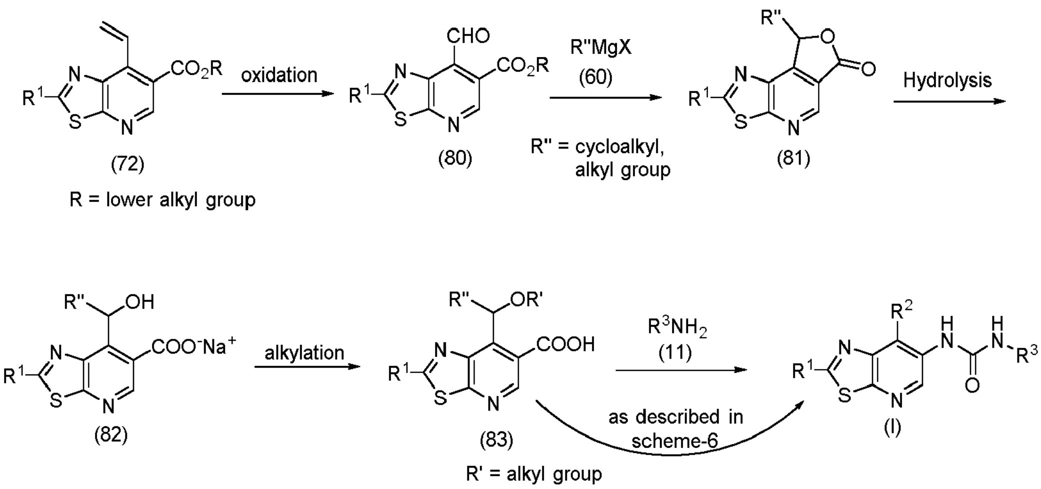

(72) (80) (81) (82) (83) (I)

In another embodiment, the compounds of the present invention of the formula (I) can also be prepared as described in scheme-15. Terminal olefin in compounds of the formula (72) can be converted to aldehyde in compounds

of the formula (80) by following the methods known in the art or as described in Scheme 13 and Scheme 14 for the transformation of compounds of the formula (66) to compounds of the formula (67).

Compounds of the formula (80) can be transformed to compounds of the formula (81) by reacting with alkyl magnesium halides of the formula (60) by following the methods known in the art or as described in scheme 12 for the transformation of compounds of the formula (57) to compounds of the formula (62).

Compounds of the formula (80) can be prepared by hydrolysis of the compounds of the formula (79). Such transformation can be carried out using the base(s) such as sodium hydroxide, potassium hydroxide, lithium hydroxide and the like; in solvents like THF, water, methanol, ethanol or a mixture(s) thereof.

Alkylation of the compounds of the formula (82) to compounds of formula (83) can be carried out by following the methods known in the art or as described in Scheme 5 for the transformation of compounds of the formula (33) to compounds of the formula (35).

Compounds of formula (83) can be converted to the compounds of present invention of the formula (I) by reacting with amines of formula (11) by following the methods as described for compounds of formula (30) as depicted in Scheme 5.

Alternatively, compounds of formula (83) can also be converted to the compounds of present invention of the formula (I) by following the methods as described for compounds of formula (26) and depicted in Scheme 6.

Scheme 16

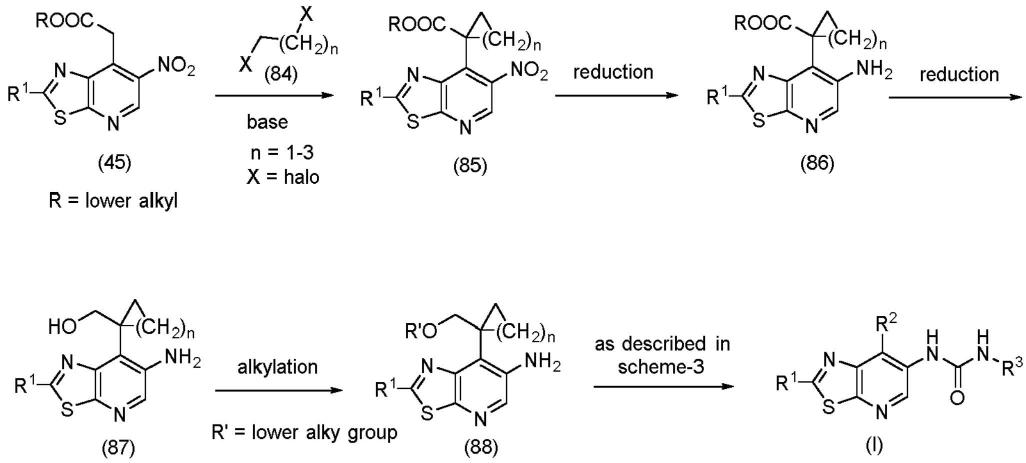


Scheme 16 depicts a method of preparation of compounds of formula (I) starting from nitro derivatives of formula (45). Alkylation of the compounds of the formula (45) with dihaloalkane derivatives of formula (84) using a base such as sodium hydride, lithium diisopropylamide, lithium hexamethyldisilazine, cesium carbonate, potassium carbonate and the like; in one or more solvents such as DMF, DMA, THF, DMSO, toluene or mixture(s) thereof can provide compounds of the formula (85).

Reduction of the nitro group in the compounds of formula (85) to produce the compounds of the formula (86) can be carried out by using reducing agents known in the art or described in Scheme 1 for the transformation of compounds of formula (3) to the compounds of formula (4).

Reduction of the ester group in the compounds of formula (86) to afford primary alcohols as in compounds of the formula (87) can be carried out by using reducing agents known in the art. Although not limited, such reducing agents include LAH, DIBAL-H, $LiBH_4$, $NaBH_4$ and the like, in the presence of ether solvents such as diethyl ether, THF, 1,4-dioxane and the like; hydrocarbons such as toluene and the like; halogenated hydrocarbons such as DCM, DCE, and the like, and alcohols such as methanol, ethanol or mixture(s) thereof.

Alkylation of the compounds of the formula (87) to compounds of formula (88) can be carried out by following methods known in the art or as described in Scheme 7 for the transformation of compounds of the formula (40) to compounds of the formula (41).

Subsequently compounds of formula (88) can be converted to compounds of the present invention of the formula (I) by following methods known in the art or as described in Scheme 3 for the transformation of compounds of formula (19) to compounds of formula (I).

Scheme 17

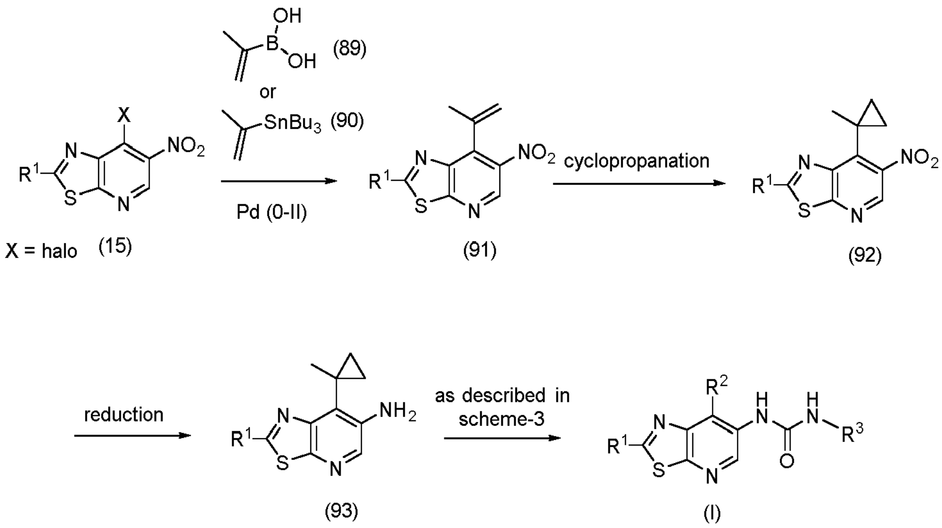


(89) (90) (15) (91) (92) (93) (I)

In another embodiment, the compounds of the present invention of the formula (I) can be prepared as described in Scheme 17. Compounds of the formula (15) can be converted to the compounds of the formula (91) by reacting with boronic acid/stanne derivatives of formula (89) or (90) by following methods known in the art or as described in Scheme 1 for the transformation of compounds of the formula (5) to compounds of the formula (7).

Cyclopropanation of alkene derivatives of the formula (91) to the compounds of the formula (92) can be carried out following the Corey-Chaykovsky reaction by using in situ generated ylides by the deprotonation of sulfonium halides such as trimethylsulfoxonium iodide with bases such as sodium hydride, potassium tert-butoxide, n-butyllithium and the like; by using solvents such as DMF, DMSO, THF, acetonitrile, diethyl ether and the like. The same transformation may also be carried out by the Simmons-Smith reaction by using zinc-copper couple and diiodomethane, dibromo methane in DCM, DCE, diethyl ether, THF and the like. Alternatively, this transformation may also be carried out by reacting the diazo compounds with transition metals compounds (typically containing Cu, Pd, Ni, Co or Rh) to form metal carbenoid complexes which add on to the olefin double bond to bring about the cyclopropanation reaction. The reagents used for the transformation are diazomethane and metal catalyst such as palladium acetate, rhodium acetate, copper triflate and the like and the transformation is carried out in solvents such as DCM, DCE, diethyl ether, THF and the like.

Reduction of the nitro group in the compounds of the formula (92) to produce the compounds of the formula (93) can be carried out by using reducing agents known in the art or described in Scheme 1 for the transformation of compounds of the formula (3) to the compounds of the formula (4).

Subsequently, compounds of formula (93) can be converted to compounds of the present invention of the formula (I) by following methods known in the art or as described in Scheme 3 for the transformation of compounds of formula (19) to compounds of formula (I).

Scheme 18

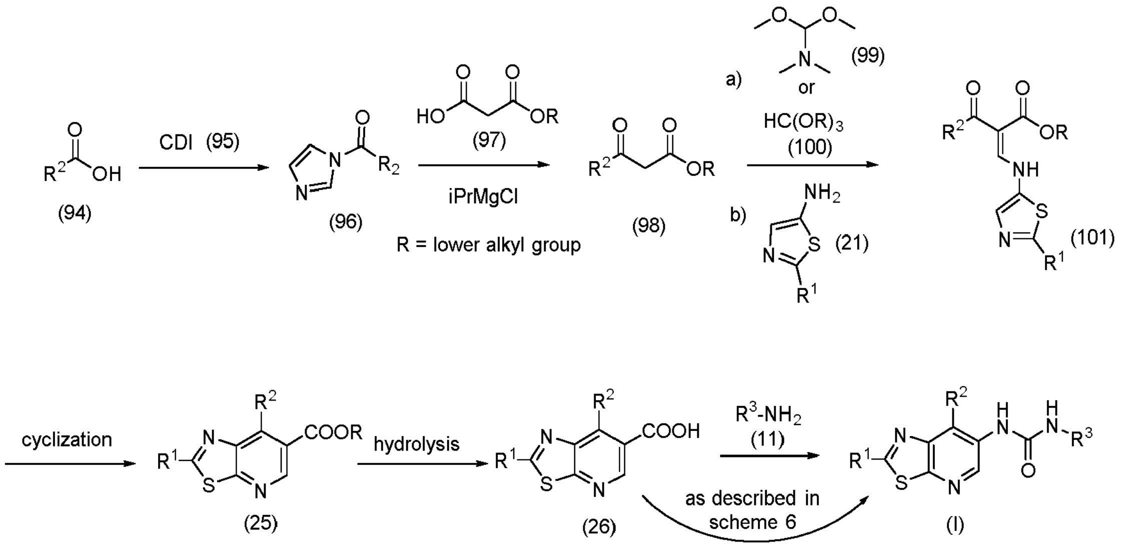


The compound of formula (I) can also be prepared as depicted in Scheme 18. The carboxylic group in compounds of formula (94) can be activated as acyl imidazoles of the general formula (96) by reaction with 1,1'- carbonyldiimidazole (95) in ether solvents - diethyl ether, THF, 1,4-dioxane and the like.

Acyl imidazoles of formula (96) can be converted to the corresponding β-ketoesters of formula (98) by reaction with a solution of a dianion of malonate mono-ester of formula (97) in a polar aprotic solvent such as THF at a temperature ranging between 0°C to 25°C, for a period of about 3 to about 24 hours.

Condensation of β-ketoesters of formula (98) with one carbon synthon equivalent viz. 1,1-dimethoxytrimethylamine (99) or trialkyl formate (100), followed by nucleophilic displacement with appropriately substituted aminothiazoles of formula (21) under reflux conditions in protic solvents such as ethanol, methanol for 2 to 24 hours, can provide compounds of formula (101).

Compounds of formula (101) can be transformed into the compounds of the formula (25) by cyclo-condensation by using 1-propanephosphonic acid cyclic anhydride ($T_3P$) alone or in the presence of organic bases such as trimethylamine, diisopropylethylamine, pyridine, 4-dimethylamino pyridine and like, under reflux conditions in a solvent such as toluene, ethyl acetate, DMF, or THF for a period of about 12 to about 72 hours.

Hydrolysis of the compounds of the formula (25) to give compounds of the formula (26) followed by Curtius rearrangement can afford the compounds of the present invention of the formula (I) by following methods known in the art or as described in Scheme-4 for the intermediate of formula (25) to (I) or as described in Scheme-6.

Scheme 19

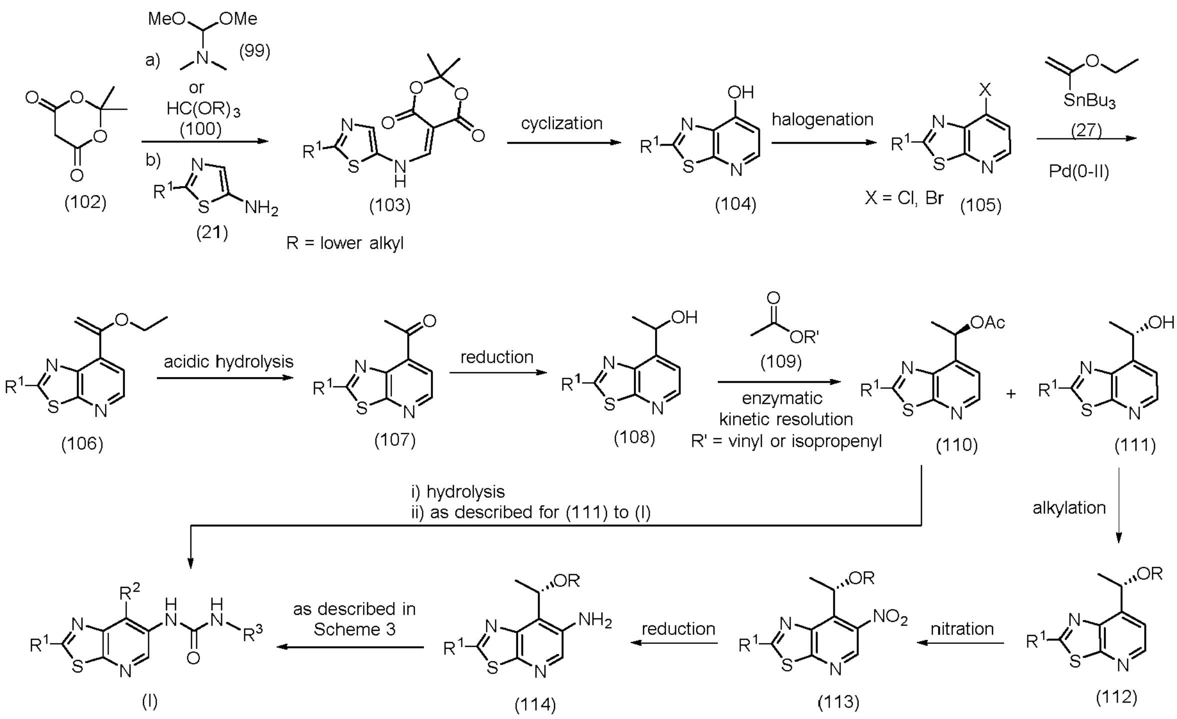


The compounds of the present invention can also be prepared as depicted in Scheme 19. Condensation of Meldrum's acid of formula (102) with one carbon synthon equivalent viz. 1,1-dimethoxytrimethylamine (99) or trialkyl formate (100) followed by nucleophilic displacement with appropriately substituted aminothiazoles of formula (21) under reflux conditions either neat or in protic solvents such as ethanol, methanol can provide compounds of formula (103).

Upon thermal cyclization at elevated temperature(s), the compounds of formula (103) can undergo ring cyclisation to produce compounds of the formula (104). Such reactions can be carried out either neat or in the presence of high boiling solvents such as diphenyl ether, chlorobenzene, xylene and the like or mixture(s) thereof. Compounds of the formula (104) can be halogenated by using reagents such as, although not limited, $POCl_3$ or $POBr_3$ to give the compounds of the formula (105). Such reactions can be carried out either neat or in the presence of hydrocarbons such as toluene, xylene and the like or mixture(s) thereof.

The compounds of formula (105) can be converted to the compounds of the formula (106) by reacting with stannane derivatives of the formula (27) by following methods known in the art or as described in Scheme-5 for the transformation of compounds of formula (24) to compounds of formula (28).

Compounds of the formula (106) can be subjected to acidic hydrolysis by using acids such as hydrochloric acid and the like, in one or more solvents like 1,4-dioxane, THF or a mixture(s) thereof, to provide compounds of the formula (107).

Reduction of the ketone compounds of the formula (107) to provide compounds of the formula (108) by treating with reducing agents such as, but not limited to, sodium borohydride, nickel boride, cobalt boride, diisobutyl aluminium hydride and the like; in one or more solvents like methanol, ethanol, THF or mixture(s) thereof. Alternatively, asymmetric reduction of the compounds of the formula (107) can be carried out by using, although not limited, CBS catalyst, DIP-Cl or under Noyori reduction conditions and the like to give enatiomerically rich (108). Such reduction reactions can be carried out in one or more solvents like THF, DCM, methanol, ethanol and the like or mixture(s) thereof. The compounds of formula (108) can be converted to the compounds of formula (110) and (111) in enatiomerically pure form via enzymatic resolution of racemate or enatiomerically enriched compounds of formula (108) with the methods known in the art. Such transformations can be carried out by using enzymes such as lipase amano PS, lipase amano PS IM, lipase candida SP, cal-B lipozyme, novozyme, and the like. Such transformations can be carried out by using appropriate acylating agents such as isopropenyl acetate, vinyl acetate and the like, by using solvents like diisopropyl ether, MTBE and the like or mixture(s) thereof. Such transformations can be carried out at temperature(s) ranging from 25 to 5°C.

Alkylation of the compounds of the formula (111) can be carried out with alkyl halides such as methyl iodide, ethyl iodide, or propyl bromide by using bases such as sodium hydride, lithium hexamethyldisilazine, cesium carbonate, potassium carbonate, sodium carbonate and the like, in one or more solvents such as DMF, DMA, THF, toluene or

mixture(s) thereof to provide compounds of the formula (112).

Nitration of the compounds of formula (112) can be carried out with nitrating agents such as $AgNO_3$, $Cu(NO_3)_2$, $KNO_3$, fuming nitric acid and the like, in the presence of oxidants like NBS, NCS and the like;,; while employing solvents such as acetic anhydride, trifluoroacetic anhydride or mixture(s) thereof, to provide compounds of the formula (113) or by using methods known in the art.

Reduction of the nitro group of the compounds of the formula (113) to produce the compounds of the formula (114) can be carried out by using reducing agents known in the art or as described in Scheme-1 for the transformation of the compounds of the formula (3) to the compounds of formula (4).

Compounds of formula (114) can be converted to the compounds of the present invention of formula (I) by following methods known in the art or as described in Scheme-3.

Acetate derivatives of formula (110) can also be converted to the compounds of present invention (I) by hydrolysis followed by carrying out steps similar to those described for the transformation of compound of formula (111) to the compound of formula (I).

All intermediates used for the preparation of the compounds of the present invention, were prepared by approaches reported in the literature or by methods known to people skilled in the art of organic synthesis. Detailed experimental procedures for the synthesis of intermediates are given below.

The intermediates and the compounds of the present invention can be obtained in a pure form by any suitable method , for example, by distilling off the solvent in vacuum and/or re-crystallizing the residue obtained from a suitable solvent, such as pentane, diethyl ether, isopropyl ether, chloroform, dichloromethane, ethyl acetate, acetone or their combinations or subjecting it to one of the purification methods, such as column chromatography (e.g., flash chromatography) on a suitable support material such as alumina or silica gel using an eluent such as dichloromethane, ethyl acetate, hexane, methanol, acetone and/or their combinations. Preparative LC-MS method can also be used for the purification of the molecules described herein.

Unless otherwise stated, work-up includes distribution of the reaction mixture between the organic and aqueous phase indicated within parentheses, separation of the layers and drying of the organic layer over sodium sulphate, filtration and evaporation of the solvent. Purification, unless otherwise mentioned, includes purification by silica gel chromatographic techniques, generally by using a mobile phase with suitable polarity, and purification using selective crystallization.

Salts of compound of formula (I) can be obtained by dissolving the compound in a suitable solvent, for example in a chlorinated hydrocarbon, such as methyl chloride or chloroform or a low molecular weight aliphatic alcohol, for example, ethanol or isopropanol, which is then treated with the desired acid or base as described in Berge S. M. et al., "Pharmaceutical Salts, a review article in Journal of Pharmaceutical sciences volume 66, page 1-19 (1977)" and in "Handbook of Pharmaceutical Salts-Properties, Selection, and Use," by P. Heinrich Stahland Camille G.wermuth, Wiley-VCH (2002). Lists of suitable salts can also be found in Remington's Pharmaceutical Sciences, 18th ed., Mack Publishing Company, Easton, PA, 1990, p. 1445, and Journal of Pharmaceutical Science, 66, 2-19 (1977). For example, the salt can be of an alkali metal (e.g., sodium or potassium), alkaline earth metal (e.g., calcium), or ammonium.

The compound of the invention or a composition thereof can potentially be administered as a pharmaceutically acceptable acid-addition, base neutralized or addition salt, formed by reaction with an inorganic acid, such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base, such as sodium hydroxide or potassium hydroxide. The conversion to a salt is accomplished by treatment of the base compound with at least a stoichiometric amount of an appropriate acid. Typically, the free base is dissolved in an inert organic solvent such as diethyl ether, ethyl acetate, chloroform, ethanol, methanol, and the like, and the acid is added in a similar solvent. The mixture is maintained at a suitable temperature (e.g., between 0°C and 50°C). The resulting salt precipitates spontaneously or can be brought out of solution with a less polar solvent.

The stereoisomers of the compounds of formula (I) of the present invention can be prepared by stereospecific syntheses or resolution of racemic compound mixture by using an optically active amine, acid or complex forming agent, and separating the diastereomeric salt/complex by fractional crystallization or by column chromatography.

Prodrugs of the compounds of the invention can be prepared in situ during the isolation and purification of the compounds, or by separately reacting the purified compound with a suitable derivatizing agent. For example, hydroxy groups can be converted to ester groups via treatment with a carboxylic acid in the presence of a catalyst. Examples of cleavable alcohol prodrug moieties include substituted or unsubstituted, branched or unbranched lower alkyl ester moieties, e.g., ethyl esters, lower alkenyl esters, di-lower alkylamino lower-alkyl esters, e.g., dimethylaminoethyl ester, acylamino lower alkyl esters, acyloxy lower alkyl esters (e.g., pivaloyloxymethyl ester), aryl esters, e.g., phenyl ester, aryl-lower alkyl esters, e.g., benzyl ester, optionally substituted, e.g., with methyl, halo, or methoxy substituents aryl and aryl-lower alkyl esters, amides, lower-alkyl amides, di-lower alkyl amides, and hydroxy amides.

The compounds of formula (I) of the present invention can exist in tautomeric forms, such as keto-enol tautomers.

Such tautomeric forms are contemplated as an aspect of the present invention and such tautomers may be in equilibrium or predominant in one of the forms.

The present invention also embraces isotopically-labelled compounds of the present invention which are identical to those recited herein, but for the fact that one or more atoms are replaced by an atom having an atomic mass or mass number different from the atomic mass or mass number usually found in abundance in nature. Examples of isotopes that can be incorporated into compounds of the invention include isotopes of hydrogen, carbon, nitrogen, oxygen, phosphorus, fluorine and chlorine and iodine, such as $^{2}H$, $^{3}H$, $^{11}C$, $^{13}C$, $^{14}C$, $^{15}N$, $^{18}O$, $^{17}O$, $^{31}P$, $^{32}P$, $^{35}S$, $^{18}F$, $^{36}Cl$, and $^{123}I$ respectively.

Thus the present invention further provides a pharmaceutical composition, containing the compounds of the general formula (I) as defined above, its tautomeric form, its stereoisomer, its polymorph, its solvate, its pharmaceutically acceptable salts in combination with pharmaceutically acceptable carriers, diluents, excipients, and the like.

The pharmaceutically acceptable carrier or excipient is preferably one that is chemically inert to the compound of the invention and one that has no detrimental side effects or toxicity under the conditions of use. Such pharmaceutically acceptable carriers or excipients include saline (e.g., 0.9% saline), Cremophor EL® (which is a derivative of castor oil and ethylene oxide available from Sigma Chemical Co., St. Louis, MO) (e.g., 5% Cremophor EL/5% ethanol/90% saline, 10% Cremophor EL/90% saline, or 50% Cremophor EL/50% ethanol), propylene glycol (e.g., 40% propylene glycol/10% ethanol/50% water), polyethylene glycol (e.g., 40% PEG 400/60% saline), and alcohol (e.g., 40% ethanol/60% water). A preferred pharmaceutical carrier is polyethylene glycol, such as PEG 400, and particularly a composition comprising 40% PEG 400 and 60% water or saline. The choice of carrier will be determined in part by the particular compound chosen, as well as by the particular method used to administer the composition. Accordingly, there is a wide variety of suitable formulations of the pharmaceutical composition of the present invention.

Formulations for oral, aerosol, parenteral, subcutaneous, intravenous, intraarterial, intramuscular, intrathecal, intraperitoneal, rectal, and vaginal administration can be developed for the compound of formula (I), its tautomeric form, its stereoisomer, its polymorph, its solvate, and its pharmaceutically acceptable salt.

The pharmaceutical compositions can be administered parenterally, e.g., intravenously, intraarterially, subcutaneously, intradermally, intrathecally, or intramuscularly. Thus, the invention provides compositions for parenteral administration that comprise a solution of the compound of the invention dissolved or suspended in an acceptable carrier suitable for parenteral administration, including aqueous and non-aqueous, isotonic sterile injection solutions.

Overall, the requirements for effective pharmaceutical carriers for parenteral compositions are well known to those of ordinary skill in the art. See Pharmaceutics and Pharmacy Practice, J.B. Lippincott Company, Philadelphia, PA, Banker and Chalmers, eds., pages 238-250 (1982), and ASHP Handbook on Injectable Drugs, Toissel, 4th ed., pages 622-630 (1986). Such compositions include solutions containing anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compound can be administered in a physiologically acceptable diluent in a pharmaceutical carrier, such as a sterile liquid or mixture of liquids, including water, saline, aqueous dextrose and related sugar solutions, an alcohol, such as ethanol, isopropanol (for example in topical applications), or hexadecyl alcohol, glycols, such as propylene glycol or polyethylene glycol, dimethylsulfoxide, glycerol ketals, such as 2,2-dimethyl-1,3-dioxolane-4-methanol, ethers, such as poly(ethyleneglycol) 400, an oil, a fatty acid, a fatty acid ester or glyceride, or an acetylated fatty acid glyceride, with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Oils useful in parenteral formulations include petroleum, animal, vegetable, and synthetic oils. Specific examples of oils useful in such formulations include peanut, soybean, sesame, cottonseed, corn, olive, petrolatum, and mineral oil. Suitable fatty acids for use in parenteral formulations include oleic acid, stearic acid, and isostearic acid. Ethyl oleate and isopropyl myristate are examples of suitable fatty acid esters.

Suitable soaps for use in parenteral formulations include fatty alkali metal, ammonium, and triethanolamine salts, and suitable detergents include (a) cationic detergents such as, for example, dimethyl dialkyl ammonium halides, and alkyl pyridinium halides, (b) anionic detergents such as, for example, alkyl, aryl, and olefin sulfonates, alkyl, olefin, ether, and monoglyceride sulfates, and sulfosuccinates, (c) nonionic detergents such as, for example, fatty amine oxides, fatty acid alkanolamides, and polyoxyethylene polypropylene copolymers, (d) amphoteric detergents such as, for example, alkyl-β-aminopropionates, and 2-alkyl-imidazoline quaternary ammonium salts, and (e) mixtures thereof.

The parenteral formulations typically will contain from about 0.5% or less to about 25% or more by weight of a compound of the invention in solution. Preservatives and buffers can be used. In order to minimize or eliminate irritation at the site of injection, such compositions can contain one or more nonionic surfactants having a hydrophile-lipophile balance (HLB) of from about 12 to about 17. The quantity of surfactant in such formulations will typically range from about 5% to about 15% by weight. Suitable surfactants include polyethylene sorbitan fatty acid esters, such as sorbitan monooleate and the high molecular weight adducts of ethylene oxide with a hydrophobic base, formed by the condensation

of propylene oxide with propylene glycol. The parenteral formulations can be presented in unit-dose or multi-dose sealed containers, such as ampoules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets.

Topical formulations, including those that are useful for transdermal drug release, are well known to those of skill in the art and are suitable in the context of the present invention for application to skin.

Formulations suitable for oral administration can consist of (a) liquid solutions, such as an effective amount of a compound of the invention dissolved in diluents, such as water, saline, or orange juice; (b) capsules, sachets, tablets, lozenges, and troches, each containing a pre-determined amount of the compound of the invention, as solids or granules; (c) powders; (d) suspensions in an appropriate liquid; and (e) suitable emulsions. Liquid formulations can include diluents, such as water and alcohols, for example, ethanol, benzyl alcohol, and the polyethylene alcohols, either with or without the addition of a pharmaceutically acceptable surfactant, suspending agent, or emulsifying agent. Capsule forms can be of the ordinary hard- or soft-shelled gelatin type containing, for example, surfactants, lubricants, and inert fillers, such as lactose, sucrose, calcium phosphate, and cornstarch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the compound ingredient in a flavor, usually sucrose and acacia or tragacanth, as well as pastilles comprising a compound of the invention in an inert base, such as gelatin and glycerin, or sucrose and acacia, emulsions, gels, and the like containing, in addition to the compound of the invention, such excipients as are known in the art.

A compound of the present invention, alone or in combination with other suitable components, can be made into aerosol formulations to be administered via inhalation. A compound of the invention is preferably supplied in finely divided form along with a surfactant and propellant. Typical percentages of the compounds of the invention can be about 0.01% to about 20% by weight, preferably about 1% to about 10% by weight. The surfactant must, of course, be nontoxic, and preferably soluble in the propellant. Representative of such surfactants are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides can be employed. The surfactant can constitute from about 0.1% to about 20% by weight of the composition, preferably from about 0.25% to about 5%. The balance of the composition is ordinarily propellant. A carrier can also be included as desired, e.g., lecithin, for intranasal delivery. These aerosol formulations can be placed into acceptable pressurized propellants, such as dichlorodifluoromethane, propane, nitrogen, and the like. They also can be formulated as pharmaceuticals for non-pressured preparations, such as in a nebulizer or an atomizer. Such spray formulations can be used to spray mucosa.

Additionally, the compound of the invention can be made into suppositories by mixing with a variety of bases, such as emulsifying bases or water-soluble bases. Formulations suitable for vaginal administration can be presented as pessaries, tampons, creams, gels, pastes, foams, or spray formulas containing, in addition to the compound ingredient, such carriers as are known in the art to be appropriate.

The concentration of the compound in the pharmaceutical formulations can vary, e.g., from less than about 1% to about 10%, to as much as about 20% to about 50% or more by weight, and can be selected primarily by fluid volumes, and viscosities, in accordance with the particular mode of administration selected.

For example, a typical pharmaceutical composition for intravenous infusion could be made up to contain 250 ml of sterile Ringer's solution, and 100 mg of at least one compound of the invention. Actual methods for preparing parenterally administrable compounds of the invention will be known or apparent to those skilled in the art and are described in more detail in, for example, Remington's Pharmaceutical Science (17th ed., Mack Publishing Company, Easton, PA, 1985).

It will be appreciated by one of ordinary skill in the art that, in addition to the aforesaid described pharmaceutical compositions, the compound of the invention can be formulated as inclusion complexes, such as cyclodextrin inclusion complexes, or liposomes. Liposomes can serve to target a compound of the invention to a particular tissue, such as lymphoid tissue or cancerous hepatic cells. Liposomes can also be used to increase the half-life of a compound of the invention. Many methods are available for preparing liposomes, as described in, for example, Szoka et al., Ann. Rev. Biophys. Bioeng., 9, 467 (1980) and U.S. Patents no. 4235871, 4501728, 4837028, and 5019369.

The compounds of the invention can be administered in a dose sufficient to treat the disease, condition or disorder. Such doses are known in the art (see, for example, the *Physicians' Desk Reference* (2004)). The compounds can be administered using techniques such as those described in, for example, Wasserman et al., Cancer, 36, pp. 1258-1268 (1975) and Physicians' Desk Reference, 58th ed., Thomson PDR (2004).

Suitable doses and dosage regimens can be determined by conventional range-finding techniques known to those of ordinary skill in the art. Generally, treatment is initiated with smaller dosages that are less than the optimum

dose of the compound of the present invention. Thereafter, the dosage is increased by small increments until the optimum effect under the circumstances is reached. The present method can involve the administration of about 0.1 μg to about 50 mg of at least one compound of the invention per kg body weight of the individual. For a 70 kg patient, dosages of from about 10 μg to about 200 mg of the compound of the invention would be more commonly used, depending on a patient's physiological response.

By way of example and not intending to limit the invention, the dose of the pharmaceutically active agent(s) described herein for methods of treating a disease or condition as described above can be about 0.001 to about 1 mg/kg body weight of the subject per day, for example, about 0.001 mg, 0.002 mg, 0.005 mg, 0.010 mg, 0.015 mg, 0.020 mg, 0.025 mg, 0.050 mg, 0.075 mg, 0.1 mg, 0.15 mg, 0.2 mg, 0.25 mg, 0.5 mg, 0.75 mg, or 1 mg/kg body weight per day. The dose of the pharmaceutically active agent(s) described herein for the described methods can be about 1 to about 1000 mg/kg body weight of the subject being treated per day, for example, about 1 mg, 2 mg, 5 mg, 10 mg, 15 mg, 0.020 mg, 25 mg, 50 mg, 75 mg, 100 mg, 150 mg, 200 mg, 250 mg, 500 mg, 750 mg, or 1000 mg/kg body weight per day.

The terms "treat," "ameliorate," and "inhibit," as well as words stemming therefrom, as used herein, do not necessarily imply 100% or complete treatment, amelioration, or inhibition. Rather, there are varying degrees of treatment, amelioration, and inhibition of which one of ordinary skill in the art recognizes as having a potential benefit or therapeutic effect. In this respect, the disclosed methods can provide any amount of any level of treatment, amelioration, or inhibition of the disorder in a mammal. For example, a disorder, including symptoms or conditions thereof, may be reduced by, for example, 100%, 90%, 80%, 70%, 60%, 50%, 40%, 30%, 20%, or 10%. Furthermore, the treatment, amelioration, or inhibition provided by the inventive method can include treatment, amelioration, or inhibition of one or more conditions or symptoms of the disorder, e.g., cancer. Also, for purposes herein, "treatment," f"amelioration," or "inhibition" can encompass delaying the onset of the disorder, or a symptom or condition thereof.

In accordance with the invention, the term subject includes an "animal" which in turn includes a mammal such as, without limitation, the order Rodentia, such as mice, and the order Lagomorpha, such as rabbits. In one aspect, the mammals are from the order Carnivora, including Felines (cats) and Canines (dogs). In another aspect, the mammals are from the order Artiodactyla, including Bovines (cows) and Swine (pigs) or of the order Perssodactyla, including Equines (horses). In a further aspect, the mammals are of the order Primates, Ceboids, or Simoids (monkeys) or of the order Anthropoids (humans and apes). In yet another aspect, the mammal is human.

The compounds of invention are useful in treating any disorders involving NF-κB pathway activation particularly inflammation related or oncological disorders dependent on NF-κB pathway deregulation.

It has been reported that inhibitors of MALT1 proteolytic activity have antiproliferative activity against ABC type DLBCL lymphoma models (Fontan et al., Clin Cancer Res, 19, 6662-68, 2013; Fontan et al., Cancer Cell, 22, 812-24, 2012; Nagel et al., Cancer Cell, 22, 825-37, 2012).

Based on the reports that describe involvement of MALT1 in several disease pathologies, the compounds can also be effective against other different types of oncological disorders like.g., lung adenocarcinoma (Jiang et al., Cancer Research, 71, 2183-92, 2011; Pan et al., Oncogene, 1-10, 2015), breast cancer (Pan et al., Mol Cancer Res, 14, 93-102, 2016), mantle cell lymphoma (Penas et al., Blood, 115, 2214-19, 2010; Rahal et al., Nature Medicine, 20, 87-95, 2014), marginal zone lymphoma (Remstein et al., Am J Pathol, 156, 1183-88, 2000; Baens et al., Cancer Res, 66, 5270-77, 2006; Ganapathi et al., Oncotarget, 1-10, 2016; Bennett et al., Am J of Surgical Pathology, 1-7, 2016), cutaneous T cell lymphomas like Sezary syndrome (Qin et al., Blood, 98, 2778-83, 2001; Doebbeling et al., J of Exp and Clin Cancer Res, 29, 1-5, 2010), certain types of Chronic lymphocytic leukemia with CARD11 mutation, and also certain subtypes of GCB-DLBCL type of cancer that involves MALT 1.

Also, targeting an immunomodulatory protein can have direct and indirect benefits in a variety of inflammatory disorders of multiple organs. In that regard, the compounds described in the invention can be useful in treating psoriasis (Lowes et al., Ann Review Immunology, 32, 227-55, 2014; Afonina et al., EMBO Reports, 1-14, 2016; Howes et al, Biochem J, 1-23, 2016), multiple schlerosis (Jabara et al., J Allergy Clin Immunology, 132, 151-58, 2013; McGuire et al., J of Neuroinflammation, 11, 1-12, 2014) Rheumatoid arthritis, Sjogren's syndrome (Streubel et al., Clin Cancer Research, 10, 476-80, 2004; Sagaert et al., Modern Pathology, 19, 225-32, 2006), ulcerative collitis (Liu et al., Oncotarget, 1-14, 2016), MALT lymphomas of different organs (Suzuki et al., Blood, 94, 3270-71, 1999; Akagi et al., Oncogene, 18, 5785-94, 1999) and different types of allergic disorders resulting from chronic inflammation.

The present invention provides a pharmaceutical composition, containing the compound of the general formula (I) as defined herein, its tautomeric form, its stereoisomer, its polymorph, its solvate, and its pharmaceutically acceptable salt in combination with the usual pharmaceutically employed carriers, diluents, and the like are useful for the treatment of a disease or disorder mediated through MALT1.

The present invention provides a pharmaceutical composition, containing the compound of the general formula (I) as defined herein, its tautomeric form, its stereoisomer, its polymorph, its solvate, and its pharmaceutically acceptable salt in combination with the usual pharmaceutically employed carriers, diluents, and the like are useful for the treatment of a disease or disorder such as cancer, inflammation or inflammatory disease or disorder, or allergic or autoimmune disease or disorder.

The present invention provides a pharmaceutical composition, containing the compound of the general formula (I) as defined herein, its tautomeric form, its stereoisomer, its polymorph, its solvate, and its pharmaceutically acceptable salt in combination with the usual pharmaceutically employed carriers, diluents, and the like are useful for the treatment of a disease or disorder such as lymphoma or leukemia.

The present invention provides a pharmaceutical composition, containing the compound of the general formula (I) as defined herein, its tautomeric form, its stereoisomer, its polymorph, its solvate, and its pharmaceutically acceptable salt in combination with the usual pharmaceutically employed carriers, diluents, and the like are useful for the treatment of a disease or disorder such as ABC-DLBCL type of lymphomas, a subset of GCB-DLBCL type of lymphomas involving MALT1, MALT lymphomas, mantle cell lymphoma, marginal zone lymphoma, cutaneous T cell lymphomas, primary effusion lymphoma, pancreatic cancer, chronic lymphocytic leukemia with CARD11 mutation, Hodgkin's and Non-Hodgkin's lymphomas, or a subset of acute myelogenous leukemia involving MALT1.

The present invention provides a pharmaceutical composition, containing the compound of the general formula (I) as defined herein, its tautomeric form, its stereoisomer, its polymorph, its solvate, and its pharmaceutically acceptable salt in combination with the usual pharmaceutically employed carriers, diluents, and the like are useful for the treatment of a disease or disorder such as germ cell tumors and neoplasm involving plasma cell, brain tumors including glioblastoma, hepatic adenomas, medulloblastoma, mesothelioma, different types of melanomas and multiple myeloma, clear cell carcinoma, or adenocarcinoma of lung, breast, bladder, skin, brain, colon, stomach, cervix, ovary, uterus, prostate, liver, and kidney.

The present invention provides a pharmaceutical composition, containing the compound of the general formula (I) as defined herein, its tautomeric form, its stereoisomer, its polymorph, its solvate, and its pharmaceutically acceptable salt in combination with the usual pharmaceutically employed carriers, diluents, and the like are useful for the treatment of a disease or disorder such as psoriasis, multiple sclerosis, systemic lupus erythematosus, BENTA disease, ulcerative colitis, pancreatitis, rheumatic fever, or rheumatoid arthritis.

The present invention provides a pharmaceutical composition, containing the compound of the general formula (I) as defined herein, its tautomeric form, its stereoisomer, its polymorph, its solvate, and its pharmaceutically acceptable salt in combination with the usual pharmaceutically employed carriers, diluents, and the like are useful for the treatment of a disease or disorder such as ankylosing spondylitis, inflammatory bowel disease, Crohn's disease, gastritis, celiac disease, gout, organ or transplant rejection, chronic allograft rejection, acute or chronic graft-versus-host disease, Behcet's disease, uveitis, dermatitis including atopic dermatitis, dermatomyositis, inflammation of skeletal muscles leading to polymyositis, myasthenia gravis, Grave's disease, Hashimoto thyroiditis, blistering disorders, vasculitis syndromes, Hennoch-Schonlein Purpura, or immune-complex vasculitides.

The present invention provides a pharmaceutical composition, containing the compound of the general formula (I) as defined herein, its tautomeric form, its stereoisomer, its polymorph, its solvate, and its pharmaceutically acceptable salt in combination with the usual pharmaceutically employed carriers, diluents, and the like are useful for the treatment of a disease or disorder such as Sjoren's syndrome, asthma, bronchitis, or chronic obstructive pulmonary disease.

The present invention provides a pharmaceutical composition, containing the compound of the general formula (I) as defined herein, its tautomeric form, its stereoisomer, its polymorph, its solvate, and its pharmaceutically acceptable salt in combination with the usual pharmaceutically employed carriers, diluents, and the like are useful for the treatment of a disease or disorder such as cystic fibrosis, respiratory diseases involving lungs leading to respiratory distress and failure.

The present invention also provides the use of a compound of formula (I) as defined herein in the preparation of a medicament for treating a disease or disorder mediated through MALT1.

The present invention also provides the use of a compound of formula (I) as defined herein in the preparation of a medicament for treating a disease or disorder such as cancer, inflammation or inflammatory disease or disorder, or allergic or autoimmune disease or disorder.

The present invention also provides the use of a compound of formula (I) as defined herein in the preparation of a medicament for treating a disease or disorder such as lymphoma or leukemia.

The present invention also provides the use of a compound of formula (I) as defined herein in the preparation of a medicament for treating a disease or disorder such as ABC-DLBCL type of lymphomas, a subset of GCB-DLBCL type of lymphomas involving MALT1, MALT lymphomas, mantle cell lymphoma, marginal zone lymphoma, cutaneous T cell lymphomas, primary effusion lymphoma, pancreatic cancer, chronic lymphocytic leukemia with CARD11 mutation, Hodgkin's and Non-Hodgkin's lymphomas, or a subset of acute myelogenous leukemia involving MALT1.

The present invention also provides the use of a compound of formula (I) as defined herein in the preparation of a medicament for treating a disease or disorder such as germ cell tumors and neoplasm involving plasma cell, brain tumors including glioblastoma, hepatic adenomas, medulloblastoma, mesothelioma, different types of melanomas and multiple myeloma, clear cell carcinoma, or adenocarcinoma of lung, breast, bladder, skin, brain, colon, stomach, cervix, ovary, uterus, prostate, liver, and kidney.

The present invention also provides the use of a compound of formula (I) as defined herein in the preparation

of a medicament for treating a disease or disorder such as psoriasis, multiple sclerosis, systemic lupus erythematosus, BENTA disease, ulcerative colitis, pancreatitis, rheumatic fever, or rheumatoid arthritis.

The present invention also provides the use of a compound of formula (I) as defined herein in the preparation of a medicament for treating a disease or disorder such as ankylosing spondylitis, inflammatory bowel disease, Crohn's disease, gastritis, celiac disease, gout, organ or transplant rejection, chronic allograft rejection, acute or chronic graft-versus-host disease, Behcet's disease, uveitis, dermatitis including atopic dermatitis, dermatomyositis, inflammation of skeletal muscles leading to polymyositis, myasthenia gravis, Grave's disease, Hashimoto thyroiditis, blistering disorders, vasculitis syndromes, Hennoch-Schonlein Purpura, or immune-complex vasculitides.

The present invention also provides the use of a compound of formula (I) as defined herein in the preparation of a medicament for treating a disease or disorder such as Sjoren's syndrome, asthma, bronchitis, or chronic obstructive pulmonary disease.

The present invention also provides the use of a compound of formula (I) as defined herein in the preparation of a medicament for treating a disease or disorder such as cystic fibrosis, respiratory diseases involving lungs leading to respiratory distress and failure.

The present invention also provides the compound of formula (I) as defined herein for use in treating a disease or disorder mediated through MALT1.

Following are the abrrevations used and meaning thereof in the specification:

| | |
|---|---|
| EtOAc: | Ethyl acetate |
| DCM: | Dichloromethane |
| ACN: | Acetonitrile |
| THF: | Tetrahydrofuran |
| DMSO: | Dimethylsulfoxide |
| MeOH: | Methanol |
| EtOH: | Ethanol |
| DMF: | *N*,*N*-Dimethylformamide |
| DMA: | *N*,*N*-Dimethylacetamide |
| DMF | DMA: *N*,*N*-Dimethylformamide dimethyl acetal |
| NBS: | N-Bromosuccinimide |
| Pd-C: | Palladium on Carbon |
| LDA: | Lithium diisopropylamide |
| TFA: | Trifluoroacetic acid |
| PTSA: | *p*-Toluenesulfonic acid |
| DIBAL-H: | Diisobutylaluminum hydride |
| LAH: | Lithium aluminum hydride |
| Py: | Pyridine |
| Dppa: | Diphenyl phosphoryl azide |
| CDI: | 1,1'-Carbonyldiimidazole |
| TEA: | Triethyl amine |
| DIPEA: | N,N-Diisopropylethyl amine |
| DMAP: | 4-(Dimethylamino)pyridine |
| EDCI: | *N*-(3-Dimethylaminopropyl)-*N'*-ethylcarbodiimide hydrochloride |
| HOBT: | 1-Hydroxybenzotriazole |
| TFOH: | Trifluoromethanesulfonic acid |
| Xantphos: | 4,5-Bis(diphenylphosphino)-9,9-dimethylxanthene |
| tBuXphos: | 2-Di-*tert*-butylphosphino-2',4',6'-triisopropylbiphenyl, |
| Xphos: | 2-Dicyclohexylphosphino-2',4',6'-triisopropylbiphenyl |
| dppf: | 1,1'-Ferrocenediyl-bis(diphenylphosphine) |
| DAST: | (Diethylamino)sulfur trifluoride |
| $Pd_2(dba)_3$: | Tris(dibenzylideneacetone) dipalladium (0) |
| Boc: | *tert*-Butoxycarbonyl |
| Ac: | Acetyl |
| TMSI: | Trimethyl silyl iodide |
| TBAI: | Tetrabutyl ammonium iodide |
| $PPh_3$: | Triphenyl phosphine |
| dba: | Dibenzylideneacetone |
| BINAP: | 2,2'-Bis(diphenylphosphino)-1,1'-binaphthyl |
| MsCl: | Methanesulfonyl chloride |

| | |
|---|---|
| TsCl: | Toluenesulfonyl chloride |
| DMAP: | 4-Dimethylaminopyridine |
| DBU: | 1,8-Diazabicyclo[5.4.0]undec-7-ene |
| NIS: | N-iodosuccinimide |
| LiHMDS: | Lithium bis(trimethylsilyl)amide |
| NaHMDS: | Sodium bis(trimethylsilyl)amide |
| CBS: | Tetrahydro-1-methyl-3,3-diphenyl-1*H*,3*H*-pyrrolo[1,2-c][1,3,2]oxazaborole |
| DIP-Cl: | B-Chlorodiisopinocampheylborane |
| DMS: | dimethyl sulfide |
| DAST: | Diethylaminosulfur trifluoride |
| DME: | dimethoxyethane |
| DCE: | Dichloroethane |
| RBF: | round bottom flask |
| NMR: | Nuclear magnetic resonance |
| LCMS: | Liquid chromatography-mass spectrometry |
| ESI-MS: | Electrospray Ionization Mass Spectrometry: |
| GCMS: | Gas chromatography-mass spectrometry |
| TLC: | thin layer chromatography |
| MALT1: | Mucosa Associated Lymphoid Tissue Lymphoma translocation protein |
| Bel-10: | B cell lymphoma-10 |
| NF- κB: | Nuclear Factor kappa beta |
| ABC - DLBCL: | Activated B cell like Diffuse Large B cell lymphoma |
| GCB-DLBCL: | Germinal center B cell like Diffuse Large B cell lymphoma |
| API-MALT1: | Inhibitor of apoptosis-MALT1 translocation |
| IgH-MALT1: | Immunoglobulin Heavy chain-MALT1 translocation |
| CARMA: | CARD containing membrane associated guanylate kinase |
| TCR: | T cell receptor |
| BCR: | B cell receptor |
| CARD: | Caspase activation and recruitment domain |
| GPCR: | G protein coupled receptor |
| AMC: | Amino methyl coumarin |
| Leu: | Leucine |
| Arg: | Arginine |
| Ser: | Serine |
| MES: | 2-(N-morpholino) ethane sulphonic acid |
| CHAPS: | 3-[(3-cholamidopropyl)dimethylammonio]-1-propanesulfonate |
| mM: | millimolar |
| μM: | micromolar |
| DTT: | Dithiothreitol |
| μl: | microliter |
| ng: | nanogram |
| nM: | nanomolar |
| nm: | nanometer |
| RFU: | Relative Fluorescence Unit |
| $IC_{50}$: | Half maximal inhibitory concentration |
| HEK-293: | Human embryonic kidney - 293 cells |
| FBS: | Fetal bovine serum |
| RLU: | Relative Luminescence Unit |
| DMEM: | Dulbecco's Modified Eagle Medium |
| CCK-8: | Cell counting kit - 8 |
| OD: | Optical density |

The following examples are provided to further illustrate the present invention and should not be constructed in any way to limit the scope of the present invention.

All $^1$HNHR spectra were determined in the solvent indicated and chemical shifts are reported in δ units downfield from the internal standard tetramethylsilane (TMS) and interproton coupling constants are reported in Hertz (Hz).

**Example-1:** Preparation of 7-Cyclopropyl-2-methylthiazolo[5,4-b]pyridin-6-amine

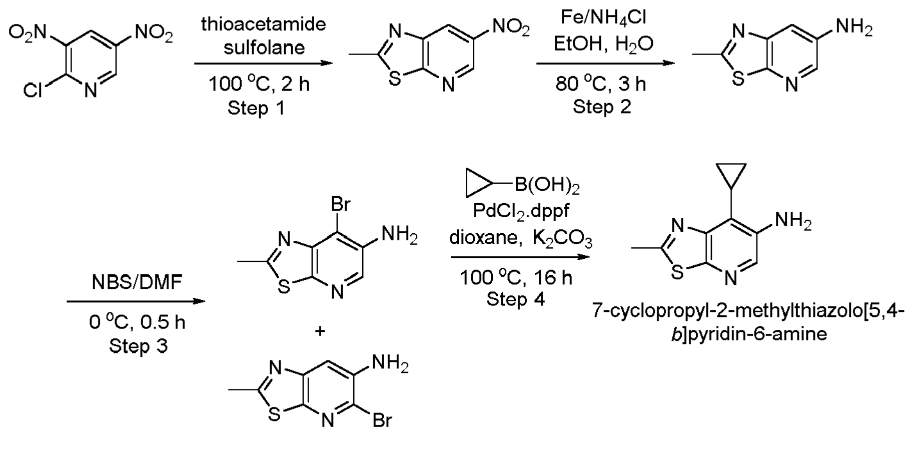


7-cyclopropyl-2-methylthiazolo[5,4-*b*]pyridin-6-amine

Step-1: 2-Methyl-6-nitrothiazolo[5,4-b]pyridine: A mixture of 2-chloro-3,5-dinitropyridine (40 g, 197 mmol) and thioacetamide (59 g, 786 mmol) in Sulfolane (500 mL) was heated at 100°C under nitrogen atmosphere for 2 h. The reaction mixture was cooled to room temperature and diluted with water (500 mL) followed by ethyl acetate (500 mL). The resulting layers were separated and the organic layer was washed several times with water. The organic layer was washed with brine (300 mL), dried ($Na_2SO_4$) and filtered. The filtrate was concentrated under vacuum and the crude product was purified by flash column chromatography (silica gel, 10% EtOAc in hexanes as eluent) to afford (8.0 g, 21%) of the titled compound as off white solid. $^1$HNMR (400 MHz, DMSO-$d_6$) δ 9.39 (d, *J* = 2.5 *Hz,* 1H), 9.07 (d, *J* = 2.5 *Hz,* 1H), 2.93 (s, 3H); ESI-MS (m/z) 195.88 $(MH)^+$.

Step-2: 2-Methylthiazolo[5,4-b]pyridin-6-amine: To a stirred solution of step-1 intermediate (8.0 g, 41.0 mmol) in ethanol (100 mL) and water (20 mL) was added ammonium chloride (21.9 g, 410 mmol) followed by iron powder (6.87 g, 123 mmol). The reaction mixture was stirred at room temperature for 15 min and then at 80°C for 3 h. The reaction mixture was cooled to room temperature and filtered through celite. The celite bed was thoroughly washed with DCM (100 mL). Water (75 mL) was added to the filtrate and the resulting layers were separated. The aqueous layer was extracted with DCM (2×100 mL) and the combined organic layers were washed with brine (75 mL), dried ($Na_2SO_4$) and filtered. The filtrate was rotary evaporated and the crude product was purified by flash column chromatography (silica gel, 2% methanol in DCM as eluent) to afford (6.0 g, 90%) of the titled compound as off white solid. $^1$HNMR (400 MHz, DMSO-$d_6$) δ 7.99 (d, *J* = 2.5 *Hz,* 1H), 7.36 (d, *J* = 2.5 *Hz,* 1H), 5.53 (s, 2H, $D_2O$ exchangeable), 2.61 (s, 3H); ESI-MS (m/z) 165.95 $(MH)^+$.

Step-3: 7-Bromo-2-methylthiazolo[5,4-b]pyridin-6-amine: To a (0°C) cooled and stirred solution of step-2 intermediate (6.0 g, 36.3 mmol) in DMF (25 mL) was added dropwise a solution of NBS (6.46 g, 36.3 mmol) in DMF (15mL). After stirring for 30 min at the same temperature, water (50 mL) was added to the reaction followed by ethyl acetate (100 mL). The layers were separated and aqueous layer was extracted with ethyl acetate (2×100 mL). The combined organic layers were washed with brine (50 mL), dried ($Na_2SO_4$) and filtered. The filtrate was rotary evaporated and the crude product was purified by flash column chromatography (silica gel, 20% ethyl acetate in hexane as eluent) followed by trituration with ethyl acetate to afford (800 mg, 9%) of the titled compound as white solid along with 3.5 g (39%) of the other isomer 5-bromo-2-methylthiazolo[5,4-b]pyridin-6-amine. $^1$HNMR (400 MHz, DMSO-$d_6$) δ 8.08 (s, 1H), 5.77 (s, 2H, $D_2O$ exchangeable), 2.78 (s, 3H). ESI-MS (m/z) 243.95$(MH)^+$.

Step-4: 7-Cyclopropyl-2-methylthiazolo[5,4-b]pyridin-6-amine: In a sealed tube containing a 1,4-Dioxane (10mL) and potassium carbonate (226 mg, 1.64 mmol) was purged nitrogen gas for 30 min and step-3 intermediate (200 mg, 0.82 mmol), cyclopropylboronic acid (282 mg, 3.28 mmol) and $PdCl_2$(dppf)-$CH_2Cl_2$ adduct (67mg, 0.082 mmol) were sequentially added. The sealed tube was capped and stirred at 110°C for 16 h. The reaction mixture was cooled to room temperature and filtered through celite. The celite cake was washed with ethyl acetate (30 mL) and the combined filtrates were rotary evaporated. The crude product was purified by column chromatography (silica gel, 2% methanol in DCM as eluent) to afford (100 mg, 60%) of the titled compound as white solid. $^1$HNMR (400 MHz, DMSO-$d_6$) δ 7.98 (s, 1H), 5.32 (s, 2H), 2.71 (s, 3H), 1.92-1.80 (m, 1H), 1.29-1.16 (m, 2H), 1.07- 0.97 (m, 2H); ESI-MS (m/z) 206.7 $(MH)^+$.

**Example-2:** Preparation of 7-Cyclopropyl-2-methylthiazolo[5,4-b]pyridin-6-amine

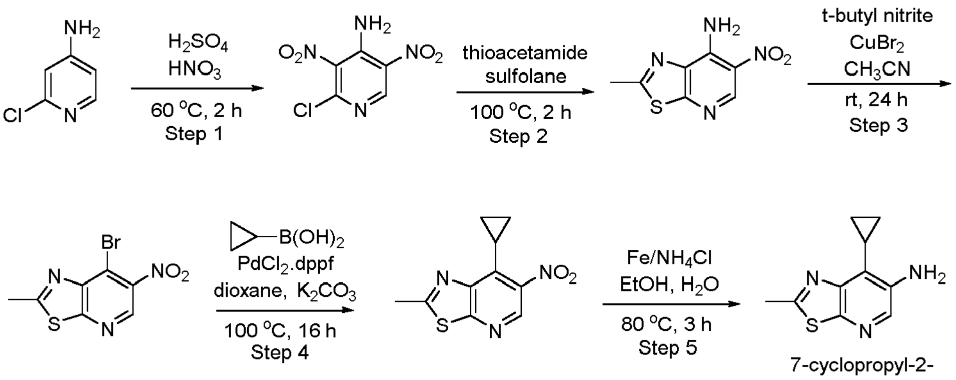


7-cyclopropyl-2-methylthiazolo[5,4-*b*]pyridin-6-amine

Step-1: 2-Chloro-3,5-dinitropyridin-4-amine: To a (0°C) cooled and stirred solution of 2-chloropyridin-4-amine (20 g, 163 mmol) in conc. $H_2SO_4$ (400 mL) was added portionwise potassium nitrate (66.1 g, 653 mmol). The resulting mixture was stirred at 0°C for 30 min and then at room temperature for 30 min. The reaction mixture was further heated to 60°C and then stirred for 2h. The reaction mixture was cooled to room temperature and poured onto crushed ice. The solid obtained was filtered and purified by flash column chromatography (silica gel, 25% ethyl acetate in hexane as eluent) to afford (24.0 g, 68%) of the titled compound as pale yellow solid. $^1$HNMR (400 MHz, DMSO-$d_6$) $\delta$ 8.99 (s, 1H), 8.54 (s, 2H, $D_2O$ exchangeable); ESI-MS (m/z) 218.79 $(MH)^+$.

Step-2: 2-Methyl-6-nitrothiazolo[5,4-b]pyridin-7-amine: The mixture of step-1 intermediate (24.0 g, 110 mmol) and thioacetamide (33.0 g, 439 mmol) in sulfolane (150 mL) was stirred at 100°C for 3 h. The reaction mixture was cooled to room temperature and cold water was added to the mixture. The solid obtained was filtered and washed with 10% ethyl acetate in hexane to afford (12.0 g, 52%) of the titled compound as yellow solid. $^1$HNMR (400 MHz, DMSO-$d_6$) $\delta$ 9.00 (s, 1H), 8.37 (brs, 2H, $D_2O$ exchangeable), 2.83 (s, 3H); ESI-MS (m/z) 211.64 $(MH)^+$.

Step-3: 7-Bromo-2-methyl-6-nitrothiazolo[5,4-b]pyridine: To a (0°C) cooled and stirred suspension of tert-butyl nitrite (13.58 mL, 114 mmol) and copper(II) bromide (25.5 g, 114 mmol) in acetonitrile (500 mL) was added dropwise step-2 intermediate (12.0 g, 57.1 mmol) in acetontrile (50 mL). The reaction mixture was stirred at 0°C for 15 min and warmed to room temperature and then stirred for 24 h. The reaction mixture was cooled to 0°C and water (100 mL) was added followed by ethyl acetate (100 mL). The resulting layers were separated and the aqueous layer was extracted with ethyl acetate (2×150 mL). The combined organic layers were washed with brine (100 mL), dried ($Na_2SO_4$) and filtered. The filtrate was rotary evaporated and the crude product was purified by flash column chromatography (silica gel, 10% ethyl acetate in hexane as eluent) to afford (6.0 g, 38%) of the titled compound as off white solid. $^1$HNMR (400 MHz, DMSO-$d_6$) $\delta$ 9.15 (s, 1H), 2.94 (s, 3H); ESI-MS (m/z) 274, 276 [$(MH)^+$, $Br^{79,81}$].

Step-4: 7-Cyclopropyl-2-methyl-6-nitrothiazolo[5,4-b]pyridine: To a nitrogen purged suspension of 1,4-dioxane (10 mL) and potassium carbonate (605 mg, 4.38mmol) was added step-3 intermediate (600 mg, 2.18 mmol), cyclopropylboronic acid (752 mg, 8.76 mmol) and $PdCl_2(dppf)$-$CH_2Cl_2$ adduct (179 mg, 0.22 mmol) sequentially. The sealed tube was capped and stirred at 100°C for 6 h. The reaction mixture was cooled to room temperature; water (20 mL) was added followed by ethyl acetate (30 mL). The resulting layers were separated and aqueous layer was extracted with ethyl acetate (2×25 mL). The combined organic layers were washed with saturated aqueous $NaHCO_3$ solution (20 mL), dried ($Na_2SO_4$) and filtered. The filtrate was rotary evaporated and the crude product was purified by flash column chromatography (silica gel, 20% ethyl acetate in hexane as eluent) to afford (410 mg, 80%) of the titled compound as white solid. $^1$HNMR (400 MHz, DMSO-$d_6$) $\delta$ 8.97 (s, 1H), 2.87 (s, 3H), 2.43-2.34 (m, 1H), 1.68-1.61 (m, 2H), 1.28-1.20 (m, 2H); ESI-MS (m/z) 236.08 $(MH)^+$.

Step-5: 7-Cyclopropyl-2-methylthiazolo[5,4-b]pyridin-6-amine: To a stirred solution of step-4 intermediate (400 mg, 1.70 mmol) in ethanol (10 mL) and water (2 mL) was added ammonium chloride (1.18 g, 22.10 mmol) followed by iron powder (1.24 g, 22.10 mmol). The reaction mixture was refluxed for 1 h. The reaction was cooled to room temperature and filtered through celite. The celite bead was washed with EtOAc (50 mL). The filterate obtained was rotary evaporated and the residue was taken in EtOAc (50mL) and water (30 mL). The layers were separated and the aqueous layer was extracted with ethyl acetate (2×50 mL). The combined organic layers were washed with brine (30 mL), dried ($Na_2SO_4$) and filtered. The filtrate was rotary evaporated and the crude product was purified by flash column chromatography (silica gel, 40% ethyl acetate in hexane as eluent) to afford (310 mg, 89%) of the titled compound as solid. $^1$HNMR (400 MHz, DMSO-$d_6$) $\delta$ 7.98 (s, 1H), 5.32 (s, 2H, $D_2O$ exchangeable), 2.72 (s, 3H), 1.94-1.80 (m, 1H), 1.29-1.18 (m, 2H), 1.08-0.98 (m, 2H); ESI-MS (m/z) 206.7 $(MH)^+$.

**Example-3:** The following compounds were prepared by using the procedure described under Example 1 or Example 2:

7-(4-Fluoro-2-methoxyphenyl)-2-methylthiazolo [5,4-b]pyridin-6-amine, ESI-MS (m/z) 289.34 $(M)^+$;

7-(2-Fluoropyridin-3-yl)-2-methylthiazolo [5,4-b]pyridin-6-amine, ESI-MS (m/z) 261.11 $(MH)^+$;

7-(3-Fluoropyridin-4-yl)-2-methylthiazolo [5,4-b]pyridin-6-amine, ESI-MS (m/z) 261.11 $(MH)^+$;

7-ethyl-2-methylthiazolo[5,4-b]pyridin-6-amine, ESI-MS (m/z) 193.82 $(MH)^+$;

7-Isopropyl-2-methylthiazolo[5,4-b]pyridin-6-amine; ESI-MS (m/z) 208.10 $(MH)^+$;

2,7-Dimethylthiazolo[5,4-b]pyridin-6-amine, ESI-MS (m/z) 180.07 $(MH)^+$;

7-Cyclopropyl-2-ethylthiazolo[5,4-b]pyridin-6-amine, ESI-MS (m/z) 220.1 $(MH)^+$; and

7-cyclopropylthiazolo[5,4-b]pyridin-6-amine, ESI-MS (m/z) 191.82 $(MH)^+$.

**Example-4:** Preparation of 7-(3,6-dihydro-2H-pyran-4-yl)-2-methylthiazolo[5,4-b]pyridin-6-amine

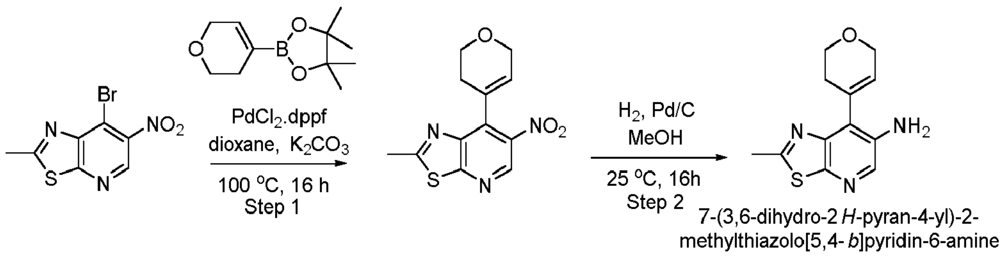

7-(3,6-dihydro-2*H*-pyran-4-yl)-2-methylthiazolo[5,4-*b*]pyridin-6-amine

Step-1: 7-(3,6-dihydro-2H-pyran-4-yl)-2-methyl-6-nitrothiazolo[5,4-b]pyridine: To a nitrogen purged suspension of 1,4-dioxane (20 mL) and potassium carbonate (1.286g, 9.30mmol) was added 7-bromo-2-methyl-6-nitrothiazolo[5,4-b]pyridine (850 mg, 3.10 mmol), 2-(3,6-dihydro-2H-pyran-4-yl)-4,4,5,5-tetramethyl-1,3,2-dioxaborolane (782 mg, 3.72 mmol) and $PdCl_2(dppf)-CH_2Cl_2$ adduct (227 mg, 0.31 mmol) sequentially. The sealed tube was capped and stirred at 100°C for 16 h. The reaction mixture was cooled to room temperature; water (20 mL) was added followed by ethyl acetate (30 mL). The resulting layers were separated and the aqueous layer was extracted with ethyl acetate (2×25 mL). The combined organic layers were washed with saturated aqueous $NaHCO_3$ solution (20 mL), dried over $Na_2SO_4$ and filtered. The filtrate was rotary evaporated and the crude product was purified by flash column chromatography (silica gel, 10% ethyl acetate in hexane as eluent) to afford (350 mg, 40%) of the titled compound as white solid. $^1$HNMR (400 MHz, DMSO-$d_6$) δ 9.14 (s, 1H), 5.88 (s, 1H), 4.20 (s, 2H), 3.95 (s, 2H), 3.89 (s, 2H), 2.91 (s, 3H).; ESI-MS (m/z) 278.03 $(MH)^+$.

Step-2: 7-(3,6-dihydro-2H-pyran-4-yl)-2-methylthiazolo[5,4-b]pyridin-6-amine: To a stirred solution of step-1 intermediate (120 mg, 1.70 mmol) in methanol (10 mL) was added 10% Pd/C (200 mg, 0.188 mmol). The reaction mixture was stirred under hydrogen atmosphere for 16 h. The reaction mixture was filtered through celite. The celite pad was washed with EtOAc (50 mL). The filtrate obtained was rotary evaporated and the residue was taken forward without purification (105 mg, 98%) of the titled compound as solid. $^1$HNMR (400 MHz, DMSO-$d_6$) δ 8.07 (s, 1H), 5.87-5.83 (m, 1H), 5.21 (s, 2H), 4.32-4.22 (m, 2H), 4.85-4.95 (m, 2H), 2.73 (s, 3H), 2.45-2.38 (m, 2H); ESI-MS (m/z) 247.98 $(MH)^+$.

**Example-5:** The following compound was prepared by using the similar procedure described in Example-4: 7-(cyclohex-1-en-1-yl)-2-methylthiazolo[5,4-b]pyridin-6-amine; ESI-MS (m/z) 246.58 $(MH)^+$.

**Example-6:** Preparation of 7-cyclopropyl-2-trifluoromethylthiazolo[5,4-b]pyridin-6-amine

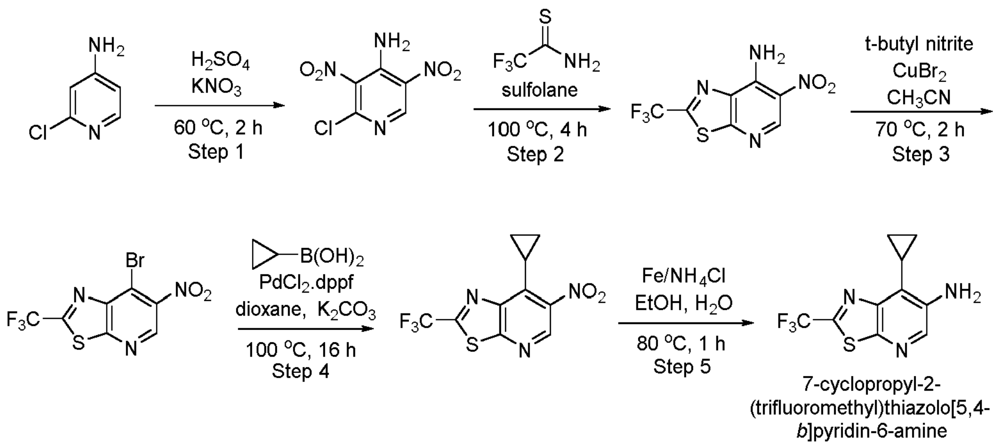


Step-1: 2-Chloro-3,5-dinitropyridin-4-amine: The titled compound was prepared by following the procedure described in step-1 of Example-2.

Step-2: 2-trifluoromethyl-6-nitrothiazolo[5,4-b]pyridin-7-amine: A mixture of step-1 intermediate (150 mg, 0.686 mmol) and 2,2,2-trifluoroethanethioamide (354 mg, 2.75 mmol) in sulfolane (3 mL) was stirred at 100°C for 4 h. The reaction mixture was cooled to room temperature and cold water (5 mL) was added to the mixture followed by ethyl acetate (5 mL). The resulting layers were separated and the aqueous layer was extracted with ethyl acetate (2×10 mL). The combined organic layers were washed with water (3×10 mL), aqueous saturated sodium bicarbonate solution (10 mL), brine (10 mL) and dried ($Na_2SO_4$) and filtered. The filtrate was rotary evaporated and the crude product was purified by flash column chromatography (silica gel, 5% EtOAc in hexanes as eluent) to afford (35 mg, 25%) of the titled compound as white solid. $^1$HNMR (400 MHz, DMSO-$d_6$) δ 9.19 (s, 1H), 9.05 (s, 1H), 8.64 (s, 1H); LC-MS (m/z), 264.7 [(MH)$^+$].

Step-3: 7-Bromo-2-trifluoromethyl-6-nitrothiazolo[5,4-b]pyridine: A (0°C) cooled and stirred suspension of tert-butyl nitrite (0.72 mL, 6.06 mmol) and copper(II) bromide (2.71 g, 12.1 mmol) in acetonitrile (20 mL) was heated for 5 min at 70 ◦C. A solution of step-2 intermediate (1.60 g, 6.06 mmol) in acetontrile (10 mL) was added to the above mixture and the reaction was continued to stir at the same temperature for 2 h. The reaction mixture was cooled to room temperature and water (20 mL) was added followed by ethyl acetate (60 mL). The resulting layers were separated and the aqueous layer was extracted with ethyl acetate (2×50 mL). The combined organic layers were washed with brine (50 mL), dried ($Na_2SO_4$) and filtered. The filtrate was rotary evaporated and the crude product was purified by flash column chromatography (silica gel, 2-3% ethyl acetate in hexane as eluent) to afford (1.0 g, 50%) of the titled compound as off white solid. $^1$HNMR (400 MHz, DMSO-$d_6$) δ 9.42 (s, 1H); ESI-MS (m/z) 327.88 (MH)$^+$.

Step-4: 7-Cyclopropyl-2-trifluoromethyl-6-nitrothiazolo[5,4-b]pyridine: To a nitrogen purged suspension of 1,4-dioxane (20 mL) and potassium carbonate (1.18 g, 8.53 mmol) was added step-3 intermediate (1.0 g, 3.05 mmol), cyclopropylboronic acid (1.05 g, 12.19 mmol) and $PdCl_2$(dppf)-$CH_2Cl_2$ adduct (250 mg, 0.305 mmol) sequentially. The sealed tube was capped and stirred at 100°C for 16 h. The reaction mixture was cooled to room temperature; water (20 mL) was added followed by ethyl acetate (30 mL). The layers were separated and aqueous layer was extracted with ethyl acetate (2×25 mL). The combined organic layers were washed with saturated aqueous $NaHCO_3$ solution (20 mL), dried ($Na_2SO_4$) and filtered. The filtrate was rotary evaporated and the crude product was purified by flash column chromatography (silica gel, 20% ethyl acetate in hexane as eluent) to afford (500 mg, 57%) of the titled compound as white solid. $^1$HNMR (400 MHz, DMSO-$d_6$) δ 9.25 (s, 1H), 2.48-2.46 (m, 1H), 1.64-1.62 (m, 2H), 1.36-1.34 (m, 2H); ESI-MS (m/z) 289.8 (MH)$^+$.

Step-5: 7-Cyclopropyl-2-methylthiazolo[5,4-b]pyridin-6-amine: To a stirred solution of step-4 intermediate (500 mg, 1.73 mmol) in ethanol (10 mL) and water (2 mL) was added ammonium chloride (370 g, 6.91 mmol) followed by iron powder (386 mg, 6.91 mmol). The reaction mixture was refluxed for 1 h. The reaction mixture was cooled to room temperature and filtered through celite. The celite bed was washed with EtOAc (50 mL). The filterate obtained was rotary evaporated and the residue was taken in EtOAc (50mL) and water (30 mL). The layers were separated and the aqueous layer was extracted with ethyl acetate (2×50 mL). The combined organic layers were washed with brine (30 mL), dried ($Na_2SO_4$) and filtered. The filtrate was rotary evaporated to afford (350 mg, 78%) of the titled compound as solid. The crude product was used as such for next step without further purification. $^1$HNMR (400 MHz, DMSO-$d_6$) δ 8.26 (s, 1H), 5.75 (s, 2H), 1.96-1.94 (m, 1H), 1.20-1.18 (m, 2H), 1.14-1.12 (m, 2H); ESI-MS (m/z) 259.7 (MH)$^+$.

**Example-7:** Preparation of 1-(6-Amino-2-methylthiazolo[5,4-b]pyridin-7-yl)ethan-1-one

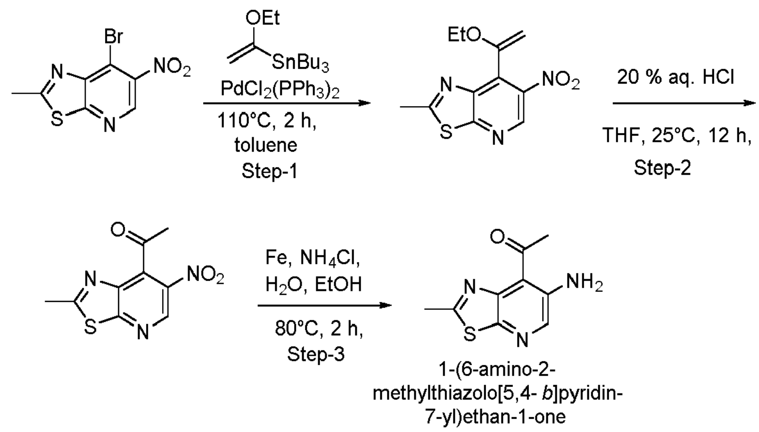


1-(6-amino-2-methylthiazolo[5,4- *b*]pyridin-7-yl)ethan-1-one

Step-1: 7-(1-Ethoxyvinyl)-2-methyl-6-nitrothiazolo [5, 4-b] pyridine: To a stirred solution of 7-bromo-2-methyl-6-nitrothiazolo[5,4-b]pyridine (5 g, 18.24 mmol) in toluene (60 mL), was added 1-ethoxyvinyltri-n-butyltin (12.43 mL, 36.5 mmol) and dichlorobis(triphenylphosphine)palladium(II) (1.280 g, 1.824 mmol) under nitrogen. Reaction mixture was heated at 110°C for 2 h. Upon completion, reaction mixture was filtered through celite bed, washed with ethyl acetate (200 mL) and concentrated to afford 6.5 g of the titled crude product which was used in next step without further purification. ESI-MS (m/z) 266.21 $(MH)^+$.

Step-2: 1-(2-Methyl-6-nitrothiazolo[5,4-b]pyridin-7-yl)ethan-1-one: To a solution of 7-(l-ethoxyvinyl)-2-methyl-6-nitrothiazolo[5,4-b]pyridine (6.5 g) in THF (50 mL) was added dropwise aq. HCl (20%) (50 mL) at 0°C and the reaction was stirred under nitrogen for 12 h at 25°C. The reaction mixture was concentrated under reduced pressure and was diluted with water (200 mL), sat. $NaHCO_3$ (200 mL) followed by extraction with ethyl acetate (100 mL x 4). The combined organic phase was dried over anhydrous sodium sulphate and rotary evaporated to afford 1-(2-methyl-6-nitrothiazolo[5,4-b]pyridin-7-yl)ethanone (3 g, 69% over two steps). $^1$HNMR (400 MHz, DMSO-$d_6$) δ 9.42 (s, 1H), 2.94 (s, 3H), 2.71 (s, 3H); ESI-MS (m/z) 237.97 $(MH)^+$

Step-3: 1-(6-amino-2-methylthiazolo[5,4-b]pyridin-7-yl)ethan-1-one: To a stirred solution of 1-(2-methyl-6-nitrothiazolo[5,4-b]pyridin-7-yl) ethan-1-one (3 g, 12.65 mmol) in water (150 mL) and ethanol (30 ml), was added $NH_4Cl$ (5.41 g, 101 mmol) and iron powder (3.53 g, 63.2 mmol). The reaction mixture was heated while stirring to 80°C for 2 h. The progress of the reaction was monitored by TLC. Upon completion of the reaction, the reaction mixture was filtered through celite bed and washed with 10% methanol in DCM (200 mL). The filtrate was rotary evaporated and residue was purified by flash column chromatography (silica gel) to afford 1.67 g (64%) of the titled product as a white solid. $^1$HNMR (400 MHz, DMSO-$d_6$) δ 8.27 (s, 1H), 7.33 (s, 2H), 2.89 (s, 3H), 2.81 (s, 3H). ESI-MS (m/z) 207.96 $(MH)^+$.

**Example-8:** Preparation of 7-(2-methoxypropan-2-yl)-2-methylthiazolo[5,4-b]pyridin-6-amine

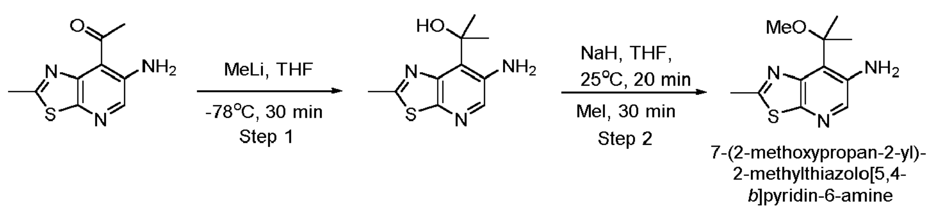


7-(2-methoxypropan-2-yl)-2-methylthiazolo[5,4-*b*]pyridin-6-amine

Step-1: 2-(6-Amino-2-methylthiazolo[5,4-b]pyridin-7-yl)propan-2-ol: To a stirred solution of 1-(6-amino-2-methylthiazolo[5,4-b]pyridin-7-yl)ethan-1-one (0.900 g, 4.34 mmol) in THF (20 mL) was added in $CH_3Li$ (3M solution in THF, 3.62 mL, 10.86 mmol) at -78°C. The resulting reaction mixture was stirred at -78°C for 30 min. Upon completion, the reaction mixture was quenched with saturated ammonium chloride solution (25 mL) and the aqueous phase was extracted with dichloromethane (50 mL x 3). The combined organic layer was dried over anhydrous sodium sulphate and filtered. The filtrate was rotary evaporated and the residue was purified by flash column chromatography (silica gel) to afford 0.800 g (83%) of the titled product as a colorless gum. $^1$H NMR (400 MHz, DMSO-$d_6$) δ 7.95 (s, 1H), 5.98 (s, exchangeable with $D_2O$, 2H), 5.76 (s, exchangeable with $D_2O$, 1H), 2.73 (s, 3H), 1.77 (s, 6H).

Step-2: 7-(2-Methoxypropan-2-yl)-2-methylthiazolo[5,4-b]pyridin-6-amine: To a solution of step-1 intermediate (800 mg, 3.58 mmol) in THF (10 mL) was added sodium hydride (60% in mineral oil, 358 mg, 8.96 mmol) at 0°C and

the mixture was stirred for 20 min at 25°C. To the reaction mixture $CH_3I$ (763 mg, 5.37 mmol) was added and the reaction mixture was stirred for 30 min. The reaction mixture was quenched with saturated ammonium chloride solution (25 mL) and the aqueous phase was extracted with dichloromethane (50 mL x 3), and the combined organic layer was dried over anhydrous sodium sulphate and filtered. The filtrate was rotary evaporated and the residue was purified by flash column chromatography (silica gel) to afford 0.500 g (59%) of the titled product as a colorless gum. $^1$HNMR (400 MHz, DMSO-$d_6$) δ 8.04 (s, 1H), 5.76 (s, 2H), 3.11 (s, 3H), 2.73 (s, 3H), 1.78 (s, 6H); ESI-MS (m/z) 237.9 $(MH)^+$.

**Example-9:** Preparation of (±)-7-(1-cyclopropyl-1-methoxyethyl)-2-methylthiazolo[5,4-b]pyridin-6-amine

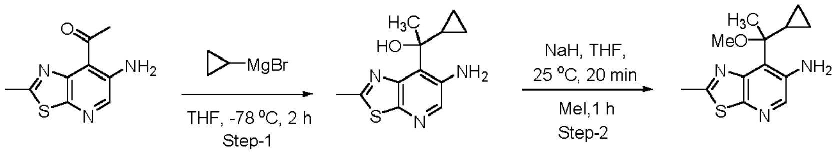


Step-1: 1-(6-Amino-2-methylthiazolo[5,4-b]pyridin-7-yl)-1-cyclopropylethan-1-ol: To a solution of 1-(6-amino-2-methylthiazolo[5,4-b]pyridin-7-yl)ethan-1-one (900 mg, 4.34 mmol) in THF (10 mL) was added cyclopropylmagnesium bromide (0.7 M in THF 6.20 mL, 4.34 mmol) at -78°C and the mixture was stirred for -78°C for 2 h. After completion of the reaction, the reaction mixture was quenched with saturated ammonium chloride (25 mL) and the aqueous phase was extracted with dichloromethane (50 mL x 3). The combined organic layer was dried over anhydrous sodium sulphate and filtered. The filtrate was rotary evaporated and the residue was purified by flash column chromatography (silica gel) to afford 0.500 g (46%) of the titled product as a colorless gum. $^1$HNMR (400 MHz, Chloroform-*d*) δ 8.16 (s, 1H), 2.80 (s, 3H), 1.71 (s, 3H), 1.70-1.64 (m, 1H), 0.82-0.73 (m, 1H), 0.70-0.44 (m, 3H); ESI-MS (m/z) 249.9 $(MH)^+$.

Step-2: 7-(1-Cyclopropyl-1-methoxyethyl)-2-methylthiazolo[5,4-b]pyridin-6-amine: To a solution of step-1 intermediate (400 mg, 1.604 mmol) in THF (10 mL) was added NaH (60% in mineral oil, 160 mg, 4.01 mmol) at 0°C and the reaction mixture was stirred for 25°C for 30 min. MeI (0.12 mL, 1.925 mmol) was added and the reaction mixture was stirred for 1h. After completion of the reaction, the reaction mixture was quenched with sat. ammonium chloride (25 mL) and the resulting aqueous phase was extracted with dichloromethane (50 mL x 3). The combined organic layer was dried over anhydrous sodium sulphate and filtered. The filtrate was rotary evaporated and residue was purified by flash column chromatography (silica gel) to afford 0.250 g (59%) of the titled product as a colorless gum. $^1$H NMR (400 MHz, DMSO-$d_6$) δ 8.01 (s, 1H), 5.80 (s, 2H), 3.16 (s, 3H), 2.72 (s, 3H), 1.85 (s, 3H), 1.50-1.40 (m, 1H), 0.44-0.25 (m, 4H).

**Example-10:** Preparation of 2-methyl-7-(1,4-oxazepan-4-yl)thiazolo [5,4-b]pyridin-6-amine

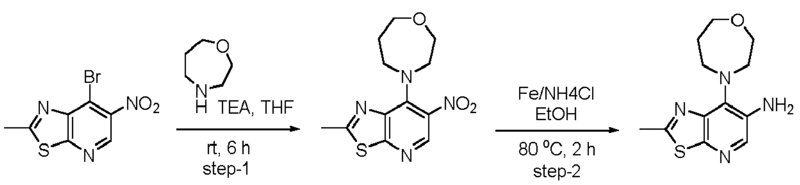


Step-1: 4-(2-Methyl-6-nitrothiazolo[5,4-b]pyridin-7-yl)-1,4-oxazepane: To a (0°C) cooled and stirred solution of 7-bromo-2-methyl-6-nitrothiazolo[5,4-b]pyridine (1 g, 3.65 mmol) in THF (15 mL) was added 1,4-oxazepane hydrochloride (0.6 g, 4.38 mmol) followed by the addition of triethylamine (1.57 mL, 10.95 mmol). After stirring the reaction at 25°C for 6 h, water (10 mL) was added, and the reaction mixture was extracted with EtOAc (2×50 mL), dried over $Na_2SO_4$, filtered, and rotary evaporated. The crude product was purified by flash column chromatography (silica gel, hexane/ethylacetate (80:20) as eluent) to afford 4-(2-methyl-6-nitrothiazolo[5,4-b]pyridin-7-yl)-1,4-oxazepane (600 mg, 56%). $^1$HNMR (400 MHz, DMSO-$d_6$) δ 8.73 (s, 1H), 3.79-3.73 (m, 4H), 3.05-2.95 (m, 4H), 2.81 (s, 3H), 2.16-1.99 (m, 2H); ESI-MS (m/z) 295.1 $(MH)^+$.

Step-2: 2-Methyl-7-(1,4-oxazepan-4-yl)thiazolo[5,4-b]pyridin-6-amine: To a stirred solution of 4-(2-methyl-6-nitrothiazolo[5,4-b]pyridin-7-yl)-1,4-oxazepane (0.55 g, 1.87 mmol) in EtOH (20 mL) was added iron powder (1.04 g, 18.69 mmol), ammonium chloride (1 g, 18.69 mmol) and $H_2O$ (2.3 mL). The reaction was heated at 80°C for 2 h. Upon completion, the reaction mixture was cooled to room temperature and filtered through celite bed, and the filtrate was rotary evaporated. Water (10 mL) was added to the residue followed by ethyl acetate (25 mL). The layers were separated

and the aqueous layer extracted with ethyl acetate (2x25 mL). The combined organic layers was washed with saturated $NaHCO_3$ (10 mL), dried over $Na_2SO_4$ and filtered. The filtrate was rotary evaporated and the crude product obtained was purified by flash column chromatography (silica gel, hexane/EtOAc (70:30) as eluent) to afford 2-methyl-7-(1,4-oxazepan-4-yl)thiazolo[5,4-b]pyridin-6-amine (450 mg, 91%). $^1$HNMR (400 MHz, DMSO-$d_6$) $\delta$ 8.04 (s, 1H), 5.03 (s, 2H), 3.86 (t, $J$ = 5.8 Hz, 2H), 3.82-3.76 (m, 2H), 3.42-3.35 (m, 4H), 2.74 (s, 3H), 2.02-1.93 (m, 2H); ESI-MS (m/z) 265.1 $(MH)^+$.

**Example-11:** The following compounds were prepared by using the similar procedure described under Example-10:

N7,N7,2-Trimethylthiazolo[5,4-b]pyridine-6,7-diamine; ESI-MS (m/z) 208.92 $(MH)^+$;

2-Methyl-7-(pyrrolidin-1-yl)thiazolo[5,4-b]pyridin-6-amine, ESI-MS (m/z) 234.94 $(MH)^+$;

7-(4,4-Difluoropiperidin-1-yl)-2-methylthiazolo[5,4-b]pyridin-6-amine, ESI-MS (m/z) 285.40 $(MH)^+$;

7-(4-methoxypiperidin-1-yl)-2-methylthiazolo[5,4-b]pyridin-6-amine, ESI-MS (m/z) 279.16 $(MH)^+$;

2-Methyl-7-morpholinothiazolo[5,4-b]pyridin-6-amine, ESI-MS (m/z) 251.12 $(MH)^+$;

N7-cyclopropyl-N7,2-dimethylthiazolo[5,4-b]pyridine-6,7-diamine, ESI-MS (m/z) 235.0 $(MH)^+$;

2-Methyl-7-(4-methylpiperidin-1-yl)thiazolo[5,4-b]pyridin-6-amine, GC-MS (m/z) 262.1$(M)^+$;

7-(2,6-Dimethylmorpholino)-2-methylthiazolo[5,4-b]pyridin-6-amine; ESI-MS (m/z) 279.65 $(MH)^+$;

2-Methyl-7-(piperidin-1-yl)thiazolo[5,4-b]pyridin-6-amine; ESI-MS (m/z) 248.93 $(MH)^+$;

7-(3-(Methoxymethyl)piperidin-1-yl)-2-methylthiazolo[5,4-b]pyridin-6-amine; ESI-MS (m/z) 293.02 $(MH)^+$;

N7-(2,3-Dimethoxypropyl)-N7,2-dimethylthiazolo[5,4-b]pyridine-6,7-diamine; ESI-MS (m/z) 297.14 $(MH)^+$;

N7-(Cyclopropylmethyl)-N7,2-dimethylthiazolo[5,4-b]pyridine-6,7-diamine; ESI-MS (m/z) 249.07 $(MH)^+$;

N7-(2-Methoxyethyl)-N7,2-dimethylthiazolo[5,4-b]pyridine-6,7-diamine; ESI-MS (m/z) 253.48 $(MH)^+$;

7-(3-Methoxypiperidin-1-yl)-2-methylthiazolo[5,4-b]pyridin-6-amine; ESI-MS (m/z) 278.97 $(MH)^+$;

N7-(1,3-Dimethoxypropan-2-yl)-N7,2-dimethylthiazolo[5,4-b]pyridine-6,7-diamine; ESI-MS (m/z) 297.14 $(MH)^+$;

N7-(1-Methoxypropan-2-yl)-N7,2-dimethylthiazolo[5,4-b]pyridine-6,7-diamine; ESI-MS (m/z) 267.08 $(MH)^+$;

N7-Cyclopropyl-N7-(2-methoxyethyl)-2-methylthiazolo[5,4-b]pyridine-6,7-diamine; ESI-MS (m/z) 279.06 $(MH)^+$;

N7-(2-Methoxypropyl)-N7,2-dimethylthiazolo[5,4-b]pyridine-6,7-diamine; ESI-MS (m/z) 267.08 $(MH)^+$; and

N7-(2-(4-fluorophenyl)-2-methoxyethyl)-N7,2-dimethylthiazolo[5,4-b]pyridine-6,7-diamine; ESI-MS (m/z) 347.15 $(MH)^+$.

**Example-12:** Preparation of 2-methyl-7-(1-methylcyclopropyl)thiazolo[5,4-b]pyridin-6-amine

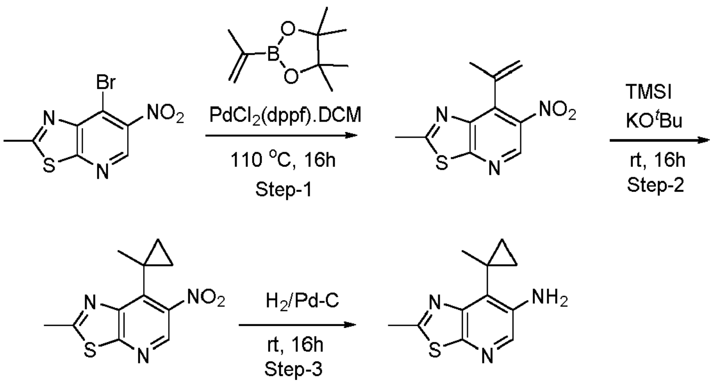


Step-1: 2-Methyl-6-nitro-7-(prop-1-en-2-yl)thiazolo[5,4-b]pyridine: In a sealed tube containing dioxane (15 mL) and potassium carbonate (1.51 g, 10.95 mmol) was purged nitrogen gas for 30 min and 7-bromo-2-methyl-6-nitrothiazolo[5,4-b]pyridine (1.50 g, 5.47 mmol), 4,4,5,5-tetramethyl-2-(prop-1-en-2-yl)-1,3,2-dioxaborolane (1.563 g, 9.30 mmol) were sequentially added. The resulting mixture was thoroughly deoxygenated by purging nitrogen and $PdCl_2$ (dppf)-$CH_2Cl_2$ adduct (0.400 g, 0.547 mmol) was added. The sealed tube was capped and heated at 110°C for 16h. The cooled reaction mixture was filtered through celite. The celite cake was washed with ethyl acetate (30 mL). The filtrate was rotary evaporated and the crude product was purified by column chromatography to afford (1.0 g, 78%) of the titled compound as white solid. $^1$HNMR (400 MHz, $CDCl_3$) $\delta$ 9.04 (s, 1H), 5.48 (s, 1H), 5.06 (s, 1H), 2.94 (s, 3H), 2.35 (s, 3H); ESI-MS (m/z) 235.9 $(MH)^+$.

Step-2: 2-Methyl-7-(1-methylcyclopropyl)-6-nitrothiazolo[5,4-b]pyridine: In a 50ml RBF containing DMSO (20 mL) and trimethyl sulfonium iodide (0.468 g, 2.125 mmol), potassium *tert*-butoxide (0.358 g, 3.19 mmol) were sequentially added. The resulting mixture was hetaed at 50°C for 1h. The reaction mass was cooled to 0-10°C, 2-methyl-6-nitro-7-(prop-1-en-2-yl)thiazolo[5,4-b]pyridine (1.0 g, 4.25 mmol) was added dropwise in DMSO (10 mL), stirred at rt for 16h. The reaction mass was diluted with saturated sodium chloride (10 mL) followed by ethyl acetate (20 mL). The layers were separated and the aqueous layer was extracted with ethyl acetate (2$\times$20 mL). The combined organic layers were washed with brine (20 mL). The organic layer was dried over anh.$Na_2SO_4$ and filtered. The filtrate was rotary evaporated and the crude product was purified by flash column chromatography (silica gel) to get (100 mg, 10%) of the desired product. $^1$HNMR (400 MHz, $CDCl_3$) $\delta$ 8.86 (s, 1H), 2.96 (s, 3H), 1.68 (s, 3H), 1.02-1.03 (m, 2H), 0.81-0.83 (m, 2H); ESI-MS (m/z) 249.9 $(MH)^+$.

Step-3: 2-Methyl-7-(1-methylcyclopropyl)thiazolo[5,4-b]pyridin-6-amine: In a 50 mL RBF containing methanol (15 mL), 2-methyl-7-(1-methylcyclopropyl)-6-nitrothiazolo[5,4-b]pyridine (100 mg, 0.401 mmol) and 10% Pd-C (42.7 mg, 0.401 mmol), were sequentially added. The resulting mixture was stirred under hydrogen atmosphere at rt for 16h. The reaction mixture was filtered through celite. The celite cake was washed with ethyl acetate (100 mL). The filtrate was rotary evaporated to get (80 mg, 91%). The crude product was used as such for the next step without further purification. $^1$HNMR (400 MHz, DMSO-$d_6$) $\delta$ 8.02 (s, 1H), 5.25 (s, 2H), 2.84 (m, 3H), 1.30 (s, 3H), 0.88-0.89 (m, 2H), 0.79 - 0.80 (m, 2H); ESI-MS (m/z) 219.9 $(MH)^+$.

**Example-13:** Preparation of 7-(2-methoxyethyl)-2-methylthiazolo[5,4-b]pyridin-6-amine

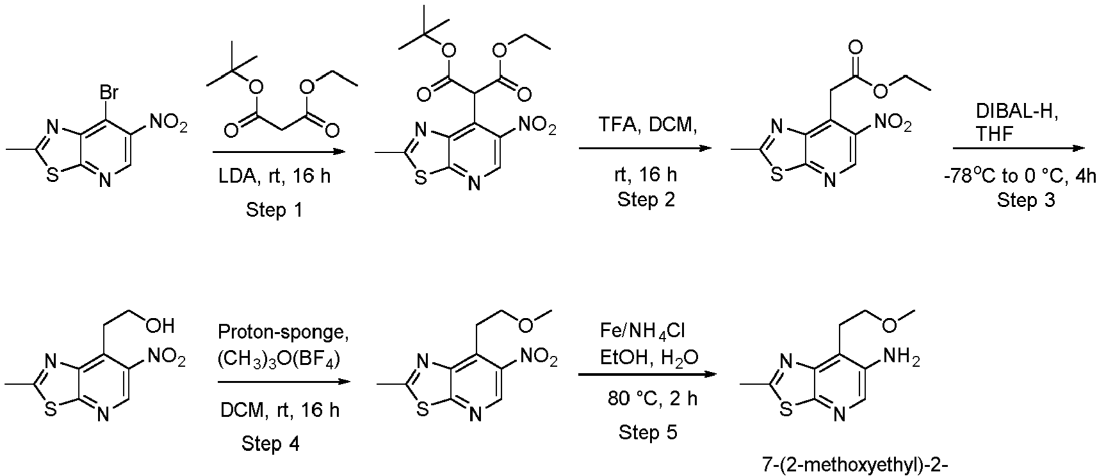

7-(2-methoxyethyl)-2-methylthiazolo[5,4-b]pyridin-6-amine

Step-1: 1-tert-Butyl 3-ethyl 2-(2-methyl-6-nitrothiazolo[5,4-b]pyridin-7-yl)malonate: To a stirred solution of 7-bromo-2-methyl-6-nitrothiazolo[5,4-b]pyridine (3.0 g, 10.95 mmol) in THF (40 mL) was added LDA (8.21 mL, 16.42 mmol, 1M in THF) at 0°C followed by dropwise addition of tert-butyl ethyl malonate (3.32 mL, 17.51 mmol). The reaction mixture was stirred at 25°C for 16 h. Upon completion, reaction was quenched with saturated aqueous $NH_4Cl$ solution (25 mL) and extracted with EtOAc. Organic layer was dried over $Na_2SO_4$, filtered and concentrated. The residue was purified by flash column chromatography on silica gel using hexane/ethyl acetate (1:9) to afford 1-tert-butyl 3-ethyl 2-(2-methyl-6-nitrothiazolo[5,4-b]pyridin-7-yl)malonate (3.5 g, 84%). $^1$HNMR (400 MHz, $CDCl_3$) δ 9.28 (s, 1H), 6.02 (s, 1H), 4.28 (q, *J* = 7.7 Hz, 2H), 2.91 (s, 3H), 1.49 (s, 9H), 1.30 (t, *J* = 7.7 Hz, 3H); ESI-MS (m/z) 382.09 $(MH)^+$.

Step-2: Ethyl 2-(2-methyl-6-nitrothiazolo[5,4-b]pyridin-7-yl)acetate: To a stirred solution of 1-tert-butyl 3-ethyl 2-(2-methyl-6-nitrothiazolo[5,4-b]pyridin-7-yl)malonate (5.00 g, 13.11 mmol) in DCM (10 mL) was added TFA (5.05 mL, 65.5 mmol) at 0°C and the reaction mixture was stirred at 25°C for 16 h. Reaction was quenched, following the addition of water and the reaction mixture was extracted with DCM, washed with aqueous saturated $NaHCO_3$, dried over $Na_2SO_4$, filtered and concentrated. The crude product was purified by flash column chromatography on silica gel using hexane/ethyl acetate (1:9) to afford ethyl 2-(2-methyl-6-nitrothiazolo[5,4-b]pyridin-7-yl)acetate (3.00 g, 81%) as a solid. $^1$HNMR (400 MHz, $CDCl_3$) δ 9.30 (s, 1H), 4.70 (s, 2H), 4.23 (q, *J* = 7.1 Hz, 2H), 2.92 (s, 3H), 1.30 (t, *J* = 7.1 Hz, 3H); ESI-MS (m/z) 282.09 $(MH)^+$.

Step-3: 2-(2-Methyl-6-nitrothiazolo[5,4-b]pyridin-7-yl)ethanol: To a stirred solution of ethyl 2-(2-methyl-6-nitrothiazolo[5,4-b]pyridin-7-yl)acetate (1.0 g, 3.56 mmol) in THF (15 mL) was added DIBAL-H (1M in toluene, 7.47 mL, 7.47 mmol) at -78°C and the reaction mixture was allowed to stir at 0°C for 4 h. After complete conversion, the reaction mixture was quenched with 2N NaOH and stirred for 30 min and thereafter extracted with ethyl acetate, washed with aqueous saturated $NaHCO_3$, dried over $Na_2SO_4$, filtered and concentrated. The crude product was purified by flash column chromatography on silica gel using hexane/ethyl acetate (1:4) to afford 2-(2-methyl-6-nitrothiazolo[5,4-b]pyridin-7-yl)ethanol (0.450 g, 53%). $^1$HNMR (400 MHz, DMSO-$d_6$) δ 9.09 (s, 1H), 4.95 (t, *J* = 5.5 Hz, 1H), 3.74-3.67 (m, 2H), 3.56 (t, *J* = 6.7 Hz, 2H), 2.92 (s, 3H); ESI-MS (m/z) 239.77 $(MH)^+$.

Step-4: 7-(2-Methoxyethyl)-2-methyl-6-nitrothiazolo[5,4-b]pyridine: To a stirred solution of 2-(2-methyl-6-nitrothiazolo[5,4-b]pyridin-7-yl)ethanol (300 mg, 1.254 mmol) in DCM (20 mL) was added 1,8-bis(dimethylamino)naphthalene (0.537 g, 2.508 mmol) and trimethyloxonium tetrafluoroborate (0.139 g, 0.940 mmol) at 0°C. Reaction was allowed to stir at 25°C for 16 h. Upon completion, water was added and the reaction mixture was extracted with DCM, washed with aqueous saturated $NaHCO_3$, dried over $Na_2SO_4$, filtered and concentrated. The crude product was purified by flash column chromatography on silica gel using hexane/ethyl acetate (1:9) to afford 7-(2-methoxyethyl)-2-methyl-6-nitrothiazolo[5,4-b]pyridine (0.250 g, 79%). $^1$HNMR (400 MHz, DMSO-$d_6$) δ 9.10 (s, 1H),3.68-3.65 (m, 5H), 3.48-3.40 (m, 2H), 2.91 (s, 3H); ESI-MS (m/z) 254.14 $(MH)^+$.

Step-5: 7-(2-Methoxyethyl)-2-methylthiazolo[5,4-b]pyridin-6-amine: To a stirred solution of 7-(2-methoxyethyl)-2-methyl-6-nitrothiazolo[5,4-b]pyridine (0.250 g, 0.987 mmol) in a mixture of ethanol and water (5:1; 24 mL) was added iron (0.551 g, 9.87 mmol) and ammonium chloride (0.528 g, 9.87 mmol). The reaction was stirred at 80°C for 2 h. The reaction mixture was filtered over celite pad and the filtrate was concentrated. The resulting residue was purified by flash column chromatography on silica gel using hexane/ethyl acetate (1:4) to provide 7-(2-methoxyethyl)-2-methylthiazolo[5,4-b]pyridin-6-amine (0.200g, 91%). $^1$HNMR (400 MHz, DMSO-$d_6$) δ 8.00 (s, 1H), 5.30 (s, 2H), 3.56 (t, *J* = 7.0 Hz, 2H), 3.25 (s, 3H), 3.19 (t, *J* = 7.1 Hz, 2H), 2.74 (s, 3H); ESI-MS (m/z) 223.78 $(MH)^+$.

**Example-14:** Preparation of 7-(1-(methoxymethyl)cyclopropyl)-2-methylthiazolo[5,4-b]pyridin-6-amine

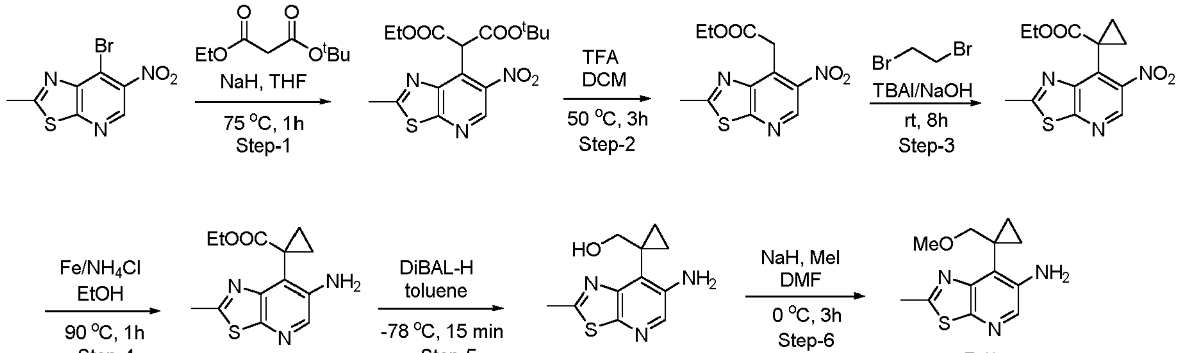


7-(1-(methoxymethyl)cyclopropyl)-2-methylthiazolo[5,4-*b*]pyridin-6-amine

Step-1: 1-tert-Butyl 3-ethyl 2-(2-methyl-6-nitrothiazolo[5,4-b]pyridin-7-yl)malonate: To a (0°C) cooled suspension of sodium hydride (0.864 g, 21.60 mmol) in THF (10 mL) was added dropwise a solution of *tert*-butyl ethyl malonate (4.09 mL, 21.60 mmol). The reaction mixture was stirred at RT for 30 min and brought back to 0°C before the addition of 7-bromo-2-methyl-6-nitrothiazolo[5,4-b]pyridine (3.70 g, 13.50 mmol) portionwise for 10 min. The reaction mixture was then stirred at RT for 1h followed by heating at 75°C for 1.0 h. The reaction was then cooled to 0°C, quenched with 5% HCl (10 mL), diluted with ethyl acetate (40 mL) and water (40 mL) was added. The layers were separated and the aqueous layer was extracted with ethyl acetate (2×40 mL) and the combined organic layers were washed with brine (70 mL), dried ($Na_2SO_4$) and filtered. The filtrate was rotary evaporated and the crude product was purified by flash column chromatography (silica gel, 10% ethyl acetate-hexane mixture as eluent) to afford 3.0 g (58%) of the titled compound as a semi-solid. $^1$HNMR (400 MHz, $CDCl_3$) δ 9.28 (s, 1H), 6.02 (s, 1H), 4.28 (q, *J* = 7.0 Hz, 2H), 2.91 (s, 3H), 1.50 (s, 9H), 1.30 (t, *J* = 7.0 Hz, 3H); ESI-MS (m/z) 382.04 $(MH)^+$.

Step-2: Ethyl 2-(2-methyl-6-nitrothiazolo[5,4-b]pyridin-7-yl)acetate: To a solution of step-1 intermediate (3.0 g, 7.87 mmol) in DCM (30 mL) was added TFA (6.06 mL, 79 mmol). The resulting mixture was stirred at 50°C for 3h. Reaction mass was cooled to RT, diluted with DCM (30 mL), basified by using saturated sodium bicarbonate solution. The layers were separated and the aqueous layer was extracted with DCM (2×40 mL) and the combined organic layers were washed with brine (70 mL), dried ($Na_2SO_4$) and filtered. The filtrate was rotary evaporated and the crude product was purified by flash column chromatography (silica gel, 20% ethyl acetate-hexane mixture as eluent) to afford 2.0 g (90%) of the titled compound as a semi solid. $^1$HNMR (400 MHz, $CDCl_3$) δ 9.30 (s, 1H), 4.70 (s, 2H), 4.22 (q, *J* = 7.0 Hz, 2H), 2.92 (s, 3H), 1.32 (t, *J* = 7.0 Hz, 3H); ESI-MS (m/z) 281.86 $(MH)^+$.

Step-3: Ethyl 1-(2-methyl-6-nitrothiazolo[5,4-b]pyridin-7-yl)cyclopropanecarboxylate: To a (0°C) cooled solution of step-2 intermediate (2.0 g, 7.11 mmol) in 1,2-dibromoethane (6.13 mL, 71.1 mmol) was added a mixture of tetrabutylammonium iodide (11.46 g, 35.6 mmol) and aqueous sodium hydroxide solution (6M, 23.70 mL, 142 mmol). The reaction mixture was stirred at 0°C for 10 min and then sturred at RT for 8h. The reaction mixture was cooled to 0°C, acidified with aqueous 10% HCl solution, diluted with ethyl acetate (40 mL), and water (30 mL) was added. The obtained layers were separated and the aqueous layer was extracted with ethyl acetate (2×30 mL) and the combined organic layers were washed with brine (70 mL), dried ($Na_2SO_4$) and filtered. The filtrate was rotary evaporated and the crude product was purified by flash column chromatography (silica gel, 15% ethyl acetate-hexane mixture as eluent) to afford 1.30 g (60%) of the titled compound as a white solid. $^1$H NMR (400 MHz, $CDCl_3$) δ 9.10 (s, 1H), 4.16 (q, *J* = 7.0 Hz, 2H), 2.93 (s, 3H), 2.02-1.90 (m, 2H), 1.37-1.25 (m, 2H), 1.16 (t, *J* = 7.0 Hz, 3H); ESI-MS (m/z) 308.21 $(MH)^+$.

Step-4: Ethyl 1-(6-amino-2-methylthiazolo[5,4-b]pyridin-yl) cyclopropanecarboxylate: To a solution of step-3 intermediate (1.20 g, 3.90 mmol) and ammonium chloride (3.90 mL, 23.43 mmol) in ethanol (10 mL) was added iron powder (1.31 g, 23.43 mmol). The resulting mixture was stirred at 90°C for 1h. The reaction was cooled to RT, diluted with ethyl acetate(30 mL), filtered through celite pad, washed with ethyl acetate (3 x 30 mL). The filtrate was rotary evaporated and the crude product was purified by flash column chromatography (silica gel, 3% methanol in DCM mixture as eluent) to afford 700 mg (65%) of the titled compound as a white solid. $^1$H NMR (400 MHz, DMSO-$d_6$) δ 8.04 (s, 1H), 5.37 (s, 2H, $D_2O$ exchangeable), 4.01 (q, *J* = 7.0 Hz, 2H), 2.74 (s, 3H), 1.75-1.71 (m, 2H), 1.25-1.20 (m, 2H), 1.07 (t, *J* = 7.0 Hz, 3H); ESI-MS (m/z) 277.91 $(M)^+$.

Step-5: (1-(6-Amino-2-methylthiazolo[5,4-b]pyridin-7-yl)cyclopropyl)methanol: To a (-78°C) cooled and stirred

solution of step-4 intermediate (550 mg, 1.98 mmol) in toluene (20 mL) was added DIBAL-H (1.0 M in toluene, 5.95 mL, 5.95 mmol) for 10 min. The resulting reaction mixture was stirred at -78°C for 15min. The reaction was quenched with saturated ammonium chloride solution (10 mL) at the same temperature, filtered through a pad of celite, washed with 10% MeOH in ethyl acetate (3 x 30mL). The filtrate was rotary evaporated and the crude product was purified by flash column chromatography (silica gel, 2% methanol in DCM as eluent) to afford 370 mg (79%) of the titled compound as a white solid. $^1$H NMR (400 MHz, DMSO-$d_6$) δ 8.01 (s, 1H), 5.26 (s, 2H, $D_2O$ exchangeable), 4.85 (s, 1H, $D_2O$ exchangeable), 3.34 (s, 2H), 2.75 (s, 3H), 1.05-1.00 (m, 2H), 0.78-0.72 (m, 2H); ESI-MS (m/z) 236.03 $(MH)^+$.

Step-6: 7-(1-(Methoxymethyl)cyclopropyl)-2-methylthiazolo[5,4-b]pyridin-6-amine: To a (0°C) cooled and stirred solution of step-5 intermediate (180 mg, 0.765 mmol) in DMF (3.0 mL) was added sodium hydride (52.0 mg, 1.30 mmol) portionwise. The resulting mixture was stirred at the same temperature for 10 min. Methyl iodide (0.081 mL, 1.30 mmol) was addded to the above mixture at 0°C and then stirred at RT for 3h. The reaction mixture was cooled to 0°C and ice water (5 mL) was added followed by ethyl acetate (10 mL). The layers were separated and aqueous layer was extracted with ethyl acetate (2×15 mL). The combined organic layers were washed with brine (40 mL), dried ($Na_2SO_4$) and filtered. The filtrate was rotary evaporated and the crude product was purified by flash column chromatography (silica gel, 30% ethyl acetate-hexane as eluent) to afford 100 mg (52%) of the titled compound as a white solid. $^1$H NMR (400 MHz, DMSO-$d_6$) δ 8.02 (s, 1H), 5.21 (s, 2H, $D_2O$ exchangeable), 3.35 (s, 2H), 3.21 (s, 3H), 2.76 (s, 3H), 1.08-1.03 (m, 2H), 0.88-0.83 (m, 2H); ESI-MS (m/z) 250.02 $(MH)^+$.

**Example-15:** Preparation of 7-(1,2-Dimethoxyethyl)-2-methylthiazolo[5,4-b]pyridin-6-amine

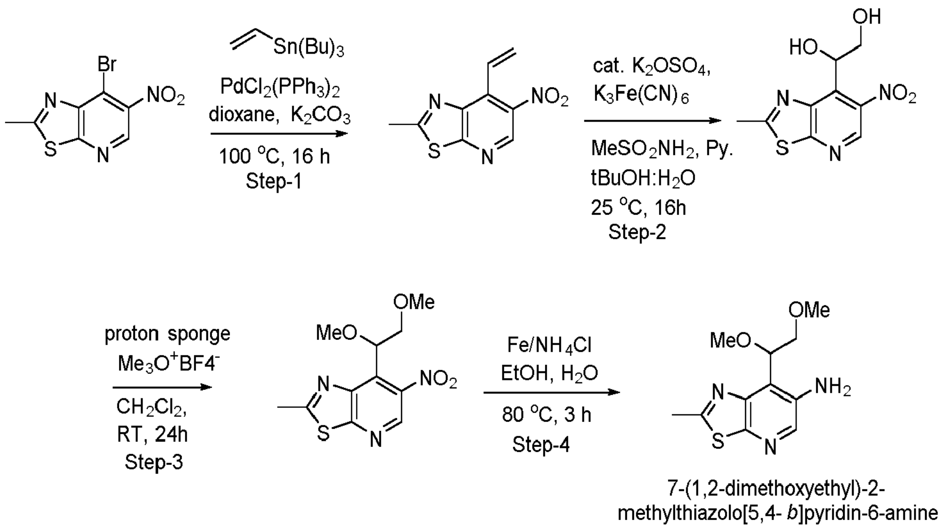


7-(1,2-dimethoxyethyl)-2-methylthiazolo[5,4- *b*]pyridin-6-amine

Step-1: 2-Methyl-6-nitro-7-vinylthiazolo[5,4-b]pyridine: To a nitrogen purged solution of 7-bromo-2-methyl-6-nitrothiazolo[5,4-b]pyridine (4.0 g, 14.59 mmol) and tributyl(vinyl)stannane (9.26 g, 29.2 mmol) in toluene (20 mL) were added potassium carbonate (1.28 g, 9.30mmol) and $PdCl_2(PPh_3)_2$ (21.0 g, 1.459 mmol) sequentially. The sealed tube was capped and stirred at 100°C for 16 h. The reaction mixture was cooled to room temperature; water (20 mL) was added followed by ethyl acetate (30 mL). The layers were separated and aqueous layer was extracted with ethyl acetate (2×25 mL). The combined organic layers were washed with saturated aqueous $NaHCO_3$ solution (20 mL), dried over $Na_2SO_4$ and filtered. The filtrate was rotary evaporated and the crude product was purified by flash column chromatography (silica gel, 10% ethyl acetate in hexane as eluent) to afford (2.0 g, 62%) of the titled compound as white solid. $^1$HNMR (400 MHz, DMSO-$d_6$) δ 9.12 (s, 1H), 7.10 (dd, *J* = 17.5, 12.1 Hz, 1H), 6.71 (d, *J* = 17.5 Hz, 1H), 6.09 (d, *J* = 11.7 Hz, 1H), 2.92 (s, 3H); ESI-MS (m/z) 221.93 $(MH)^+$.

Step-2: 1-(2-Methyl-6-nitrothiazolo[5,4-b]pyridin-7-yl)ethane-1,2-diol: To a stirred solution of 2-methyl-6-nitro-7-vinylthiazolo[5,4-b]pyridine (2.0 g, 9.04 mmol) in water (15 mL)and tert-butanol were added potassium osmate(VI) dihydrate (0.665 g, 1.808 mmol), potassium ferricyanide (8.93 g, 27.1 mmol), methanesulfonamide (0.860 g, 9.04 mmol), $K_2CO_3$ (3.75 g, 27.1 mmol) and pyridine (0.073 mL, 0.904 mmol) (200 mg, 0.188 mmol). The reaction mixture was stirred for 16 h. Reaction mixture was quenched with aq. sodium bisulphate solution (25 mL). Aqueuos phase was extracted with ethyl acetate (3 x 50 mL). The combined organic layers was dried over $Na_2SO_4$ and filtered. The filtrate was rotary evaporated to afford the crude product was purified by flash column chromatography (flash silica, 70% ethyl acetate in hexane as eluent) to afford (1.384 g, 60%) of the titled compound. $^1$HNMR (400 MHz, DMSO-$d_6$) δ 8.93 (s, 1H),

6.06-6.00(m, 1H), 5.59-5.51(m, 1H), 5.16-5.09 (m, 1H), 3.96-3.82 (m, 1H), 3.78-3.64 (m, 1H), 2.92 (s, 3H); ESI-MS (m/z) 256.17 $(MH)^+$.

Step-3: 7-(1,2-Dimethoxyethyl)-2-methyl-6-nitrothiazolo[5,4-b]pyridine: To a stirred solution of 1-(2-methyl-6-nitrothiazolo[5,4-b]pyridin-7-yl)ethane-1,2-diol (1.5 g, 5.88 mmol) in dichloromethane (6 mL) was added 1,8-bis(dimethylamino)naphthalene (4.41 g, 20.57 mmol) at 0°C followed by trimethyloxonium tetrafluoroborate (3.04 g, 20.57 mmol). The reaction mass stirred at room temperature for 24 h. Reaction mixture was diluted with dichlomethane (50 mL) and washed with aq. HCl (1N, 10 mL) followed by saturated $NaHCO_3$ solution (25 mL). The organic layers was dried over $Na_2SO_4$ and filtered. The filtrate was rotary evaporated to afford the crude product was purified by flash column chromatography (silica, 40% ethyl acetate in hexane as eluent) to afford (250 mg, 15%) of the titled compound. ESI-MS (m/z) 284.03 $(MH)^+$.

Step-4: 7-(1,2-dimethoxyethyl)-2-methylthiazolo[5,4-b]pyridin-6-amine: To a stirred solution of 7-(1,2-dimethoxyethyl)-2-methyl-6-nitrothiazolo[5,4-b]pyridine (250 mg, 0.882 mmol) in EtOH (4 mL) was added iron powder (246 mg, 4.41 mmol), ammonium chloride (378 mg, 7.06 mmol) and $H_2O$ (5 mL) at 25°C and then heated the reaction mixture at 80°C for 3 h. The reaction mixture was then cooled to room temperature and filtered through celite bed, and the filtrate was rotary evaporated. Water (5 mL) was added to the residue followed by ethyl acetate (10 mL). The layers were separated and the aqueous layer extracted with ethyl acetate (2x10 mL). The combined organic layers was washed with saturated $NaHCO_3$ (10 mL), dried ($Na_2SO_4$) and filtered. The filtrate was rotary evaporated to afford the crude product (200 mg) which was carried forward without purification. $^1$HNMR (400 MHz, DMSO-$d_6$) $\delta$ 8.03 (s, 1H), 5.44 (s, 2H), 3.84-3.74 (m, 1H), 3.67-3.53 (m, 2H), 3.27 (s, 6H), 2.74 (s, 3H).

**Example-16:** Preparation of 7-cyclopropyl-2-methylthiazolo[5,4-b]pyridine-6-carboxylic acid

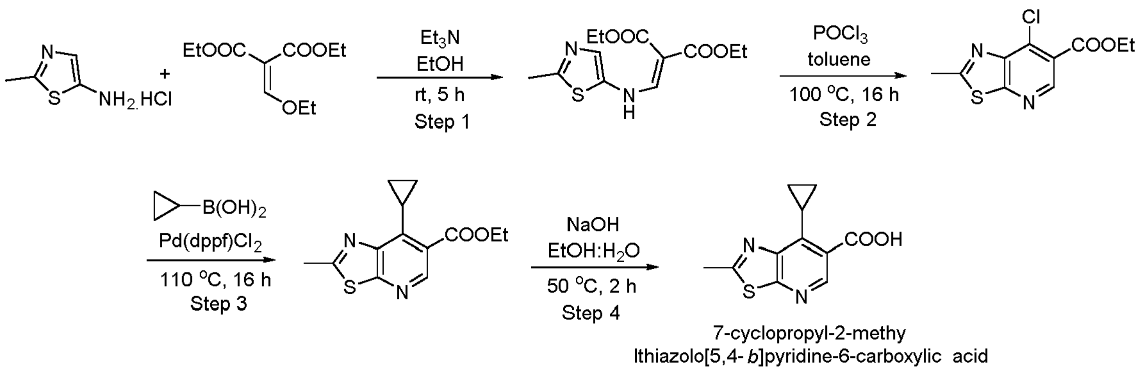


7-cyclopropyl-2-methy lthiazolo[5,4-*b*]pyridine-6-carboxylic acid

Step-1: Diethyl 2-(((2-methylthiazol-5-yl)amino)methylene)malonate: To a stirred suspension of 2-methylthiazol-5-amine hydrochloride (6.0 g, 39.8 mmol) in ethanol (30 mL)) was added triethylamine (16.6 mL, 119 mmol) followed by diethyl 2-(ethoxymethylene)malonate (7.24 mL, 35.8 mmol). The resulting mixture was stirred at room temperature for 5 h. The reaction mixture was evaporated and the residue obtained was dissolved in DCM (200 mL) and water (50 mL). The layers were separated and aqueous layer was extracted with DCM (2×50 mL). The combined organic layers were washed with brine (50 mL), dried ($Na_2SO_4$) and filtered. The filtrate was rotary evaporated and the crude product was purified by flash column chromatography (silica gel, 25% ethyl acetate in hexane as eluent) to afford 6.0 g (53%) of the titled compound as off white solid. $^1$HNMR (400 MHz, DMSO-$d_6$) $\delta$ 10.74 (d, $J$ = 13.0 *Hz,* 1H), 7.93 (d, $J$ = 13.0 *Hz,* 1H), 7.54 (s, 1H), 4.20 (q, $J$ = 7.0 *Hz,* 2H), 4.11 (q, $J$ = 7.0 *Hz,* 2H), 2.58 (s, 3H), 1.28-1.15 (m, 6H); ESI-MS (m/z) 285.13 $(MH)^+$.

Step-2: Ethyl 7-chloro-2-methylthiazolo[5,4-b]pyridine-6-carboxylate: To a stirred solution of step-1 intermediate (2.0 g, 7.03 mmol) in toluene (30 mL) was added $POCl_3$ (3.93 mL, 42.2 mmol). The resulting mixture was stirred at 100°C for 16 h. The reaction was cooled back down to room temperature and the solvent was rotary evaporated. The residue obtained was dissolved in ethyl acetate (50 mL) and poured in ice water (50 mL). The mixture was basified to pH-9 using 1M aq.NaOH solution.

The layers were separated and the aqueous layer was extracted with ethyl acetate (2×100 mL). The combined organic layers were washed with brine (30 mL), dried ($Na_2SO_4$) and filtered. The filtrate was rotary evaporated and the crude product was purified by flash column chromatography (silica gel, 20% ethyl acetate in hexane as eluent) to afford 1.0 g (55%) of the titled compound as off white solid. $^1$HNMR (400 MHz, DMSO-$d_6$) $\delta$ 8.93 (s, 1H), 4.41 (q, $J$ = 7.0 *Hz,* 2H), 2.91 (s, 3H), 1.37 (t, $J$ = 7.0 *Hz,* 3H); ESI-MS (m/z) 255.96 $(M)^+$.

Step-3: Ethyl 7-cyclopropyl-2-methylthiazolo[5,4-b]pyridine-6-carboxylate: To a nitrogen purged mixture of step-2 intermediate (300 mg, 1.17 mmol), cyclopropylboronic acid (402 mg, 4.67 mmol) and potassium carbonate(323 mg, 2.33 mmol) in dioxane (10 mL) was added $PdCl_2$(dppf)-$CH_2Cl_2$ adduct (95 mg, 0.117mmol). The resulting mixture waas stirred at 110°C for 16 h. The reaction was cooled to room temperature and water (25 mL) was added to the reaction mixture followed by ethyl acetate (30 mL). The layers were separated and the aqueous layer was extracted with ethyl acetate (2×25 mL). The combined organic layers were washed with brine (15 mL), dried ($Na_2SO_4$) and filtered. The filtrate was rotary evaporated and the crude product was purified by flash column chromatography (silica gel, 20% ethyl acetate in hexane as eluent) to afford 110 mg (36%) of the titled compound as white solid. $^1$HNMR (400 MHz, DMSO-$d_6$) δ 8.71 (s, 1H), 4.38 (q, *J* = 7.0 *Hz,* 2H), 2.82 (s, 3H), 2.77-2.71 (m, 1H), 1.72-1.66 (m, 2H), 1.36 (t, *J* = 7.0 *Hz,* 3H), 1.21-1.15 (m, 2H); ESI-MS (m/z) 262.94 $(MH)^+$.

Step-4: 7-Cyclopropyl-2-methylthiazolo[5,4-b]pyridine-6-carboxylic acid: To a (0°C) cooled and stirred solution of step-3 intermediate (110 mg, 0.42 mmol) in ethanol (5 mL) and water (1 mL) was added NaOH (34 mg, 0.84 mmol). The reaction was stirred at room temperature for 15 min and then at 50°C for 2 h. The reaction mixture was cooled to room temperature and the solvent was rotary evaporated. Water (20 mL) was added to the reaction and the pH was adjusted to 4 using 10% aq.HCl followed by addition of ethyl acetate (30 mL). The layers were separated and the aqueous layer was extracted with ethyl acetate (2×20 mL). The combined organic layers were washed with brine (20 mL), dried ($Na_2SO_4$) and filtered. The filtrate was rotary evaporated to afford 98 mg (100%) of the titled compound as white solid. $^1$HNMR (400 MHz, DMSO-$d_6$) δ 13.54 (s, 1H), 8.72 (s, 1H), 2.96-2.85 (m, 1H), 2.81 (s, 3H), 1.78-1.67 (m, 2H), 1.25-1.11 (m, 2H); ESI-MS (m/z) 234.85 $(MH)^+$.

**Example-17:** Preparation of 7-(methoxymethyl)-2-methylthiazolo[5,4-b]pyridine-6-carboxylic acid

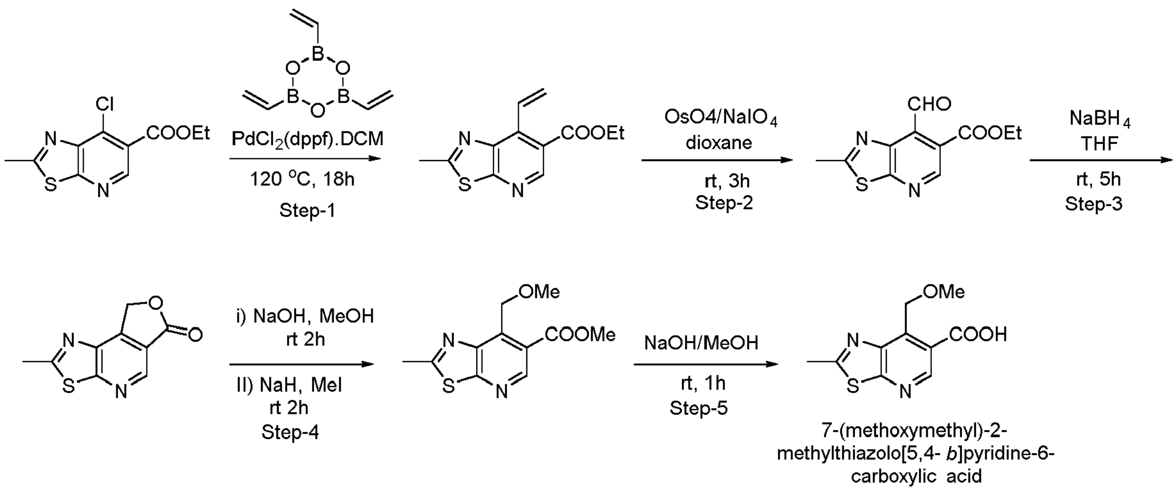

7-(methoxymethyl)-2-methylthiazolo[5,4- *b*]pyridine-6-carboxylic acid

Step-1: Ethyl-2-methyl-7-vinylthiazolo[5,4-b]pyridine-6-carboxylate: To a stirred solution of ethyl 7-chloro-2-methylthiazolo[5,4-b]pyridine-6-carboxylate (5.60 g, 21.8 mmol), 2,4,6-trivinyl-1,3,5,2,4,6-trioxatriborinane compound with pyridine (1:1) (5.25 g, 21.81 mmol) and potasssium carbonate (6 g, 43.6 mmol) in dioxane (60 mL) was purged nitrogen gas for 30 min and $PdCl_2$(dppf)-DCM adduct (1.78 g, 2.18 mmol) was added. The reaction mixture was heated at 120°C for 18 h in a sealed tube. The reaction mass was cooled to room temperature and filtered through celite. The filtrate was rotary evaporated and the crude product was purified by column chromatography (silica gel, 25% EtOAc in hexane as eluent) to afford (4.20 g, 78%) of the titled compound as a pale yellow solid. $^1$HNMR (400 MHz, $CDCl_3$) δ 9.08 (s, 1H), 7.62 (dd, *J* = 17.5 & 11.5 *Hz,* 1H), 6.73 (dd, *J* = 17.5 & 2.0 *Hz,* 1H), 6.09 (dd, *J* = 11.5 & 2.0 *Hz,* 1H), 4.56 (q, *J* = 7.0 *Hz,* 2H), 3.01 (s, 3H), 1.56 (t, *J* = 7.0 *Hz,* 3H); ESI-MS (m/z) 249.03 $(MH)^+$.

Step-2: Ethyl 7-formyl-2-methylthiazolo[5,4-b]pyridine-6-carboxylate: To a (10°C) cooled and stirred solution of step-1 intermediate (2.5 g, 10 mmol) in dioxane (250 mL), water (50 mL) was added osmium tetroxide (5.1 mL, 0.40 mmol) and sodium periodate (6.46 g, 30.2 mmol). The reaction mixture was warmed to room temperature and stirred for 3h. The reaction mixture was cooled to 0°C and water (50 mL) was added followed by ethyl acetate (100 mL). The layers were separated and the aqueous layer was extracted with ethyl acetate (2×50 mL). The combined organic layers were washed with brine (50 mL), dried ($Na_2SO_4$) and filtered. The filtrate was rotary evaporated to afford (2.1 g, 83%) of the titled compound as an off white solid. $^1$H MR (400 MHz, $CDCl_3$) δ 10.93 (s, 1H), 9.22 (s, 1H), 4.61 (q, *J* = 7.2 *Hz,* 2H), 3.05 (s, 3H), 1.57 (t, *J* = 7.2 *Hz,* 3H); ESI-MS (m/z) 250.97 $(MH)^+$.

Step-3: 2-Methylfuro[3,4-d]thiazolo[5,4-b]pyridin-6(8H)-one: To a stirred solution of step-2 intermediate (800

mg, 3.20 mmol) in THF was added $NaBH_4$ (121 mg, 3.2 mmol) portionwise at 0°C and the reaction mixture was stirred for 15 min. The reaction mixture was warmed to room temperature and stirred for 5 h. The reaction mixture was cooled to 0°C and quenched by addition of acetone (1 mL). The solvent was rotary evaporated. Water (10 mL) was added to the residue and extracted with ethyl acetate (50 mL). The layers were separated and the aqueous layer was extracted with ethyl acetate (2×10mL). The combined organic layers were washed with brine (10 mL), dried ($Na_2SO_4$) and filtered. The filtrate was rotary evaporated to afford (350 mg, 53%) the titled compound as a white solid. $^1$HNMR (400 MHz, $CDCl_3$) δ 9.22 (s, 1H), 5.83 (s, 2H), 3.08 (s, 3H); ESI-MS (m/z) 207.02 $(MH)^+$.

Step-4: Methyl 7-(methoxymethyl)-2-methylthiazolo[5,4-b]pyridine-6-carboxylate: To a (0°C) cooled and stirred solution of step-3 intermediate (400 mg, 1.94 mmol) in methanol (20 mL) was added NaOH (101 mg, 2.52 mmol) in water (2 mL). The reaction mixture was warmed to room temperature and stirred for 2h. The reaction mixture was charged with conc HCl (48 μL, 0.58 mmol) and stirred for 2 min and concentrated to dryness. The residue was azeotropped with toluene to obtain intermediate sodium 7-(hydroxymethyl)-2-methylthiazolo[5,4-b]pyridine-6-carboxylate as brownish yellow solid. The solid obtained was dissolved in DMA (10mL) and cooled to 0°C. NaH (93 mg, 2.32 mmol) was added to the reaction and the suspension obtained was stirred for 15 min. The reaction mixture was warmed to room temperature and stirred for 5 min. The reaction mixture was cooled to 0°C followed by addition of iodomethane (364 μL, 5.82 mmol). The reaction mixture was warmed to room temperature and stirred for 10 min and cooled to 0°C and quenched with saturated aqueous $NH_4Cl$ solution (2 mL). Water (10 mL) was added and reaction mixture was extracted in EtOAc (30 mL). The layers were separated and the aqueous layer was extracted with ethyl acetate (2×10 mL). The combined organic layers were washed with brine (10 mL), dried ($Na_2SO_4$) and filtered. The filtrate was rotary evaporated and the crude product was purified by column chromatography (silica gel, 10% EtOAc in hexane as eluent) to afford (200 mg, 41%) of the titled compound as white solid. ESI-MS (m/z) 253.01 $(MH)^+$.

Step-5: 7-(Methoxymethyl)-2-methylthiazolo[5,4-b]pyridine-6-carboxylic acid: To a 0°C stirred and cooled solution of step-4 intermediate (200 mg, 0.79 mmol) in MeOH (10 mL) was added NaOH (38 mg, 0.95 mmol) in water (1 mL). The reaction mixture was warmed to room temperature and stirred for 1h. The solvent was evaporated under vacuum. The residue obtained was dissolved in water (10 mL) and acidified with 10% HCl till pH-4. The suspension obtained was extracted with ethyl acetate (20 mL). The layers were separated and the aqueous layer was extracted with ethyl acetate (2×10 mL). The combined organic layers were washed with brine (5 mL), dried ($Na_2SO_4$) and filtered. The filtrate was rotary evaporated to afford (180 mg, 95%) of the titled compound as white solid. $^1$HNMR (400 MHz, DMSO-$d_6$) δ 13.50 (s, 1H, $D_2O$ exchangeable), 8.88 (s, 1H), 5.16 (s, 2H), 3.31 (s, 3H), 2.89 (s, 3H); ESI-MS (m/z) 239.05 $(MH)^+$.

**Example-18:** Preparation of (±)-7-(1-methoxy-2-methylpropyl)-2-methylthiazolo[5,4-b]pyridine-6-carboxylic acid

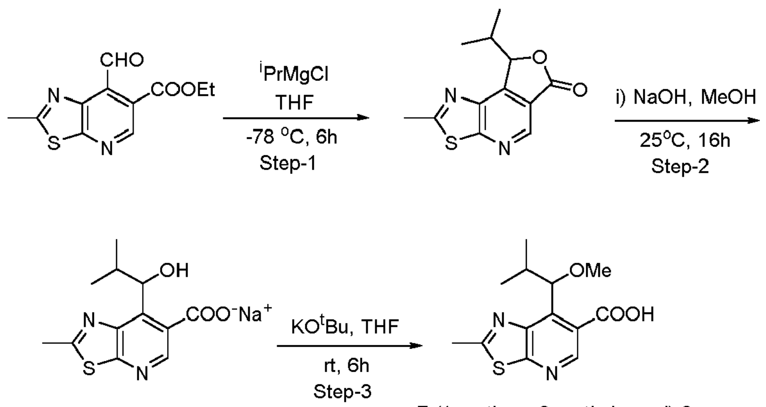


7-(1-methoxy-2-methylpropyl)-2-methylthiazolo[5,4- *b*]pyridine-6-carboxylic acid

Step-1: 8-Isopropyl-2-methylfuro[3,4-d]thiazolo[5,4-b]pyridin-6(8H)-one: To a (-78°C) cooled and stirred solution of ethyl 7-formyl-2-methylthiazolo[5,4-b]pyridine-6-carboxylate (2.0 g, 7.99 mmol) in THF (20 mL) was added dropwise a solution of isopropyl magnesium chloride (2.9M in 2-methylfuran, 4.13 mL, 11.99 mmol). After stirring for 6 h at the same temperature, reaction mass was quenched with saturated ammonium chloride in water (50 mL) followed by ethyl acetate (50 mL). The layers were seprated and aqueous layer was extracted with ethyl acetate (2×50 mL). The combined organic layers were washed with brine (50 mL), dried ($Na_2SO_4$) and filtered. The filtrate was rotary evaporated

and the crude product was purified by flash column chromatography (silica gel, 30% ethyl acetate in hexane as eluent) to afford 0.65 g (33%) of the desired product. $^1$HNMR (400 MHz, $CDCl_3$) δ 9.09 (s, 1H), 4.53-4.48 (m, 1H), 2.96 (s, 3H), 2.94-2.87 (m, 1H), 1.35 (d, *J* = 6.5 Hz, 3H), 0.65 (d, *J* = 6.5 Hz, 3H); ESI-MS (m/z) 248.88 $(MH)^+$.

Step-2: Sodium 7-(1-hydroxy-2-methylpropyl)-2-methylthiazolo[5,4-b]pyridine-6-carboxylate: To a stirred solution of step-1 intermediate (0.65 g, 2.62 mmol) in MeOH (10 mL) was added solution of NaOH (0.021 g, 0.524 mmol) in water (5 mL) at 0°C and the resulting mixture was allowed to reach to room temperature and stirred for 16h at RT. The reaction mass was concentrated and azeotroped with toluene to afford 0.6 g (80%) of the titled product. ESI-MS (m/z) 288.89 $(MH)^+$.

Step-3: 7-(1-Methoxy-2-methylpropyl)-2-methylthiazolo[5,4-b]pyridine-6-carboxylic acid: To a (0°C) cooled and stirred solution of step-2 intermediate (0.6 g, 2.08 mmol) in THF (50 mL) was added potassium *tert*-butoxide (0.467 g, 4.16 mmol) followed by methyl iodide (0.521 ml, 8.32 mmol). The resulting mass was allowed to warm to RT and continued stirring for 6 h at RT. Reaction mass was diluted with ethyl acetate (60 mL), acidified with 10% HCl, separated organic layer was washed with water and brine. The organic layer was dried ($Na_2SO_4$) and concentrated under vacuum. The crude mass was washed with diethyl ether (20 mL) to afford 0.35 g (60%) of the desired product.

**Example-19:** The following examples were prepared by using the similar procedure described in Example-18:

(±)-7-(1-Methoxypropyl)-2-methylthiazolo[5,4-b]pyridine-6-carboxylic acid; ESI-MS (m/z) 267.03 $(MH)^+$ and

(±)-7-(Cyclopropyl(methoxy)methyl)-2-methylthiazolo[5,4-b]pyridine-6-carboxylic acid; ESI-MS (m/z) 278.83 $(MH)^+$.

**Example-20:** (±)-7-(1-Methoxyethyl)-2-methylthiazolo[5,4-b]pyridine-6-carboxylic acid

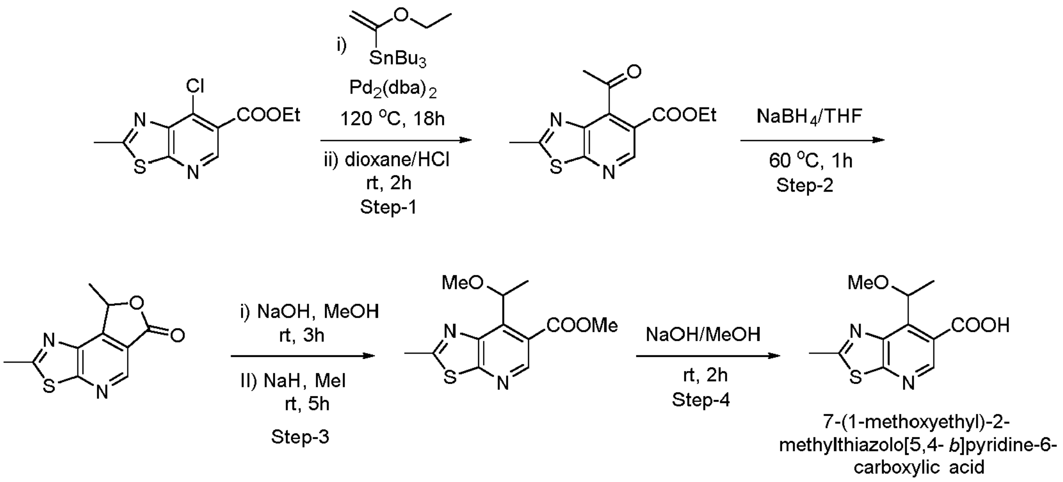

7-(1-methoxyethyl)-2-methylthiazolo[5,4- *b*]pyridine-6-carboxylic acid

Step-1: Ethyl 7-acetyl-2-methylthiazolo[5,4-b]pyridine-6-carboxylate: To a stirred solution of ethyl 7-chloro-2-methylthiazolo[5,4-b]pyridine-6-carboxylate (15 g, 58.4 mmol), tributyl(1-ethoxyvinyl)stannane (21.1 g, 58.4 mmol) and triphenylphosphine (1.22 g, 4.67 mmol) in toluene (150 mL) was purged nitrogen gas for 30 min. $Pd_2dba_2$(1.34 g, 2.33 mmol) was then added to the above mixture. The resulting mixture was heated at 120°C for 18 h in a sealed tube. The intermediate (ethyl 7-(1-ethoxyvinyl)-2-methylthiazolo[5,4-b]pyridine-6-carboxylate) formation was observed by LCMS and TLC. The reaction mass was cooled to room temperature and filtered through celite. The filtrate was evaporated. The residue obtained was dissolved in THF (100 mL) and 10% HCl (50 mL) was added at 0°C. The suspension was warmed to room temperature and stirred for 2 h. The reaction mass was diluted with water (50 mL) followed by ethyl acetate (200 mL). The layers were separated and the aqueous layer was extracted with ethyl acetate (2×100 mL). The combined organic layers was washed with brine (100 mL), dried ($Na_2SO_4$) and filtered. The filtrate was rotary evaporated and the crude product was purified by column chromatography (silica gel, 25% EtOAc in hexane as eluent) to afford (13 g, 84%) of the titled compound as pale yellow solid. $^1$HNMR (400 MHz, $CDCl_3$) δ 9.14 (s, 1H), 4.45 (q, *J* = 7.0 *Hz*, 2H), 2.89 (s, 3H), 2.76 (s, 3H), 1.44 (t, *J* = 7.0 *Hz*, 3H); ESI-MS (m/z) 265.10 (MH)+.

Step-2: 2,8-Dimethylfuro[3,4-d]thiazolo[5,4-b]pyridin-6(8H)-one: To a stirred solution of step-1 intermediate (6.5 g, 24.59 mmol) in methanol was added $NaBH_4$ (1.2 g, 32.0 mmol) portionwise at 0°C and the reaction mixture was stirred for 15 min. The reaction mass was warmed to room temperature and heated at 60°C for 1 h. The reaction was cooled

to 0°C and quenched by the addition of acetone (5 mL).

The solvent was evaporated. Water (50 mL) was added to the residue followed by ethyl acetate (250 mL). The layers were separated and the aqueous layer was extracted with ethyl acetate (2×100mL). The combined organic layers were washed with brine (50 mL), dried ($Na_2SO_4$) and filtered. The filtrate was rotary evaporated to afford (5.1g, 94%) of the titled compound as yellowish brown solid. $^1$HNMR (400 MHz, DMSO-$d_6$) δ 9.04 (s, 1H), 6.24-6.03 (m, 1H), 2.95 (s, 3H), 1.76 (d, *J* = 7.0 *Hz,* 3H); ESI-MS (m/z) 221.01 (MH)+.

Step-3: Methyl 7-(1-methoxyethyl)-2-methylthiazolo[5,4-b]pyridine-6-carboxylate: To a (0°C) cooled and stirred solution of step-2 intermediate (3.47 g, 15.75 mmol) in methanol (50 mL) was added NaOH (819 mg, 20.48 mmol) in water (5 mL). The reaction mixture was warmed to room temperature and stirred for 3h. The reaction mixture was charged with conc HCl (394 μL, 4.73 mmol) and stirred for 2 min and concentrated to dryness. The residue was azeotropped with toluene to obtain intermediate sodium 7-(1-hydroxyethyl)-2-methylthiazolo[5,4-b]pyridine-6-carboxylate as brownish yellow solid. The solid obtained was dissolved in DMA (20 mL) and cooled to 0°C. NaH (0.756 g, 18.91 mmol, 60% dispersion in mineral oil) was added to the reaction and the suspension obtained was stirred for 15 min. The reaction mixture was warmed to room temperature and stirred for 5 min. The reaction mixture was cooled to 0°C followed by addition of iodomethane (1.28 mL, 20.48 mmol) in five equal portions over a period of 5h. The reaction was warmed to room temperature and stirred for 10 min and cooled to 0°C and quenched with sat. saturated aqueous $NH_4Cl$ solution (20 mL). Water (50 mL) was added to the reaction mixture followed by EtOAc (200 mL). The layers were separated and the aqueous layer was extracted with ethyl acetate (2×100 mL). The combined organic layers were washed with brine (100 mL), dried ($Na_2SO_4$) and filtered. The filtrate was rotary evaporated and the crude product was purified by column chromatography (silica gel, 8% EtOAc in hexane as eluent) to afford (2.75 g, 65%) of the titled compound as a white solid. $^1$HNMR (400 MHz, $CDCl_3$) δ 8.56 (s, 1H), 5.29 (q, *J* = 6.5 *Hz,* 1H), 3.89 (s, 3H), 3.19 (s, 3H), 2.82 (s, 3H), 1.65 (d, *J* = 6.5 *Hz,* 3H); ESI-MS (m/z) 266.86 (MH)+.

Step-4: 7-(1-methoxyethyl)-2-methylthiazolo[5,4-b]pyridine-6-carboxylic acid: To a 0°C stirred and cooled solution of step-3 intermediate (2.75 g, 10.33 mmol) in MeOH (30 mL) was added NaOH (1.23 g, 31.0 mmol) in water (10 mL). The reaction mixture was warmed to room temperature and stirred for 1h. The solvent was evaporated under vacuum. The residue obtained was dissolved in water (20 mL) and acidified with 10% HCl till pH- 4. The suspension obtained was diluted with ethyl acetate (50 mL). The layers were separated and the aqueous layer was extracted with ethyl acetate (2×50 mL). The combined organic layers were washed with brine (10 mL), dried ($Na_2SO_4$) and filtered. The filtrate was rotary evaporated to afford (2.4 g, 92%) of the titled compound as white solid. $^1$HNMR (400 MHz, DMSO-$d_6$) δ 13.50 (s, 1H, $D_2O$ exchangeable), 8.74 (s, 1H), 5.42 (q, *J* = 6.5 *Hz,* 1H), 3.25 (s, 3H), 3.00 (s, 3H), 1.73 (d, *J* = 6.5 *Hz,* 3H); ESI-MS (m/z) 253.02 $(MH)^+$.

**Example-21:** Preparation of (±)-7-(1-(2-methoxyethoxy)ethyl)-2-methylthiazolo[5,4-b]pyridine-6-carboxylic acid

The titled compound was prepared by following the similar procedure described for example-20. ESI-MS (m/z) 297.21 $(MH)^+$.

**Example-22:** Preparation of (±)-7-(1-Methoxyethyl)-2-methylthiazolo[5,4-b]pyridin-6-amine

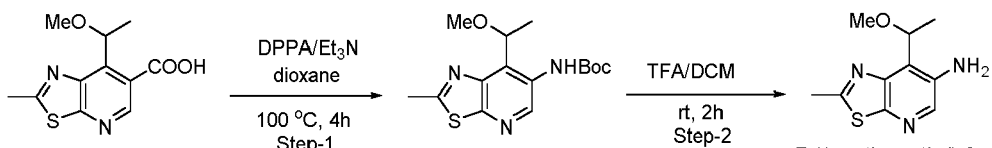


7-(1-methoxyethyl)-2-methylthiazolo[5,4-*b*]pyridin-6-amine

Step-1: *tert*-Butyl (7-(1-methoxyethyl)-2-methylthiazolo[5,4-b]pyridin-6-yl)carbamate: To a stirred solution of 7-(1-methoxyethyl)-2-methylthiazolo[5,4-b]pyridine-6-carboxylic acid (1.0 g, 3.96 mmol) in *t*-butanol (20 mL) was added triethylamine (1.11 mL, 7.93 mmol) at rt followed by diphenyl phosphorazidate (1.00 mL, 4.36 mmol) and stirred for 10 min at same temp and then 4h at 100°C. The reaction mixture was diluted with water (10 mL) followed by DCM (10 mL). The layers were separated and the aqueous layer was extracted with DCM (2×15 mL). The combined organic layers were washed with brine (10 mL), dried ($Na_2SO_4$) and filtered. The filtrate was rotary evaporated and the crude product was purified by flash chromatography (silica gel) to afford 800 mg (62%) of the desired product as a white solid. $^1$HNMR (400 MHz, DMSO-$d_6$) δ 8.88 (s, 1H), 8.59 (s, 1H), 5.34 (q, *J* = 6.5 *Hz,* 1H), 3.22 (s, 3H), 2.84 (s, 3H), 1.53 (d, *J* = 6.5 *Hz,* 3H), 1.49 (s, 9H); ESI-MS (m/z) 323.97 $(MH)^+$.

Step-2: 7-(1-Methoxyethyl)-2-methylthiazolo[5,4-b]pyridin-6-amine: To a stired solution of step-1 intermediate (450 mg, 1.391 mmol) in DCM (20 mL) was added trifluoroacetic acid (1.07 mL, 13.91 mmol) and stirred at room temperature for 2 h. The reaction mixture was diluted with water (5 mL) and basified with sodium bicarbonate solution (3 mL) and extracted with DCM (3×10 mL). The combined organic layers were washed with brine (15 mL), dried ($Na_2SO_4$) and filtered. The filtrate was rotary evaporated to afford 300 mg (97%) of the desired product as white solid. $^1H$ NMR (400 MHz, DMSO-$d_6$) δ 8.04 (s, 1H), 5.36 (q, *J* = 6.5 *Hz,* 1H), 3.21 (s, 3H), 2.76 (s, 3H), 1.45 (d, *J* = 6.5 *Hz,* 3H); ESI-MS (m/z) 223.79 (MH)+.

**Example-23:** Preparation of 7-(1-ethoxyethyl)-2-methylthiazolo[5,4-b]pyridine-6-carboxylic acid

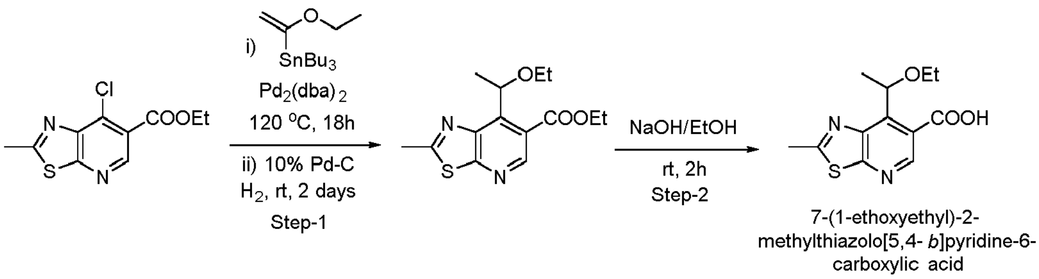


7-(1-ethoxyethyl)-2-methylthiazolo[5,4- *b*]pyridine-6-carboxylic acid

Step-1:Ethyl 7-(1-ethoxyethyl)-2-methylthiazolo[5,4-b]pyridine-6-carboxylate: To a stirred solution of ethyl 7-chloro-2-methylthiazolo[5,4-b]pyridine-6-carboxylate (9.0 g, 35.1 mmol) in toluene (100 mL) was added tributyl(1-ethoxyvinyl)stannane (12.66 g, 35.1 mmol) and triphenylphosphine (736 mg, 2.80 mmol). The resulting mixture was purged nitrogen gas for 30 min and $Pd_2dba_2$ (806 mg, 1.4 mmol) was added. The reaction mixture was heated at 120°C for 18 h in a sealed tube. The intermediate (ethyl 7-(1-ethoxyvinyl)-2-methylthiazolo[5,4-b]pyridine-6-carboxylate) formation was observed by LCMS and TLC. The reaction mass was cooled to room temperature and filtered through celite. The filtrate was transferred to RB flask and charged with 10% Pd/C and hydrogenated at atmospheric pressure for 48h. The reaction mass was filtered through celite. The filtrate was rotary evaporated and the crude product was purified by column chromatography (silica gel, 15% EtOAc in hexane as eluent) to afford (4.0 g, 38%) of the titled compound as white solid. $^1$HNMR (400 MHz, $CDCl_3$) δ 8.62 (s, 1H), 5.47 (q, *J* = 6.5 *Hz,* 1H), 4.47-4.39 (m, 2H), 3.50-3.40 (m, 1H), 3.39-3.30 (m, 1H), 2.90 (s, 3H), 1.75 (d, *J* = 6.5 *Hz,* 3H), 1.44 (t, *J* = 7.0 *Hz,* 3H), 1.18 (t, *J* = 7.0 *Hz,* 3H); ESI-MS (m/z) 295.09 $(MH)^+$.

Step-2: 7-(1-ethoxyethyl)-2-methylthiazolo[5,4-b]pyridine-6-carboxylic acid: To the 0°C stirred and cooled solution of ethyl 7-(1-ethoxyethyl)-2-methylthiazolo[5,4-b]pyridine-6-carboxylate (3.0 g, 10.19 mmol) in ethanol (30 mL) was added NaOH (815 mg, 20.38 mmol) in water (10 mL). The reaction mixture was warmed to room temperature and stirred for 2h. The solvent was evaporated under vacuum. The residue obtained was dissolved in water (20 mL) and acidified with 10% HCl (pH- 4). The suspension obtained was diluted with ethyl acetate (50 mL). The layers were separated and the aqueous layer was extracted with ethyl acetate (2×50 mL). The combined organic layers were washed with brine (10 mL), dried ($Na_2SO_4$) and filtered. The filtrate was rotary evaporated to afford (2.6 g, 95%) of the titled compound as white solid. $^1$HNMR (400 MHz, DMSO-$d_6$) δ 13.34 (s, 1H, $D_2O$ exchangeable), 8.59 (s, 1H), 5.42-5.36 (m, 1H), 3.42-3.31 (m, 1H), 3.27-3.19 (m, 1H), 2.87 (s, 3H), 1.65-1.55 (m, 3H), 1.13-1.01 (m, 3H); ESI-MS (m/z) 267.09 $(MH)^+$.

**Example-24:** Preparation of (±)-7-(1-methoxypropan-2-yl)-2-methylthiazolo[5,4-b]pyridine-6-carboxylic acid

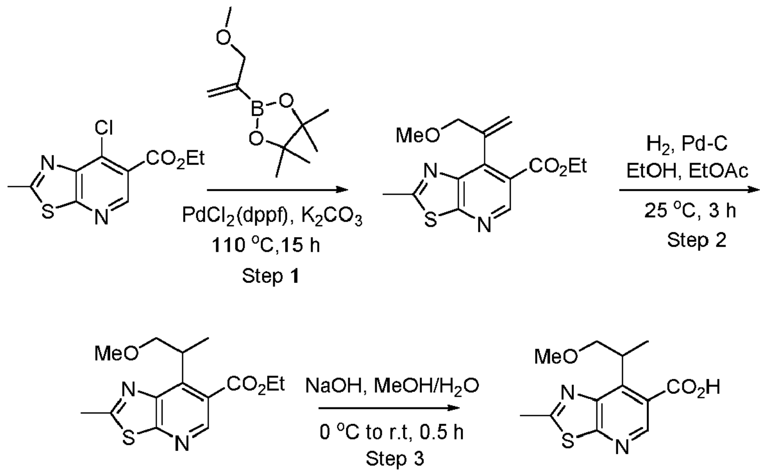


Step-1: Ethyl 7-(3-methoxyprop-1-en-2-yl)-2-methylthiazolo[5,4-b]pyridine-6-carboxylate: To a nitrogen purged suspension of 1,4-dioxane (50 mL) and potassium carbonate (7.68 g, 55.5 mmol) was added 2-(3-methoxyprop-1-en-2-yl)-4,4,5,5-tetramethyl-1,3,2-dioxaborolane (5.50 g, 27.8 mmol), ethyl 7-chloro-2-methylthiazolo[5,4-b]pyridine-6-carboxylate (7.13 g, 27.8 mmol) and $PdCl_2$(dppf) (2.032 g, 2.78 mmol) sequentially. The sealed tube was capped and stirred at 110°C for 15 h. The reaction mixture was cooled to room temperature, water (50 mL) was added followed by ethyl acetate (50 mL). The layers were separated and aqueous layer was extracted with ethyl acetate (2×25 mL). The combined organic layers were washed with saturated aqueous $NaHCO_3$ solution (20 mL), dried ($Na_2SO_4$) and filtered. The filtrate was rotary evaporated and the crude product was purified by flash column chromatography (silica gel, 20% ethyl acetate in hexane as eluent) to afford (1.30 g, 16%) of the title compound as white solid. $^1$HNMR (400 MHz, Chloroform-d) δ 8.97 (s, 1H), 5.71 (s, 1H), 5.23 (s, 1H), 4.43 (s, 2H), 4.42 - 4.37 (m, 2H), 3.46 (s, 3H), 2.88 (s, 3H), 1.41 (t, *J* = 7.1 Hz, 3H); ESI-MS (m/z) 292.93$(MH)^+$.

Step-2: Ethyl 7-(1-methoxypropan-2-yl)-2-methylthiazolo[5,4-b]pyridine-6-carboxylate: To the stirred solution of Ethyl 7-(3-methoxyprop-1-en-2-yl)-2-methylthiazolo[5,4-b]pyridine-6-carboxylate (1.25 g, 4.28 mmol) in methanol (10 mL) and ethyl acetate (10 mL) was added 10% Pd-C (1.138 g, 1.069 mmol). The reaction was allowed to continue for 3 h in parr reactor under hydrogen pressure (60 psi). Upon completion, the reaction mixture was filtered through celite. The celite bed was washed with EtOAc (50 mL) and the filtrate was rotary evaporated. The crude product was purified by flash column chromatography (silica gel, 15% ethyl acetate in hexane as eluent) to afford (250 mg, 20%) of title compound as white solid. $^1$H NMR (400 MHz, Chloroform-*d*) δ 8.83 (s, 1H), 4.46 (q, *J* = 6.9 Hz, 2H), 4.35 - 4.23 (m, 1H), 4.18 (t, *J* = 8.3 Hz, 1H), 3.90 (t, *J* = 8.1 Hz, 1H), 1.54 (d, *J* = 6.9 Hz, 3H), 1.45 (t, *J* = 7.1 Hz, 3H); ESI-MS (m/z) 294.99 $(MH)^+$.

Step 3: 7-(1-Methoxypropan-2-yl)-2-methylthiazolo[5,4-b]pyridine-6-carboxylic acid: To a (0°C) cooled and stirred solution of Ethyl 7-(1-methoxypropan-2-yl)-2-methylthiazolo[5,4-b]pyridine-6-carboxylate (220 mg, 0.75 mmol) in methanol (10 mL) and water (1 mL) was added NaOH (60 mg, 1.50 mmol). The reaction was stirred at room temperature for 0.5 h. The solvent was rotary evaporated. Water (10 mL) was added to the reaction and pH was adjusted to 4 using 10% aq.HCl followed by addition of ethyl acetate (30 mL). The layers were separated and the aqueous layer was extracted with ethyl acetate (2×20 mL). The combined organic layers were washed with brine (20 mL), dried over ($Na_2SO_4$) and filtered. The filtrate was rotary evaporated to afford 200 mg (100%) of title compound as white solid. $^1$H NMR (400 MHz, DMSO-$d_6$) δ 13.61 (bs, Exchanges with $D_2O$, 1H), 8.77 (s, 1H), 4.28 - 4.17 (m, 1H), 4.04 (t, *J* = 8.6 Hz, 1H), 3.79 (t, *J* = 8.2 Hz, 1H), 3.16 (s, 3H), 2.88 (s, 3H), 1.44 (d, *J* = 6.8 Hz, 3H); ESI-MS (m/z) 267.03 $(MH)^+$.

**Example-25:** The following compounds were prepared by using the steps 1-3 as described under Example-24:

7-isopropyl-2-methylthiazolo[5,4-b]pyridine-6-carboxylic acid; ESI-MS (m/z) 237.02 (MH)+;

(±)-2-methyl-7-(tetrahydrofuran-2-yl)thiazolo[5,4-b]pyridine-6-carboxylic acid; ESI-MS (m/z) 265.08 $(MH)^+$; and

(±)-2-methyl-7-(tetrahydro-2H-pyran-2-yl)thiazolo[5,4-b]pyridine-6-carboxylic acid; ESI-MS (m/z) 279.07 $(MH)^+$.

**Example-26:** Preparation of (1S, 2S) or (1R, 2R)-7-(2-(methoxymethyl)cyclopropyl)-2-methylthiazolo[5,4-b]pyridine-6-carboxylic acid [Stereochemistry assigned tentatively it could be (1S, 2S) or (1R, 2R)] and

**Example-27:** Preparation of (1R, 2R) or (1S, 2S)-7-(2-(methoxymethyl)cyclopropyl)-2-methylthiazolo[5,4-b]pyridine-6-carboxylic acid [Stereochemistry assigned tentatively it could be (1R, 2R) or (1S, 2S)]

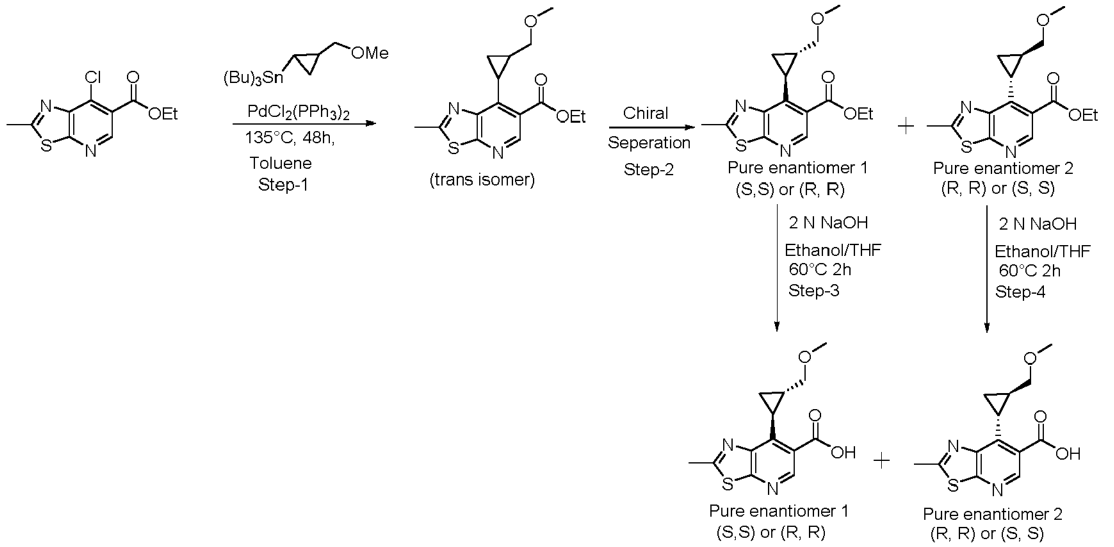


Step-1: Ethyl 7-(2-(methoxymethyl)cyclopropyl)-2-methylthiazolo[5,4-b]pyridine-6-carboxylate: To a nitrogen purged solution of ethyl 7-chloro-2-methylthiazolo[5,4-b]pyridine-6-carboxylate (1.0 g, 3.90 mmol) and tributyl-(trans-2-methoxymethyl-cyclopropyl)-stannane (Prepared by the procedure reported in WO2009/125365) (1.61 g, 4.29 mmol) in toluene (20 mL) were added $PdCl_2(PPh_3)_2$ (0.45 g, 0.39 mmol). The sealed tube was capped and stirred at 135°C for 48 h. The reaction mixture was rotary evaporated and the crude product was purified by flash column chromatography (silica gel, 10% ethyl acetate in hexane as eluent) to afford (0.9 g, 75%) of the title compound as an oil. $^1$HNMR (400 MHz, $CDCl_3$) δ 8.84 (s, 1H), 4.49-4.43 (m,2H), 3.59 (dd, *J* = 10.3, 6.4 Hz, 1H), 3.47 (dd, *J* = 10.3, 7.1 Hz, 1H), 3.42 (s, 3H), 2.82 (s, 3H), 2.63-253 (m, 1H), 1.48-1.44 (m, 3H), 1.27-1.25 (m, 2H),1.20-1.15(m,1H); ESI-MS (m/z) 306.94 $(MH)^+$.

Step-2: Separation of ethyl 7-(2-(methoxymethyl)cyclopropyl)-2-methylthiazolo[5,4-b]pyridine-6-carboxylate was carried out using chiral column to afford enantiomer 1 & enantiomer 2.

(Enantiomer 1): (1S, 2S) or (1R, 2R)-Ethyl 7-(2-(methoxymethyl)cyclopropyl)-2-methylthiazolo[5,4-b]pyridine-6-carboxylate [Stereochemistry assigned tentatively it could be (1S, 2S) or (1R, 2R)].

Chiral HPLC RT: 8.11 min; ESI-MS (m/z) 306.94 $(MH)^+$.

(Enantiomer 2); (1R, 2R) or (1S, 2S)-Ethyl 7-(2-(methoxymethyl)cyclopropyl)-2-methylthiazolo[5,4-b]pyridine-6-carboxylate [Stereochemistry assigned tentatively it could be (1R, 2R) or (1S, 2S)].

Chiral HPLC RT: 9.63 min, ESI-MS (m/z) 306.94 $(MH)^+$.

Step-3: (1S, 2S) or (1R, 2R)-7-(2-(Methoxymethyl)cyclopropyl)-2-methylthiazolo[5,4-b]pyridine-6-carboxylic acid: To a (0°C) cooled and stirred solution of (1S, 2S) or (1R, 2R)-Ethyl 7-(2-(methoxymethyl)cyclopropyl)-2-methylthiazolo[5,4-b]pyridine-6-carboxylate (enantiomer 1) (150 mg, 0.49 mmol) in ethanol (10 mL) and THF (20 mL) was added 2M NaOH (0.49 mL, 0.79 mmol). The reaction was stirred at room temperature for 15 min and then at 60°C for 2 h. The reaction mixture was cooled to room temperature and the solvent was rotary evaporated. Water (30 mL) was added to the reaction and pH was adjusted to 2 using 10% aq. HCl followed by addition of ethyl acetate (50 mL). The layers were separated and the aqueous layer was extracted with ethyl acetate (2×50 mL). The combined organic layer was washed with brine (20 mL), dried over ($Na_2SO_4$) and filtered. The filtrate was rotary evaporated to afford 100 mg (73.4%) of the title compound as white solid. $^1$HNMR (400 MHz, DMSO-$d_6$) δ 13.53 (s, 1H), 8.74 (s, 1H), 3.46-3.41 (m, 2H), 3.26 (s, 3H), 2.82 (s, 3H), 2.63-253 (m, 1H), 1.94-1.89 (m, 1H), 1.27-1.25 (m, 2H); ESI-MS (m/z) 278.92 $(MH)^+$.

Step-4: (1R, 2R) or (1S, 2S)-7-(2-(methoxymethyl)cyclopropyl)-2-methylthiazolo[5,4-b]pyridine-6-carboxylic acid: Following the procedure as described in step 3, (1R, 2R) or (1S, 2S)-7-(2-(methoxymethyl)cyclopropyl)-2-methylthiazolo[5,4-b]pyridine-6-carboxylic acid was obtained from (1R, 2R) or (1S, 2S)-Ethyl 7-(2-(methoxymethyl)cyclopropyl)-2-methylthiazolo[5,4-b]pyridine-6-carboxylate (enantiomer 2).

$^1$HNMR (400 MHz, DMSO-$d_6$) δ 13.53 (s, 1H), 8.74 (s, 1H), 3.46-3.41 (m, 2H), 3.26 (s, 3H), 2.82 (s, 3H), 2.63-253 (m,

1H), 1.94-1.89 (m, 1H), 1.27-1.25 (m, 2H); ESI-MS (m/z) 278.92 $(MH)^+$.

**Example-28:** Preparation of (±)-7-(sec-butyl)-2-methylthiazolo[5,4-b]pyridine-6-carboxylic acid

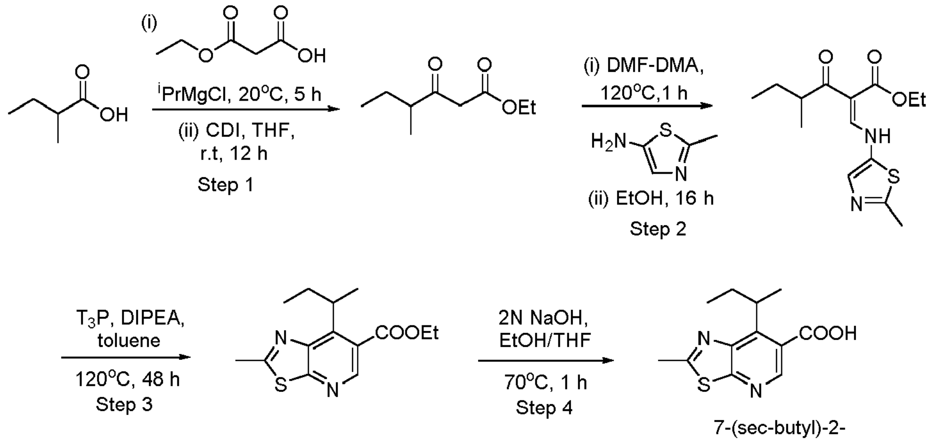


7-(sec-butyl)-2-methylthiazolo[5,4-b]pyridine-6-carboxylic acid

Step-1: Ethyl 4-methyl-3-oxohexanoate: To a solution of 3-ethoxy-3-oxopropanoic acid (6.47 g, 49.0 mmol) in THF (20 mL) at 0°C was added dropwise Isopropylmagnesium chloride solution (2M in THF, 47.3 mL, 95 mmol) and the reaction mixture was stirred for 5 h at 20°C. Thereafter, this solution was cooled to 0°C and then added dropwise to a THF (25 mL) solution of 2-methylbutanoic acid (5.34 mL, 49.0 mmol) and CDI (6.35 g, 39.2 mmol) which was preformed after stirring at room temperature for 12 h. The combined reaction mixture was stirred for 2 h at room temperature. Upon completion, the reaction mixture was quenched with 10% aqueous citric acid (25 mL), extracted with EtOAc, washed with aqueous saturated $NaHCO_3$, dried over $Na_2SO_4$, filtered and concentrated. The crude residue was purified by flash column chromatography on silica gel using hexane/ethyl acetate (5:95) to afford desired product (4 g, 71% yield). $^1H$ NMR (400 MHz, DMSO-$d_6$) δ 4.10 (q, *J*= 7.0 Hz, 2H), 3.64 (s, 2H), 2.63 - 2.52 (m, 1H), 1.70 - 1.54 (m, 1H), 1.43 - 1.29 (m, 1H), 1.19 (t, *J* = 7.1 Hz, 3H), 1.01 (d, *J* = 6.9 Hz, 3H), 0.83 (t, *J* = 7.5 Hz, 3H); GCMS (m/z) 172.2(M)+.

Step-2: Ethyl 4-methyl-2-(((2-methylthiazol-5-yl)amino)methylene)-3-oxohexanoate: A mixture of step-1 intermediate (2 g, 11.61 mmol), and N,N-dimethylformamide-dimethyl acetal (1.54 mL, 11.61 mmol) was stirred at 120°C for 1 h. Thereafter the reaction mixture was cooled to 0°C followed by addition of solution of 2-methylthiazol-5-amine hydrochloride (1.749 g, 11.61 mmol) and TEA (4.86 mL, 34.8 mmol) in EtOH (20 mL). The resulting mixture was stirred for 16 h at 25°C. The reaction mixture was concentrated under vacuum and the residue was diluted with water (25 mL) followed by ethyl acetate (50 mL). The layers were separated and the aqueous layer was extracted with ethyl acetate (2×50 mL). The combined organic layers were washed with brine (50 mL), dried over ($Na_2SO_4$) and filtered. The filtrate was rotary evaporated and the crude product was purified by flash column chromatography (silica gel, 20% ethyl acetate in hexane as eluent) to afford 1.5 g (44%) of the desired product. $^1H$ NMR (400 MHz, DMSO-$d_6$) δ 12.42 (d, *J* = 13.0 Hz, 1H), 8.02 (d, *J* = 13.0 Hz, 1H), 7.63 (s, 1H), 4.15 (q, *J* = 7.1 Hz, 2H), 3.60 - 3.50 (m, 1H), 2.59 (s, 3H), 1.71- 1.60 (m, 1H), 1.36 - 1.21 (m, 4H), 1.01 (d, *J* = 6.8 Hz, 3H), 0.83 (t, *J* = 7.4 Hz, 3H); ESI-MS (m/z) 297.0 $(MH)^+$.

Step-3: Ethyl 7-(sec-butyl)-2-methylthiazolo[5,4-b]pyridine-6-carboxylate: To a solution of step-2 intermediate (1.5 g, 5.06 mmol) in toluene (25 mL) was added DIPEA (6.19 g, 35.4 mmol) and propylphosphonic anhydride (50% in ethyl acetate) (8.06 mL, 12.65 mmol) at room temperature. The resulting mixture was stirred at 120°C for 48 h and then poured into ice water and extracted with ethyl acetate (3 x 50mL). The combined organic layers were washed with water (2 × 50 mL), brine (50 mL), dried over ($Na_2SO_4$) and filtered. The filtrate was concentrated under reduced pressure and the crude product was purified by flash column chromatography (silica gel, 20-30% EtOAc in haxane system as eluent) to afford 600 mg (43%) of the titled compound. $^1H$ NMR (400 MHz, DMSO-$d_6$) δ 8.70 (s, 1H), 4.38 (q, *J* = 7.1 Hz, 2H), 3.62 - 3.52 (m, 1H), 2.87 (s, 3H), 2.20 - 2.06 (m, 1H), 1.97 - 1.82 (m, 1H), 1.48 (d, *J* = 7.0 Hz, 3H), 1.35 (t, *J* = 7.1 Hz, 3H), 0.73 (t, *J* = 7.4 Hz, 3H). ESI-MS (m/z) 278.9 $(MH)^+$.

Step-4: 7-(sec-Butyl)-2-methylthiazolo[5,4-b]pyridine-6-carboxylic acid: To a 0°C cooled and stirred solution of ethyl 7-(sec-butyl)-2-methylthiazolo[5,4-b]pyridine-6-carboxylate (0.6 g, 2.155 mmol) in EtOH (10 mL) and THF (20 mL) was added 2M NaOH (2.15 mL, 4.31 mmol). The reaction mixture was warmed to room temperature and stirred at 70°C for 1h. The solvent was evaporated under vacuum. The residue thus obtained was dissolved in water (20 mL) and acidified with 10% HCl until pH-2. The resulting suspension was extracted with ethyl acetate (2 × 50 mL). The combined

organic layers were washed with brine (10 mL), dried over ($Na_2SO_4$) and filtered. The filtrate was rotary evaporated to afford (0.5 g, 93%) of the titled compound as white solid. $^1$H NMR (400 MHz, DMSO-$d_6$) δ 8.71 (s, 1H), 3.77 - 3.68 (m, 1H), 2.86 (s, 3H), 2.20-2.06 (m, 1H),1.96 -1.82 (m, 1H), 1.47 (d, $J$ = 6.9 Hz, 3H), 0.72 (t, $J$ = 7.6 Hz, 3H); ESI-MS (m/z) 251.1 $(MH)^+$.

**Example-29:** The following compound was prepared by using the similar procedure described in example-28: 2-Methyl-7-(1-methylcyclopropyl)thiazolo[5,4-b]pyridine-6-carboxylic acid, ESI-MS (m/z) 249.2 $(MH)^+$.

**Example-30:** Preparation of 7-(2-(2-methoxyethoxy)ethyl)-2-methylthiazolo[5,4-b]pyridin -6-amine

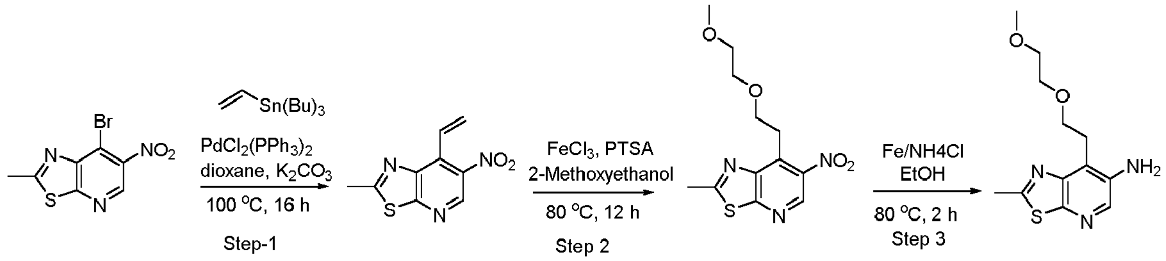


Step-1: 2-Methyl-6-nitro-7-vinylthiazolo[5,4-b]pyridine: To a nitrogen purged solution of 7-bromo-2-methyl-6-nitrothiazolo[5,4-b]pyridine (4.0 g, 14.59 mmol) and tributyl(vinyl)stannane (9.26 mL, 29.2 mmol) in 1,4-dioxane (20 mL) was added potassium carbonate (1.28 g, 9.30 mmol) and $PdCl_2(PPh_3)_2$ (21.0 g, 1.459 mmol) sequentially. The sealed tube was capped and stirred at 100°C for 16 h. The reaction mixture was cooled to room temperature; water (20 mL) was added followed by ethyl acetate (30 mL). The layers were separated and aqueous layer was extracted with ethyl acetate (2×25 mL). The combined organic layers were washed with saturated aqueous $NaHCO_3$ solution (20 mL), dried over $Na_2SO_4$ and filtered. The filtrate was rotary evaporated and the crude product was purified by flash column chromatography (silica gel, 10% ethyl acetate in hexane as eluent) to afford (2.0 g, 62%) of the title compound as white solid. $^1$HNMR (400 MHz, DMSO-$d_6$) δ 9.12 (s, 1H), 7.10 (dd, $J$ = 17.5, 12.1 Hz, 1H), 6.71 (d, $J$ = 17.5 Hz, 1H), 6.09 (d, $J$ = 11.7 Hz, 1H), 2.92 (s, 3H); ESI-MS (m/z) 221.93 $(MH)^+$.

Step-2: 7-(2-(2-Methoxyethoxy)ethyl)-2-methyl-6-nitrothiazolo[5,4-b]pyridine: To a stirred solution of 2-methyl-6-nitro-7-vinylthiazolo[5,4-b]pyridine (600 mg, 2.71 mmol) in chlorobenzene (20 mL) was added ferric chloride (17.60 mg, 0.108 mmol), PTSA (18.68 mg, 0.108 mmol) and 2-Methoxyethanol (0.85 mL, 10.85 mmol) sequentially. The sealed tube was capped and stirred at 80°C for 12 h. The reaction was cooled to room temperature and the solvent was rotary evaporated. The crude product was purified by flash column chromatography (10% Ethyl acetate in hexane as eluent) to afford (300 mg, 37.2%) of the title compound as solid. $^1$HNMR (400 MHz, DMSO-$d_6$) δ 9.12 (s, 1H), 3.72 (t, $J$ = 6.6 Hz, 2H), 3.66 (t, $J$ = 6.3 Hz, 2H), 3.47 (t, $J$ = 4.7 Hz, 2H), 3.36 (t, $J$ = 4.7 Hz, 2H), 3.17 (s, 3H), 2.93 (s, 3H); ESI-MS (m/z) 298.21 (MH)+.

Step-3: 7-(2-(2-Methoxyethoxy)ethyl)-2-methylthiazolo[5,4-b]pyridin-6-amine: To a stirred solution of 7-(2-(2-methoxyethoxy)ethyl)-2-methyl-6-nitrothiazolo[5,4-b]pyridine (300 mg, 1.0 mmol) in EtOH (20 mL) was added iron powder (563 mg, 10.09 mmol), ammonium chloride (540 mg, 10.00 mmol) and $H_2O$ (5.0 mL). The reaction was heated at 80°C for 2 h. Upon completion, the reaction mixture was cooled to room temperature and filtered through celite bed, and the filtrate was rotary evaporated. Water (30 mL) was added to the residue followed by ethyl acetate (50 mL). The layers were separated and the aqueous layer extracted with ethyl acetate (2x25 mL). The combined organic layer was washed with saturated $NaHCO_3$ (20 mL), dried over $Na_2SO_4$ and filtered. The filtrate was rotary evaporated and the solid residue (230 mg, 85%) was carried forward without purification. $^1$HNMR (400 MHz, DMSO-$d_6$) δ 8.01 (s, 1H), 5.31 (bs, 2H), 3.61 (t, $J$ = 7.1 Hz, 2H), 3.55 (t, $J$ = 4.7 Hz, 2H), 3.43 (t, $J$ = 4.7 Hz, 2H), 3.23 (s, 3H), 3.20 (t, $J$ = 7.3 Hz, 2H), 2.75 (s, 3H); ESI-MS (m/z) 268.3 $(MH)^+$.

**Example-31:** Preparation of *tert*-butyl (7-formyl-2-methylthiazolo[5,4-b]pyridin-6-yl)carbamate

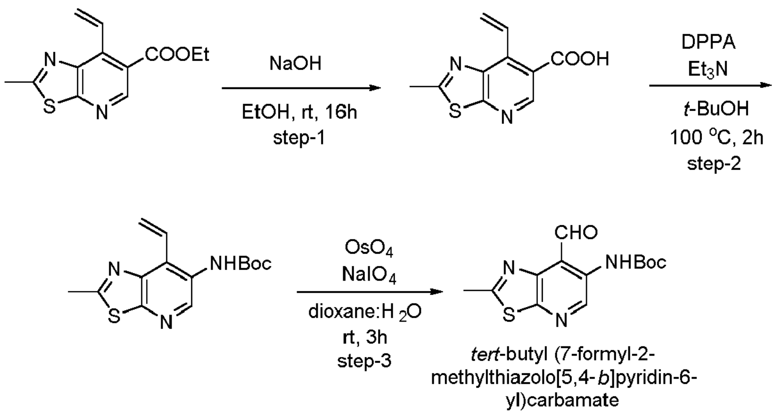


*tert*-butyl (7-formyl-2-methylthiazolo[5,4-*b*]pyridin-6-yl)carbamate

Step-1: 2-Methyl-7-vinyl-7,7a-dihydrothiazolo[5,4-b]pyridine-6-carboxylic acid: To a stirred solution of ethyl 2-methyl-7-vinylthiazolo[5,4-b]pyridine-6-carboxylate (12.0 g, 47.9 mmol) in ethanol (150 mL) was added a solution of NaOH (2.30 g, 57.5 mmol) dissolved in water (25 mL) and stirred at room temperature for 16h. The solvent was evaporated under vacuum and the residue was acidifed with aqueous HCl solution (10%) and the resulting precipitate was filtered and dried to afford 9.0 g (84%) of the titled compound as white solid. $^1$HNMR (400 MHz, DMSO-$d_6$) $\delta$ 13.61 (s, 1H), 8.87 (s, 1H), 7.45 (dd, *J* = 17.5, 11.5 Hz, 1H), 6.74 (dd, *J* = 17.5, 2.5 Hz, 1H), 5.94 (dd, *J* = 11.5, 2.5 Hz, 1H), 2.87 (s, 3H); ESI-MS (m/z) 220.87 $(MH)^+$.

Step-2: *tert*-Butyl (2-methyl-7-vinylthiazolo[5,4-b]pyridin-6-yl)carbamate: To a stirred solution of step-1 intermediate (8 g, 36.0 mmol) in tert. butanol (100 mL) was added triethyl amine (10.0 mL, 72.0 mmol) followed by diphenyl phosphorazidate (8.25 mL, 36.0 mmol) and then stirred the resulting mixture at 100°C for 2h. Reaction was cooled to room temperature and the solvent was evaporated under vacuum. The crude residue was purified by flash column chromatography (silica gel) to afford 5.0 g (48%) of the titled compound as white solid. $^1$HNMR (400 MHz, DMSO-$d_6$) $\delta$ 9.12 (s, 1H), 8.43 (s, 1H), 7.03-6.96 (m, 1H), 6.89-6.85 (m, 1H), 5.91-5.88 (m, 1H), 2.86 (s, 3H), 1.46 (s, 9H); ESI-MS (m/z) 292.10 $(MH)^+$.

Step-3: tert-Butyl (7-formyl-2-methylthiazolo[5,4-b]pyridin-6-yl)carbamate: To a (0°C) cooled and stirred solution of step-2 intermediate (5.0 g, 17.16 mmol) in 1,4-dioxane (100 mL) and water (20 mL) was added osmium tetraoxide (0.436 g, 1.716 mmol) and sodium metaperiodate (11.0 g, 51.5 mmol). The reaction mixture was brought to room temperature and then stirred for 3h at the same temperature. The reaction was cooled back down to 0°C and water (50 mL) was added followed by ethyl acetate (100 mL). The layers were separated and the aqueous layer was extracted with ethyl acetate (2×50 mL). The combined organic layers were washed with brine (50 mL), dried ($Na_2SO_4$) and filtered. The filtrate was rotary evaporated and residue was purified by flash column chromatography (silica gel) to afford 4.0 g (79%) of the titled compound as pale yellow solid. $^1$HNMR (400 MHz, DMSO-$d_6$) $\delta$ 10.81 (s, 1H), 10.17 (s, 1H), 9.40 (s, 1H), 2.92 (s, 3H), 1.52 (s, 9H); ESI-MS (m/z) 294.13 $(MH)^+$.

**Example-32:** Preparation of (±)-2-methyl-7-(1-(pyrrolidin-1-yl)ethyl)thiazolo[5,4-b]pyridin-6-amine

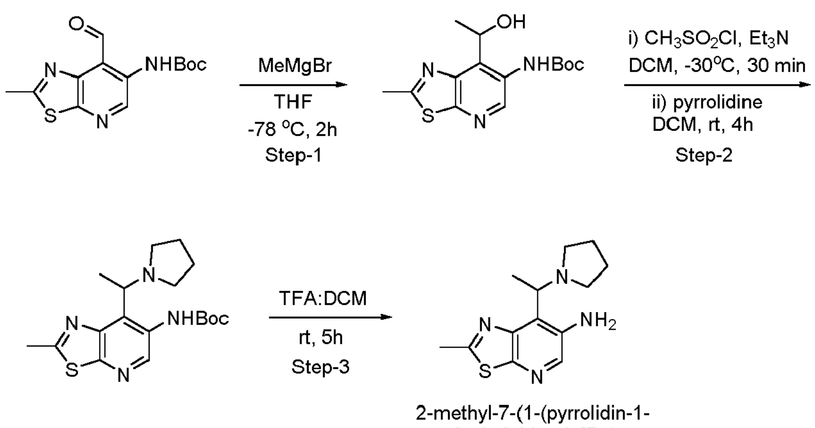


2-methyl-7-(1-(pyrrolidin-1-yl)ethyl)thiazolo[5,4-*b*]pyridin-6-amine

Step-1: tert-Butyl (7-(1-hydroxyethyl)-2-methylthiazolo[5,4-b]pyridin-6-yl)carbamate: To a stirred solution of tert-butyl (7-formyl-2-methylthiazolo[5,4-b]pyridin-6-yl)carbamate (500 mg, 1.70 mmol) in THF (15 mL) was added methylmagnesium bromide (1.12 mL, 3.41 mmol, 3M in THF) at -78°C and stirred the resulting mixture at the same temperature for 2h. The resulting mixture was quenched with saturated aqueous ammonium chloride solution (2 mL) followed by the addition of water (5 mL) and extracted with ethyl acetate (2x50mL). The combined organic layers were washed with brine (10 mL), dried ($Na_2SO_4$) and filtered. The filtrate was rotary evaporated and the crude product was purified by flash column chromatography (40% ethyl acetate in hexane as eluent) to afford 270 mg (51%) of the titled compound as white solid. ESI-MS (m/z) 310.22 $(MH)^+$.

Step-2: tert-Butyl (2-methyl-7-(1-(pyrrolidin-1-yl)ethyl)thiazolo[5,4-b]pyridin-6-yl)carbamate: To a (-30°C) cooled and stirred solution of step-1 intermediate (200 mg, 0.646 mmol) in DCM (20 mL) was added $Et_3N$ (180 μL, 1.293 mmol) followed by methanesulfonyl chloride (60 μL, 0.776 mmol). The reaction mixture was stirred for 30 min at -30°C. The intermediate formation was monitored by TLC and pyrrolidine (214 μL, 2.59 mmol) was added at -30°C to the above mixture. The reaction mixture was warmed to room temperature and then stirred for 4h. The réaction mixture was rotary evaporated and the crude product was purified by flash column chromatography (20% ethyl acetate in hexane as eluent) to afford 95 mg (40%) of the titled compound as white solid. $^1$HNMR (400 MHz, DMSO-$d_6$) δ 10.94 (s, 1H), 9.31 (s, 1H), 4.62 (q, *J* = 6.5 Hz, 1H), 2.83 (s, 3H), 2.79-2.66 (m, 2H), 2.58-2.47 (m, 2H), 1.90-1.82 (m, 4H), 1.56 (s, 9H), 1.49 (d, *J* = 6.5 Hz, 3H); ESI-MS (m/z) 363.41 $(MH)^+$.

Step-3: 2-Methyl-7-(1-(pyrrolidin-1-yl)ethyl)thiazolo[5,4-b]pyridin-6-amine:

To a (0°C) cooled and stirred solution of step-2 intermediate (150 mg, 0.414 mmol) in DCM (10 mL) was added trifluoroacetic acid (319 μL, 4.14 mmol). The resulting mixture was warmed to room temperature and then stirred for 5 h. The réaction mixture was cooled to 0°C and sat. aq. $NaHCO_3$ solutioin (5 mL) was added followed by DCM (10 mL). The layers were separated and the aqueous layer was extracted with DCM (2×10 mL). The combined organic layers were washed with brine (10 mL), dried ($Na_2SO_4$) and filtered. The filtrate was rotary evaporated to afford 100mg (92%) of the titled compound as pale yellow semi solid. ESI-MS (m/z) 263.08 $(MH)^+$.

**Example-33:** The following examples were prepared by following the similar procedure described in example-32:

(±)-7-(1-(Dimethylamino)ethyl)-2-methylthiazolo[5,4-b]pyridin-6-amine, ESI-MS (m/z) 237.11 $(MH)^+$;

(±)-7-(1-(Dimethylamino)propyl)-2-methylthiazolo[5,4-b]pyridin-6-amine, ESI-MS (m/z) 250.27 (M)+; and

(±)-7-(Cyclopropyl(dimethylamino)methyl)-2-methylthiazolo[5,4-b]pyridin-6-amine, ESI-MS (m/z) 262.93 $(MH)^+$.

**Example-34:** Preparation of 7-(1-Methoxy-2-methylpropyl)-2-methylthiazolo[5,4-b]pyridin-6-amine

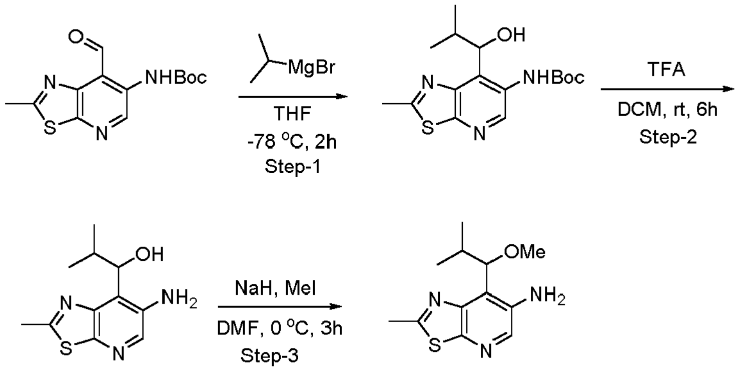


7-(1-methoxy-2-methylpropyl)-2-methylthiazolo[5,4-*b*]pyridin-6-amine

Step-1: *tert*-Butyl (7-(1-hydroxy-2-methylpropyl)-2-methylthiazolo[5,4-b]pyridin-6-yl)carbamate: To a (-78 °C) cooled and stirred solution of tert-butyl (7-formyl-2-methylthiazolo[5,4-b]pyridin-6-yl)carbamate (1.65 g, 5.62 mmol) in THF (25 mL) was added isopropylmagnesium bromide (2.9M in 2-methylfuran, 4.85 mL, 14.06 mmol) dropwise and the

resulting mixture was stirred for 2h at the same temperature. Reaction mass was quenched with saturated ammonium chloride solution at 0°C and then diluted with ethyl acetate (50 mL) followed by the addition of water (20 mL). The layers were separated and the aqueous layer was extracted with ethyl acetate (2×75 mL). The combined organic layers were washed with water (50 mL) and brine (50 mL), dried ($Na_2SO_4$) and filtered. The filtrate was concentrated under vacuum and the crude product was purified by flash column chromatography (silica gel, 20-30% EtOAc in hexanes as eluent) to afford 1.10 g (58%) of the titled compound as white solid. $^1$HNMR (400 MHz, DMSO-$d_6$) δ 9.10 (s, 1H), 8.15 (brs, 1H, $D_2O$ exchangeable), 5.44-5.40 (m, 1H), 2.85 (s, 3H), 2.27-2.15 (m, 1H), 1.55 (s, 9H), 1.13 (d, *J* = 6.5 Hz, 3H), 0.85 (d, *J* = 6.5 Hz, 3H); ESI-MS (m/z) 338.40 $(MH)^+$.

Step-2: 1-(6-Amino-2-methylthiazolo[5,4-b]pyridin-7-yl)-2-methylpropan-1-ol: To a (0°C) cooled and stirred solution of step-1 intermediate (1.0 g, 2.96 mmol) in DCM (20 mL) was added TFA (1.142 mL, 14.82 mmol) dropwise. The resulting mixture was allowed to warm to room temperature and then stirred for 6 h. The solvent was rotary evaporated and the residue was basified with aq. saturated sodium bicarbonate solution and then extracted with DCM (2x75 mL). The combined organic layers were washed with water (50 mL), brine (50 mL), dried ($Na_2SO_4$) and filtered. The filtrate was concentrated under vacuum and the crude product was triturated with hexane and filtered off to afford 0.7 g (100%) of the titled compound. The crude product was used as such for next step without further purification.

Step-3: 7-(1-Methoxy-2-methylpropyl)-2-methylthiazolo[5,4-b]pyridin-6-amine: To a stirred solution of step-2 intermediate (0.56 g, 2.36 mmol) in DMF (10 mL) was added 60% sodium hydride (0.113 g, 2.83 mmol) at 0°C and then stirred for 10 min at the same temperature followed by the addition of methyl iodide (0.162 mL, 2.60 mmol). The resulting mixture was then stirred at 0°C for 3 h and then diluted with ethyl acetate (30 mL) followed by water (10 mL). The layers were separated and the organic layer was washed with water (2×20 mL), brine (20 mL), dried ($Na_2SO_4$) and filtered. the filtrate was concentrated under vacuum and the crude product was purified by flash column chromatography (silica gel, 20-30% EtOAc in hexanes as eluent) to afford 0.56 g (94%) of the titled product as white solid. $^1$HNMR (400 MHz, DMSO-$d_6$) δ 8.03 (s, 1H), 5.37 (brs, 2H, $D_2O$ exchangeable), 4.86 (d, *J* = 8.5 Hz, 1H), 3.20 (s, 3H), 2.75 (s, 3H), 2.32-2.28 (m, 1H), 1.07 (d, *J* = 6.5 Hz, 3H), 0.65 (d, *J* = 6.5 Hz, 3H); ESI-MS (m/z) 252.21 $(MH)^+$.

**Example-35:** The following examples were prepared by using the similar procedure described in example-34:

(±)-7-(Methoxy(phenyl)methyl)-2-methylthiazolo[5,4-b]pyridin-6-amine; ESI-MS (m/z) 285.9 $(MH)^+$;

(±)-7-((4-Fluorophenyl)(methoxy)methyl)-2-methylthiazolo[5,4-b]pyridin-6-amine; ESI-MS (m/z) 304.0 $(MH)^+$;

(±)-7-(Cyclopropyl(methoxy)methyl)-2-methylthiazolo[5,4-b]pyridin-6-amine; ESI-MS (m/z) 250.1 $(MH)^+$; and

(±)-7-(Cyclobutyl(methoxy)methyl)-2-methylthiazolo[5,4-b]pyridin-6-amine; ESI-MS (m/z) 264.15 $(MH)^+$.

**Example-36:** Preparation of (±)-7-(1-methoxy-2,2-dimethylpropyl)-2-methylthiazolo [5, 4-b] pyridin-6-amine

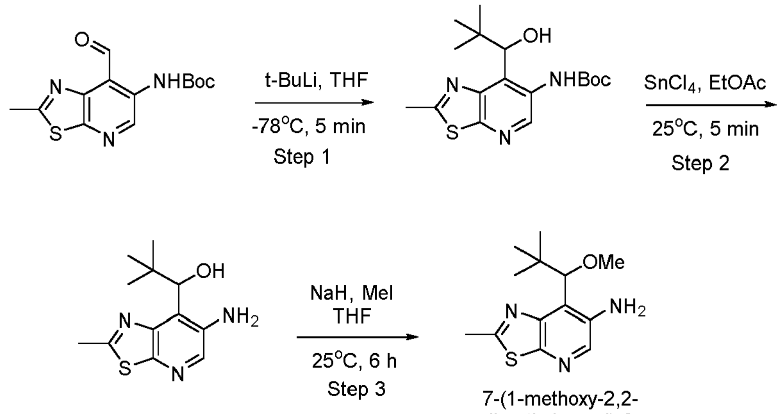


7-(1-methoxy-2,2-dimethylpropyl)-2-methylthiazolo[5,4- *b*]pyridin-6-amine

Step-1: tert-Butyl (7-(1-hydroxy-2,2-dimethylpropyl)-2-methylthiazolo[5,4-b] pyridin-6-yl)carbamate: To a solution of tert-butyl (7-formyl-2-methylthiazolo[5,4-b]pyridin-6-yl)carbamate (2.0 g, 6.82 mmol) in THF (40 mL) was added tert-butyllithium (1.9 M solution in pentane, 4.0 mL, 7.5 mmol) dropwise over a period of 10 min. at -78°C. The resulting

mixture was stirred at -78°C for 5 min. The reaction was quenched with sat. aq. $NH_4Cl$ (20 mL) and ethyl acetate (50 mL), organic layer was separated, dried over anhydrous sodium sulphate and filtered. The filtrate was rotary evaporated and residue was purified by flash column chromatography (silica gel) to afford 0.64 g (27%) of the titled product as a yellow solid. $^1$H NMR (400 MHz, DMSO-$d_6$) δ 9.11 (s, 1H), 8.93 (s, 1H), 6.76 (d, *J* = 5.0 Hz, 1H), 5.47 (d, *J* = 5.0 Hz, 1H), 2.81 (s, 3H), 1.47 (s, 9H), 0.90 (s, 9H); ESI-MS (m/z) 352.41 $(MH)^+$.

Step-2: 1-(6-Amino-2-methylthiazolo[5,4-b]pyridin-7-yl)-2,2-dimethylpropan-1-ol: To a solution of step-1 intermediate (0.5 g, 1.423 mmol) in ethyl acetate (5 mL) was added tin(IV) chloride (0.501 mL, 4.27 mmol) at 25°C and reaction was stirred for 5 min. Upon completion, reaction mixture was quenched with aq. $NaHCO_3$ (20 mL) and diluted with ethyl acetate (50 mL). The reaction mass was filtered through celite bed and organic layer was separated, dried over anhydrous sodium sulphate, concentrated under reduced pressure and the crude residue was purified by flash column chromatography (silica gel) to afford 0.3 g (84%) of the titled product as a white solid. $^1$H NMR (400 MHz, DMSO-$d_6$) δ 7.97 (s, 1H), 5.85 (d, *J* = 4.5 Hz, 1H), 5.52 (s, 2H), 5.36 (d, *J* = 4.6 Hz, 1H), 2.73 (s, 3H), 0.93 (s, 9H). ESI-MS (m/z) 252.1 (MH)+.

Step-3: 7-(1-Methoxy-2,2-dimethylpropyl)-2-methylthiazolo[5,4-b]pyridin-6-amine: To a solution of step-2 intermedaite (0.3 g, 1.591 mmol) in THF (10 mL), sodium hydride (60% in mineral oil, 0.095 g, 2.38 mmol) was added portionwise at 0°C and reaction mixture was stirred for 30 min at 0-10°C . To the reaction mixture, MeI (0.80 mL, 1.430 mmol) was added dropwise at 10°C and reaction was continued to stir for 6 h at 25°C. After completion, the reaction mixture was quenched with sat. $NH_4Cl$ solution (20 mL), extracted with ethyl acetate (25 mL x 3). Organic layer was separated, rotary evaporated and residue was purified by flash column chromatography (silica gel) to afford 0.173 g (55%) of the titled product. $^1$H NMR (400 MHz, DMSO-$d_6$) δ 8.02 (s, 1H), 5.45 (s, 2H), 4.97 (s, 1H), 3.23 (s, 3H), 2.74 (s, 3H), 0.95 (s, 9H); ESI-MS (m/z) 265.9$(MH)^+$.

**Example-37:** Preparation of (±)-2-methyl-7-(2,2,2-trifluoro-1-methoxyethyl)thiazolo[5,4-b]pyridin -6-amine

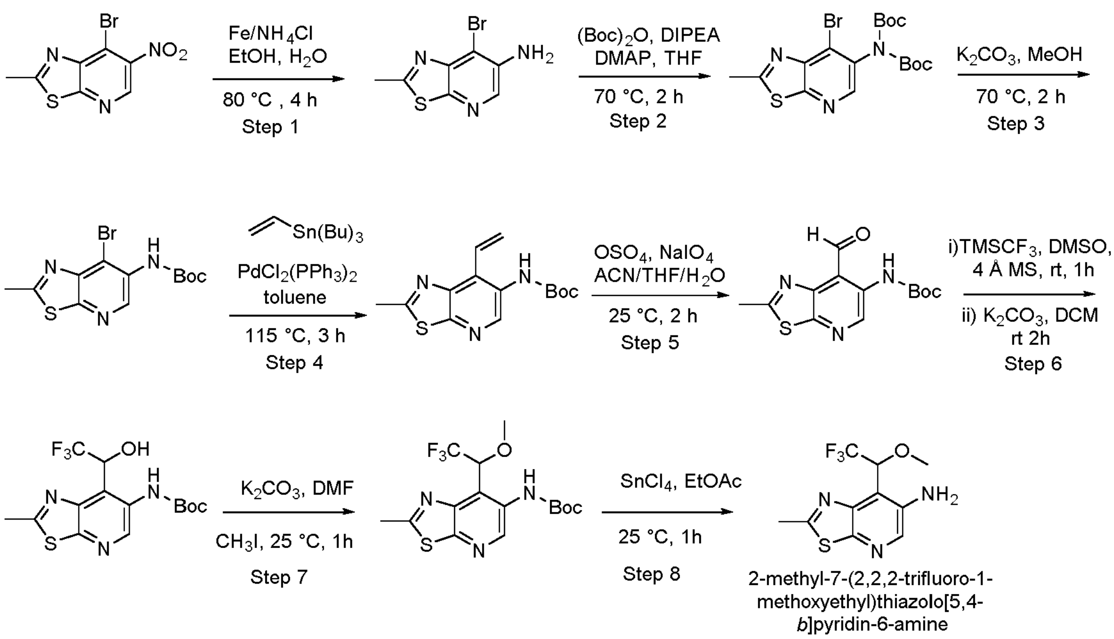

2-methyl-7-(2,2,2-trifluoro-1-methoxyethyl)thiazolo[5,4-*b*]pyridin-6-amine

Step-1: 7-Bromo-2-methylthiazolo[5,4-b]pyridin-6-amine: To a stirred solution of 7-bromo-2-methyl-6-nitrothiazolo[5,4-b]pyridine (10 g, 36.5 mmol) in EtOH (100 mL) was added iron powder (20.37 g, 365 mmol), ammonium chloride (19.52 g, 365 mmol) and $H_2O$ (20 mL). The reaction was heated at 80°C for 4 h. Upon completion, the reaction mixture was cooled to room temperature and filtered through celite bed, and the filtrate was rotary evaporated. Water (50 mL) was added to the residue followed by ethyl acetate (100 mL). The layers were separated and the aqueous layer extracted with ethyl acetate (2 x 100 mL). The combined organic layer was washed with saturated $NaHCO_3$ (20 mL), dried over $Na_2SO_4$ and filtered. The filtrate was rotary evaporated and the solid residue (6.5 g, 75%) was carried forward without purification. $^1$HNMR (400 MHz, DMSO-$d_6$) δ 8.09 (s, 1H), 5.75 (s, 2H), 2.78 (s, 3H); ESI-MS (m/z) 244.0 $(MH)^+$.

Step-2: Di-tert-butyl (7-bromo-2-methylthiazolo[5,4-b]pyridin-6-yl)dicarbamate: To a stirred solution of step-1 intermedaite (6.5 g, 26.6 mmol) in THF (65 mL) was added DIPEA (13.95 mL, 80 mmol), DMAP (0.325 g, 2.66 mmol)

and Di-*tert*-butyl dicarbonate (15.46 mL, 66.6 mmol) simultaneously. The resultant mixture was heated at 70°C for 2 h. The reaction mixture was concentrated under vacuum and the residue was purified by flash column chromatography (silica gel, 15% ethyl acetate-hexane mixture as eluent) to afford 9 g (76%) of the titled compound as a yellow solid. $^1$H NMR (400 MHz, DMSO-$d_6$) δ 8.59 (s, 1H), 2.89 (s, 3H), 1.35 (s, 18H); ESI-MS (m/z) 444.0 $(MH)^+$.

Step-3: tert-Butyl (7-bromo-2-methylthiazolo[5,4-b]pyridin-6-yl)carbamate: To a solution of step-2 intermediate (9 g, 20.25 mmol) in MeOH (90 mL) was added potassium carbonate (9.01 g, 65.2 mmol) and reaction mixture was stirred at 70°C for 2 h. Methanol was evaporated under vacuum and the residue was diluted with ethyl acetate (50 mL) and filtered. Water (50 mL) was added to the filtrate and two layers were separated and the aqueous layer was extracted with ethyl acetate (2 x 100 mL). The combined organic layers was washed with brine (50 mL), dried over ($Na_2SO_4$) and filtered. The filtrate was rotary evaporated to afford the crude product which was washed with hexane (50 mL) to afford pure (4.4 g, 63%) of titled compound as a white solid. $^1$H NMR (400 MHz, DMSO-$d_6$) δ 9.16 (s, 1H), 8.52 (s, 1H), 2.86 (s, 3H), 1.47 (s, 9H); ESI-MS (m/z) 344.0 $(MH)^+$.

Step-4: tert-Butyl (2-methyl-7-vinylthiazolo[5,4-b]pyridin-6-yl)carbamate: To a stirred solution of step-3 intermediate (7.0 g, 20.34 mmol) and tributyl(vinyl)stannane (9.67 g, 30.5 mmol) in toluene (100 mL) was added $PdCl_2(PPh_3)_2$ (1.43 g, 2.03 mmol) and the reaction was stirred at 115°C for 3 h. The reaction mixture was concentrated under reduced pressure and the residue was purified by flash column chromatography (silica gel, 20% ethyl acetate-hexane mixture as eluent) to afford 3.5 g (59%) of the titled compound as a solid. $^1$H NMR (400 MHz, DMSO-$d_6$) δ 9.11 (s, 1H), 8.43 (s, 1H), 6.99 (dd, *J* = 17.7, 11.4 Hz, 1H), 6.87 (dd, *J* = 17.7, 2.5 Hz, 1H), 5.90 (dd, *J* = 11.4, 2.5 Hz, 1H), 2.86 (s, 3H), 1.46 (s, 9H); ESI-MS (m/z) 292.4 $(MH)^+$.

Step-5: tert-Butyl (7-formyl-2-methylthiazolo[5,4-b]pyridin-6-yl)carbamate: Osmium tetroxide (0.305 g, 1.201 mmol) and sodium periodate (7.71 g, 36.0 mmol) were added to a stirred solution of tert-butyl (2-methyl-7-vinylthiazolo[5,4-b]pyridin-6-yl)carbamate (3.5 g, 12.01 mmol) in ACN/THF/Water (1:1:1, 50 mL). The resulting mixture was stirred at 25°C for 2 h. Upon completion, the reaction mixture was cooled to 0°C and water (50 mL) was added followed by ethyl acetate (100 mL). The layers were separated and the aqueous layer was extracted with ethyl acetate (2x50 mL). The combined organic layers were washed with brine (50 mL), dried over ($Na_2SO_4$) and filtered. The filtrate was rotary evaporated and the crude product was purified by flash column chromatography (silica gel, 20% ethyl acetate in hexane as eluent) to afford 2.5 g (71%) of the desired product. $^1$H NMR (400 MHz, DMSO-$d_6$) δ 10.81 (s, 1H), 10.17 (s, 1H), 9.41 (s, 1H), 2.92 (s, 3H), 1.52 (s, 9H). ESI-MS (m/z) 294.1 $(MH)^+$.

Step 6: tert-Butyl (2-methyl-7-(2,2,2-trifluoro-1-hydroxyethyl)thiazolo[5,4-b]pyridin-6-yl)carbamate: To a stirred solution of step-5 intermedaite (2.5 g, 8.52 mmol) in DMSO (15 mL) was added molecular sieves 4 Å and trifluoromethyltrimethylsilane (1.51 mL, 10.23 mmol). The reaction mixture was stirred at 25°C under nitrogen atmosphere. After 1h, potassium carbonate (1.18 g, 8.52 mmol) and DCM (20 mL) was added and reaction was continued to stir for another 2 h. The reaction mixture was quenched with water (5 mL) and extracted with ethyl acetate (25 mL × 3). The combined organic layers were washed with brine (25 mL), dried over ($Na_2SO_4$) and filtered. The filtrate was rotary evaporated and the crude residue was purified by flash column chromatography (silica gel, 30% ethyl acetate-hexane mixture as eluent) to afford 2 g (65%) of the desired product as solid. $^1$H NMR (400 MHz, DMSO-$d_6$) δ 9.08 (s, 1H), 8.64 (s, 1H), 8.34 (d, *J = 6.2* Hz, 1H), 6.23 - 6.11 (m, 1H), 2.86 (s, 3H), 1.48 (s, 9H); ESI-MS (m/z) 364.3 $(MH)^+$.

Step-7: tert-Butyl (2-methyl-7-(2,2,2-trifluoro-1-methoxyethyl)thiazolo[5,4-b]pyridin-6-yl)carbamate: To a stirred solution of step-6 intermediate (2 g, 5.50 mmol) in DMF (10 mL) was added potassium carbonate (0.913 g, 6.61 mmol) and MeI (0.413 mL, 6.61 mmol). The resulting mixture was stirred at 25°C under nitrogen atmosphere. After 1h, the reaction mixture was quenched with water (10 mL) and extracted with ethyl acetate (25 mL × 3). The combined organic layers were washed with water (25 mL), brine (25 mL), dried over ($Na_2SO_4$) and filtered. The filtrate was rotary evaporated and the crude residue was purified by flash column chromatography (silica gel, 30% ethyl acetate-hexane mixture as eluent) to afford 1.7 g (82%) of the desired product as solid. $^1$H NMR (400 MHz, DMSO-$d_6$) δ 8.97 (s, 1H), 8.29 (s, 1H), 5.98 (q, *J* = 7.5 Hz, 1H), 3.54 (s, 3H), 2.88 (s, 3H), 1.48 (s, 9H); ESI-MS (m/z) 378.2 $(MH)^+$.

Step-8: 2-methyl-7-(2,2,2-trifluoro-1-methoxyethyl)thiazolo[5,4-b]pyridin-6-amine: Tin(IV) chloride (2.23 ml, 19.08 mmol) was added dropwise to a solution of tert-butyl (2-methyl-7-(2,2,2-trifluoro-1-methoxyethyl)thiazolo[5,4-b]pyridin-6-yl)carbamate (1.8 g, 4.77 mmol) in ethyl acetate (5 mL) under nitrogen atmosphere and the resulting mixture was stirred for 1h at room temperature. After completion, reaction mixture was quenched with aq. $NaHCO_3$ (20 mL) and diluted with ethyl acetate (50 mL). The reaction mass was filtered through celite bed and organic layer was separated and dried over anhydrous sodium sulphate. The crude residue was then purified by flash column chromatography (silica gel, 30% ethyl acetate-hexane mixture as eluent) to afford 1 g (76%) of the titled product as solid compound. $^1$HNMR (400 MHz, DMSO-$d_6$) δ 8.13 (s, 1H), 5.84 (q, *J* = 7.7 Hz, 1H), 5.66 (s, 2H), 3.44 (s, 3H), 2.78 (s, 3H); ESI-MS (m/z) 278.2 $(MH)^+$.

**Example-38:** Preparation of 4-(Difluoromethoxy)-3-(trifluoromethyl)aniline

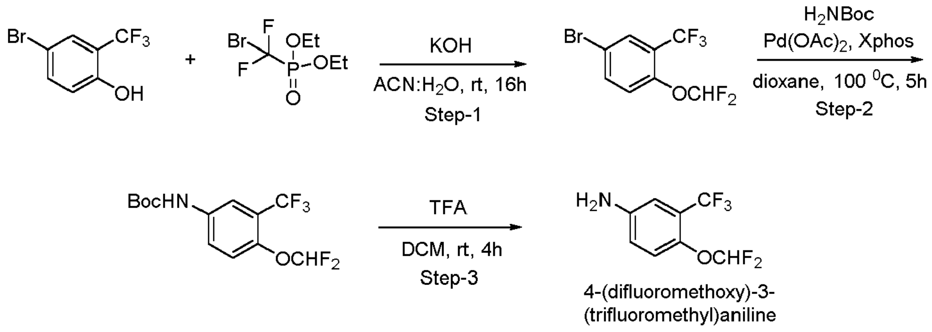


Step-1: 4-Bromo-1-(difluoromethoxy)-2-(trifluoromethyl)benzene: To a stirred solution of 4-bromo-2-(trifluoromethyl)phenol (1.0 g, 4.15 mmol) in acetonitrile (25 mL) diethyl (bromodifluoromethyl)phosphonate (2.216 g, 8.30 mmol) was added at 0°C. After stirring for 15 min at the same temperature, a solution of potassium hydroxide (2.32 g, 41.5 mmol) in water (25.0 mL) was added dropwise. The resulting mixture was then stirred at 25°C for 16 h. Reaction mixture was then poured onto ice water followed by the addition of ethyl acetate (15 mL). The layers were separated, and the aqueous layer was extracted with ethyl acetate (2×20 mL). The combined organic layers were washed with brine (20 mL), dried ($Na_2SO_4$) and filtered. The filtrate was rotary evaporated and the crude product was purified by flash column chromatography (silica gel) to afford 0.7 g (58%) of the titled product. $^1$HNMR (400 MHz, DMSO-$d_6$) δ 8.04-7.96 (m, 2H), 7.64-7.21 (m, 2H); GC-MS 289.84 $(M)^+$.

Step-2: tert-Butyl (4-(difluoromethoxy)-3-(trifluoromethyl)phenyl)carbamate: To a stirred solution of step-1 intermediate (0.5 g, 1.718 mmol) in dioxane (10 mL) was added tert-butyl carbamate (0.302 g, 2.58 mmol), $Pd(OAc)_2$ (0.039 g, 0.172 mmol), XPhos (0.082 g, 0.172 mmol) and $Cs_2CO_3$ (1.120 g, 3.44 mmol) sequentially. The reaction was stirred at 100°C for 5 h. The reaction mixture was cooled to room temperature and then filtered through celite. The celite bed was washed thoroughly with ethyl acetate (20 mL) and the combined filtrates were evaporated under vacuum. The crude product was then purified by flash column chromatography to get 0.35 g (62%) of the titled compound. $^1$HNMR (400 MHz, DMSO-$d_6$) δ 9.76 (s, 1H), 7.95 (s, 1H), 7.71 (d, *J* = 9.0 Hz, 1H), 7.48-7.01 (m, 2H), 1.52-1.45 (s, 9H).

Step-3: 4-(Difluoromethoxy)-3-(trifluoromethyl)aniline: To a stirred solution of step-2 intermmediate (0.2 g, 0.611 mmol) in DCM (5.0 mL) was added TFA (0.141 mL, 1.833 mmol) at 0°C. The resulting mixture was stirred at rt for 4 h. The reaction was then diluted with ethyl acetate (10 mL) and basified with saturated aqueous sodium bicarbonate solution. The layers were separated and the aqueous layer was extracted with ethyl acetate (3×10 mL). The combined organic layers were washed with brine (10 mL), dried ($Na_2SO_4$) and filtered. The filtrate was rotary evaporated to afford 0.12 g (86%) of the titled compound. $^1$H NMR (400 MHz, DMSO-$d_6$) δ 7.10 (d, *J* = 8.5 Hz, 1H), 7.03 (t, *J* = 74.0 Hz, 1H), 6.90 (d, *J* = 2.5 Hz, 1H), 6.81 (dd, *J* = 8.5, 2.5 Hz, 1H), 5.56 (s, 2H); GCMS 227.07 (M)+.

**Example-39:** Preparation of 3-chloro-4-(1,3,4-oxadiazol-2-yl)aniline

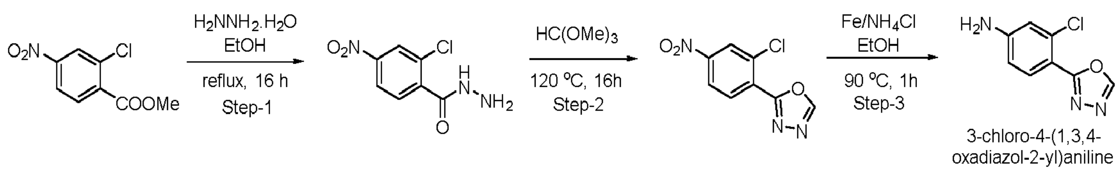


Step-1: 2-Chloro-4-nitrobenzohydrazide: To a stirred solution of methyl 2-chloro-4-nitrobenzoate (3 g, 13.92 mmol) in ethanol (30 mL), hydrazine hydrate (2.09 mL, 41.7 mmol) was added at r.t. The resulting mixture was refluxed at 80°C for 16 h. The solvent was concentrated under vacuum to get the 2.50g (83%) of the desired product as white solid. $^1$HNMR (400 MHz, DMSO-$d_6$) δ 9.81 (s, 1H), 8.35 (d, *J*= 2.0 Hz, 1H), 8.23 (dd, *J* = 8.5, 2.0 Hz, 1H), 7.68 (d, *J* = 8.5 Hz, 1H), 4.61 (s, 2H); ESI-MS (m/z) 216.14 $(MH)^+$.

Step-2: 2-(2-Chloro-4-nitrophenyl)-1,3,4-oxadiazole: A mixture of 2-chloro-4-nitrobenzohydrazide (0.5 g, 2.32 mmol) in trimethylorthoformate (7.72 mL, 46.4 mmol) was heated at 120°C for 16 h. The reaction mixture was concentrated under vacuum and the crude product was purified by columnn chromatography (silica gel) to afford 0.3 g (57%) the desired product. $^1$HNMR (400 MHz, DMSO-$d_6$) δ 9.58 (s, 1H), 8.54 (d, *J* = 2.0 Hz, 1H), 8.39 (dd, *J* = 8.5, 2.0 Hz, 1H), 8.31 (d, *J* = 8.5 Hz, 1H); ESI-MS (m/z) 225.78 $(MH)^+$.

Step-3: 3-Chloro-4-(1,3,4-oxadiazol-2-yl)aniline: To a stirred solution of 2-(2-chloro-4-nitrophenyl)-1,3,4-oxadiazole (0.25 g, 1.11 mmol) in ethanol (10 mL) was added iron powder (0.31 g, 5.54 mmol) followed by a solution of ammonium chloride (0.296 g, 5.54 mmol) in water (2.0 mL). The resulting mixture was stirred at 90°C for 1 h. The solvent was concentrated under vacuum and the residue was diluted with ethyl acetate (5 mL) and filtered through celite. The filtrate was evaporated under vacuum and the crude product was purified by flash column chromatography (silica gel) to afford 0.15 g (69%) of the titled compound as white solid. $^1$HNMR (400 MHz, DMSO-$d_6$) $\delta$ 9.25 (s, 1H), 7.64 (d, *J* = 8.5 Hz, 1H), 6.76 (d, *J* = 2.0 Hz, 1H), 6.64 (dd, *J* = 8.5, 2.0 Hz, 1H), 6.19 (s, 2H); MS (m/z) 195.71 (MH)$^+$.

**Example-40:** Preparation of 5-chloro-2-methoxypyridin-3-amine

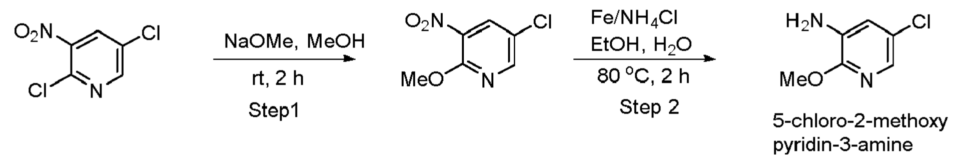


5-chloro-2-methoxy pyridin-3-amine

Step-1: 5-Chloro-2-methoxy-3-nitropyridine: To a (0°C) cooled and stirred solution of 2,5-dichloro-3-nitropyridine (3.0 g, 15.55 mmol) in MeOH (60 mL) was added dropwise sodium methoxide (5M in methanol, 31.1 mL, 155 mmol) and the reaction was allowed to stir at 25°C for 2 h. Upon completion, cold water (10 mL) was added and the mixture was extracted with EtOAc (2×50 mL), dried over $Na_2SO_4$, filtered and rotary evaporated to afford 5-chloro-2-methoxy-3-nitropyridine (2.81 g, 96%). $^1$HNMR (400 MHz, DMSO-$d_6$) $\delta$ 8.64 (d, *J* = 2.4 Hz, 1H), 8.62 (d, *J* = 2.4 Hz, 1H), 4.03 (s, 3H); ESI-MS (m/z) 188.9 (MH)$^+$.

Step-2: 5-Chloro-2-methoxypyridin-3-amine: To a stirred solution of 5-chloro-2-methoxy-3-nitropyridine (2.8 g, 14.85 mmol) in EtOH (50 mL) was added iron powder (10.78 g, 193 mmol), ammonium chloride (10.33 g, 193 mmol) and $H_2O$ (18.7 mL). The reaction was heated at 80°C for 2 h. The reaction mixture was cooled to room temperature and filtered through celite bed, and the filtrate was rotary evaporated. Water (25 mL) was added to the residue followed by ethyl acetate (50 mL). The layers were separated and the aqueous layer extracted with ethyl acetate (2x25 mL). The combined organic layers was washed with saturated $NaHCO_3$ (25 mL), dried over $Na_2SO_4$ and filtered. The filtrate was rotary evaporated and the crude product was purified by flash column chromatography (silica gel, hexane/EtOAc (60:40) as eluent) to afford 5-chloro-2-methoxypyridin-3-amine (1.85 g, 79%). $^1$HNMR (500 MHz, DMSO-$d_6$) $\delta$ 7.32 (d, *J* = 2.4 Hz, 1H), 6.88 (d, *J* = 2.4 Hz, 1H), 5.31 (s, 2H), 3.85 (s, 3H); ESI-MS (m/z) 158.9 (MH)$^+$.

**Example-41:** Preparation of 5-chloro-2,6-dimethoxypyridin-3-amine

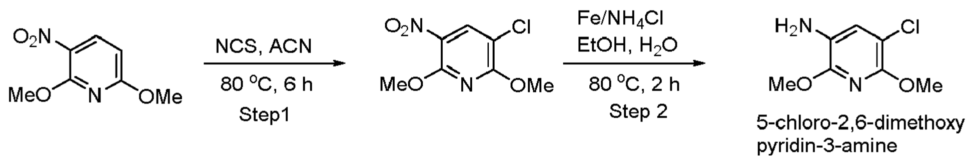


5-chloro-2,6-dimethoxy pyridin-3-amine

Step-1: 3-Chloro-2,6-dimethoxy-5-nitropyridine: To a stirred solution of 2,6-dimethoxy-3-nitropyridine (5 g, 27.2 mmol) in acetonitrile (60 mL) was added N-chloro succinimide and reaction was allowed to stir at 80°C for 6 h. After cooling to room temperature, reaction was quenched by addition of water (25 mL). The reaction mixture was extracted by ethyl acetate (2 x 50 mL). The combined organic layer was washed with 10% aqueous sodium bisulfite solution (25 mL), dried over $Na_2SO_4$ and filtered. The filtrate was rotary evaporated and the crude product was purified by flash column chromatography (silica gel, hexane/EtOAc (80:20) as eluent) to afford 3-chloro-2,6-dimethoxy-5-nitropyridine (2 g, 33%). $^1$HNMR (400 MHz, DMSO-$d_6$) $\delta$ 8.73 (s, 1H), 4.21 (s, 3H), 4.20 (s, 3H); ESI-MS (m/z), 218.8(MH)$^+$.

Step-2: 5-Chloro-2,6-dimethoxypyridin-3-amine: To a stirred solution of 3-chloro-2,6-dimethoxy-5-nitropyridine (1 g, 4.57 mmol) in EtOH (20 mL) was added iron powder (3.32 g, 59.5 mmol), ammonium chloride (3.18 g, 59.5 mmol) and $H_2O$ (5.8 mL). The reaction was heated at 80°C for 2 h. The reaction mixture was cooled to room temperature and filtered through celite bed, and the filtrate was rotary evaporated. Water (25 mL) was added to the residue followed by ethyl acetate (50 mL). The layers were separated and the aqueous layer extracted with ethyl acetate (2x25 mL). The combined organic layers was washed with saturated $NaHCO_3$ (25 mL), dried over $Na_2SO_4$ and filtered. The filtrate was rotary evaporated to 5-chloro-2,6-dimethoxypyridin-3-amine (0.6 g, 69.5%). $^1$HNMR (400 MHz, DMSO-$d_6$) $\delta$ 7.16 (s, 1H), 4.72 (s, 2H), 4.00 (s, 3H), 3.95 (s, 3H).

**Example-42:** Preparation of 5-chloro-6-(isoxazol-4-yl)pyridin-3-amine

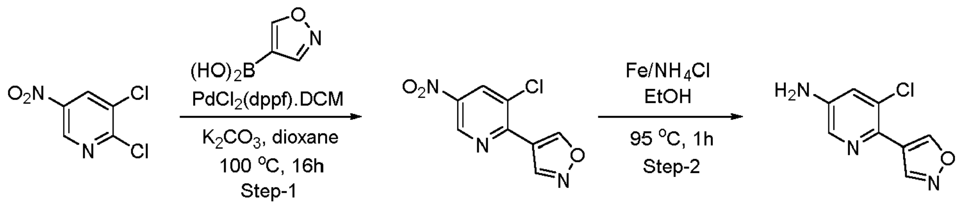


5-chloro-6-(isoxazol-4-yl)pyridin-3-amine

Step-1: 4-(3-Chloro-5-nitropyridin-2-yl)isoxazole: To a solution of 2,3-dichloro-5-nitropyridine (0.5 g, 2.59 mmol) and isoxazol-4-ylboronic acid (0.292 g, 2.59 mmol) in dioxane (10 mL) and water (2 mL) was added $K_2CO_3$ (0.716 g, 5.18 mmol). The resulting mixture was thoroughly deoxygenated by purging nitrogen for 30 min and then $PdCl_2$(dppf)-$CH_2Cl_2$ adduct (0.212 g, 0.259 mmol) was addded. The resulting mixture was heated at 100°C for 16 h. The reaction was cooled to room temperature and filtered through celite. The filtrate was concentrated under vacuum and the crude product was purified by flash column chromatography (silica gel) to afford 0.11 g (19%) of the titled compound as a yellow solid.ESI-MS (m/z) 225.75 $(MH)^+$.

Step 2: 5-chloro-6-(isoxazol-4-yl)pyridin-3-amine: To a stirred solution of 4-(3-chloro-5-nitropyridin-2-yl)isoxazole (0.110 g, 0.488 mmol) in EtOH (5 mL) was added iron powder (0.272 g, 4.88 mmol) and then a solution of ammonium chloride (0.261 g, 4.88 mmol) in water (2 mL) at RT. The reaction mass was heated at 95°C and stirred for 1h. Reaction mass was diluted with ethyl acetate (5 mL) and water (5 mL). The layers were separated and the organic layer was extracted with ethyl acetate (2x5 mL). The combined organic layers were washed with water (5 mL), brine (5 mL), dried ($Na_2SO_4$) and filtered. The filtrate was rotary evaporated to afford 90 mg (94%) of the titled compound. ESI-MS (*m*/*z*) 195.95 $(MH)^+$.

**Example-43:** Preparation of 3-chloro-4-(pyrazin-2-yl)aniline

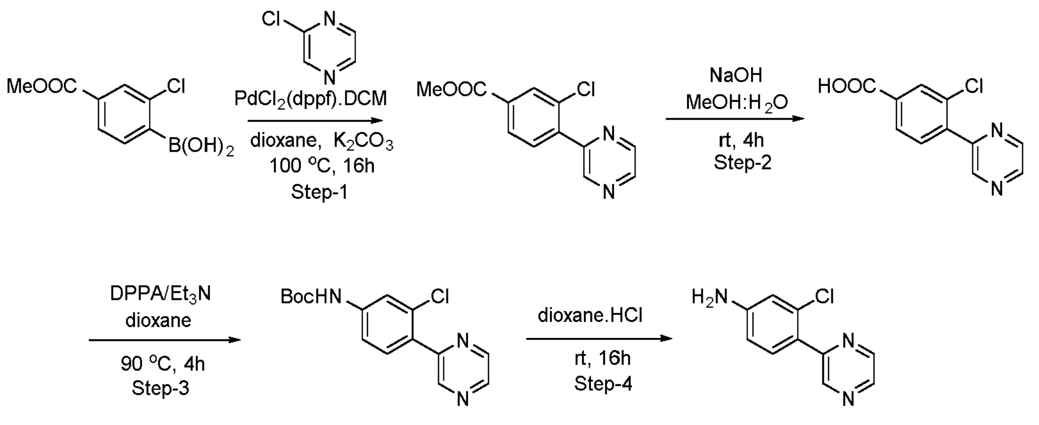


Step-1: Methyl 3-chloro-4-(pyrazin-2-yl)benzoate: To a solution of (2-chloro-4-(methoxycarbonyl)phenyl)boronic acid (2.05 g, 9.60 mmol) in dioxane (10 mL) was added 2-chloropyrazine (0.78 mL, 8.73 mmol) and $K_2CO_3$ (2.41 g, 17.46 mmol). The resulting mixture was thoroughly deoxygenated by subjecting to nitrogen cycle three times and then $PdCl_2$(dppf)-$CH_2Cl_2$ adduct (0.71 g, 0.873 mmol) was added and the resulting mixture was heated at 100°C for 16 h. The reaction was cooled to room temperature and filtered through celite. The filtrate was concentrated under vacuum and the crude product was purified by flash column chromatography (silica gel) to afford 1.0 g (46%) of the titled compound as a white solid. ESI-MS (m/z) 248.84 (MH)+.

Step-2: 3-Chloro-4-(pyrazin-2-yl)benzoic acid: To a stirred solution of step-1 intermediate (1.0 g, 4.02 mmol) in MeOH (10 mL) & water (2 mL) was added NaOH (0.241 g, 6.03 mmol) at 0°C and the reaction mixture was stirred at room temperature for 4 h. The reaction was cooled to room temperature and the solvent was evaporated under vacuum. Water (10 mL) was added to the reaction and pH was adjusted to 1 using 10% aq.HCl, followed by addition of ethyl

acetate (20 mL). The layers were separated and aqueous layer was extracted with ethyl acetate (2×20 mL). The combined organic layers were washed with brine (20 mL), dried ($Na_2SO_4$) and filtered. The filtrate was rotary evaporated to afford 0.8 g (85%) as a white solid. $^1$HNMR (400 MHz, DMSO-*d*$_6$) δ 13.52 (s, 1H), 9.01 (s, 1H), 8.83 (d, *J* = 2.5 Hz, 1H), 8.75 (d, *J* = 2.5 Hz, 1H), 8.09 (s, *J* = 2.0 Hz, 1H), 8.04 (d, *J* = 8.0 Hz, 1H), 7.80 (dd, *J* = 8.0, 2.0 Hz, 1H); ESI-MS (m/z) 235.08 (MH)$^+$.

Step-3: tert-Butyl (3-chloro-4-(pyrazin-2-yl)phenyl)carbamate: To a stirred solution of step-2 intermediate (0.8 g, 3.41 mmol) in *tert*-butanol (10 mL) was added $Et_3N$ (0.95 mL, 6.82 mmol) and [azido(phenoxy)phosphoryl]oxybenzene (0.813 mL, 3.75 mmol). The resulting mixture was stirred at room temperature for 5 min and then heated to 90°C and stirred for 4h. The reaction was cooled to room temperature and the solvent was evaporated under vacuum and the crude product was purified by flash column chromatography (silica gel, 20% ethyl acetate in hexane) to afford 0.540 g (52%) as a white solid. ESI-MS (m/z) 305.97 (MH)$^+$.

Step-4: 3-Chloro-4-(pyrazin-2-yl)aniline: To a stirred solution of step-3 intermediate (0.540 g, 1.766 mmol) in dioxane (2 mL) was added 4M HCl in dioxane (4.42 mL, 17.66 mmol,). The reaction mixture was stirred at room temperature for 16h. The solvent was evaporated and azeotropped with toluene followed by washing with diethyl ether. Ethyl acetate (10 mL) was added to the above obtained residue followed by the addition of saturated solution of sodium bicarbonate (5 mL) and the pH was adjusteed to 9-10. The layers were separated and aqueous layer was extracted with ethyl acetate (2×10 mL). The combined organic layers were washed with brine (10 mL), dried ($Na_2SO_4$) and filtered. The filtrate was rotary evaporated to afford 0.3 g, 83%) as yellow solid. $^1$HNMR (400 MHz, DMSO-*d*$_6$) δ 8.86 (s, 1H), 8.68 (d, *J* = 2.5 Hz, 1H), 8.53 (d, *J* = 2.5 Hz, 1H), 7.36 (d, *J* = 8.5 Hz, 1H), 6.74 (d, *J* = 2.0 Hz, 1H), 6.65 (dd, *J* = 8.5, 2.0 Hz, 1H), 5.81 (s, 2H); ESI-MS (m/z) 206.26 (MH)$^+$.

**Example-44:** The below compound was prepared by following the similar procedure described in example-43: 3-Chloro-4-(pyrimidin-2-yl)aniline; ESI-MS (m/z) 206.04 (MH)$^+$.

**Example-45:** Preparation of 3-Chloro-4-(3-(methoxymethyl)-5-methyl-1H-pyrazol-1-yl)aniline

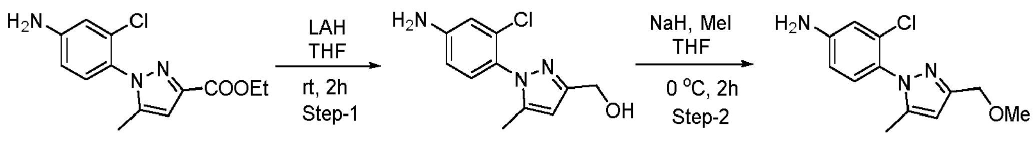


3-chloro-4-(3-(methoxymethyl)-5-methyl-1*H*-pyrazol-1-yl)aniline

Step-1: (1-(4-Amino-2-chlorophenyl)-5-methyl-1H-pyrazol-3-yl)methanol: To a stirred and (0 °C) cooled suspension of lithium aluminium hydride (0.204 g, 5.36 mmol) in THF (5 mL) was added dropwise a solution of ethyl 1-(4-amino-2-chlorophenyl)-5-methyl-1H-pyrazole-3-carboxylate (1.0 g, 3.57 mmol) in THF (5 mL). After stirring the reaction mixture at rt for 2 h, reaction mixture was quenched with ice cold water and filtered through celite. The filtrate was evaporated under reduced pressure to give the crude product which was purified by flash column cromatgraphy to give 0.8 g (94%) as off white solid. $^1$HNMR (400 MHz, DMSO-*d*$_6$) δ 7.04 (d, *J* = 8.5 Hz, 1H), 6.73 (d, *J* = 2.5 Hz, 1H), 6.57 (dd, *J* = 8.5, 2.5 Hz, 1H), 6.13 (s, 1H), 5.74 (s, 2H), 5.02 (t, *J* = 5.5 Hz, 1H), 4.38 (d, *J* = 5.5 Hz, 2H), 2.02 (s, 3H); ESI-MS (m/z) 237.84 (MH)$^+$.

Step-2: 3-Chloro-4-(3-(methoxymethyl)-5-methyl-1H-pyrazol-1-yl)aniline: To a (0°C) cooled and stirred suspension of sodium hydride (0.101 g, 2.52 mmol) in dry THF (3 mL) was added dropwise a solution of (1-(4-amino-2-chlorophenyl)-5-methyl-1H-pyrazol-3-yl)methanol (0.3 g, 1.262 mmol) in dry THF (5 mL). The resulting mixture was stirred for 15 min at 0°C. Methyl iodide (0.158 mL, 2.52 mmol) was then added dropwise at the same teparature to the above mixture and the resulting mixture was then stirred at the same temperaturee for 2 h. The reaction mixture was quenched with ice cold water and extracted with EtOAc (3x10 mL). The combined orginic layers were washed with brine (10 mL), dried ($Na_2SO_4$) and filtered. The filtrate was rotary evaporated and the crude product was purified by flash column chromatography (silica gel) to give 0.2 g (63%) of the titled product as off white solid. $^1$HNMR (400 MHz, DMSO-*d*$_6$) δ 7.06 (d, *J* = 8.5 Hz, 1H), 6.73 (d, *J* = 2.5 Hz, 1H), 6.58 (dd, *J* = 8.5, 2.5 Hz, 1H), 6.15 (s, 1H), 5.76 (s, 2H), 4.30 (s, 2H), 3.25 (s, 3H), 2.03 (s, 3H); ESI-MS (m/z) 251.83 (MH)$^+$.

**Example-46:** Preparation of 5-chloro-6-(5-methyloxazol-2-yl)pyridin-3-amine.

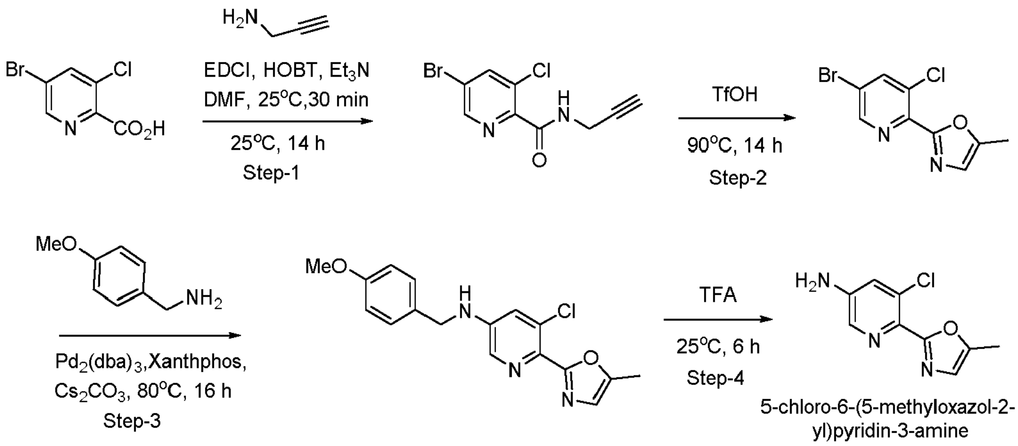

5-chloro-6-(5-methyloxazol-2-yl)pyridin-3-amine

Step-1: 5-Bromo-3-chloro-N-(prop-2-yn-1-yl)picolinamide: To a stirred solution of 5-bromo-3-chloropicolinic acid (10 g, 42.3 mmol) in DMF (100 mL) was added HOBT (3.24 g, 21.15 mmol), EDCI (6.57 g, 42.3 mmol) and $Et_3N$ (11.79 mL, 85 mmol) and the reaction mixture was allowed to stir for 30 min. Thereafter prop-2-yn-1-amine (3.30 mL, 51.6 mmol) was added and reaction mixture was allowed to stir for 14 h at 25°C. Upon completion, reaction mixture was quenched with water (500 mL) and aqueous phase was extracted with ethyl acetate (200 mL x 3), combined organic layer was dried over anhydrous sodium sulphate and filtered. The filtrate was rotary evaporated and residue was purified by flash column chromatography (silica gel) to afford 3.50 g (30%) of the titled product as a white solid. $^1H$ NMR (400 MHz, DMSO-$d_6$) $\delta$ 9.12 (t, $J$ = 6.0 Hz, 1H), 8.73 (s, 1H), 8.48 (s, 1H), 4.12 - 3.98 (m, 2H), 3.17 (s, 1H). ESI-MS (m/z) 274.9 $(MH)^+$.

Step-2: 2-(5-bromo-3-chloropyridin-2-yl)-5-methyloxazole: In a 25 mL sealed tube containing a solution of step-1 intermediate (1.8 g, 6.58 mmol) in dichloromethane (10 mL) was added triflic acid (5.84 mL, 65.8 mmol) dropwise at 25°C and the reaction was heated at 90°C for 14 h. The solvent was removed under reduced pressure and the residue was dissolved in water (20 mL) and neutralized with sat. aq. $NaHCO_3$ solution (20 mL). Aqueous phase was extracted with ethyl acetate (20 mL x 3). Combined organic layer was washed with brine (20 mL), dried over anhydrous sodium sulphate and filtered. The filtrate was rotary evaporated and residue was purified by flash column chromatography (silica gel) to afford 1.60 g (89%) of the titled product as a white solid. $^1H$ NMR (400 MHz, DMSO-$d_6$) $\delta$ 8.82 (s, 1H), 8.54 (s, 1H), 7.17 (s, 1H), 2.09 (s, 3H). ESI-MS (m/z) 273.13 $(MH)^+$.

Step-3: 5-Chloro-N-(4-methoxybenzyl)-6-(5-methyloxazol-2-yl)pyridin-3-amine: To a stirred solution of step-2 intermediate (1.55 g, 5.67 mmol) and (4-methoxyphenyl)methanamine (0.740 ml, 5.67 mmol) in toluene (100 mL), $Pd_2(dba)_3$ (1.55 g, 1.69 mmol), xantphos (0.492 g, 0.850 mmol) and $Cs_2CO_3$ (2.77 g, 8.50 mmol) were added under nitrogen purging. The reaction was heated to 80°C for 16 h. Upon completion, reaction mixture allowed to cool to room temperature, diluted with diethyl ether (400 mL) and washed with brine (100 mL x 2). Organic phase was dried over anhydrous sodium sulphate and filtered. The filtrate was rotary evaporated and residue was carried forward without purification. ESI-MS (m/z) 330.28 $(MH)^+$.

Step-4: 5-Chloro-6-(5-methyloxazol-2-yl)pyridin-3-amine: To a stirred solution of step-3 intermediate (1.87 g) in DCM (50 mL), TFA (20 mL) was added dropwise and the reaction was stirred for 6 h at 25°C. The solvent was removed under reduced pressure and the residue was diluted with water (20 mL), ethyl acetate (20 mL) and neutralized with sat. aq. $NaHCO_3$ (20 mL). Aqueous phase was extracted with ethyl acetate (20 mL x 3). Combined organic layer was washed with brine (20 mL), dried over anhydrous sodium sulphate and filtered. The filtrate was rotary evaporated and residue was purified by flash column chromatography (silica gel) to afford 0.4 g (34% over two steps) of the titled product. $^1H$NMR (400 MHz, DMSO-$d_6$) $\delta$ 7.97 (s, 1H), 7.07 (s, 1H), 6.96 (s, 1H), 6.16 (s, 2H), 2.35 (s, 3H); MS (m/z) 210.33 $(MH)^+$.

**Example-47:** Preparation of 3-(2H-1,2,3-triazol-2-yl)-5-(trifluoromethyl)aniline

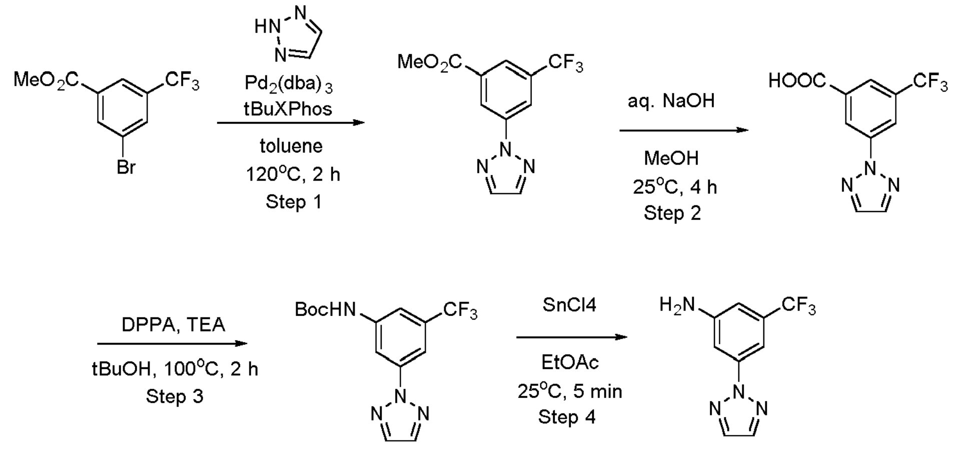


3-(2*H*-1,2,3-triazol-2-yl)-5-(trifluoromethyl)aniline

Step-1: Methyl 3-(2H-1,2,3-triazol-2-yl)-5-(trifluoromethyl)benzoate: To a stirred solution of methyl 3-bromo-5-(trifluoromethyl)benzoate (2.5 g, 8.83 mmol), 2H-1,2,3-triazole (0.732 g, 10.60 mmol) and $K_2HPO_4$ (3.08 g, 17.67 mmol) in toluene (25 mL) was added $Pd_2(dba)_3$ (0.607 g, 0.662 mmol) and di-tert-butyl-[2-[2,4,6-tri(propan-2-yl)phenyl]phenyl]phosphane (0.563 g, 1.325 mmol) under nitrogen purging. The reaction mixture was heated under stirring at 120°C for 2 h. Upon completion, reaction mixture was filtered through celite bed, and the bed was washed with ethyl acetate (200 mL). The filtrate was rotary evaporated and residue was purified by flash column chromatography (silica gel) to afford 1.3 g (54%) of the titled product. $^1$H NMR (400 MHz, DMSO-d6) $\delta$ 8.78 (t, *J* = 1.8 Hz, 1H), 8.53 (d, *J* = 2.0 Hz, 1H), 8.29 (s, 2H), 8.23-8.21(m, 1H), 3.97 (s, 3H); ESI-MS (m/z) 271.87 $(MH)^+$.

Step-2: 3-(2H-1,2,3-Triazol-2-yl)-5-(trifluoromethyl)benzoic acid: To a solution of step-1 intermediate (1.0 g, 3.69 mmol) in MeOH (10 mL), was added aq. NaOH (2.458 mL, 7.37 mmol) and reaction mixture was stirred for 4 h at 25°C. Upon completion of the reaction, solvent was evaporated, and the residue thus obtained was washed with ether (25 mL), dissolved in water (10 mL) and acidified with 10% aq. HCl until pH 2-3. Resulting precipitate was extracted with ethyl acetate (20 mL x 3), combined organic layer was dried over anhydrous sodium sulphate and filtered. The filtrate was rotary evaporated and residue was purified by flash column chromatography (silica gel) to afford 0.5 g (53%) of the titled product. $^1$HNMR (400 MHz, DMSO-$d_6$) $\delta$ 13.92 (s, 1H), 8.76 (t, *J* = 1.8 Hz, 1H), 8.48 (d, *J* = 2.1 Hz, 1H), 8.26 (s, 2H), 8.23-8.21(m, 1H); ESI-MS (m/z) 257.82 $(MH)^+$.

Step-3: tert-Butyl (3-(2H-1,2,3-triazol-2-yl)-5-(trifluoromethyl)phenyl)carbamate: To a solution of step-2 intermediate (0.4 g, 1.55 mmol) in tert-butanol (2.231 mL, 23.33 mmol) was added DPPA (0.368 mL, 1.711 mmol) and $Et_3N$ (0.650 mL, 4.67 mmol). Reaction mass was stirred at 100°C for 2h. Upon completion, the solvent was evaporated, diluted with water (25 mL) and extracted with ethyl acetate (25 mL x 3). Combined organic layer was dried over anhydrous sodium sulphate and filtered. The filtrate was rotary evaporated and residue was purified by flash column chromatography (silica gel) to afford 0.25 g (49%) of the titled product. $^1$H NMR (400 MHz, DMSO-$d_6$) $\delta$ 10.09 (s, 1H), 8.55 (t, *J*= 2.0 Hz, 1H), 8.20 (s, 2H), 7.90 - 7.83 (m, 2H), 1.51 (s, 9H).

Step-4: 3-(2H-1,2,3-Triazol-2-yl)-5-(trifluoromethyl)aniline: To a stirred solution of step-3 intermediate (0.25 g, 0.762 mmol) in ethyl acetate (5 mL), was added SnCl4 (0.36 mL, 3.05 mmol) at 25°C and reaction mixture was stirred at 25°C for 5 min. Thereafter reaction was quenched with aq. $NaHCO_3$ solution (20 mL), extracted with ethyl acetate (20 mL x 3). Combined organic layer was dried over anhydrous sodium sulphate and filtered. The filtrate was rotary evaporated and residue was purified by flash column chromatography (silica gel) to afford 0.130 g (75%) of the titled product. $^1$H NMR (400 MHz, DMSO-$d_6$) $\delta$ 8.13 (s, 2H), 7.52 (t, *J* = 2.1 Hz, 1H), 7.36-7.34 (m, 1H), 6.90-6.87 (m, 1H), 6.08 (s, 2H); ESI-MS (m/z) 229.33 $(MH)^+$.

**Example-48:** Preparation of 3-chloro-5-(5-methyl-1,2,4-oxadiazol-3-yl)aniline.

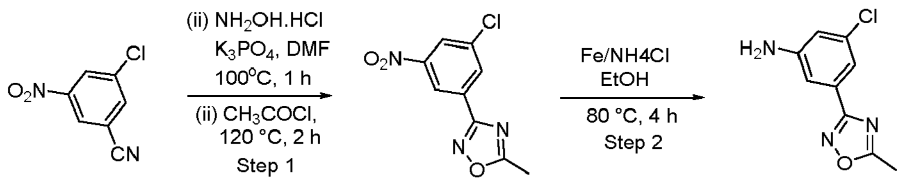


3-chloro-5-(5-methyl-1,2,4-oxadiazol-3-yl)aniline

Step-1: 3-(3-Chloro-5-nitrophenyl)-5-methyl-1,2,4-oxadiazole: To a stirred solution of 3-chloro-5-nitrobenzonitrile (2.0 g, 10.96 mmol) in DMF (20 mL) was added hydroxylamine hydrochloride (0.91 g, 13.15 mmol) and $K_3PO_4$ (3.49 g, 16.43 mmol). The resulting mixture was heated at 100°C for 1 h. After complete conversion to the corresponding amidoxime as indicated by TLC monitoring, acetyl chloride (0.78 ml, 10.96 mmol) was added dropwise and the reaction mixture was heated at 120°C for 2 h. Upon completion, the hot mixture was poured onto crushed ice. The solid obtained was filtered and purified by flash column chromatography (silica gel, 10% ethyl acetate in hexane as eluent) to afford (0.81 g, 31%) of the titled compound as solid. $^1$H NMR (400 MHz, DMSO-$d_6$) δ 8.63 - 8.59 (m, 1H), 8.54 - 8.51 (m, 1H), 8.38 -8.42 (m, 1H), 2.73 (s, 3H); ESI-MS (m/z) 239.8 $(MH)^+$.

Step-2: 3-Chloro-5-(5-methyl-1,2,4-oxadiazol-3-yl)aniline: To a stirred solution of step-1 intermediate (0.8 g, 3.34 mmol) in EtOH (10 mL) was added iron powder (1.86 g, 33.4 mmol), ammonium chloride (1.79 g, 33.4 mmol) and $H_2O$ (2.5 mL). The reaction was heated at 80°C for 4 h. Upon completion, the reaction mixture was cooled to room temperature and filtered through celite bed, and the filtrate was rotary evaporated. Water (20 mL) was added to the residue followed by ethyl acetate (50 mL). The layers were separated and the aqueous layer extracted with ethyl acetate (2 x 25 mL). The combined organic layer was washed with saturated $NaHCO_3$ (20 mL), dried over $Na_2SO_4$ and filtered. The filtrate was rotary evaporated and the solid residue (0.21 g, 30%) was carried forward without purification. $^1$H NMR (400 MHz, DMSO-$d_6$) δ 7.18 (dd, *J* = 2.1, 1.4 Hz, 1H), 7.05 (t, *J* = 1.7 Hz, 1H), 6.76 (t, *J* = 2.0 Hz, 1H), 5.79 (s, 2H), 2.65 (s, 3H); ESI-MS (m/z) 210.3 $(MH)^+$.

**Example-49:** Preparation of 3-chloro-4-(2H-1,2,3-triazol-2-yl)aniline and Preparation of 3-chloro-4-(1H-1,2,3-triazol-1-yl)aniline

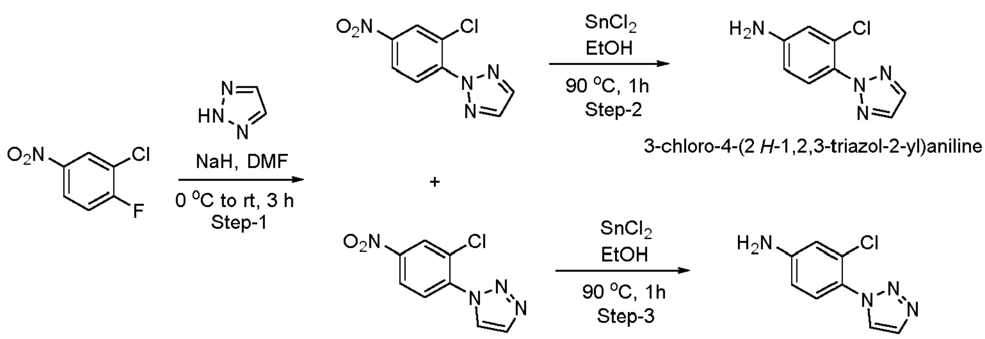


3-chloro-4-(2*H*-1,2,3-triazol-2-yl)aniline

3-chloro-4-(1*H*-1,2,3-triazol-1-yl)aniline

Step-1: 2-(2-chloro-4-nitrophenyl)-2H-1,2,3-triazole and 1-(2-chloro-4-nitrophenyl)-1H-1,2,3-triazole: To a solution of 2(H)-1,2,3-triazole (0.649 g, 9.40 mmol) in DMF (20 mL) was added portionwise sodium hydride (0.376 g, 9.40 mmol) at rt and then the stirred mixture for 1 h at room temperature. The reaction mixture was then cooled back down to 0°C, and a solution of 2-chloro-1-fluoro-4-nitrobenzene (1.50 g, 8.54 mmol) in DMF (10 mL) was added dropwise. The resulting mixture was stirred for 1.5 h at 0°C and then at rt for 1.5 h. The mixture was quenched with ice cooled water and extracted with EtOAc (2×50 mL). The combined organic layers were washed with water (2×30 mL), brine (30 mL), dried ($Na_2SO_4$) and filtered. The filtrate was rotary evaporated to leave a crude which was purified by flash column chromatography (silica gel, 20-30% EtOAc in hexanes as eluent) to give 2-(2-chloro-4-nitrophenyl)-2H-1,2,3-triazole (0.6 g, 31%) & 1-(2-chloro-4-nitrophenyl)-1H-1,2,3-triazole (0.8 g, 42%).

2-(2-Chloro-4-nitrophenyl)-2H-1,2,3-triazole: $^1$HNMR (400 MHz, DMSO-$d_6$) δ 8.60 (d, *J* = 2.5 Hz, 1H), 8.39 (dd, *J* = 8.5, 2.5 Hz, 1H), 8.30 (s, 2H), 8.05 (d, *J* = 8.5 Hz, 1H). ESI-MS (m/z) 224.7 $(MH)^+$.

1-(2-chloro-4-nitrophenyl)-1H-1,2,3-triazole: $^1$HNMR (400 MHz, DMSO-$d_6$) δ 8.74 (d, *J* = 1.5 Hz, 1H), 8.65 (d,

$J$ = 2.5 Hz, 1H), 8.42 (dd, $J$ = 8.5, 2.5 Hz, 1H), 8.07 (d, $J$ = 1.5 Hz, 1H), 8.03 (d, $J$ = 8.5 Hz, 1H). ESI-MS (m/z) 224.7 $(MH)^+$.

Step-2: 3-Chloro-4-(2(H)-1,2,3-triazol-2-yl)aniline: To a solution 2-(2-chloro-4-nitrophenyl)-2H-1,2,3-triazole (0.6 g, 2.67 mmol) in EtOH (20 mL), 2N HCl (aq) (16.9 mL) was added tin(II) chloride (2.53 g, 13.36 mmol) at rt . The resulting white suspension was heated at 90°C for 1 h. The reaction mass was cooled to room temperature and concentrated in vacuum. The residue was diluted with EtOAc (100 mL) followed by water (50 mL). The mixture was basified with 1 N aqueous NaOH (5 mL) and the layers were separated. The aqueous layer was extracted with ethyl acetate (2×30 mL) and the combined oraganic layers were washed with water (30 mL), brine (30 mL), dried ($Na_2SO_4$) and filtered. The filtrate was rotary evaporated to get crude which was purified by flash column chromatography (silica gel, 30% EtOAc in hexane) to give (500 mg, 96%) of the titled compound as off white solid. $^1$HNMR (400 MHz, DMSO-$d_6$) δ 8.01 (s, 2H), 7.22 (d, $J$ = 8.5 Hz, 1H), 6.75 (d, $J$ = 2.5 Hz, 1H), 6.61 (dd, $J$ = 8.5, 2.5 Hz, 1H), 5.88 (s, 2H); ESI-MS (m/z) 195.0 $(MH)^+$.

Step-3: 3-Chloro-4-(1H-1,2,3-triazol-1-yl)aniline: The titled compound was prepared from 1-(2-chloro-4-nitrophenyl)-1H-1,2,3-triazole (0.8 g, 3.56 mmol) by following the similar procedure described in above step-2 to afford 600 mg (87%) of 3-chloro-4-(1H-1,2,3-triazol-1-yl)aniline as off white solid. $^1$HNMR (400 MHz, DMSO-$d_6$) δ 8.35 (s, 1H), 7.89 (s, 1H), 7.23 (d, $J$ = 8.5 Hz, 1H), 6.79 (d, $J$ = 2.5 Hz, 1H), 6.63 (dd, $J$ = 8.5, 2.5 Hz, 1H), 5.91 (s, 2H); ESI-MS (m/z) 195.2 $(MH)^+$.

**Example-50:** Following compounds were prepared by using the procedure described under example-49:

3,5-dichloro-4-(1H-1,2,3-triazol-1-yl)aniline, ESI-MS (m/z) 229.4 $(MH)^+$;

3-Chloro-4-(3-methyl-1H-1,2,4-triazol-1-yl)aniline, ESI-MS (m/z) 208.9 $(MH)^+$;

3-Chloro-4-(5-methyl-1H-1,2,4-triazol-1-yl)aniline, ESI-MS (m/z) 208.8 $(MH)^+$;

5-Amino-2-(3-methyl-1H-1,2,4-triazol-1-yl)benzonitrile, ESI-MS (m/z) 199.88 $(MH)^+$;

5-Amino-2-(5-methyl-1H-1,2,4-triazol-1-yl)benzonitrile, ESI-MS (m/z) 200.76 $(MH)^+$; and

6-(1H-1,2,3-Triazol-1-yl)-5-(trifluoromethyl)pyridin-3-amine, ESI-MS (m/z) 229.80 (MH)+.

**Example-51:** Preparation of 5-amino-2-(2H-1,2,3-triazol-2-yl)benzonitrile

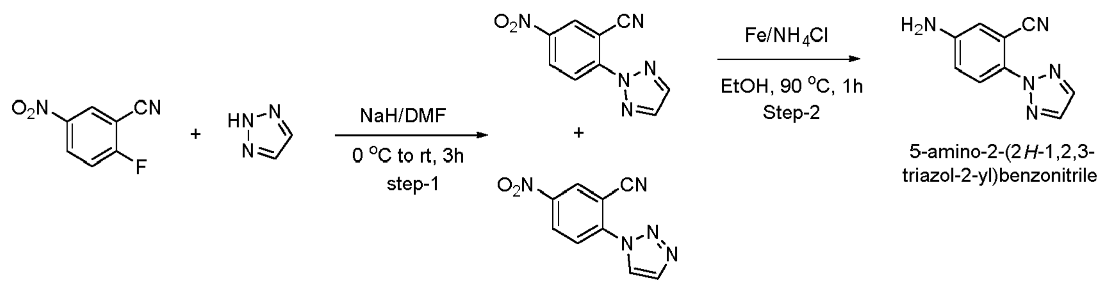


Step-1: 5-Nitro-2-(2H-1,2,3-triazol-2-yl)benzonitrile: To a solution of 2(H)-1,2,3-triazole (0.913 g, 13.24 mmol) in DMF (15 mL) was added portionwise sodium hydride (60% suspension in mineral oil, 0.530 g, 13.24 mmol) at rt and then stirred the mixture for 1 h at room temperature. The reaction mixture was then cooled back down to 0°C, and a solution of 2-fluoro-5-nitrobenzonitrile (2.0 g, 12.04 mmol) in DMF (10 mL) was added dropwise. The resulting mixture was stirred for 1.5 h at 0°C and then at RT for 1.5 h. The mixture was quenched with ice cooled water and extracted with EtOAc (2×50 mL). The combined organic layers were washed with water (2×50 mL), brine (50 mL), dried ($Na_2SO_4$) and filtered. The filtrate was rotary evaporated to leave a crude which was purified by flash column chromatography (silica gel, 20-30% EtOAc in hexanes as eluent) to give 5-nitro-2-(2H-1,2,3-triazol-2-yl)benzonitrile (1.2 g, 46.3% yield) & 5-nitro-2-(1H-1,2,3-triazol-1-yl)benzonitrile (0.7 g, 27.0% yield).

5-nitro-2-(2H-1,2,3-triazol-2-yl)benzonitrile: $^1$HNMR (400 MHz, DMSO-$d_6$) δ 8.93 (d, $J$ = 2.5 *Hz,* 1H), 8.68 (dd, $J$ = 8.5, 2.5 *Hz,* 1H), 8.44 (s, 2H), 8.40 (d, $J$ = 8.5 *Hz,* 1H). ESI-MS (m/z) 216.04 $(MH)^+$.

5-nitro-2-(1H-1,2,3-triazol-1-yl)benzonitrile: $^1$HNMR (400 MHz, DMSO-$d_6$) δ 9.05 (d, $J$ = 2.5 *Hz,* 1H), 8.95 (d, $J$ = 1.3 *Hz,* 1H), 8.75 (dd, $J$ = 8.5, 2.5 *Hz,* 1H), 8.20 (d, $J$ = 8.5 *Hz,* 1H), 8.15 (d, $J$ = 1.3 *Hz,* 1H); ESI-MS (m/z) 216.00 $(MH)^+$.

Step-2: 5-Amino-2-(2H-1,2,3-triazol-2-yl)benzonitrile: To a solution 5-nitro-2-(2H-1,2,3-triazol-2-yl)benzonitrile

(1.20 g, 5.58 mmol) in EtOH (20 mL)) was added iron powdder (1.24 g, 22.31 mmol) and ammonium chloride (1.193 g, 22.31 mmol) at RT. The resulting white suspension was stirred at 90°C for 1 h. The reaction mass was cooled to room temperature and concentrated in vacuum. The residue was diluted with EtOAc (100 mL) and filtred through the celite bed and washed with EtOAc (50 mL). The combined filltrates were washed with water and the layers were separated. The aqueous layer was extracted with ethyl acetate (2x50 mL) and the combined oraganic layers were washed with brine (30 mL), dried ($Na_2SO_4$) and filtered. The filtrate was rotary evaporated to get crude which was purified by flash column chromatography (silica gel, 30% EtOAc in hexane) to afford 800 mg (77%) of the titled compound as off white solid. $^1$HNMR (400 MHz, DMSO-$d_6$) δ 8.11 (s, 2H), 7.61 (d, *J*= 8.5 *Hz,* 1H), 7.02 (d, *J*= 2.5 *Hz,* 1H), 6.98 (dd, *J*= 8.5, 2.5 *Hz,* 1H), 6.01 (s, 2H); ESI-MS (m/z) 186.39 $(MH)^+$.

**Example-52:** The following compounds were prepared by using the procedure described in Example-51:

4-(1H-Pyrazol-1-yl)-3-(trifluoromethyl)aniline; ESI-MS (m/z) 227.98 $(MH)^+$;

4-(2H-1,2,3-Triazol-2-yl)-3-(trifluoromethyl)aniline; ESI-MS (m/z) 229.02 $(MH)^+$;

3-fluoro-4-(2H-1,2,3-triazol-2-yl)aniline; ESI-MS (m/z) 179.32 $(MH)^+$;

5-fluoro-6-(2H-1,2,3-triazol-2-yl)pyridin-3-amine; ESI-MS (m/z) 180.06 $(MH)^+$;

5-Chloro-2-(2H-1,2,3-triazol-2-yl)aniline; ESI-MS (m/z) 194.51 $(MH)^+$;

5-Amino-2-(2H-1,2,3-triazol-2-yl)nicotinonitrile, ESI-MS (m/z) 186.96 $(MH)^+$;

3-Chloro-4-(1H-imidazol-1-yl)aniline; ESI-MS (m/z) 193.07 $(MH)^+$;

3-chloro-4-(1H-pyrazol-1-yl)aniline; ESI-MS (m/z) 193.07 $(MH)^+$;

5-chloro-6-(1H-pyrazol-1-yl)pyridin-3-amine, GC-MS (m/z) 194.08 $(M)^+$; and

6-(1H-Pyrazol-1-yl)-5-(trifluoromethyl)pyridin-3-amine; ESI-MS (m/z) 228.98 $(M)^+$.

**Example-53:** Preparation of 5-chloro-2-methoxy-6-(2H-1,2,3-triazol-2-yl)pyridin-3-amine

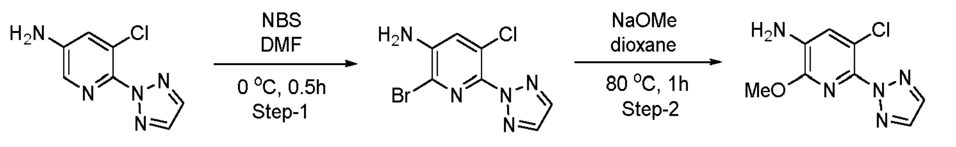


5-chloro-2-methoxy-6-(2 *H*-1,2,3-triazol-2-yl)pyridin-3-amine

Step-1: Preparation of 2-bromo-5-chloro-6-(2H-1,2,3-triazol-2-yl)pyridin-3-amine: To a (0°C) cooled and stirred solution of 5-chloro-6-(2H-1,2,3-triazol-2-yl)pyridin-3-amine (1.0 g, 0.511 mmol) in DMF (10 mL) was added dropwise a solution of NBS (0.91 g, 0.511 mmol) in DMF (5 mL). After stirring for 0.5 h at room temperature, water (20 mL) was added to the reaction followed by ethyl acetate (20 mL). The layers were separated and aqueous layer was extracted with ethyl acetate (2×50 mL). The combined organic layers were washed with brine (50 mL), dried ($Na_2SO_4$) and filtered. The filtrate was rotary evaporated and the crude product was purified by flash column chromatography (silica gel, 20% ethyl acetate in hexane system as eluent) followed by trituration with ethyl acetate to afford 1.0 g (71%) of the desired compound as off white solid. $^1$HNMR (400 MHz, DMSO-$d_6$) δ 8.10 (s, 2H), 7.37 (s, 1H), 5.75 (s, 2H); ESI-MS (m/z) 274.02 $(MH)^+$.

Step-2: 5-chloro-2-methoxy-6-(2H-1,2,3-triazol-2-yl)pyridin-3-amine: To a stirred solution of step-1 intermediate (1.0 g, 0.511 mmol) in dioxane (10 mL) was added a solution of sodium methoxide in methanol (0.4 mL, 12.75 mmol, 25% wt in methanol) at 0°C. The reaction mixture was stirred at room temperature for 15 min and then at 80°C for 1 h. The reaction was cooled to room temperature and ice was added. The aqueous layer was extracted with ethyl acetate (2x50 mL). The combined organic layers were washed with brine (50 mL), dried ($Na_2SO_4$) and filtered. The filtrate was rotary evaporated and the crude product was purified by flash column chromatography (silica gel, 20% ethyl acetate in

hexane system as eluent) to afford 600 mg (73%) of the desired compound as off white solid. $^1$HNMR (400 MHz, DMSO-$d_6$) δ 8.04 (s, 2H), 7.07 (s, 1H), 5.86 (s, 2H), 3.85 (s, 3H); ESI-MS (m/z) 226.0 $(MH)^+$.

**Example-54:** The following compounds were prepared by using a similar procedure similar to the one described in Example-53:

5-Chloro-2-methoxy-6-(1H-1,2,3-triazol-1-yl)pyridin-3-amine; ESI-MS (m/z) 225.83 (MH)+;

2-Methoxy-6-(2H-1,2,3-triazol-2-yl)-5-(trifluoromethyl)pyridin-3-amine, GC-MS (m/z) 259.13 (M)+;

5-Chloro-2-methoxy-6-(1H-pyrazol-1-yl)pyridin-3-amine; GC-MS (m/z) 223.98 $(M)^+$; and

2-Ethoxy-6-(2H-1,2,3-triazol-2-yl)-5-(trifluoromethyl)pyridin-3-amine; ESI-MS (m/z) 274.1 $(MH)^+$.

**Example-55:** Preparation of 5-chloro-6-methoxy-2-(2H-1,2,3-triazol-2-yl)pyridin-3-amine

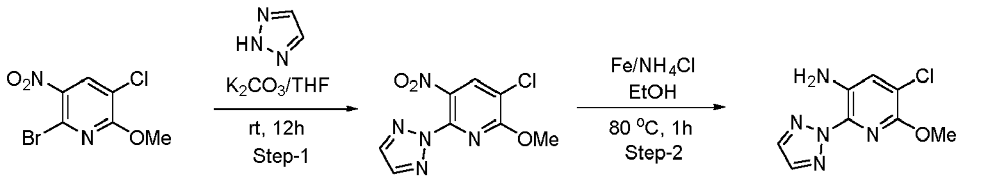


5-chloro-6-methoxy-2-(2 *H*-1,2,3-triazol-2-yl)pyridin-3-amine

Step-1: 3-Chloro-2-methoxy-5-nitro-6-(2H-1,2,3-triazol-2-yl)pyridine: To a (0°C) cooled and stirred solution of 2-bromo-5-chloro-6-methoxy-3-nitropyridine (3.5 g, 13.09 mmol), in tetrahydrofuran (30 mL) was added dropwise a solution of 2H-1,2,3-triazole (0.904 g, 13.09 mmol) in THF (5 mL) and potassium carbonate (1.809 g, 13.09 mmol). After stirring the resulting mixture at room temperature for 12 h, water (20 mL) was added followed by ethyl acetate (100 mL). The layers were separated and aqueous layer was extracted with ethyl acetate (2x100 mL). The combined organic layers were washed with brine (100 mL), dried ($Na_2SO_4$) and filtered. The filtrate was rotary evaporated and the crude product was purified by flash column chromatography (silica gel, 30% ethyl acetate in hexane as eluent) to afford 1.0 g (30%) of the desired compound as off white solid. $^1$HNMR (400 MHz, DMSO-$d_6$) δ 8.85 (s, 1H), 8.26 (s, 2H), 4.10 (s, 3H); ESI-MS (m/z) 255.87 $(MH)^+$.

Step-2: 5-Chloro-6-methoxy-2-(2H-1,2,3-triazol-2-yl)pyridin-3-amine: To a stirred solution of step-1 intermediate (1.0 g, 3.91 mmol) in ethanol (20 mL) was added ammonium chloride (0.628 g, 11.8 mmol) and iron powder (0.655 g, 11.8 mmol) at 0°C. The reaction mixture was then stirred at 80°C for 1 h. The reaction was cooled to room temperature and filtered through celite bed and washed with ethyl acetate (50 mL). The filtrate was rotary evaporated and the crude product was purified by flash column chromatography (silica gel, 50% ethyl acetate in hexane as eluent) to afford 0.6 g (68%) of the desired compound as off white solid. $^1$HNMR (400 MHz, DMSO-$d_6$) δ 8.15 (s, 2H), 7.60 (s, 1H), 5.74 (s, 2H), 3.87 (s, 3H); ESI-MS (m/z) 225.83 (MH)+.

**Example-56:** preparation of 5-chloro-2-methoxy-4-(2H-1,2,3-triazol-2-yl)aniline And

**Example-57:** Preparation of 5-chloro-2-methoxy-4-(1H-1,2,3-triazol-1-yl)aniline

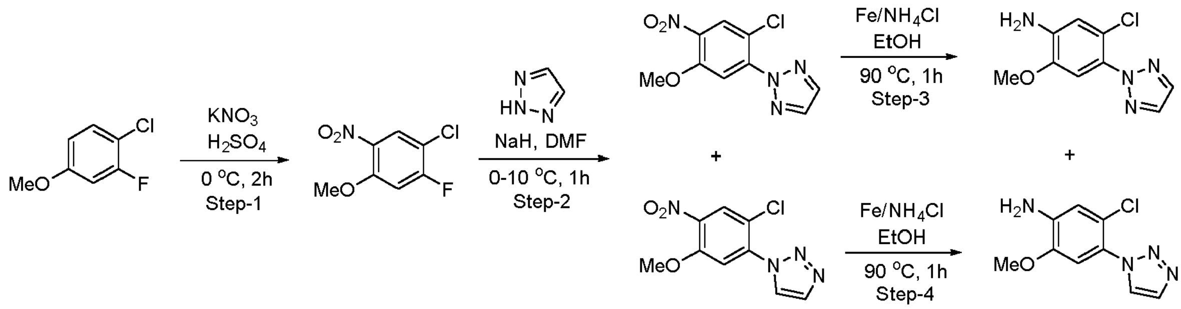


Step-1: 1-Chloro-2-fluoro-4-methoxy-5-nitrobenzene: To a mixture of 1-chloro-2-fluoro-4-methoxybenzene (5.00 g, 31.1 mmol) in conc. $H_2SO_4$ (30 mL) at 0-10°C was added portionwise potassium nitrate (3.78 g, 37.4 mmol) and the reaction was stirred at 0°C for 2 h. The reaction was quenched with ice water and filtered. The obtained solids were recrystallized with hexanes to give 4.50 g (70%) of 1-chloro-2-fluoro-4-methoxy-5-nitrobenzene. $^1$HNMR (400 MHz, DMSO-$d_6$) δ 8.30 (d, *J* = 2.0 Hz, 1H), 7.61 (d, *J*= 2.3 Hz, 1H), 3.96 (s, 3H); ESI-MS (m/z) 205.76 $(MH)^+$.

Step-2: 2-(2-Chloro-5-methoxy-4-nitrophenyl)-2H-1,2,3-triazole & 1-(2-chloro-5-methoxy-4-nitrophenyl)-1H-1,2,3-triazole: To a stirred suspension of NaH (0.467 g, 11.67 mmol, 60% in mineral oil) in DMF (5 mL) was added 2H-1,2,3-triazole (0.739 g, 10.70 mmol) in DMF (10 mL) at 0°C. The resulting mixture was stirred at the same temp for another 20 min. A solution of 1-chloro-2-fluoro-4-methoxy-5-nitrobenzene (2.00 g, 9.73 mmol) in DMF (5 mL) was then added to the above reaction mixture and stirred at 0-10°C for another 1h. Reaction mixture was poured in ice water and extracted with EtOAc (2×50 mL). The combined organic layers were washed with water (2×50 mL), brine (50 mL), dried ($Na_2SO_4$) and filtered. The filtrate was concentrated in vacuum. The crude product was purified by column chromatography (silica gel) to afford 2-(2-chloro-5-methoxy-4-nitrophenyl)-2H-1,2,3-triazole (0.750 g, 30.3% yield) and 1-(2-chloro-5-methoxy-4-nitrophenyl)-1H-1,2,3-triazole (1.120 g, 45.2% yield).

2-(2-Chloro-5-methoxy-4-nitrophenyl)-2H-1,2,3-triazole; $^1$HNMR (400 MHz, DMSO-$d_6$) δ 8.39 (s, 1H), 8.28 (s, 2H), 7.74 (s, 1H), 4.01 (s, 3H); ESI-MS (m/z) 254.82 $(MH)^+$.

1-(2-Chloro-5-methoxy-4-nitrophenyl)-1H-1,2,3-triazole; $^1$HNMR (400 MHz, DMSO-$d_6$) δ 8.68 (s, 1H), 8.42 (s, 1H), 8.06 (s, 1H), 7.79 (s, 1H), 4.01 (s, 3H); ESI-MS (m/z) 255 $(MH)^+$.

Step-3: 2-(2-Chloro-5-methoxy-4-nitrophenyl)-2H-1,2,3-triazole: To a suspension of 2-(2-chloro-5-methoxy-4-nitrophenyl)-2H-1,2,3-triazole (0.750 g, 2.95 mmol) in ethanol (20 mL) was added iron powder (0.987 g, 17.67 mmol) followed by a solution of ammonium chloride (0.945 g, 17.67 mmol) in water (6 mL) and the resulting mixture was heated at 90°C for 1 h. The reaction was cooled down to RT and filtered through the celite. The residue was washed with 5% MeOH:DCM (2 x 30 mL). The organic layer was conc in vaccum and the residue was diluted with DCM and washed with water. The combined organic layers were washed with brine (50 mL), dried over anhydrous $Na_2SO_4$ and concentrated under vaccum. The crude product was purified by flash column chromatrography (silica gel) to afford 0.550 g (83%) of the desired compound. $^1$HNMR (400 MHz, DMSO-$d_6$) δ 8.03 (s, 2H), 6.99 (s, 1H), 6.80 (s, 1H), 2.22 (s, 2H, $D_2O$ exchangeable), 3.32 (s, 3H); ESI-MS (m/z) 224.82 $(MH)^+$.

Step-4: 5-(Difluoromethyl)-6-(1H-1,2,3-triazol-1-yl)pyridin-3-amine: The titled compound was prepared by following the similar procedure described in step-3 by using 1-(2-Chloro-5-methoxy-4-nitrophenyl)-1H-1,2,3-triazole. $^1$HNMR (400 MHz, DMSO-$d_6$) δ 8.37 (s, 1H), 7.90 (s, 1H), 7.02 (s, 1H), 6.82 (s, 1H), 5.53 (s, 2H, $D_2O$ exchangeable), 3.80 (s, 3H); ESI-MS (m/z) 224.78 $(MH)^+$.

**Example-58:** Preparation of 5-(difluoromethyl)-6-(2H-1,2,3-triazol-2-yl)pyridin-3-amine and

**Example-59:** Preparation of 5-(difluoromethyl)-6-(1H-1,2,3-triazol-1-yl)pyridin-3-amine

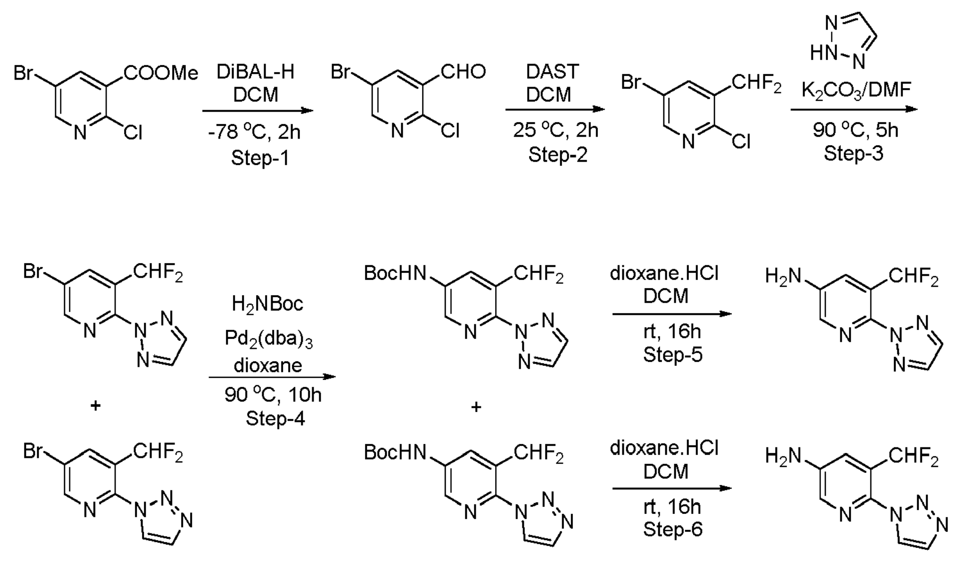


Step-1: 5-Bromo-2-chloronicotinaldehyde: To a (-78°C) cooled and stirred solution of methyl 5-bromo-2-chloronicotinate (10.0 g, 39.9 mmol) in DCM (100 mL) was added DIBAL-H (43.9 mL, 43.9 mmol, 1.6 M in hexane) dropwise and then stirred for 2h at the same temperature. Reaction was quenched with 2M aqueous HCl (50 mL) and stirred for 30 min at room temperature. Reaction mixture was filtered through the celite. The layers were separated and the aqueous layer was extracted with ethyl acetate (2×100 mL). The combined organic layer was washed with brine (100 mL), dried ($Na_2SO_4$) and filtered. The filtrate was concentrated under vacuum and the crude product was purified by flash column chromatography (silica gel, 20-30% EtOAc in hexane system as eluent) to afford 6.50 g (74%) of the titled compound. $^1$HNMR (400 MHz, DMSO-$d_6$) δ 10.19 (s, 1H), 8.86 (brs, 1H), 8.40 (brs, 1H); GCMS (m/z) 218.94(M)+.

Step-2: 5-Bromo-2-chloro-3-(difluoromethyl)pyridine: To a solution of step-1 intermediate (4.79 g, 21.73 mmol) in DCM (125 mL) was added catalytic amount of ethanol (0.127 mL, 2.173 mmol) followed by the addition of DAST (5.74 mL, 43.5 mmol) dropwise at 25°C for 15 min. Reaction mixture was stirred at same temperature for 2h before quenching with aqueous saturated solution of $NaHCO_3$ at 0°C. The layers were separated and the aqueous layer was washed with DCM. Combined organic layer was washed with brine, dried over $Na_2SO_4$ and concentrated in vaccum and the crude product was purified by flash column chromatography (silica gel) to afford 4.60 g (87%) of the titled compound. $^1$HNMR (400 MHz, DMSO-$d_6$) δ 8.79 (d, $J$ = 2.5 Hz, 1H), 8.42 (d, $J$ = 2.5 Hz, 1H), 7.21 (t, $J$ = 53.5 Hz, 1H); GCMS (m/z) 240.85 (M)+.

Step-3: 5-Bromo-3-(difluoromethyl)-2-(2H-1,2,3-triazol-2-yl)pyridine and 5-bromo-3-(difluoromethyl)-2-(1H-1,2,3-triazol-1-yl)pyridine: To a solution of step-2 intermediate (4.60 g, 18.97 mmol) in DMF (25 mL) was added potassium carbonate (5.24 g, 37.9 mmol) and 2H-1,2,3-triazole (1.966 g, 28.5 mmol). The resulting mixture was heated at 90°C for 5 h. The reaction mixture was poured in ice water and extracted with ethyl acetate (2×100 mL). The organic layer was washed with brine (50 mL), dried ($Na_2SO_4$) and filtered. The filtrate was concentrateed under vaccum. The crude product was purified by flash column chromatography (silica gel) to afford mixture of of 5-bromo-3-(difluoromethyl)-2-(2H-1,2,3-triazol-2-yl)pyridine and 5-bromo-3-(difluoromethyl)-2-(1H-1,2,3-triazol-1-yl)pyridine (4.2 g, 82%). The mixture was used as such for next step without separation of regioisomers. GCMS (m/z) 273.98$(M)^+$.

Step-4: t-Butyl (5-(difluoromethyl)-6-(2H-1,2,3-triazol-2-yl)pyridin-3-yl)carbamate and tert-butyl (5-(difluoromethyl)-6-(1H-1,2,3-triazol-1-yl)pyridin-3-yl)carbamate: To a mixture of 5-bromo-3-(difluoromethyl)-2-(2H-1,2,3-triazol-2-yl)pyridine and 5-bromo-3-(difluoromethyl)-2-(1H-1,2,3-triazol-1-yl)pyridine (4.20 g, 15.2 mmol) and tert-butyl carbamate (1.789 g, 15.27 mmol) in dioxane (150 mL) was added $Pd_2(dba)_3$ (0.350 g, 0.382 mmol) and xantphos (0.442 g, 0.763 mmol) in a sealed tube. The resulting mixture was purged with nitrogen gas for 15 min and then $Cs_2CO_3$ (4.98 g, 15.27 mmol) was added. Reaction mixture was sealed and heated at 90°C for the 10 h. Reaction mixture was filtered through the celite and concentrated in vacuum. The crude product was purified by flash column chromatography (silica gel, 20-30% EtOAc in hexane system as eluent) to afford tert-butyl (5-(difluoromethyl)-6-(2H-1,2,3-triazol-2-yl)pyridin-3-yl)carbamate (1.100 g, 46% yield) and tert-butyl (5-(difluoromethyl)-6-(1H-1,2,3-triazol-1-yl)pyridin-3-yl)carbamate (0.900 g, 38% yield).

tert-Butyl (5-(difluoromethyl)-6-(2H-1,2,3-triazol-2-yl)pyridin-3-yl)carbamate: $^1$HNMR (400 MHz, DMSO-$d_6$) δ 10.18 (s, 1H, $D_2O$ exchangeable), 8.74 (brs, 1H), 8.48 (brs, 1H), 8.20 (s, 2H), 7.28 (t, $J$= 54.0 Hz, 1H), 1.52 (s, 9H); ESI-MS (m/z) 312.28 $(MH)^+$.

tert-Butyl (5-(difluoromethyl)-6-(1H-1,2,3-triazol-1-yl)pyridin-3-yl)carbamate: $^1$HNMR (400 MHz, DMSO-$d_6$) δ 10.20 (s, 1H, $D_2O$ exchangeable), 8.76-8.72 (m, 2H), 8.52 (brs, 1H), 8.01 (brs, 1H), 7.37 (t, $J$= 54.0 Hz, 1H), 1.53 (s, 9H); ESI-MS (m/z) 312.02 $(MH)^+$.

Step-5: 5-(Difluoromethyl)-6-(2H-1,2,3-triazol-2-yl)pyridin-3-amine: To a solution of tert-butyl (5-(difluoromethyl)-6-(2H-1,2,3-triazol-2-yl)pyridin-3-yl)carbamate (1.100 g, 3.53 mmol) in DCM (10 mL) was added 4 M HCl in dioxane (10 mL) dropwise and resulting reaction mixture was stirred at RT for 16 h. Reaction mixture was concentrated in vacuum and the residue was diluted with EtOAc. The organic layer was washed with aqeuous $NaHCO_3$ (10 mL), brine (10 mL), dried ($Na_2SO_4$) and concentrated in vacuum to afford 0.450 g (60%) of the 5-(difluoromethyl)-6-(2H-1,2,3-triazol-2-yl)pyridin-3-amine. $^1$HNMR (400 MHz, DMSO-$d_6$) δ 8.09 (s, 2H), 8.00 (brs, 1H), 7.35 (brs, 1H), 6.97 (t, *J* = 54.5 Hz, 1H), 6.15 (s, 2H, $D_2O$ exchangeable); ESI-MS (m/z) 212.33 $(MH)^+$.

Step-6: 5-(difluoromethyl)-6-(1H-1,2,3-triazol-1-yl)pyridin-3-amine: To a solution of tert-butyl (5-(difluoromethyl)-6-(1H-1,2,3-triazol-1-yl)pyridin-3-yl)carbamate (0.150 g, 0.482 mmol) in DCM (2 mL) was added 4 M HCl in dioxane dropwise (3 mL) and resulting reaction mixture was stirred at RT for 16 h. Reaction mixture was concentrated in vaccum and the residue was diluted with EtOAc. The organic layer was washed with aqeuous $NaHCO_3$, brine, dried over $Na_2SO_4$ and concentrated in vacuum to afford 0.064 g (63%) of the 5-(difluoromethyl)-6-(1H-1,2,3-triazol-1-yl)pyridin-3-amine. $^1$HNMR (400 MHz, DMSO-$d_6$) δ 8.56 (s, 1H), 8.02 (s, 1H), 7.94 (s, 1H), 7.41 (s, 1H), 7.12 (t, *J*= 54.5 Hz, 1H), 6.17 (s, 2H, $D_2O$ exchangeable); ESI-MS (m/z) 212.26 $(MH)^+$.

**Example-60:** Preparation of 3-(difluoromethyl)-4-(2H-1,2,3-triazol-2-yl)aniline.

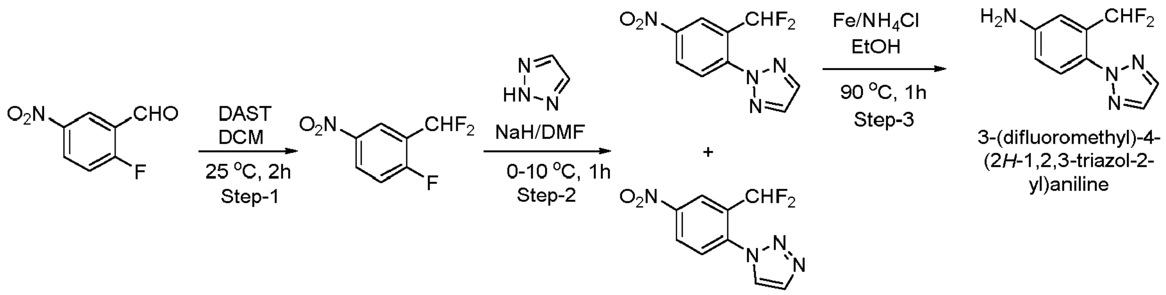

3-(difluoromethyl)-4-(2*H*-1,2,3-triazol-2-yl)aniline

Step-1: 2-(Difluoromethyl)-1-fluoro-4-nitrobenzene: To a solution of 2-fluoro-5-nitrobenzaldehyde (2.00 g, 11.83 mmol) in DCM (50 mL) was added ethanol (0.069 mL, 1.183 mmol) followed by the addition of DAST (3.28 mL, 24.84 mmol) dropwise at RT. Reaction mixture was stirred at RT for the 2 h before quenching with aqueous saturated solution of $NaHCO_3$ at 0°C. The layers were separated and aqueous layer was extracted with DCM (2×50 mL). The combined organic layers were washed with brine (50 mL), dried ($Na_2SO_4$) and filtered. The filtrate was concentrated in vacuum and the crude product was purified by flash column chromatography (silica gel) to afford 1.6 g (71%) of the titled compound. $^1$HNMR (400 MHz, DMSO-$d_6$) δ 8.54-8.48 (m, 2H), 7.72 (t, *J*= 9.5 Hz, 1H), 7.35 (t, *J*= 53.5 Hz, 1H); GC-MS (m/z) 191.05 $(M)^+$.

Step-2: 2-(2-(Difluoromethyl)-4-nitrophenyl)-2H-1,2,3-triazole and 1-(2-(difluoromethyl)-4-nitrophenyl)-1H-1,2,3-triazole: To a stirred solution of 2H-1,2,3-triazole (0.361 g, 5.23 mmol) in DMF (3 mL) was added NaH (0.209 g, 5.23 mmol, 60% suspension in oil) portionwise at 0°C and stirred for 1 h at the same temperature. A solution of 2-(difluoromethyl)-1-fluoro-4-nitrobenzene (1.00 g, 5.23 mmol) in DMF (5 mL) at 0°C was then added to the above mixture dropwise and continued to stir for 1h at 0-10 °C. The reaction was diluted with ethyl acetate (10 mL) followed by water (10 mL). The layers were separated and aqueous layer was extracted with ethyl acetate (2×15 mL). The combined organic layers were washed with water (2×20 mL), brine (20 mL), dried ($Na_2SO_4$) and filtered. The filtrate was rotary evaporated and the crude product was purified by flash column chromatography (silica gel) to afford (0.500 g, 40%) of the 2-(2-(difluoromethyl)-4-nitrophenyl)-2H-1,2,3-triazole and (0.400 g, 32%) of the 1-(2-(difluoromethyl)-4-nitrophenyl)-1H-1,2,3-triazole.

2-(2-(Difluoromethyl)-4-nitrophenyl)-2H-1,2,3-triazole: $^1$HNMR (400 MHz, DMSO-$d_6$) δ 8.62-8.52 (m, 2H), 8.37 (s, 2H), 8.31 (d, *J*= 9.0 Hz, 1H), 7.72 (t, *J*= 54.0 Hz, 1H); ESI-MS (m/z) 241.08 $(MH)^+$.

1-(2-(Difluoromethyl)-4-nitrophenyl)-1H-1,2,3-triazole: $^1$HNMR (400 MHz, DMSO-$d_6$) δ 8.80 (s, 1H), 8.66-8.56 (m, 2H), 8.10 (s, 1H), 8.05 (d, *J* = 8.5 Hz, 1H), 7.29 (t, *J* = 54.0 Hz, 1H); ESI-MS (m/z) 241.08 $(MH)^+$.

Step-3: 3-(difluoromethyl)-4-(2H-1,2,3-triazol-2-yl)aniline: To a stirreed suspension of 2-(2-(difluoromethyl)-4-nitrophenyl)-2H-1,2,3-triazole (0.550 g, 2.290 mmol) in ethanol (15 mL) was added iron powder (0.639 g, 11.45 mmol) and a solution of ammonium chloride (0.612 g, 11.45 mmol) in water (6 mL). The resulting mixture was stirred at 90°C for 1h and then cooled to room temperature and filtered through the celite bed. The celite bed was washed with 5% MeOH:DCM(2x30mL). The combined filtrates were concentrated in vaccum and the residue was diluted with DCM (50 mL) and washed with water (20 mL), brine (20 mL), dried ($Na_2SO_4$) and filtered. The filtrate was concentrated in vacuum

and the crude product was purified by flash column chromatrography to afford 0.450 g (93%) of the titled compound. $^1$HNMR (400 MHz, DMSO-$d_6$) δ 8.05 (s, 2H), 8.41 (d, *J* = 8.5 Hz, 1H), 7.25-6.88 (m, 2H), 6.81 (d, *J* = 8.5 Hz, 1H), 5.84 (s, 2H, $D_2O$ exchangeable); ESI-MS (m/z) 210.8 $(MH)^+$.

**Example-61:** Preparation of 3-Amino-1-methyl-5-(trifluoromethyl)pyridin-2(1H)-one

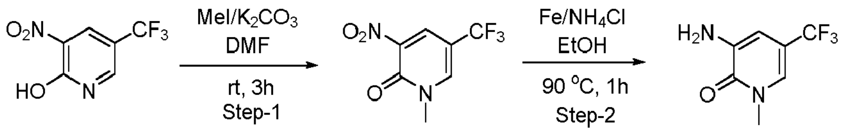


3-amino-1-methyl-5-(trifluoromethyl)pyrid in-2(1*H*)-one

Step-1: 1-methyl-3-nitro-5-(trifluoromethyl)pyridin-2(1H)-one: To a solution of 3-nitro-5-(trifluoromethyl)pyridin-2-ol (2.00 g, 9.61 mmol) in DMF (30 mL) was added $K_2CO_3$ (2.66 g, 19.22 mmol) and iodomethane (0.897 mL, 14.42 mmol) at room temperature. The resulting mixture was stirred at 25°C for 3 h and then poured into ice water and extracted with ethyl acetate (3x50mL). The combined organic layers were washed with water (2×50 mL), brine (50 mL), dried ($Na_2SO_4$) and filteered. The filtrate was concentrated in vaccum and the crude product was purified by flash column chromatography (silica gel, 30-40% EtOAc in hexane system as eluent) to afford 1.50 g (70%) of the titled compound. $^1$HNMR (400 MHz, DMSO-$d_6$) δ 8.90 (d, *J* = 2.5 Hz, 1H), 8.69 (d, *J* = 2.5 Hz, 1H), 3.62 (s, 3H); ESI-MS (m/z) 222.7 $(MH)^+$.

Step-2: 3-Amino-1-methyl-5-(trifluoromethyl)pyridin-2(1H)-one: To a suspension of 1-methyl-3-nitro-5-(trifluoromethyl)pyridin-2(1H)-one (1.00 g, 4.50 mmol) in ethanol (20 mL) was added iron powder (1.257 g, 22.51 mmol) and a solution of ammonium chloride (1.204 g, 22.51 mmol) in water (5 mL) and then stirred at 90°C for 1h. The reaction mixture was cooled down to RT and filtered through celite, residue was washed with 5% MeOH:DCM (2x30mL). The combined organic filtrates were concentrated in vacuum and the residue was diluted with DCM (20 mL) and washed with water (20 mL), brine (20 mL), dried ($Na_2SO_4$) and filtered. The filtrate was concentrated in vacuum and the crude product was purified by flash column chromatrography (silica gel) to afford 0.820 g (95%) of the titled compound. $^1$HNMR (400 MHz, DMSO-$d_6$) δ 7.55 (d, *J* = 2.5 Hz, 1H), 6.51 (d, *J* = 2.5 Hz, 1H), 5.50 (s, 2H, $D_2O$ exchangeable), 3.50 (s, 3H); ESI-MS (m/z) 192.76 $(MH)^+$.

**Example-62:** Preparation of 5-Chloro-6-(2-(1-methylpiperidin-4-yl)ethoxy)pyridin-3-amine.

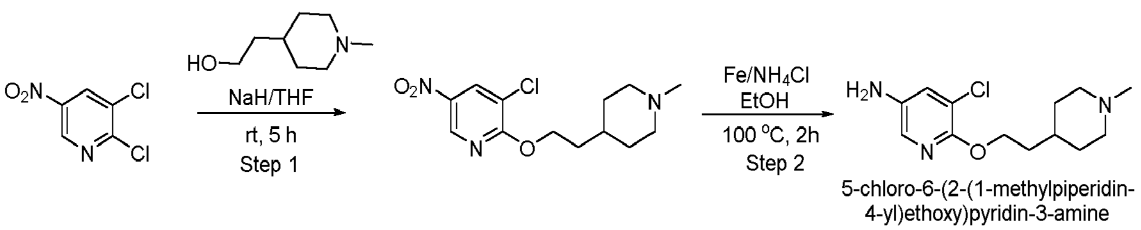


5-chloro-6-(2-(1-methylpiperidin-4-yl)ethoxy)pyridin-3-amine

Step-1: 3-Chloro-2-(2-(1-methylpiperidin-4-yl)ethoxy)-5-nitropyridine: At a stirreed and cooled (0°C) solution of 2-(1-methylpiperidin-4-yl)ethanol (3.50 g, 24.4 mmol) in tetrahydrofuran (100 mL) was added NaH (1.46 g, 36.7 mmol) portionwise and the resulting mixture was heated at 50°C for 30 min. The reaction was then cooled to 0°C before the addition of a solution of 2,3-dichloro-5-nitropyridine (4.72 g, 24.4 mmol) in tetrahydrofuran (25 mL). The resulting mixture was then stirred at RT for 5 h. Reaction mass was cooled to 0°C, diluted with ethyl acetate (100 mL) and 10% MeOH in DCM (30 mL) followed by the addition of crushed ice (2.0 g). The solvent was rotary evaporated and the crude product was purified by flash column chromatography (silica gel, 7% MeOH in DCM as eluent) to afford 4.80 g (65%) of the titled product as white solid. $^1$HNMR (400 MHz, DMSO-$d_6$) δ 9.05 (d, *J* = 2.5 Hz, 1H), 8.73 (d, *J* = 2.5 Hz, 1H), 4.54 (t, *J* = 6.5 Hz, 2H), 2.95 (d, *J* = 11.5 Hz, 2H), 2.33 (s, 3H), 2.19 (t, *J* = 11.7 Hz, 2H), 1.80-1.67 (m, 4H), 1.36-1.16 (m, 3H); ESI-MS (m/z) 300.46 $(MH)^+$.

Step-2: 5-Chloro-6-(2-(1-methylpiperidin-4-yl)ethoxy)pyridin-3-amine: To a solution of step-1 intermediate (2.0 g, 6.67 mmol), in ethanol (20 mL) and water (4 mL) was added ammonium chloride (3.57 g, 66.7 mmol) followed by iron

powder (1.49 g, 26.7 mmol) and the resulting mixture was heated at 100°C for 2 h. The reaction was cooled back down to rt, filtered through celite and the filtrate was rotary evaporated. The crude product was purified by flash column chromatography (silica gel, 11% methanol in dichloromethane as eluent) to afford 1.50 g (83%) of the titled product as white solid. $^1$HNMR (400 MHz, DMSO-$d_6$) δ 7.46 (d, *J* = 2.6 Hz, 1H), 7.15 (d, *J* = 2.6 Hz, 1H), 5.04 (s, $D_2O$ exchangeable, 2H), 4.23-4.20 (m, 2H), 3.36-3.34 (m, 2H), 2.89-2.86 (m, 2H), 2.68 (s, 3H), 1.90-1.87 (m, 2H), 1.67-1.62 (m, 3H), 1.53-1.45 (m, 2H); ESI-MS (m/z) 270.46 $(MH)^+$.

**Example-63:** Preparation of phenyl (5-chloro-6-methoxypyridin-3-yl)carbamate

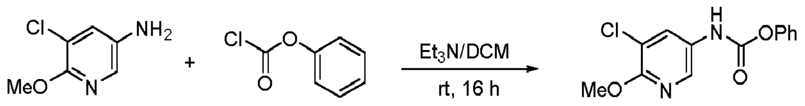

phenyl (5-chloro-6-methoxypyridin-3-yl)carbamate

To a (0°C) cooled and stirred solution of 5-chloro-6-methoxypyridin-3-amine (500 mg, 3.15 mmol) in DCM (10 mL) was added phenyl carbonochloridate (396 μL, 3.15 mmol) followed by pyridine (306 μL, 3.78 mmol). The reaction mixture was warmed to room temperature and then stirred for 16 h. The reaction was then cooled back down to 0°C and water (10 mL) was added followed by DCM (30 mL). The layers were separated and the aqueous layer was extracted with DCM (2×30 mL). The combined organic layers were washed with brine (50 mL), dried ($Na_2SO_4$) and filtered. The filtrate was rotary evaporated and the crude product was purified by flash column chromatography (silica gel, 10% ethyl acetate in hexane as eluent) to afford (500 mg, 57%) of the titled compound as white solid. $^1$HNMR (400 MHz, DMSO-$d_6$) δ 10.41 (s, 1H, $D_2O$ exchangeable), 8.25 (d, *J* = 2.5 *Hz,* 1H), 8.02 (d, *J* = 2.5 *Hz,* 1H), 7.48-7.41 (m, 2H), 7.33-7.21 (m, 3H), 3.92 (s, 3H); ESI-MS (m/z) 278.96$(MH)^+$.

**Example-64:** Following compounds were prepared using the similar procedure described in example-63:

Phenyl (3-chloro-4-methoxyphenyl)carbamate, ESI-MS (m/z) 278.00$(MH)^+$;

Phenyl (2-(trifluoromethyl)pyridin-4-yl)carbamate, ESI-MS (m/z) 283.34 $(MH)^+$;

Phenyl (5-chloro-6-ethoxypyridin-3-yl)carbamate, ESI-MS (m/z) 293.14$(MH)^+$;

Phenyl (1-methyl-2-oxo-5-(trifluoromethyl)-1,2-dihydropyridin-3-yl)carbamate, ESI-MS (m/z) 313.41 $(MH)^+$;

Phenyl (5-chloro-6-isopropoxypyridin-3-yl)carbamate, ESI-MS (m/z) 307.40 $(MH)^+$;

Phenyl (5-(trifluoromethyl)pyridin-3-yl)carbamate, ESI-MS (m/z) 283.40 $(MH)^+$;

Phenyl (6-methoxy-5-(trifluoromethyl)pyridin-3-yl)carbamate, ESI-MS (m/z) 313.03 $(MH)^+$;

Phenyl (5-chloro-6-(2H-1,2,3-triazol-2-yl)pyridin-3-yl)carbamate, ESI-MS (m/z) 316.06 $(MH)^+$;

Phenyl (5-cyanopyridin-3-yl)carbamate, ESI-MS (m/z) 239.92 $(MH)^+$;

Phenyl (5-(difluoromethyl)pyridin-3-yl)carbamate, ESI-MS (m/z) 265 $(MH)^+$;

Phenyl (2-cyanopyridin-4-yl) carbamate, ESI-MS (m/z) 239.71 $(MH)^+$;

Phenyl (5-chloro-6-(difluoromethoxy)pyridin-3-yl)carbamate, ESI-MS (m/z) 315.08 $(MH)^+$;

Phenyl (5-methoxy-6-(1H-1,2,3-triazol-1-yl)pyridin-3-yl)carbamate, ESI-MS (m/z) 312.46 $(MH)^+$;

Phenyl (6-(2H-1,2,3-triazol-2-yl)-5-(trifluoromethyl)pyridin-3-yl)carbamate, ESI-MS (m/z) 350.34 $(MH)^+$;

Phenyl (5-methoxy-6-(2H-1,2,3-triazol-2-yl)pyridin-3-yl)carbamate, ESI-MS (m/z) 312.46 $(MH)^+$;

Phenyl (6-(1,1-dioxidoisothiazolidin-2-yl)-5-(trifluoromethyl)pyridin-3-yl)carbamate, ESI-MS (m/z) 402.10 $(MH)^+$;

Phenyl (7-cyclopropyl-2-methylthiazolo[5,4-b]pyridin-6-yl)carbamate, ESI-MS (m/z) 326.07 $(MH)^+$;

Phenyl (5-chloro-6-(1H-1,2,3-triazol-1-yl)pyridin-3-yl)carbamate; ESI-MS (m/z) 316.09 $(MH)^+$;

Phenyl (5-cyano-6-methoxypyridin-3-yl)carbamate; ESI-MS (m/z) 270.08 $(MH)^+$;

Phenyl (5-chloro-6-cyanopyridin-3-yl)carbamate; ESI-MS (m/z) 274.05 $(MH)^+$; and

Phenyl (3,5-dichloro-4-(1H-1,2,3-triazol-1-yl)phenyl)carbamate; ESI-MS (m/z) 348.9 $(MH)^+$.

**Example-65:** Preparation of phenyl (3-chloro-4-(1,3,4-oxadiazol-2-yl)phenyl) carbamate

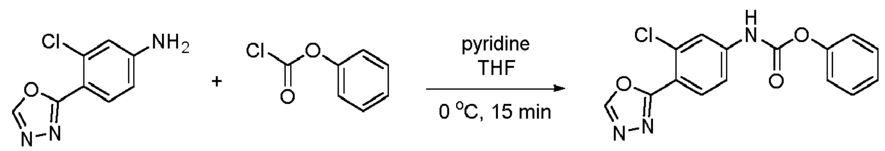


phenyl (3-chloro-4-(1,3,4-oxadiazol-2-yl)phenyl)carbamate

To a (0 °C) cooled and stirred solution of 3-chloro-4-(1,3,4-oxadiazol-2-yl)aniline (0.1 g, 0.51 mmol) in THF (3.0 mL) was added pyridine (0.054 mL, 0.665 mmol) followed by phenyl carbonochloridate (0.071 mL, 0.562 mmol). After stirring at 0°C for 15 min the reaction mixture was diluted with water (5 mL) followed by ethyl acetate (5 mL). The layers were separated and the aqueous layer was extracted with ethyl acetate (2×5 mL). The combined organic layers were washed by water (5 mL), brine (5 mL), dried ($Na_2SO_4$) and filtered. The filtrate was rotary evaporated and the crude product was purified by flash column chromatography (silica gel) to afford 60 mg (37%) of the titled compound as a white solid. $^1$HNMR (400 MHz, DMSO-$d_6$) δ 10.86 (s, 1H), 9.42 (s, 1H), 8.00 (d, $J$ = 8.5 *Hz,* 1H), 7.88 (d, $J$ = 2.0 *Hz,* 1H), 7.66 (dd, $J$ = 8.5, 2.0 *Hz,* 1H), 7.47 (t, $J$ = 7.5 Hz, 2H), 7.30 (m, 3H).

**Example-66:** Preparation of 1-(5-Chloro-6-methoxypyridin-3-yl)-3-(7-cyclopropyl-2-methylthiazolo [5,4-b]pyridin-6-yl)urea (compound 1)

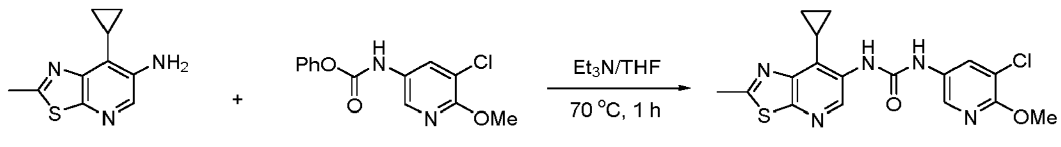


7-cyclopropyl-2-methylthiazolo[5,4-*b*]pyridin-6-amine

phenyl (5-chloro-6-methoxypyridin-3-yl)carbamate

**Compound 1**

To a stirred solution of 7-cyclopropyl-2-methylthiazolo[5,4-b]pyridin-6-amine (30 mg, 0.15 mmol) in THF (3 mL) in a sealed tube was added phenyl (5-chloro-6-methoxypyridin-3-yl)carbamate(41 mg, 0.15 mmol) followed by triethylamine (41 μL, 0.29 mmol). After stirring for 1 h at 70°C the reaction was cooled to room temperature and the solvent was rotary evaporated. The crude product was purified by flash column chromatography (3% MeOH in DCM as eluent) to afford (16 mg, 28%) of the titled compound as white solid. $^1$HNMR (400 MHz, DMSO-$d_6$) δ 9.14 (s, 1H, $D_2O$ exchangeable), 8.61 (s, 1H), 8.50 (s, 1H, $D_2O$ exchangeable), 8.15 (brs, 2H), 3.91 (s, 3H), 2.80 (s, 3H), 2.23-2.12 (m, 1H), 1.58-1.46 (m, 2H), 1.20-1.07 (m, 2H); ESI-MS (m/z) 390.09 $(MH)^+$.

**Example-67:** Following compounds were prepared from the corresponding intermediates by using the similar procedure described in Example-66:

1-(3-Chloro-4-methoxyphenyl)-3-(7-cyclopropyl-2-methylthiazolo[5,4-b]pyridin-6-yl)urea (Compound 2)

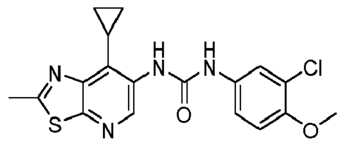

$^1$HNMR (400 MHz, DMSO-$d_6$) 9.03 (s, 1H, $D_2O$ exchangeable), 8.65 (s, 1H), 8.33 (s, 1H, $D_2O$ exchangeable), 7.70 (s, 1H), 7.29 (d, *J* = 8.5 *Hz,* 1H), 7.09 (d, *J* = 8.5 *Hz,* 1H), 3.82 (s, 3H), 2.80 (s, 3H), 2.23-2.09 (m, 1H), 1.53-1.46 (m, 2H), 1.19-1.11 (m, 2H); ESI-MS (m/z) 389.04$(MH)^+$;

1-(7-Cyclopropyl-2-methylthiazolo[5,4-b]pyridin-6-yl)-3-(2-(trifluoromethyl)pyridin-4-yl)urea (Compound 3)

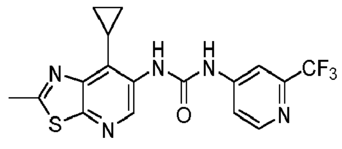

$^1$HNMR (400 MHz, DMSO-$d_6$) $\delta$ 9.89 (s, 1H, $D_2O$ exchangeable), 8.74 (s, 1H, $D_2O$ exchangeable), 8.57 (s, 1H), 8.54 (d, *J* = 8.0 *Hz,* 1H), 8.08 (d, *J* = 2.0 *Hz,* 1H), 7.64 (d, *J* = 8.0 *Hz,* 1H), 2.81 (s, 3H), 2.23-2.16 (m, 1H), 1.57-1.53 (m, 2H), 1.23-1.12 (m, 2H); ESI-MS (m/z) 394.15 $(MH)^+$;

1-(5-Chloro-6-ethoxypyridin-3-yl)-3-(7-cyclopropyl-2-methylthiazolo[5,4-b]pyridin-6-yl)urea (Compound 4)

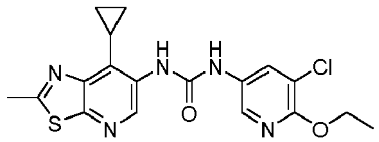

$^1$HNMR (400 MHz, DMSO-$d_6$) $\delta$ 9.13 (s, 1H, $D_2O$ exchangeable), 8.61 (s, 1H), 8.49 (s, 1H, $D_2O$ exchangeable), 8.14 (d, *J* = 2.5 *Hz,* 1H), 8.12 (d, *J* = 2.5 *Hz,* 1H), 4.35 (q, *J* = 7.0 *Hz,* 2H), 2.80 (s, 3H), 2.29- 2.07 (m, 1H), 1.59-1.48 (m, 2H), 1.34 (t, *J* = 7.0 *Hz,* 3H), 1.19-1.10 (m, 2H); ESI-MS (m/z) 404.06$(MH)^+$;

1-(7-Cyclopropyl-2-methylthiazolo[5,4-b]pyridin-6-yl)-3-(1-methyl-2-oxo-5-(trifluoromethyl)-1,2-dihydropyridin-3-yl)urea (Compound 5)

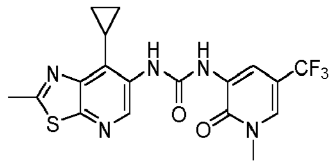

$^1$HNMR (400 MHz, DMSO-$d_6$) $\delta$ 9.31 (s, 1H), 9.28 (s, 1H), 8.65 (s, 1H), 8.29-8.24 (m, 1H), 8.05 (s, 1H), 3.61 (s, 3H), 2.80 (s, 3H), 2.22-2.15 (m, 1H), 1.58-1.52 (m, 2H), 1.14-1.05 (m, 2H); ESI-MS (m/z) 424.10 $(MH)^+$;

1-(5-Chloro-6-isopropoxypyridin-3-yl)-3-(7-cyclopropyl-2 methylthiazolo[5,4-b]pyridin-6-yl)urea (Compound 6)

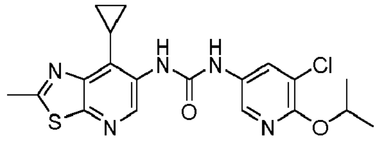

$^1$HNMR (400 MHz, DMSO-$d_6$) $\delta$ 9.11 (s, 1H), 8.61 (s, 1H), 8.49 (s, 1H), 8.12 (s, 2H), 5.27-5.19 (m, 1H), 2.80 (s, 3H), 2.19-2.14 (m, 1H), 1.55-1.50 (m, 2H), 1.32 (d, *J* = 6.1 Hz, 6H), 1.16-1.12 (m, 2H); ESI-MS (m/z) 418.04 $(MH)^+$;

1-(7-Cyclopropyl-2-methylthiazolo[5,4-b]pyridin-6-yl)-3-(5-(trifluoromethyl)pyridin-3-yl)urea (Compound 7)

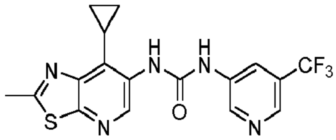

$^1$HNMR (400 MHz, DMSO-$d_6$) δ 9.62 (s, 1H), 8.83 (s, 1H), 8.70 (s, 1H), 8.59 (s, 1H), 8.58 (s, 1H), 8.45 (s, 1H), 2.81 (s, 3H), 2.24-2.17 (m, 1H), 1.58-1.54 (m, 2H), 1.20-1.12 (m, 2H); ESI-MS (m/z) 394.16 $(MH)^+$;

1-(7-Cyclopropyl-2-methylthiazolo[5,4-b]pyridin-6-yl)-3-(6-methoxy-5-(trifluoromethyl)pyridin-3-yl)urea (Compound 8)

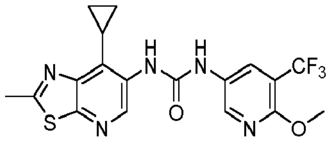

$^1$HNMR (400 MHz, DMSO-$d_6$) δ 9.25 (s, 1H, $D_2O$ exchangeable), 8.60 (s, 1H), 8.55 (s, 1H, $D_2O$ exchangeable), 8.44 (d, *J* = 2.5 *Hz,* 1H), 8.34 (d, *J* = 2.5 *Hz,* 1H), 3.95 (s, 3H), 2.80 (s, 3H), 2.21-2.18 (m, 1H), 1.56-1.52 (m, 2H), 1.17-1.12 (m, 2H); ESI-MS(m/z) 424.05 $(MH)^+$;

1-(5-Chloro-6-(2H-1,2,3-triazol-2-yl)pyridin-3-yl)-3-(7-cyclopropyl-2-methylthiazolo[5,4-b]pyridin-6-yl)urea (Compound 9)

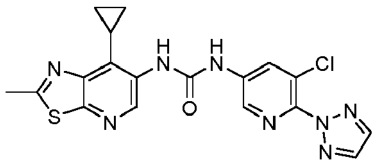

$^1$HNMR (400 MHz, DMSO-$d_6$) δ 9.79 (s, 1H), 8.78 (s, 1H), 8.61 (s, 1H), 8.58 (s, 1H), 8.49 (s, 1H), 8.16 (s, 2H), 2.81 (s, 3H), 2.25-2.18 (m, 1H), 1.59-1.57 (m, 2H), 1.20-1.14 (m, 2H); ESI-MS (m/z) 427.10 $(MH)^+$;

1-(5-Cyanopyridin-3-yl)-3-(7-cyclopropyl-2-methylthiazolo[5,4-b]pyridin-6-yl)urea (Compound 10)

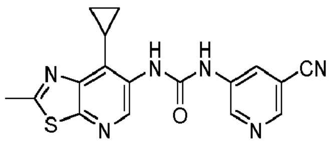

$^1$HNMR (400 MHz, DMSO-$d_6$) δ 9.58 (s, 1H), 8.86 (s, 1H), 8.71 (s, 1H), 8.63 (s, 1H), 8.59 (s, 1H), 8.43 (s, 1H), 2.81 (s, 3H), 2.23-2.13 (m, 1H), 1.58-1.55 (m, 2H), 1.20-1.12 (m, 2H); ESI-MS (m/z) 351.34 $(MH)^+$;

1-(7-Cyclopropyl-2-methylthiazolo[5,4-b]pyridin-6-yl)-3-(5-(difluoromethyl)pyridin-3-yl)urea (Compound 11)

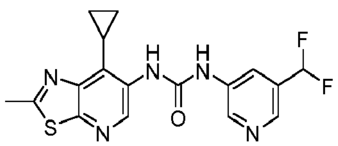

$^1$HNMR (400 MHz, DMSO-$d_6$) δ 9.49 (s, 1H), 8.71 (s, 1H), 8.63-8.60 (m, 2H), 8.40 (s, 1H), 8.29 (s, 1H), 7.16 (t, *J* = 55 *Hz,* 1H), 2.81 (s, 3H), 2.20 (m, 1H), 1.56-1.53(m, 2H), 1.17-1.14 (m, 2H); ESI-MS (m/z) 376.28 $(MH)^+$;

1-(2-Cyanopyridin-4-yl)-3-(7-cyclopropyl-2-methylthiazolo[5,4-b]pyridin-6-yl)urea (Compound 12)

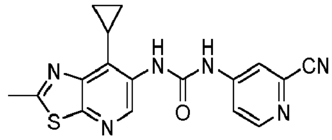

$^1$HNMR (400 MHz, DMSO-$d_6$) δ 9.87 (s, $D_2O$ exchangeable, 1H), 8.79 (s, 1H), 8.57 (s, $D_2O$ exchangeable, 1H), 8.52 (d, *J* = 5.5 *Hz,* 1H), 8.07 (d, *J* = 2.0 *Hz,* 1H), 7.71 (dd, *J* = 5.5 & 2.0 *Hz,* 1H), 2.81 (s, 3H), 2.22-2.15 (m, 1H), 1.58-1.55 (m, 2H), 1.20-1.12 (m, 2H); ESI-MS (m/z) 351.10 $(MH)^+$;

1-(5-Chloro-6-methoxypyridin-3-yl)-3-(2,7-dimethylthiazolo[5, 4-b]pyridin-6-yl)urea (Compound 13)

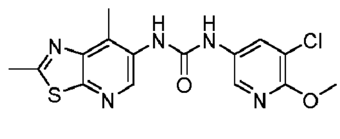

$^1$HNMR (400 MHz, DMSO-$d_6$) δ 9.15 (s, 1H, $D_2O$ exchangeable), 8.76 (s, 1H), 8.48 (s, 1H, $D_2O$ exchangeable), 8.48-8.14 (m, 2H), 3.91 (s, 3H), 2.83 (s, 3H), 2.58 (s, 3H); ESI-MS (m/z) 364.03 $(MH)^+$;

1-(3-Chloro-4-methoxyphenyl)-3-(2,7-dimethylthiazolo[5,4-b]pyridin-6-yl)urea (Compound 14)

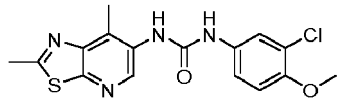

$^1$HNMR (400 MHz, DMSO-$d_6$) δ 9.02 (s, 1H, $D_2O$ exchangeable), 8.80 (s, 1H), 8.31 (s, 1H, $D_2O$ exchangeable), 7.70 (d, *J* = 2.5 *Hz,* 1H), 7.29 (dd, *J* = 8.5 & 2.5 *Hz,* 1H), 7.10 (d, *J* = 8.5 *Hz,* 1H), 3.82 (s, 3H), 2.83 (s, 3H), 2.58 (s, 3H); ESI-MS (m/z) 363.35 $(MH)^+$;

1-(5-Chloro-6-methoxypyridin-3-yl)-3-(7-(4-fluoro-2-methoxyphenyl)-2-methylthiazolo[5,4-b]pyridin-6-yl)urea (Compound 15)

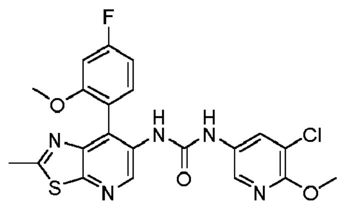

$^1$HNMR (400 MHz, DMSO-$d_6$) δ 9.23 (s, 1H, $D_2O$ exchangeable), 9.08 (s, 1H), 8.11 (s, 1H), 8.03 (s, 1H), 7.64 (s, 1H, $D_2O$ exchangeable), 7.38-7.36 (m, 1H), 7.19-7.17 (m, 1H), 7.00- 6.96 (m, 1H), 3.90 (s, 3H), 3.71 (s, 3H), 2.74 (s, 3H)); ESI-MS (m/z) 474.05 $(MH)^+$;

1-(5-Chloro-6-methoxypyridin-3-yl)-3-(7-(2-fluoropyridin-3-yl)-2-methylthiazolo[5,4-b]pyridin-6-yl)urea (Compound 16)

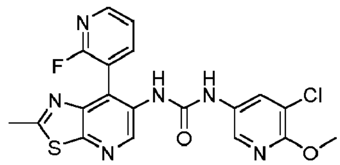

$^1$HNMR (400 MHz, DMSO-$d_6$) δ 9.04 (s, 1H, $D_2O$ exchangeable), 9.00 (s, 1H), 8.46 - 8.40 (m, 1H), 8.31 (s, 1H, $D_2O$ exchangeable), 8.14-8.07 (m, 1H), 8.06-8.00 (m, 2H), 7.62-7.53 (m, 1H), 3.89 (s, 3H), 2.78 (s, 3H); ESI-MS (m/z) 445.02 $(MH)^+$;

1-(3-Chloro-4-methoxyphenyl)-3-(7-(2-fluoropyridin-3-yl)-2-methylthiazolo[5,4-b]pyridin-6-yl)urea (Compound 17)

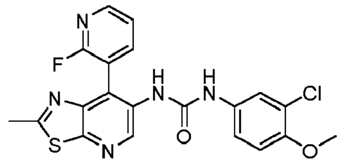

$^1$HNMR (400 MHz, DMSO-$d_6$) δ 9.06 (s, 1H), 8.88 (s, 1H, $D_2O$ exchangeable), 8.43-8.42 (m, 1H), 8.13 (s, 1H, $D_2O$ exchangeable), 8.11-8.06 (m, 1H), 7.62-7.55 (m, 2H), 7.18-7.16 (m, 1H), 7.07-7.05 (m, 1H), 3.35 (s, 3H), 2.77 (s, 3H); ESI-MS (m/z) 444.10 $(MH)^+$;

1-(5-Chloro-6-methoxypyridin-3-yl)-3-(7-(3-fluoropyridin-4-yl)-2-methylthiazolo[5,4-b]pyridin-6-yl)urea (Compound 18)

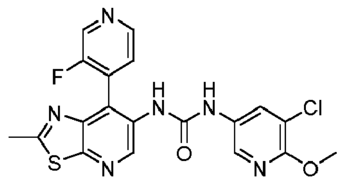

$^1$HNMR (400 MHz, DMSO-$d_6$) δ 9.05 (s, 1H, $D_2O$ exchangeable), 9.02 (s, 1H), 8.82 (s, 1H), 8.63 (d, *J* = 5.0 *Hz,* 1H), 8.31 (s, 1H, $D_2O$ exchangeable), 8.04-8.01 (m, 2H), 7.63-7.59 (m, 1H), 3.89 (s, 3H), 2.78 (s, 3H); ESI-MS (m/z) 445.05 $(MH)^+$;

1-(6-(2H-1,2,3-Triazol-2-yl)-5-(trifluoromethyl)pyridin-3-yl)-3-(7-cyclopropyl-2-methylthiazolo[5,4-b]pyridin-6-yl)urea (Compound 19)

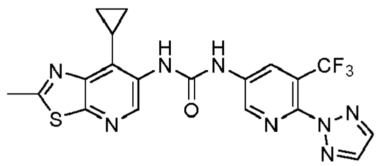

$^1$HNMR (400 MHz, DMSO-d6) δ 10.82 (s, $D_2O$ exchangeable, 1H), 9.48 (s, $D_2O$ exchangeable, 1H), 8.93 (s, 1H), 8.74 (s, 1H), 8.59 (s, 1H), 8.17 (s, 2H), 2.81 (s, 3H), 2.31-2.27 (m, 1H), 1.59-1.55 (m, 2H), 1.24-1.08 (m, 2H); ESI-MS (m/z) 461.1 $(MH)^+$;

1-(5-Chloro-6-(difluoromethoxy)pyridin-3-yl)-3-(7-cyclopropyl-2-methylthiazolo[5,4-b]pyridin-6-yl)urea (Compound 20)

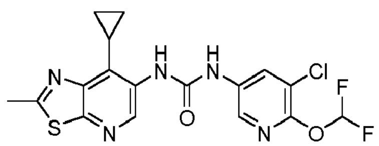

$^1$HNMR (400 MHz, DMSO-$d_6$) δ 9.40 (s, 1H), 8.63 (s, 1H), 8.59 (s, 1H), 8.32 (d, *J = 2.5 Hz,* 1H), 8.24 (d, *J* = 2.5 *Hz,* 1H), 7.67 (t, *J* = 72.4 Hz, 1H), 2.80 (s, 3H), 2.22-2.18 (m, 1H), 1.57-1.54 (m, 2H), 1.18-1.12 (m, 2H); ESI-MS (m/z) 426.04 $(MH)^+$;

1-(5-Chloro-2-oxoindolin-7-yl)-3-(7-cyclopropyl-2-methylthiazolo[5,4-b]pyridin-6-yl)urea (Compound 21)

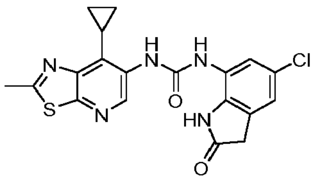

$^1$HNMR (400 MHz, DMSO-$d_6$) δ 10.25 (s, 1H), 8.70-8.55 (m, 2H), 8.55 (s, 1H), 7.44 (s, 1H), 7.05 (s, 1H), 3.57 (s, 2H), 2.80 (s, 3H), 2.24-2.21 (m, 1H), 1.58-1.56 (m, 2H), 1.16-1.08 (m, 2H); ESI-MS (m/z) 414.03 $(MH)^+$;

1-(7-Cyclopropyl-2-methylthiazolo[5,4-b]pyridin-6-yl)-3-(5-methoxy-6-(2H-1,2,3-triazol-2-yl)pyridin-3-yl)urea (Compound 22)

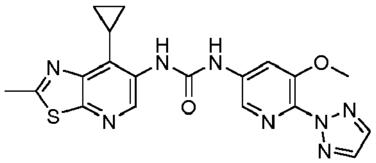

$^1$HNMR (400 MHz, DMSO-$d_6$) δ 9.65 (s, $D_2O$ exchangeable, 1H), 8.66 (s, $D_2O$ exchangeable, 1H), 8.65 (s, 1H), 8.18 (s, 1H), 8.06-8.05 (m, 3H), 3.80 (s, 3H), 2.81 (s, 3H), 2.24-2.18 (m, 1H), 1.58-1.54 (m, 2H), 1.24-1.12 (m, 2H); ESI-MS (m/z) 423.06 $(MH)^+$;

1-(7-Cyclopropyl-2-methylthiazolo[5,4-b]pyridin-6-yl)-3-(6-(1,1-dioxidoisothiazolidin-2-yl)-5-(trifluoromethyl)pyridin-3-yl)urea (Compound 23)

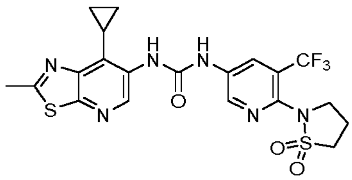

$^1$HNMR (400 MHz, $CDCl_3$) δ 8.74 (s, 1H), 8.53 (d, *J* = 2.5 *Hz,* 1H), 8.35 (d, *J* = 2.5 *Hz,* 1H), 8.24 (s, $D_2O$ exchangeable, 1H), 7.50 (s, $D_2O$ exchangeable, 1H), 3.91 (t, *J* = 7.0 *Hz,* 2H), 3.35 (t, *J* = 7.5 *Hz,* 2H), 2.82 (s, 3H), 2.69-2.62 (m, 2H), 2.19-2.05 (m, 1H), 1.55-1.51 (m, 2H), 1.28-1.20 (m, 2H); ESI-MS (m/z) 512.9 $(MH)^+$;

1-(7-Cyclopropyl-2-methylthiazolo[5,4-b]pyridin-6-yl)-3-(5-methoxy-6-(1H-1,2,3-triazol-1-yl)pyridin-3-yl)urea (Compound 24)

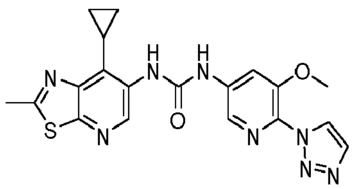

$^1$HNMR (400 MHz, DMSO-$d_6$): δ 9.68 (s, 1H), 8.69 (s, 1H), 8.65 (s, 1H), 8.46 (s, 1H), 8.22 (d, *J* = 2.0 *Hz,* 1H), 8.08 (d, *J* = 2.0 *Hz,* 1H), 7.91 (s, 1H), 3.84 (s, 3H), 2.81 (s, 3H), 2.22-2.18 (m, 1H), 1.57-1.54 (m, 2H), 1.18-1.12 (m, 2H); ESI-MS (m/z) 423.04 $(MH)^+$;

1-(3-Chloro-4-methoxyphenyl)-3-(7-ethyl-2-methylthiazolo[5,4-b]pyridin-6-yl)urea (Compound 25)

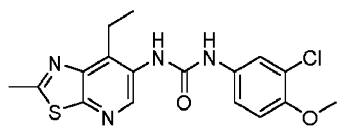

$^1$HNMR (400 MHz, DMSO-$d_6$) δ 9.00 (s, 1H), 8.78 (s, 1H), 8.24 (s, 1H), 7.70 (d, *J = 2.5 Hz,* 1H), 7.28 (dd, *J* = 8.5 & 2.5 *Hz* 1H), 7.10 (d, *J* = 8.5 *Hz,* 1H), 3.82 (s, 3H), 3.08 (q, *J* = 7.5 *Hz,* 2H), 2.84 (s, 3H), 1.23 (t, *J* = 7.5 *Hz,* 3H); ESI-MS (m/z) 377.28 (MH)$^+$;

1-(5-Chloro-6-methoxypyridin-3-yl)-3-(7-ethyl-2-methylthiazolo[5,4-b]pyridin-6-yl)urea (Compound 26)

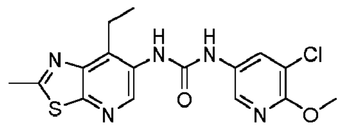

$^1$HNMR (400 MHz, DMSO-$d_6$) δ 9.13 (s, 1H), 8.76 (s, 1H), 8.41 (s, 1H), 8.16 (d, *J* = 2.5 *Hz,* 1H), 8.14 (d, *J* = 2.5 *Hz,* 1H), 3.91 (s, 3H), 3.08 (q, *J* = 7.5 *Hz,* 2H), 2.84 (s, 3H), 1.23 (t, *J* = 7.5 *Hz,* 3H) ; ESI-MS (m/z) 378.17 (MH)$^+$;

1-(5-Chloro-6-(2H-1,2,3-triazol-2-yl)pyridin-3-yl)-3-(7-(4,4-difluoropiperidin-1-yl)-2-methylthiazolo[5,4-b]pyridin-6-yl)urea (Compound 27)

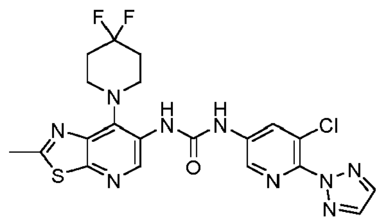

$^1$HNMR (400 MHz, DMSO-$d_6$) δ 10.06 (s, $D_2O$ exchangeable, 1H), 8.91 (s, 1H), 8.58 (d, *J* = 2.5 *Hz,* 1H), 8.52 (d, *J* = 2.5 *Hz,* 1H), 8.45 (s, $D_2O$ exchangeable, 1H), 8.17 (s, 2H), 3.50-3.48 (m, 4H), 2.83 (s, 3H), 2.30-2.22 (m, 4H); ESI-MS (m/z) 506.1 (MH)$^+$;

1-(5-Chloro-6-(2H-1,2,3-triazol-2-yl)pyridin-3-yl)-3-(2-methyl-7-morpholinothiazolo[5,4-b]pyridin-6-yl)urea (Compound 28)

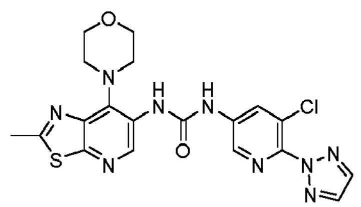

$^1$HNMR (400 MHz, DMSO-$d_6$) δ 10.12 (s, 1H), 8.86 (s, 1H), 8.57 (d, *J* = 2.3 *Hz,* 1H), 8.52 (d, *J* = 2.3 *Hz,* 1H), 8.50 (s, 1H), 8.17 (s, 2H), 3.84 (t, *J* = 4.5 *Hz,* 4H), 3.43 (t, *J* = 4.6 Hz, 4H), 2.83 (s, 3H); ESI-MS (m/z) 472.2 (MH)$^+$;

1-(5-Chloro-6-(2H-1,2,3-triazol-2-yl)pyridin-3-yl)-3-(7-(4-methoxypiperidin-1-yl)-2-methylthiazolo[5,4-b]pyridin-6-yl)urea (Compound 29)

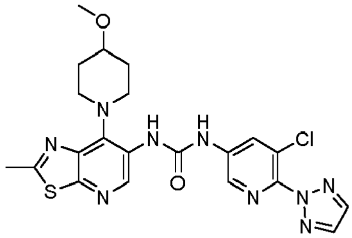

$^1$HNMR (400 MHz, DMSO-$d_6$) δ 10.14 (s, 1H), 8.85 (s, 1H), 8.57 (d, *J* = 2.5 *Hz,* 1H), 8.52 (d, *J* = 2.5 *Hz,* 1H), 8.37 (s, 1H), 8.17 (s, 2H), 3.47-3.42 (m, 5H), 3.31 (s, 3H), 2.82 (s, 3H), 2.07-2.02 (m, 2H), 1.80-1.75 (m, 2H); ESI-MS (m/z) 500.02 $(MH)^+$;

1-(5-Chloro-6-methoxypyridin-3-yl)-3-(7-(4-methoxypiperidin-1-yl)-2-methylthiazolo[5,4-b]pyridin-6-yl)urea (Compound 30)

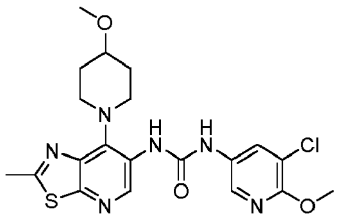

$^1$HNMR (400 MHz, DMSO-$d_6$) δ 9.55 (s, 1H), 8.89 (s, 1H), 8.17 (d, *J* = 2.5 *Hz,* 1H), 8.14 (d, *J* = 2.5 *Hz,* 1H), 8.12 (s, 1H), 3.92 (s, 3H), 3.43-3.37 (m, 5H), 3.31 (s, 3H), 2.81 (s, 3H), 2.06-2.03 (m, 2H), 1.80-1.72 (m, 2H); ESI-MS (m/z) 463.04 $(MH)^+$;

1-(5-Chloro-6-(2*H*-1,2,3-triazol-2-yl)pyridin-3-yl)-3-(7-cyclopropyl-2-ethylthiazolo[5,4-b]pyridin-6-yl)urea (Compound 31)

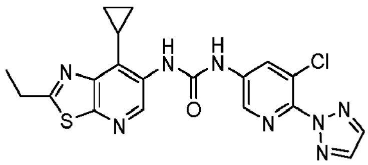

$^1$HNMR (400 MHz, DMSO-$d_6$) δ 9.80 (s, 1H), 8.81 (s, 1H), 8.61 (s, 1H), 8.58 (d, *J* = 2.5 Hz, 1H), 8.49 (d, *J* = 2.5 Hz, 1H), 8.16 (s, 2H), 3.13 (q, *J* = 7.5 Hz, 2H), 2.26-2.18 (m, 1H), 1.63-1.57 (m, 2H), 1.37 (t, *J* = 7.5 Hz, 3H), 1.21-1.12 (m, 2H); ESI-MS 441.1 $(MH)^+$;

1-(5-Chloro-6-(2H-1,2,3-triazol-2-yl)pyridin-3-yl)-3-(7-(2-methoxyethyl)-2-methylthiazolo[5,4-b]pyridin-6-yl)urea (Compound 32)

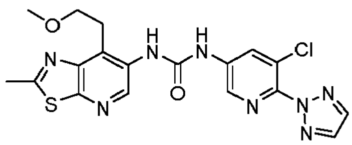

$^1$HNMR (400 MHz, DMSO-$d_6$) δ 9.96 (s, 1H), 8.77 (s, 1H), 8.61-8.54 (m, 2H), 8.52-8.47 (m, 1H), 8.16 (s, 2H), 3.67 (t, J = 6.7 Hz, 2H), 3.39-3.33 (m, 2H), 3.26 (s, 3H), 2.86 (s, 3H); ESI-MS (m/z) 444.99 $(MH)^+$;

1-(5-Chloro-6-methoxypyridin-3-yl)-3-(7-(2-methoxyethyl)-2-methylthiazolo[5,4-b]pyridin-6-yl)urea (Compound 33)

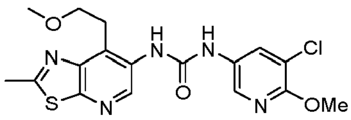

$^1$HNMR (400 MHz, DMSO-$d_6$) δ 9.31 (s, 1H), 8.77 (s, 1H), 8.34 (s, 1H), 8.15 (s, 2H), 3.92 (s, 3H), 3.65 (t, *J* = 6.8 Hz, 2H), 3.36-3.30 (m, 2H), 3.25 (s, 3H), 2.85 (s, 3H); ESI-MS (m/z) 407.98 $(MH)^+$;

(±)-1-(5-Chloro-6-methoxypyridin-3-yl)-3-(7-(1,2-dimethoxyethyl)-2-methylthiazolo[5,4-b]pyridin-6-yl)urea (Compound 34)

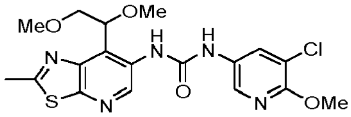

ESI-MS (m/z) 438.06 $(MH)^+$;
Chiral separation of racemic compound 34 was carried out using chiral column and afforded the below isomers 34a and 34b:

1-(5-chloro-6-methoxypyridin-3-yl)-3-(7-(1,2-dimethoxyethyl)-2-methylthiazolo[5,4-b]pyridin-6-yl)urea (Compound 34a)
Chiral HPLC RT: 10.27 min
$^1$HNMR (500 MHz, DMSO-$d_6$) δ 9.85 (s, 1H), 9.07 (s, 1H), 8.48 (s, 1H), 8.17 (d, *J* = 2.4 Hz, 1H), 8.15 (d, *J* = 2.4 Hz, 1H), 5.52 (dd, *J* = 6.4, 3.7 Hz, 1H), 3.92 (s, 3H), 3.82 (dd, *J* = 10.7, 6.5 Hz, 1H), 3.67 (dd, *J* = 10.7, 3.7 Hz, 1H), 3.31 (s, 3H), 3.27 (s, 3H), 2.85 (s, 3H); ESI-MS (m/z) 437.98 $(MH)^+$;

1-(5-Chloro-6-methoxypyridin-3-yl)-3-(7-(1,2-dimethoxyethyl)-2-methylthiazolo[5,4-b]pyridin-6-yl)urea (Compound 34b)
Chiral HPLC RT: 11.18 min
$^1$HNMR (500 MHz, DMSO-$d_6$) δ 9.85 (s, 1H), 9.07 (s, 1H), 8.48 (s, 1H), 8.17 (d, *J* = 2.4 Hz, 1H), 8.15 (d, *J* = 2.4 Hz, 1H), 5.52 (dd, *J* = 6.4, 3.7 Hz, 1H), 3.92 (s, 3H), 3.82 (dd, *J* = 10.7, 6.5 Hz, 1H), 3.67 (dd, *J* = 10.7, 3.7 Hz, 1H), 3.31 (s, 3H), 3.27 (s, 3H), 2.85 (s, 3H); ESI-MS (m/z) 437.98 $(MH)^+$;

1-(5-Chloro-6-(2H-1,2,3-triazol-2-yl)pyridin-3-yl)-3-(7-cyclopropylthiazolo[5,4-b]pyridin-6-yl)urea (Compound 35)

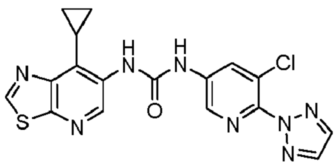

$^1$HNMR (400 MHz, DMSO-$d_6$) δ 9.83 (s, 1H), 9.47 (d, *J* = 2.6 *Hz,* 1H), 8.88 (s, 1H), 8.71 (s, 1H), 8.58 (s, 1H),8.49 (s, 1H), 8.16 (d, *J* = 2.6 *Hz,* 2H), 2.30-2.28 (m, 1H), 1.64-1.62 (m, 2H), 1.24-1.19 (m, 2H); ESI-MS (m/z) 412.9 $(MH)^+$;

1-(5-Chloro-6-methoxypyridin-3-yl)-3-(7-cyclopropylthiazolo[5,4-b]pyridin-6-yl)urea (Compound 36)

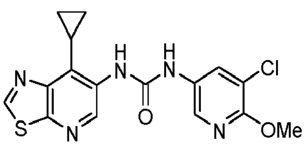

$^1$HNMR (400 MHz, DMSO-$d_6$) δ 9.45 (s, 1H), 9.24 (s, 1H), 8.72 (s, 1H), 8.61 (s, 1H), 8.15 (d, J = 2.9 Hz, 2H),3.91 (s, 3H), 2.30-2.28 (m, 1H), 1.59-1.58 (m, 2H), 1.24-1.19 (m, 2H); ESI-MS (m/z) 376.20 $(MH)^+$;

1-(5-Chloro-6-(2H-1,2,3-triazol-2-yl)pyridin-3-yl)-3-(2-methyl-7-(4-methylpiperidin-1-yl)thiazolo[5,4-b]pyridin-6-yl)urea (Compound 37)

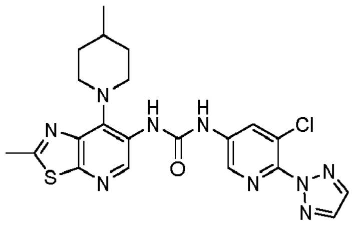

$^1$HNMR (400 MHz, DMSO-$d_6$) δ 10.12 (s, 1H), 8.75 (s, 1H), 8.57 (d, *J* = 2.5 Hz, 1H), 8.52 (d, *J* = 2.5 Hz, 1H), 8.39 (s, 1H), 8.17 (s, 2H), 3.43-3.39 (m, 2H), 3.36-3.20 (m, 2H), 2.81 (s, 3H), 1.81-1.65 (m, 2H), 1.61-1.54 (m, 1H), 1.47-1.39 (m, 2H), 0.97 (d, *J* = 6.5 Hz, 3H); ESI-MS (m/z) 484.05 $(MH)^+$;

1-(5-Chloro-6-methoxypyridin-3-yl)-3-(7-(2,6-dimethylmorpholino)-2-methylthiazolo[5,4-b]pyridin-6-yl)urea (Compound 38)

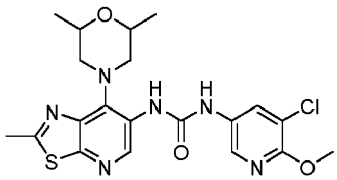

$^1$HNMR (400 MHz, DMSO-$d_6$) δ 9.50 (s, 1H), 8.91 (s, 1H), 8.26 (s, 1H), 8.16 (d, *J* = 2.4 Hz, 1H), 8.14 (d, *J* = 2.4 Hz, 1H), 3.92 (s, 3H overlap with m, 2H), 3.20 (d, *J* = 11 Hz, 2H), 3.12 (t, *J* = 11.0 Hz, 2H), 2.82 (s, 3H), 1.12 (d, *J* = 6.2 Hz, 6H); ESI-MS (m/z) 463.05 $(MH)^+$;

1-(5-Chloro-6-(2H-1,2,3-triazol-2-yl)pyridin-3-yl)-3-(7-(2,6-dimethylmorpholino)-2-methylthiazolo[5,4-b]pyridin-6-yl)urea (Compound 39)

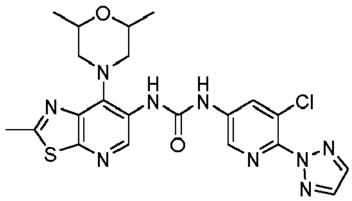

$^1$HNMR (400 MHz, DMSO-$d_6$) δ 10.08 (s, 1H), 8.87 (s, 1H), 8.57 (d, *J* = 2.3 Hz, 1H), 8.51 (d, *J* = 2.3 Hz, 1H), 8.49 (s, 1H), 8.17 (s, 2H), 3.96-3.88 (m, 2H), 3.29 (d, *J* = 11.7 Hz, 2H), 3.12 (t, *J* = 11.0 Hz, 2H), 2.83 (s, 3H), 1.13 (d, *J* = 6.2 Hz, 6H); ESI-MS (m/z) 499.94 $(MH)^+$;

1-(5-Chloro-6-(2H-1,2,3-triazol-2-yl)pyridin-3-yl)-3-(2-methyl-7-(piperidin-1-yl)thiazolo[5,4-b]pyridin-6-yl)urea (Compound 40)

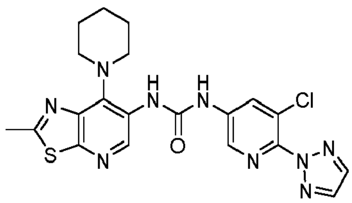

$^1$HNMR (400 MHz, DMSO-$d_6$) δ 10.12 (s, 1H), 8.81 (s, 1H), 8.57 (d, *J* = 2.4 Hz, 1H), 8.52 (d, *J* = 2.3 Hz, 1H), 8.36 (s, 1H), 8.16 (s, 2H), 3.38-3.34 (m, 4H), 2.81 (s, 3H), 1.80-1.70 (m, 4H), 1.67-1.60 (m, 2H); ESI-MS (m/z) 469.99 $(MH)^+$;

1-(5-Chloro-6-methoxypyridin-3-yl)-3-(7-((cyclopropylmethyl)(methyl)amino)-2-methylthiazolo[5,4-b]pyridin-6-yl)urea (Compound 41)

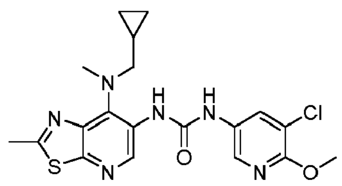

$^1$HNMR (400 MHz, DMSO-$d_6$) δ 9.59 (s, 1H), 9.04 (s, 1H), 8.53 (s, 1H), 8.18 (d, $J$ = 2.4 Hz, 1H), 8.12 (d, $J$ = 2.4 Hz, 1H), 3.91 (s, 3H), 3.21 (d, $J$ = 6.8 Hz, 2H), 3.04 (s, 3H), 2.81 (s, 3H), 0.97-0.85 (m, 1H), 0.38-0.28 (m, 2H), 0.06-0.01 (m, 2H); ESI-MS (m/z) 432.99 (MH)$^+$;

1-(5-Chloro-6-(2H-1,2,3-triazol-2-yl)pyridin-3-yl)-3-(7-((cyclopropylmethyl)(methyl)amino)-2-methylthiazolo[5,4-b]pyridin-6-yl)urea (Compound 42)

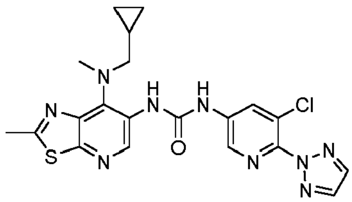

$^1$HNMR (400 MHz, DMSO-$d_6$) δ 10.19 (s, 1H), 9.02 (s, 1H), 8.76 (s, 1H), 8.56-8.49 (m, 2H), 8.16 (s, 2H), 3.25 (d, $J$ = 6.8 Hz, 2H), 3.08 (s, 3H), 2.82 (s, 3H), 1.00-0.88 (m, 1H), 0.39-0.30 (m, 2H), 0.08-0.02 (m, 2H); ESI-MS (m/z) 469.95 (MH)$^+$;

1-(5-Chloro-6-(2H-1,2,3-triazol-2-yl)pyridin-3-yl)-3-(7-((2,3-dimethoxypropyl)(methyl)amino)-2-methylthiazolo[5,4-b]pyridin-6-yl)urea (Compound 43)

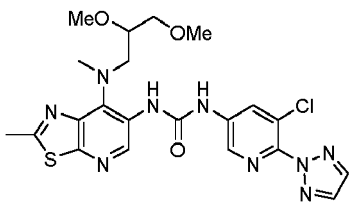

$^1$HNMR (400 MHz, DMSO-$d_6$) δ 10.14 (s, 1H), 8.92 (s, 1H), 8.56 (s, 1H), 8.55 (d, $J$ = 2.3 Hz, 1H), 8.52 (d, $J$ = 2.3 Hz, 1H), 8.16 (s, 2H), 3.55 (d, $J$ = 5.7 Hz, 2H), 3.45-3.37 (m, 2H), 3.33-3.28 (m, 1H), 3.18 (s, 3H), 3.14 (s, 3H), 3.08 (s, 3H), 2.82 (s, 3H); ESI-MS (m/z) 518.06 (MH)$^+$;

1-(5-Chloro-6-(2H-1,2,3-triazol-2-yl)pyridin-3-yl)-3-(7-((2-methoxyethyl)(methyl)amino)-2-methylthiazolo[5,4-b]pyridin-6-yl)urea (Compound 44)

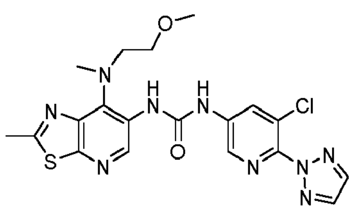

$^1$HNMR (400 MHz, DMSO-$d_6$) δ 10.14 (s, 1H), 8.92 (s, 1H), 8.62 (s, 1H), 8.56 (d, $J$ = 2.3 Hz, 1H), 8.52 (d, $J$ = 2.4 Hz, 1H), 8.17 (s, 2H), 3.60 (t, $J$ = 5.8 Hz, 2H), 3.46 (t, $J$ = 5.7 Hz, 2H), 3.17 (s, 3H), 3.07 (s, 3H), 2.82 (s, 3H); ESI-MS (m/z) 474.20 (MH)$^+$;

1-(5-Chloro-6-methoxypyridin-3-yl)-3-(7-((2-methoxyethyl)(methyl)amino)-2-methylthiazolo[5,4-b]pyridin-6-yl)urea (Compound 45)

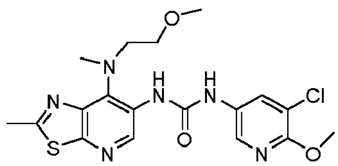

$^1$HNMR (400 MHz, DMSO-$d_6$) δ 9.53 (s, 1H), 8.95 (s, 1H), 8.40 (s, 1H), 8.18 (d, $J$ = 2.4 Hz, 1H), 8.14 (d, $J$ = 2.5 Hz, 1H), 3.91 (s, 3H), 3.56 (t, $J$ = 5.8 Hz, 2H), 3.43 (t, $J$ = 5.7 Hz, 2H), 3.16 (s, 3H), 3.03 (s, 3H), 2.81 (s, 3H); ESI-MS (m/z) 437.0 (MH)$^+$;

1-(5-Chloro-6-(2H-1,2,3-triazol-2-yl)pyridin-3-yl)-3-(7-((1,3-dimethoxypropan-2-yl)(methyl)amino)-2-methylthiazolo[5,4-b]pyridin-6-yl)urea (Compound 46)

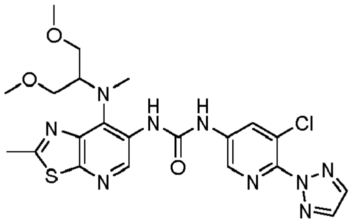

$^1$HNMR (400 MHz, DMSO-d6) δ 10.03 (s, 1H), 8.80 (s, 1H), 8.57 (d, $J$ = 2.3 Hz, 1H), 8.49 (d, $J$ = 2.3Hz, 1H), 8.40 (s, 1H), 8.17 (s, 2H), 3.85-3.76 (m, 1H), 3.74-3.65 (m, 2H), 3.58-3.50 (m, 2H), 3.23 (s, 6H), 3.06 (s, 3H), 2.82 (s, 3H); ESI-MS (m/z) 518.13 (MH)$^+$;

1-(5-Chloro-6-(2H-1,2,3-triazol-2-yl)pyridin-3-yl)-3-(7-((2-(4-fluorophenyl)-2-methoxyethyl)(methyl)amino)-2-methylthiazolo[5,4-b]pyridin-6-yl)urea (Compound 47)

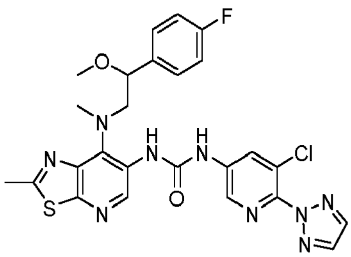

$^1$HNMR (400 MHz, DMSO-$d_6$) δ 10.11 (s, 1H), 8.90 (s, 1H), 8.57-8.50 (m, 3H), 8.17 (s, 2H), 7.28-7.20 (m, 2H), 7.12-7.02 (m, 2H), 4.40-4.30 (m, 1H), 3.71-3.58 (m, 2H), 3.12 (s, 3H), 3.00 (s, 3H), 2.81 (s, 3H); ESI-MS (m/z) 568.07 (MH)$^+$;

1-(5-Chloro-6-methoxypyridin-3-yl)-3-(7-(cyclopropyl(2-methoxyethyl)amino)-2-methylthiazolo[5,4-b]pyridin-6-yl)urea (Compound 48)

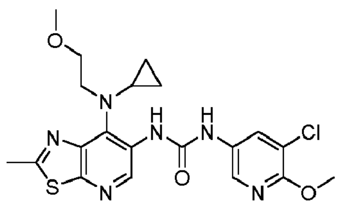

$^1$HNMR (400 MHz, DMSO-$d_6$) δ 9.55 (s, 1H), 9.00 (s, 1H), 8.17 (s, 1H), 8.13 (s, 1H), 8.10 (s, 1H), 3.91 (s, 3H), 3.62 -3.52 (m, 2H), 3.42-3.38 (m, 2H), 3.10 (s, 3H), 2.82 (s, 3H), 1.30-1.20 (m, 1H), 0.59-0.50 (m, 2H), 0.49-0.41 (m, 2H); ESI-MS (m/z) 463.09 $(MH)^+$;

1-(5-Chloro-6-(2H-1,2,3-triazol-2-yl)pyridin-3-yl)-3-(7-(cyclopropyl(2-methoxyethyl)amino)-2-methylthiazolo[5,4-b]pyridin-6-yl)urea (Compound 49)

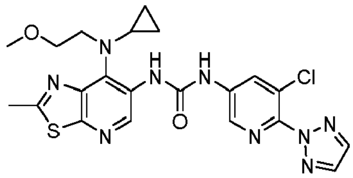

$^1$HNMR (500 MHz, DMSO-$d_6$) δ 10.13 (s, 1H), 8.96 (s, 1H), 8.55 (d, *J* = 2.3 Hz, 1H), 8.52 (d, *J* = 2.4 Hz, 1H), 8.31 (s, 1H), 8.16 (s, 2H), 3.63 (t, *J* = 5.8 Hz, 2H), 3.45-3.35 (m, 3H), 3.11 (s, 3H), 2.83 (s, 3H), 0.61-0.53 (m, 2H), 0.51-0.40 (m, 2H); ESI-MS (m/z) 500.22 $(MH)^+$;

(±)-1-(5-Chloro-6-(2H-1,2,3-triazol-2-yl)pyridin-3-yl)-3-(7-(3-(methoxymethyl)piperidin-1-yl)-2-methylthiazolo[5,4-b]pyridin-6-yl)urea (Compound 50)

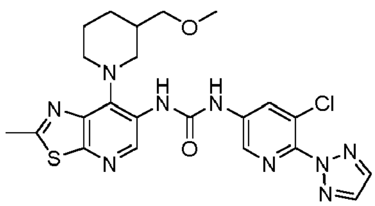

$^1$HNMR (400 MHz, CDC13) δ 9.19 (s, 1H, $D_2O$ exchangeable), 9.10 (s, 1H), 8.53 (s, 1H), 8.44 (s, 1H), 8.36 (s, 1H, $D_2O$ exchangeable), 7.94 (s, 2H), 3.94-3.92 (m, 1H), 3.84 -3.78 (m, 1H), 3.62-3.57 (m, 1H), 3.51-3.43 (m, 2H), 3.41 (s, 3H), 3.25-3.20 (m, 1H), 2.82 (s, 3H), 2.32-2.30 (m, 1H), 1.96-1.74 (m, 4H); ESI-MS (m/z) 514.06 $(MH)^+$;

(±)-1-(5-Chloro-6-methoxypyridin-3-yl)-3-(7-(3-(methoxymethyl)piperidin-1-yl)-2-methylthiazolo[5,4-b]pyridin-6-yl)urea (Compound 51)

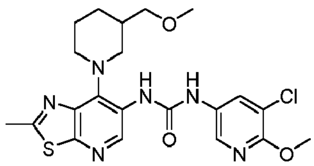

ESI-MS (m/z) 477.05 $(MH)^+$;
Chiral separation of racemic compound 51 was carried out using chiral column and afforded the below isomers 51a and 51b:

1-(5-Chloro-6-methoxypyridin-3-yl)-3-(7-(3-(methoxymethyl)piperidin-1-yl)-2-methylthiazolo[5,4-b]pyridin-6-yl)urea (Compound 51a)
Chiral HPLC RT: 4.02 min
$^1$HNMR (400 MHz, DMSO-$d_6$) δ 9.51(s, 1H, $D_2O$ exchangeable), 8.85 (s, 1H), 8.16 (d, *J* = 2.5 Hz, 1H), 8.13 (d, *J* = 2.5 Hz, 1H), 8.11 (s, 1H, $D_2O$ exchangeable), 3.92 (s, 3H), 3.37 (s, 3H), 3.37-3.21 (m, 5H), 3.07-3.01 (m, 1H), 2.81 (s, 3H), 2.17-2.15 (m, 1H), 1.89-1.72 (m, 3H), 1.24-1.17 (m, 1H); ESI-MS (m/z) 477.05 $(MH)^+$;

1-(5-chloro-6-methoxypyridin-3-yl)-3-(7-(3-(methoxymethyl)piperidin-1-yl)-2-methylthiazolo[5,4-b]pyridin-6-yl)urea (Compound 51b)
Chiral HPLC RT: 7.58 min $^1$HNMR (400 MHz, DMSO-$d_6$) δ 9.51 (s, 1H, $D_2O$ exchangeable), 8.85 (s, 1H), 8.16 (d, *J*= 2.5 Hz, 1H), 8.13 (d, *J*= 2.5 Hz, 1H), 8.11 (s, 1H, $D_2O$ exchangeable), 3.92 (s, 3H), 3.37 (s, 3H), 3.37-3.21

(m, 5H), 3.07-3.01 (m, 1H), 2.81 (s, 3H), 2.17-2.15 (m, 1H), 1.89-1.72 (m, 3H), 1.24-1.17 (m, 1H); ESI-MS (m/z) 477.05 $(MH)^+$;

(±)-1-(5-Chloro-6-(2H-1,2,3-triazol-2-yl)pyridin-3-yl)-3-(7-(3-methoxypiperidin-1-yl)-2-methylthiazolo[5,4-b]pyridin-6-yl)urea (Compound 52)

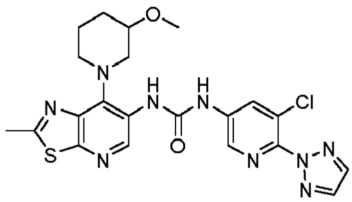

ESI-MS (m/z) 500.03 $(MH)^+$;
Chiral separation of racemic compound 52 was carried out using chiral column and afforded the below isomers 52a and 52b:

1-(5-Chloro-6-(2H-1,2,3-triazol-2-yl)pyridin-3-yl)-3-(7-(3-methoxypiperidin-1-yl)-2-methylthiazolo[5,4-b]pyridin-6-yl)urea (Compound 52a)
Chiral HPLC RT: 6.58 min
$^1$HNMR (400 MHz, DMSO-$d_6$) δ 10.04 (s, 1H), 8.69 (s, 1H), 8.58 (d, $J$ = 2.3 Hz, 1H), 8.51 (d, $J$= 2.3 Hz, 1H), 8.36 (s, 1H), 8.16 (s, 2H), 3.71-3.65 (m, 1H), 3.55-3.48 (m, 1H), 3.42-3.37 (m, 1H), 3.29 (s, 3H), 3.26-3.20 (m, 1H), 3.13-3.06 (m, 1H), 2.81 (s, 3H), 2.10-2.04 (m, 1H), 1.87-1.80 (m, 1H), 1.76-1.69 (m, 1H), 1.44-1.34 (m, 1H); ESI-MS (m/z) 500.06 (MH)+;

1-(5-Chloro-6-(2H-1,2,3-triazol-2-yl)pyridin-3-yl)-3-(7-(3-methoxypiperidin-1-yl)-2-methylthiazolo[5,4-b]pyridin-6-yl)urea (Compound 52b)
Chiral HPLC RT: 6.80 min
$^1$HNMR (400 MHz, DMSO-$d_6$) δ 10.06 (s, 1H), 8.69 (s, 1H), 8.58 (d, $J$ = 2.3 Hz, 1H), 8.51 (d, $J$ = 2.3 Hz, 1H), 8.38 (s, 1H), 8.16 (s, 2H), 3.71-3.67 (m, 1H), 3.51 (s, 1H), 3.42-3.38 (m, 1H), 3.29 (s, 3H), 3.26-3.20 (m, 1H), 3.12-3.05 (m, 1H), 2.81 (s, 3H), 2.10-2.04 (m, 1H), 1.82 (s, 1H), 1.76-1.69 (m, 1H), 1.42-1.36 (m, 1H); ESI-MS (m/z) 499.98 $(MH)^+$;

(±)-1-(5-Chloro-6-(2H-1,2,3-triazol-2-yl)pyridin-3-yl)-3-(7-((1-methoxypropan-2-yl)(methyl)amino)-2-methylthiazolo[5,4-b]pyridin-6-yl)urea (Compound 53)

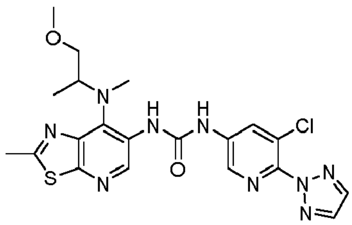

ESI-MS (m/z) 488.19 $(MH)^+$;
Chiral separation of racemic compound 53 was carried out using chiral column and afforded the below isomers 53a and 53b:

1-(5-Chloro-6-(2H-1,2,3-triazol-2-yl)pyridin-3-yl)-3-(7-((1-methoxypropan-2-yl)(methyl)amino)-2-methylthiazolo[5,4-b]pyridin-6-yl)urea (Compound 53a)
Chiral HPLC RT: 6.21 min
$^1$HNMR (400 MHz, $CDCl_3$) δ 9.25 (s, 1H), 8.93 (s, 1H), 8.62 (s, 1H), 8.36 (s, 1H), 8.06 (s, 1H), 7.95 (s, 2H), 3.91-3.85 (m, 1H), 3.76-3.71 (m, 1H), 3.67 (s, 3H), 3.64-3.60 (m, 1H), 3.17 (s, 3H), 2.83 (s, 3H), 1.35 (d, $J$ = 7.8 Hz, 3H); ESI-MS (m/z) 488.19 $(MH)^+$;

1-(5-Chloro-6-(2H-1,2,3-triazol-2-yl)pyridin-3-yl)-3-(7-((1-methoxypropan-2-yl)(methyl)amino)-2-methylthiazolo[5,4-b]pyridin-6-yl)urea (Compound 53b)
Chiral HPLC RT: 7.26 min
$^1$HNMR (400 MHz, Chloroform-*d*) δ 9.27 (s, 1H), 8.91 (s, 1H), 8.63 (s, 1H), 8.34 (s, 1H), 7.95 (s, 3H), 3.85-3.79 (m, 1H), 3.75-3.72 (m, 1H), 3.68 (s, 3H), 3.65-3.61 (m, 1H), 3.16 (s, 3H), 2.83 (s, 3H), 1.22 (d, *J* = 5.4 Hz, 3H); ESI-MS (m/z) 488.19 $(MH)^+$;

(±)-1-(5-Chloro-6-methoxypyridin-3-yl)-3-(7-((1-methoxypropan-2-yl)(methyl)amino)-2-methylthiazolo[5,4-b]pyridin-6-yl)urea (Compound 54)

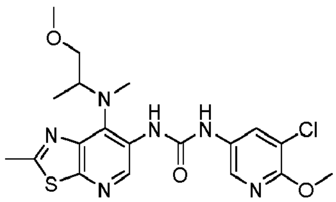

ESI-MS (m/z) 451.12 $(MH)^+$;
Chiral separation of racemic compound 54 was carried out using chiral column and afforded the below isomers 54a and 54b:

1-(5-Chloro-6-methoxypyridin-3-yl)-3-(7-((1-methoxypropan-2-yl)(methyl)amino)-2-methylthiazolo[5,4-b]pyridin-6-yl)urea (Compound 54a)
Chiral HPLC RT: 6.11 min
$^1$HNMR (400 MHz, DMSO-$d_6$) δ 9.40 (s, 1H), 8.85 (s, 1H), 8.25 (s, 1H), 8.15 (s, 2H), 3.91 (s, 3H), 3.79-3.72 (m, 1H), 3.60-3.54 (m, 1H), 3.33-3.28 (m, 1H), 3.23 (s, 3H), 2.97 (s, 3H), 2.81 (s, 3H), 1.15 (d, *J* = 6.5 Hz, 3H); ESI-MS (m/z) 451.12 $(MH)^+$;

1-(5-Chloro-6-methoxypyridin-3-yl)-3-(7-((1-methoxypropan-2-yl)(methyl)amino)-2-methylthiazolo[5,4-b]pyridin-6-yl)urea (Compound 54b)
Chiral HPLC RT: 5.60 min
$^1$HNMR (400 MHz, DMSO-$d_6$) δ 9.40 (s, 1H), 8.85 (s, 1H), 8.25 (s, 1H), 8.15 (s, 2H), 3.91 (s, 3H), 3.79-3.74 (m, 1H), 3.59-3.54 (m, 1H), 3.33 (m, 1H) 3.23 (s, 3H), 2.96 (s, 3H), 2.81 (s, 3H), 1.15 (d, *J* = 6.5 Hz, 3H); ESI-MS (m/z) 451.12 $(MH)^+$;

(±)-1-(5-Chloro-6-methoxypyridin-3-yl)-3-(7-((2-methoxypropyl)(methyl)amino)-2-methylthiazolo[5,4-b]pyridin-6-yl)urea (Compound 55)

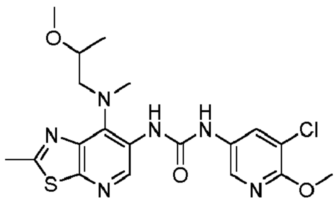

ESI-MS (m/z) 451.09 $(MH)^+$;
Chiral separation of racemic compound 55 was carried out using chiral column and afforded the below isomers 55a and 55b;

1-(5-Chloro-6-methoxypyridin-3-yl)-3-(7-((2-methoxypropyl)(methyl)amino)-2-methylthiazolo[5,4-b]pyridin-6-yl)urea (Compound 55a)
Chiral HPLC RT: 10.0 min $^1$HNMR (500 MHz, DMSO-$d_6$) δ 9.51 (s, 1H), 8.91 (s, 1H), 8.32 (s, 1H), 8.17 (d, *J* = 2.4 Hz, 1H), 8.13 (d, *J* = 2.4 Hz, 1H), 3.91 (s, 3H), 3.51-3.46 (m, 1H), 3.41-3.40 (m, 2H), 3.13 (s, 3H), 3.05 (s, 3H), 2.81 (s, 3H), 1.01 (d, *J* = 5.8 Hz, 3H); ESI-MS (m/z) 451.09 $(MH)^+$;

1-(5-Chloro-6-methoxypyridin-3-yl)-3-(7-((2-methoxypropyl)(methyl)amino)-2-methylthiazolo[5,4-b]pyridin-6-yl)urea (Compound 55b)

Chiral HPLC RT: 11.43 min

$^1$HNMR (500 MHz, DMSO-$d_6$) δ 9.51 (s, 1H), 8.91 (s, 1H), 8.32 (s, 1H), 8.17 (d, *J* = 2.4 Hz, 1H), 8.13 (d, *J* = 2.4 Hz, 1H), 3.91 (s, 3H), 3.51-3.49 (m, 1H), 3.41-3.40 (m, 2H), 3.13 (s, 3H), 3.05 (s, 3H), 2.81 (s, 3H), 1.01 (d, *J* = 5.8 Hz, 3H); ESI-MS (m/z) 451.12 $(MH)^+$;

(±)-1-(5-Chloro-6-(2H-1,2,3-triazol-2-yl)pyridin-3-yl)-3-(7-(1-methoxy-2,2-dimethylpropyl)-2-methylthiazolo[5,4-b]pyridin-6-yl)urea (Compound 56)

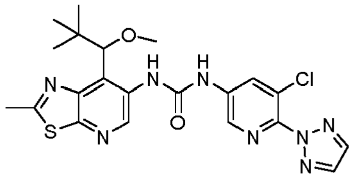

ESI-MS (m/z) 487.30 $(MH)^+$;

Chiral separation of racemic compound 56 was carried out using chiral column and afforded the below isomers 56a and 56b:

1-(5-Chloro-6-(2H-1,2,3-triazol-2-yl)pyridin-3-yl)-3-(7-(1-methoxy-2,2-dimethylpropyl)-2-methylthiazolo[5,4-b]pyridin-6-yl)urea (Compound 56a)

Chiral HPLC RT: 6.14 min

$^1$HNMR (400 MHz, DMSO-$d_6$) δ 10.58 (s, $D_2O$ exchangeable, 1H), 9.07 (s, 1H), 8.81 (s, $D_2O$ exchangeable, 1H), 8.56 (d, *J* = 2.4 Hz, 1H), 8.51 (d, *J* = 2.3 Hz, 1H), 8.16 (s, 2H), 5.16 (s, 1H), 3.38 (s, 3H), 2.85 (s, 3H), 0.97 (s, 9H); ESI-MS (m/z) 487.30 $(MH)^+$;

1-(5-chloro-6-(2H-1,2,3-triazol-2-yl)pyridin-3-yl)-3-(7-(1-methoxy-2,2-dimethylpropyl)-2-methylthiazolo[5,4-b]pyridin-6-yl)urea (Compound 56b)

Chiral HPLC RT: 7.13 min $^1$HNMR (400 MHz, DMSO-$d_6$) δ 10.57 (s, $D_2O$ exchangeable, 1H), 9.07 (s, 1H), 8.81 (s, $D_2O$ exchangeable, 1H), 8.56 (d, *J* = 2.3 Hz, 1H), 8.51 (d, *J* = 2.3 Hz, 1H), 8.16 (s, 2H), 5.16 (s, 1H), 3.37 (s, 3H), 2.84 (s, 3H), 0.97 (s, 9H); ESI-MS (m/z) 487.30 $(MH)^+$;

(±)-1-(6-(2H-1,2,3-Triazol-2-yl)-5-(trifluoromethyl)pyridin-3-yl)-3-(7-(1-methoxy-2,2-dimethylpropyl)-2-methylthiazolo[5,4-b]pyridin-6-yl)urea (Compound 57)

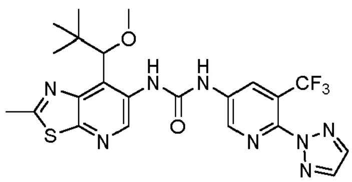

ESI-MS (m/z) 521.32 $(MH)^+$;

Chiral separation of racemic compound 57 was carried out using chiral column and afforded the below isomers 57a and 57b:

1-(6-(2H-1,2,3-Triazol-2-yl)-5-(trifluoromethyl)pyridin-3-yl)-3-(7-(1-methoxy-2,2-dimethylpropyl)-2-methylthiazolo[5,4-b]pyridin-6-yl)urea (Compound 57a)

Chiral HPLC RT: 6.26 min

$^1$HNMR (400 MHz, DMSO-$d_6$) d 10.73 (s, 1H), 9.10 (s, 1H), 8.89 (d, *J* = 2.5 Hz, 1H), 8.86 (s, 1H), 8.72 (d, *J* = 2.5 Hz, 1H), 8.18 (s, 2H), 5.17 (s, 1H), 3.38 (s, 3H), 2.85 (s, 3H), 0.97 (s, 9H); ESI-MS (m/z) 521.32 $(MH)^+$;

1-(6-(2H-1,2,3-Triazol-2-yl)-5-(trifluoromethyl)pyridin-3-yl)-3-(7-(1-methoxy-2 ,2-dimethylpropyl)-2-methylthiazolo[5,4-b]pyridin-6-yl)urea (Compound 57b)

Chiral HPLC RT: 7.20 min

$^1$HNMR (400 MHz, DMSO-$d_6$) d 10.74 (s, 1H), 9.10 (s, 1H), 8.89 (d, *J* = 2.5 Hz, 1H), 8.86 (s, 1H), 8.72 (d, *J* = 2.5 Hz, 1H), 8.18 (s, 2H), 5.17 (s, 1H), 3.38 (s, 3H), 2.85 (s, 3H), 0.97 (s, 9H); ESI-MS (m/z) 521.32 $(MH)^+$;

1-(5-Chloro-6-methoxypyridin-3-yl)-3-(7-(3,6-dihydro-2H-pyran-4-yl)-2-methylthiazolo[5,4-b]pyridin-6-yl)urea (Compound 58)

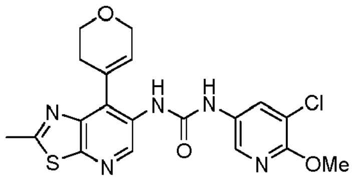

$^1$HNMR (400 MHz, DMSO-$d_6$) δ 9.36 (s, 1H), 8.94 (s, 1H), 8.15 (s, 2H), 8.12 (s, 1H), 5.99-5.95 (m, 1H), 4.29-4.27 (m, 2H), 3.95-3.85 (m, 5H), 2.82 (s, 3H), 2.52-2.48 (m, 2H); ESI-MS (m/z) 432.04 $(MH)^+$;

1-(5-Chloro-6-(2H-1,2,3-triazol-2-yl)pyridin-3-yl)-3-(7-(cyclohex-1-en-1-yl)-2-methylthiazolo[5,4-b]pyridin-6-yl)urea (Compound 59)

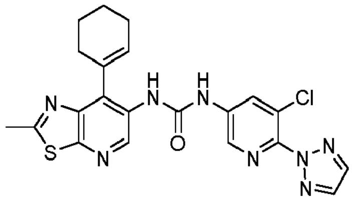

$^1$HNMR (400 MHz, DMSO-$d_6$) d 9.98 (s, 1H), 8.88 (s, 1H), 8.55 (d, $J$ = 2.5 Hz, 1H), 8.50 (d, $J$ = 2.5 Hz, 1H), 8.30 (s, 1H), 8.17 (s, 2H), 5.87-5.82 (m, 1H), 2.83 (s, 3H), 2.40-2.30 (m, 2H), 2.27-2.24 (m, 2H), 1.78-1.72 (m, 4H); ESI-MS (m/z) 467.04 $(MH)^+$;

1-(5-Chloro-6-cyanopyridin-3-yl)-3-(7-cyclopropyl-2-methylthiazolo[5,4-b]pyridin-6-yl)urea (Compound 60)

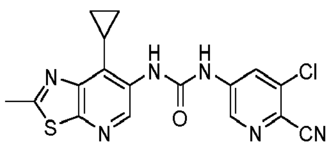

$^1$HNMR (400 MHz, DMSO-$d_6$) δ 10.10 (s, 1H), 8.99 (s, 1H), 8.77 (d, $J$ = 2.2 Hz, 1H), 8.67 (s, 1H), 8.54 (d, $J$ = 2.2 Hz, 1H), 2.92 (s, 3H), 2.33 - 2.29 (m, 1H), 1.70-1.66 (m, 2H), 1.36-1.23 (m, 2H); ESI-MS (m/z) 385.07 $(MH)^+$;

1-(5-Chloro-6-(1H-1,2,3-triazol-1-yl)pyridin-3-yl)-3-(7-cyclopropyl-2-methylthiazolo[5,4-b]pyridin-6-yl)urea (Compound 61)

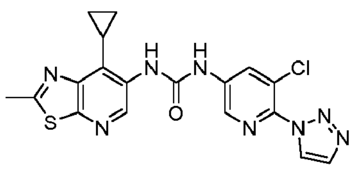

$^1$HNMR (400 MHz, DMSO-$d_6$) δ 9.78 (s, 1H), 8.78 (s, 1H), 8.65-8.58 (m, 3H), 8.51 (dd, $J$ = 2.3 Hz, 1H), 7.99 (dd, $J$ = 5.0 Hz, 1H), 2.81 (s, 3H), 2.27-2.18 (m, 1H), 1.62-1.53 (m, 2H), 1.21-1.12 (m, 2H); ESI-MS (m/z) 426.96 $(MH)^+$;

1-(5-Cyano-6-methoxypyridin-3-yl)-3-(7-cyclopropyl-2-methylthiazolo[5,4-b]pyridin-6-yl)urea (Compound 62)

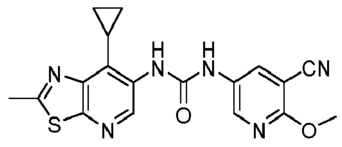

$^1$HNMR (400 MHz, DMSO-$d_6$) δ 9.29 (s, 1H), 8.64 (s, 1H), 8.59 (s, 1H), 8.49 (d, *J* = 2.8 Hz, 1H), 8.36 (d, *J* = 2.5 Hz, 1H), 3.97 (s, 3H), 2.80 (s, 3H), 2.21-217 (m, 1H), 1.55-1.53 (m, 2H), 1.15-1.10 (m, 2H); ESI-MS (m/z) 380.97 $(MH)^+$;

1-(3-Chloro-4-(1,3,4-oxadiazol-2-yl)phenyl)-3-(7-cyclopropyl-2-methylthiazolo[5,4-b]pyridin-6-yl)urea (Compound 63)

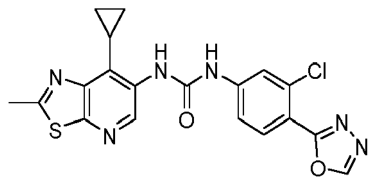

$^1$HNMR (400 MHz, DMSO-$d_6$) δ 9.64 (s, 1H), 9.40 (s, 1H), 8.62 (s, 1H), 8.60 (s, 1H), 7.98 (d, *J* = 2.0 *Hz,* 1H), 7.93 (d, *J* = 8.5 *Hz,* 1H), 7.55 (dd, *J* = 8.5, 2.0 *Hz,* 1H), 2.81 (s, 3H), 2.20 (m, 1H), 1.63-1.50 (m, 2H), 1.21-1.08 (m, 2H); ESI-MS (m/z) 426.98 $(MH)^+$;

1-(3-Chloro-4-(2H-1,2,3-triazol-2-yl)phenyl)-3-(7-(1-(methoxymethyl)cyclopropyl)-2-methylthiazolo[5,4-b]pyridin-6-yl)urea (Compound 64)

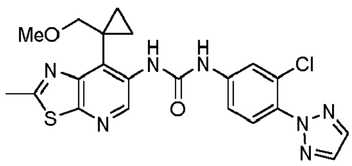

$^1$HNMR (400 MHz, DMSO-$d_6$) δ 10.20 (s, 1H, $D_2O$ exchangeable), 8.87 (s, 1H), 8.15-8.13 (m, 3H), 8.00 (s, 1H), 7.63-7.56 (m, 2H), 3.56 (s, 2H), 3.24 (s, 3H), 2.86 (s, 3H), 1.24-1.08 (m, 2H), 0.91-0.85 (m, 2H); ESI-MS (m/z) 470.09 $(MH)^+$;

1-(5-Chloro-6-methoxypyridin-3-yl)-3-(7-(1-(methoxymethyl)cyclopropyl)-2-methylthiazolo[5,4-b]pyridin-6-yl)urea (Compound 65)

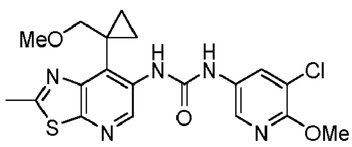

$^1$HNMR (400 MHz, DMSO-$d_6$) δ 9.80 (s, 1H), 8.86 (s, 1H), 8.18 (d, *J* = 2.5 *Hz,* 1H), 8.16 (d, *J* = 2.5 *Hz,* 1H), 8.05 (s, 1H), 3.92 (s, 3H), 3.53 (s, 2H), 3.21 (s, 3H), 2.84 (s, 3H), 1.21-1.09 (m, 2H), 0.98-0.80 (m, 2H); ESI-MS (m/z) 434.29 $(MH)^+$;

1-(5-Chloro-6-methoxypyridin-3-yl)-3-(2-methyl-7-(1,4-oxazepan-4-yl)thiazolo[5,4-b]pyridin-6-yl)urea (Compound 66)

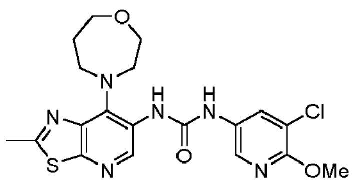

$^1$HNMR (400 MHz, DMSO-$d_6$) δ 9.33 (s, 1H), 8.67 (s, 1H), 8.20-8.11 (m, 3H), 3.91 (s, 3H), 3.83 (t, $J$ = 5.1 Hz, 4H), 3.65- 3.55 (m, 4H), 2.80 (s, 3H), 2.08-1.98 (m, 2H); ESI-MS 449.0 $(MH)^+$;

1-(5-Chloro-6-(2H-1,2,3-triazol-2-yl)pyridin-3-yl)-3-(2-methyl-7-(1,4-oxazepan-4-yl)thiazolo[5,4-b]pyridin-6-yl)urea (Compound 67)

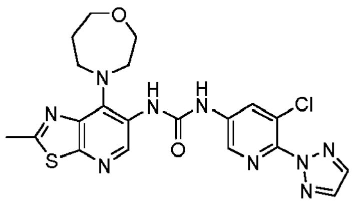

$^1$HNMR (400 MHz, DMSO-$d_6$) δ 9.93 (s, 1H), 8.63 (s, 1H), 8.58 (d, J = 2.4 Hz, 1H), 8.51 (d, J = 2.4 Hz, 1H), 8.43 (s, 1H), 8.16 (s, 2H), 3.88-3.80 (m, 4H), 3.69-3.61 (m, 4H), 2.81 (s, 3H), 2.07-2.02 (m, 2H); ESI-MS (m/z) 486.0 $(MH)^+$;

1-(3-Chloro-4-(2H-1,2,3-triazol-2-yl)phenyl)-3-(7-(cyclopropyl(2-methoxyethyl)amino)-2-methylthiazolo[5,4-b]pyridin-6-yl)urea (Compound 68)

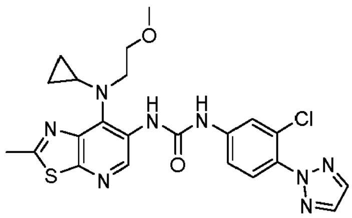

$^1$HNMR (400 MHz, DMSO-$d_6$) δ 9.88 (s, 1H), 8.99 (s, 1H), 8.17 (s, 1H), 8.12 (s, 2H), 7.98 (d, $J$ = 2.3 Hz, 1H), 7.60 (d, $J$ = 8.7 Hz, 1H), 7.53 (dd, $J$ = 8.8, 2.3 Hz, 1H), 3.60 (t, $J$ = 5.8 Hz, 2H), 3.40 (t, $J$ = 5.7 Hz, 2H), 3.11 (s, 3H), 2.83 (s, 3H), 1.27-1.20 (m, 1H), 0.60-0.53 (m, 2H), 0.50-0.43 (m, 2H); ESI-MS (m/z) 499.0 $(MH)^+$;

1-(6-(2H-1,2,3-Triazol-2-yl)-5-(trifluoromethyl)pyridin-3-yl)-3-(7-(cyclopropyl(2-methoxyethyl)amino)-2-methylthiazolo[5,4-b]pyridin-6-yl)urea (Compound 69)

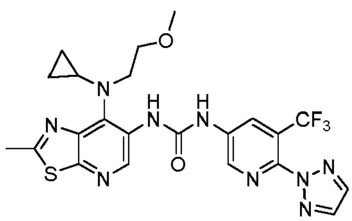

$^1$HNMR (400 MHz, DMSO-$d_6$) δ 10.27 (s, 1H), 8.96 (s, 1H), 8.85 (d, $J$ = 2.5 Hz, 1H), 8.73 (d, $J$ = 2.5 Hz, 1H), 8.35 (s, 1H), 8.18 (s, 2H), 3.64 (t, $J$ = 5.8 Hz, 2H), 3.41 (t, $J$ = 5.8 Hz, 2H), 3.37-3.31(m, 1H), 3.11 (s, 3H), 2.83 (s, 3H), 0.63-0.55 (m, 2H),0.50-0.44 (m, 2H); ESI-MS (m/z) 534.4 $(MH)^+$; and

1-(4-(2H-1,2,3-Triazol-2-yl)-3-(trifluoromethyl)phenyl)-3-(7-(cyclopropyl(2-methoxyethyl)amino)-2-methylthiazolo[5,4-b]pyridin-6-yl)urea (Compound 70)

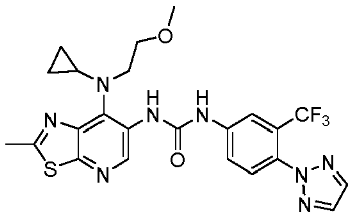

$^1$HNMR (400 MHz, DMSO-$d_6$) δ 10.03 (s, 1H), 9.00 (s, 1H), 8.22-8.16 (m, 2H), 8.13 (s, 2H), 7.90 (dd, *J* = 8.7, 2.5 Hz, 1H), 7.69 (d, *J* = 8.7 Hz, 1H), 3.62 (t, *J* = 5.8 Hz, 2H), 3.39 (t, *J* = 5.8 Hz, 2H), 3.36-3.31 (m, 1H), 3.11 (s, 3H), 2.83 (s, 3H), 0.60-0.54 (m, 2H), 0.50-0.43 (m, 2H); ESI-MS (m/z) 533.1 $(MH)^+$.

**Example-68:** Preparation of (±)-1-(6-(2H-1,2,3-Triazol-2-yl)-5-(trifluoromethyl)pyridin-3-yl)-3-(7-(1-hydroxyethyl)-2-methylthiazolo[5,4-b]pyridin-6-yl)urea (Compound 71)

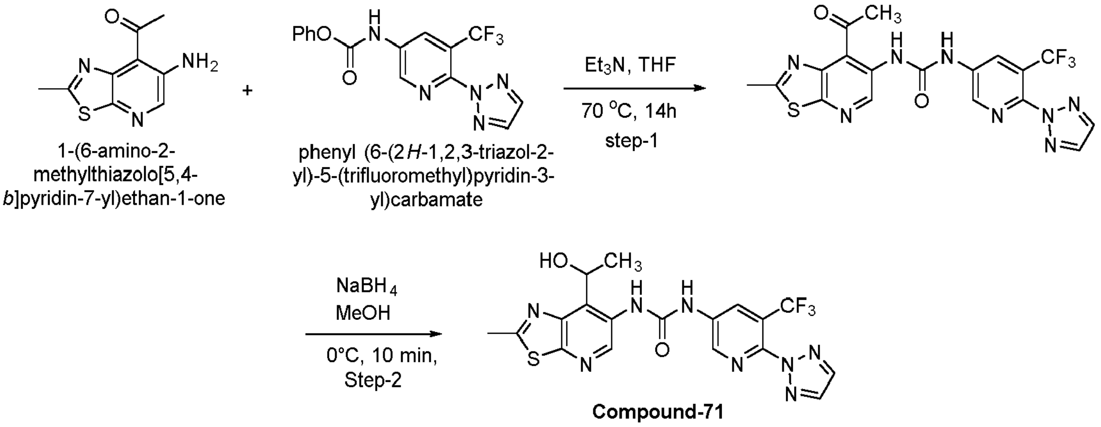


1-(6-amino-2-methylthiazolo[5,4-*b*]pyridin-7-yl)ethan-1-one

phenyl (6-(2*H*-1,2,3-triazol-2-yl)-5-(trifluoromethyl)pyridin-3-yl)carbamate

**Compound-71**

Step-1: 1-(6-(2H-1,2,3-Triazol-2-yl)-5-(trifluoromethyl)pyridin-3-yl)-3-(7-acetyl-2-methylthiazolo[5,4-b]pyridin-6-yl)urea: To a stirred solution of 1-(6-amino-2-methylthiazolo[5,4-b]pyridin-7-yl) ethan-1-one (1 g, 4.83 mmol) and phenyl (6-(2H-1,2,3-triazol-2-yl)-5-(trifluoromethyl)pyridin-3-yl)carbamate (1.854 g, 5.31 mmol) in THF (20 mL) was added $Et_3N$ (1.34 mL, 9.65 mmol) and reaction mixture was heated at 70°C for 14 h. Progress of the reaction was monitored on TLC. After completion of reaction, water (25 mL) was added and the reaction mixture was extracted with ethyl acetate (25 mL x 3). Combined organic layer was washed with saturated brine solution (10 mL), dried over anhydrous sodium sulfate and filtered. Filtrate was rotary evaporated and residue was purified by flash column chromatography (silica gel) to afford 0.40 g (18%) of the titled product as a white solid. $^1$HNMR (400 MHz, DMSO-$d_6$) δ 10.70 (s, 1H), 8.93 (s, 1H), 8.61 (s, 1H), 8.31 (s, 1H), 8.29 (s, 2H), 7.44 (s, 1H), 2.85 (s, 3H), 2.02 (s, 3H); ESI-MS (m/z) 463.18 $(MH)^+$.

Step-2: 1-(6-(2H-1,2,3-Triazol-2-yl)-5-(trifluoromethyl)pyridin-3-yl)-3-(7-(1-hydroxyethyl)-2-methylthiazolo[5,4-b]pyridin-6-yl)urea: To a stirred solution of step-1 intermediate (200 mg, 0.433 mmol) was added $NaBH_4$ (32.7 mg, 0.865 mmol) in MeOH (5 mL) and reaction mass was stirred at 0°C for 10 min. After completion of reaction, water (10 mL) was added and the reaction mixture was extracted with ethyl acetate (10 mL x 4). Combined organic layer was washed with saturated brine solution (10 mL), dried over anhydrous sodium sulfate and filtered. Filtrate was rotary evaporated and residue was purified by flash column chromatography (silica gel) to afford 0.090 g (45%) of the titled product as a white solid. $^1$HNMR (400 MHz, DMSO-$d_6$) δ 10.77 (s, 1H), 9.35 (s, 1H), 9.12 (s, 1H), 8.88 (s, 1H), 8.74 (s, 1H), 8.18 (s, 2H), 6.60 (s, 1H), 5.88 (d, *J* = 8.6 Hz, 1H), 2.85 (d, *J* = 3.8 Hz, 3H), 1.51 (s, 3H); ESI-MS (m/z) 465.12 $(MH)^+$.

Chiral separation of racemic compound 71 was carried out using chiral column and afforded the below isomers 71a and 71b:

1-(6-(2H-1,2,3-Triazol-2-yl)-5-(trifluoromethyl)pyridin-3-yl)-3-(7-(1-hydroxyethyl)-2-methylthiazolo[5,4-b]pyridin-6-yl)urea (Compound 71a)
Chiral HPLC RT: 3.67 min
$^1$HNMR (400 MHz, DMSO-$d_6$) δ 10.77 (s, 1H), 9.36 (s, 1H), 9.13 (s, 1H), 8.89 (s, 1H), 8.74 (s, 1H), 8.18 (s, 2H), 6.70-6.50 (m, 1H), 5.90-5.86 (m, 1H), 2.85 (s, 3H), 1.52 (d, *J*= 6.2 Hz, 3H); ESI-MS (m/z) 465.31 $(MH)^+$;

1-(6-(2H-1,2,3-Triazol-2-yl)-5-(trifluoromethyl)pyridin-3-yl)-3-(7-(1-hydroxyethyl)-2-methylthiazolo[5,4-b]pyridin-6-yl)urea (Compound 71b)
Chiral HPLC RT: 5.03 min
$^1$HNMR (400 MHz, DMSO-$d_6$) $\delta$ 10.77 (s, 1H), 9.36 (s, 1H), 9.13(s, 1H), 8.89 (s, 1H), 8.74 (s, 1H), 8.18 (s, 2H), 6.70-6.50 (m, 1H) 5.90-5.86 (m, 1H), 2.85 (s, 3H), 1.52 (d, *J* = 6.2 Hz, 3H); ESI-MS (m/z) 465.31 (MH)$^+$.

**Example-69:** Preparation of (±)-1-(6-(2H-1,2,3-Triazol-2-yl)-5-(trifluoromethyl)pyridin-3-yl)-3-(7-(1-fluoroethyl)-2-methylthiazolo[5,4-b]pyridin-6-yl)urea (Compound 72)

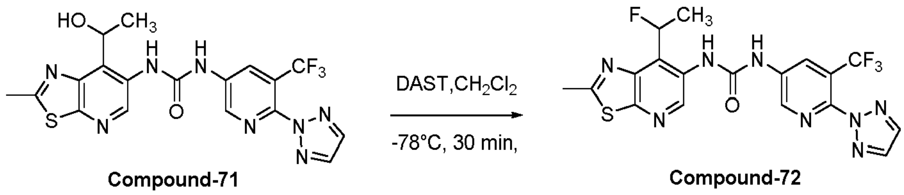

**Compound-71** **Compound-72**

To a solution of compound 71 (180 mg, 0.388 mmol) in dichloromethane (5 mL), was added DAST (0.077 mL, 0.581 mmol) dropwise at -78°C and reaction mixture was continued to stir 30 min. After completion of the reaction, sat. $NaHCO_3$ solution (10 mL) was added and the reaction mixture was extracted with ethyl acetate (20 mL x 3). Combined organic layer was washed with saturated brine solution (10 mL), dried over anhydrous sodium sulfate and filtered. Filtrate was rotary evaporated and residue was purified by flash column chromatography (silica gel) to afford 0.080 g (44%) of the titled product as a white solid. $^1$HNMR (400 MHz, DMSO-$d_6$) $\delta$ 10.24 (s, 1H), 8.85 (s, 2H), 8.72 (s, 2H), 8.18 (s, 2H), 6.57-6.38 (m, 1H), 2.89 (s, 3H), 1.86 (dd, *J*= 23.6, 6.6 Hz, 3H); ESI-MS (m/z) 467.12 (MH)$^+$.

Chiral separation of racemic compound 72 was carried out using chiral column and afforded the below isomers 72a and 72b:

1-(6-(2H-1,2,3-triazol-2-yl)-5-(trifluoromethyl)pyridin-3-yl)-3-(7-(1-fluoroethyl)-2-methylthiazolo[5,4-b]pyridin-6-yl)urea (Compound 72a)
Chiral HPLC RT = 4.76 min
$^1$HNMR (400 MHz, DMSO-$d_6$) $\delta$ 10.23 (s, $D_2O$ exchangeable, 1H), 8.84 (s, 2H), 8.72 (d, *J* = 3.6 Hz, 1H overlap with bs, $D_2O$ exchangeable, 1H), 8.18 (s, 2H), 6.59-6.37 (dq, *J* = 47.4, 6.5 Hz, 1H), 2.88 (s, 3H), 1.85 (dd, *J* = 23.8, 6.5 Hz, 3H); ESI-MS (m/z) 467.12 (MH)$^+$;

1-(6-(2H-1,2,3-triazol-2-yl)-5-(trifluoromethyl)pyridin-3-yl)-3-(7-(1-fluoroethyl)-2-methylthiazolo[5,4-b]pyridin-6-yl)urea (Compound 72b)
Chiral HPLC RT = 5.73 min
$^1$HNMR (400 MHz, DMSO-$d_6$) $\delta$ 10.24 (s, $D_2O$ exchangeable, 1H), 8.85 (s, 2H), 8.72 (d, *J* = 3.6 Hz, 1H overlap with bs, $D_2O$ exchangeable, 1H), 8.18 (s, 2H), 6.47 (dq, *J* = 47.4, 6.5 Hz, 1H), 2.89 (s, 3H), 1.86 (dd, *J* = 23.6, 6.6 Hz, 3H); ESI-MS (m/z) 467.13 (MH)$^+$.

**Example-70:** Preparation of 1-(5-Chloro-6-(2-(1-methylpiperidin-4-yl)ethoxy)pyridin-3-yl)-3-(7-cyclopropyl-2-methylthiazolo[5,4-b]pyridin-6-yl)urea (Compound 73).

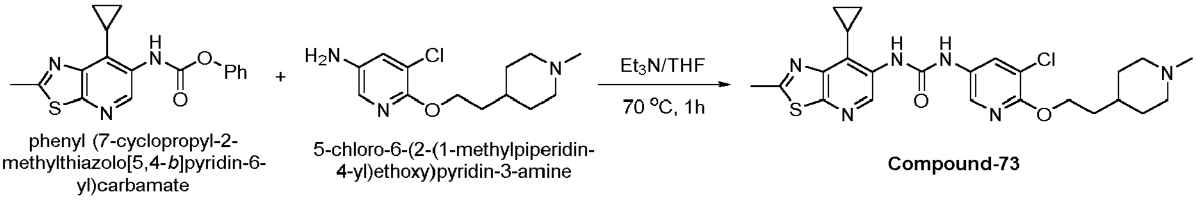

phenyl (7-cyclopropyl-2-methylthiazolo[5,4-*b*]pyridin-6-yl)carbamate
5-chloro-6-(2-(1-methylpiperidin-4-yl)ethoxy)pyridin-3-amine
**Compound-73**

The titled compound was prepared from the corresponding intermediates by following the similar procedure described for Example-66. $^1$HNMR (400 MHz, DMSO-$d_6$) $\delta$ 9.33 (s, 1H), 8.68 (s, 1H), 8.54 (s, 1H), 8.11-8.07 (m, 2H),

4.32-4.28 (m, 2H), 2.82-2.77 (m, 5H), 2.21 (s, 3H), 2.00-1.98 (m, 2H), 1.72-1.63 (m, 4H), 1.49-1.41 (m, 3H), 1.27-1.18 (m, 3H), 1.11-1.09 (m, 2H); ESI-MS (m/z) 501.0 (MH)$^+$.

**Example-71:** Preparation of (±)-1-(6-(2H-1,2,3-Triazol-2-yl)-5-(trifluoromethyl)pyridin-3-yl)-3-(7-(1-(dimethylamino)ethyl)-2-methylthiazolo[5,4-b]pyridin-6-yl)urea (Compound 74)

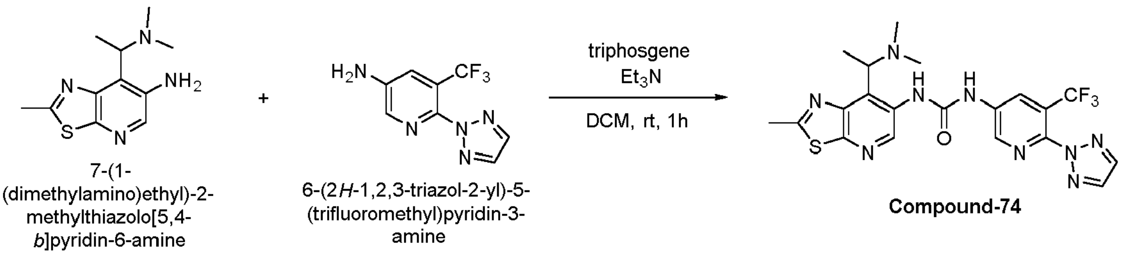


7-(1-(dimethylamino)ethyl)-2-methylthiazolo[5,4-*b*]pyridin-6-amine

6-(2*H*-1,2,3-triazol-2-yl)-5-(trifluoromethyl)pyridin-3-amine

**Compound-74**

A solution of 6-(2H-1,2,3-triazol-2-yl)-5-(trifluoromethyl)pyridin-3-amine (145 mg, 0.63 mmol) and triethyl amine (265 μL, 1.90 mmol) in DCM (5 mL) was added to a (0°C) cooled and stirred solution of triphosgene (62 mg, 0.209 mmol) in DCM (2 mL). The resulting mixture was stirred at 0°C for 20 min and then a solution of 7-(1-(dimethylamino)ethyl)-2-methylthiazolo[5,4-b]pyridin-6-amine (120 mg, 0.508 mmol) in DCM (2 mL) was added dropwise to the above mixture. The resulting mixture was then continued to stir at 0°C for 1h. The reaction mass was warmed to room temperature and stirred for 1h. The réaction mixture was rotary evaporated and the crude product was purified by flash column chromatography (2% MeOH in DCM as eluent) to afford 130 mg (42%) of the desired product as white solid. ESI-MS (m/z) 492.16 (MH)$^+$.

Chiral separation of racemic compound 74 was carried out using chiral column and afforded the below isomers 74a and 74b:

1-(6-(2H-1,2,3-Triazol-2-yl)-5-(trifluoromethyl)pyridin-3-yl)-3-(7-(1-(dimethylamino)ethyl)-2-methylthiazolo[5,4-b]pyridin-6-yl)urea (compound 74a)
Chiral HPLC RT: 5.74 min
$^1$HNMR (400 MHz, DMSO-$d_6$) δ 10.79 (s, 1H), 10.51 (s, 1H), 9.17 (s, 1H), 8.95 (d, *J* = 2.5 Hz, 1H), 8.73 (d, *J* = 2.5 Hz, 1H), 8.18 (s, 2H), 4.42 (q, *J* = 6.5 Hz, 1H), 2.84 (s, 3H), 2.31 (s, 6H), 1.41 (d, *J* = 6.5 Hz, 3H); ESI-MS (m/z) 492.2 (MH)$^+$;

1-(6-(2H-1,2,3-Triazol-2-yl)-5-(trifluoromethyl)pyridin-3-yl)-3-(7-(1-(dimethylamino)ethyl)-2-methylthiazolo[5,4-b]pyridin-6-yl)urea (compound 74b)
Chiral HPLC RT: 6.59 min
$^1$HNMR (400 MHz, DMSO-$d_6$) δ 10.79 (s, 1H), 10.51 (s, 1H), 9.17 (s, 1H), 8.95 (d, *J* = 2.5 Hz, 1H), 8.73 (d, *J* = 2.5 Hz, 1H), 8.18 (s, 2H), 4.42 (q, *J* = 6.5 Hz, 1H), 2.84 (s, 3H), 2.31 (s, 6H), 1.41 (d, *J* = 6.5 Hz, 3H); ESI-MS (m/z) 492.2 (MH)$^+$.

**Example-72:** The following compounds were prepared by using the similar procedure described for example-71 from the appropriate intermediates:

(±)-1-(6-(2H-1,2,3-Triazol-2-yl)-5-(trifluoromethyl)pyridin-3-yl)-3-(7-(1-(dimethylamino)propyl)-2-methylthiazolo[5,4-b]pyridin-6-yl)urea (Compound 75)

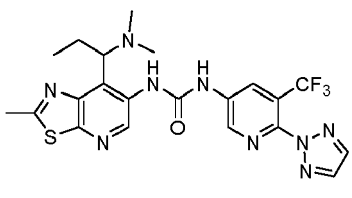

ESI-MS (m/z) 506.07 (MH)$^+$
Chiral separation of racemic compound 75 was carried out using chiral column and afforded the below isomers 75a

and 75b:

1-(6-(2H-1,2,3-Triazol-2-yl)-5-(trifluoromethyl)pyridin-3-yl)-3-(7-(1-(dimethylamino)propyl)-2-methylthiazolo[5,4-b]pyridin-6-yl)urea (Compound 75a)
Chiral HPLC RT 6.42 min
$^1$HNMR (400 MHz, DMSO-$d_6$) δ 10.63 (s, 1H), 10.48 (s, 1H), 9.12 (s, 1H), 8.94 (d, $J$ = 2.5 Hz, 1H), 8.72 (d, $J$ = 2.5 Hz, 1H), 8.18 (s, 2H), 4.34-4.27 (m, 1H), 2.83 (s, 3H), 2.31 (s, 6H), 2.13-1.98 (m, 1H), 1.95-1.74 (m, 1H), 0.59 (t, $J$ = 7.5 Hz, 3H); ESI-MS (m/z) 506.1 $(MH)^+$;

1-(6-(2H-1,2,3-Triazol-2-yl)-5-(trifluoromethyl)pyridin-3-yl)-3-(7-(1-(dimethylamino)propyl)-2-methylthiazolo[5,4-b]pyridin-6-yl)urea (Compound 75b)
Chiral HPLC RT 7.64 min
$^1$HNMR (400 MHz, DMSO-$d_6$) δ 10.64 (s, 1H), 10.49 (s, 1H), 9.13 (s, 1H), 8.94 (d, $J$ = 2.5 Hz, 1H), 8.72 (d, $J$ = 2.5 Hz, 1H), 8.18 (s, 2H), 4.34-4.27 (m, 1H), 2.83 (s, 3H), 2.31 (s, 6H), 2.13-1.98 (m, 1H), 1.96-1.73 (m, 1H), 0.58 (t, $J$ = 7.5 Hz, 3H); ESI-MS (m/z) 506.1 $(MH)^+$;

(±)-1-(6-(2H-1,2,3-Triazol-2-yl)-5-(trifluoromethyl)pyridin-3-yl)-3-(7-(cyclopropyl(dimethylamino)methyl)-2-methylthiazolo[5,4-b]pyridin-6-yl)urea (Compound 76)

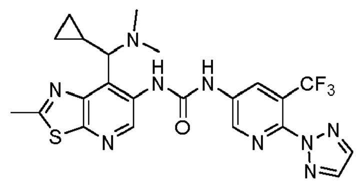

ESI-MS (m/z) 518.32 $(MH)^+$
Chiral separation of racemic compound 76 was carried out using chiral column and afforded the below isomers 76a and 76b:

1-(6-(2H-1,2,3-Triazol-2-yl)-5-(trifluoromethyl)pyridin-3-yl)-3-(7-(cyclopropyl(dimethylamino)methyl)-2-methylthiazolo[5,4-b]pyridin-6-yl)urea (Compound 76a)
Chiral HPLC RT: 5.75 min
$^1$HNMR (400 MHz, DMSO-$d_6$) δ 10.78 (s, 1H), 10.49 (s, 1H), 9.17 (s, 1H), 8.94 (d, $J$ = 2.5 Hz, 1H), 8.73 (d, $J$ = 2.5 Hz, 1H), 8.18 (s, 2H), 3.52 (d, $J$ = 9.8 Hz, 1H), 2.82 (s, 3H), 2.38 (s, 6H), 0.88-0.78 (m, 2H), 0.64-0.55 (m, 1H), 0.26-0.11 (m, 2H); ESI-MS (m/z) 518.30 $(MH)^+$;

1-(6-(2H-1,2,3-Triazol-2-yl)-5-(trifluoromethyl)pyridin-3-yl)-3-(7-(cyclopropyl(dimethylamino)methyl)-2-methylthiazolo[5,4-b]pyridin-6-yl)urea (Compound 76b)
Chiral HPLC RT: 6.48 min
$^1$HNMR (400 MHz, DMSO-$d_6$) δ 10.78 (s, 1H), 10.49 (s, 1H), 9.16 (s, 1H), 8.94 (d, $J$ = 2.5 Hz, 1H), 8.73 (d, $J$ = 2.5 Hz, 1H), 8.18 (s, 2H), 3.52 (d, $J$ = 9.8 Hz, 1H), 2.82 (s, 3H), 2.38 (s, 6H), 0.87-0.82(m, 2H), 0.63-0.57 (m, 1H), 0.24-0.12 (m, 2H); ESI-MS (m/z) 518.30 $(MH)^+$;

(±)-1-(3-Chloro-4-(2H-1,2,3-triazol-2-yl)phenyl)-3-(2-methyl-7-(1-(pyrrolidin-1-yl)ethyl)thiazolo[5,4-b]pyridin-6-yl)urea (Compound 77)

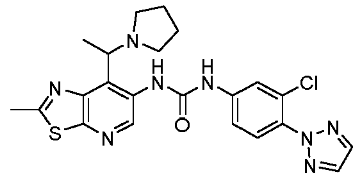

ESI-MS (m/z) 483.42 $(MH)^+$
Chiral separation of racemic compound 77 was carried out using chiral column and afforded the below isomers 77a and 77b:

1-(3-Chloro-4-(2H-1,2,3-triazol-2-yl)phenyl)-3-(2-methyl-7-(1-(pyrrolidin-1-yl)ethyl)thiazolo[5,4-b]pyridin-6-yl)urea (Compound 77a)
Chiral HPLC RT: 8.10 min
$^1$HNMR (400 MHz, DMSO-$d_6$) δ 10.58 (s, 1H), 9.94 (s, 1H), 9.21 (s, 1H), 8.15-8.10 (m, 2H), 7.98 (s, 1H), 7.62 (s, 2H), 4.57-4.51 (m, 1H), 2.83 (s, 3H), 2.72-2.61 (m, 2H), 2.48-3.37(m, 2H), 1.91-1.70 (m, 4H), 1.45 (d, *J* = 6.5 Hz, 3H); ESI-MS (m/z) 483.36 $(MH)^+$;

1-(3-Chloro-4-(2H-1,2,3-triazol-2-yl)phenyl)-3-(2-methyl-7-(1-(pyrrolidin-1-yl)ethyl)thiazolo[5,4-b]pyridin-6-yl)urea (Compound 77b)
Chiral HPLC RT: 9.97 min
$^1$HNMR (400 MHz, DMSO-$d_6$) δ 10.58 (s, 1H), 9.94 (s, 1H), 9.21 (s, 1H), 8.15-8.10 (m, 2H), 7.98 (s, 1H), 7.62 (s, 2H), 4.57-4.51 (m, 1H), 2.83 (s, 3H), 2.72-2.61 (m, 2H), 2.48-3.37(m, 2H), 1.91-1.70 (m, 4H), 1.45 (d, *J* = 6.5 Hz, 3H); ESI-MS (m/z) 483.36 $(MH)^+$;

(±)-1-(6-(2H-1,2,3-Triazol-2-yl)-5-(trifluoromethyl)pyridin-3-yl)-3-(7-(1-methoxy-2-methylpropyl)-2-methylthiazolo[5,4-b]pyridin-6-yl)urea (Compound 78)

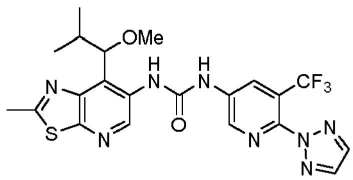

ESI-MS (m/z) 507.17 $(MH)^+$
Chiral separation of racemic compound 78 was carried out using chiral column and afforded the below isomers 78a and 78b:

1-(6-(2H-1,2,3-Triazol-2-yl)-5-(trifluoromethyl)pyridin-3-yl)-3-(7-(1-methoxy-2-methylpropyl)-2-methylthiazolo[5,4-b]pyridin-6-yl)urea (Compound 78a)
Chiral HPLC RT 6.01 min
$^1$HNMR (400 MHz, DMSO-$d_6$) δ 10.67 (s, 1H, $D_2O$ exchangeable), 9.10 (s, 1H), 8.86 (d, *J* = 2.5 *Hz,* 1H), 8.73 (d, *J* = 2.5 *Hz,* 1H), 8.69 (s, 1H, $D_2O$ exchangeable), 8.18 (s, 2H), 5.05 (d, *J* = 8.5 *Hz,* 1H), 3.31 (s, 3H), 2.85 (s, 3H), 2.31-2.22 (m, 1H), 1.13 (d, *J* = 6.5 *Hz,* 3H), 0.69 (d, *J* = 6.5 *Hz,* 3H); ESI-MS (m/z) 507.21 $(MH)^+$;

1-(6-(2H-1,2,3-Triazol-2-yl)-5-(trifluoromethyl)pyridin-3-yl)-3-(7-(1-methoxy-2-methylpropyl)-2-methylthiazolo[5,4-b]pyridin-6-yl)urea (Compound 78b)
Chiral HPLC RT 6.92 min
$^1$HNMR (400 MHz, DMSO-$d_6$) δ 10.68 (s, 1H, $D_2O$ exchangeable), 9.10 (s, 1H), 8.86 (d, *J* = 2.5 *Hz,* 1H), 8.73 (d, *J* = 2.5 *Hz,* 1H), 8.68 (s, 1H, $D_2O$ exchangeable), 8.18 (s, 2H), 5.05 (d, *J* = 8.5 *Hz,* 1H), 3.31 (s, 3H), 2.85 (s, 3H), 2.31-2.22 (m, 1H), 1.13 (d, *J* = 6.5 *Hz,* 3H), 0.69 (d, *J* = 6.5 *Hz,* 3H); ESI-MS (m/z) 507.21 $(MH)^+$;

(±)-1-(5-Chloro-6-(2H-1,2,3-triazol-2-yl)pyridin-3-yl)-3-(7-(1-methoxy-2-methylpropyl)-2-methylthiazolo[5,4-b]pyridin-6-yl)urea (Compound 79)

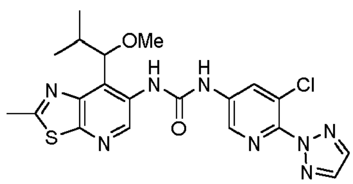

ESI-MS (m/z) 473.08 $(MH)^+$
Chiral separation of racemic compound 79 was carried out using chiral column and afforded the below isomers 79a and 79b:

1-(5-Chloro-6-(2H-1,2,3-triazol-2-yl)pyridin-3-yl)-3-(7-(1-methoxy-2-methylpropyl)-2-methylthiazolo[5,4-b]pyridin-6-yl)urea (Compound 79a).

Chiral HPLC RT 10.00 min
$^1$HNMR (400 MHz, DMSO-$d_6$) δ 10.53 (s, 1H, $D_2O$ exchangeable), 9.08 (s, 1H), 8.64 (s, 1H, $D_2O$ exchangeable), 8.55 (*d, J* = 2.5 *Hz,* 1H), 8.52 (d, *J* = 2.5 *Hz,* 1H), 8.17 (s, 2H), 5.04 (d, *J* = 8.5 *Hz,* 1H), 3.30 (s, 3H), 2.85 (s, 3H), 2.38-2.18 (m, 1H), 1.13 (d, *J* = 6.5 *Hz,* 3H), 0.68 (d, *J* = 6.5 *Hz,* 3H); ESI-MS (m/z) 473.21 $(MH)^+$;

1-(5-Chloro-6-(2H-1,2,3-triazol-2-yl)pyridin-3-yl)-3-(7-(1-methoxy-2-methylpropyl)-2-methylthiazolo[5,4-b]pyridin-6-yl)urea (Compound 79b)
Chiral HPLC RT 11.02 min
$^1$HNMR (400 MHz, DMSO-$d_6$) δ 10.54 (s, 1H, $D_2O$ exchangeable), 9.09 (s, 1H), 8.64 (s, 1H, $D_2O$ exchangeable), 8.55 (d, *J* = 2.5 *Hz,* 1H), 8.52 (d, *J* = 2.5 *Hz,* 1H), 8.17 (s, 2H), 5.04 (d, *J* = 8.5 *Hz,* 1H), 3.30 (s, 3H), 2.85 (s, 3H), 2.38-2.20 (m, 1H), 1.13 (d, *J* = 6.5 *Hz,* 3H), 0.68 (d, *J* = 6.5 *Hz,* 3H); ESI-MS (m/z) 473.19 $(MH)^+$;

(±)-1-(7-(1-Methoxy-2-methylpropyl)-2-methylthiazolo[5,4-b]pyridin-6-yl)-3-(2-methoxy-6-(2H-1,2,3-triazol-2-yl)-5-(trifluoromethyl)pyridin-3-yl)urea (Compound 80)

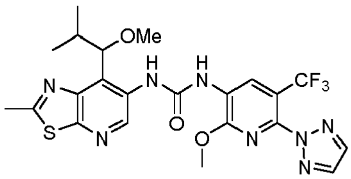

ESI-MS (m/z) 537.20 $(MH)^+$
Chiral separation of racemic compound 80 was carried out using chiral column and afforded the below isomers 80a and 80b:

1-(7-(1-Methoxy-2-methylpropyl)-2-methylthiazolo[5,4-b]pyridin-6-yl)-3-(2-methoxy-6-(2H-1,2,3-triazol-2-yl)-5-(trifluoromethyl)pyridin-3-yl)urea (Compound 80a)
Chiral HPLC RT 7.78 min
$^1$HNMR (400 MHz, DMSO-$d_6$) δ 9.90 (s, 1H, $D_2O$ exchangeable), 9.00 (s, 1H), 8.90 (s, 1H, $D_2O$ exchangeable), 8.79 (s, 1H), 8.16 (s, 2H), 4.93 (d, *J* = 9.0 *Hz,* 1H), 4.09 (s, 3H), 3.22 (s, 3H), 2.85 (s, 3H), 2.37-2.30 (m, 1H), 1.14 (d, *J* = 6.5 *Hz,* 3H), 0.62 (d, *J* = 6.5 *Hz,* 3H); ESI-MS (m/z) 537.03 $(MH)^+$;

1-(7-(1-Methoxy-2-methylpropyl)-2-methylthiazolo[5,4-b]pyridin-6-yl)-3-(2-methoxy-6-(2H-1,2,3-triazol-2-yl)-5-(trifluoromethyl)pyridin-3-yl)urea (Compound 80b)
Chiral HPLC RT 9.79 min
$^1$HNMR (400 M*Hz*, DMSO-$d_6$) δ 9.90 (s, 1H, $D_2O$ exchangeable), 9.00 (s, 1H), 8.90 (s, 1H, $D_2O$ exchangeable), 8.79 (s, 1H), 8.16 (s, 2H), 4.93 (d, *J* = 9.0 *Hz,* 1H), 4.09 (s, 3H), 3.22 (s, 3H), 2.85 (s, 3H), 2.36-3.30 (s, 1H), 1.14 (d, *J* = 6.5 *Hz,* 3H), 0.62 (d, *J* = 6.5 *Hz,* 3H); ESI-MS (m/z) 537.01 $(MH)^+$;

(±)-1-(5-Chloro-2-methoxy-6-(2H-1,2,3-triazol-2-yl)pyridin-3-yl)-3-(7-(1-methoxy-2-methylpropyl)-2-methylthiazolo[5,4-b]pyridin-6-yl)urea (Compound 81)

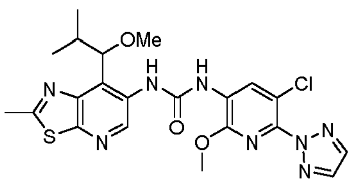

ESI-MS (m/z) 503.2 $(MH)^+$
Chiral separation of racemic compound 81 was carried out using chiral column and afforded the below isomers 81a and 81b:

1-(5-Chloro-2-methoxy-6-(2H-1,2,3-triazol-2-yl)pyridin-3-yl)-3-(7-(1-methoxy-2-methylpropyl)-2-methylthiazolo[5,4-b]pyridin-6-yl)urea (Compound 81a)
Chiral HPLC RT 4.94 min
$^1$HNMR (400 M*Hz*, DMSO-$d_6$) δ 9.78 (s, 1H, $D_2O$ exchangeable), 8.87 (s, 1H, $D_2O$ exchangeable), 8.79 (s,

1H), 8.74 (s, 1H), 8.15 (s, 2H), 4.93 (d, *J* = 9.0 *Hz,* 1H), 4.02 (s, 3H), 3.21 (s, 3H), 2.85 (s, 3H), 2.39-2.30 (m, 1H), 1.14 (d, *J* = 6.5 *Hz,* 3H), 0.62 (d, *J* = 6.5 *Hz,* 3H); ESI-MS (m/z) 503.04 $(MH)^+$;

1-(5-Chloro-2-methoxy-6-(2H-1,2,3-triazol-2-yl)pyridin-3-yl)-3-(7-(1-methoxy-2-methylpropyl)-2-methylthiazolo[5,4-b]pyridin-6-yl)urea (Compound 81b)
Chiral HPLC RT 5.78 min
$^1$HNMR (400 M*Hz*, DMSO-$d_6$) δ 9.78 (s, 1H, $D_2O$ exchangeable), 8.87 (s, 1H, $D_2O$ exchangeable), 8.79 (s, 1H), 8.74 (s, 1H), 8.15 (s, 2H), 4.93 (d, *J* = 9.0 *Hz,* 1H), 4.02 (s, 3H), 3.21 (s, 3H), 2.85 (s, 3H), 2.39-2.32 (m, 1H), 1.14 (d, *J* = 6.5 *Hz,* 3H), 0.62 (d, *J* = 6.5 *Hz,* 3H); ESI-MS (m/z) 503.04 $(MH)^+$;

(±)-1-(3-Chloro-4-(2H-1,2,3-triazol-2-yl)phenyl)-3-(7-(methoxy(phenyl)methyl)-2-methylthiazolo[5,4-b]pyridin-6-yl)urea (Compound 82)

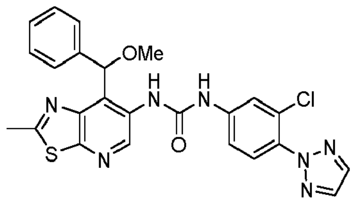

ESI-MS (m/z) 506.19 $(MH)^+$
Chiral separation of racemic compound 82 was carried out using chiral column and afforded the below isomers 82a and 82b:

1-(3-Chloro-4-(2H-1,2,3-triazol-2-yl)phenyl)-3-(7-(methoxy(phenyl)methyl)-2-methylthiazolo[5,4-b]pyridin-6-yl)urea (Compound 82a)
Chiral HPLC RT: 8.91 min
$^1$HNMR (400 MHz, DMSO-$d_6$) δ 10.20 (s, 1H, $D_2O$ exchangeable), 8.98 (s, 1H), 8.56 (s, 1H, $D_2O$ exchangeable), 8.12 (s, 2H), 7.89 (d, *J* = 2.5 *Hz,* 1H), 7.59 (d, *J* = 8.5 *Hz,* 1H), 7.49 (dd, *J* = 8.5, 2.5 *Hz,* 1H), 7.46-7.39 (m, 2H), 7.33-7.30 (m, 2H), 7.24-7.22 (m, 1H), 6.56 (s, 1H), 3.52 (s, 3H), 2.90 (s, 3H); ESI-MS (m/z) 506.5 $(MH)^+$;

1-(3-Chloro-4-(2H-1,2,3-triazol-2-yl)phenyl)-3-(7-(methoxy(phenyl)methyl)-2-methylthiazolo[5,4-b]pyridin-6-yl)urea (Compound 82b)
Chiral HPLC RT: 10.73 min
$^1$HNMR (400 MHz, DMSO-$d_6$) δ 10.20 (s, 1H, $D_2O$ exchangeable), 8.98 (s, 1H), 8.56 (s, 1H, $D_2O$ exchangeable), 8.12 (s, 2H), 7.89 (d, *J* = 2.5 *Hz,* 1H), 7.59 (d, *J* = 8.5 *Hz,* 1H), 7.49 (dd, *J* = 8.5, 2.5 *Hz,* 1H), 7.46-7.41 (m, 2H), 7.33-7.28 (m, 2H), 7.24-7.20 (m, 1H), 6.56 (s, 1H), 3.52 (s, 3H), 2.90 (s, 3H); ESI-MS (m/z) 506.0 $(MH)^+$;

(±)-1-(6-(2H-1,2,3-Triazol-2-yl)-5-(trifluoromethyl)pyridin-3-yl)-3-(7-(methoxy(phenyl)methyl)-2-methylthiazolo[5,4-b]pyridin-6-yl)urea (Compound 83)

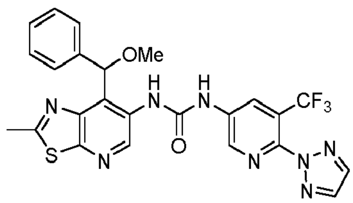

ESI-MS (m/z) 541.32 $(MH)^+$
Chiral separation of racemic compound 83 was carried out using chiral column and afforded the below isomers 83a and 83b:

1-(6-(2H-1,2,3-Triazol-2-yl)-5-(trifluoromethyl)pyridin-3-yl)-3-(7-(methoxy(phenyl)methyl)-2-methylthiazolo[5,4-b]pyridin-6-yl)urea (Compound 83a)
Chiral HPLC RT 5.69 min
$^1$HNMR (400 MHz, DMSO-$d_6$) δ 10.63 (s, 1H, $D_2O$ exchangeable), 9.02 (s, 1H), 8.81 (d, *J* = 2.5 *Hz,* 1H), 8.74

(s, 1H, $D_2O$ exchangeable), 8.62 (d, *J* = 2.5 *Hz,* 1H), 8.18 (s, 2H), 7.50-7.40 (m, 2H), 7.35-7.29 (m, 2H), 7.27-7.20 (m, 1H), 6.57 (s, 1H), 3.53 (s, 3H), 2.90 (s, 3H); ESI-MS (m/z) 541.45 $(MH)^+$;

1-(6-(2H-1,2,3-Triazol-2-yl)-5-(trifluoromethyl)pyridin-3-yl)-3-(7-(methoxy(phenyl)methyl)-2-methylthiazolo[5,4-b]pyridin-6-yl)urea (Compound 83b)
Chiral HPLC RT 6.39 min
$^1$HNMR (400 MHz, DMSO-$d_6$) δ 10.63 (s, 1H, $D_2O$ exchangeable), 9.02 (s, 1H), 8.81 (d, *J* = 2.5 *Hz,* 1H), 8.74 (s, 1H, $D_2O$ exchangeable), 8.62 (d, *J* = 2.5 *Hz,* 1H), 8.18 (s, 2H), 7.50-7.42 (m, 2H), 7.35-7.29 (m, 2H), 7.26-7.19 (m, 1H), 6.57 (s, 1H), 3.53 (s, 3H), 2.90 (s, 3H); ESI-MS (m/z) 541.45 $(MH)^+$;

(±)-1-(5-Chloro-6-(2H-1,2,3-triazol-2-yl)pyridin-3-yl)-3-(7-(methoxy(phenyl)methyl)-2-methylthiazolo[5,4-b]pyridin-6-yl)urea (Compound 84)

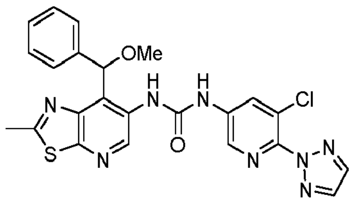

ESI-MS (m/z) 507.17 $(MH)^+$
Chiral separation of racemic compound 84 was carried out using chiral column and afforded the below isomers 84a and 84b:

1-(5-Chloro-6-(2H-1,2,3-triazol-2-yl)pyridin-3-yl)-3-(7-(methoxy(phenyl)methyl)-2-methylthiazolo[5,4-b]pyridin-6-yl)urea (Compound 84a)
Chiral HPLC RT 7.77 min
$^1$HNMR (400 MHz, DMSO-$d_6$) δ 10.48 (s, 1H, $D_2O$ exchangeable), 8.99 (s, 1H), 8.69 (s, 1H, $D_2O$ exchangeable), 8.51 (d, *J* = 2.5 *Hz,* 1H), 8.41 (d, *J* = 2.5 *Hz,* 1H), 8.16 (s, 2H), 7.45-7.43 (m, 2H), 7.33-7.29 (m, 2H), 7.26-7.19 (m, 1H), 6.56 (s, 1H), 3.52 (s, 3H), 2.90 (s, 3H); ESI-MS (m/z) 507.03 $(MH)^+$;

1-(5-Chloro-6-(2H-1,2,3-triazol-2-yl)pyridin-3-yl)-3-(7-(methoxy(phenyl)methyl)-2-methylthiazolo[5,4-b]pyridin-6-yl)urea (Compound 84b)
Chiral HPLC RT 9.16 min
$^1$HNMR (400 MHz, DMSO-$d_6$) δ 10.48 (s, 1H, $D_2O$ exchangeable), 8.99 (s, 1H), 8.69 (s, 1H, $D_2O$ exchangeable), 8.51 (d, *J* = 2.5 *Hz,* 1H), 8.41 (d, *J* = 2.5 *Hz,* 1H), 8.16 (s, 2H), 7.45-7.43 (m, 2H), 7.33-7.29 (m, 2H), 7.26-7.19 (m, 1H), 6.56 (s, 1H), 3.52 (s, 3H), 2.90 (s, 3H); ESI-MS (m/z) 507.04 $(MH)^+$;

(±)-1-(6-(2H-1,2,3-Triazol-2-yl)-5-(trifluoromethyl)pyridin-3-yl)-3-(7-((4-fluorophenyl)(methoxy)methyl)-2-methylthiazolo[5,4-b]pyridin-6-yl)urea (Compound 85)

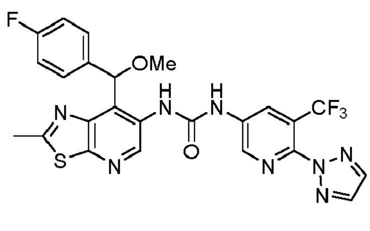

ESI-MS (m/z) 559.32 $(MH)^+$
Chiral separation of racemic compound 85 was carried out using chiral column and afforded the below isomers 85a and 85b:

1-(6-(2H-1,2,3-Triazol-2-yl)-5-(trifluoromethyl)pyridin-3-yl)-3-(7-((4-fluorophenyl)(methoxy)methyl)-2-methylthiazolo[5,4-b]pyridin-6-yl)urea (Compound 85a)
Chiral HPLC RT 5.79 min
$^1$HNMR (400 MHz, DMSO-d6) δ 10.60 (s, 1H, $D_2O$ exchangeable), 9.01 (s, 1H), 8.80 (d, *J* = 2.5 *Hz,* 1H), 8.70

(s, 1H, $D_2O$ exchangeable), 8.62 (d, *J* = 2.5 *Hz,* 1H), 8.18 (s, 2H), 7.58-7.36 (m, 2H), 7.21-7.09 (m, 2H), 6.55 (s, 1H), 3.52 (s, 3H), 2.90 (s, 3H); ESI-MS (m/z) 559.18 $(MH)^+$;

1-(6-(2H-1,2,3-Triazol-2-yl)-5-(trifluoromethyl)pyridin-3-yl)-3-(7-((4-fluorophenyl)(methoxy)methyl)-2-methylthiazolo[5,4-b]pyridin-6-yl)urea (Compound 85b)
Chiral HPLC RT 6.86 min
$^1$HNMR (400 MHz, DMSO-$d_6$) δ 10.60 (s, 1H, $D_2O$ exchangeable), 9.01 (s, 1H), 8.81 (d, *J* = 2.5 *Hz,* 1H), 8.70 (s, 1H, $D_2O$ exchangeable), 8.62 (d, *J* = 2.5 *Hz,* 1H), 8.18 (s, 2H), 7.57-7.36 (m, 2H), 7.21-7.08 (m, 2H), 6.55 (s, 1H), 3.52 (s, 3H), 2.90 (s, 3H); ESI-MS (m/z) 559.2 $(MH)^+$;

(±)-1-(4-(2H-1,2,3-Triazol-2-yl)-3-(trifluoromethyl)phenyl)-3-(7-((4-fluorophenyl)(methoxy)methyl)-2-methylthiazolo[5,4-b]pyridin-6-yl)urea (Compound 86)

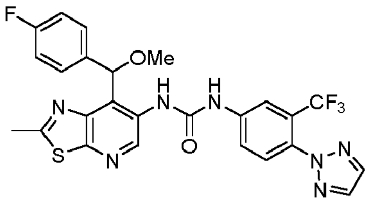

ESI-MS (m/z) 558.3 $(MH)^+$
Chiral separation of racemic compound 86 was carried out using chiral column and afforded the below isomers 86a and 86b:

1-(4-(2H-1,2,3-Triazol-2-yl)-3-(trifluoromethyl)phenyl)-3-(7-((4-fluorophenyl)(methoxy)methyl)-2-methylthiazolo[5,4-b]pyridin-6-yl)urea (Compound 86a)
Chiral HPLC RT 5.26 min
$^1$HNMR (400 MHz, DMSO-$d_6$) δ 10.31 (s, 1H, $D_2O$ exchangeable), 9.01 (s, 1H), 8.56 (s, 1H, $D_2O$ exchangeable), 8.13 (s, 2H), 8.10 (d, *J* = 2.5 *Hz,* 1H), 7.85 (dd, *J* = 8.5, *2.5 Hz,* 1H), 7.68 (d, *J* = 8.5 *Hz,* 1H), 7.51-7.35 (m, 2H), 7.23-7.04 (m, 2H), 6.55 (s, 1H), 3.51 (s, 3H), 2.89 (s, 3H); ESI-MS (m/z) 558.3 $(MH)^+$;
1-(4-(2H-1,2,3-Triazol-2-yl)-3-(trifluoromethyl)phenyl)-3-(7-((4-fluorophenyl)(methoxy)methyl)-2-methylthiazolo[5,4-b]pyridin-6-yl)urea (Compound 86b)
Chiral HPLC RT 6.67 min
$^1$HNMR (400 MHz, DMSO-$d_6$) δ 10.31 (s, 1H, $D_2O$ exchangeable), 9.01 (s, 1H), 8.56 (s, 1H, $D_2O$ exchangeable), 8.13 (s, 2H), 8.10 (d, *J* = 2.5 *Hz,* 1H), 7.85 (dd, *J* = 8.5, *2.5 Hz,* 1H), 7.68 (d, *J* = 8.5 *Hz,* 1H), 7.52-7.35 (m, 2H), 7.23-7.04 (m, 2H), 6.55 (s, 1H), 3.51 (s, 3H), 2.89 (s, 3H); ESI-MS (m/z) 558.2 $(MH)^+$;

(±)-1-(3-Chloro-4-(2H-1,2,3-triazol-2-yl)phenyl)-3-(7-((4-fluorophenyl)(methoxy)methyl)-2-methylthiazolo[5,4-b]pyridin-6-yl)urea (Compound 87)

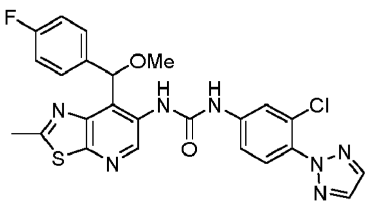

ESI-MS (m/z) 524.30 $(MH)^+$
Chiral separation of racemic compound 87 was carried out using chiral column and afforded the below isomers 87a and 87b:

1-(3-Chloro-4-(2H-1,2,3-triazol-2-yl)phenyl)-3-(7-((4-fluorophenyl)(methoxy)methyl)-2-methylthiazolo[5,4-b]pyridin-6-yl)urea (Compound 87a)
Chiral HPLC RT: 4.97 min
$^1$HNMR (400 MHz, DMSO-$d_6$) δ 10.15 (s, 1H, $D_2O$ exchangeable), 8.99 (s, 1H), 8.52 (s, 1H, $D_2O$ exchangeable), 8.12 (s, 2H), 7.89 (d, *J* = 2.5 *Hz,* 1H), 7.60 (d, *J* = 8.5 *Hz,* 1H), 7.53-7.32 (m, 3H), 7.20-7.04 (m, 2H), 6.54 (s,

1H), 3.50 (s, 3H), 2.89 (s, 3H); ESI-MS (m/z) 524.24 $(MH)^+$;

1-(3-Chloro-4-(2H-1,2,3-triazol-2-yl)phenyl)-3-(7-((4-fluorophenyl)(methoxy)methyl)-2-methylthiazolo[5,4-b]pyridin-6-yl)urea (Compound 87b)
Chiral HPLC RT: 6.40 min
$^1$HNMR (400 MHz, DMSO-d6) δ 10.21 (s, 1H, $D_2O$ exchangeable), 8.99 (s, 1H), 8.52 (s, 1H, $D_2O$ exchangeable), 8.12 (s, 2H), 7.89 (d, *J* = 2.5 *Hz,* 1H), 7.59 (d, *J* = 8.5 *Hz,* 1H), 7.55-7.34 (m, 3H), 7.21-7.07 (m, 2H), 6.54 (s, 1H), 3.50 (s, 3H), 2.89 (s, 3H); ESI-MS (m/z) 524.24 $(MH)^+$;

(±)-1-(2-Methoxy-6-(2H-1,2,3-triazol-2-yl)-5-(trifluoromethyl)pyridin-3-yl)-3-(7-(1-methoxyethyl)-2-methylthiazolo[5,4-b]pyridin-6-yl)urea (Compound 88)

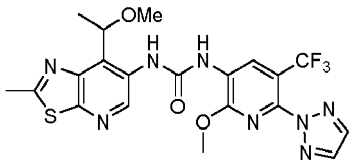

ESI-MS (m/z) 509.14 $(MH)^+$
Chiral separation of racemic compound 88 was carried out using chiral column and afforded the below isomers 88a and 88b:

1-(2-Methoxy-6-(2H-1,2,3-triazol-2-yl)-5-(trifluoromethyl)pyridin-3-yl)-3-(7-(1-methoxyethyl)-2-methylthiazolo[5,4-b]pyridin-6-yl)urea (Compound 88a)
Chiral HPLC RT 6.47 min
$^1$HNMR (400 MHz, DMSO-$d_6$) δ 9.90 (s, 1H), 9.01 (s, 2H), 8.85 (s, 1H), 8.17 (s, 2H), 5.45 (q, *J* = 7.0 Hz, 1H), 4.09 (s, 3H), 3.24 (s, 3H), 2.86 (s, 3H), 1.56 (d, *J* = 7.0 Hz, 3H); ESI-MS (m/z) 509.31 $(MH)^+$;

1-(2-Methoxy-6-(2H-1,2,3-triazol-2-yl)-5-(trifluoromethyl)pyridin-3-yl)-3-(7-(1-methoxyethyl)-2-methylthiazolo[5,4-b]pyridin-6-yl)urea (Compound 88b)
Chiral HPLC RT 7.71 min
$^1$HNMR (400 MHz, DMSO-$d_6$) δ 9.90 (s, 1H), 9.01 (s, 2H), 8.86 (s, 1H), 8.17 (s, 2H), 5.45 (q, *J* = 7.0 Hz, 1H), 4.10 (s, 3H), 3.24 (s, 3H), 2.86 (s, 3H), 1.57 (d, *J* = 7.0 Hz, 3H); ESI-MS (m/z) 509.31 $(MH)^+$;

1-(5-Chloro-2-(2H-1,2,3-triazol-2-yl)phenyl)-3-(7-(1-methoxyethyl)-2-methylthiazolo[5,4-b]pyridin-6-yl)urea (Compound 89)

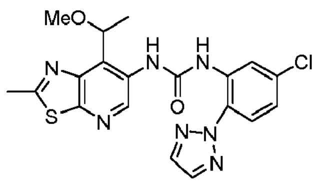

ESI-MS (m/z) 444.04 $(MH)^+$
Chiral separation of racemic compound 89 was carried out using chiral column and afforded the below isomers 89a and 89b:

1-(5-chloro-2-(2H-1,2,3-triazol-2-yl)phenyl)-3-(7-(1-methoxyethyl)-2-methylthiazolo[5,4-b]pyridin-6-yl)urea (Compound 89a)
Chiral HPLC RT: 5.13 min
$^1$HNMR (400 MHz, DMSO-$d_6$) δ 9.47 (s, 1H), 8.85 (s, 1H), 8.79 (s, 1H), 8.17 (s, 2H), 8.10 (s, 1H), 7.72 (d, *J* = 8.5 *Hz,* 1H), 7.34 (d, *J* = 8.5 *Hz,* 1H), 5.36 (q, *J* = 6.5 *Hz,* 1H), 3.17 (s, 3H), 2.83 (s, 3H), 1.54 (d, *J* = 6.5 *Hz,* 3H); ESI-MS (m/z) 444.11 $(MH)^+$;

1-(5-chloro-2-(2H-1,2,3-triazol-2-yl)phenyl)-3-(7-(1-methoxyethyl)-2-methylthiazolo[5,4-b]pyridin-6-yl)urea (Compound 89b)

Chiral HPLC RT: 5.55 min
$^1$HNMR (400 MHz, DMSO-$d_6$) δ 9.47 (s, 1H), 8.85 (s, 1H), 8.79 (s, 1H), 8.17 (s, 2H), 8.10 (s, 1H), 7.72 (d, *J* = 8.5 *Hz,* 1H), 7.34 (d, *J* = 8.5 *Hz,* 1H), 5.36 (q, *J* = 6.5 *Hz,* 1H), 3.17 (s, 3H), 2.83 (s, 3H), 1.54 (d, *J* = 6.5 *Hz,* 3H); ESI-MS (m/z) 443.99 (MH)$^+$;

(±)-1-(5-Chloro-2-methoxy-6-(2H-1,2,3-triazol-2-yl)pyridin-3-yl)-3-(7-(1-methoxyethyl)-2-methylthiazolo[5,4-b]pyridin-6-yl)urea (Compound 90)

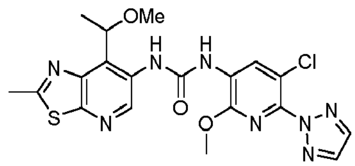

ESI-MS (*m*/*z*) 474.93 (MH)$^+$
Chiral separation of racemic compound 90 was carried out using chiral column and afforded the below isomers 90a and 90b:

1-(5-Chloro-2-methoxy-6-(2H-1,2,3-triazol-2-yl)pyridin-3-yl)-3-(7-(1-methoxyethyl)-2-methylthiazolo[5,4-b]pyridin-6-yl)urea (Compound 90a)
Chiral HPLC RT 6.21 min
$^1$HNMR (400 MHz, DMSO-$d_6$) δ 9.78 (s, 1H), 8.97 (s, 1H), 8.85 (s, 1H), 8.75 (s, 1H), 8.15 (s, 2H), 5.44 (q, *J* = 6.5 Hz, 1H), 4.02 (s, 3H), 3.23 (s, 3H), 2.86 (s, 3H), 1.56 (d, *J* = 6.5 Hz, 3H); ESI-MS (*m*/*z*) 475.01 (MH)$^+$;

1-(5-Chloro-2-methoxy-6-(2H-1,2,3-triazol-2-yl)pyridin-3-yl)-3-(7-(1-methoxyethyl)-2-methylthiazolo[5,4-b]pyridin-6-yl)urea (Compound 90b)
Chiral HPLC RT 6.86 min
$^1$HNMR (400 MHz, DMSO-$d_6$) δ 9.78 (s, 1H), 8.97 (s, 1H), 8.85 (s, 1H), 8.75 (s, 1H), 8.15 (s, 2H), 5.44 (q, *J* = 6.5 Hz, 1H), 4.02 (s, 3H), 3.23 (s, 3H), 2.86 (s, 3H), 1.56 (d, *J* = 6.5 Hz, 3H); ESI-MS (*m*/*z*) 475.00 (MH)$^+$;

(±)-1-(5-Chloro-2-methoxy-6-(1H-1,2,3-triazol-1-yl)pyridin-3-yl)-3-(7-(1-methoxyethyl)-2-methylthiazolo[5,4-b]pyridin-6-yl)urea (Compound 91)

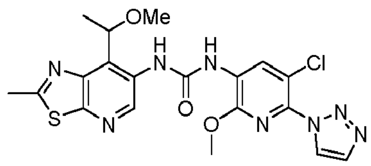

ESI-MS (*m*/*z*) 475.12 (MH)$^+$
Chiral separation of racemic compound 91 was carried out using chiral column and afforded the below isomers 91a and 91b:

1-(5-Chloro-2-methoxy-6-(1H-1,2,3-triazol-1-yl)pyridin-3-yl)-3-(7-(1-methoxyethyl)-2-methylthiazolo[5,4-b]pyridin-6-yl)urea (Compound 91a)
Chiral HPLC RT: 5.01 min
$^1$HNMR (400 MHz, DMSO-$d_6$) δ 9.79 (s, 1H), 8.97 (s, 1H), 8.85 (s, 1H), 8.77 (s, 1H), 8.62 (s, 1H), 7.99 (s, 1H), 5.44 (q, *J* = 6.5 Hz, 1H), 4.04 (s, 3H), 3.23 (s, 3H), 2.86 (s, 3H), 1.56 (d, *J* = 6.5 Hz, 3H); ESI-MS (*m*/*z*) 475.01 (MH)$^+$;

1-(5-Chloro-2-methoxy-6-(1H-1,2,3-triazol-1-yl)pyridin-3-yl)-3-(7-(1-methoxyethyl)-2-methylthiazolo[5,4-b]pyridin-6-yl)urea (Compound 91b)
Chiral HPLC RT: 5.51 min
$^1$HNMR (400 MHz, DMSO-$d_6$) δ 9.79 (s, 1H), 8.97 (s, 1H), 8.85 (s, 1H), 8.77 (s, 1H), 8.62 (s, 1H), 7.99 (s, 1H), 5.44 (q, *J* = 6.5 Hz, 1H), 4.04 (s, 3H), 3.23 (s, 3H), 2.86 (s, 3H), 1.56 (d, *J* = 6.5 Hz, 3H); ESI-MS (*m*/*z*) 475.00 (MH)$^+$;

(±)-1-(5-Chloro-6-methoxy-2-(2H-1,2,3-triazol-2-yl)pyridin-3-yl)-3-(7-(1-methoxyethyl)-2-methylthiazolo[5,4-b]pyridin-6-yl)urea (Compound 92)

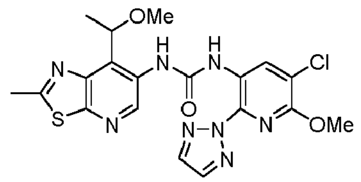

ESI-MS (*m/z*) 474.98 $(MH)^+$
Chiral separation of racemic compound 92 was carried out using chiral column and afforded the below isomers 92a and 92b:

1-(5-Chloro-6-methoxy-2-(2H-1,2,3-triazol-2-yl)pyridin-3-yl)-3-(7-(1-methoxyethyl)-2-methylthiazolo[5,4-b]pyridin-6-yl)urea (Compound 92a)
Chiral HPLC RT 5.45 min
$^1$HNMR (400 MHz, DMSO-$d_6$) δ 9.30 (s, 1H), 8.85 (s, 1H), 8.68 (s, 1H), 8.44 (s, 1H), 8.20 (s, 2H), 5.38 (q, *J* = 7.0 Hz, 1H), 3.34 (s, 3H), 3.18 (s, 3H), 2.84 (s, 3H), 1.52 (d, *J* = 7.0 Hz, 3H); ESI-MS (*m/z*) 474.81 $(MH)^+$;

1-(5-Chloro-6-methoxy-2-(2H-1,2,3-triazol-2-yl)pyridin-3-yl)-3-(7-(1-methoxyethyl)-2-methylthiazolo[5,4-b]pyridin-6-yl)urea (Compound 92b)
Chiral HPLC RT 6.22 min
$^1$HNMR (400 MHz, DMSO-$d_6$) δ 9.30 (s, 1H), 8.85 (s, 1H), 8.68 (s, 1H), 8.44 (s, 1H), 8.20 (s, 2H), 5.38 (q, *J* = 7.0 Hz, 1H), 3.34 (s, 3H), 3.18 (s, 3H), 2.84 (s, 3H), 1.52 (d, *J* = 7.0 Hz, 3H); ESI-MS (*m/z*) 474.81 $(MH)^+$;

(±)-1-(6-(2H-1,2,3-Triazol-2-yl)-5-(trifluoromethyl)pyridin-3-yl)-3-(7-(1-cyclopropyl-1-methoxyethyl)-2-methylthiazolo[5,4-b]pyridin-6-yl)urea (Compound 93)

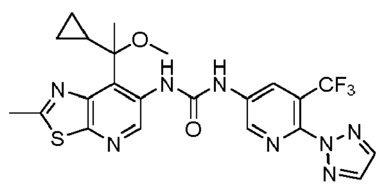

ESI-MS (m/z) 519.44 $(MH)^+$
Chiral separation of racemic compound 93 was carried out using chiral column and afforded the below isomers 93a and 93b:

1-(6-(2H-1,2,3-triazol-2-yl)-5-(trifluoromethyl)pyridin-3-yl)-3-(7-(1-cyclopropyl-1-methoxyethyl)-2-methylthiazolo[5,4-b]pyridin-6-yl)urea (Compound 93a)
Chiral HPLC RT 7.10 min
$^1$HNMR (400 MHz, DMSO-$d_6$) δ 10.71 (s, 1H), 9.44 (s, 1H), 9.07 (s, 1H), 8.89 (d, *J* = 2.4 Hz, 1H), 8.73 (d, *J* = 2.4 Hz, 1H), 8.18 (s, 2H), 3.26 (s, 3H), 2.83 (s, 3H), 1.97 (s, 3H), 1.57-1.48 (m, 1H), 0.64-0.55 (m, 1H), 0.52-0.36 (m, 3H); ESI-MS (m/z) 519.44 $(MH)^+$;

1-(6-(2H-1,2,3-triazol-2-yl)-5-(trifluoromethyl)pyridin-3-yl)-3-(7-(1-cyclopropyl-1-methoxyethyl)-2-methylthiazolo[5,4-b]pyridin-6-yl)urea (Compound 93b)
Chiral HPLC RT 8.51 min
$^1$HNMR (400 MHz, DMSO-$d_6$) δ 10.71 (s, 1H), 9.44 (s, 1H), 9.07 (s, 1H), 8.88 (d, *J* = 2.4 Hz, 1H), 8.73 (d, *J* = 2.4 Hz, 1H), 8.18 (s, 2H), 3.26 (s, 3H), 2.83 (s, 3H), 1.97 (s, 3H), 1.57-1.47 (m, 1H), 0.65-0.54 (m, 1H), 0.51-0.37 (m, 3H); ESI-MS (m/z) 519.44 $(MH)^+$;

(±)-1-(4-(2H-1,2,3-triazol-2-yl)-3-(trifluoromethyl)phenyl)-3-(7-(1-cyclopropyl-1-methoxyethyl)-2-methylthiazolo[5,4-b]pyridin-6-yl)urea (Compound 94)

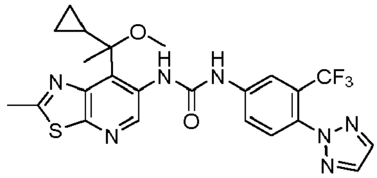

ESI-MS (m/z) 518.32 $(MH)^+$

Chiral separation of racemic compound 94 was carried out using chiral column and afforded the below isomers 94a and 94b:

1-(4-(2H-1,2,3-triazol-2-yl)-3-(trifluoromethyl)phenyl)-3-(7-(1-cyclopropyl-1-methoxyethyl)-2-methylthiazolo[5,4-b]pyridin-6-yl)urea (Compound 94a)

Chiral HPLC RT 5.93 min

$^1$HNMR (400 MHz, DMSO-$d_6$) δ 10.41 (s, 1H), 9.30 (s, 1H), 9.04 (s, 1H), 8.19 (d, *J* = 2.3 Hz, 1H), 8.13 (s, 2H), 7.95 (dd, *J* = 8.8, 2.3 Hz, 1H), 7.70 (d, *J* = 8.8 Hz, 1H), 3.25 (s, 3H), 2.82 (s, 3H), 1.97 (s, 3H), 1.57-1.44 (m, 1H), 0.62-0.53 (m, 1H), 0.49-0.36 (m, 3H); ESI-MS (m/z) 518.32 $(MH)^+$;

1-(4-(2H-1,2,3-triazol-2-yl)-3-(trifluoromethyl)phenyl)-3-(7-(1-cyclopropyl-1-methoxyethyl)-2-methylthiazolo[5,4-b]pyridin-6-yl)urea (Compound 94b)

Chiral HPLC RT 5.09 min

$^1$HNMR (400 MHz, DMSO-$d_6$) δ 10.41 (s, 1H), 9.30 (s, 1H), 9.04 (s, 1H), 8.19 (d, *J* = 2.1 Hz, 1H), 8.13 (s, 2H), 7.95 (dd, *J* = 8.7, 2.1 Hz, 1H), 7.70 (d, *J* = 8.7 Hz, 1H), 3.25 (s, 3H), 2.82 (s, 3H), 1.97 (s, 3H), 1.57-1.44 (m, 1H), 0.62-0.53 (m, 1H), 0.49-0.36 (m, 3H); ESI-MS (m/z) 518 $(MH)^+$;

(±)-1-(6-(2H-1,2,3-triazol-2-yl)-5-(trifluoromethyl)pyridin-3-yl)-3-(2-methyl-7-(2,2,2-trifluoro-1-methoxyethyl)thiazolo[5,4-b]pyridin-6-yl)urea (Compound 95)

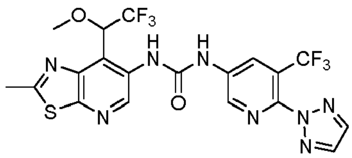

ESI-MS (m/z) 533.32 $(MH)^+$

Chiral separation of racemic compound 95 was carried out using chiral column and afforded the below isomers 95a and 95b:

1-(6-(2H-1,2,3-triazol-2-yl)-5-(trifluoromethyl)pyridin-3-yl)-3-(2-methyl-7-(2,2,2-trifluoro-1-methoxyethyl)thiazolo[5,4-b]pyridin-6-yl)urea (Compound 95a)

Chiral HPLC RT 5.96 min

$^1$HNMR (400 MHz, DMSO-$d_6$) δ 10.74 (s, 1H), 9.16 (s, 1H), 8.86 (d, *J* = 2.5 Hz, 1H), 8.73 (d, *J* = 2.5 Hz, 1H), 8.60 (s, 1H), 8.18 (s, 2H), 6.14-6.07 (m, 1H), 3.61 (s, 3H), 2.90 (s, 3H); ESI-MS (m/z) 533.2 $(MH)^+$;

1-(6-(2H-1,2,3-triazol-2-yl)-5-(trifluoromethyl)pyridin-3-yl)-3-(2-methyl-7-(2,2,2-trifluoro-1-methoxyethyl)thiazolo[5,4-b]pyridin-6-yl)urea (Compound 95b)

Chiral HPLC RT 7.26 min

$^1$HNMR (400 MHz, DMSO-$d_6$) δ 10.74 (s, 1H), 9.16 (s, 1H), 8.86 (d, *J* = 2.5 Hz, 1H), 8.73 (d, *J* = 2.5 Hz, 1H), 8.60 (s, 1H), 8.18 (s, 2H), 6.12 - 6.07 (m, 1H), 3.61 (s, 3H), 2.90 (s, 3H); ESI-MS (m/z) 533.2 $(MH)^+$;

1-(6-(2H-1,2,3-Triazol-2-yl)-5-(trifluoromethyl)pyridin-3-yl)-3-(7-(2-methoxypropan-2-yl)-2-methylthiazolo[5,4-b]pyridin-6-yl)urea (Compound 96)

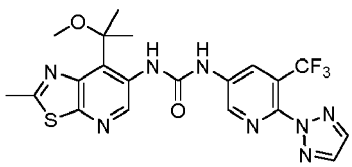

$^1$HNMR (400 MHz, DMSO-$d_6$) δ 10.70 (s, 1H), 9.43 (s, 1H), 9.08 (s, 1H), 8.88 (d, $J$ = 1.9 Hz, 1H), 8.73 (d, $J$ = 1.9 Hz, 1H), 8.19 (s, 2H), 3.24 (s, 3H), 2.84 (s, 3H), 1.89 (s, 6H); ESI-MS (m/z) 493.2 $(MH)^+$;

1-(4-(2H-1,2,3-Triazol-2-yl)-3-(trifluoromethyl)phenyl)-3-(7-(2-methoxypropan-2-yl)-2-methylthiazolo[5,4-b]pyridin-6-yl)urea (Compound 97)

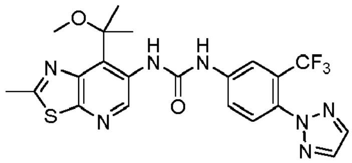

$^1$HNMR (400 MHz, DMSO-$d_6$) δ 10.41 (s, 1H), 9.27 (s, 1H), 9.05 (s, 1H), 8.20 (s, 1H), 8.13 (s, 2H), 7.93 (d, $J$ = 8.7 Hz, 1H), 7.70 (d, $J$ = 8.7 Hz, 1H), 3.22 (s, 3H), 2.83 (s, 3H), 1.88 (s, 6H); ESI-MS (m/z) 492.3 $(MH)^+$;

1-(3-Chloro-4-(2H-1,2,3-triazol-2-yl)phenyl)-3-(7-(2-methoxypropan-2-yl)-2-methylthiazolo[5,4-b]pyridin-6-yl)urea (Compound 98)

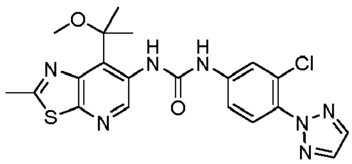

$^1$HNMR (400 MHz, DMSO-$d_6$) δ 10.24 (s, 1H), 9.21 (s, 1H), 9.02 (s, 1H), 8.12 (s, 2H), 7.99 (d, $J$ = 2.1 Hz, 1H), 7.61 (d, $J$ = 8.7 Hz, 1H), 7.56 (d, $J$ = 8.6 Hz, 1H), 3.21 (s, 3H), 2.83 (s, 3H), 1.87 (s, 6H); ESI-MS (m/z) 458.3 $(MH)^+$;

1-(4-(2H-1,2,3-triazol-2-yl)-3-(trifluoromethyl)phenyl)-3-(7-(cyclopropyl(methyl)amino)-2-methylthiazolo[5,4-b]pyridin-6-yl)urea (Compound 99)

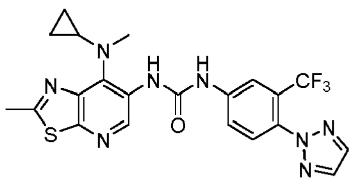

$^1$HNMR (400 MHz, DMSO-$d_6$) δ 9.96 (s, 1H), 8.97 (s, 1H), 8.31 (s, 1H), 8.18 (d, $J$ = 2.3 Hz, 1H), 8.13 (s, 2H), 7.88 (dd, $J$ = 8.8, 2.3 Hz, 1H), 7.68 (d, $J$ = 8.8 Hz, 1H), 3.32-3.26 (m, 1H), 3.04 (s, 3H), 2.82 (s, 3H), 0.61-0.54 (m, 2H), 0.50-0.43 (m, 2H); ESI-MS (m/z) 489.36 $(MH)^+$;

1-(6-(2H-1,2,3-Triazol-2-yl)-5-(trifluoromethyl)pyridin-3-yl)-3-(7-(cyclopropyl(methyl)amino)-2-methylthiazolo[5,4-b]pyridin-6-yl)urea (Compound 100)

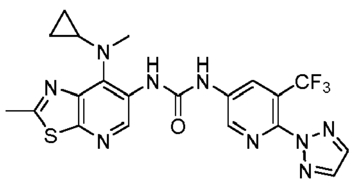

$^1$HNMR (400 MHz, DMSO-$d_6$) δ 10.17 (s, 1H), 8.92 (s, 1H), 8.83 (d, $J$ = 2.2 Hz, 1H), 8.73 (d, $J$ = 2.2 Hz, 1H), 8.49 (s, 1H), 8.18 (s, 2H), 3.32-3.26 (m, 1H), 3.06 (s, 3H), 2.82 (s, 3H), 0.64-0.53 (m, 2H), 0.51-0.41 (m, 2H); ESI-MS (m/z) 490.24 $(MH)^+$;

1-(5-Chloro-6-(2H-1,2,3-triazol-2-yl)pyridin-3-yl)-3-(7-(1-(methoxymethyl)cyclopropyl)-2-methylthiazolo[5,4-b]pyridin-6-yl)urea (Compound 101)

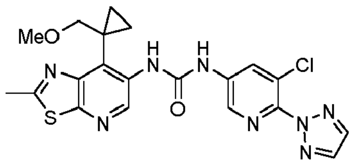

$^1$HNMR (400 MHz, DMSO-$d_6$) δ 10.48 (s, 1H), 8.87 (s, 1H), 8.58 (d, *J* = 2.5 *Hz,* 1H), 8.53 (d, *J* = 2.5 Hz, 1H), 8.27 (s, 1H), 8.17 (s, 2H), 3.56 (s, 2H), 3.24 (s, 3H), 2.86 (s, 3H), 1.21-1.17 (m, 2H), 0.94-0.90 (m, 2H); ESI-MS (m/z) 471.30 (MH)$^+$;

1-(6-(2H-1,2,3-Triazol-2-yl)-5-(trifluoromethyl)pyridin-3-yl)-3-(7-(1-(methoxymethyl)cyclopropyl)-2-methylthiazolo[5,4-b]pyridin-6-yl)urea (Compound 102)

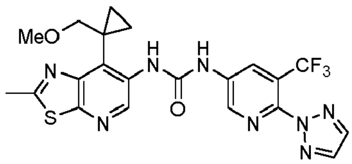

$^1$HNMR (400 MHz, DMSO-$d_6$) δ 10.61 (s, 1H), 8.89 (d, *J* = 2.5 Hz, 1H), 8.88 (s, 1H), 8.74 (d, *J* = 2.5 Hz, 1H), 8.31 (s, 1H), 8.18 (s, 2H), 3.57 (s, 2H), 3.25 (s, 3H), 2.86 (s, 3H), 1.19 (t, *J* = 6.0 Hz, 2H), 0.93 (t, *J* = 6.0 Hz, 3H); ESI-MS (m/z) 505.20 (MH)$^+$;

1-(5-Chloro-6-methoxypyridin-3-yl)-3-(7-cyclopropyl-2-(trifluoromethyl)thiazolo[5,4-b]pyridin-6-yl)urea (Compound 103)

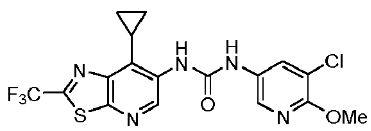

$^1$HNMR (400 MHz, DMSO-$d_6$) δ 9.30 (s, 1H), 8.97 (s, 1H), 8.76 (s, 1H), 8.16 (d, *J = 2.0 Hz,* 2H), 3.92 (s, 3H), 2.30-2.28 (m, 1H), 1.48-1.46 (m, 2H), 1.29-1.23 (m, 2H); ESI-MS (m/z) 444.1 (MH)$^+$;

1-(5-Chloro-6-(2H-1,2,3-triazol-2-yl)pyridin-3-yl)-3-(7-cyclopropyl-2-(trifluoromethyl)thiazolo[5,4-b]pyridin-6-yl)urea (Compound 104)

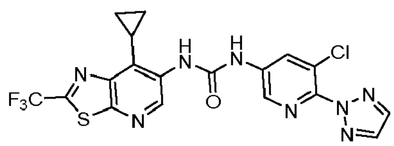

$^1$HNMR (400 MHz, DMSO-$d_6$) δ 9.95 (s, 1H), 9.05 (s, 1H), 8.95 (s, 1H), 8.59 (s, 1H), 8.50 (s,1H), 8.17 (s, 2H), 2.36-2.34 (m,1H), 1.54-1.52 (m, 2H), 1.26-1.24 (m, 2H); ESI-MS (m/z) 480.89 (MH)$^+$.

**Example-73:** 1-(5-Chloro-6-(2H-1,2,3-triazol-2-yl)pyridin-3-yl)-3-(7-(1-(hydroxymethyl)cyclopropyl)-2-methylthiazolo[5,4-b]pyridin-6-yl)urea (Compound 105)

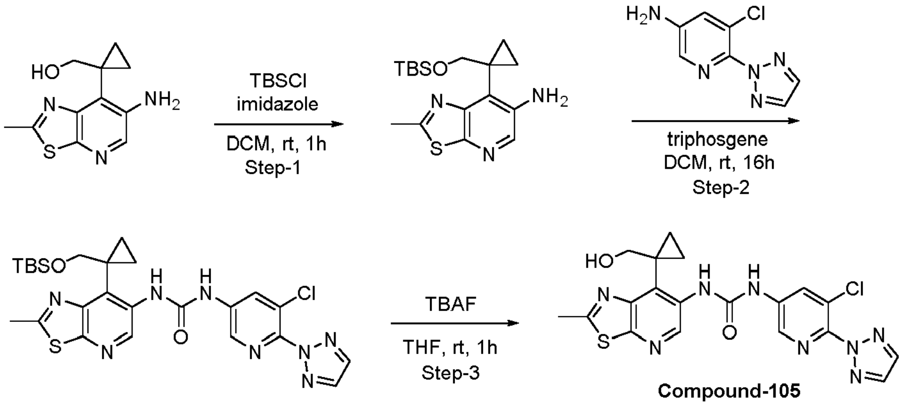

Compound-105

Step-1: 7-(1-(((tert-Butyldimethylsilyl)oxy)methyl)cyclopropyl)-2-methylthiazolo[5,4-b]pyridin-6-amine: To a stirred solution of (1-(6-amino-2-methylthiazolo[5,4-b]pyridin-7-yl)cyclopropyl)methanol (0.600 g, 2.55 mmol) and imidazole (0.521 g, 7.65 mmol) in DCM (25 mL) was added tert-butylchlorodimethylsilane (0.461 g, 3.06 mmol) and the resulting mixture was stirred at RT for 1h. The reaction mixture was diluted with DCM (20 mL) followed by water (20 mL). The layers were seperated and the aqueous layer was extracted with DCM (2×20 mL). The combined organic layers were washed with brine (50mL), dried ($Na_2SO_4$) and concentrated in vacuo. The crude product was purified by flash column chromatography (silica gel, 2-3% MeOH in DCM as eluent) to afford 0.820 g (92%) of the titled product.ESI-MS (m/z) 350.47 (MH)+.

Step-2: 1-(7-(1-(((tert-butyldimethylsilyl)oxy)methyl)cyclopropyl)-2-methylthiazolo[5,4-b]pyridin-6-yl)-3-(5-chloro-6-(2H-1,2,3-triazol-2-yl)pyridin-3-yl)urea: To a 0°C cooled and stirred solution of triphosgene (0.020 g, 0.069 mmol) in DCM (2 mL) was added a solution of 5-chloro-6-(2H-1,2,3-triazol-2-yl)pyridin-3-amine (0.038 g, 0.192 mmol) in DCM (1 mL) dropwise. The resulting mixture was then stirred for 30min at 0°C. A solution of step-1 intermediate (0.056 g, 0.160 mmol) in DCM (2 mL) was then added to the above mixture at the same temperature. The Reaction was then warmed to RT and stirred for 15 h. Reaction mixtrure was diluted with DCM (30mL) and water (20mL). The layers were separated and the aqueous layer was extracted with DCM (2×10 mL). The combined oranic layers were washed with brine (20 mL), dried ($Na_2SO_4$) and concentrated under vacuuo. The crude product was purified by flash column chromatography (silica gel, 15-20% acetonitrile in DCM as eluent) to afford 0.070 g (77%) of the titled compound. ESI-MS (m/z) 571.20 $(MH)^+$.

Step-3: To a stirred solution of step-2 intermediate (0.065 g, 0.114 mmol) in THF (2 mL) was added TBAF (0.114 mL, 0.114 mmol) at room temperature and then stirred for 1h. The reaction mixture was diluted with the ethyl acetate (5 mL) followed by water (5 mL). The layers were separated and the aqueous layer was extracted with EtOAc (3×5 mL). The combined organic layers were washed with brine (5 mL), dried ($Na_2SO_4$) and filtered. The filtrate was concentrated under vacuum. The crude product was purified by flash column chromatography (silica gel, 3-4% methanol in DCM as eluent) followed by trituration with ether and n-pentane to afford 16 mg (31%) of the titled compound as white solid. $^1$HNMR (400 MHz, DMSO-$d_6$) δ 10.53 (s, 1H), 8.87 (s, 1H), 8.66 (s, 1H), 8.59 (d, $J$ = 2.5 *Hz,* 1H), 8.52 (d, $J$ = 2.5 Hz, 1H), 8.16 (s, 2H), 5.43 (t, $J$ = 5.0 Hz, 1H, $D_2O$ exchangeable), 3.65 (d, $J$ = 5.0 Hz, 2H), 2.85 (s, 3H), 1.19-1.10 (m, 2H), 0.88-0.82 (m, 2H); ESI-MS (m/z) 457.20 $(MH)^+$.

**Example-74:** The following compound was prepared by following the similar procedure described for example-73 from the corresponding intermediates:

1-(3-Chloro-4-(2H-1,2,3-triazol-2-yl)phenyl)-3-(7-(1-(hydroxymethyl)cyclopropyl)-2-methylthiazolo[5,4-b]pyridin-6-yl)urea (Compound 106)

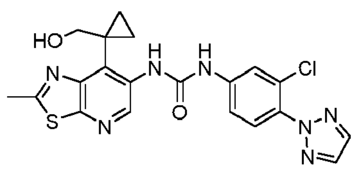

$^1$HNMR (400 MHz, DMSO-$d_6$) δ 10.21 (s, 1H), 8.86 (s, 1H), 8.48 (s, 1H), 8.12 (s, 2H), 8.00 (d, $J$ = 2.0 Hz, 1H), 7.63-7.54 (m, 2H), 5.36 (t, $J$ = 5.0 Hz, 1H), 3.65 (d, $J$ = 5.0 Hz, 2H), 2.85 (s, 3H), 1.14 (t, $J$ = 4.5 Hz, 2H), 0.83 (t, $J$ = 4.5 Hz, 2H); ESI-MS (m/z) 456.29 $(MH)^+$.

**Example-75:** 1-(3-Chloro-4-(2H-1,2,3-triazol-2-yl)phenyl)-3-(7-(1-(fluoromethyl)cyclopropyl)-2-methylthiazolo[5,4-b]pyridin-6-yl)urea (Compound 107)

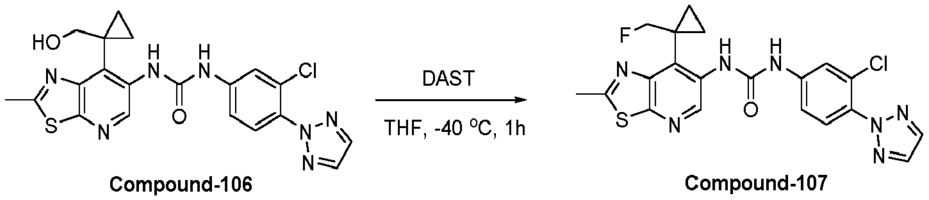


Compound-106 Compound-107

To a (-40°C) cooled and stirred solution of compound 106 (0.150 g, 0.329 mmol) in THF (15 mL) was added a solution of DAST (0.065 ml, 0.494 mmol) in THF (1 mL) dropwise. The resulting mixture was then continued to stir for 1h at the same temeparature. The reaction was quenched with aq. $NaHCO_3$ and exctratced with DCM (2×40mL). The combined organic layers were washed with brine (20 mL), dried ($Na_2SO_4$) and concentrated in vacuo. The crude product was purified by flash column chromatography (silica gel, 4-5% methanol in DCM as eluent) to afford 42 mg (28%) of the titled compound as white solid. $^1$HNMR (400 MHz, DMSO-d6) δ 10.15 (s, 1H), 8.94 (s, 1H), 8.13 (s, 2H), 8.04 (s, 1H), 7.99 (d, *J* = 2.5 Hz, 1H), 7.61 (d, *J* = 8.5 Hz, 1H), 7.52 (dd, *J* = 8.5, 2.5 Hz, 1H), 4.62 (d, *J* = 48.7 Hz, 2H), 2.86 (s, 3H), 1.30-1.28 (m, 2H), 1.08-1.04 (m, 2H); ESI-MS (m/z) 458.00 $(MH)^+$.

**Example-76:** 1-(5-Chloro-6-(2H-l,2,3-triazol-2-yl)pyridin-3-yl)-3-(7-(1-((dimethylamino)methyl)cyclopropyl)-2-methylthiazolo[5,4-b]pyridin-6-yl)urea (Compound 108)

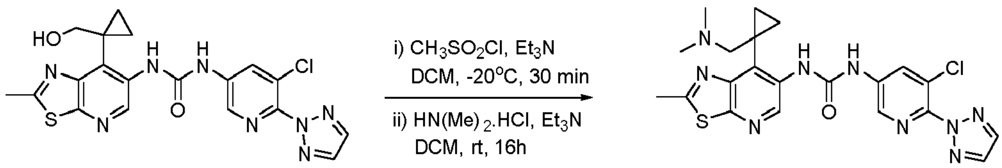


Compound-105 Compound-108

To a (-20°C) cooled and stirred solution of 1-(5-chloro-6-(2H-1,2,3-triazol-2-yl)pyridin-3-yl)-3-(7-(1-(hydroxymethyl)cyclopropyl)-2-methylthiazolo[5,4-b]pyridin-6-yl)urea (0.060 g, 0.131 mmol) in DCM (10 mL) was added dropwise methanesulfonyL chloride (0.012 mL, 0.158 mmol) followed by triethyl amine (0.027 mL, 0.197 mmol). The reaction mixture was stirred at the same temperature for 30 min. Then a solution of dimethylamine hydrochloride (0.032 g, 0.394 mmol) in DCM (2mL) and triethyl amine (0.092 mL, 0.657 mmol) was added to the above reaction mixture and the resulting mixture was stirred at 25°C for 16 hrs. The reaction mixture was quenched with ice cold water. The layers were separated and aqueous layer was extracted with DCM (2×10 mL). The combined organic layers were dried ($Na_2SO_4$) and filtered. The filtrate was rotary evaporated and the crude product was purified by fast column cromatography to give 10 mg (16%) of the titled compound as off white solid. $^1$HNMR (400 MHz, DMSO-$d_6$) δ 10.45 (s, 1H), 8.81 (s, 1H), 8.60 (d, *J* = 2.5 Hz, 1H), 8.52 (d, *J* = 2.5 Hz, 1H), 8.20 (s, 1H), 8.16 (s, 2H), 2.85 (s, 3H), 2.71 (s, 2H), 2.29 (s, 6H), 1.11-1.09 (m, 2H), 1.07-1.03 (m, 2H); ESI-MS (m/z) 483.97 (MH)+.

**Example-77:** Preparation of 1-(5-chloro-2,4-dimethoxyphenyl)-3-(7-(1-methoxyethyl)-2-methylthiazolo[5,4-b]pyridin-6-yl)urea (Compound 109)

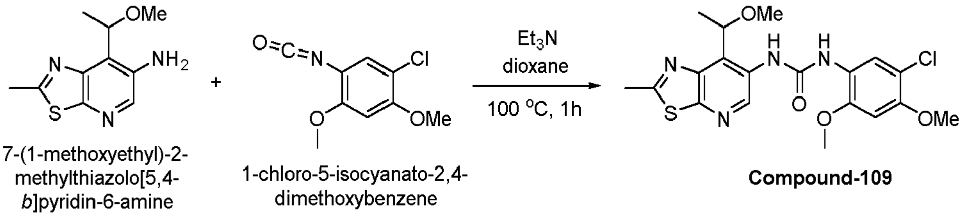


7-(1-methoxyethyl)-2-methylthiazolo[5,4-*b*]pyridin-6-amine

1-chloro-5-isocyanato-2,4-dimethoxybenzene

**Compound-109**

To a stirred solution of 1-chloro-5-isocyanato-2,4-dimethoxybenzene (0.191 g, 0.896 mmol) in dioxane (5 mL) was added a solution of 7-(1-methoxyethyl)-2-methylthiazolo[5,4-b]pyridin-6-amine (0.2 g, 0.896 mmol) in dioxane (5 mL) followed by the addition of triethylamine (0.25 mL, 1.79 mmol) at RT. The resulting mixture was stirred at 100°C for 1 h. The reaction mass was concentrated and the crude product was purified by flash column chromatography (silica gel, 40% EtOAc in hexane system as eluent) to afford 100 mg (66%) of the desired product as white solid. ESI-MS (*m/z*) 436.99 $(MH)^+$;

Chiral separation of racemic compound 109 was carried out using chiral column and afforded the below isomers 109a and 109b:

1-(5-Chloro-2,4-dimethoxyphenyl)-3-(7-(1-methoxyethyl)-2-methylthiazolo[5,4-b]pyridin-6-yl)urea (Compound 109a)
Chiral HPLC RT: 4.56 min
$^1$HNMR (400 MHz, DMSO-$d_6$) $\delta$ 8.98 (s, 1H, $D_2O$exchangeable), 8.88 (s, 1H), 8.62 (s, 1H, $D_2O$ exchangeable), 7.97 (s, 1H), 6.88 (s, 1H), 5.41 (q, *J* = 7.0 *Hz,* 1H), 3.94 (s, 3H), 3.88 (s, 3H), 3.18 (s, 3H), 2.84 (s, 3H), 1.52 (d, *J* = 7.0 *Hz,* 3H); ESI-MS (*m/z*) 437.00 $(MH)^+$;

1-(5-Chloro-2,4-dimethoxyphenyl)-3-(7-(1-methoxyethyl)-2-methylthiazolo[5,4-b]pyridin-6-yl)urea (Compound 109b)
Chiral HPLC RT: 5.10 min
$^1$HNMR (400 MHz, DMSO-$d_6$) $\delta$ 8.98 (s, 1H, $D_2O$exchangeable), 8.88 (s, 1H), 8.62 (s, 1H, $D_2O$ exchangeable), 7.97 (s, 1H), 6.88 (s, 1H), 5.41 (q, *J* = 7.0 *Hz,* 1H), 3.94 (s, 3H), 3.88 (s, 3H), 3.18 (s, 3H), 2.84 (s, 3H), 1.52 (d, *J* = 7.0 *Hz,* 3H); ESI-MS (*m/z*) 436.09 $(MH)^+$.

**Example-78:** Preparation of 1-(5-Chloro-6-methoxypyridin-3-yl)-3-(7-(dimethylamino)-2-methylthiazolo[5,4-b]pyridin-6-yl)urea (compound 110)

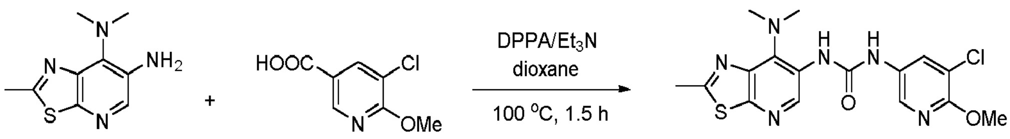


*N*$^7$,*N*$^7$,2-trimethylthiazolo[5,4-*b*]pyridine-6,7-diamine

**compound 110**

To a stirred solution of 5-chloro-6-methoxynicotinic acid (90 mg, 0.48 mmol) in 1,4-dioxane (5 mL) in a sealed vial, was added DPPA (0.13 mL, 0.57 mmol) and TEA (0.20 mL, 1.44 mmol). The reaction mixture was stirred 25°C for 45 min. Then N$^7$,N$^7$,2-trimethylthiazolo[5,4-b]pyridine-6,7-diamine (120 mg, 0.57 mmol) was added and heated the reaction mixture at 100°C for 1.5 h. After cooling to RT, water was added (5 mL) and extracted with ethyl acetate (10 mL×3). The combined organic layers were washed with saturated $NaHCO_3$ (10 mL), dried ($Na_2SO_4$) and filtered. The filtrate was rotary evaporated. The crude residue was then purified by flash column chromatography (silica gel, MeOH/DCM (5:95) as eluent) to provide 75 mg (40%) of the desired product as white solid. $^1$HNMR (400 MHz, DMSO-$d_6$) $\delta$ 9.42 (s, 1H), 8.86 (s, 1H), 8.41 (s, 1H), 8.16 (d, *J* = 2.5 *Hz,* 1H), 8.12 (d, *J* = 2.5 *Hz,* 1H), 3.91 (s, 3H), 3.05 (s, 6H), 2.80 (s, 3H); ESI-MS (m/z) 393.22 $(MH)^+$.

**Example-79:** The following examples were prepared from the corresponding intermediates by following the similar procedure described for example-78:

1-(5-Chloro-6-methoxypyridin-3-yl)-3-(2-methyl-7-(pyrrolidin-1-yl)thiazolo[5,4-b]pyridin-6-yl)urea (Compound 111)

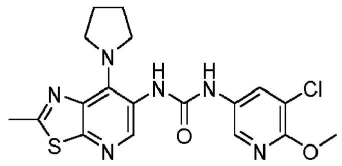

$^1$HNMR (400 MHz, DMSO-$d_6$) 8.86 (s, 1H), 8.24-8.03 (m, 3H), 7.96 (s, 1H), 3.98-3.85 (m, 7H), 2.73 (s, 3H), 1.94-1.82 (m, 4H); ESI-MS (m/z) 419.06 (MH)$^+$;

1-(5-Chloro-6-methoxypyridin-3-yl)-3-(2-methyl-7-morpholinothiazolo[5,4-b]pyridin-6-yl)urea (Compound 112)

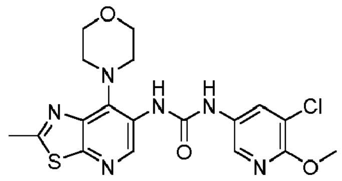

$^1$HNMR (400 MHz, DMSO-$d_6$) δ 9.51 (s, 1H), 8.90 (s, 1H), 8.26 (s, 1H), 8.17 (d, *J* = 2.5 Hz, 1H), 8.14 (d, *J* = 2.5 Hz, 1H), 3.92 (s, 3H), 3.83 (t, *J* = 4.5 *Hz,* 4H), 3.37 (t, *J* = 4.5 *Hz,* 4H), 2.82 (s, 3H); ESI-MS (m/z) 435.03 (MH)$^+$;

1-(5-Chloro-6-methoxypyridin-3-yl)-3-(7-(4,4-difluoropiperidin-1-yl)-2-methylthiazolo[5,4-b]pyridin-6-yl)urea (Compound 113)

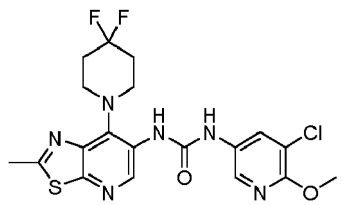

$^1$HNMR (400 MHz, DMSO-$d_6$) δ 9.49 (s, $D_2O$ exchangeable, 1H), 8.95 (s, 1H), 8.23 (s, $D_2O$ exchangeable, 1H), 8.18 (d, *J* = 2.5 *Hz,* 1H), 8.15 (d, *J* = 2.5 *Hz,* 1H), 3.92 (s, 3H), 3.46-3.43 (m, 4H), 2.82 (s, 3H), 2.29-2.21 (m, 4H ESI-MS (m/z) 469.01 (MH)$^+$;

1-(7-Cyclopropyl-2-methylthiazolo[5,4-b]pyridin-6-yl)-3-(2-(difluoromethyl)pyridin-4-yl)urea (Compound 114)

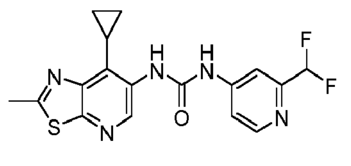

$^1$HNMR (400 MHz, DMSO-$d_6$) δ 9.77 (s, $D_2O$ exchangeable, 1H), 8.67 (s, $D_2O$ exchangeable, 1H), 8.59 (s, 1H), 8.46 (d, *J* = 5.5 *Hz,* 1H), 7.88 (d, *J* = 2.0 *Hz,* 1H), 7.53 (dd, *J* = 5.5, 2.0 *Hz,* 1H), 6.89 (t, *J* = 55.0 *Hz,* 1H), 2.81 (s, 3H), 2.23-2.11 (m, 1H), 1.56-1.54 (m, 2H), 1.19-1.07 (m, 2H); ESI-MS (m/z) 376.28 (MH)$^+$;

1-(5-Chloro-6-(2H-1,2,3-triazol-2-yl)pyridin-3-yl)-3-(7-isopropyl-2-methylthiazolo[5,4-b]pyridin-6-yl)urea (Compound 115)

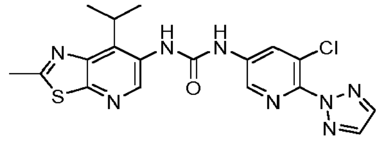

$^1$H NMR (400 MHz, DMSO-$d_6$) δ 9.74 (s, 1H), 8.76 (s, 1H), 8.58 (s, 1H), 8.54 (s, 1H), 8.47 (s, 1H), 8.16 (s, 2H), 3.61-3.52 (m, 1H), 2.86 (s, 3H), 1.49 (d, *J* = 6.9 Hz, 6H); ESI-MS (m/z) 429.10 (MH)$^+$;

1-(5-Chloro-6-methoxypyridin-3-yl)-3-(7-cyclopropyl-2-ethylthiazolo[5,4-b]pyridin-6-yl)urea (Compound 116)

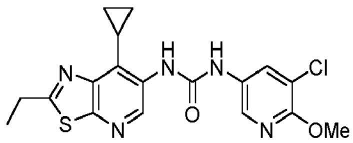

$^1$HNMR (400 MHz, DMSO-$d_6$) δ 9.12 (s, 1H), 8.62 (s, 1H), 8.50 (s, 1H), 8.15 (s, 2H), 3.91 (s, 3H), 3.12 (q, *J* = 7.5 Hz, 2H), 2.24-2.14 (m, 1H), 1.60-1.54 (m, 2H), 1.37 (t, *J* = 7.5 Hz, 3H), 1.19-1.11 (m, 2H); ESI-MS (m/z) 404.1 (MH)$^+$; and

1-(5-Chloro-6-methoxypyridin-3-yl)-3-(2-methyl-7-(1-methylcyclopropyl)thiazolo[5,4-b]pyridin-6-yl)urea (Compound 117)

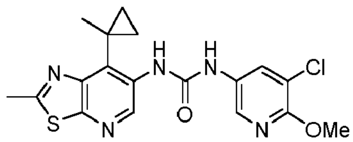

$^1$HNMR (400 MHz, DMSO-$d_6$) δ 9.62 (s, 1H), 8.94 (s, 1H), 8.20 (s, 1H), 8.13 (s, 1H), 8.07 (s, 1H), 3.92 (s, 3H), 2.85 (s, 3H), 1.42 (s, 3H), 1.00-1.02 (m, 2H), 0.88-0.90 (m, 2H); ESI-MS (m/z) 404.1 (MH)$^+$.

**Example-80:** Preparation of (±)-1-(3-chloro-4-methoxyphenyl)-3-(7-(1-methoxyethyl)-2-methylthiazolo[5,4-b]pyridin-6-yl)urea (Compound 118)

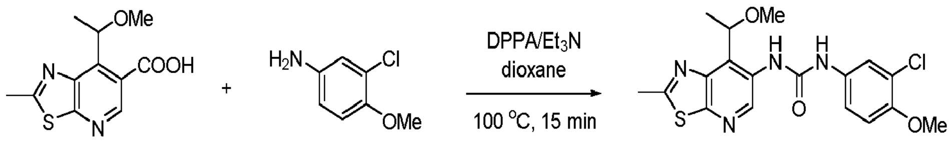


Compound-118

To a stirred solution of 7-(1-methoxyethyl)-2-methylthiazolo[5,4-b]pyridine-6-carboxylic acid (165 mg, 0.65 mmol) in dioxane (10 mL) was added triethylamine (182 μL, 1.31 mmol). The clear solution obtained was charged with [azido(phenoxy)phosphoryl]oxybenzene (163 μL, 0.75 mmol) and the reaction mixture was stirred at rt for 15 min in a sealed tube. Intermediate formation was observed by TLC and a solution of 3-chloro-4-methoxyaniline (103 mg, 0.65 mmol) in dioxane (2 mL) was added in 1 min to the above reaction mixture. The sealed tube was then heated at 100°C for 15 min. The reaction was cooled to room temperature and the solvent was evaporated under vacuum and the crude product was purified by flash column chromatography (silica gel, 0.7% MeOH in DCM) to afford 85 mg (32%) of the titled compound as white solid. ESI-MS (m/z) 406.96 (MH)$^+$.

Chiral separation of racemic compound 118 was carried out using chiral column and afforded the below isomers 118a and 118b:

1-(3-chloro-4-methoxyphenyl)-3-(7-(1-methoxyethyl)-2-methylthiazolo[5,4-b]pyridin-6-yl)urea (compound 118a)
Chiral HPLC RT 9.4 min
$^1$HNMR (400 MHz, DMSO-$d_6$) δ 9.71 (s, 1H), 9.10 (s, 1H), 8.41 (s, 1H), 7.72 (d, *J* = 2.5 *Hz,* 1H), 7.32 (dd, *J* = 8.5, 2.5 *Hz,* 1H), 7.12 (d, *J* = 8.5 *Hz,* 1H), 5.49 (q, *J* = 6.5 *Hz,* 1H), 3.82 (s, 3H), 3.28 (s, 3H), 2.84 (s, 3H), 1.52 (d, *J* = 6.5 *Hz,* 3H); ESI-MS (m/z), 406.98 (MH)$^+$ and

1-(3-chloro-4-methoxyphenyl)-3-(7-(1-methoxyethyl)-2-methylthiazolo[5,4-b]pyridin-6-yl)urea (compound 118b)
Chiral HPLC RT 10.12 min

$^1$HNMR (400 MHz, DMSO-$d_6$) δ 9.71 (s, 1H), 9.10 (s, 1H), 8.41 (s, 1H), 7.72 (d, *J = 2.5 Hz,* 1H), 7.32 (dd, *J* = 8.5, 2.5 *Hz,* 1H), 7.12 (d, *J* = 8.5 *Hz,* 1H), 5.49 (q, *J* = 6.5 *Hz,* 1H), 3.82 (s, 3H), 3.28 (s, 3H), 2.84 (s, 3H), 1.52 (d, *J* = 6.5 *Hz,* 3H); ESI-MS (m/z) 406.98 $(MH)^+$.

**Example-81:** The following compounds were prepared by using the similar procedure described for example-80 from the corresponding intermediates:

1-(5-Chloro-6-methoxypyridin-3-yl)-3-(7-isopropyl-2-methylthiazolo[5,4-b]pyridin-6-yl)urea (Compound 119)

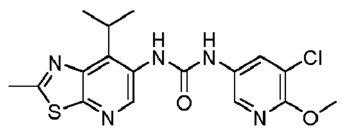

$^1$HNMR (400 MHz, DMSO-$d_6$) δ 9.03 (s, 1H), 8.52 (s, 1H), 8.44 (s, 1H), 8.16-8.11 (m, 2H), 3.91 (s, 3H), 3.63-3.48 (m, 1H), 2.85 (s, 3H), 1.48 (d, *J* = 6.9 Hz, 6H); ESI-MS (m/z) 392.04 $(MH)^+$;

(±)-1-(5-Chloro-2-methoxypyridin-3-yl)-3-(7-(1-methoxyethyl)-2-methylthiazolo[5,4-b]pyridin-6-yl)urea (Compound 120)

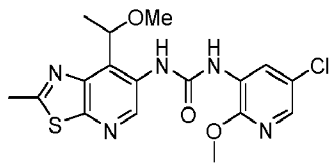

ESI-MS (m/z) 407.98 $(MH)^+$
Chiral separation of racemic compound 120 was carried out using chiral column and afforded the below isomers 120a and 120b:

1-(5-Chloro-2-methoxypyridin-3-yl)-3-(7-(1-methoxyethyl)-2-methylthiazolo[5,4-b]pyridin-6-yl)urea (compound 120a)
Chiral HPLC RT 5.37 min
$^1$HNMR (400 MHz, DMSO-$d_6$) δ 9.48 (s, 1H), 8.87 (s, 1H), 8.81 (s, 1H), 8.48 (d, *J = 2.5 Hz,* 1H), 7.84 (d, *J* = 2.5 *Hz,* 1H), 5.42 (q, *J* = 6.5 *Hz,* 1H), 4.01 (s, 3H), 3.21 (s, 3H), 2.85 (s, 3H), 1.54 (d, *J* = 6.5 *Hz,* 3H); ESI-MS (m/z) 408.00 $(MH)^+$;

1-(5-chloro-2-methoxypyridin-3-yl)-3-(7-(1-methoxyethyl)-2-methylthiazolo[5,4-b]pyridin-6-yl)urea (compound 120b)
Chiral HPLC RT 6.09 min
$^1$HNMR (400 MHz, DMSO-$d_6$) δ 9.48 (s, 1H), 8.87 (s, 1H), 8.81 (s, 1H), 8.48 (d, *J = 2.5 Hz,* 1H), 7.84 (d, *J* = 2.5 *Hz,* 1H), 5.42 (q, *J* = 6.5 *Hz,* 1H), 4.01 (s, 3H), 3.21 (s, 3H), 2.85 (s, 3H), 1.54 (d, *J* = 6.5 *Hz,* 3H); ESI-MS (m/z) 408.03 $(MH)^+$;

(±)-1-(5-Chloro-6-methoxypyridin-3-yl)-3-(7-(1-(2-methoxyethoxy)ethyl)-2-methylthiazolo[5,4-b]pyridin-6-yl)urea (Compound 121)

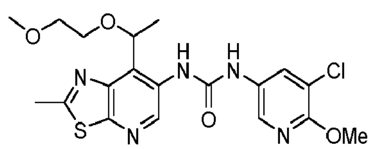

ESI-MS (m/z), 451.93 $(MH)^+$
Chiral separation of racemic compound 121 was carried out using chiral column and afforded the below isomers 121a and 121b:

1-(5-Chloro-6-methoxypyridin-3-yl)-3-(7-(1-(2-methoxyethoxy)ethyl)-2-methylthiazolo[5,4-b]pyridin-6-yl)urea (Compound 121a)
Chiral HPLC RT 5.93 min
$^1$HNMR (400 MHz, DMSO-$d_6$) δ 9.79 (s, 1H), 9.17 (s, 1H), 8.52 (s, 1H), 8.21 - 8.12 (m, 2H), 5.63 (q, *J* = 6.5 *Hz,* 1H), 3.92 (s, 3H), 3.59-3.53 (m, 1H), 3.52 - 3.40 (m, 3H), 3.17 (s, 3H), 2.84 (s, 3H), 1.55 (d, *J* = 6.5 *Hz,* 3H); ESI-MS (m/z), 451.93 $(MH)^+$;

1-(5-Chloro-6-methoxypyridin-3-yl)-3-(7-(1-(2-methoxyethoxy)ethyl)-2-methylthiazolo[5,4-b]pyridin-6-yl)urea (Compound 121b)
Chiral HPLC RT 6.81 min
$^1$HNMR (400 MHz, DMSO-$d_6$) δ 9.79 (s, 1H), 9.17 (s, 1H), 8.52 (s, 1H), 8.21 - 8.12 (m, 2H), 5.63 (q, *J* = 6.5 *Hz,* 1H), 3.92 (s, 3H), 3.59-3.53 (m, 1H), 3.52 - 3.40 (m, 3H), 3.17 (s, 3H), 2.84 (s, 3H), 1.55 (d, *J* = 6.5 *Hz,* 3H); ESI-MS (m/z), 451.93$(MH)^+$;

(±)-1-(5-Chloro-2,6-dimethoxypyridin-3-yl)-3-(7-(1-methoxyethyl)-2-methylthiazolo[5,4-b]pyridin-6-yl)urea (Compound 122)

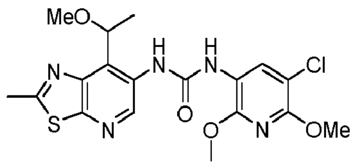

ESI-MS (*m/z*) 437.97 $(MH)^+$
Chiral separation of racemic compound 122 was carried out using chiral column and afforded the below isomers 122a and 122b:

1-(5-Chloro-2,6-dimethoxypyridin-3-yl)-3-(7-(1-methoxyethyl)-2-methylthiazolo[5,4-b]pyridin-6-yl)urea (Compound 122a)
Chiral HPLC RT 7.95 min
$^1$HNMR (400 M*Hz*, DMSO-$d_6$) δ 9.20 (s, 1H), 8.91(s, 1H), 8.66 (s, 1H), 8.35 (s, 1H), 5.43 (q, *J* = 7.0 *Hz*,1H), 4.02 (s, 3H), 3.95 (s, 3H), 3.21 (s, 3H), 2.84 (s, 3H), 1.52 (d, *J* = 7.0 *Hz,* 3H); ESI-MS (*m/z*) 437.97 $(MH)^+$;

1-(5-Chloro-2,6-dimethoxypyridin-3-yl)-3-(7-(1-methoxyethyl)-2-methylthiazolo[5,4-b]pyridin-6-yl)urea (Compound 122b)
Chiral HPLC RT 9.32 min
$^1$HNMR (400 M*Hz*, DMSO-$d_6$) δ 9.20 (s, 1H), 8.91(s, 1H), 8.66 (s, 1H), 8.35 (s, 1H), 5.43 (q, *J* = 7.0 *Hz*,1H), 4.02 (s, 3H), 3.95 (s, 3H), 3.21 (s, 3H), 2.84 (s, 3H), 1.52 (d, *J* = 7.0 *Hz,* 3H); ESI-MS (*m/z*) 437.96 $(MH)^+$;

(±)-1-(5-Chloro-6-(1H-1,2,3-triazol-1-yl)pyridin-3-yl)-3-(7-(1-methoxyethyl)-2-methylthiazolo[5,4-b]pyridin-6-yl)urea (Compound 123)

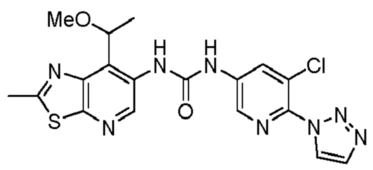

ESI-MS (*m/z*) 445 $(MH)^+$
Chiral separation of racemic compound 123 was carried out using chiral column and afforded the below isomers 123a and 123b:

1-(5-Chloro-6-(1H-1,2,3-triazol-1-yl)pyridin-3-yl)-3-(7-(1-methoxyethyl)-2-methylthiazolo[5,4-b]pyridin-6-yl)urea (Compound 123a)
Chiral HPLC RT 6.91 min
$^1$HNMR (400 M*Hz*, DMSO-$d_6$) δ 10.54 (s, 1H, $D_2O$ exchangeable), 9.12 (s, 1H), 8.75 (s, 1H, $D_2O$ exchangeable),

8.61 (s, 1H), 8.59 (d, *J* = 2.5 *Hz,* 1H), 8.53 (d, *J* = 2.5 *Hz,* 1H), 7.99 (s, 1H), 5.51 (q, *J* = 7.0 *Hz,* 1H), 3.32 (s, 3H), 2.86 (s, 3H), 1.56 (d, *J* = 7.0 *Hz,* 3H); ESI-MS (*m*/*z*) 445 $(MH)^+$;

1-(5-Chloro-6-(1H-1,2,3-triazol-1-yl)pyridin-3-yl)-3-(7-(1-methoxyethyl)-2-methylthiazolo[5,4-b]pyridin-6-yl)urea (Compound 123b)

Chiral HPLC RT 7.55 min

$^1$HNMR (400 M*Hz*, DMSO-$d_6$) δ 10.54 (s, 1H, $D_2O$ exchangeable), 9.12 (s, 1H), 8.75 (s, 1H, $D_2O$ exchangeable), 8.61 (s, 1H), 8.59 (d, *J* = 2.5 *Hz,* 1H), 8.53 (d, *J* = 2.5 *Hz,* 1H), 7.99 (s, 1H), 5.51 (q, *J* = 7.0 *Hz,* 1H), 3.32 (s, 3H), 2.86 (s, 3H), 1.56 (d, *J* = 7.0 *Hz,* 3H); ESI-MS (*m*/*z*) 445 $(MH)^+$;

(±)-1-(5-Chloro-6-methoxypyridin-3-yl)-3-(7-(1-methoxy-2-methylpropyl)-2-methylthiazolo[5,4-b]pyridin-6-yl)urea (Compound 124)

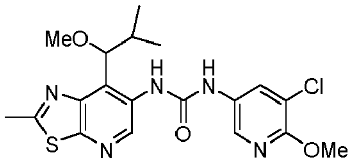

ESI-MS (*m*/*z*) 436.17 $(MH)^+$

Chiral separation of racemic compound 124 was carried out using chiral column and afforded the below isomers 124a and 124b:

1-(5-Chloro-6-methoxypyridin-3-yl)-3-(7-(1-methoxy-2-methylpropyl)-2-methylthiazolo[5,4-b]pyridin-6-yl)urea (Compound 124a)

Chiral HPLC RT: 4.45 min

$^1$HNMR (400 M*Hz*, DMSO-$d_6$) δ 9.87 (s, 1H, $D_2O$ exchangeable), 9.07 (s, 1H), 8.42 (s, 1H, $D_2O$ exchangeable), 8.17 (s, 1H), 8.14 (s, 1H), 5.02 (d, *J* = 8.0 *Hz,* 1H), 3.92 (s, 3H), 3.27 (s, 3H), 2.84 (s, 3H), 2.26-2.22 (m, 1H), 1.11 (d, *J* = 6.5 *Hz,* 3H), 0.66 (d, *J* = 6.5 *Hz,* 3H); ESI-MS (*m*/*z*) 436.09 $(MH)^+$;

1-(5-Chloro-6-methoxypyridin-3-yl)-3-(7-(1-methoxy-2-methylpropyl)-2-methylthiazolo[5,4-b]pyridin-6-yl)urea (Compound 124b)

Chiral HPLC RT: 5.10 min

$^1$HNMR (400 M*Hz*, DMSO-$d_6$) δ 9.87 (s, 1H, $D_2O$ exchangeable), 9.07 (s, 1H), 8.42 (s, 1H, $D_2O$ exchangeable), 8.17 (s, 1H), 8.14 (s, 1H), 5.02 (d, *J* = 8.0 *Hz,* 1H), 3.92 (s, 3H), 3.27 (s, 3H), 2.84 (s, 3H), 2.26-2.22 (m, 1H), 1.11 (d, *J* = 6.5 *Hz,* 3H), 0.66 (d, *J* = 6.5 *Hz,* 3H); ESI-MS (*m*/*z*) 436.09 $(MH)^+$;

(±)-1-(3-Chloro-4-(2H-1,2,3-triazol-2-yl)phenyl)-3-(7-(1-methoxy-2-methylpropyl)-2-methylthiazolo[5,4-b]pyridin-6-yl)urea (Compound 125)

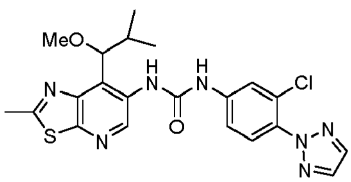

ESI-MS (*m*/*z*) 472.06 $(MH)^+$

Chiral separation of racemic compound 125 was carried out using chiral column and afforded the below isomers 125a and 125b:

1-(3-Chloro-4-(2H-1,2,3-triazol-2-yl)phenyl)-3-(7-(1-methoxy-2-methylpropyl)-2-methylthiazolo[5,4-b]pyridin-6-yl)urea (Compound 125a)

Chiral HPLC RT: 5.88 min

$^1$HNMR (400 M*Hz*, $CDCl_3$) δ 9.37 (s,1H), 7.89 (s, 2H), 7.83 (s, 1H), 7.54 (m, 2H), 7.29 (m, 2H), 5.18-5.10 (m, 1H), 3.40 (s, 3H), 2.83 (s, 3H), 2.29-2.26 (m, 1H), 1.19 (d, *J* = 7.0 *Hz,* 3H), 0.73 (d, *J* = 7.0 *Hz,* 3H); ESI-MS (*m*/*z*) 472.16 $(MH)^+$;

1-(3-Chloro-4-(2H-1,2,3-triazol-2-yl)phenyl)-3-(7-(1-methoxy-2-methylpropyl)-2-methylthiazolo[5,4-b]pyridin-6-yl)urea (Compound 125b)
Chiral HPLC RT: 7.69 min
$^1$H NMR (400 M*Hz*, DMSO-$d_6$) δ 10.23 (s, 1H, $D_2O$ exchangeable), 9.05 (s, 1H), 8.49 (s, 1H, $D_2O$ exchangeable), 8.12 (s, 2H), 7.98 (s, 1H), 7.62-7.60 (m, 1H), 7.56-7.54 (m, 1H), 5.04-5.02 (m, 1H), 3.29 (s, 3H), 2.85 (s, 3H), 2.27-2.25 (m, 1H), 1.12 (d, *J* = 6.5 *Hz,* 3H), 0.67 (d, *J* = 6.5 *Hz,* 3H); ESI-MS (*m*/*z*) 472.21 $(MH)^+$;

(±)-1-(3-Chloro-4-(2H-1,2,3-triazol-2-yl)phenyl)-3-(7-(1-methoxypropyl)-2-methylthiazolo[5,4-b]pyridin-6-yl)urea (Compound 126)

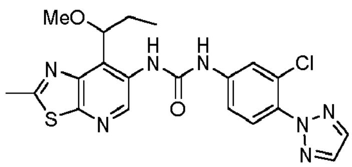

ESI-MS (m/z) 458.14 $(MH)^+$
Chiral separation of racemic compound 126 was carried out using chiral column and afforded the below isomers 126a and 126b:

1-(3-Chloro-4-(2H-1,2,3-triazol-2-yl)phenyl)-3-(7-(1-methoxypropyl)-2-methylthiazolo[5,4-b]pyridin-6-yl)urea (Compound 126a)
Chiral HPLC RT: 6.44 min
$^1$HNMR (400 MHz, DMSO-$d_6$) δ 10.24 (s, $D_2O$ exchangable,1H), 9.09 (s, 1H), 8.56 (s, $D_2O$ exchangeable, 1H), 8.13 (s, 2H), 7.99 (s, 1H), 7.61 (d, *J* = 8.5 *Hz,* 1H), 7.55 (d, *J* = 8.5 *Hz,* 1H), 5.36-5.24 (m, 1H), 3.31 (s, 3H), 2.85 (s, 3H), 2.09-2.02 (m, 1H), 1.84-1.77 (m, 1H), 0.92-0.86 (m, 3H); ESI-MS (m/z) 458.18 $(MH)^+$;

1-(3-Chloro-4-(2H-1,2,3-triazol-2-yl)phenyl)-3-(7-(1-methoxypropyl)-2-methylthiazolo[5,4-b]pyridin-6-yl)urea (Compound 126b)
Chiral HPLC RT: 8.12 min
$^1$HNMR (400 MHz, DMSO-$d_6$) δ 10.24 (s, $D_2O$ exchangable,1H), 9.09 (s, 1H), 8.56 (s, $D_2O$ exchangeable, 1H), 8.13 (s, 2H), 7.99 (s, 1H), 7.61 (d, *J* = 8.5 *Hz,* 1H), 7.55 (d, *J* = 8.5 *Hz,* 1H), 5.36-5.24 (m, 1H), 3.31 (s, 3H), 2.85 (s, 3H), 2.09-2.02 (m, 1H), 1.84-1.77 (m, 1H), 0.92-0.86 (m, 3H); ESI-MS (m/z) 458.18 $(MH)^+$;

(±)-1-(5-Chloro-6-methoxypyridin-3-yl)-3-(7-(1-methoxypropyl)-2-methylthiazolo[5,4-b]pyridin-6-yl)urea (Compound 127)

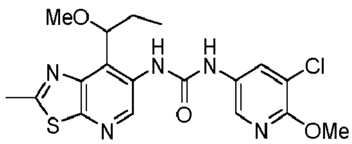

ESI-MS (m/z) 422.17 $(MH)^+$
Chiral separation of racemic compound 127 was carried out using chiral column and afforded the below isomers 127a and 127b:

1-(5-Chloro-6-methoxypyridin-3-yl)-3-(7-(1-methoxypropyl)-2-methylthiazolo[5,4-b]pyridin-6-yl)urea (Compound 127a)
Chiral HPLC RT: 4.60 min
$^1$HNMR (400 MHz, DMSO-$d_6$) δ 9.90 (s, 1H), 9.10 (s, 1H), 8.50 (s, 1H), 8.18 (s,1H), 8.15 (s, 1H), 5.31-5.27 (m, 1H), 3.92 (s, 3H), 3.30 (s, 3H), 2.85 (s, 3H), 2.06-1.98 (m, 1H), 1.82-1.78 (m,1H), 0.92-0.88 (m, 3H); ESI-MS (m/z) 422.16 $(MH)^+$;

1-(5-Chloro-6-methoxypyridin-3-yl)-3-(7-(1-methoxypropyl)-2-methylthiazolo[5,4-b]pyridin-6-yl)urea (Compound 127b)
Chiral HPLC RT: 4.97 min

$^1$HNMR (400 MHz, DMSO-$d_6$) δ 9.90 (s, 1H), 9.10 (s, 1H), 8.50 (s, 1H), 8.18 (s,1H), 8.15 (s, 1H), 5.31-5.27 (m, 1H), 3.92 (s, 3H), 3.30 (s, 3H), 2.85 (s, 3H), 2.06-1.98 (m, 1H), 1.82-1.78 (m,1H), 0.92-0.88 (m, 3H); ESI-MS (m/z) 422.16 (MH)$^+$;

(±)-1-(5-Chloro-2-methoxyphenyl)-3-(7-(1-methoxyethyl)-2-methylthiazolo[5,4-b]pyridin-6-yl)urea (Compound 128)

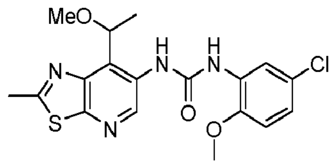

ESI-MS (*m/z*) 406.98 (MH)$^+$
Chiral separation of racemic compound 128 was carried out using chiral column and afforded the below isomers 128a and 128b:

1-(5-Chloro-2-methoxyphenyl)-3-(7-(1-methoxyethyl)-2-methylthiazolo[5,4-b]pyridin-6-yl)urea (Compound 128a)
Chiral HPLC RT: 6.41 min
$^1$HNMR (400 M*Hz*, DMSO-$d_6$) δ 9.21 (s, 1H), 8.80 (s, 1H), 8.79 (d, *J* = 2.0 *Hz,* 1H), 8.16 (d, *J* = 2.5 *Hz,* 1H), 7.09-6.96 (m, 2H), 5.40 (q, *J* = 7.0 *Hz,* 1H), 3.91 (s, 3H), 3.20 (s, 3H), 2.85 (s, 3H), 1.54 (d, *J* = 7.0 *Hz,* 3H); ESI-MS (*m/z*) 406.98 (MH)$^+$;

1-(5-Chloro-2-methoxyphenyl)-3-(7-(1-methoxyethyl)-2-methylthiazolo[5,4-b]pyridin-6-yl)urea (Compound 128b)
Chiral HPLC RT: 7.23 min
$^1$HNMR (400 M*Hz*, DMSO-$d_6$) δ 9.21 (s, 1H), 8.80 (s, 1H), 8.79 (d, *J* = 2.0 *Hz,* 1H), 8.16 (d, *J* = 2.5 *Hz,* 1H), 7.09-6.96 (m, 2H), 5.40 (q, *J* = 7.0 *Hz,* 1H), 3.91 (s, 3H), 3.20 (s, 3H), 2.85 (s, 3H), 1.54 (d, *J* = 7.0 *Hz,* 3H); ESI-MS (*m/z*) 406.98 (MH)$^+$;

(±)-1-(5-Cyanopyridin-3-yl)-3-(7-(1-methoxyethyl)-2-methylthiazolo[5,4-b]pyridin-6-yl)urea (Compound 129)

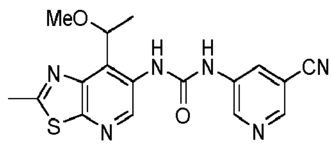

ESI-MS (m/z) 369.16 (MH)$^+$
Chiral separation of racemic compound 129 was carried out using chiral column and afforded the below isomers 129a and 129b:

1-(5-Cyanopyridin-3-yl)-3-(7-(1-methoxyethyl)-2-methylthiazolo[5,4-b]pyridin-6-yl)urea (Compound 129a)
Chiral HPLC RT: 5.26 min
$^1$HNMR (400 M*Hz*, DMSO-$d_6$) δ 10.33 (s, 1H), 9.08 (s, 1H), 8.84 (s, 1H), 8.67 (s,1H), 8.65 (s, 1H), 8.45 (s, 1H), 5.49 (q, *J* = 6.5 *Hz,* 1H), 3.30 (s, 3H), 2.85 (s, 3H),1.54 (d, *J* = 6.5 *Hz,* 3H); ESI-MS (m/z) 369.16 (MH)$^+$;

1-(5-Cyanopyridin-3-yl)-3-(7-(1-methoxyethyl)-2-methylthiazolo[5,4-b]pyridin-6-yl)urea (Compound 129b)
Chiral HPLC RT: 6.51 min
$^1$HNMR (400 M*Hz*, DMSO-$d_6$) δ 10.33 (s, 1H), 9.08 (s, 1H), 8.84 (s, 1H), 8.67 (s,1H), 8.65 (s, 1H), 8.45 (s, 1H), 5.49 (q, *J* = 6.5 *Hz,* 1H), 3.30 (s, 3H), 2.85 (s, 3H),1.54 (d, *J* = 6.5 *Hz,* 3H); ESI-MS (m/z) 369.16 (MH)$^+$;

(±)-1-(7-(1-Methoxyethyl)-2-methylthiazolo[5,4-b]pyridin-6-yl)-3-(2-(trifluoromethyl)pyridin-4-yl)urea (Compound 130)

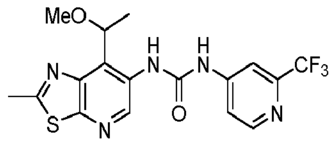

ESI-MS (m/z) 412.04 $(MH)^+$

Chiral separation of racemic compound 130 was carried out using chiral column and afforded the below isomers 130a and 130b:

1-(7-(1-Methoxyethyl)-2-methylthiazolo[5,4-b]pyridin-6-yl)-3-(2-(trifluoromethyl)pyridin-4-yl)urea (Compound 130a)

Chiral HPLC RT: 7.56 min

$^1$HNMR (400 MHz, DMSO-$d_6$) δ 10.60 (s, 1H, $D_2O$ exchangeable), 9.08 (s, 1H), 8.73 (s, 1H, $D_2O$ exchangeable), 8.58 (d, *J* = 8.0 *Hz,* 1H), 8.08 (d, *J* = 2.0 *Hz,* 1H), 7.66 (dd, *J* = 8.0 & 2.0 *Hz,* 1H), 5.49 (q, *J* = 6.5 *Hz,* 1H), 3.30 (s, 3H), 2.86 (s, 3H), 1.54 (d, *J* = 6.5 *Hz,* 3H); ESI-MS (m/z) 411.97 $(MH)^+$;

1-(7-(1-Methoxyethyl)-2-methylthiazolo[5,4-b]pyridin-6-yl)-3-(2-(trifluoromethyl)pyridin-4-yl)urea (Compound 130b)

Chiral HPLC RT: 8.21 min

$^1$HNMR (400 MHz, DMSO-$d_6$) δ 10.60 (s, 1H, $D_2O$ exchangeable), 9.08 (s, 1H), 8.73 (s, 1H, $D_2O$ exchangeable), 8.58 (d, *J* = 8.0 *Hz,* 1H), 8.08 (d, *J* = 2.0 *Hz,* 1H), 7.66 (dd, *J* = 8.0 & 2.0 *Hz,* 1H), 5.49 (q, *J* = 6.5 *Hz,* 1H), 3.30 (s, 3H), 2.86 (s, 3H), 1.54 (d, *J* = 6.5 *Hz,* 3H); ESI-MS (m/z) 412.10 $(MH)^+$;

(±)-1-(5-Chloro-2-methoxy-4-(2H-1,2,3-triazol-2-yl)phenyl)-3-(7-(1-methoxyethyl)-2-methylthiazolo[5,4-b]pyridin-6-yl)urea (Compound 131)

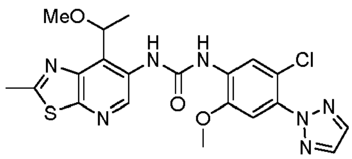

ESI-MS (m/z) 474.01 $(MH)^+$

Chiral separation of racemic compound 131 was carried out using chiral column and afforded the below isomers 131a and 131b:

1-(5-Chloro-2-methoxy-4-(2H-1,2,3-triazol-2-yl)phenyl)-3-(7-(1-methoxyethyl)-2-methylthiazolo[5,4-b]pyridin-6-yl)urea (Compound 131a)

Chiral HPLC RT: 5.78 min

$^1$HNMR (400 M*Hz*, DMSO-$d_6$) δ 9.48 (s, 1H), 8.91 (s, 1H), 8.82 (s, 1H), 8.44 (s, 1H), 8.13 (s, 2H), 7.34 (s, 1H), 5.42 (q, *J* = 6.5 *Hz,* 1H), 3.97 (s, 3H), 3.21 (s, 3H), 2.86 (s, 3H), 1.56 (d, *J* = 6.5 *Hz,* 3H); ESI-MS (m/z) 473.93 $(MH)^+$;

1-(5-Chloro-2-methoxy-4-(2H-1,2,3-triazol-2-yl)phenyl)-3-(7-(1-methoxyethyl)-2-methylthiazolo[5,4-b]pyridin-6-yl)urea (Compound 131b)

Chiral HPLC RT: 6.96 min

$^1$HNMR (400 M*Hz*, DMSO-$d_6$) δ 9.48 (s, 1H), 8.91 (s, 1H), 8.82 (s, 1H), 8.44 (s, 1H), 8.13 (s, 2H), 7.34 (s, 1H), 5.42 (q, *J* = 6.5 *Hz,* 1H), 3.97 (s, 3H), 3.21 (s, 3H), 2.86 (s, 3H), 1.56 (d, *J* = 6.5 *Hz,* 3H); ESI-MS (m/z) 473.93 $(MH)^+$;

(±)-1-(5-Chloro-2-methoxy-4-(1H-1,2,3-triazol-1-yl)phenyl)-3-(7-(1-methoxyethyl)-2-methylthiazolo[5,4-b]pyridin-6-yl)urea (Compound 132)

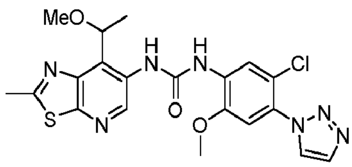

ESI-MS (m/z) 473.93 $(MH)^+$

Chiral separation of racemic compound 132 was carried out using chiral column and afforded the below isomers 132a and 132b:

1-(5-Chloro-2-methoxy-4-(1H-1,2,3-triazol-1-yl)phenyl)-3-(7-(1-methoxyethyl)-2-methylthiazolo[5,4-b]pyridin-6-yl)urea (Compound 132a)
Chiral HPLC RT: 7.00 min
$^1$HNMR (400 M*Hz*, DMSO-$d_6$) $\delta$ 9.50 (s, 1H, $D_2O$ Exchangeable), 8.91 (s, 1H, $D_2O$ Exchangeable), 8.82 (s, 1H), 8.53 (s, 1H), 8.47 (s, 1H), 7.98 (s, 1H), 7.38 (s, 1H), 5.42 (q, *J* = 6.5 *Hz,* 1H), 3.98 (s, 3H), 3.22 (s, 3H), 2.86 (s, 3H), 1.56 (d, *J* = 6.5 *Hz,* 3H); ESI-MS (m/z) 473.93 $(MH)^+$;

1-(5-Chloro-2-methoxy-4-(1H-1,2,3-triazol-1-yl)phenyl)-3-(7-(1-methoxyethyl)-2-methylthiazolo[5,4-b]pyridin-6-yl)urea (Compound 132b)
Chiral HPLC RT: 8.06 min
$^1$HNMR (400 M*Hz*, DMSO-$d_6$) $\delta$ 9.50 (s, 1H, $D_2O$ Exchangeable), 8.91 (s, 1H, $D_2O$ Exchangeable), 8.82 (s, 1H), 8.53 (s, 1H), 8.47 (s, 1H), 7.98 (s, 1H), 7.38 (s, 1H), 5.42 (q, *J* = 6.5 *Hz,* 1H), 3.98 (s, 3H), 3.22 (s, 3H), 2.86 (s, 3H), 1.56 (d, *J* = 6.5 *Hz,* 3H); ESI-MS (m/z) 474.01 $(MH)^+$;

(±)-1-(7-(1-Methoxyethyl)-2-methylthiazolo[5,4-b]pyridin-6-yl)-3-(1-methyl-2-oxo-5-(trifluoromethyl)-1,2-dihydropyridin-3-yl)urea (Compound 133)

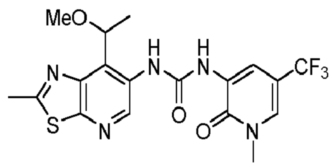

ESI-MS (m/z) 442.11 $(MH)^+$

Chiral separation of racemic compound 133 was carried out using chiral column and afforded the below isomers 133a and 133b:

1-(7-(1-Methoxyethyl)-2-methylthiazolo[5,4-b]pyridin-6-yl)-3-(1-methyl-2-oxo-5-(trifluoromethyl)-1,2-dihydropyridin-3-yl)urea (Compound 133a)
Chiral HPLC RT: 7.34 min
$^1$HNMR (400 M*Hz*, DMSO-$d_6$): $\delta$ 9.74 (s, 1H, $D_2O$ exchangeable), 9.08 (s, 1H, $D_2O$ exchangeable), 8.78 (s, 1H), 8.30 (s, 1H), 8.06 (s, 1H), 5.40 (q, *J* = 6.5 *Hz,* 1H), 3.61 (s, 3H), 3.18 (s, 3H), 2.85 (s, 3H), 1.53 (d, *J* = 6.5 *Hz,* 3H); ESI-MS (m/z) 441.98 $(MH)^+$;

1-(7-(1-methoxyethyl)-2-methylthiazolo[5,4-b]pyridin-6-yl)-3-(1-methyl-2-oxo-5-(trifluoromethyl)-1,2-dihydropyridin-3-yl)urea (Compound 133b)
Chiral HPLC RT: 9.22 min
$^1$HNMR (400 M*Hz*, DMSO-$d_6$): $\delta$ 9.74 (s, 1H, $D_2O$ exchangeable), 9.08 (s, 1H, $D_2O$ exchangeable), 8.78 (s, 1H), 8.30 (s, 1H), 8.06 (s, 1H), 5.40 (q, *J* = 6.5 *Hz,* 1H), 3.61 (s, 3H), 3.18 (s, 3H), 2.85 (s, 3H), 1.53 (d, *J* = 6.5 *Hz,* 3H); ESI-MS (m/z) 441.97 (MH)+;

(±)-1-(3-Chloro-4-(2H-1,2,3-triazol-2-yl)phenyl)-3-(7-(1-methoxypropan-2-yl)-2-methylthiazolo[5,4-b]pyridin-6-yl)urea (Compound 134)

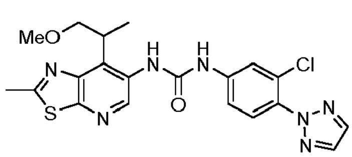

ESI-MS (m/z) 458.00 $(MH)^+$

Chiral separation of racemic compound 134 was carried out using chiral column and afforded the below isomers 134a and 134b:

1-(3-Chloro-4-(2H-1,2,3-triazol-2-yl)phenyl)-3-(7-(1-methoxypropan-2-yl)-2-methylthiazolo[5,4-b]pyridin-6-yl)urea (Compound 134a)
Chiral HPLC RT: 8.03 min
$^1$HNMR (400 MHz, DMSO-$d_6$) δ 9.53 (s, 1H), 8.60 (s, 1H), 8.48 (s, 1H), 8.12 (s, 2H), 7.95 (s, 1H), 7.59 (d, *J* = 8.5 Hz, 1H), 7.52 (d, *J* = 9.1 Hz, 1H), 3.91 (t, *J* = 8.1 Hz, 1H), 3.83 (t, *J* = 7.7 Hz, 1H), 3.71 (q, *J* = 7.0 Hz, 1H), 3.20 (s, 3H), 2.86 (s, 3H), 1.45 (d, *J* = 6.8 Hz, 3H); ESI-MS (m/z) 458.00 $(MH)^+$;

1-(3-Chloro-4-(2H-1,2,3-triazol-2-yl)phenyl)-3-(7-(1-methoxypropan-2-yl)-2-methylthiazolo[5,4-b]pyridin-6-yl)urea (Compound 134b)
Chiral HPLC RT: 8.72 min
$^1$H NMR (400 MHz, DMSO-$d_6$) δ 9.54 (s, 1H), 8.60 (s, 1H), 8.49 (s, 1H), 8.12 (s, 2H), 7.95 (s, 1H), 7.59 (d, *J* = 8.4 Hz, 1H), 7.52 (d, *J* = 8.6 Hz, 1H), 3.91 (t, *J* = 7.4 Hz, 1H), 3.83 (t, *J* = 7.3 Hz, 1H), 3.76 -3.65 (m, 1H), 3.20 (s, 3H), 2.85 (s, 3H), 1.45 (d, *J* = 6.5 Hz, 3H); ESI-MS (m/z) 458.00$(MH)^+$;

(±)-1-(5-Chloro-6-methoxypyridin-3-yl)-3-(2-methyl-7-(tetrahydrofuran-2-yl)thiazolo[5,4-b]pyridin-6-yl)urea (Compound 135)

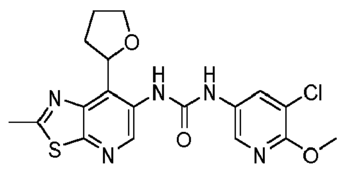

ESI-MS (m/z) 419.98 $(MH)^+$
Chiral separation of racemic compound 135 was carried out using chiral column and afforded the below isomers 135a and 135b:

1-(5-Chloro-6-methoxypyridin-3-yl)-3-(2-methyl-7-(tetrahydrofuran-2-yl)thiazolo[5,4-b]pyridin-6-yl)urea (Compound 135a)
Chiral HPLC RT: 5.35 min
$^1$HNMR (400 MHz, DMSO-d6) δ 9.69 (s, 1H), 8.87 (s, 1H), 8.56 (s, 1H), 8.14 (s, 2H), 5.65 (t, *J* = 7.2 Hz, 1H), 4.31 (dd, J = 7.2, 6.5 Hz, 1H), 3.92 (s, 3H, overlap with m, 1H), 2.84 (s, 3H), 2.36-2.34 (m, 1H), 2.11-2.09 (m, 2H), 1.91-1.89 (m, 1H); ESI-MS (m/z) 419.95 $(MH)^+$;

1-(5-Chloro-6-methoxypyridin-3-yl)-3-(2-methyl-7-(tetrahydrofuran-2-yl)thiazolo[5,4-b]pyridin-6-yl)urea (Compound 135b)
Chiral HPLC RT: 5.93 min
$^1$HNMR (400 MHz, DMSO-$d_6$) δ 9.69 (s, 1H), 8.87 (s, 1H), 8.56 (s, 1H), 8.14 (s, 2H), 5.65 (t, *J* = 7.2 Hz, 1H), 4.31 (dd, J = 7.2, 6.5 Hz, 1H), 3.92 (s, 3H, overlap with m, 1H), 2.84 (s, 3H), 2.39-2.33 (m, 1H), 2.13-2.06 (m, 2H), 1.94-1.86 (m, 1H); ESI-MS (m/z) 419.96 $(MH)^+$;

(±)-1-(5-Chloro-6-(2H-1,2,3-triazol-2-yl)pyridin-3-yl)-3-(2-methyl-7-(tetrahydrofuran-2-yl)thiazolo[5,4-b]pyridin-6-yl)urea; (Compound 136)

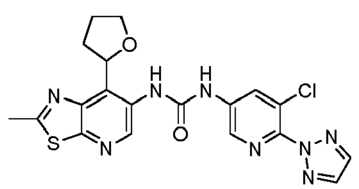

ESI-MS (m/z) 456.8 $(MH)^+$
Chiral separation of racemic compound 136 was carried out using chiral column and afforded the below isomers 136a and 136b:

1-(5-Chloro-6-(2H-1,2,3-triazol-2-yl)pyridin-3-yl)-3-(2-methyl-7-(tetrahydrofuran-2-yl)thiazolo[5,4-b]pyridin-6-yl)urea (Compound 136a)

Chiral HPLC RT: 4.48 min
$^1$HNMR (400 MHz, DMSO-$d_6$) δ 10.34 (s, 1H), 8.87 (s, 1H), 8.79 (s, 1H), 8.55 (d, J = 2.3 Hz, 1H), 8.49 (d, J = 2.4 Hz, 1H), 8.17 (s, 2H), 5.67 (dd, J = 9.5, 6.7 Hz, 1H), 4.34 (q, J = 7.5 Hz, 1H), 3.95 - 3.90 (m, 1H), 2.85 (s, 3H), 2.38-2.36 (m, 1H), 2.16 - 2.07 (m, 2H), 1.97 - 1.88 (m, 1H); ESI-MS (m/z) 456.81 (MH)$^+$;

1-(5-Chloro-6-(2H-1,2,3-triazol-2-yl)pyridin-3-yl)-3-(2-methyl-7-(tetrahydrofuran-2-yl)thiazolo[5,4-b]pyridin-6-yl)urea (Compound 136b)
Chiral HPLC RT: 6.56 min
$^1$HNMR (400 MHz, DMSO-$d_6$) δ 10.34 (s, 1H), 8.88 (s, 1H), 8.79 (s, 1H), 8.55 (d, *J* = 2.4 Hz, 1H), 8.49 (d, *J* = 2.3 Hz, 1H), 8.17 (s, 2H), 5.69-5.64 (m, 1H), 4.34 (q, *J* = 7.5 Hz, 1H), 3.95-3.90 (m, 1H), 2.85 (s, 3H), 2.38-2.35 (m, 1H), 2.15-2.09 (m, 2H), 1.97-1.88 (m, 1H); ESI-MS (m/z) 456.81 (MH)$^+$;

(±)-1-(3-Chloro-4-(2H-1,2,3-triazol-2-yl)phenyl)-3-(2-methyl-7-(tetrahydrofuran-2-yl)thiazolo[5,4-b]pyridin-6-yl)urea (Compound 137)

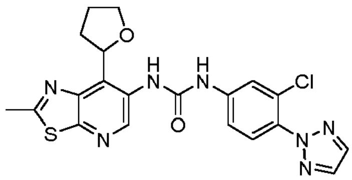

ESI-MS (m/z) 456.04 (MH)$^+$
Chiral separation of racemic compound 137 was carried out using chiral column and afforded the below isomers 137a and 137b:

1-(3-Chloro-4-(2H-1,2,3-triazol-2-yl)phenyl)-3-(2-methyl-7-(tetrahydrofuran-2-yl)thiazolo[5,4-b]pyridin-6-yl)urea; (Compound 137a)
Chiral HPLC RT: 4.35 min
$^1$HNMR (400 MHz, DMSO-d6) δ 8.76 (s, 1H), 8.02 (s, 2H), 7.87 (d, J = 2.1 Hz, 1H), 7.49 (d, J = 8.7 Hz, 1H), 7.43 (dd, J = 8.8, 2.1 Hz, 1H), 5.66 (dd, *J* = 9.6, 6.8 Hz, 1H), 4.23 (q, J = 7.2 Hz, 1H), 3.94-3.88 (m, 1H), 2.64 (s, 3H), 2.18-2.10 (m, 1H), 1.95 - 1.88 (m, 2H), 1.77-1.69 (m, 1H); ESI-MS (m/z) 455.93 (MH)$^+$;

1-(3-Chloro-4-(2H-1,2,3-triazol-2-yl)phenyl)-3-(2-methyl-7-(tetrahydrofuran-2-yl)thiazolo[5,4-b]pyridin-6-yl)urea; (Compound 137b)
Chiral HPLC RT: 4.94 min
$^1$HNMR (400 MHz, DMSO-$d_6$) δ 8.85 (s, 1H), 8.12 (s, 2H), 7.97 (d, J = 2.2 Hz, 1H), 7.60 (d, *J* = 8.7 Hz, 1H), 7.55 (d, *J* = 2.2 Hz, 1H), 5.66 (dd, *J* = 9.5, 6.7 Hz, 1H), 4.33 (d, *J* = 7.4 Hz, 1H), 3.94-3.88 (m, 1H), 2.84 (s, 3H), 2.40-2.35 (m, 1H), 2.14-2.07 (m, 2H), 1.95-1.90 (m, 1H); ESI-MS (m/z) 455.94 (MH)$^+$;

(±)-1-(3-Chloro-4-(2H-1,2,3-triazol-2-yl)phenyl)-3-(2-methyl-7-(tetrahydro-2H-pyran-2-yl)thiazolo[5,4-b]pyridin-6-yl)urea (Compound 138)

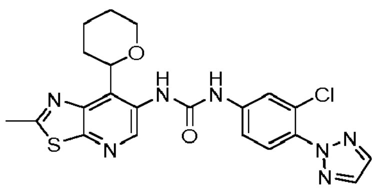

ESI-MS (m/z) 469.93 (MH)$^+$
Chiral separation of racemic compound 138 was carried out using chiral column and afforded the below isomers 138a and 138b:

1-(3-Chloro-4-(2H-1,2,3-triazol-2-yl)phenyl)-3-(2-methyl-7-(tetrahydro-2H-pyran-2-yl)thiazolo[5,4-b]pyridin-6-yl)urea (Compound 138a)
Chiral HPLC RT: 5.78 min
$^1$HNMR (400 MHz, DMSO-$d_6$) δ 10.24 (s, 1H), 8.92 (s, 1H), 8.75 (s, 1H), 8.13 (s, 2H), 7.98 (s, 1H), 7.62 (d, *J*

= 8.5 Hz, 1H), 7.56 (d, *J* = 8.7 Hz, 1H), 5.47 (d, *J* = 10.4 Hz, 1H), 4.23 (d, *J* = 10.8 Hz, 1H), 3.65 (t, *J* = 10.9 Hz, 1H), 2.85 (s, 3H), 1.98-1.56 (m, 6H); ESI-MS (m/z) 469.93 $(MH)^+$;

1-(3-Chloro-4-(2H-1,2,3-triazol-2-yl)phenyl)-3-(2-methyl-7-(tetrahydro-2H-pyran-2-yl)thiazolo[5,4-b]pyridin-6-yl)urea (Compound 138b)
Chiral HPLC RT: 7.25 min
$^1$HNMR (400 MHz, DMSO-$d_6$) $\delta$ 10.24 (s, 1H), 8.92 (s, 1H), 8.75 (s, 1H), 8.13 (s, 2H), 7.98 (s, 1H), 7.62 (d, *J* = 8.6 Hz, 1H), 7.56 (d, *J* = 8.7 Hz, 1H), 5.47 (d, *J* = 10.4 Hz, 1H), 4.23 (d, *J* = 11.9 Hz, 1H), 3.65 (t, *J* = 11.2 Hz, 1H), 2.85 (s, 3H), 1.96-1.56 (m, 6H); ESI-MS (m/z) 469.93 $(MH)^+$;

(±)-1-(5-Chloro-6-methoxypyridin-3-yl)-3-(2-methyl-7-(tetrahydro-2H-pyran-2-yl)thiazolo[5,4-b]pyridin-6-yl)urea (Compound 139)

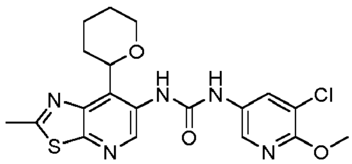

ESI-MS (m/z) 434.10 $(MH)^+$
Chiral separation of racemic compound 139 was carried out using chiral column and afforded the below isomers 139a and 139b:

1-(5-Chloro-6-methoxypyridin-3-yl)-3-(2-methyl-7-(tetrahydro-2H-pyran-2-yl)thiazolo[5,4-b]pyridin-6-yl)urea (Compound 139a)
Chiral HPLC RT: 5.03 min
$^1$HNMR (400 MHz, DMSO-$d_6$) $\delta$ 9.86 (s, 1H), 8.94 (s, 1H), 8.67 (s, 1H), 8.16 (s, 2H), 5.46 (d, *J* = 9.5 Hz, 1H), 4.18 (d, *J* = 11.4 Hz, 1H), 3.93 (s, 3H),3.63 (t, *J* = 10.5 Hz, 1H), 2.84 (s, 3H), 1.96 - 1.56 (m, 6H); ESI-MS (m/z) 434.10 $(MH)^+$;

1-(5-Chloro-6-methoxypyridin-3-yl)-3-(2-methyl-7-(tetrahydro-2H-pyran-2-yl)thiazolo[5,4-b]pyridin-6-yl)urea (Compound 139b)
Chiral HPLC RT: 5.76 min
$^1$HNMR (400 MHz, DMSO-$d_6$) $\delta$ 9.86 (s, 1H), 8.95 (s, 1H), 8.68 (s, 1H), 8.16 (s, 2H), 5.46 (d, *J* = 9.6 Hz, 1H), 4.18 (d, *J* = 10.2 Hz, 1H), 3.93 (s, 3H), 3.63 (t, *J* = 11.4 Hz, 1H), 2.84 (s, 3H), 1.95 - 1.55 (m, 6H); ESI-MS (m/z) 434.10 $(MH)^+$;

(±)-1-(5-Chloro-6-(2H-1,2,3-triazol-2-yl)pyridin-3-yl)-3-(2-methyl-7-(tetrahydro-2H-pyran-2-yl)thiazolo[5,4-b]pyridin-6-yl)urea (Compound 140)

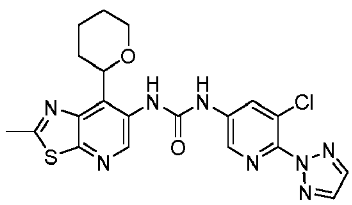

ESI-MS (m/z) 471.15 $(MH)^+$
Chiral separation of racemic compound 140 was carried out using chiral column and afforded the below isomers 140a and 140b:

1-(5-Chloro-6-(2H-1,2,3-triazol-2-yl)pyridin-3-yl)-3-(2-methyl-7-(tetrahydro-2H-pyran-2-yl)thiazolo[5,4-b]pyridin-6-yl)urea (Compound 140a)
Chiral HPLC RT: 5.26 min
$^1$HNMR (400 MHz, DMSO-$d_6$) $\delta$ 10.54 (s, 1H), 8.94 (s, 1H), 8.88 (s, 1H), 8.57 (s, 1H), 8.51 (s, 1H), 8.17 (s, 2H), 5.48 (d, *J* = 9.8 Hz, 1H), 4.24 (d, *J* = 11.9 Hz, 1H), 3.66 (t, *J* = 11.5 Hz, 1H), 2.85 (s, 3H), 2.00-1.56 (m, 6H);

ESI-MS (m/z) 471.12 $(MH)^+$;

1-(5-Chloro-6-(2H-1,2,3-triazol-2-yl)pyridin-3-yl)-3-(2-methyl-7-(tetrahydro-2H-pyran-2-yl)thiazolo[5,4-b]pyridin-6-yl)urea (Compound 140b)
Chiral HPLC RT: 6.55 min
$^1$HNMR (400 MHz, DMSO-$d_6$) δ 10.54 (s, 1H), 8.94 (s, 1H), 8.88 (s, 1H), 8.57 (s, 1H), 8.51 (s, 1H), 8.17 (s, 2H), 5.48 (d, *J* = 11.3 Hz, 1H), 4.23 (d, *J* = 8.8 Hz, 1H), 3.67 (d, *J* = 11.8 Hz, 1H), 2.85 (s, 3H), 1.96-1.59 (m, 6H); ESI-MS (m/z) 471.15 $(MH)^+$;

(±)-1-(6-(1H-1,2,3-Triazol-1-yl)-5-(trifluoromethyl)pyridin-3-yl)-3-(7-(1-methoxyethyl)-2-methylthiazolo[5,4-b]pyridin-6-yl)urea (Compound 141)

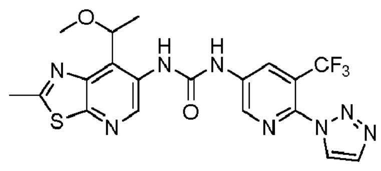

ESI-MS (m/z) 479.24 $(MH)^+$
Chiral separation of racemic compound 141 was carried out using chiral column and afforded the below isomers 141a and 141b:

1-(6-(1H-1,2,3-Triazol-1-yl)-5-(trifluoromethyl)pyridin-3-yl)-3-(7-(1-methoxyethyl)-2-methylthiazolo[5,4-b]pyridin-6-yl)urea (Compound 141a)
Chiral HPLC RT: 9.25 min
$^1$HNMR (400 MHz, DMSO-$d_6$) δ 10.73 (s, 1H), 9.13 (s, 1H), 8.89 (d, *J* = 2.5 Hz, 1H), 8.80 (s, 1H), 8.74 (d, *J* = 2.5 Hz, 1H), 8.64 (s, 1H), 8.00 (s, 1H), 5.51 (q, *J* = 6.7 Hz, 1H), 3.35 (s, 3H), 2.86 (s, 3H), 1.57 (d, *J* = 6.7 Hz, 3H); ESI-MS (m/z) 479.24 $(MH)^+$;

1-(6-(1H-1,2,3-Triazol-1-yl)-5-(trifluoromethyl)pyridin-3-yl)-3-(7-(1-methoxyethyl)-2-methylthiazolo[5,4-b]pyridin-6-yl)urea (Compound 141b)
Chiral HPLC RT: 10.41 min
$^1$HNMR (400 MHz, DMSO-$d_6$) δ 10.70 (s, 1H), 9.13 (s, 1H), 8.89 (d, *J* = 2.5 Hz, 1H), 8.79 (s, 1H), 8.74 (d, *J* = 2.5 Hz, 1H), 8.64 (s, 1H), 8.00 (s, 1H), 5.50 (q, J = 6.7 Hz, 1H), 3.34 (s, 3H), 2.86 (s, 3H), 1.57 (d, *J* = 6.7 Hz, 3H); ESI-MS (m/z) 479.19 $(MH)^+$;

(±)-1-(6-(2H-1,2,3-Triazol-2-yl)-5-(trifluoromethyl)pyridin-3-yl)-3-(7-(1-methoxyethyl)-2-methylthiazolo[5,4-b]pyridin-6-yl)urea (Compound 142)

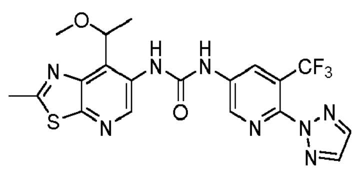

ESI-MS (m/z) 479.07 $(MH)^+$
Chiral separation of racemic compound 142 was carried out using chiral column and afforded the below isomers 142a and 142b:

1-(6-(2H-1,2,3-Triazol-2-yl)-5-(trifluoromethyl)pyridin-3-yl)-3-(7-(1-methoxyethyl)-2-methylthiazolo[5,4-b]pyridin-6-yl)urea (Compound 142a)
Chiral HPLC RT: 5.85 min
$^1$HNMR (400 MHz, DMSO-$d_6$) d 10.71 (s, 1H, $D_2O$ exchangeable), 9.14 (s, 1H), 8.86 (d, *J* = 2.4 Hz, 1H), 8.80 (s, 1H, $D_2O$ exchangeable), 8.74 (d, *J* = 2.5 Hz, 1H), 8.18 (s, 2H), 5.51 (q, *J* = 6.7 Hz, 1H), 3.32 (s, 3H), 2.86 (s, 3H), 1.57 (d, *J* = 6.7 Hz, 3H); ESI-MS (m/z) 479.12 $(MH)^+$;

1-(6-(2H-1,2,3-Triazol-2-yl)-5-(trifluoromethyl)pyridin-3-yl)-3-(7-(1-methoxyethyl)-2-methylthiazolo[5,4-b]pyridin-6-yl)urea (Compound 142b)
Chiral HPLC RT: 6.53 min
$^1$HNMR (400 MHz, DMSO-$d_6$) d 10.71 (s, 1H, $D_2O$ exchangeable), 9.14 (s, 1H), 8.86 (d, *J* = 2.4 Hz, 1H), 8.80 (s, 1H, $D_2O$ exchangeable), 8.74 (d, *J* = 2.5 Hz, 1H), 8.18 (s, 2H), 5.51 (q, *J* = 6.7 Hz, 1H), 3.32 (s, 3H), 2.86 (s, 3H), 1.57 (d, *J* = 6.7 Hz, 3H); ESI-MS 479.12 (m/z) $(MH)^+$;

(±)-1-(4-(2H-1,2,3-Triazol-2-yl)-3-(trifluoromethyl)phenyl)-3-(7-(1-methoxyethyl)-2-methylthiazolo[5,4-b]pyridin-6-yl)urea (Compound 143)

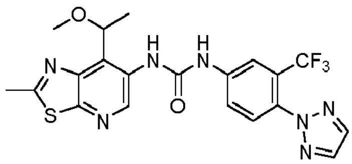

ESI-MS (m/z) 478.18 $(MH)^+$
Chiral separation of racemic compound 143 was carried out using chiral column and afforded the below isomers 143a and 143b:

1-(4-(2H-1,2,3-Triazol-2-yl)-3-(trifluoromethyl)phenyl)-3-(7-(1-methoxyethyl)-2-methylthiazolo[5,4-b]pyridin-6-yl)urea (Compound 143a)
Chiral HPLC RT: 4.90 min
$^1$HNMR (400 MHz, DMSO-$d_6$) δ 10.43 (s, 1H), 9.13 (s, 1H), 8.65 (s, 1H), 8.20 (s, 1H), 8.14 (s, 2H), 7.93 (d, *J* = 8.9 Hz, 1H), 7.70 (d, *J* = 8.8 Hz, 1H), 5.51 (q, *J* = 6.7 Hz, 1H), 3.31 (s, 3H), 2.86 (s, 3H), 1.56 (d, *J* = 6.7 Hz, 3H); ESI-MS (m/z) 478.18 $(MH)^+$;

1-(4-(2H-1,2,3-Triazol-2-yl)-3-(trifluoromethyl)phenyl)-3-(7-(1-methoxyethyl)-2-methylthiazolo[5,4-b]pyridin-6-yl)urea (Compound 143b)
Chiral HPLC RT: 5.61 min
$^1$HNMR (400 MHz, DMSO-*$d_6$*) δ 10.44 (s, 1H), 9.12 (s, 1H), 8.65 (s, 1H), 8.20 (s, 1H), 8.14 (s, 2H), 7.93 (d, *J* = 8.9 Hz, 1H), 7.70 (d, *J* = 8.9 Hz, 1H), 5.51 (q, *J* = 6.7 Hz, 1H), 3.31 (s, 3H), 2.86 (s, 3H), 1.56 (d, *J* = 6.7 Hz, 3H); ESI-MS (m/z) 478.18 $(MH)^+$;

(±)-1-(3-Chloro-4-(2H-1,2,3-triazol-2-yl)phenyl)-3-(7-(cyclopropyl(methoxy)methyl)-2-methylthiazolo[5,4-b]pyridin-6-yl)urea (Compound 144)

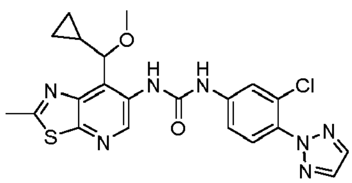

ESI-MS (m/z) 470.03 $(MH)^+$
Chiral separation of racemic compound 144 was carried out using chiral column and afforded the below isomers 144a and 144b:

1-(3-Chloro-4-(2H-1,2,3-triazol-2-yl)phenyl)-3-(7-(cyclopropyl(methoxy)methyl)-2-methylthiazolo[5,4-b]pyridin-6-yl)urea (Compound 144a)
Chiral HPLC RT: 6.39 min
$^1$HNMR (400 MHz, DMSO-*$d_6$*) δ 10.32 (s, 1H), 9.12 (s, 1H), 8.65 (s, 1H), 8.13 (s, 2H), 8.00 (s, 1H), 7.61 (d, *J* = 8.7 Hz, 1H), 7.55 (d, *J* = 8.8 Hz, 1H), 4.68 (d, *J* = 9.1 Hz, 1H), 3.33 (s, 3H), 2.84 (s, 3H), 1.04 (m, 1H), 0.71 (m, 2H), 0.35 (m, 2H); ESI-MS 469.93 (m/z) $(MH)^+$;

1-(3-Chloro-4-(2H-1,2,3-triazol-2-yl)phenyl)-3-(7-(cyclopropyl(methoxy)methyl)-2-methylthiazolo[5,4-b]pyridin-6-yl)urea (Compound 144b)

Chiral HPLC RT: 7.44 min
$^1$HNMR (400 MHz, DMSO-$d_6$) δ 10.33 (s, 1H), 9.12 (s, 1H), 8.65 (s, 1H), 8.13 (s, 2H), 8.00 (s, 1H), 7.63 (d, *J* = 8.0 Hz, 1H), 7.56 (d, *J* = 8.0 Hz, 1H), 4.68 (d, *J* = 9.3 Hz, 1H), 3.33 (s, 3H), 2.84 (s, 3H), 1.05 (m, 1H), 0.66 (m, 2H), 0.34 (m, 2H); ESI-MS 469.93 (m/z) $(MH)^+$;

(±)-1-(5-Chloro-6-(2H-1,2,3-triazol-2-yl)pyridin-3-yl)-3-(7 -(1-methoxypropyl)-2-methylthiazolo[5,4-b]pyridin-6-yl)urea (Compound 145)

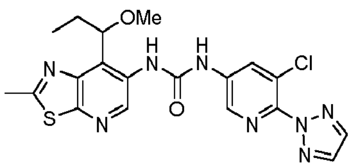

ESI-MS (m/z) 459.18 $(MH)^+$
Chiral separation of racemic compound 145 was carried out using chiral column and afforded the below isomers 145a and 145b:

1-(5-Chloro-6-(2H-1,2,3-triazol-2-yl)pyridin-3-yl)-3-(7-(1-methoxypropyl)-2-methylthiazolo[5,4-b]pyridin-6-yl)urea (Compound 145a)
Chiral HPLC RT: 10.31 min
$^1$HNMR (400 MHz, DMSO-$d_6$) δ 10.53 (s, 1H), 9.11 (d, *J* = 2.5 Hz, 1H), 8.70 (s, 1H), 8.56 (d, *J* = 2.5 Hz, 1H), 8.52 (s, 1H), 8.17 (s, 2H), 5.35-5.26 (m, 1H), 3.33 (s, 3H), 2.85 (s, 3H), 2.08-1.96 (m, 1H), 1.91-1.73 (m, 1H), 0.97-0.88 (m, 3H); ESI-MS (m/z) 459.14 $(MH)^+$;
1-(5-Chloro-6-(2H-1,2,3-triazol-2-yl)pyridin-3-yl)-3-(7-(1-methoxypropyl)-2-methylthiazolo[5,4-b]pyridin-6-yl)urea (Compound 145b)
Chiral HPLC RT: 11.59 min
$^1$HNMR (400 MHz, DMSO-$d_6$) δ 10.53 (s, 1H), 9.11 (d, *J* = 2.5 Hz, 1H), 8.70 (s, 1H), 8.56 (d, *J* = 2.5 Hz, 1H), 8.52 (s, 1H), 8.17 (s, 2H), 5.35-5.26 (m, 1H), 3.33 (s, 3H), 2.85 (s, 3H), 2.08-1.96 (m, 1H), 1.91-1.73 (m, 1H), 0.97-0.88 (m, 3H); ESI-MS (m/z) 459.14 $(MH)^+$;

(±)-1-(6-(2H-1,2,3-Triazol-2-yl)-5-(trifluoromethyl)pyridin-3-yl)-3-(7-(1-methoxypropyl)-2-methylthiazolo[5,4-b]pyridin-6-yl)urea (Compound 146)

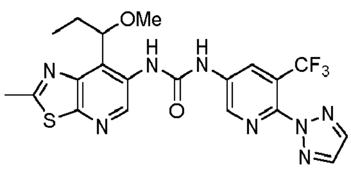

ESI-MS (m/z) 493.36 $(MH)^+$
Chiral separation of racemic compound 146 was carried out using chiral column and afforded the below isomers 146a and 146b:

1-(6-(2H-1,2,3-Triazol-2-yl)-5-(trifluoromethyl)pyridin-3-yl)-3-(7-(1-methoxypropyl)-2-methylthiazolo[5,4-b]pyridin-6-yl)urea (Compound 146a)
Chiral HPLC RT 6.08 min
$^1$HNMR (400 MHz, DMSO-$d_6$) δ 10.69 (s, 1H, $D_2O$ exchangeable), 9.13 (s, 1H), 8.86 (d, *J* = 2.5 Hz, 1H), 8.75 (s, 1H, $D_2O$ exchangeable), 8.73 (d, *J* = 2.5 Hz, 1H), 8.18 (s, 2H), 5.31 (t, *J* = 7.0 Hz, 1H), 3.33 (s, 3H), 2.86 (s, 3H), 2.08-2.02 (m, 1H), 1.87-1.80 (m, 1H), 0.91 (t, *J* = 7.0 Hz, 3H); ESI-MS (m/z) 493.31 $(MH)^+$;

1-(6-(2H-1,2,3-Triazol-2-yl)-5-(trifluoromethyl)pyridin-3-yl)-3-(7-(1-methoxypropyl)-2-methylthiazolo[5,4-b]pyridin-6-yl)urea (Compound 146b)
Chiral HPLC RT 7.10 min
$^1$HNMR (400 MHz, DMSO-$d_6$) δ 10.68 (s, 1H, $D_2O$ exchangeable), 9.13 (s, 1H), 8.86 (d, *J* = 2.5 Hz, 1H), 8.75 (s, 1H, $D_2O$ exchangeable), 8.73 (d, *J* = 2.5 Hz, 1H), 8.18 (s, 2H), 5.31 (t, *J* = 7.0 Hz, 1H), 3.33 (s, 3H), 2.86

(s, 3H), 2.09-2.01 (m, 1H), 1.85-1.76 (m, 1H), 0.91 (t, *J* = 7.0 Hz, 3H); ESI-MS (m/z) 493.31 $(MH)^+$;

(±)-1-(4-(2H-1,2,3-Triazol-2-yl)-3-(trifluoromethyl)phenyl)-3-(7-(1-methoxypropyl)-2-methylthiazolo[5,4-b]pyridin-6-yl)urea (Compound 147)

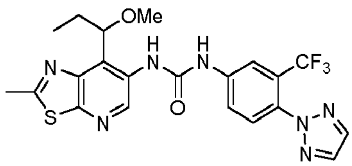

ESI-MS (m/z) 492.18 $(MH)^+$
Chiral separation of racemic compound 147 was carried out using chiral column and afforded the below isomers 147a and 147b:

1-(4-(2H-1,2,3-Triazol-2-yl)-3-(trifluoromethyl)phenyl)-3-(7-(1-methoxypropyl)-2-methylthiazolo[5,4-b]pyridin-6-yl)urea (Compound 147a)
Chiral HPLC RT 6.76 min
$^1$HNMR (400 MHz, DMSO-$d_6$) δ 10.40 (s, 1H, $D_2O$ exchangeable), 9.11 (s, 1H), 8.60 (s, 1H, $D_2O$ exchangeable), 8.19 (d, *J* = 2.5 Hz, 1H), 8.13 (s, 2H), 7.93 (dd, *J* = 8.5, 2.5 Hz, 1H), 7.70 (d, *J* = 8.5 Hz, 1H), 5.33-05.28 (m, 1H), 3.32 (s, 3H), 2.85 (s, 3H), 2.14-1.94 (m, 1H), 1.86-1.75 (m, 1H), 0.91 (t, *J* = 7.5 Hz, 3H); ESI-MS (m/z) 492.06 (MH)+;

1-(4-(2H-1,2,3-Triazol-2-yl)-3-(trifluoromethyl)phenyl)-3-(7-(1-methoxypropyl)-2-methylthiazolo[5,4-b]pyridin-6-yl)urea (Compound 147b)
Chiral HPLC RT 8.07 min
$^1$HNMR (400 MHz, DMSO-$d_6$) δ 10.41 (s, 1H, $D_2O$ exchangeable), 9.11 (s, 1H), 8.60 (s, 1H, $D_2O$ exchangeable), 8.19 (d, *J* = 2.5 Hz, 1H), 8.13 (s, 2H), 7.92 (dd, *J* = 8.5, 2.5 Hz, 1H), 7.70 (d, *J* = 8.5 Hz, 1H), 5.36 - 5.27 (m, 1H), 3.32 (s, 3H), 2.85 (s, 3H), 2.14-1.94 (m, 1H), 1.86-1.75 (m, 1H), 0.91 (t, *J* = 7.5 Hz, 3H); ESI-MS (m/z) 492.06 (MH)+;

(±)-1-(5-Chloro-6-(5-methyloxazol-2-yl)pyridin -3-yl)-3-(7-(1-methoxyethyl)-2-methylthiazolo[5,4-b]pyridin-6-yl)urea (Compound 148)

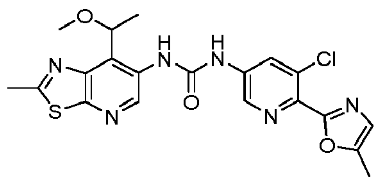

ESI-MS (m/z) 459.29 $(MH)^+$
Chiral separation of racemic compound 148 was carried out by using chiral column and afforded the below isomers 148a and 148b:

1-(5-Chloro-6-(5-methyloxazol-2-yl)pyridin-3-yl)-3-(7-(1-methoxyethyl)-2-methylthiazolo[5,4-b]pyridin-6-yl)urea (Compound 148a)
Chiral HPLC RT: 6.58
$^1$HNMR (400 MHz, DMSO-$d_6$) δ 10.45 (s, 1H), 9.11 (s, 1H), 8.70 (s, 1H), 8.62 (s, 1H), 8.39 (s, 1H), 7.09 (s, 1H), 5.50 (q, *J* = 6.7 Hz, 1H), 3.31 (s, 3H), 2.85 (s, 3H), 2.41 (s, 3H), 1.55 (d, *J* = 6.7 Hz, 3H); ESI-MS (m/z) 459.18 $(MH)^+$;

1-(5-Chloro-6-(5-methyloxazol-2-yl)pyridin-3-yl)-3-(7-(1-methoxyethyl)-2-methylthiazolo[5,4-b]pyridin-6-yl)urea (Compound 148b)
Chiral HPLC RT: 7.23
$^1$HNMR (400 MHz, DMSO-$d_6$) δ 10.48 (s,1H), 9.11 (s, 1H), 8.72 (s, 1H), 8.63 (s, 1H), 8.39 (s, 1H), 7.10 (s, 1H), 5.50 (q, *J* = 6.6 Hz, 1H), 3.31 (s, 3H), 2.85 (s, 3H), 2.41 (s, 3H), 1.55 (d, *J* = 6.7 Hz, 3H); ESI-MS (m/z) 459.3 $(MH)^+$;

(±)-1-(5-Chloro-6-(difluoromethoxy)pyridin-3-yl)-3-(7-(1-methoxyethyl)-2-methylthiazolo[5,4-b]pyridin-6-yl)urea (Compound 149)

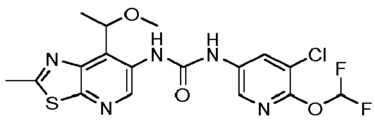

ESI-MS (m/z) 444.1 $(MH)^+$
Chiral separation of racemic compound 149 was carried out using chiral column and afforded the below isomers 149a and 149b:

1-(5-Chloro-6-(difluoromethoxy)pyridin-3-yl)-3-(7-(1-methoxyethyl)-2-methylthiazolo[5,4-b]pyridin-6-yl)urea (Compound 149a)
Chiral HPLC RT 6.03
$^1$HNMR (400 MHz, DMSO-$d_6$) δ 10.14 (s, 1H), 9.10 (s, 1H), 8.61 (s, 1H), 8.35 (d, *J* = 2.1 Hz, 1H), 8.23 (d, *J* = 2.1 Hz, 1H),7.68 (t, *J* = 72.0 Hz, 1H), 5.49 (q, *J* = 6.8 Hz, 1H), 3.30 (s, 3H), 2.85 (s, 3H), 1.54 (d, *J* = 6.7 Hz, 3H); ESI-MS (m/z) 444.1 $(MH)^+$;

1-(5-Chloro-6-(difluoromethoxy)pyridin-3-yl)-3-(7-(1-methoxyethyl)-2-methylthiazolo[5,4-b]pyridin-6-yl)urea (Compound 149b)
Chiral HPLC RT 6.67
$^1$HNMR (400 MHz, DMSO-$d_6$) δ 10.14 (s, 1H), 9.10 (s, 1H), 8.61 (s, 1H), 8.35 (d, *J* = 2.1 Hz, 1H), 8.23 (d, *J* = 2.1 Hz, 1H),7.68 (t, *J* = 72.0 Hz, 1H), 5.49 (q, *J* = 6.8 Hz, 1H), 3.30 (s, 3H), 2.85 (s, 3H), 1.54 (d, *J* = 6.7 Hz, 3H); ESI-MS (m/z) 444.3 $(MH)^+$;

(±)-1-(5-Chloro-6-methoxypyridin-3-yl)-3-(7-(cyclopropyl(methoxy)methyl)-2-methylthiazolo[5,4-b]pyridin-6-yl)urea (Compound 150)

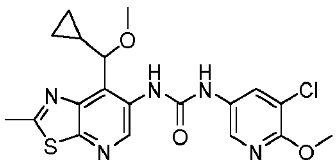

ESI-MS (m/z) 434.17 $(MH)^+$
Chiral separation of racemic compound 150 was carried out using chiral column and afforded the below isomers 150a and 150b:

1-(5-Chloro-6-methoxypyridin-3-yl)-3-(7-(cyclopropyl(methoxy)methyl)-2-methylthiazolo[5,4-b]pyridin-6-yl)urea (Compound 150a)
Chiral HPLC RT 5.06
$^1$HNMR (400 MHz, DMSO-$d_6$) δ 9.95 (s, 1H), 9.12 (s, 1H), 8.57 (s, 1H), 8.19 (s, 1H), 8.15 (s, 1H), 4.66 (d, *J* = 9.0 Hz, 1H), 3.92 (s, 3H), 3.31 (s, 3H), 2.83 (s, 3H), 1.42-1.40 (m, 1H), 0.71-0.63 (m, 2H), 0.36-0.32 (m, 2H); ESI-MS (m/z) 434.15 $(MH)^+$;

1-(5-Chloro-6-methoxypyridin-3-yl)-3-(7-(cyclopropyl(methoxy)methyl)-2-methylthiazolo[5,4-b]pyridin-6-yl)urea (Compound 150b)
Chiral HPLC RT 5.88
$^1$HNMR (400 MHz, DMSO-$d_6$) δ 9.95 (s, 1H), 9.12 (s, 1H), 8.57 (s, 1H), 8.18 (s, 1H), 8.14 (s, 1H),4.66 (d, *J* = 9.1 Hz, 1H), 3.92 (s, 3H), 3.31 (d, *J* = 2.2 Hz, 3H), 2.83 (s, 3H), 1.39-1.41 (m, 1H), 0.66-0.69 (m, 2H), 0.32-0.33 (m, 2H); ESI-MS (m/z) 434.16 (MH)+;

(±)-1-(6-(2H -1,2,3-Triazol-2-yl)-5-(trifluoromethyl)pyridin-3-yl)-3-(7-(cyclopropyl(methoxy)methyl)-2-methylthiazolo[5,4-b]pyridin-6-yl)urea (Compound 151)

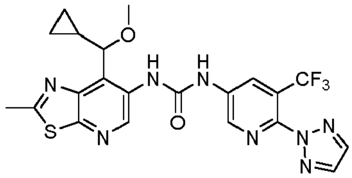

ESI-MS (m/z) 505.16 (MH)$^+$

Chiral separation of racemic compound 151 was carried out using chiral column and afforded the below isomers 151a and 151b:

1-(6-(2H-1,2,3-Triazol-2-yl)-5-(trifluoromethyl)pyridin-3-yl)-3-(7-(cyclopropyl(methoxy)methyl)-2-methylthiazolo[5,4-b]pyridin-6-yl)urea (Compound 151a)

Chiral HPLC RT 7.64

$^1$HNMR (400 MHz, DMSO-$d_6$) δ 10.77 (s, 1H), 9.16 (s, 1H), 8.87 (s, 1H), 8.83 (s, 1H), 8.74 (s, 1H), 8.19 (s, 2H), 4.69 (d, *J* = 9.1 Hz, 1H), 3.34 (s, 3H), 2.85 (s, 3H), 1.48-1.42 (m, 1H), 0.73-0.65 (m, 2H), 0.39-0.33 (m, 2H); ESI-MS (m/z) 505.44 (MH)$^+$;

1-(6-(2H-1,2,3-Triazol-2-yl)-5-(trifluoromethyl)pyridin-3-yl)-3-(7-(cyclopropyl(methoxy)methyl)-2-methylthiazolo[5,4-b]pyridin-6-yl)urea (Compound 151b)

Chiral HPLC RT 9.64

$^1$HNMR (400 MHz, DMSO-$d_6$) δ 10.84 (s, 1H), 9.15 (s, 1H), 8.87 (s, 2H), 8.75 (s, 1H), 8.18 (s, 2H), 4.68 (d, *J* = 9.2 Hz, 1H), 3.33 (s, 3H), 2.85 (s, 3H), 1.48-1.43 (m, 1H), 0.69 (m, 2H), 0.39-0.33 (m, 2H); ESI-MS (m/z) 505.07 (MH)$^+$;

(±)-Methyl 3-chloro-5-(3-(7-(1-methoxyethyl)-2-methylthiazolo[5,4-b]pyridin-6-yl)ureido)benzoate (Compound 152)

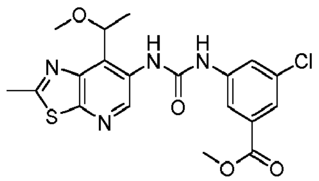

ESI-MS (m/z) 435.2 (MH)$^+$

Chiral separation of racemic compound 152 was carried out using chiral column and afforded the below isomers 152a and 152b:

Methyl 3-chloro-5-(3-(7-(1-methoxyethyl)-2-methylthiazolo[5,4-b]pyridin-6-yl)ureido)benzoate (Compound 152a)

Chiral HPLC RT 8.10

$^1$HNMR (400 MHz, DMSO-$d_6$) δ 10.24 (s, 1H), 9.11 (s, 1H), 8.58 (s, 1H), 8.02 (s, 1H), 7.99 (s, 1H), 7.54 (s, 1H), 5.50 (q, *J* = 6.7 Hz, 1H), 3.88 (s, 3H), 3.34 (s, 3H), 2.85 (s, 3H), 1.54 (d, *J* = 6.7 Hz, 3H); ESI-MS (m/z) 435.1 (MH)$^+$;

Methyl 3-chloro-5-(3-(7-(1-methoxyethyl)-2-methylthiazolo[5,4-b]pyridin-6-yl)ureido)benzoate (Compound 152b)

Chiral HPLC RT 9.88

$^1$HNMR (400 MHz, DMSO-$d_6$) δ 10.26 (s, 1H), 9.11 (s, 1H), 8.59 (s, 1H), 8.02 (s, 1H), 8.00 (s, 1H), 7.54 (s, 1H), 5.50 (q, *J* = 6.7 Hz, 1H), 3.89 (s, 3H), 3.30 (s, 3H), 2.85 (s, 3H), 1.54 (d, *J* = 6.7 Hz, 3H); ESI-MS (m/z) 435.0 (MH)$^+$;

(±)-1-(4-(2H -1,2,3-Triazol-2-yl)-3-(trifluoromethyl)phenyl)-3-(7-(cyclopropyl(methoxy)methyl)-2-methylthiazolo[5,4-b]pyridin-6-yl)urea (Compound 153)

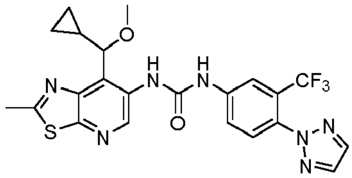

ESI-MS (m/z) 504.31 $(MH)^+$

Chiral separation of racemic compound 153 was carried out using chiral column and afforded the below isomers 153a and 153b:

1-(4-(2H-1,2,3-Triazol-2-yl)-3-(trifluoromethyl)phenyl)-3-(7-(cyclopropyl(methoxy)methyl)-2-methylthiazolo[5,4-b]pyridin-6-yl)urea (Compound 153a)
Chiral HPLC RT 8.47
$^1$HNMR (400 MHz, DMSO-$d_6$) δ 10.48 (s, 1H), 9.15 (s, 1H), 8.69 (s, 1H), 8.20 (d, *J* = 2.4 Hz, 1H), 8.13 (s, 2H), 7.94 (dd, *J* = 2.4, 8.8 Hz, 1H), 7.70 (d, *J* = 8.8 Hz, 1H), 4.69 (d, *J* = 9.1 Hz, 1H), 3.34 (s, 3H), 2.84 (s, 3H), 1.46-1.42 (m, 1H), 0.72-0.65 (m, 2H), 0.38-0.33 (m, 2H); ESI-MS (m/z) 504.31 $(MH)^+$;

1-(4-(2H -1,2,3-Triazol-2-yl)-3-(trifluoromethyl)phenyl)-3-(7-(cyclopropyl(methoxy)methyl)-2-methylthiazolo[5,4-b]pyridin-6-yl)urea (Compound 153b)
Chiral HPLC RT 9.59
$^1$HNMR (400 MHz, DMSO-$d_6$) δ 10.48 (s, 1H), 9.15 (s, 1H), 8.69 (s, 1H), 8.20 (d, *J* = 2.4 Hz, 1H), 8.13 (s, 2H), 7.94 (dd, *J* = 2.4, 8.7 Hz, 1H), 7.70 (d, *J* = 8.7 Hz, 1H), 4.69 (d, *J* = 9.1 Hz, 1H), 3.34 (s, 3H), 2.84 (s, 3H),1.46-1.43 (m, 1H), 0.71-0.63 (m, 2H), 0.38-0.32 (m, 2H); ESI-MS (m/z) 504.31 $(MH)^+$;

(±)-1-(5-Chloro-6-(2H-1,2,3-triazol-2-yl)pyridin-3-yl)-3-(7-(cyclopropyl(methoxy)methyl)-2-methylthiazolo[5,4-b]pyridin-6-yl)urea (Compound 154)

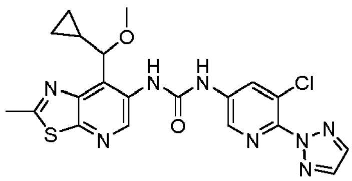

ESI-MS (m/z) 471.17 $(MH)^+$

Chiral separation of racemic compound 154 was carried out using chiral column and afforded the below isomers 154a and 154b:

1-(5-Chloro-6-(2H-1,2,3-triazol-2-yl)pyridin-3-yl)-3-(7-(cyclopropyl(methoxy)methyl)-2-methylthiazolo[5,4-b]pyridin-6-yl)urea (Compound 154a)
Chiral HPLC RT 6.38 $^1$HNMR (400 MHz, DMSO-$d_6$) δ 10.61 (s, 1H), 9.14 (s, 1H), 8.78 (s, 1H), 8.56 (d, *J* = 2.4 Hz, 1H), 8.52 (d, *J* = 2.4 Hz, 1H), 8.17 (s, 2H), 4.68 (d, *J* = 9.2 Hz, 1H), 3.33 (s, 3H), 2.84 (s, 3H), 1.46-1.41 (m, 1H), 0.71-0.65 (m, 2H), 0.37-0.32 (m, 2H); ESI-MS (m/z) 471.16 $(MH)^+$;

1-(5-Chloro-6-(2H-1,2,3-triazol-2-yl)pyridin-3-yl)-3-(7-(cyclopropyl(methoxy)methyl)-2-methylthiazolo[5,4-b]pyridin-6-yl)urea (Compound 154b)
Chiral HPLC RT 8.17
$^1$HNMR (400 MHz, DMSO-$d_6$) δ 10.61 (s, 1H), 9.14 (s, 1H), 8.79 (s, 1H), 8.56 (d, *J* = 2.4 Hz, 1H), 8.53 (d, *J* = 2.4 Hz, 1H), 8.17 (s, 2H), 4.68 (d, *J* = 9.1 Hz, 1H), 3.34 (s, 3H), 2.84 (s, 3H), 1.47-1.42 (m, 1H), 0.72-0.65 (m, 2H), 0.39 - 0.33 (m, 2H); ESI-MS (m/z) 471.31 $(MH)^+$;

(±)-1-(7-(Cyclopropyl(methoxy)methyl)-2-methylthiazolo[5,4-b]pyridin-6-yl)- 3-(2-methoxy-6-(2H-1,2,3-triazol-2-yl)-5-(trifluoromethyl)pyridin-3-yl)urea (Compound 155)

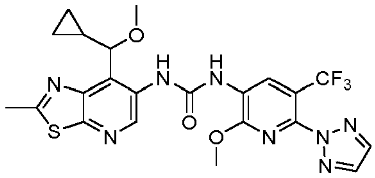

ESI-MS (m/z) 535.32 $(MH)^+$

Chiral separation of racemic compound 155 was carried out using chiral column and afforded the below isomers 155a and 155b:

1-(7-(Cyclopropyl(methoxy)methyl)-2-methylthiazolo[5,4-b]pyridin-6-yl)-3-(2-methoxy-6-(2H-1,2,3-triazol-2-yl)-5-(trifluoromethyl)pyridin-3-yl)urea (Compound 155a)
Chiral HPLC RT 4.88
$^1$HNMR (400 MHz, DMSO-$d_6$) δ 9.98 (s, 1H), 9.04 (s, 1H), 9.01 (s, 1H), 8.87 (s, 1H), 8.16 (s, 2H), 4.58 (d, *J* = 9.2 Hz, 1H), 4.09 (s, 3H), 3.26 (s, 3H), 2.84 (s, 3H), 1.50-1.46 (m, 1H), 0.71-0.62 (m, 2H), 0.37-0.26 (m, 2H); ESI-MS (m/z) 535.32 $(MH)^+$;
1-(7-(Cyclopropyl(methoxy)methyl)-2-methylthiazolo[5,4-b]pyridin-6-yl)- 3-(2-methoxy-6-(2H-1,2,3-triazol-2-yl)-5-(trifluoromethyl)pyridin-3-yl)urea (Compound 155b)
Chiral HPLC RT 6.03
$^1$HNMR (400 MHz, DMSO-$d_6$) δ 9.98 (s, 1H), 9.04 (s, 1H), 9.01 (s, 1H), 8.88 (s, 1H), 8.16 (s, 2H), 4.58 (d, *J* = 9.3 Hz, 1H), 4.09 (s, 3H), 3.26 (s, 3H), 2.84 (s, 3H), 1.50-1.45 (m, 1H), 0.72-0.62 (m, 2H), 0.36-0.27 (m, 2H); ESI-MS (m/z) 535.32 $(MH)^+$;

(±)-1-(5-Chloro-2-methoxy-6-(2H-1,2,3-triazol-2-yl)pyridin-3-yl)-3-(7-(cyclopropyl(methoxy)methyl)-2-methylthiazolo[5,4-b]pyridin-6-yl)urea (Compound 156)

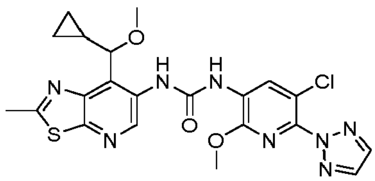

ESI-MS (m/z) 501.31 $(MH)^+$

Chiral separation of racemic compound 156 was carried out using chiral column and afforded the below isomers 156a and 156b:

1-(5-Chloro-2-methoxy-6-(2H-1,2,3-triazol-2-yl)pyridin-3-yl)-3-(7-(cyclopropyl(methoxy)methyl)-2-methylthiazolo[5,4-b]pyridin-6-yl)urea (Compound 156a)
Chiral HPLC RT 5.56
$^1$HNMR (400 MHz, DMSO-$d_6$) δ 9.87 (s, 1H), 9.01 (s, 1H), 8.87 (s, 1H), 8.75 (s, 1H), 8.15 (s, 2H), 4.57 (d, *J* = 9.2 Hz, 1H), 4.02 (s, 3H), 3.26 (s, 3H), 2.84 (s, 3H), 1.49-1.46 (m, 1H), 0.67-0.61 (m, 2H), 0.32-0.28 (m, 2H); ESI-MS (m/z) 501.4 $(MH)^+$;

1-(5-Chloro-2-methoxy-6-(2H-1,2,3-triazol-2-yl)pyridin-3-yl)-3-(7-(cyclopropyl(methoxy)methyl)-2-methylthiazolo[5,4-b]pyridin-6-yl)urea (Compound 156b)
Chiral HPLC RT 6.95 $^1$HNMR (400 MHz, DMSO-$d_6$) δ 9.86 (s, 1H), 9.01 (s, 1H), 8.87 (s, 1H), 8.75 (s, 1H), 8.15 (s, 2H), 4.57 (d, *J* = 9.3 Hz,1H), 4.02 (s, 3H), 3.26 (s, 3H), 2.84 (s, 3H), 1.50-1.45 (m,1H), 0.72-0.62 (m, 2H), 0.39-0.25 (m, 2H); ESI-MS (m/z) 501.31 $(MH)^+$;

(±)-1-(7-(*sec*-Butyl)-2-methylthiazolo[5,4-b]pyridin-6-yl)-3-(5-chloro-6-(2H-1,2,3-triazol-2-yl)pyridin-3-yl)urea (Compound 157)

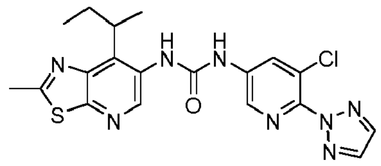

ESI-MS (m/z) 443.2 $(MH)^+$
Chiral separation of racemic compound 157 was carried out using chiral column and afforded the below isomers 157a and 157b:

1-(7-(sec-Butyl)-2-methylthiazolo[5,4-b]pyridin-6-yl)-3-(5-chloro-6-(2H-1,2,3-triazol-2-yl)pyridin-3-yl)urea (Compound 157a)
Chiral HPLC RT 7.30
$^1$HNMR (400 MHz, DMSO-$d_6$) $\delta$ 9.67 (s, 1H), 8.66 (s, 1H), 8.57 (d, $J$ = 2.3 Hz, 1H), 8.56 (s, 1H), 8.48 (d, $J$ = 2.3 Hz, 1H), 8.16 (s, 2H), 2.85 (s, 3H), 2.50-2.56 (m, 1H), 2.15-1.99 (m, 1H), 1.97-1.90 (m, 1H), 1.47 (d, $J$ = 7.0 Hz, 3H), 0.76 (t, $J$ = 7.4 Hz, 3H); ESI-MS (m/z) 443.17 $(MH)^+$;

1-(7-(sec-Butyl)-2-methylthiazolo[5,4-b]pyridin-6-yl)-3-(5-chloro-6-(2H-l,2,3-triazol-2-yl)pyridin-3-yl)urea (Compound 157b)
Chiral HPLC RT 8.54
$^1$HNMR (400 MHz, DMSO-$d_6$) $\delta$ 9.68 (s, 1H), 8.67 (s, 1H), 8.57 (d, $J$ = 2.3 Hz, 1H), 8.56 (s, 1H), 8.48 (d, $J$ = 2.3 Hz, 1H), 8.16 (s, 2H), 2.85 (s, 3H), 2.50-2.56 (m, 1H), 2.15-1.99 (m, 1H), 1.97-1.90 (m, 1H), 1.47 (d, $J$ = 7.0 Hz, 3H), 0.76 (t, $J$ = 7.4 Hz, 3H); ESI-MS (m/z) 443.17 $(MH)^+$;

(±)-1-(3-Chloro-4-(2H-1,2,3-triazol-2-yl)phenyl)-3-(7-(1-methoxyethyl)-2-methylthiazolo[5,4-b]pyridin-6-yl)urea (Compound 158)

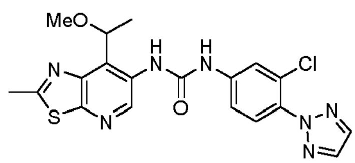

$^1$HNMR (400 MHz, DMSO-$d_6$) $\delta$ 10.25 (s, 1H), 9.10 (s, 1H), 8.59 (s, 1H), 8.12 (s, 2H), 7.99 (d, $J$ = 2.0 *Hz,* 1H), 7.64-7.58 (m, 1H), 7.57-7.52 (m, 1H), 5.53-5.45 (m, 1H), 3.34 (s, 3H), 2.85 (s, 3H), 1.54 (d, $J$ = 6.5 *Hz*, 3H); ESI-MS (m/z) 444.11 $(MH)^+$;
Chiral separation of racemic compound 158 was carried out using chiral column and afforded the below isomers 158a and 158b:

1-(3-Chloro-4-(2H-1,2,3-triazol-2-yl)phenyl)-3-(7-(1-methoxyethyl)-2-methylthiazolo[5,4-b]pyridin-6-yl)urea (Compound 158a)
Chiral HPLC RT: 6.72 min
1H NMR (400 MHz, DMSO-$d_6$) $\delta$ 10.25 (s, 1H), 9.10 (s, 1H), 8.59 (s, 1H), 8.12 (s, 2H), 7.99 (d, $J$ = 2.0 *Hz,* 1H), 7.64 -7.58 (m, 1H), 7.57-7.52 (m, 1H), 5.53 - 5.45 (m, 1H), 3.34 (s, 3H), 2.85 (s, 3H), 1.54 (d, $J$ = 6.5Hz, 3H); ESI-MS (m/z) 444.11 $(MH)^+$;

1-(3-Chloro-4-(2H-1,2,3-triazol-2-yl)phenyl)-3-(7-(1-methoxyethyl)-2-methylthiazolo[5,4-b]pyridin-6-yl)urea (Compound 158b)
Chiral HPLC RT: 8.21 min
$^1$HNMR (400 MHz, DMSO-$d_6$) $\delta$ 10.26 (s, 1H), 9.11 (s, 1H), 8.59 (s, 1H), 8.12 (s, 2H), 7.99 (d, $J$ = 2.0 *Hz,* 1H), 7.64 -7.58 (m, 1H), 7.57-7.52 (m, 1H), 5.53 - 5.45 (m, 1H), 3.34 (s, 3H), 2.85 (s, 3H), 1.54 (d, $J$ = 6.5Hz, 3H); ESI-MS (m/z) 444.12 $(MH)^+$;

(±)-1-(5-Chloro-6-methoxypyridin-3-yl)-3-(7-(1-methoxyethyl)-2-methylthiazolo[5,4-b]pyridin-6-yl)urea (Compound 159)

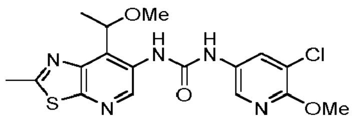

$^1$HNMR (400 MHz, DMSO-$d_6$) δ 9.89 (s, 1H), 9.11 (d, *J* = 2.5 *Hz,* 1H), 8.52 (s, 1H), 8.21-8.11 (m, 2H), 5.55-5.43 (m, 1H), 3.92 (s, 3H), 3.29 (s, 3H), 2.84 (s, 3H), 1.60-1.46 (m, 3H); ESI-MS (m/z) 408.08 $(MH)^+$;
Chiral separation of racemic compound 159 was carried out using chiral column and afforded the below isomers 159a and 159b:

1-(5-Chloro-6-methoxypyridin-3-yl)-3-(7-(1-methoxyethyl)-2-methylthiazolo[5,4-b]pyridin-6-yl)urea (Compound 159a)
Chiral HPLC RT: 7.32 min
$^1$HNMR (400 MHz, DMSO-$d_6$) δ 9.89 (s, 1H), 9.11 (d, *J* = 2.5 *Hz,* 1H), 8.52 (s, 1H), 8.21-8.11 (m, 2H), 5.55-5.43 (m, 1H), 3.92 (s, 3H), 3.29 (s, 3H), 2.84 (s, 3H), 1.60-1.46 (m, 3H); ESI-MS (m/z) 408.09 $(MH)^+$;

1-(5-Chloro-6-methoxypyridin-3-yl)-3-(7-(1-methoxyethyl)-2-methylthiazolo[5,4-b]pyridin-6-yl)urea (Compound 159b)
Chiral HPLC RT: 8.76 min
$^1$HNMR (400 MHz, DMSO-$d_6$) δ 9.89 (s, 1H), 9.11 (d, *J* = 2.5 *Hz,* 1H), 8.52 (s, 1H), 8.21-8.11 (m, 2H), 5.55-5.43 (m, 1H), 3.92 (s, 3H), 3.29 (s, 3H), 2.84 (s, 3H), 1.60-1.46 (m, 3H); ESI-MS (m/z) 408.09 $(MH)^+$;

(±)-1-(5-Chloro-6-(2*H*-1,2,3-triazol-2-yl)pyridin-3-yl)-3-(7-(1-methoxyethyl)-2-methylthiazolo[5,4-b]pyridin-6-yl)urea (Compound 160)

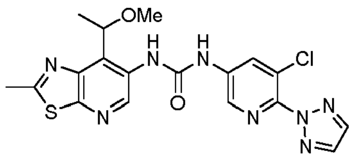

$^1$HNMR (400 MHz, DMSO-$d_6$) δ 10.54 (s, 1H), 9.12 (s, 1H), 8.74 (s, 1H), 8.56 (d, *J* = 2.5 *Hz,* 1H), 8.52 (d, *J* = 2.5 *Hz,* 1H), 8.17 (s, 2H), 5.51 (q, *J* = 6.5 *Hz,* 1H), 3.32 (s, 3H), 2.86 (s, 3H), 1.56 (d, *J* = 6.5 *Hz,* 3H); ESI-MS (m/z) 444.98 $(MH)^+$;
Chiral separation of racemic compound160 was carried out using chiral column and afforded the below isomers 160a and 160b:

1-(5-Chloro-6-(2*H*-1,2,3-triazol-2-yl)pyridin-3-yl)-3-(7-(1-methoxyethyl)-2-methylthiazolo[5,4-b]pyridin-6-yl)urea (Compound 160a)
Chiral HPLC RT: 8.16 min
$^1$HNMR (400 MHz, DMSO-$d_6$) δ 10.54 (s, 1H), 9.12 (s, 1H), 8.74 (s, 1H), 8.56 (d, *J* = 2.5 *Hz,* 1H), 8.52 (d, *J* = 2.5 *Hz,* 1H), 8.17 (s, 2H), 5.51 (q, *J* = 6.5 *Hz,* 1H), 3.32 (s, 3H), 2.86 (s, 3H), 1.56 (d, *J* = 6.5 *Hz,* 3H); ESI-MS (m/z) 444.99 $(MH)^+$;

1-(5-Chloro-6-(2*H*-1,2,3-triazol-2-yl)pyridin-3-yl)-3-(7-(1-methoxyethyl)-2-methylthiazolo[5,4-b]pyridin-6-yl)urea (Compound 160b)
Chiral HPLC RT: 9.03 min
$^1$HNMR (400 MHz, DMSO-$d_6$) δ 10.54 (s, 1H), 9.12 (s, 1H), 8.74 (s, 1H), 8.56 (d, *J* = 2.5 *Hz,* 1H), 8.52 (d, *J* = 2.5 *Hz,* 1H), 8.17 (s, 2H), 5.51 (q, *J* = 6.5 *Hz,* 1H), 3.32 (s, 3H), 2.86 (s, 3H), 1.56 (d, *J* = 6.5 *Hz,* 3H); ESI-MS (m/z) 444.98 $(MH)^+$;

(±)-1-(3-Chloro-4-(2H-1,2,3-triazol-2-yl)phenyl)-3-(7-(1-ethoxyethyl)-2-methylthiazolo[5,4-b]pyridin-6-yl)urea (Compound 161)

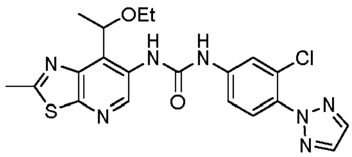

$^1$HNMR (400 MHz, DMSO-$d_6$) δ 10.24 (s, 1H), 9.10 (s, 1H), 8.56 (s, 1H), 8.12 (s, 2H), 7.99 (d, *J* = 2.0 *Hz,* 1H), 7.68-7.51 (m, 2H), 5.61 (q, *J* = 6.5 *Hz,* 1H), 3.58-3.35 (m, 2H), 2.85 (s, 3H), 1.56 (d, *J* = 6.5 *Hz,* 3H), 1.18 (t, *J* = 7.0 *Hz,* 3H); ESI-MS (m/z) 458.06 $(MH)^+$;

Chiral separation of racemic compound 161 was carried out using chiral column and afforded the below isomers 161a and 161b:

1-(3-Chloro-4-(2H-1,2,3-triazol-2-yl)phenyl)-3-(7-(1-ethoxyethyl)-2-methylthiazolo[5,4-b]pyridin-6-yl)urea (Compound 161a)
Chiral HPLC RT: 6.98 min
$^1$HNMR (400 MHz, DMSO-$d_6$) δ 10.24 (s, 1H), 9.10 (s, 1H), 8.56 (s, 1H), 8.12 (s, 2H), 7.99 (d, *J* = 2.0 *Hz,* 1H), 7.68-7.51 (m, 2H), 5.61 (q, *J* = 6.5 *Hz,* 1H), 3.58-3.35 (m, 2H), 2.85 (s, 3H), 1.56 (d, *J* = 6.5 *Hz,* 3H), 1.18 (t, *J* = 7.0 *Hz,* 3H); ESI-MS (m/z) 458.06 $(MH)^+$;

1-(3-Chloro-4-(2H-1,2,3-triazol-2-yl)phenyl)-3-(7-(1-ethoxyethyl)-2-methylthiazolo[5,4-b]pyridin-6-yl)urea (Compound 161b)
Chiral HPLC RT: 8.17 min
$^1$HNMR (400 MHz, DMSO-$d_6$) δ 10.24 (s, 1H), 9.10 (s, 1H), 8.56 (s, 1H), 8.12 (s, 2H), 7.99 (d, *J* = 2.0 *Hz,* 1H), 7.68-7.51 (m, 2H), 5.61 (q, *J* = 6.5 *Hz,* 1H), 3.58-3.35 (m, 2H), 2.85 (s, 3H), 1.56 (d, *J* = 6.5 *Hz,* 3H), 1.18 (t, *J* = 7.0 *Hz,* 3H); ESI-MS (m/z) 458.08 $(MH)^+$;

(±)-1-(5-Chloro-6-methoxypyridin-3-yl)-3-(7-(1-ethoxyethyl)-2-methylthiazolo[5,4-b]pyridin-6-yl)urea (Compound 162)

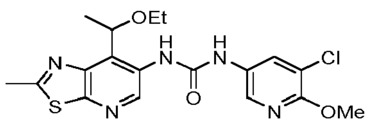

$^1$HNMR (400 MHz, DMSO-$d_6$) δ 9.87 (s, 1H), 9.11 (s, 1H), 8.49 (s, 1H), 8.20-8.13 (m, 2H), 5.60 (q, *J* = 6.5 *Hz,* 1H), 3.92 (s, 3H), 3.52-3.36 (m, 2H), 2.84 (s, 3H), 1.54 (d, *J* = 6.5 *Hz,* 3H), 1.16 (t, *J* = 7.0 *Hz,* 3H); ESI-MS (m/z) 422.01 $(MH)^+$;

Chiral separation of racemic compound 162 was carried out using chiral column and afforded the below isomers 162a and 162b:

1-(5-Chloro-6-methoxypyridin -3-yl)-3-(7-(1-ethoxyethyl)-2-methylthiazolo[5,4-b]pyridin-6-yl)urea (Compound 162a)
Chiral HPLC RT: 7.03 min
$^1$HNMR (400 MHz, DMSO-$d_6$) δ 9.87 (s, 1H), 9.11 (s, 1H), 8.49 (s, 1H), 8.20-8.13 (m, 2H), 5.60 (q, *J* = 6.5 *Hz,* 1H), 3.92 (s, 3H), 3.52-3.36 (m, 2H), 2.84 (s, 3H), 1.54 (d, *J* = 6.5 *Hz,* 3H), 1.16 (t, *J* = 7.0 *Hz,* 3H); ESI-MS (m/z) 422.01 $(MH)^+$;

1-(5-Chloro-6-methoxypyridin-3-yl)-3-(7-(1-ethoxyethyl)-2-methylthiazolo[5,4-b]pyridin-6-yl)urea (Compound 162b)
Chiral HPLC RT: 8.27 min
$^1$HNMR (400 MHz, DMSO-$d_6$) δ 9.87 (s, 1H), 9.11 (s, 1H), 8.49 (s, 1H), 8.20-8.13 (m, 2H), 5.60 (q, *J* = 6.5 *Hz,* 1H), 3.92 (s, 3H), 3.52-3.36 (m, 2H), 2.84 (s, 3H), 1.54 (d, *J* = 6.5 *Hz,* 3H), 1.16 (t, *J* = 7.0 *Hz,* 3H); ESI-MS (m/z) 422.01 $(MH)^+$;

(±)-1-(3-Chloro-4-methoxyphenyl)-3-(7-(1-ethoxyethyl)-2-methylthiazolo[5,4-b]pyridin-6-yl)urea (Compound 163)

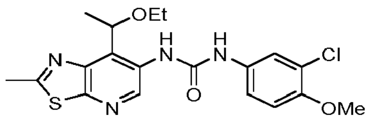

$^1$HNMR (400 MHz, DMSO-$d_6$) δ 9.71 (s, 1H), 9.10 (s, 1H), 8.39 (s, 1H), 7.71 (s, 1H), 7.33 (m, 1H), 7.16-7.07 (m, 1H), 5.60 (q, *J* = 7.5 *Hz,* 1H), 3.82 (s, 3H), 3.55-3.44 (m, 1H), 3.34-3.31 (m, 1H), 2.84 (s, 3H), 1.53 (d, *J* = 7.5 *Hz,* 3H), 1.21-1.09 (m, 3H); ESI-MS (m/z) 421.02 $(MH)^+$;
Chiral separation of racemic compound 163 was carried out using chiral column and afforded the below isomers 163a and 163b:

1-(3-Chloro-4-methoxyphenyl)-3-(7-(1-ethoxyethyl)-2-methylthiazolo[5,4-b]pyridin-6-yl)urea (Compound 163a)
Chiral HPLC RT: 6.88 min
$^1$HNMR (400 MHz, DMSO-$d_6$) δ 9.71 (s, 1H), 9.10 (s, 1H), 8.39 (s, 1H), 7.71 (s, 1H), 7.33 (m, 1H), 7.16-7.07 (m, 1H), 5.60 (q, *J* = 7.5 *Hz,* 1H), 3.82 (s, 3H), 3.55-3.44 (m, 1H), 3.34-3.31 (m, 1H), 2.84 (s, 3H), 1.53 (d, *J* = 7.5 *Hz,* 3H), 1.21-1.09 (m, 3H); ESI-MS (m/z) 421.03 $(MH)^+$;

1-(3-Chloro-4-methoxyphenyl)-3-(7-(1-ethoxyethyl)-2-methylthiazolo[5,4-b]pyridin-6-yl)urea (Compound 163b)
Chiral HPLC RT: 8.13 min
$^1$HNMR (400 MHz, DMSO-$d_6$) δ 9.71 (s, 1H), 9.10 (s, 1H), 8.39 (s, 1H), 7.71 (s, 1H), 7.33 (m, 1H), 7.16-7.07 (m, 1H), 5.60 (q, *J* = 7.5 *Hz,* 1H), 3.82 (s, 3H), 3.55-3.44 (m, 1H), 3.34-3.31 (m, 1H), 2.84 (s, 3H), 1.53 (d, *J* = 7.5 *Hz,* 3H), 1.21-1.09 (m, 3H); ESI-MS (m/z) 421.05 $(MH)^+$;

(±)-1-(5-Chloro-6-(2H-1,2,3-triazol-2-yl)pyridin-3-yl)-3-(7-(1-ethoxyethyl)-2-methylthiazolo[5,4-b]pyridin-6-yl)urea (Compound 164)

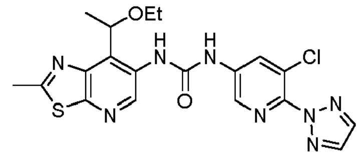

$^1$HNMR (400 MHz, DMSO-$d_6$) δ 10.54 (s, 1H), 9.13 (s, 1H), 8.71 (s, 1H), 8.58 (d, *J* = 2.5 *Hz,* 1H), 8.52 (d, *J* = 2.5 *Hz,* 1H), 8.17 (s, 2H), 5.61 (q, *J* = 6.5 *Hz,* 1H), 3.57-3.34 (m, 2H), 2.85 (s, 3H), 1.57 (d, *J* = 6.5 *Hz,* 3H), 1.19 (t, *J* = 7.0 Hz, 3H); ESI-MS (m/z) 458.98 (MH)+;
Chiral separation of racemic compound 164 was carried out using chiral column and afforded the below isomers 164a and 164b:

1-(5-chloro-6-(2H-1,2,3-triazol-2-yl)pyridin-3-yl)-3-(7-(1-ethoxyethyl)-2-methylthiazolo[5,4-b]pyridin-6-yl)urea (Compound 164a)
Chiral HPLC RT: 8.16 min
$^1$HNMR (400 MHz, DMSO-$d_6$) δ 10.54 (s, 1H), 9.13 (s, 1H), 8.71 (s, 1H), 8.58 (d, *J* = 2.5 *Hz,* 1H), 8.52 (d, *J* = 2.5 *Hz,* 1H), 8.17 (s, 2H), 5.61 (q, *J* = 6.5 *Hz,* 1H), 3.57-3.34 (m, 2H), 2.85 (s, 3H), 1.57 (d, *J* = 6.5 *Hz,* 3H), 1.19 (t, *J* = 7.0 Hz, 3H); ESI-MS (m/z) 458.99 (MH)+;

1-(5-chloro-6-(2H-1,2,3-triazol-2-yl)pyridin-3-yl)-3-(7-(1-ethoxyethyl)-2-methylthiazolo[5,4-b]pyridin-6-yl)urea (Compound 164b)
Chiral HPLC RT: 9.48 min
$^1$HNMR (400 MHz, DMSO-$d_6$) δ 10.54 (s, 1H), 9.13 (s, 1H), 8.71 (s, 1H), 8.58 (d, *J* = 2.5 *Hz,* 1H), 8.52 (d, *J* = 2.5 *Hz,* 1H), 8.17 (s, 2H), 5.61 (q, *J* = 6.5 *Hz,* 1H), 3.57-3.34 (m, 2H), 2.85 (s, 3H), 1.57 (d, *J* = 6.5 *Hz,* 3H), 1.19 (t, *J* = 7.0 Hz, 3H); ESI-MS (m/z) 458.99 (MH)+;

(±)-1-(6-(2H-1,2,3-Triazol-2-yl)-5-(trifluoromethyl)pyridin-3-yl)-3-(7-(1-ethoxyethyl)-2-methylthiazolo[5,4-b]pyridin-6-yl)urea (Compound 165)

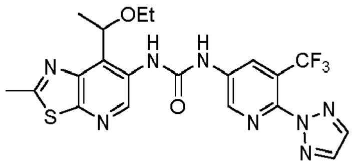

ESI-MS (m/z) 493.16 $(MH)^+$
Chiral separation of racemic compound 165 was carried out using chiral column and afforded the below isomers 165a and 165b:

1-(6-(2H-1,2,3-Triazol-2-yl)-5-(trifluoromethyl)pyridin-3-yl)-3-(7-(1-ethoxyethyl)-2-methylthiazolo[5,4-b]pyridin-6-yl)urea (Compound 165a)
Chiral HPLC RT: 7.23 min.
$^1$HNMR (400 MHz, DMSO-$d_6$) $\delta$ 10.68 (s, 1H), 9.14 (s, 1H), 8.89 (d, $J$ = 2.5 *Hz,* 1H), 8.75 (s, 1H), 8.73 (d, $J$ = 2.5 *Hz,* 1H), 8.18 (s, 2H), 5.63 (q, $J$ = 6.5 *Hz,* 1H), 3.57-3.49 (m, 1H), 3.49-3.39 (m, 1H), 2.86 (s, 3H), 1.58 (d, $J$ = 6.5 *Hz,* 3H), 1.20 (t, $J$ = 7.0 Hz, 3H); ESI-MS (m/z) 493.42 $(MH)^+$;

1-(6-(2H-1,2,3-Triazol-2-yl)-5-(trifluoromethyl)pyridin-3-yl)-3-(7-(1-ethoxyethyl)-2-methylthiazolo[5,4-b]pyridin-6-yl)urea (Compound 165b)
Chiral HPLC RT: 8.46 min
$^1$HNMR (400 MHz, DMSO-$d_6$) $\delta$ 10.67 (s, 1H), 9.13 (s, 1H), 8.89 (d, $J$ = 2.5 *Hz,* 1H), 8.75 (s, 1H), 8.73 (d, $J$ = 2.5 *Hz,* 1H), 8.18 (s, 2H), 5.64 (q, $J$ = 6.5 *Hz,* 1H), 3.57-3.49 (m, 1H), 3.49-3.39 (m, 1H), 2.86 (s, 3H), 1.58 (d, $J$ = 6.5 *Hz,* 3H), 1.20 (t, $J$ = 7.0, 3H); ESI-MS (m/z) 493.42 $(MH)^+$;

(±)-1-(5-Chloro-2-methoxy-6-(2H-1,2,3-triazol-2-yl)pyridin-3-yl)-3-(7-(1-ethoxyethyl)-2-methylthiazolo[5,4-b]pyridin-6-yl)urea (Compound 166)

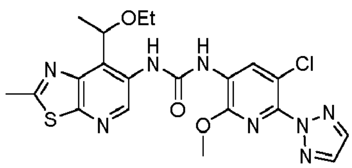

ESI-MS (m/z) 489.42 $(MH)^+$
Chiral separation of racemic compound 166 was carried out using chiral column and afforded the below isomers 166a and 166b:

1-(5-chloro-2-methoxy-6-(2H-1,2,3-triazol-2-yl)pyridin-3-yl)-3-(7-(1-ethoxyethyl)-2-methylthiazolo[5,4-b]pyridin-6-yl)urea (Compound 166a)
Chiral HPLC RT 6.86 min
$^1$HNMR (400 MHz, DMSO-$d_6$) $\delta$ 9.74 (s, 1H), 8.93 (s, 1H), 8.84 (s,1H), 8.74 (s, 1H), 8.15 (s, 2H), 5.55 (q, $J$ = 6.5 Hz, 1H), 4.02 (s, 3H), 3.45-3.37 (m, 1H), 3.32-3.25 (m, 1H), 2.85 (s, 3H), 1.58 (d, $J$ = 6.5 Hz, 3H), 1.11 (t, $J$ = 7.0 Hz, 3H); ESI-MS (m/z) 489.17 (MH)+;

1-(5-Chloro-2-methoxy-6-(2H-1,2,3-triazol-2-yl)pyridin-3-yl)-3-(7-(1-ethoxyethyl)-2-methylthiazolo[5,4-b]pyridin-6-yl)urea (Compound 166b)
Chiral HPLC RT 8.01 min
$^1$HNMR (400 MHz, DMSO-$d_6$) $\delta$ 9.74 (s, 1H), 8.93 (s, 1H), 8.83 (s,1H), 8.74 (s, 1H), 8.15 (s, 2H), 5.54 (t, $J$ = 6.5 Hz, 1H), 4.02 (s, 3H), 3.45-3.37 (m, 1H), 3.32-3.25 (m, 1H), 2.85 (s, 3H), 1.58 (d, $J$ = 7.0 Hz, 3H), 1.11 (t, $J$ = 7.0 Hz, 3H); ESI-MS (m/z) 489.17 (MH)+;

(±)-1-(5-Chloro-2-methoxypyridin-3-yl)-3-(7-(1-ethoxyethyl)-2-methylthiazolo[5,4-b]pyridin-6-yl)urea (Compound 167)

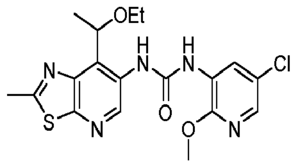

$^1$HNMR (500 MHz, DMSO-$d_6$) δ 9.43 (s, 1H), 8.83 (s, 1H), 8.81 (s, 1H), 8.46 (d, *J* = 2.5 *Hz,* 1H), 7.84 (d, *J* = 2.5 *Hz,* 1H), 5.53 (q, *J* = 6.5 *Hz,* 1H), 4.01 (s, 3H), 3.44-3.23 (m, 2H), 2.85 (s, 3H), 1.56 (d, *J* = 6.5 *Hz,* 3H), 1.09 (t, *J* = 7.0 *Hz,* 3H); ESI-MS (m/z) 422.12 (MH)+;
Chiral separation of racemic compound 167 was carried out using chiral column and afforded the below isomers 167a and 167b:

1-(5-Chloro-2-methoxypyridin-3-yl)-3-(7-(1-ethoxyethyl)-2-methylthiazolo[5,4-b]pyridin-6-yl)urea (Compound 167a)
Chiral HPLC RT: 6.12 min
$^1$HNMR (500 MHz, DMSO-$d_6$) δ 9.43 (s, 1H), 8.83 (s, 1H), 8.81 (s, 1H), 8.46 (d, *J* = 2.5 *Hz,* 1H), 7.84 (d, *J* = 2.5 *Hz,* 1H), 5.53 (q, *J* = 6.5 *Hz,* 1H), 4.01 (s, 3H), 3.44-3.23 (m, 2H), 2.85 (s, 3H), 1.56 (d, *J* = 6.5 *Hz,* 3H), 1.09 (t, *J* = 7.0 *Hz,* 3H); ESI-MS (m/z) 422.11 $(MH)^+$;

1-(5-Chloro-2-methoxypyridin-3-yl)-3-(7-(1-ethoxyethyl)-2-methylthiazolo[5,4-b]pyridin-6-yl)urea (Compound 167b)
Chiral HPLC RT: 7.11 min
$^1$HNMR (500 MHz, DMSO-$d_6$) δ 9.43 (s, 1H), 8.83 (s, 1H), 8.81 (s, 1H), 8.46 (d, *J* = 2.5 *Hz,* 1H), 7.84 (d, *J* = 2.5 *Hz,* 1H), 5.53 (q, *J* = 6.5 *Hz,* 1H), 4.01 (s, 3H), 3.44-3.23 (m, 2H), 2.85 (s, 3H), 1.56 (d, *J* = 6.5 *Hz,* 3H), 1.09 (t, *J* = 7.0 *Hz,* 3H); ESI-MS (m/z) 422.12 $(MH)^+$;

1-(3-Chloro-4-(2H -1,2,3-triazol-2-yl)phenyl)-3-(7-cyclopropyl-2-methylthiazolo[5,4-b]pyridin-6-yl)urea (Compound 168)

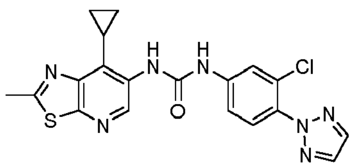

$^1$HNMR (400 MHz, DMSO-$d_6$) δ 9.55 (s, 1H), 8.63 (s, 1H), 8.57 (s,1H), 8.12 (s, 2H), 7.97 (d, *J* = 2.0 Hz, 1H), 7.59 (d, *J* = 8.5 Hz, 1H), 7.53 (dd, *J* = 8.5, 2.0 Hz, 1H), 2.81 (s, 3H), 2.26-2.17 (m, 1H), 1.59-1.52 (m, 2H), 1.19-1.12 (m, 2H). ESI-MS (m/z) 425.98 $(MH)^+$;

1-(3-Chloro-4-(1H-1,2,3-triazol-1-yl)phenyl)-3-(7-cyclopropyl-2-methylthiazolo[5,4-b]pyridin-6-yl)urea (Compound 169)

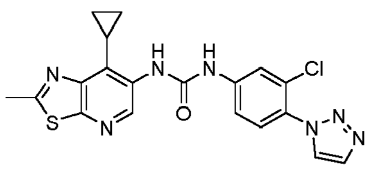

$^1$HNMR (400 MHz, DMSO-*$d_6$*) δ 9.58 (s, 1H), 8.64-8.59 (m, 2H), 8.53 (s, 1H), 8.01-7.97 (m, 2H), 7.63-7.53 (m, 2H), 2.81 (s, 3H), 2.23-2.18 (m, 1H), 1.58-1.53 (m, 2H), 1.20-1.12 (m, 2H); ESI-MS (m/z) 426.04 $(MH)^+$;

1-(7-Cyclopropyl-2-methylthiazolo[5,4-b]pyridin-6-yl)-3-(3,5-dichloro-4-(1H-1,2,3-triazol-1-yl)phenyl)urea (Compound 170)

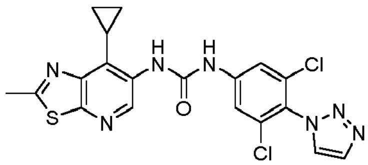

$^1$HNMR (400 MHz, DMSO-$d_6$) δ 9.75 (s, 1H), 8.80 (s, 1H), 8.58 (s, 1H), 8.52 (s,1H), 8.01 (s, 1H), 7.87 (s, 2H), 2.81 (s, 3H), 2.29-2.11 (m, 1H), 1.65-1.46 (m, 2H), 1.22-1.03 (m, 2H); ESI-MS (m/z) 459.9 $(MH)^+$;

1-(3-Cyano-4-(3-methyl-1H-1,2,4-triazol-1-yl)phenyl)-3-(7 -cyclopropyl-2-methylthiazolo[5,4-b]pyridin-6-yl)urea (Compound 171)

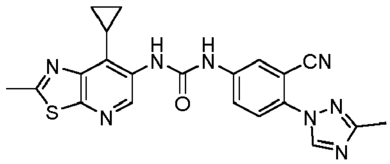

$^1$HNMR (400 MHz, DMSO-$d_6$) δ 9.75 (s, 1H), 8.95 (s, 1H), 8.71 (s, 1H), 8.62 (s, 1H), 8.17 (d, *J* = 2.5 Hz, 1H), 7.87 (dd, *J* = 8.5, 2.5 Hz, 1H), 7.73 (d, *J* = 8.5 Hz, 1H), 2.81 (s, 3H), 2.38 (s, 3H), 2.26-2.17 (m, 1H), 1.58-1.54 (m, 2H), 1.17-1.13 (m, 2H); ESI-MS (m/z) 431.0 $(MH)^+$;

1-(3-Cyano-4-(5-methyl-1H-1,2,4-triazol-1-yl)phenyl)-3-(7 -cyclopropyl-2-methylthiazolo[5,4-b]pyridin-6-yl)urea (Compound 172)

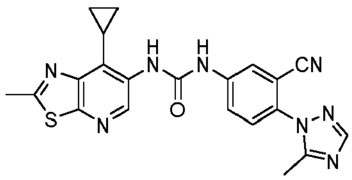

$^1$HNMR (400 MHz, DMSO-$d_6$) δ 9.68 (s, 1H), 8.68 (s, 1H), 8.60 (s, 1H), 8.20 (d, *J* = 2.5 Hz, 1H), 8.12 (s, 1H), 7.90 (dd, *J* = 8.5, 2.5 Hz, 1H), 7.71 (d, *J* = 8.5 Hz,1H), 2.81 (s, 3H), 2.39 (s, 3H), 2.24-2.16 (m, 1H), 1.59-1.53 (m, 2H), 1.19-1.16 (m, 2H); ESI-MS (m/z) 431.1 $(MH)^+$;

1-(3-Chloro-4-(3-methyl-1H-1,2,4-triazol-1-yl)phenyl)-3-(7 -cyclopropyl-2-methylthiazolo[5,4-b]pyridin-6-yl)urea (Compound 173)

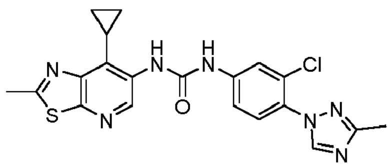

$^1$HNMR (400 MHz, DMSO-$d_6$) δ 9.51 (s, 1H), 8.73 (s, 1H), 8.62 (s, 1H), 8.56 (s, 1H), 7.95 (d, *J* = 2.0 Hz, 1H), 7.53 (d, *J* = 8.5 Hz, 1H), 7.49 (dd, *J* = 8.5, 2.0 Hz, 1H), 2.80 (s, 3H), 2.35 (s, 3H), 2.24-2.14 (m, 1H), 1.59-1.49 (m, 2H), 1.20-1.10 (m, 2H); ESI-MS (m/z) 439.9 $(MH)^+$;

1-(3-Chloro-4-(5-methyl-1H-1,2,4-triazol-1-yl)phenyl)-3-(7-cyclopropyl-2-methylthiazolo[5,4-b]pyridin-6-yl)urea (Compound 174)

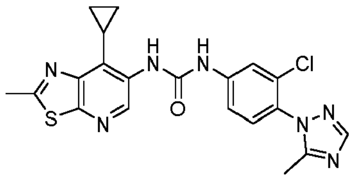

$^1$HNMR (400 MHz, DMSO-$d_6$) δ 9.57 (s, 1H), 8.61 (s, 1H), 8.59 (s, 1H), 8.05 (s, 1H), 7.99 (d, *J* = 2.0 Hz, 1H), 7.53-7.51 (m, 2H), 2.81 (s, 3H), 2.26 (s, 3H), 2.24-2.17 (m, 1H), 1.58-1.52 (m, 2H), 1.19-1.12 (m, 2H); ESI-MS (m/z) 440.1 $(MH)^+$;

1-(5-Bromo-6-methoxypyridin-3-yl)-3-(7-cyclopropyl-2-methylthiazolo[5,4-b]pyridin-6-yl)urea (Compound 175)

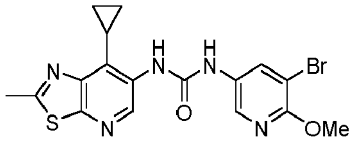

$^1$HNMR (400 MHz, DMSO-$d_6$) δ 9.11 (s, 1H), 8.61 (s, 1H), 8.48 (s, 1H), 8.29 (d, *J* = 2.5 Hz, 1H), 8.17 (d, *J* = 2.5 Hz, 1H), 3.89 (s, 3H), 2.80 (s, 3H), 2.22-2.13 (m, 1H), 1.55-1.50 (m, 2H), 1.18-1.11 (m, 2H); ESI-MS (m/z) 433.8 $(MH)^+$;

1-(5-Chloro-6-(2H-1,2,3-triazol-2-yl)pyridin-3-yl)-3-(7-(methoxymethyl)-2-methylthiazolo[5,4-b]pyridin-6-yl)urea (Compound 176)

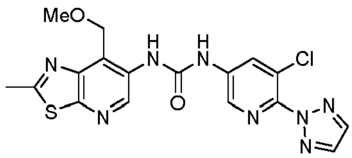

$^1$HNMR (400 MHz, DMSO-$d_6$) δ 10.27 (s, 1H), 9.04 (s, 1H), 8.60 (s, 1H), 8.55 (d, *J = 2.5 Hz,* 1H), 8.51 (d, *J* = 2.5 *Hz,* 1H), 8.17 (s, 2H), 5.02 (s, 2H), 3.37 (s, 3H), 2.86 (s, 3H); ESI-MS (m/z) 430.94 $(MH)^+$;

1-(6-(2H-1,2,3-triazol-2-yl)-5-(trifluoromethyl)pyridin-3-yl)-3-(2-methyl-7-(1-methylcyclopropyl)thiazolo[5,4-b]pyridin-6-yl)urea (Compound 177)

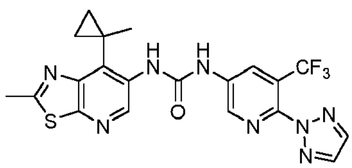

$^1$HNMR (400 MHz, DMSO-$d_6$) δ 10.35 (s, 1H), 8.92 (s, 1H), 8.85 (s, 1H), 8.76 (s, 1H), 8.37 (s, 1H), 8.18 (s, 2H), 2.86 (s, 3H), 1.45 (s, 3H), 1.04-1.02 (m, 2H), 0.94-0.92 (m, 2H); ESI-MS (m/z) 475.30 $(MH)^+$;

1-(5-Chloro-2-methoxy-6-(2H-1,2,3-triazol-2-yl)pyridin-3-yl)-3-(2-methyl-7-(1-methylcyclopropyl)thiazolo[5,4-b]pyridin-6-yl)urea (Compound 178)

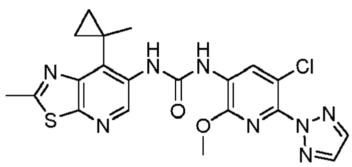

$^1$HNMR (400 MHz, DMSO-$d_6$) δ 9.66 (s, 1H), 8.85 (s, 1H), 8.82 (s, 1H), 8.79 (s, 1H), 8.16 (s, 2H), 4.05 (s, 3H), 2.87

(s, 3H), 1.45 (s, 3H), 1.03-1.00 (m, 2H), 0.90-0.86 (m, 2H); ESI-MS (m/z) 471.30 $(MH)^+$;

1-(3-Chloro-4-(2H-1,2,3-triazol-2-yl)phenyl)-3-(2-methyl-7-(1-methylcyclopropyl)thiazolo[5,4-b]pyridin-6-yl)urea (Compound 179)

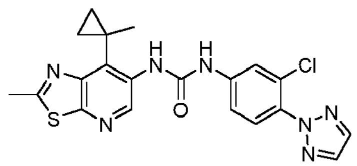

$^1$HNMR (400 MHz, DMSO-$d_6$) δ 9.97 (s, 1H), 8.94 (s, 1H), 8.15 (s, 1H), 8.13 (s, 2H), 8.01 (s, 1H), 7.62 (d, $J$ = 9.0 Hz, 1H), 7.53 (d, $J$ = 9.0 Hz, 1H), 2.86 (s, 3H), 1.44 (s, 3H), 1.04 (s, 2H), 0.92 (s, 2H); ESI-MS (m/z) 440.23 $(MH)^+$;

1-(2-Methoxy-6-(2H-1,2,3-triazol-2-yl)-5-(trifluoromethyl)pyridin-3-yl)-3-(2-methyl-7-(1-methylcyclopropyl)thiazolo[5,4-b]pyridin-6-yl)urea (Compound 180)

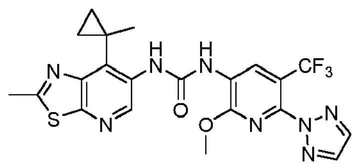

$^1$HNMR (400 MHz, DMSO-$d_6$) δ 9.76 (s, 1H), 9.04 (s, 1H), 8.86 (s, 1H), 8.82 (s, 1H), 8.17 (s, 2H), 4.12 (s, 3H), 2.86 (s, 3H), 1.45 (s, 3H), 1.01-1.00 (m, 2H), 0.90-0.87 (m, 2H); ESI-MS (m/z) 505.2 $(MH)^+$;

1-(5-Chloro-6-(1H-pyrazol-1-yl)pyridin-3-yl)-3-(7-cyclopropyl-2-methylthiazolo[5,4-b]pyridin-6-yl)urea (Compound 181)

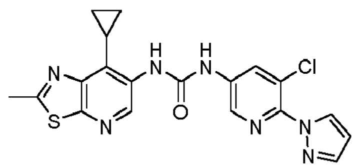

$^1$HNMR (400 MHz, DMSO-$d_6$) δ 9.65 (s, 1H), 8.72 (s, 1H), 8.62 (s, 1H), 8.52 (s,1H), 8.42 (s, 1H), 8.19 (s, 1H), 7.78 (s, 1H), 6.54 (s, 1H), 2.81 (s, 3H), 2.27-2.17 (m, 1H), 1.61-1.52 (m, 2H), 1.21-1.10 (m, 2H); ESI-MS (m/z) 425.96 $(MH)^+$;

1-(3-Chloro-4-(1H-pyrazol-1-yl)phenyl)-3-(7-cyclopropyl-2-methylthiazolo[5,4-b]pyridin-6-yl)urea (Compound 182)

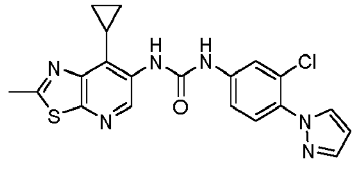

$^1$HNMR (400 MHz, DMSO-$d_6$) δ 9.46 (s, 1H), 8.64 (s, 1H), 8.53 (s,1H), 8.05 (s, 1H), 7.93 (s, 1H), 7.72 (s, 1H), 7.48 (s, 2H), 6.51 (s, 1H), 2.81 (s, 3H), 2.25-2.13 (m, 1H), 1.58-1.51 (m, 2H), 1.19-1.11 (m, 2H); ESI-MS (m/z) 424.95 $(MH)^+$;

1-(3-Chloro-4-(3-(methoxymethyl)-5-methyl-1H-pyrazol-1-yl)phenyl)-3-(7-cyclopropyl-2-methylthiazolo[5,4-b]pyridin-6-yl)urea (Compound 183)

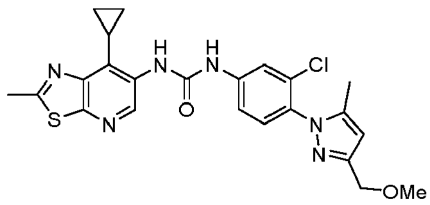

$^1$HNMR (400 MHz, DMSO-$d_6$) δ 9.50 (s, 1H), 8.62 (s, 1H), 8.55 (s,1H), 7.93 (d, $J$ = 2.0 Hz, 1H), 7.48 (dd, $J$ = 8.5, 2.0 Hz, 1H), 7.41 (d, $J$ = 8.5 Hz, 1H), 6.22 (s, 1H), 4.34 (s, 2H), 3.27 (s, 3H), 2.81 (s, 3H),2.25-2.15 (m, 1H), 2.08 (s, 3H), 1.58-1.51 (m, 2H), 1.19-1.11(m, 2H); ESI-MS (m/z) 483.30 $(MH)^+$;

1-(5-Chloro-2-methoxy-6-(2H-1,2,3-triazol-2-yl)pyridin-3-yl)-3-(7-isopropyl-2-methylthiazolo[5,4-b]pyridin-6-yl)urea (Compound 184)

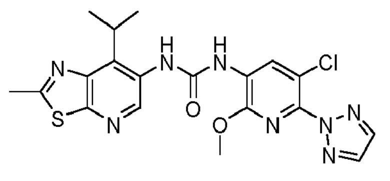

$^1$HNMR (400 MHz, DMSO-$d_6$) δ 9.19 (s, 1H), 9.06 (s, 1H), 8.73 (s, 1H), 8.62 (s, 1H), 8.15 (s, 2H), 4.03 (s, 3H), 3.60-3.50 (m, 1H), 2.86 (s, 3H), 1.50 (d, $J$ = 6.9 Hz, 6H); ESI-MS (m/z) 459.01 $(MH)^+$;

1-(5-Chloro-6-(2H-1,2,3-triazol-2-yl)pyridin-3-yl)-3-(7-(2-(2-methoxyethoxy)ethyl)-2-methylthiazolo[5,4-b]pyridin-6-yl)urea (Compound 185)

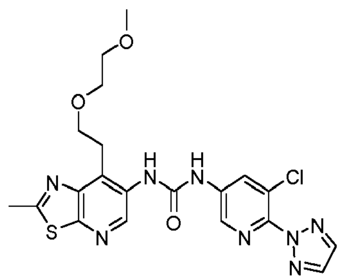

$^1$HNMR (400 MHz, DMSO-$d_6$) δ 9.91 (s, 1H), 8.79 (s, 1H), 8.66 (s, 1H), 8.58 (d, $J$ = 2.1 Hz, 1H), 8.50 (d, $J$ = 2.1 Hz, 1H), 8.17 (s, 2H), 3.73 (t, $J$ = 7.0 Hz, 2H), 3.53 (t, $J$ = 4.8 Hz, 2H), 3.41-3.37 (m, 4H), 3.16 (s, 3H), 2.86 (s, 3H); ESI-MS (m/z) 488.81 (MH)+;

1-(5-Chloro-2,6-dimethoxypyridin-3-yl)-3-(7-isopropyl-2-methylthiazolo[5,4-b]pyridin-6-yl)urea (Compound 186)

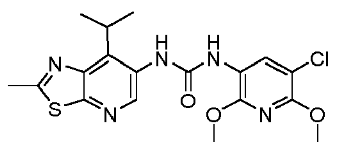

$^1$HNMR (400 MHz, DMSO-$d_6$) δ 8.73 (s, 1H), 8.59 (s, 2H), 8.43 (s, 1H), 4.04 (s, 3H), 3.94 (s, 3H), 3.60-3.56 (m, 1H), 2.85 (s, 3H), 1.48 (d, $J$ = 6.9 Hz, 6H); ESI-MS (m/z) 421.97 $(MH)^+$;

1-(5-Chloro-2-methoxypyridin-3-yl)-3-(7-isopropyl-2-methylthiazolo[5,4-b]pyridin-6-yl)urea (Compound 187)

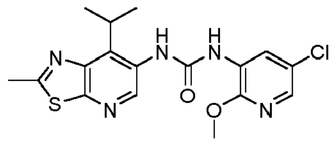

$^1$HNMR (400 MHz, DMSO-$d_6$) δ 8.96 (s, 1H), 8.90 (s, 1H), 8.58 (s, 1H), 8.48 (d, $J$ = 2.4 Hz, 1H), 7.82 (d, $J$ = 2.4 Hz, 1H), 4.01 (s, 3H), 3.60-3.57 (m, 1H), 2.85 (s, 3H), 1.49 (d, $J$ = 6.9 Hz, 6H); ESI-MS (m/z) 391.87 (MH)$^+$;

1-(3-Chloro-4-(2H-1,2,3-triazol-2-yl)phenyl)-3-(7-isopropyl-2-methylthiazolo[5,4-b]pyridin-6-yl)urea (Compound 188)

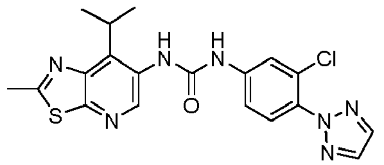

$^1$HNMR (400 MHz, DMSO-$d_6$) d 9.46 (s, 1H), 8.58-8.49 (m, 2H), 8.12 (s, 2H), 7.98-7.93 (m, 1H), 7.62-7.56 (m, 1H), 7.56-7.50 (m, 1H), 3.62-3.50 (m, 1H), 2.86 (s, 3H), 1.49 (d, $J$ = 6.9 Hz, 6H); ESI-MS (m/z) 427.98 (MH)$^+$;

1-(5-Chlorothiophen-3-yl)-3-(7-isopropyl-2-methylthiazolo[5,4-b]pyridin-6-yl)urea (Compound 189)

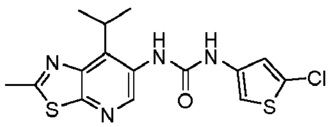

$^1$HNMR (400 MHz, DMSO-$d_6$) d 9.20 (s, 1H), 8.51 (s, 1H, $D_2O$ exchangeable), 8.32 (s, $D_2O$ exchangeable, 1H), 7.12 (s, 2H), 3.61-3.47 (m, 1H), 2.85 (s, 3H), 1.47 (d, $J$= 6.8 Hz, 6H); ESI-MS (m/z) 367.0 (MH)$^+$;

1-(5-Chlorothiophen-3-yl)-3-(7-cyclopropyl-2-methylthiazolo[5,4-b]pyridin-6-yl)urea (Compound 190)

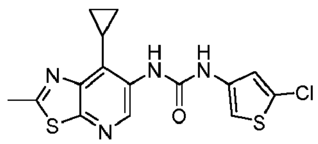

$^1$HNMR (400 MHz, DMSO-$d_6$) d 9.32 (s, 1H, $D_2O$ exchangeable), 8.63 (s, 1H), 8.38 (s, 1H, $D_2O$ exchangeable), 7.17-7.09 (m, 2H), 2.80 (s, 3H), 2.20-2.11 (m, 1H), 1.54-1.47 (m, 2H), 1.17-1.10 (m, 2H); ESI-MS (m/z) 364.88 (MH)$^+$;

1-(6-(2H-1,2,3-triazol-2-yl)-5-(trifluoromethyl)pyridin-3-yl)-3-(7-isopropyl-2-methylthiazolo[5,4-b]pyridin-6-yl)urea (Compound 191)

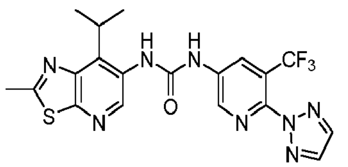

$^1$HNMR (400 MHz, DMSO-$d_6$) δ 9.84 (s, 1H), 8.87 (s, 1H), 8.79 (s, 1H), 8.71 (s, 1H), 8.54 (s, 1H), 8.18 (s, 2H), 3.64-3.51 (m, 1H), 2.86 (s, 3H), 1.49 (d, $J$ = 7.0 Hz, 6H); ESI-MS (m/z) 463.12 (MH)$^+$;

1-(5-Chloro-2-methoxypyridin-3-yl)-3-(7-cyclopropyl-2-methylthiazolo[5,4-b]pyridin-6-yl)urea (Compound 192)

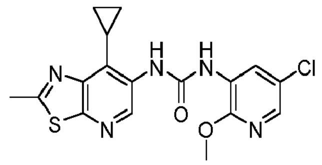

$^1$HNMR (500 MHz, DMSO-$d_6$) δ 9.10 (s, 1H), 8.98 (s, 1H), 8.65 (s, 1H), 8.49 (d, *J* = 2.4 Hz, 1H), 7.82 (d, *J* = 2.4 Hz, 1H), 4.01 (s, 3H), 3.17 (s, 3H), 2.22-2.15 (m, 1H), 1.62-1.52 (m, 2H), 1.20-1.10 (m, 2H); ESI-MS (m/z) 390.8 $(MH)^+$;

1-(3-Chloro-4-(difluoromethoxy)phenyl)-3-(7-cyclopropyl-2-methylthiazolo[5,4-b]pyridin-6-yl)urea (Compound 193)

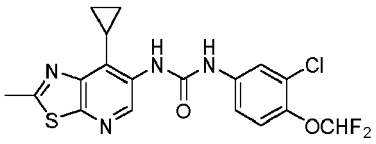

$^1$HNMR (400 MHz, DMSO-$d_6$) δ 9.31 (s, 1H), 8.62 (s, 1H), 8.46 (s, 1H), 7.86 (d, *J = 2.5 Hz,* 1H), 7.39-7.35 (m, 1H), 7.32-7.29 (m, 1H), 7.18 (t, *J*= 80.0 *Hz,* 1H), 2.80 (s, 3H), 2.22-2.12 (m, 1H), 1.55-1.50 (m, 2H), 1.17-1.13 (m, 2H); ESI-MS (m/z) 425.04 (MH)+;

1-(5-Chloro-6-(1-methyl-1H-pyrazol-5-yl)pyridin-3-yl)-3-(7-cyclopropyl-2-methylthiazolo[5,4-b]pyridin-6-yl)urea (Compound 194)

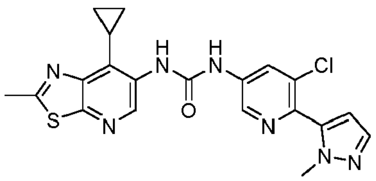

$^1$HNMR (400 MHz, DMSO-$d_6$) δ 9.77 (s, 1H), 8.83 (s, 1H), 8.68 (d, *J* = 2.5 *Hz,* 1H), 8.60 (s, 1H), 8.35 (d, *J* = 2.5 *Hz,* 1H), 7.52 (d, *J* = 2.0 *Hz,* 1H), 6.58 (d, *J* = 2.0 *Hz,* 1H), 3.83 (s, 3H), 2.81 (s, 3H), 2.26-2.17 (m, 1H), 1.59-1.53 (m, 2H), 1.19-1.13 (m, 2H); ESI-MS (m/z) 440.02 $(MH)^+$; and

1-(5-Chloro-2-(2-(dimethylamino)ethoxy)pyridin-3-yl)-3-(7-cyclopropyl-2-methylthiazolo[5,4-b]pyridin-6-yl)urea (Compound 195)

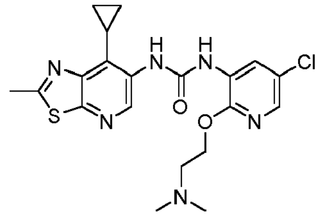

$^1$HNMR (400 MHz, DMSO-$d_6$) δ: 9.22 (s, 1H), 8.72 (s, 1H), 8.60 (s, 1H), 8.48 (d, *J* = 2.5 *Hz,* 1H), 7.80(d, *J* = 2.5 *Hz,* 1H), 4.51-4.48 (m, 2H), 2.80 (s, 3H), 2.71-2.68 (m, 2H), 2.22 (s, 6H), 2.21-2.19 (m, 1H), 1.60-1.58 (m, 2H), 1.18-1.13 (m, 2H); ESI-MS (m/z) 447.26 $(MH)^+$.

**Example-82:** Preparation of (R) or (S)-1-(5-chloro-6-(1H-pyrazol-1-yl)pyridin-3-yl)-3-(7-(1-methoxyethyl)-2-methylthiazolo[5,4-b]pyridin-6-yl)urea (Compound 196a) [Stereochemistry tentatively assigned, it could be either (R) or (S)] And

**Example-83:** Preparation of (S) or (R)-1-(5-chloro-6-(1H-pyrazol-1-yl)pyridin-3-yl)-3-(7-(1-methoxyethyl)-2-methylthiazolo[5,4-b]pyridin-6-yl)urea compound 196b) [Stereochemistry tentatively assigned, it could be either (S) or (R)]

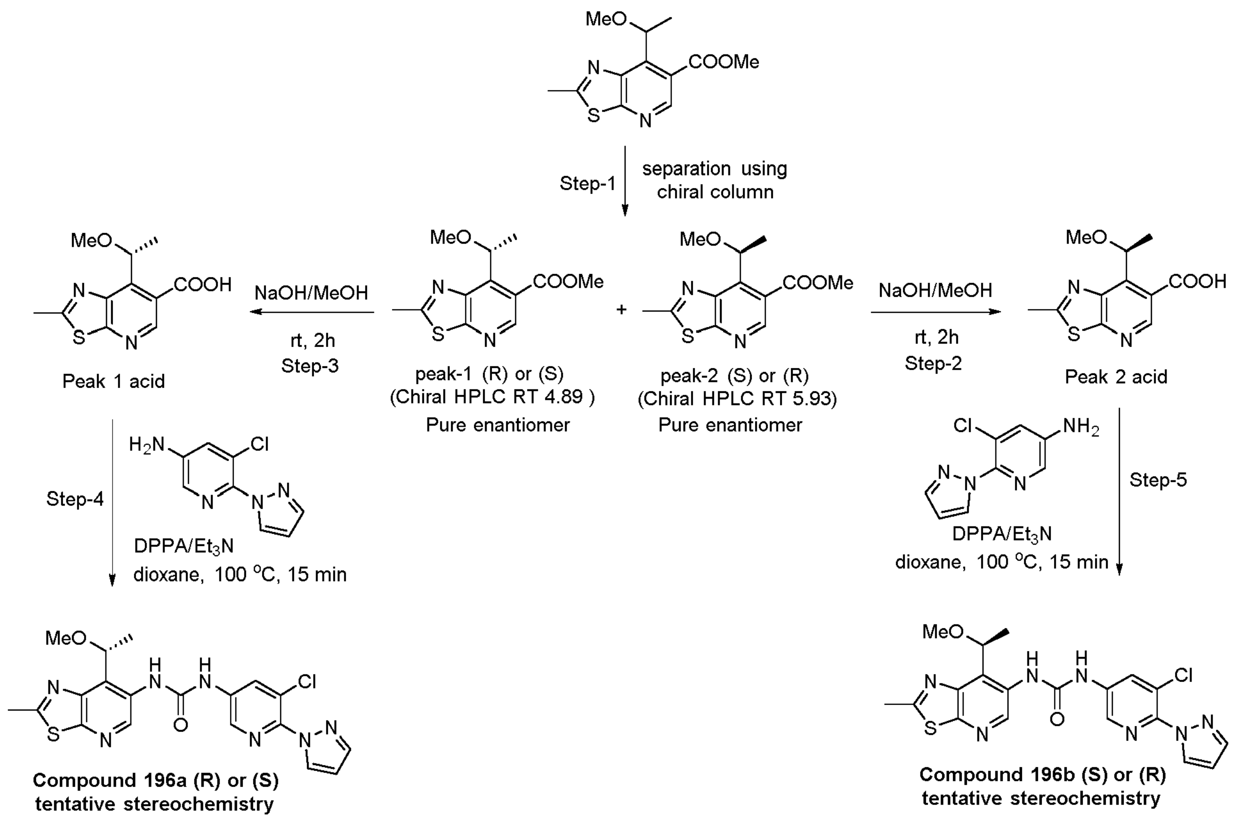


Step-1: Chiral separation: The racemic methyl 7-(1-methoxyethyl)-2-methylthiazolo[5,4-b]pyridine-6-carboxylate was resolved into corresponding enantiomers (peak-1 rt-4.89 min and peak-2 rt-5.93 min) by using chiral column. [Stereochemistry tentatively assigned, it could be either (S) or (R)].

Step-2 & Step-3: preparation of 7-(1-methoxy-2-methylpropyl)-2-methylthiazolo[5,4-b]pyridine-6-carboxylic acid: The two enantiomers obtained in step-1 were separately hydrolysed by treating with sodium hydroxide in methanol by following the similar procedure described for the hydrolysis of racemic ester in step-4 of Example-20 to afford the corresponding acids (peak-1 acid and peak-2 acid).

Step-4: Preparation of (R) or (S)-1-(5-chloro-6-(1H-pyrazol-1-yl)pyridin-3-yl)-3-(7-(1-methoxyethyl)-2-methylthiazolo[5,4-b]pyridin-6-yl)urea (compound 48a) [Stereochemistry tentatively assigned, it could be either (R) or (S)].

To a stirred solution of 7-(1-methoxy-2-methylpropyl)-2-methylthiazolo[5,4-b]pyridine-6-carboxylic acid (peak-1 acid), obtained from step-3 (50 mg, 0.198 mmol) in 1,4-dioxane (3 mL) in a sealed vial, was added DPPA (0.052 mL, 0.238 mmol) and triethylamine (0.55 $\mu$L, 0.396 mmol). The reaction mixture was stirred at 25°C for 15 min. Then 5-chloro-6-(1H-pyrazol-1-yl)pyridin-3-amine (38 mg, 0.194 mmol) was added and heated the reaction mixture at 100°C for 15 min. After cooling to RT, water (5 mL) was added to the reaction mixture and extracted with ethyl acetate (3$\times$10 mL). The combined organic layers were washed with brine (15 mL), saturated aqueous $NaHCO_3$ (10 mL), dried ($Na_2SO_4$) and filtered. The filtrate was rotary evaporated and the residue was then purified by flash column chromatography (silica gel) to provide (10 mg, 14%) of the desired product as white solid. $^1$HNMR (400 MHz, DMSO-$d_6$) $\delta$ 10.39 (s, 1H), 9.12 (s, 1H), 8.69 (s, 1H), 8.51 (d, *J* = 2.5 *Hz,* 1H), 8.44 (d, *J* = 2.5 *Hz,* 1H), 8.20 (d, *J* = 2.5 *Hz,* 1H), 7.78 (d, *J* = 1.5 *Hz,* 1H), 6.55 - 6.53 (m, 1H), 5.51 (q, *J* = 6.5 *Hz,* 1H), 3.31 (s, 3H), 2.86 (s, 3H), 1.56 (d, *J* = 6.5 *Hz,* 3H); ESI-MS (m/z), 443.99 $(MH)^+$.

Step-5: Preparation of (S) or (R)-1-(5-chloro-6-(1H-pyrazol-1-yl)pyridin-3-yl)-3-(7-(1-methoxyethyl)-2-methylthiazolo[5,4-b]pyridin-6-yl)urea (compound 196b) [Stereochemistry tentatively assigned, it could be either (S) or (R)]

The tilted compound was prepared by following the similar procedure described in step-4 by reacting the 7-(1-methoxy-2-methylpropyl)-2-methylthiazolo[5,4-b]pyridine-6-carboxylic acid (peak-2 acid) obtained from step-2 with 5-chloro-6-(1H-pyrazol-1-yl)pyridin-3-amine. $^1$HNMR (400 MHz, DMSO-$d_6$) $\delta$ 10.39 (s, 1H), 9.12 (s, 1H), 8.69 (s, 1H), 8.51 (d, *J* = 2.5 *Hz,* 1H), 8.44 (d, *J* = 2.5 *Hz,* 1H), 8.20 (d, *J* = 2.5 *Hz,* 1H), 7.78 (d, *J* = 1.5 *Hz,* 1H), 6.55 - 6.53 (m, 1H), 5.51 (q, *J* = 6.5 *Hz,* 1H), 3.31 (s, 3H), 2.86 (s, 3H), 1.56 (d, *J* = 6.5 *Hz,* 3H); ESI-MS (m/z), 444.11 $(MH)^+$.

**Example-84:** The following compounds were prepared by using the similar procedure described in example-82 or example-83 from the corresponding intermediates:

(S) or (R) -1-(5-chloro-6-(isoxazol-4-yl)pyridin-3-yl)-3-(7-(1-methoxyethyl)-2-methylthiazolo[5,4-b]pyridin-6-yl)urea (Compound 197) [Stereochemistry tentatively assigned, it could be either (S) or (R)]

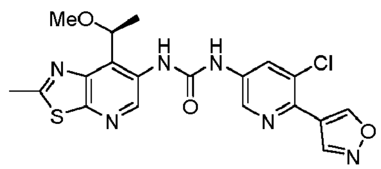

The compound 197 was prepared by using step-5 intermediate (peak-2 acid) of example-83. $^1$HNMR (400 M*Hz*, DMSO-*d*$_6$) δ 10.35 (s, 1H, $D_2O$ exchangeable), 9.64 (s, 1H), 9.15 (s, 1H), 9.11 (s, 1H), 8.69 (s, 1H, $D_2O$ exchangeable), 8.60 (s, 1H), 8.38 (s, 1H), 5.50 (q, *J* = 7.0 *Hz,* 1H), 3.31 (s, 3H), 2.86 (s, 3H), 1.56 (d, *J* = 7.0 *Hz,* 3H); ESI-MS (*m*/*z*) 445.04 (MH)$^+$;

(R) or (S) -1-(3-Chloro-4-(1H-1,2,3-triazol-1-yl)phenyl)-3-(7-(1-methoxyethyl)-2-methylthiazolo[5,4-b]pyridin-6-yl)urea (Compound 198a) [Stereochemistry tentatively assigned, it could be either (R) or (S)]

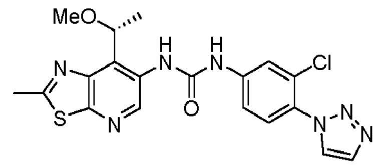

The compound 198a was prepared by using step-4 intermediate (peak-1 acid) of example-82. $^1$HNMR (400 M*Hz*, DMSO-*d*$_6$) δ 10.28 (s, 1H, $D_2O$ exchangeable), 9.10 (s, 1H), 8.61 (s, 1H, $D_2O$ exchangeable), 8.53 (s, 1H), 8.03 (s, 1H), 7.97 (s, 1H), 7.62-7.55 (m, 2H), 5.50 (q, *J*= 7.0 *Hz,* 1H), 3.31 (s, 3H), 2.85 (s, 3H), 1.56 (d, *J*= 7.0 *Hz,* 3H); ESI-MS (*m*/*z*) 443.99 (MH)$^+$;

(S) or (R) -1-(3-Chloro-4-(1H-1,2,3-triazol-1-yl)phenyl)-3-(7-(1-methoxyethyl)-2-methylthiazolo[5,4-b]pyridin-6-yl)urea (Compound 198b) [Stereochemistry tentatively assigned, it could be either (S) or (R)]

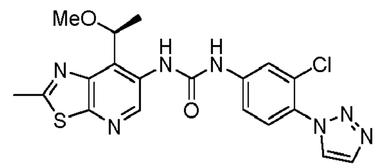

The compound 198b was prepared by using step-5 intermediate (peak-2 acid) of example-83. $^1$HNMR (400 M*Hz*, DMSO-*d*$_6$) δ 10.28 (s, 1H, $D_2O$ exchangeable), 9.10 (s, 1H), 8.61 (s, 1H, $D_2O$ exchangeable), 8.53 (s, 1H), 8.03 (s, 1H), 7.97 (s, 1H), 7.62-7.55 (m, 2H), 5.50 (q, *J*= 7.0 *Hz,* 1H), 3.31 (s, 3H), 2.85 (s, 3H), 1.56 (d, *J*= 7.0 *Hz,* 3H); ESI-MS (*m*/*z*) 443.96 (MH)$^+$;

(S) or (R) -1-(3-Chloro-4-(pyrazin-2-yl)phenyl)-3-(7-(1-methoxyethyl)-2-methylthiazolo[5,4-b]pyridin-6-yl)urea (Compound 199) [Stereochemistry tentatively assigned, it could be either (S) or (R)]

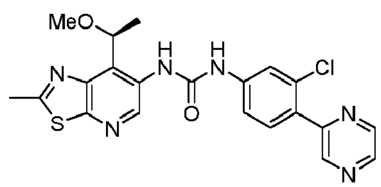

The compound 199 was prepared by using step-5 intermediate (peak-2 acid) of example-83. $^1$HNMR (400 MHz, DMSO-*d*$_6$) δ 10.16 (s, 1H), 9.11 (s, 1H), 8.96 (s, 1H), 8.78 (s, 1H), 8.66 (s, 1H), 8.57 (s, 1H), 7.93 (s, 1H), 7.64 (d, *J* = 8.5 *Hz,* 1H), 7.54 (d, *J* = 8.5 *Hz,* 1H), 5.51 (q, *J* = 6.5 *Hz,* 1H), 3.34 (s, 3H), 2.85 (s, 3H), 1.55 (d, *J* = 6.5 *Hz,* 3H); ESI-MS (m/z) 454.93 (MH)$^+$;

(R) or (S)-1-(5-Cyano-6-(2H-1,2,3-triazol-2-yl)pyridin-3-yl)-3-(7-(1-methoxyethyl)-2-methylthiazolo[5,4-b]pyridin-6-yl)urea (Compound 200a) [Stereochemistry tentatively assigned, it could be either (R) or (S)]

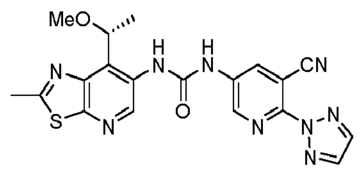

The compound 200a was prepared by using step-4 intermediate (peak-1 acid) of example-82. $^1$HNMR (400 M*Hz*, DMSO-$d_6$) δ 10.57 (s, 1H), 9.12 (s, 1H), 8.84 (s, 1H), 8.76 (s,1H), 8.72 (s, 1H), 8.29 (s, 2H), 5.51 (d, *J* = 6.5 *Hz,* 1H), 3.32 (s, 3H), 2.86 (s, 3H),1.56 (d, *J* = 6.5 *Hz,* 3H); ESI-MS (m/z) 436.17 (MH)$^+$;

(S) or (R) -1-(5-Cyano-6-(2H-1,2,3-triazol-2-yl)pyridin-3-yl)-3-(7-(1-methoxyethyl)-2-methylthiazolo[5,4-b]pyridin-6-yl)urea (Compound 200b) [Stereochemistry tentatively assigned, it could be either (S) or (R)]

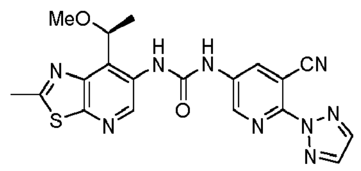

The compound 200b was prepared by using step-5 intermediate (peak-2 acid) of example-83. $^1$HNMR (400 M*Hz*, DMSO-$d_6$) δ 10.57 (s, 1H), 9.12 (s, 1H), 8.84 (s, 1H), 8.76 (s,1H), 8.72 (s, 1H), 8.29 (s, 2H), 5.51 (d, *J*= 6.5 *Hz,* 1H), 3.32 (s, 3H), 2.86 (s, 3H),1.56 (d, *J*= 6.5 *Hz,* 3H); ESI-MS (m/z) 436.12 (MH)$^+$;

(R) or (S) -1-(3-Chloro-4-(1H-pyrazol-1-yl)phenyl)-3-(7-(1-methoxyethyl)-2-methylthiazolo[5,4-b]pyridin-6-yl)urea (Compound 201a) [Stereochemistry tentatively assigned, it could be either (R) or (S)]

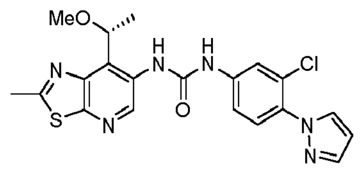

The compound 201a was prepared by using step-4 intermediate (peak-1 acid) of example-82. $^1$HNMR (400 MHz, DMSO-$d_6$) δ 10.14 (s, 1H), 9.10 (s, 1H), 8.56 (s, 1H), 8.06 (s, 1H), 7.95 (s, 1H), 7.73 (s, 1H), 7.50 (s, 2H), 6.51 (s, 1H), 5.50 (q, *J* = 6.5 *Hz,* 1H), 3.31 (s, 3H), 2.85 (s, 3H), 1.55 (d, *J* = 6.5 Hz, 3H); ESI-MS (m/z) 443.11 (MH)$^+$;

(S) or (R) -1-(3-Chloro-4-(1H-pyrazol-1-yl)phenyl)-3-(7-(1-methoxyethyl)-2-methylthiazolo[5,4-b]pyridin-6-yl)urea (Compound 201b) [Stereochemistry tentatively assigned, it could be either (S) or (R)]

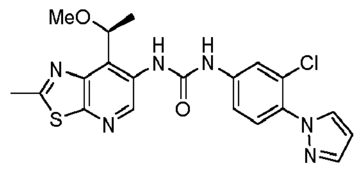

The compound 201b was prepared by using step-5 intermediate (peak-2 acid) of example-83. $^1$HNMR (400 MHz, DMSO-$d_6$) δ 10.14 (s, 1H), 9.10 (s, 1H), 8.56 (s, 1H), 8.06 (s, 1H), 7.95 (s, 1H), 7.73 (s, 1H), 7.50 (s, 2H), 6.51 (s, 1H), 5.50 (q, *J* = 6.5 *Hz,* 1H), 3.31 (s, 3H), 2.85 (s, 3H), 1.55 (d, *J* = 6.5 *Hz,* 3H); ESI-MS (m/z) 443.00 (MH)$^+$;

(S) or (R) -1-(3-Chloro-4-(pyrimidin-2-yl)phenyl)-3-(7-(1-methoxyethyl)-2-methylthiazolo[5,4-b]pyridin-6-yl)urea (Compound 202) [Stereochemistry tentatively assigned, it could be either (S) or (R)]

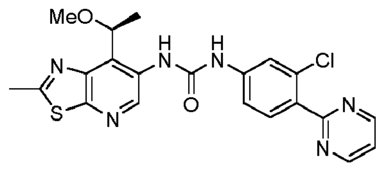

The compound 202 was prepared by using step-5 intermediate (peak-2 acid) of example-83. 1H NMR (400 MHz, DMSO-$d_6$) δ 10.15 (s, 1H), 9.11 (s, 1H), 8.95 (s, 2H), 8.57 (s, 1H), 7.89 (s, 1H), 7.78 (d, *J* = 8.5 *Hz,* 1H), 7.52 (s, 2H), 5.52 (q, *J* = 6.5 *Hz,* 1H), 3.31 (s, 3H), 2.86 (s, 3H), 1.56 (d, *J* = 6.5 *Hz,* 3H); ESI-MS (m/z) 455.18 $(MH)^+$;

(R) or (S)-1-(3-Chloro-4-(1,3,4-oxadiazol-2-yl)phenyl)-3-(7-(1-methoxyethyl)-2-methylthiazolo[5,4-b]pyridin-6-yl)urea (Compound 203a) [Stereochemistry tentatively assigned, it could be either (R) or (S)]

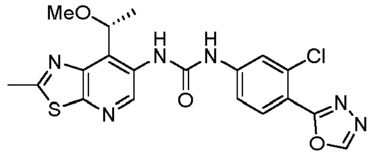

The compound 203a was prepared by using step-4 intermediate (peak-1 acid) of example-82. $^1$HNMR (400 MHz, DMSO-$d_6$) 10.34 (s, 1H, $D_2O$ exchangeable), 9.41 (s, 1H, $D_2O$ exchangeable), 9.09 (s, 1H), 8.63 (s, 1H), 8.01 (s, 1H), 7.96 (d, *J* = 8.5 *Hz,* 1H), 7.58 (d, *J* = 8.5 *Hz,* 1H), 5.50 (q, *J* = 6.5 *Hz,* 1H), 3.30 (s, 3H), 2.85 (s, 3H), 1.55 (d, *J* = 6.5 *Hz,* 3H); ESI-MS (m/z) 444.97 $(MH)^+$;

(S) or (R) -1-(3-Chloro-4-(1,3,4-oxadiazol-2-yl)phenyl)-3-(7-(1-methoxyethyl)-2-methylthiazolo[5,4-b]pyridin-6-yl)urea (Compound 203b) [Stereochemistry tentatively assigned, it could be either (S) or (R)]

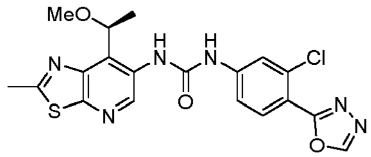

The compound 203b was prepared by using step-5 intermediate (peak-2 acid) of example-83. $^1$HNMR (400 MHz, DMSO-$d_6$) 10.34 (s, 1H, $D_2O$ exchangeable), 9.41 (s, 1H, $D_2O$ exchangeable), 9.09 (s, 1H), 8.63 (s, 1H), 8.01 (s, 1H), 7.96 (d, *J* = 8.5 *Hz,* 1H), 7.58 (d, *J*= 8.5 *Hz,* 1H), 5.50 (t, *J*= 6.5 *Hz,* 1H), 3.17 (s, 3H), 2.85 (s, 3H), 1.55 (d, *J*= 6.5 *Hz,* 3H); ESI-MS (m/z) 445.11 $(MH)^+$;

(S) or (R) -1-(3-Chloro-4-(oxazol-5-yl)phenyl)-3-(7-(1-methoxyethyl)-2-methylthiazolo[5,4-b]pyridin-6-yl)urea (Compound 204) [Stereochemistry tentatively assigned, it could be either (S) or (R)]

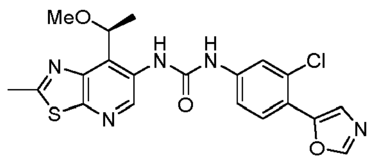

The compound 204 was prepared by using step-5 intermediate (peak-2 acid) of example-83. $^1$HNMR (400 MHz, DMSO-$d_6$) δ 10.16 (s, 1H, $D_2O$ exchangeable), 9.09 (s, 1H), 8.56 (s, 1H, $D_2O$ exchangeable), 8.52 (s, 1H), 7.94 (s, 1H), 7.78 (d, *J* = 8.5 *Hz,* 1H), 7.70 (s, 1H), 7.51 (d, *J* = 8.5 *Hz,* 1H), 5.50 (q, *J* = 6.5 Hz, 1H), 3.30 (s, 3H), 2.85 (s, 3H), 1.54 (d, *J* = 6.5 *Hz,* 3H); ESI-MS (m/z) 444.29 $(MH)^+$;

(R) or (S) -1-(5-(Difluoromethyl)-6-(2H-1,2,3-triazol-2-yl)pyridin-3-yl)-3-(7-(1-methoxyethyl)-2-methylthiazolo[5,4-b]pyridin-6-yl)urea (Compound 205a) [Stereochemistry tentatively assigned, it could be either (R) or (S)]

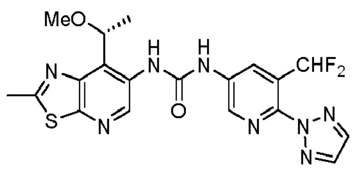

The compound 205a was prepared by using step-4 intermediate (peak-1 acid) of example-82. $^1$HNMR (400 M*Hz*, DMSO-$d_6$) δ 10.52 (s, 1H, $D_2O$ Exchangeable), 9.15 (d, *J* = 4.5 *Hz,* 1H), 8.78 (s, 1H), 8.73 (s, 1H, $D_2O$ Exchangeable), 8.62 (s, 1H), 8.21 (s, 2H), 7.32 (t, *J* = 54 *Hz,* 1H), 5.52 (q, *J* = 6.5 *Hz,* 1H), 3.32 (s, 3H), 2.86 (s, 3H), 1.57 (s, 3H); ESI-MS (m/z) 460.93 $(MH)^+$;

(S) or (R) -1-(5-(Difluoromethyl)-6-(2H-1,2,3-triazol-2-yl)pyridin-3-yl)-3-(7-(1-methoxyethyl)-2-methylthiazolo[5,4-b]pyridin-6-yl)urea (Compound 205b) [Stereochemistry tentatively assigned, it could be either (S) or (R)]

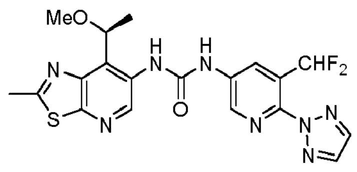

The compound 205b was prepared by using step-5 intermediate (peak-2 acid) of example-83. $^{1}$HNMR (400 M*Hz*, DMSO-*d*$_6$) δ 10.52 (s, 1H, $D_2O$ Exchangeable), 9.15 (d, *J* = 4.5 *Hz,* 1H), 8.78 (s, 1H), 8.73 (s, 1H, $D_2O$ Exchangeable), 8.62 (s, 1H), 8.21 (s, 2H), 7.32 (t, *J* = 54 *Hz,* 1H), 5.52 (q, *J* = 6.5 *Hz,* 1H), 3.32 (s, 3H), 2.86 (s, 3H), 1.57 (s, 3H); ESI-MS (m/z) 461.01 $(MH)^+$;

(R) or (S) -1-(5-(Difluoromethyl)-6-(1H-1,2,3-triazol-1-yl)pyridin-3-yl)-3-(7-(1-methoxyethyl)-2-methylthiazolo[5,4-b]pyridin-6-yl)urea (Compound 206a) [Stereochemistry tentatively assigned, it could be either (R) or (S)]

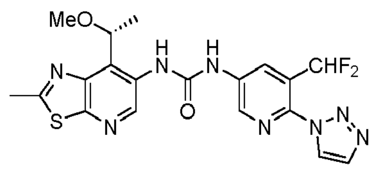

The compound 206a was prepared by using step-4 intermediate (peak-1 acid) of example-82. $^{1}$HNMR (400 M*Hz*, DMSO-*d*$_6$) δ 10.54 (s, 1H, $D_2O$ Exchangeable), 9.15 (d, *J* = 4.0 *Hz,* 1H), 8.81 (s, 1H), 8.77 (s,1H), 8.74 (s, 1H, $D_2O$ Exchangeable), 8.64 (s, 1H), 8.03 (d, *J* = 4.0 *Hz,* 1H), 7.40 (t, *J* = 56.0 *Hz,* 1H), 5.52 (q, *J* = 6.0 *Hz,* 1H), 3.32 (s, 3H), 2.86 (s, 3H), 1.57 (d, *J* = 6.0 *Hz,* 3H). ESI-MS (m/z) 460.93 $(MH)^+$;

(S) or (R) -1-(5-(Difluoromethyl)-6-(1H-1,2,3-triazol-1-yl)pyridin-3-yl)-3-(7-(1-methoxyethyl)-2-methylthiazolo[5,4-b]pyridin-6-yl)urea (Compound 206b) [Stereochemistry tentatively assigned, it could be either (S) or (R)]

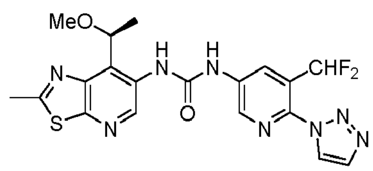

The compound 206b was prepared by using step-5 intermediate (peak-2 acid) of example-83. $^{1}$HNMR (400 M*Hz*, DMSO-d$_6$) δ 10.54 (s, 1H, $D_2O$ Exchangeable), 9.15 (d, *J* = 4.0 *Hz,* 1H), 8.81 (s, 1H), 8.77 (s,1H), 8.74 (s, 1H, $D_2O$ Exchangeable), 8.64 (s, 1H), 8.03 (d, *J* = 4.0 *Hz,* 1H), 7.40 (t, *J* = 56.0 *Hz,* 1H), 5.52 (q, *J* = 6.0 *Hz,* 1H), 3.32 (s, 3H), 2.86 (s, 3H), 1.57 (d, *J* = 6.0 *Hz,* 3H). ESI-MS (m/z) 461.10 $(MH)^+$;

(R) or (S)-1-(3-(Difluoromethyl)-4-(2H-1,2,3-triazol-2-yl)phenyl)-3-(7-(1-methoxyethyl)-2-methylthiazolo[5,4-b]pyridin-6-yl)urea (Compound 207a) [Stereochemistry tentatively assigned, it could be either (R) or (S)]

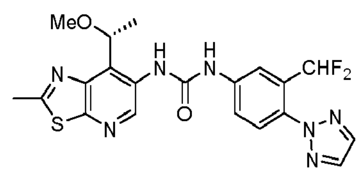

The compound 207a was prepared by using step-4 intermediate (peak-1 acid) of example-82. $^{1}$HNMR (400 M*Hz*, DMSO-*d*$_6$) δ 10.28 (s, 1H, $D_2O$ Exchangeable), 9.14 (s, 1H), 8.59 (s, 1H, $D_2O$ Exchangeable), 8.18 (s, 2H), 8.07 (s, 1H), 7.89-7.83 (m, 2H), 7.39 (t, *J* = 56 *Hz,* 1H), 5.57-5.47 (d, *J* = 6.5 *Hz,* 1H), 3.32 (s, 3H), 2.86 (s, 3H), 1.56 (d, *J* = 6.5 *Hz,* 3H). ESI-MS (m/z) 460.18 $(MH)^+$;

(S) or (R) -1-(3-(Difluoromethyl)-4-(2H-1,2,3-triazol-2-yl)phenyl)-3-(7-(1-methoxyethyl)-2-methylthiazolo[5,4-b]pyridin-6-yl)urea (Compound 207b) [Stereochemistry tentatively assigned, it could be either (S) or (R)]

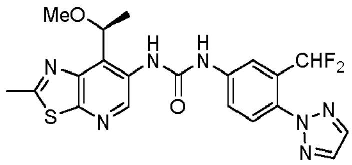

The compound 207b was prepared by using step-5 intermediate (peak-2 acid) of example-83. $^1$HNMR (400 M*Hz*, DMSO-*d*$_6$) $\delta$ 10.28 (s, 1H, $D_2O$ Exchangeable), 9.14 (s, 1H), 8.59 (s, 1H, $D_2O$ Exchangeable), 8.18 (s, 2H), 8.07 (s, 1H), 7.89-7.83 (m, 2H), 7.39 (t, *J* = 56 *Hz,* 1H), 5.57-5.47 (d, *J* = 6.5 *Hz,* 1H), 3.32 (s, 3H), 2.86 (s, 3H), 1.56 (d, *J* = 6.5 *Hz,* 3H). ESI-MS (m/z) 460.30 $(MH)^+$;

(R) or (S)-1-(3-Cyano-4-(2H-1,2,3-triazol-2-yl)phenyl)-3-(7-(1-methoxyethyl)-2-methylthiazolo[5,4-b]pyridin-6-yl)urea (Compound 208a) [Stereochemistry tentatively assigned, it could be either (R) or (S)]

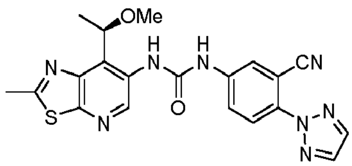

The compound 208a was prepared by using step-4 intermediate (peak-1 acid) of example-82. $^1$HNMR (400 MHz, DMSO-*d*$_6$) $\delta$ 10.36 (s, 1H), 9.11 (s, 1H), 8.65 (s, 1H), 8.24 (s, 2H), 8.22 (d, *J* = 2.5 *Hz,* 1H), 8.01 (d, *J* = 8.5 *Hz,* 1H), 7.90 (dd, *J* = 8.5, 2.5 *Hz,* 1H), 5.50 (q, *J* = 6.5 *Hz,* 1H), 3.31 (s, 3H), 2.85 (s, 3H), 1.55 (d, *J* = 6.5 *Hz,* 3H); ESI-MS (m/z) 435.2 $(MH)^+$;

(S) or (R)-1-(3-Cyano-4-(2H-1,2,3-triazol-2-yl)phenyl)-3-(7-(1-methoxyethyl)-2-methylthiazolo[5,4-b]pyridin-6-yl)urea (Compound 208b) [Stereochemistry tentatively assigned, it could be either (S) or (R)]

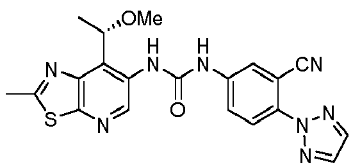

The compound 208b was prepared by using step-5 intermediate (peak-2 acid) of example-83. $^1$HNMR (400 MHz, DMSO-*d*$_6$) $\delta$ 10.37 (s, 1H), 9.10 (s, 1H), 8.64 (s, 1H), 8.24 (s, 2H), 8.22 (d, *J* = 2.5 *Hz,* 1H), 8.01 (d, *J* = 8.5 *Hz,* 1H), 7.90 (dd, *J* = 8.5, 2.5 *Hz,* 1H), 5.50 (q, *J* = 6.5 *Hz,* 1H), 3.31 (s, 3H), 2.85 (s, 3H), 1.55 (d, *J* = 6.5 *Hz,* 3H); ESI-MS (m/z) 435.2 $(MH)^+$;

(R) or (S)-1-(5-Chloro-2-methoxy-6-(1H-pyrazol-1-yl)pyridin-3-yl)-3-(7-(1-methoxyethyl)-2-methylthiazolo[5,4-b]pyridin-6-yl)urea (Compound 209a) [Stereochemistry tentatively assigned, it could be either (R) or (S)]

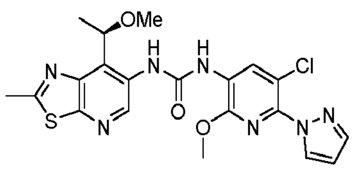

The compound 209a was prepared by using step-4 intermediate (peak-1 acid) of example-82. $^1$HNMR (400 MHz, DMSO-d$_6$) $\delta$ 9.66 (s, 1H), 8.91 (s, 1H), 8.85 (s, 1H), 8.68 (s, 1H), 8.23 (s, 1H), 7.77 (s, 1H), 6.54 (s, 1H), 5.44 (q, *J* = 5.5 Hz, 1H), 4.04 (s, 3H), 3.23 (s, 3H), 2.85 (s, 3H), 1.55 (d, *J* = 5.5 Hz, 3H); ESI-MS (m/z) 474.23 $(MH)^+$;

(S) or (R)-1-(5-Chloro-2-methoxy-6-(1H-pyrazol-1-yl)pyridin-3-yl)-3-(7-(1-methoxyethyl)-2-methylthiazolo[5,4-b]pyridin-6-yl)urea (Compound 209b) [Stereochemistry tentatively assigned, it could be either (S) or (R)]

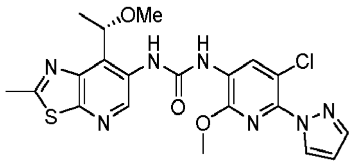

The compound 209b was prepared by using step-5 intermediate (peak-2 acid) of example-83. $^1$HNMR (400 MHz, DMSO-$d_6$) δ 9.66 (s, 1H), 8.91 (s, 1H), 8.85 (s, 1H), 8.68 (s, 1H), 8.23 (s, 1H), 7.77 (s, 1H), 6.54 (s, 1H), 5.44 (q, *J* = 5.5 Hz, 1H), 4.04 (s, 3H), 3.22 (s, 3H), 2.85 (s, 3H), 1.55 (d, *J* = 5.5 Hz, 3H); ESI-MS (m/z) 474.15 $(MH)^+$;

(R) or (S)-1-(4-(1H-Pyrazol-1-yl)-3-(trifluoromethyl)phenyl)-3-(7-(1-methoxyethyl)-2-methylthiazolo[5,4-b]pyridin-6-yl)urea (Compound 210a) [Stereochemistry tentatively assigned, it could be either (R) or (S)]

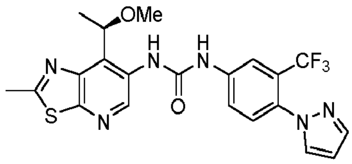

The compound 210a was prepared by using step-4 intermediate (peak-1 acid) of example-82. $^1$HNMR (400 MHz, DMSO-*d*$_6$) δ 10.32 (s, 1H), 9.12 (d, *J* = 2.5 Hz,1H), 8.61 (s, 1H), 8.13 (d, *J* = 2.5 *Hz,* 1H), 7.97 (s, 1H), 7.86 (dd, *J* = 8.5 & 2,5 *Hz,* 1H), 7.72 (d, *J* = 2.0 *Hz,* 1H), 7.53 (d, *J* = 8.5 *Hz,* 1H), 6.50 (s, 1H), 5.51 (q, *J* = 7.0 Hz, 1H),3.31 (s, 3H) 2.85 (s, 3H), 1.55 (d, *J* =7.0 Hz, 3H); ESI-MS (m/z) 477.30 $(MH)^+$;

(S) or (R)-1-(4-(1H-Pyrazol-1-yl)-3-(trifluoromethyl)phenyl)-3-(7-(1-methoxyethyl)-2-methylthiazolo[5,4-b]pyridin-6-yl)urea (Compound 210b) [Stereochemistry tentatively assigned, it could be either (S) or (R)]

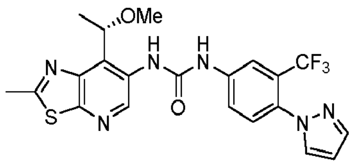

The compound 210b was prepared by using step-5 intermediate (peak-2 acid) of example-83. $^1$HNMR (400 MHz, DMSO-$d_6$) δ 10.32 (s, 1H), 9.12 (s, 1H), 8.61(s, 1H), 8.13 (d, *J* = 2.5 Hz, 1H), 7.97 (d, *J* = 2.5 Hz, 1H), 7.86 (dd, *J* = 8.5, 2.5 *Hz,* 1H), 7.72 (d, *J* = 2.0 Hz, 1H), 7.53 (d, *J* = 8.5 Hz,1H), 6.50 (t, *J* = 2.0 Hz, 1H), 5.51 (q, *J* = 6.5 Hz, 1H), 3.31 (s, 3H), 2.85 (s, 3H), 1.55 (d, *J* = 6.5 Hz, 3H); ESI-MS (m/z) 477.06 $(MH)^+$;

(S) or (R)-1-(3-Fluoro-4-(2H-1,2,3-triazol-2-yl)phenyl)-3-(7-(1-methoxyethyl)-2-methylthiazolo[5,4-b]pyridin-6-yl)urea (Compound 211) [Stereochemistry tentatively assigned, it could be either (S) or (R)]

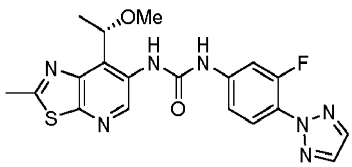

The compound 211 was prepared by using step-5 intermediate (peak-2 acid) of example-83. $^1$HNMR (400 MHz, DMSO-$d_6$) δ 10.24 (s, 1H), 9.09 (s, 1H), 8.58 (s, 1H), 8.14 (s, 2H), 7.81 (dd, *J* = 13.5, 2.5 *Hz,* 1H), 7.74 (t, *J* = 8.5 Hz, 1H), 7.37 (d, *J* = 8.5, 2.5 *Hz,* 1H), 5.50 (q, *J* = 6.5 Hz, 1H), 3.30 (s, 3H), 2.85 (s, 3H), 1.55 (d, *J* = 6.5 Hz, 3H); ESI-MS (m/z) 428.23 $(MH)^+$;

(S) or (R)-1-(5-Fluoro-6-(2H-1,2,3-triazol-2-yl)pyridin-3-yl)-3-(7-(1-methoxyethyl)-2-methylthiazolo[5,4-b]pyridin-6-yl)urea (Compound 212) [Stereochemistry tentatively assigned, it could be either (S) or (R)]

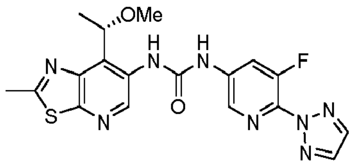

The compound 212 was prepared by using step-5 intermediate (peak-2 acid) of example-83. $^1$HNMR (400 MHz, DMSO-$d_6$) δ 10.55 (s, 1H), 9.12 (s, 1H), 8.73 (s, 1H), 8.45 (s, 1H), 8.34 (d, *J* = 12.5 Hz, 1H), 8.20 (s, 2H), 5.51 (q, *J* = 6.5 Hz, 1H), 3.32 (s, 3H), 2.86 (s, 3H), 1.56 (d, *J* = 6.5 Hz, 3H); ESI-MS (m/z) 429.16 (MH)$^+$;

(R) or (S)-1-(6-(1H-Pyrazol-1-yl)-5-(trifluoromethyl)pyridin-3-yl)-3-(7-(1-methoxyethyl)-2-methylthiazolo[5,4-b]pyridin-6-yl)urea (Compound 213a) [Stereochemistry tentatively assigned, it could be either (R) or (S)]

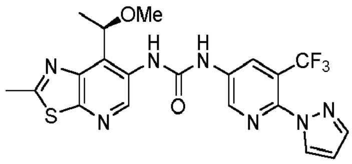

The compound 213a was prepared by using step-4 intermediate (peak-1 acid) of example-82. $^1$HNMR (400 MHz, DMSO-$d_6$) δ 10.52 (s, 1H, $D_2O$ exchangeable), 9.13 (s, 1H), 8.80 (s, 1H), 8.73 (s, 1H, $D_2O$ exchangeable), 8.66 (s, 1H), 8.26 (s, 1H), 7.79 (s, 1H), 6.56 (s, 1H), 5.55-5.46 (q, *J* = 6.5 Hz, 1H), 3.32 (s, 3H), 2.86 (s, 3H), 1.59-1.55 (d, J = 6.5 Hz, 3H); ESI-MS (m/z) 478.30 (MH)$^+$;

(S) or (R)-1-(6-(1H-Pyrazol-1-yl)-5-(trifluoromethyl)pyridin-3-yl)-3-(7-(1-methoxyethyl)-2-methylthiazolo[5,4-b]pyridin-6-yl)urea (Compound 213b) [Stereochemistry tentatively assigned, it could be either (S) or (R)]

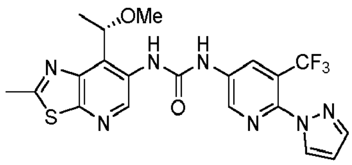

The compound 213b was prepared by using step-5 intermediate (peak-2 acid) of example-83. $^1$HNMR (400 MHz, DMSO-$d_6$) δ 10.51 (s, 1H, $D_2O$ exchangeable), 9.13 (s, 1H), 8.80 (s, 1H), 8.73 (s, 1H), 8.65 (s, 1H), 8.26 (s, 1H), 7.79 (s, 1H), 6.56 (s, 1H), 5.51 (q, *J* = 6.5 Hz, 1H), 3.32 (s, 3H), 2.85 (s, 3H), 1.56 (d, *J* = 6.5 Hz, 3H); ESI-MS (m/z) 478.14 (MH)$^+$;

(S) or (R)-1-(4-(Difluoromethoxy)-3-(trifluoromethyl)phenyl)-3-(7-(1-methoxyethyl)-2-methylthiazolo[5,4-b]pyridin-6-yl)urea (Compound 214) [Stereochemistry tentatively assigned, it could be either (S) or (R)]

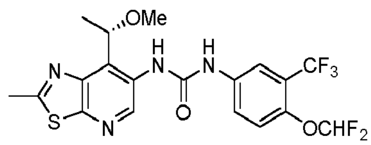

The compound 214 was prepared by using step-5 intermediate (peak-2 acid) of example-83. $^1$HNMR (400 MHz, DMSO-$d_6$) δ 10.13 (s, 1H), 9.10 (s, 1H), 8.53 (s, 1H), 8.03 (d, *J* = 2.5 *Hz,* 1H),7.77 (dd, *J* = 9.0, 2.5 *Hz,* 1H), 7.59-6.96 (m, 2H), 5.50 (q, *J* = 6.5 *Hz,* 1H), 3.30 (s, 3H), 2.85 (s, 3H),1.53 (d, *J* = 6.5 *Hz,* 3H); ESI-MS (m/z) 477.30 (MH)+;

(S) or (R) -1-(3-Chloro-4-(1H-imidazol-1-yl)phenyl)-3-(7-(1-methoxyethyl)-2-methylthiazolo[5,4-b]pyridin-6-yl)urea (Compound 215) [Stereochemistry tentatively assigned, it could be either (S) or (R)]

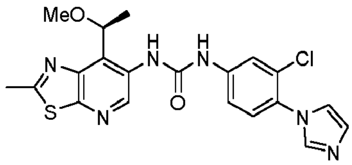

The compound 215 was prepared by using step-5 intermediate (peak-2 acid) of example-83. $^1$HNMR (400 MHz, DMSO-$d_6$) δ 10.16 (s, 1H), 9.10 (d, *J* = 2.0 *Hz,* 1H), 8.56 (s, 1H), 7.96 (s, 1H), 7.86 (s, 1H), 7.49 (s, 2H), 7.41 (s, 1H), 7.10 (s, 1H), 5.50 (q, *J* = 7.0 *Hz,* 1H), 3.30 (s, 3H), 2.85 (s, 3H), 1.54 (d, *J* = 7.0 *Hz,* 3H); ESI-MS (m/z) 443.29 $(MH)^+$;

(S) or (R)-1-(3-Chloro-5-(5-methyl-1,2,4-oxadiazol-3-yl)phenyl)-3-(7-(1-methoxyethyl)-2-methylthiazolo[5,4-b]pyridin-6-yl)urea (Compound 216) [Stereochemistry tentatively assigned, it could be either (S) or (R)]

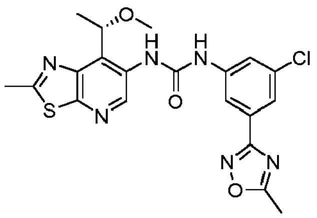

The compound 216 was prepared by using step-5 intermediate (peak-2 acid) of example-83. $^1$HNMR (400 MHz, DMSO-$d_6$) δ 10.25 (s, 1H), 9.12 (s, 1H), 8.58 (s, 1H), 8.12 (t, *J* = 1.7 Hz, 1H), 7.91 (t, *J* = 2.0 Hz, 1H), 7.59 (t, *J* = 1.7 Hz, 1H), 5.50 (q, *J* = 6.7 Hz, 1H), 3.31 (s, 3H), 2.85 (s, 3H), 2.69 (s, 3H), 1.54 (d, *J* = 6.7 Hz, 3H); ESI-MS (m/z) 459.3 $(MH)^+$;

(R) or (S) -1-(3-(2H-1,2,3-Triazol-2-yl)-5-(trifluoromethyl)phenyl)-3-(7-(1-methoxyethyl)-2-methylthiazolo[5,4-b]pyridin-6-yl)urea (Compound 217a) [Stereochemistry tentatively assigned, it could be either (R) or (S)]

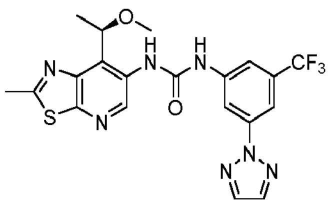

The compound 217a was prepared by using step-4 intermediate (peak-1 acid) of example-82; $^1$HNMR (400 MHz, DMSO-$d_6$) δ 10.47 (s, 1H), 9.15 (s, 1H), 8.64 (s, 1H), 8.56 (d, *J* = 2.0 Hz, 1H), 8.22 (s, 2H), 7.98 (d, *J* = 1.8 Hz, 1H), 7.89 (d, *J* = 1.8 Hz, 1H), 5.51 (q, *J* = 6.7 Hz, 1H), 3.32 (s, 3H), 2.85 (s, 3H), 1.56 (d, *J* = 6.7 Hz, 3H); ESI-MS (m/z) 478.3 $(MH)^+$;

(S) or (R) -1-(3-(2H-1,2,3-triazol-2-yl)-5-(trifluoromethyl)phenyl)-3-(7-(1-methoxyethyl)-2-methylthiazolo[5,4-b]pyridin-6-yl)urea (Compound 217b) [Stereochemistry tentatively assigned, it could be either (S) or (R)]

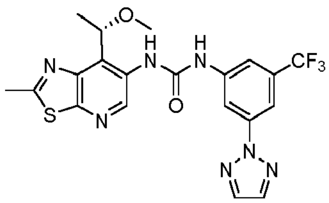

The compound 217b was prepared by using step-5 intermediate (peak-2 acid) of example-83; $^1$HNMR (400 MHz, DMSO-$d_6$) δ 10.46 (s, 1H), 9.15 (s, 1H), 8.64 (s, 1H), 8.56 (d, *J* = 2.1 Hz, 1H), 8.22 (s, 2H), 7.98 (d, *J* = 1.8 Hz, 1H), 7.89 (d, *J* = 1.8 Hz, 1H), 5.51 (q, *J* = 6.7 Hz, 1H), 3.32 (s, 3H), 2.85 (s, 3H), 1.56 (d, *J* = 6.7 Hz, 3H); ESI-MS (m/z) 478.36 $(MH)^+$;

(S) or (R)-1-(5-Chloro-6-(2-methoxyethoxy)pyridin-3-yl)-3-(7-(1-methoxyethyl)-2-methylthiazolo[5,4-b]pyridin-6-yl)urea (Compound 218) [Stereochemistry tentatively assigned, it could be either (S) or (R)]

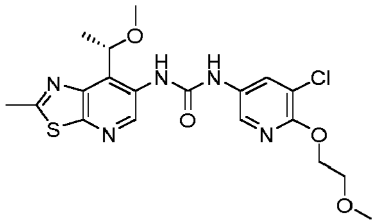

The compound 218 was prepared by using step-5 intermediate (peak-2 acid) of example-83; $^1$HNMR (400 MHz, DMSO-$d_6$) δ 9.90 (s, 1H), 9.10 (s, 1H), 8.54 (s, 1H), 8.18 (d, *J* = 2.4 Hz, 1H), 8.13 (d, *J* = 2.4 Hz, 1H), 5.48 (q, *J* = 6.7 Hz, 1H), 4.46-4.39 (m, 2H), 3.72-3.65 (m, 2H), 3.32 (s, 3H), 3.28 (s, 3H), 2.84 (s, 3H), 1.53 (d, *J* = 6.7 Hz, 3H); ESI-MS (m/z) 452.23 $(MH)^+$;

(S) or (R) -1-(5-Chloro-2-(2-methoxyethoxy)pyridin-3-yl)-3-(7-(1-methoxyethyl)-2-methylthiazolo[5,4-b]pyridin-6-yl)urea (Compound 219) [Stereochemistry tentatively assigned, it could be either (S) or (R)]

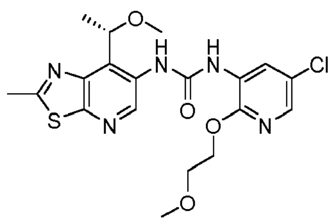

The compound 219 was prepared by using step-5 intermediate (peak-2 acid) of example-83; $^1$HNMR (400 MHz, DMSO-$d_6$) δ 9.28 (s, 1H), 8.93 (s, 1H), 8.77 (s, 1H), 8.45 (d, *J* = 2.4 Hz, 1H), 7.83 (d, *J* = 2.4 Hz, 1H), 5.40 (q, *J* = 6.7 Hz, 1H), 5.62-5.52 (m, 2H), 3.74 (t, *J* = 4.7 Hz, 2H), 3.32 (s, 3H), 3.20 (s, 3H), 2.85 (s, 3H), 1.55 (d, *J* = 6.7 Hz, 3H); ESI-MS (m/z) 451.9 $(MH)^+$;

(1S, 2S) or (1R, 2R)-1-(5-chloro-6-(2H-1,2,3-triazol-2-yl)pyridin-3-yl)-3-(7-(2-(methoxymethyl)cyclopropyl)-2-methylthiazolo[5,4-b]pyridin-6-yl)urea (Compound 220a) [Stereochemistry tentatively assigned, it could be either (1S, 2S) or (1R, 2R)]

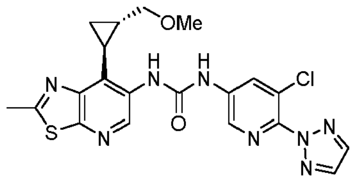

The compound 220a was prepared by using pure enantiomer-1 obtained from step-3 of example-26. $^1$HNMR (400 MHz, DMSO-$d_6$) δ 10.03 (s, 1H), 9.03 (s, 1H), 8.60 (d, *J* = 2.3 Hz, 1H), 8.55 (s, 1H), 8.48 (d, *J* = 2.3 Hz, 1H), 8.16 (s, 2H), 3.48-3.37 (m, 2H), 3.26 (s, 3H), 2.82 (s, 3H), 2.35-2.25 (m, 1H), 2.22-2.10 (m, 1H), 1.74-1.71 (m, 1H), 1.15-1.09 (m, 1H); ESI-MS (m/z) 471.0 $(MH)^+$;

(1R, 2R) or (1S, 2S)-1-(5-chloro-6-(2H-1,2,3-triazol-2-yl)pyridin-3-yl)-3-(7-((1R,2R)-2-(methoxymethyl)cyclopropyl)-2-methylthiazolo[5,4-b]pyridin-6-yl)urea (Compound 220b) [Stereochemistry tentatively assigned, it could be either (1R, 2R) or (1S, 2S)]

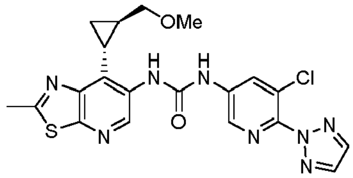

The compound 60b was prepared by using pure enantiomer-2 obtained from step-4 of example-27. $^1$HNMR (400 MHz, DMSO-d$_6$) δ 10.16 (s, 1H), 9.15 (s, 1H), 8.60 (d, *J* = 2.3 Hz, 1H), 8.54 (s, 1H), 8.48 (d, *J* = 2.3 Hz, 1H), 8.16 (s, 2H), 3.51-3.38 (m, 2H), 3.26 (s, 3H), 2.82 (s, 3H), 2.35-2.30 (m, 1H), 2.22-2.10 (m, 1H), 1.76-1.72 (m, 1H), 1.15-1.00 (m, 1H); ESI-MS (m/z) 471.0 $(MH)^+$;

(S) or (R)-1-(2-ethoxy-6-(2H-1,2,3-triazol-2-yl)-5-(trifluoromethyl)pyridin-3-yl)-3-(7-(1-methoxyethyl)-2-methylthiazolo[5,4-b]pyridin-6-yl)urea (Compound 221) [Stereochemistry tentatively assigned, it could be either (S) or (R)]

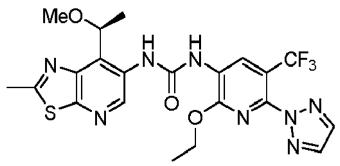

The compound 221 was prepared by using step-5 intermediate (peak-2 acid) of example-83. $^1$HNMR (400 MHz, DMSO-$d_6$) δ 9.69 (s, 1H, $D_2O$ exchangeable), 9.00 (s, 1H, $D_2O$ exchangeable), 8.98 (s, 1H), 8.81 (s, 1H), 8.16 (s, 2H), 5.43 (q, *J* = 6.5 Hz, 1H), 4.60-4.52 (q, *J* = 7.0 Hz, 2H), 3.23 (s, 3H), 2.86 (s, 3H), 1.57 (d, *J* = 6.5 Hz, 3H), 1.44 (t, *J* = 7.0 Hz, 3H); ESI-MS (m/z) 523.1$(MH)^+$; and

(S) or (R)-1-(7-(1-methoxyethyl)-2-methylthiazolo[5,4-b]pyridin-6-yl)-3-(5-(trifluoromethyl)pyridin-3-yl)urea (Compound 222) [Stereochemistry tentatively assigned, it could be either (S) or (R)]

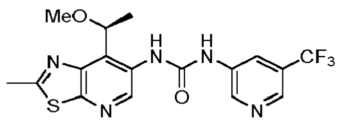

The compound 222 was prepared by using step-5 intermediate (peak-2 acid) of example-83. $^1$HNMR (400 MHz, DMSO- $d_6$) δ 10.40 (s, 1H), 9.09 (s, 1H), 8.84 (d, *J* = 2.4 Hz, 1H), 8.71 (s, 1H), 8.60 (d, *J* = 2.4 Hz, 1H), 8.47 (t, *J* = 2.4 Hz, 1H), 5.49 (d, *J* = 6.7 Hz, 1H), 3.30 (s, 3H), 2.85 (s, 3H), 1.55 (d, *J* = 6.7 Hz, 3H); ESI-MS (m/z) 412.1 (MH)+.

**Example-85:** Preparation of 1-(6-(2H-1,2,3-Triazol-2-yl)-5-(trifluoromethyl)pyridin-3-yl)-3-(7-(1-(hydroxymethyl)cyclopropyl)-2-methylthiazolo[5,4-b]pyridin-6-yl)urea (Compound 223)

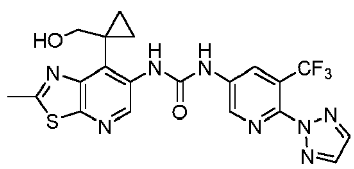

The titled compound was prepared by following the similar procedure described for example-73. $^1$HNMR (400 MHz, DMSO-$d_6$) δ 10.67 (s, 1H), 8.91 (d, *J* = 2.5 Hz, 1H), 8.88 (s, 1H), 8.74 (d, *J* = 2.5 Hz, 1H), 8.71 (s, 1H), 8.18 (s, 2H), 5.46 (t, *J* = 5.0 Hz, 1H), 3.66 (d, *J* = 5.0 Hz, 2H), 2.85 (s, 3H), 1.17-1.12 (m, 2H), 0.88-0.83 (m, 2H); ESI-MS (m/z) 491.31 $(MH)^+$.

**Example-86:** The following compounds were prepared by using the similar procedure described in example-66 from the corresponding intermediates:

(±)-1-(6-(2H -1,2,3-Triazol-2-yl)-5-(trifluoromethyl)pyridin-3-yl)-3-(7-(cyclobutyl(methoxy)methyl)-2-methylthiazolo[5,4-b]pyridin-6-yl)urea (Compound 224)

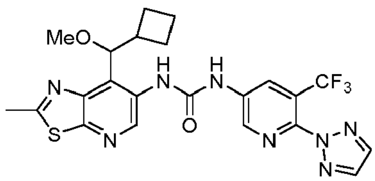

ESI-MS (m/z) 519.19 $(MH)^+$

Chiral separation of racemic compound 224 was carried out using chiral column and afforded the below isomers 224a and 224b:

1-(6-(2H-1,2,3-Triazol-2-yl)-5-(trifluoromethyl)pyridin-3-yl)-3-(7-(cyclobutyl(methoxy)methyl)-2-methylthiazolo[5,4-b]pyridin-6-yl)urea (Compound 224a)
Chiral HPLC RT = 5.09 min
$^1$HNMR (400 MHz, DMSO-$d_6$) δ 10.73 (s, 1H), 9.13 (s, 1H), 8.87 (d, $J$ = 2.5 Hz, 1H), 8.73 (d, $J$ = 2.5 Hz, 1H), 8.71 (s, 1H), 8.18 (s, 2H), 5.39 (d, $J$ = 9.0 Hz, 1H), 3.33 (s, 3H), 2.97-2.90 (m, 1H), 2.87 (s, 3H), 2.14-2.03 (m, 2H), 1.88-1.79 (m, 2H), 1.78-1.69 (m, 1H), 1.64-1.56 (m, 1H); ESI-MS (m/z) 519.19 $(MH)^+$;

1-(6-(2H-1,2,3-Triazol-2-yl)-5-(trifluoromethyl)pyridin-3-yl)-3-(7-(cyclobutyl(methoxy)methyl)-2-methylthiazolo[5,4-b]pyridin-6-yl)urea (Compound 224b)
Chiral HPLC RT: 6.23 min
$^1$HNMR (400 MHz, DMSO-$d_6$) δ 10.73 (s, 1H), 9.13 (s, 1H), 8.87 (d, $J$ = 2.5 Hz, 1H), 8.73 (d, $J$ = 2.5 Hz, 1H), 8.71 (s, 1H), 8.18 (s, 2H), 5.39 (d, $J$ = 9.0 Hz, 1H), 3.33 (s, 3H), 2.97-2.90 (m, 1H), 2.87 (s, 3H), 2.13 (s, 2H), 1.86-1.78 (m, 2H), 1.78-1.68 (m, 1H), 1.64-1.53 (m, 1H); ESI-MS (m/z) 519.19 $(MH)^+$;

(±)-1-(5-Chloro-6-(2H-1,2,3-triazol-2-yl)pyridin-3-yl)-3-(7-(cyclobutyl(methoxy)methyl)-2-methylthiazolo[5,4-b]pyridin-6-yl)urea (Compound 225)

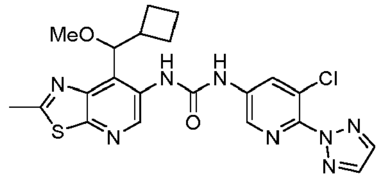

ESI-MS (m/z) 485.24 $(MH)^+$
Chiral separation of racemic compound 225 was carried out using chiral column and afforded the below isomers 225a and 225b:

1-(S-Chloro-6-(2H-1,2,3-triazol-2-yl)pyridin-3-yl)-3-(7-(cyclobutyl(methoxy)methyl)-2-methylthiazolo[5,4-b]pyridin-6-yl)urea (Compound 225a)
Chiral HPLC RT: 4.64 min
$^1$HNMR (400 MHz, DMSO-$d_6$) δ 10.57 (s, 1H), 9.11 (s, 1H), 8.66 (s, 1H), 8.56 (d, $J$ = 2.5 Hz, 1H), 8.52 (d, $J$ = 2.5 Hz, 1H), 8.17 (s, 2H), 5.38 (d, $J$ = 9.0 Hz, 1H), 3.32 (s, 3H), 2.96-2.89 (m, 1H), 2.87 (s, 3H), 2.14-2.03 (m, 2H), 1.86-1.78 (m, 2H), 1.77-1.68 (m, 1H), 1.62-1.55 (m, 1H); ESI-MS (m/z) 485.17 $(MH)^+$;

1-(5-Chloro-6-(2H-l,2,3-triazol-2-yl)pyridin-3-yl)-3-(7-(cyclobutyl(methoxy)methyl)-2-methylthiazolo[5,4-b]pyridin-6-yl)urea (Compound 225b)
Chiral HPLC RT: 5.24 min
$^1$H NMR (400 MHz, DMSO-$d_6$) δ 10.57 (s, 1H), 9.11 (s, 1H), 8.66 (s, 1H), 8.56 (d, $J$= 2.5 Hz, 1H), 8.52 (d, $J$= 2.5 Hz, 1H), 8.17 (s, 2H), 5.39 (d, $J$= 9.0 Hz, 1H), 3.32 (s, 3H), 2.96-2.88 (m, 1H), 2.87 (s, 3H), 2.16-2.03 (m, 2H), 1.88-1.77 (m, 2H), 1.77-1.68 (m, 1H), 1.63-1.54 (m, 1H); ESI-MS (m/z) 485.17 $(MH)^+$.

**Example-87:** The following compounds were prepared by the using the similar procedure described in example-71 from the corresponding intermediates.

(±)-1-(7-(Cyclopropyl(methoxy)methyl)-2-methylthiazolo[5,4-b]pyridin-6-yl)-3-(2-ethoxy-6-(2H-1,2,3-triazol-2-yl)-5-(trifluoromethyl)pyridin-3-yl)urea (Compound 226)

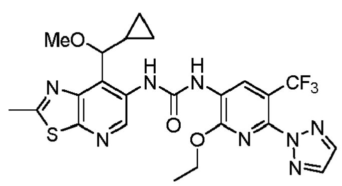

ESI-MS (m/z) 549.02 $(MH)^+$
Chiral separation of racemic compound 226 was carried out using chiral column and afforded the below isomers 226a and 226b:

1-(7-(Cyclopropyl(methoxy)methyl)-2-methylthiazolo[5,4-b]pyridin-6-yl)-3-(2-ethoxy-6-(2H-1,2,3-triazol-2-yl)-5-(trifluoromethyl)pyridin-3-yl)urea (Compound 226a) Chiral HPLC RT = 4.63 min

$^1$HNMR (400 MHz, DMSO-$d_6$) δ 9.79 (s, 1H, $D_2O$ exchangeable), 9.03 (s, 1H, $D_2O$ exchangeable), 8.98 (s, 1H), 8.84 (s, 1H), 8.16 (s, 2H), 4.60-4.52 (m, 3H), 3.27 (s, 3H), 2.84 (s, 3H), 1.53-1.40 (m, 4H), 0.72-0.63 (m, 2H), 0.41-0.25 (m, 2H); ESI-MS (m/z) 548.98 $(MH)^+$;

1-(7-(Cyclopropyl(methoxy)methyl)-2-methylthiazolo[5,4-b]pyridin-6-yl)-3-(2-ethoxy-6-(2H-1,2,3-triazol-2-yl)-5-(trifluoromethyl)pyridin-3-yl)urea (Compound 226b)

Chiral HPLC RT = 5.53 min

$^1$HNMR (400 MHz, DMSO-$d_6$) δ 9.79 (s, 1H, $D_2O$ exchangeable), 9.03 (s, 1H, $D_2O$ exchangeable), 8.98 (s, 1H), 8.84 (s, 1H), 8.16 (s, 2H), 4.61-4.51 (m, 3H), 3.27 (s, 3H), 2.84 (s, 3H), 1.53-1.40 (m, 4H), 0.75-0.60 (m, 2H), 0.39-0.26 (m, 2H); ESI-MS (m/z) 549.32 $(MH)^+$;

(±)-1-(5-Chloro-6-(2H-1,2,3-triazol-2-yl)pyridin-3-yl)-3-(7-(1-(dimethylamino)propyl)-2-methylthiazolo[5,4-b]pyridin-6-yl)urea (Compound 227)

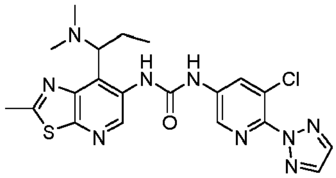

ESI-MS (m/z) 472.30 $(MH)^+$

Chiral separation of racemic compound 227 was carried out using chiral column and afforded the below isomers 227a and 227b:

1-(5-Chloro-6-(2H-1,2,3-triazol-2-yl)pyridin-3-yl)-3-(7-(1-(dimethylamino)propyl)-2-methylthiazolo[5,4-b]pyridin-6-yl)urea (Compound 227a)

Chiral HPLC RT = 7.64 min

$^1$HNMR (400 MHz, DMSO-$d_6$) δ 10.55 (s, 1H), 10.32 (s, 1H), 9.11 (s, 1H), 8.62 (d, $J$ = 2.5 Hz, 1H), 8.51 (d, $J$ = 2.5 Hz, 1H), 8.16 (s, 2H), 4.35-4.23 (m, 1H), 2.83 (s, 3H), 2.30 (s, 6H), 2.09-1.99 (m, 1H), 1.88-1.77 (m, 1H), 0.58 (t, $J$ = 7.5 Hz, 3H); ESI-MS (m/z) 471.97 $(MH)^+$;

1-(5-Chloro-6-(2H-1,2,3-triazol-2-yl)pyridin-3-yl)-3-(7-(1-(dimethylamino)propyl)-2-methylthiazolo[5,4-b]pyridin-6-yl)urea (Compound 227b)

Chiral HPLC RT = 8.83 min

$^1$HNMR (400 MHz, DMSO-$d_6$) δ 10.55 (s, 1H), 10.33 (s, 1H), 9.11 (s, 1H), 8.62 (d, $J$ = 2.5 Hz, 1H), 8.51 (d, $J$ = 2.5 Hz, 1H), 8.16 (s,2H), 4.35-4.24 (m, 1H), 2.83 (s, 3H), 2.30 (s, 6H), 2.11-1.98 (m,1H), 1.88-1.77 (m, 1H), 0.58 (t, $J$ = 7.5 Hz, 3H); ESI-MS (m/z) 471.97 $(MH)^+$;

(±)-1-(5-Chloro-6-(2H-1,2,3-triazol-2-yl)pyridin-3-yl)-3-(7-(cyclopropyl(dimethylamino)methyl)-2-methylthiazolo[5,4-b]pyridin-6-yl)urea (Compound 228)

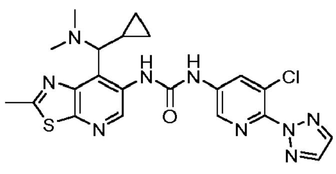

ESI-MS (m/z) 484.30 $(MH)^+$

Chiral separation of racemic compound 228 was carried out using chiral column and afforded the below isomers 228a and 228b:

1-(5-Chloro-6-(2H-1,2,3-triazol-2-yl)pyridin-3-yl)-3-(7-(cyclopropyl(dimethylamino)methyl)-2-methylthiazolo[5,4-b]pyridin-6-yl)urea (Compound 228a)

Chiral HPLC RT = 4.69 min

$^1$HNMR (400 MHz, DMSO-$d_6$) δ 10.71 (s, 1H), 10.36 (s, 1H), 9.15 (s, 1H), 8.62 (d, $J$ = 2.5 Hz, 1H), 8.52 (d, $J$

= 2.3 Hz, 1H), 8.17 (s, 2H), 3.51 (d, *J* = 9.5 Hz, 1H), 2.81 (s, 3H), 2.37 (s, 6H), 0.89-0.80 (m, 2H), 0.63-0.53 (m, 1H), 0.27-0.10 (m, 2H); ESI-MS (m/z) 484.36 [$(MH)^+$;

1-(5-Chloro-6-(2H-1,2,3-triazol-2-yl)pyridin-3-yl)-3-(7-(cyclopropyl(dimethylamino)methyl)-2-methylthiazolo[5,4-b]pyridin-6-yl)urea (Compound 228b)
Chiral HPLC RT = 5.64
$^1$HNMR (400 MHz, DMSO-$d_6$) δ 10.71 (s, 1H), 10.36 (s, 1H), 9.15 (s, 1H), 8.62 (d, *J* = 2.5 Hz, 1H), 8.52 (d, *J* = 2.5 Hz, 1H), 8.17 (s, 2H), 3.51 (d, *J* = 9.5 Hz, 1H), 2.81 (s, 3H), 2.37 (s, 6H), 0.88-0.80 (m, 2H), 0.64-0.55 (m, 1H), 0.27-0.10 (m, 2H); ESI-MS (m/z) 484.36 $(MH)^+$.

**Example-88:** The following examples were prepared by using the similar procedure described in example-32:

(±)-7-(1-(3,3-difluoroazetidin-1-yl)propyl)-2-methylthiazolo[5,4-b]pyridin-6-amine. ESI-MS (m/z) 299.34 $(MH)^+$ and

(±)-7-(1-(Dimethylamino)-2,2,2-trifluoroethyl)-2-methylthiazolo[5,4-b]pyridin-6-amine. GS-MS (m/z) 290.17$(M)^+$.

**Example-89:** The following compounds were prepared by using the similar procedure described for example-66 from the corresponding intermediates:

(±)-1-(6-(2H-1,2,3-triazol-2-yl)-5-(trifluoromethyl)pyridin-3-yl)-3-(7-(1-(3,3-difluoroazetidin-1-yl)propyl)-2-methylthiazolo[5,4-b]pyridin-6-yl)urea (Compound 229)

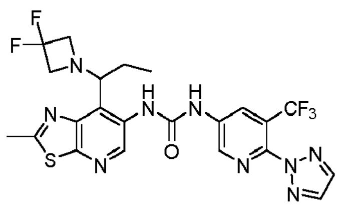

ESI-MS (m/z) 554.20 $(MH)^+$
Chiral separation of racemic compound 229 was carried out using chiral column and afforded the below isomers 229a and 229b:

1-(6-(2H-1,2,3-Triazol-2-yl)-5-(trifluoromethyl)pyridin-3-yl)-3-(7-(1-(3,3-difluoroazetidin-1-yl)propyl)-2-methylthiazolo[5,4-b]pyridin-6-yl)urea (Compound 229a)
Chiral HPLC RT = 4.71 min
$^1$HNMR (400 MHz, DMSO-$d_6$) δ 10.61 (s, 1H), 9.78 (s, 1H), 9.13 (s, 1H), 8.93 (d, *J* = 2.5 Hz, 1H), 8.73 (d, *J* = 2.5 Hz, 1H), 8.19 (s, 2H), 4.79-4.64 (m, 1H), 3.88-3.60 (m, 4H), 2.86 (s, 3H), 1.94-1.75 (m, 2H), 0.65 (t, *J* = 7.5 Hz, 3H); ESI-MS (m/z) 554.30 (MH)+;

1-(6-(2H-1,2,3-Triazol-2-yl)-5-(trifluoromethyl)pyridin-3-yl)-3-(7-(1-(3,3-difluoroazetidin-1-yl)propyl)-2-methylthiazolo[5,4-b]pyridin-6-yl)urea (Compound 229b)
Chiral HPLC RT = 6.11 min
$^1$HNMR (400 MHz, DMSO-$d_6$) δ 10.61 (s, 1H), 9.78 (s, 1H), 9.13 (s, 1H), 8.93 (d, *J* = 2.5 Hz, 1H), 8.73 (d, *J* = 2.5 Hz, 1H), 8.18 (s, 2H), 4.81-4.63 (m, 1H), 3.88-3.60 (m, 4H), 2.86 (s, 3H), 1.94-1.75 (m, 2H), 0.65 (t, *J* = 7.5 Hz, 3H); ESI-MS (m/z) 554.20 (MH)+;

(±)-1-(6-(2H-1,2,3-triazol-2-yl)-5-(trifluoromethyl)pyridin-3-yl)-3-(7-(1-(dimethylamino)-2,2,2-trifluoroethyl)-2-methylthiazolo[5,4-b]pyridin-6-yl)urea (Compound 230)

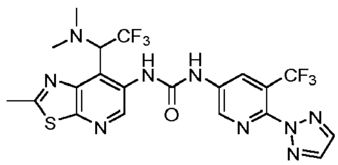

ESI-MS (m/z) 546.20 $(MH)^+$

Chiral separation of racemic compound 230 was carried out using chiral column and afforded the below isomers 230a and 230b:

1-(6-(2H-1,2,3-triazol-2-yl)-5-(trifluoromethyl)pyridin-3-yl)-3-(7-(1-(dimethylamino)-2,2,2-trifluoroethyl)-2-methylthiazolo[5,4-b]pyridin-6-yl)urea (Compound 230a)

Chiral HPLC RT = 4.34 min

$^1$HNMR (400 MHz, DMSO-$d_6$) $\delta$ 10.63 (s, 1H, $D_2O$ exchangeable), 10.00 (s, 1H, $D_2O$ exchangeable 9.14 (s, 1H), 8.92 (d, *J* = 2.5 Hz, 1H), 8.72 (d, *J* = 2.5 Hz, 1H), 8.19 (s, 2H), 5.21 (q, *J*= 8.5 Hz, 1H), 2.88 (s, 3H), 2.44 (s, 6H); ESI-MS (m/z) 546.20 $(MH)^+$;

1-(6-(2H-1,2,3-triazol-2-yl)-5-(trifluoromethyl)pyridin-3-yl)-3-(7-(1-(dimethylamino)-2,2,2-trifluoroethyl)-2-methylthiazolo[5,4-b]pyridin-6-yl)urea (Compound 230b)

Chiral HPLC RT = 5.31 min

$^1$HNMR (400 MHz, DMSO-$d_6$) $\delta$ 10.64 (s, 1H, $D_2O$ exchangeable), 10.00 (s, 1H, $D_2O$ exchangeable), 9.14 (s, 1H), 8.92 (d, *J* = 2.5 Hz, 1H), 8.72 (d, *J* = 2.5 Hz, 1H), 8.19 (s, 2H), 5.21 (q, *J* = 8.6 Hz, 1H), 2.88 (s, 3H), 2.44 (s, 6H); ESI-MS (m/z) 546.20 $(MH)^+$;

(±)-1-(5-Chloro-6-(2H-1,2,3-triazol-2-yl)pyridin-3-yl)-3-(7-(1-(dimethylamino)-2,2,2-trifluoroethyl)-2-methylthiazolo[5,4-b]pyridin-6-yl)urea (Compound 231)

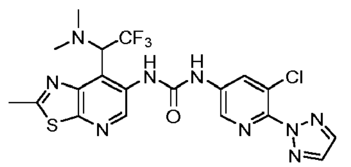

ESI-MS (m/z) 511.98 $(MH)^+$

Chiral separation of racemic compound 231 was carried out using chiral column and afforded the below isomers 231a and 231b:

1-(5-Chloro-6-(2H-1,2,3-triazol-2-yl)pyridin-3-yl)-3-(7-(1-(dimethylamino)-2,2,2-trifluoroethyl)-2-methylthiazolo[5,4-b]pyridin-6-yl)urea (Compound 231a)

Chiral HPLC RT = 6.76 min

$^1$HNMR (400 MHz, DMSO-$_{d6}$) $\delta$ 10.48 (s, 1H, $D_2O$ exchangeable), 9.93 (s, 1H, $D_2O$ exchangeable), 9.12 (s, 1H), 8.60 (d, *J* = 2.5 Hz, 1H), 8.51 (d, *J* = 2.5 Hz, 1H), 8.17 (s, 2H), 5.20 (q, *J*= 8.5 Hz, 1H), 2.88 (s, 3H), 2.43 (s, 6H); ESI-MS (m/z) 512.00 $(MH)^+$;

1-(5-Chloro-6-(2H-1,2,3-triazol-2-yl)pyridin-3-yl)-3-(7-(1-(dimethylamino)-2,2,2-trifluoroethyl)-2-methylthiazolo[5,4-b]pyridin-6-yl)urea (Compound 231b)

Chiral HPLC RT = 8.57 min

$^1$HNMR (400 MHz, DMSO-$d_6$) $\delta$ 10.48 (s, 1H, $D_2O$ exchangeable), 9.93 (s, 1H, $D_2O$ exchangeable), 9.12 (s, 1H), 8.60 (d, *J* = 2.5 Hz, 1H), 8.51 (d, *J* = 2.5 Hz, 1H), 8.17 (s, 2H), 5.20 (q, *J* = 8.5 Hz, 1H), 2.88 (s, 3H), 2.43 (s, 6H); ESI-MS (m/z) 511.99 $(MH)^+$.

**Example-90:** Preparation of (±)-2-ethyl-7-(l-methoxyethyl)thiazolo[5,4-b]pyridine-6-carboxylic acid

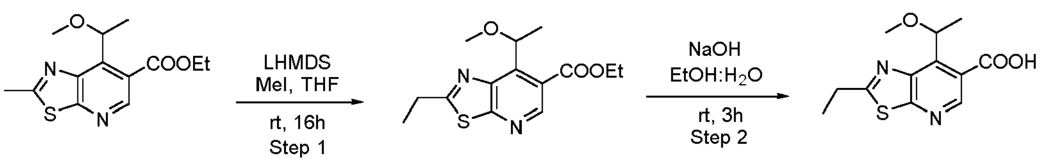


Step-1: Ethyl 2-ethyl-7-(1-methoxyethyl)thiazolo[5,4-b]pyridine-6-carboxylate: To a (-78 °c) cooled and stirred solution of ethyl 7-(1-methoxyethyl)-2-methylthiazolo[5,4-b]pyridine-6-carboxylate (600 mg, 2.140 mmol) in THF (20 mL)

was added lithium bis(trimethylsilyl)amide (3.21 mL, 3.21 mmol) drop wise and then stirred at the same temperature for 30 min. Methyl iodide (0.294 mL, 4.71 mmol) was then added to the above reaction mixture and stirred at the same temperature for 2h and then at rt for 16h. The reaction mixture was diluted with saturated aqueous ammonium chloride solution (20 mL) followed by water (20 mL) and ethyl acetate (50 mL). The layers were separated and the aqueous layer was extracted with ethyl aceate (25 mL x 3). The combined organic layera were washed with brine (50 mL), dried ($Na_2SO_4$) and filtered. The filtrate was rotary evaporated and the crude product was purified by flash column chromatography (silica gel) to afford (550 mg, 87%) of the titled compound. $^1$HNMR (400 MHz, DMSO-$d_6$) δ 8.60 (s, 1H), 5.26 (q, *J* = 6.5 Hz, 1H), 4.37 (q , *J* = 6.5 Hz, 2H) , 3.20 (q, *J* = 7.5 Hz, 2H), 3.14 (s, 3H), 1.60 (d, *J* = 6.5 Hz, 3H), 1.40 (t, *J* = 7.5 Hz, 3H), 1.33 (t, *J* = 7.0 Hz, 3H); ESI-MS (m/z) 295.1 $(MH)^+$.

Step-2: 2-Ethyl-7-(1-methoxyethyl)thiazolo[5,4-b]pyridine-6-carboxylic acid: To a stirred solution of ethyl 7-(1-methoxyethyl)-2-methylthiazolo[5,4-b]pyridine-6-carboxylate (550 mg, 1.96 mmol) in ethanol (15 mL) was added a solution of NaOH (157 mg, 3.92 mmol) dissolved in water (3mL) and stirred at room temperature for 3h. The solvent was rotary evaporated and the residue was diluted with water (3 mL), acidifed with aqueous hydrochloric acid solution (10%) and the precipitated solid was filltered off and dried to afford (500 mg, 96%) of the titled compound as white solid. $^1$HNMR (400 MHz, DMSO-$d_6$) δ 13.36 (s, 1H), 8.63 (s, 1H), 5.31 (q, *J* = 6.5 Hz, 1H), 3.19 (q, *J* = 7.5 Hz, 2H), 3.14 (s, 3H), 1.62 (d, *J* = 6.5 Hz, 3H), 1.40 (t, *J* = 7.5 Hz, 3H); ESI-MS (m/z) 267.21 $(MH)^+$.

**Example-91:** The following compounds were prepared by using the similar procedure described in example-80 from the corresponding intermediates:

(±)-1-(6-(2H-1,2,3-Triazol-2-yl)-5-(trifluoromethyl)pyridin-3-yl)-3-(2-ethyl-7 -(1-methoxyethyl)thiazolo[5,4-b]pyridin-6-yl)urea (Compound 232)

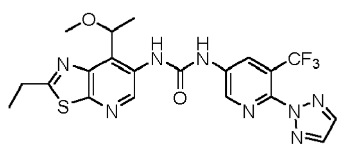

ESI-MS (m/z) 493.30 $(MH)^+$

Chiral separation of racemic compound 232 was carried out using chiral column and afforded the below isomers 232a and 232b:

1-(6-(2H-1,2,3-Triazol-2-yl)-5-(trifluoromethyl)pyridin-3-yl)-3-(2-ethyl-7-(1-methoxyethyl)thiazolo[5,4-b]pyridin-6-yl)urea (Compound 232a)

Chiral HPLC RT = 5.35 min

$^1$HNMR (400 MHz, DMSO-$d_6$) δ 10.69 (s, 1H), 9.14 (s, 1H), 8.86 (d, *J* = 2.5 Hz, 1H), 8.79 (s, 1H), 8.73 (d, *J* = 2.5 Hz, 1H), 8.18 (s, 2H), 5.52 (q, *J* = 6.5 Hz, 1H), 3.33 (s, 3H), 3.17 (q, *J* = 7.5 Hz, 2H), 1.57 (d, *J* = 6.5 Hz, 3H), 1.40 (t, *J* = 7.5 Hz, 3H); ESI-MS (m/z) 493.30 $(MH)^+$;

1-(6-(2H-1,2,3-Triazol-2-yl)-5-(trifluoromethyl)pyridin-3-yl)-3-(2-ethyl-7-(1-methoxyethyl)thiazolo[5,4-b]pyridin-6-yl)urea (Compound 232b)

Chiral HPLC RT = 5.98 min

$^1$HNMR (400 MHz, DMSO-$d_6$) δ 10.69 (s, 1H), 9.14 (s, 1H), 8.86 (d, *J*= 2.5 Hz, 1H), 8.79 (s, 1H), 8.73 (d, *J* = 2.5 Hz, 1H), 8.18 (s, 2H), 5.52 (q, *J* = 6.5 Hz, 1H), 3.33 (s, 3H), 3.17 (q, *J* = 7.5 Hz, 2H), 1.57 (d, *J* = 6.5 Hz, 3H), 1.40 (t, *J* = 7.5 Hz, 3H); ESI-MS (m/z) 493.30 $(MH)^+$;

(±)-1-(3-Chloro-4-(2H-1,2,3-triazol-2-yl)phenyl)-3-(2-ethyl-7-(1-methoxyethyl)thiazolo[5,4-b]pyridin-6-yl)urea (Compound 233)

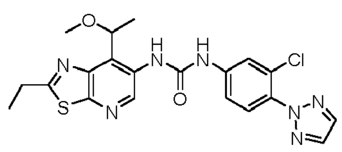

ESI-MS (m/z) 458.04 $(MH)^+$

Chiral separation of racemic compound 233 was carried out using chiral column and afforded the below isomers 233a and 233b:

1-(3-Chloro-4-(2H-1,2,3-triazol-2-yl)phenyl)-3-(2-ethyl-7-(1-methoxyethyl)thiazolo[5,4-b]pyridin-6-yl)urea (Compound 233a)
Chiral HPLC RT = 6.60 min
$^1$HNMR (400 MHz, DMSO-$d_6$) δ 10.24 (s, 1H), 9.11 (s, 1H), 8.60 (s,1H), 8.12 (s, 2H), 7.99 (d, $J$ = 2.5 Hz, 1H), 7.61 (d, $J$= 8.5 Hz, 1H),7.55 (dd, $J$ = 8.5, 2.5 Hz, 1H), 5.51 (q, $J$ = 6.5 Hz, 1H), 3.31 (s, 3H), 3.17 (q, $J$ = 7.5 Hz, 2H), 1.55 (d, $J$ = 6.5 Hz, 3H), 1.39 (t, $J$ = 7.5 Hz, 3H); ESI-MS (m/z) 458.2 $(MH)^+$;

1-(3-Chloro-4-(2H-1,2,3-triazol-2-yl)phenyl)-3-(2-ethyl-7-(1-methoxyethyl)thiazolo[5,4-b]pyridin-6-yl)urea (Compound 233b)
Chiral HPLC RT = 8.21 min
$^1$HNMR (400 MHz, DMSO-$d_6$) δ 10.24 (s, 1H), 9.11 (s, 1H), 8.60 (s, 1H), 8.12 (s, 2H), 7.99 (d, $J$ = 2.5 Hz, 1H), 7.61 (d, $J$ = 8.5 Hz, 1H), 7.55 (dd, $J$ = 8.5, 2.5 Hz, 1H), 5.51 (q, $J$ = 6.5 Hz, 1H), 3.31 (s, 3H), 3.17 (q, $J$ = 7.5 Hz, 2H),1.56 (d, $J$ = 6.5 Hz, 3H), 1.39 (t, $J$ = 7.5 Hz, 3H). ESI-MS (m/z) 458.4 $(MH)^+$.

**Example-92:** Preparation of N2-(thiazol-2-yl)-3-(trifluoromethyl) pyridine-2,5-diamine.

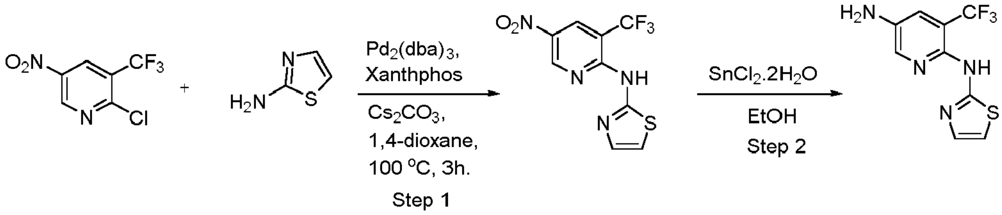


Step-1: N-(5-Nitro-3-(trifluoromethyl) pyridin-2-yl) thiazol-2-amine: To a stirred solution of 2-chloro-5-nitro-3-(trifluoromethyl)pyridine (2.0 g, 8.83 mmol) in 1,4-dioxane (20 mL) was added, cesium carbonate (5.75 g, 17.66 mmol) and the contents were purged with nitrogen for 30 min followed by sequential addition of thiazol-2-amine (1.32 g, 13.24 mmol), xantphos (0.511 g, 0.883 mmol) and $Pd_2(dba)_3$ (0.808 g, 0.883 mmol). The resulting reaction mixture was heated at 100°C for 3 h. After completion of the reaction, the reaction mixture was filtered through celite. The filtrate was rotary evaporated and residue was purified by flash column chromatography (silica gel) to afford 0.850 g (33%) of the titled product as a colorless gum. $^1$HNMR (400 MHz, DMSO-$d_6$) δ 13.32 (s, 1H), 9.32 (d, $J$ = 2.7 Hz, 1H), 8.50 (d, $J$ = 2.7 Hz, 1H), 7.50 (d, $J$ = 4.6 Hz, 1H), 7.12 (d, $J$ = 4.6 Hz, 1H); ESI-MS (m/z) 291.21 $(MH)^+$.

Step-2: N2-(thiazol-2-yl)-3-(trifluoromethyl) pyridine-2, 5-diamine: To a stirred solution of N-(5-nitro-3-(trifluoromethyl)pyridin-2-yl)thiazol-2-amine (0.3 g, 1.03 mmol) in ethanol (10 mL) was added $SnCl_2.2H_2O$ (0.933 g, 4.13 mmol) at 25°C. The resulting reaction mixture was heated at reflux temperature for 2 h. After completion of the reaction, the reaction mixture was concentrated under vacuum, diluted with water (10 mL) and basified with 10% NaOH. Aqueous phase was extracted with ethyl acetate (20 mL x 3). The combined organic layers were dried ($Na_2SO_4$)and filtered. The filtrate was rotary evaporated and residue was purified by flash column chromatography (silica gel) to afford 0.120 g (45%) of the titled product. $^1$HNMR (400 MHz, DMSO-$d_6$) δ 7.97 (d, $J$ = 2.1 Hz, 1H), 7.33 (d, $J$ = 2.5 Hz, 1H, overlap with s, 1H), 7.13 (s, 1H), 6.60 (s, 1H), 5.25 (s, 2H); ESI-MS (m/z) 261.15 $(MH)^+$.

**Example-93:** Preparation of N-(5-amino-3-(trifluoromethyl) pyridin-2-yl) acetamide.

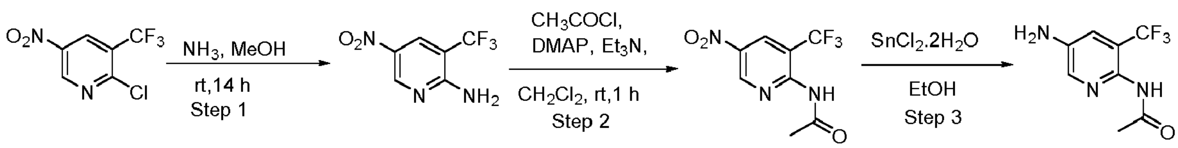


Step-1: 5-Nitro-3-(trifluoromethyl) pyridin-2-amine: A solution of 2-chloro-5-nitro-3-(trifluoromethyl) pyridine (15 g, 66.2 mmol) and ammonia solution in MeOH (7 N, 150 mL, 1.05 mol) was stirred at room temperature for 14 h. After completion of the reaction, reaction mixture was concentrated under vacuum and residue was diluted with water (100 mL) and aqueous phase was extracted with ethyl acetate (100 mL x 3), combined organic layer was dried over anhydrous sodium sulphate and filtered. The filtrate was rotary evaporated to afford 11 g (80%) of the titled product as yellow solid.

$^{1}$HNMR (400 MHz, DMSO-$d_6$) δ 9.06 (d, *J* = 2.7 Hz, 1H), 8.40 (d, *J* = 2.7 Hz, 1H), 8.04 (s, 2H); ESI-MS (m/z) 208.33 $(MH)^+$.

Step-2: N-(5-Nitro-3-(trifluoromethyl) pyridin-2-yl) acetamide: To a stirred solution of 5-nitro-3-(trifluoromethyl)pyridin-2-amine (2 g, 9.66 mmol) in dichloromethane (20 mL) was added DMAP (1.29 g, 10.62 mmol), $Et_3N$ (2.69 mL, 19.31 mmol) and acetyl chloride (0.758 mL, 10.62 mmol) at room temperature and the resulting reaction mixture was stirred for 1 h. After completion of the reaction, reaction mixture was neutralized with aqueous (1M) solution of potassium carbonate. Aqueous phase was extracted with ethyl acetate (20 mL x 3), combined organic layer was dried over anhydrous sodium sulphate and filtered. The filtrate was rotary evaporated and residue was purified by flash column chromatography (silica gel) to afford 1.1 g (46%) of the titled product as yellow solid. $^{1}$HNMR (400 MHz, DMSO-$d_6$) δ 10.73 (s, 1H), 9.47 (d, *J* = 2.6 Hz, 1H), 8.84 (d, *J* = 2.6 Hz, 1H), 2.14 (s, 3H); ESI-MS (m/z) 249.80 $(MH)^+$.

Step-3: N-(5-Amino-3-(trifluoromethyl) pyridin-2-yl) acetamide: To a stirred solution of N-(5-nitro-3-(trifluoromethyl)pyridin-2-yl)acetamide (0.7 g, 2.81 mmol) in ethanol (7 mL) was added $SnCl_2.2H_2O$ (2.134 g, 11.24 mmol) at 25°C. The resulting reaction mixture was heated at 25°C for 2 h. After completion of the reaction, the reaction mixture was concentrated under vacuum, diluted with water (10 mL) and basified with 10% NaOH. Aqueous phase was extracted with ethyl acetate (20 mL x 3), combined organic layer was dried over anhydrous sodium sulphate and filtered. The filtrate was rotary evaporated and residue was purified by flash column chromatography (silica gel) to afford 300 mg (49%) of the titled product. $^{1}$HNMR (400 MHz, DMSO-$d_6$) δ 9.65 (s, 1H), 8.00 (d, *J* = 2.8 Hz, 1H), 7.26 (d, *J* = 2.8 Hz, 1H), 5.83 (s, 2H), 1.92 (s, 3H); ESI-MS (m/z) 220.20 $(MH)^+$.

**Example-94:** Preparation of (*R*)-N2-(1-methoxypropan-2-yl)-N2-methyl-3-(trifluoromethyl) pyridine-2, 5-diamine.

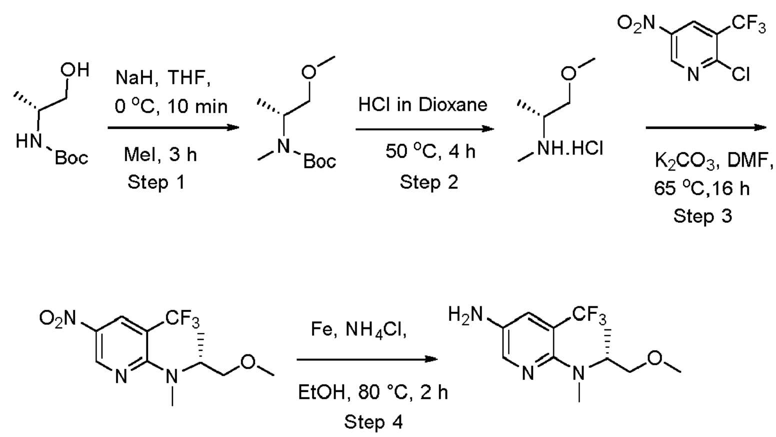


Step-1: tert-Butyl (R)-(1-methoxypropan-2-yl) (methyl) carbamate: To a stirred solution of (*R*)-tert-butyl (1-hydroxypropan-2-yl) carbamate (3.00 g, 17.12 mmol) in THF (30 mL) was added NaH (60% in mineral oil) (2.74 g, 68.5 mmol) portionwise at 0°C. The resulting reaction mixture was stirred at 25°C for 10 min. MeI (4.28 ml, 68.5 mmol) was added dropwise to the reaction mixture and the reaction was allowed to stir at 25°C for 3 h. After completion of the reaction, reaction mixture was quenched by drop wise addition of water (40 mL) and aqueous phase was extracted with ethyl acetate (50 mL x 3), combined organic layer was dried over anhydrous sodium sulphate and filtered. The filtrate was rotary evaporated and residue was purified by flash column chromatography (silica gel) to afford to afford 2.0 g (57%) of the titled product. $^{1}$HNMR (400 MHz, DMSO-$d_6$) δ 4.34-4.10 (m, 1H), 3.39-3.33 (m, 1H), 3.24 (s, 3H), 3.23-3.19 (m, 1H), 2.64 (s, 3H), 1.40 (s, 9H), 1.00 (d, *J* = 6.8 Hz, 3H); ESI-MS (m/z) 204.92 $(MH)^+$.

Step-2: (R)-1-Methoxy-N-methylpropan-2-amine hydrochloride: To a stirred solution of (R)-tert-butyl (1-methoxypropan-2-yl)(methyl)carbamate (2.0 g, 9.84 mmol) in 1,4-dioxane (10 mL), HC1 solution (4 M in 1,4-Dioxane, 24.60 mL, 98 mmol) was added and the resulting mixture was stirred at 50°C for 4 h. After completion of the reaction as monitored on TLC, reaction mass was concentrated under vacuum and co-distilled with toluene to afford 1.0 g (73%) of (R)-1-methoxy-N-methylpropan-2-amine as hydrochloride salt. $^{1}$HNMR (400 MHz, DMSO-$d_6$) δ 9.14 (s, 1H), 8.96 (s, 1H), 3.56-3.46 (m, 2H), 3.32-3.31 (m, 3H), 3.30 (s, 1H), 2.51 (s, 3H), 1.20 (d, *J* = 6.7 Hz, 3H).

Step-3: (*R*)-N-(1-Methoxypropan-2-yl)-N-methyl-5-nitro-3-(trifluoromethyl)pyridin-2-amine: To a solution of (R)-1-methoxy-N-methylpropan-2-amine hydrochloride (1.017 g, 7.28 mmol) in DMF (10 mL) was added $K_2CO_3$ (3.02 g, 21.85 mmol) and 2-chloro-5-nitro-3-(trifluoromethyl) pyridine (1.650 g, 7.28 mmol). The resulting reaction mixture was stirred at 65°C for 16 h. After completion of the reaction as monitored on TLC, reaction mixture was quenched with water (20 mL) and aqueous phase was extracted with ethyl acetate (20 mL x 3). The combined organic layer was dried over anhydrous sodium sulphate and filtered. The filtrate was rotary evaporated and residue was purified by flash column

chromatography (silica gel) to afford to afford 1.0 g (47%) of the titled product. $^1$HNMR (400 MHz, DMSO-d$_6$) δ 9.10 (d, *J* = 2.6 Hz, 1H), 8.59 (d, *J* = 2.6 Hz, 1H), 4.90-4.80 (m, 1H), 3.58 (dd, *J* = 10.5, 8.7 Hz, 1H), 3.40 (dd, *J* = 10.4, 5.0 Hz, 1H), 3.22 (s, 3H), 3.01 (s, 3H), 1.20 (d, *J*= 6.8 Hz, 3H); ESI-MS (m/z) 293.93 (MH)$^+$.

Step-4: (*R*)-N2-(1-Methoxypropan-2-yl)-N2-methyl-3-(trifluoromethyl) pyridine-2, 5-diamine: To a stirred solution of (*R*)-N-(1-methoxypropan-2-yl)-N-methyl-5-nitro-3-(trifluoromethyl)pyridin-2-amine (1.0 g, 3.41 mmol) in a ethanol:water (5:1; 24 mL), were added iron powder (1.90 g, 34.1 mmol) and $NH_4Cl$ (1.824 g, 34.1 mmol). The resulting reaction mixture was stirred at 80°C for 2h. Progress of the reaction was monitored by TLC. After completion of the reaction, reaction mixture was filtered through celite pad, washed with ethyl acetate (100 mL) and the combined filtrate was concentrated to afford 0.5 g, (58%) of the titled product. $^1$HNMR (400 MHz, DMSO-*d*$_6$) δ 7.96 (d, *J* = 2.8 Hz, 1H), 7.21 (d, *J* = 2.8 Hz, 1H), 5.51 (s, 2H), 3.45-3.41 (m, 1H), 3.33 (s, 3H), 3.30-3.25 (m, 2H), 2.53 (s, 3H), 1.00 (d, *J* = 6.3 Hz, 3H); ESI-MS (m/z) 264.21 (MH)$^+$.

**Example-95:** 6-(Methoxymethyl)-5-(trifluoromethyl)pyridin-3-amine

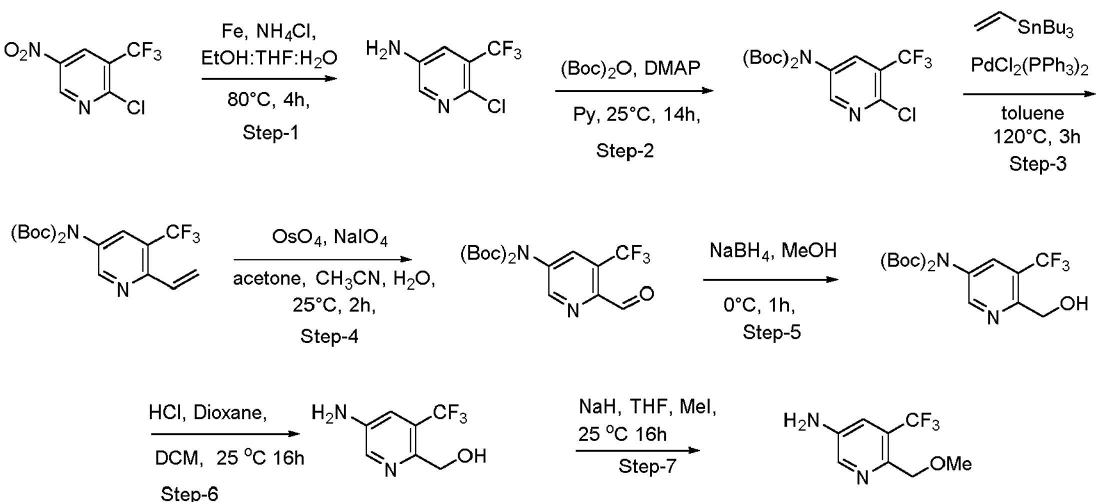

6-(Methoxymethyl)-5-(trifluoromethyl)pyridin-3-amine

Step-1: 6-Chloro-5-(trifluoromethyl)pyridin-3-amine: To a solution of 2-chloro-5-nitro-3-(trifluoromethyl)pyridine (10.0 g, 44.1 mmol) in ethanol:water:THF (2:2:1, 150 mL) were added, $NH_4Cl$ (16.53 g, 309 mmol) and iron powder (17.26 g, 309 mmol). The resulting reaction mixture was stirred at 80°C for 4 h. After completion of the reaction, the reaction mixture was cooled to room temprature, filtered over celite bed and filtrate bed was washed with ethyl acteate (200 mL). Combined filtrate was concentrated to afford 8.0 g (92%) of the titled product as a brown solid. $^1$H NMR (400 MHz, DMSO-d$_6$) δ 7.93 (d, *J* = 2.7 Hz, 1H), 7.39 (d, *J* = 2.8 Hz, 1H), 6.02 (s, 2H); ESI-MS (m/z) 197.26 (MH)$^+$.

Step-2: 6-Chloro-5-(trifluoromethyl)pyridin-3-(di-tert-butyloxycarbonyl)amine: To a stirred solution of 6-chloro-5-(trifluoromethyl)pyridin-3-amine (7.00 g, 35.6 mmol) in pyridine (70 mL) were added, DMAP (0.218 g, 1.781 mmol) and di-tert-butyl dicarbonate (12.40 mL, 53.4 mmol) dropwise. Resulting reaction mixture was stirred at RT for the 14h. After completion of the reaction, toluene (20 mL) was added and reaction mixture was concentared under vaccum. Residue thus obtained was purified by flash column chromatography (silica gel) to afford 8.0 g (56%) of the titled product as a colorless gum. $^1$HNMR (400 MHz, DMSO-*d*$_6$) δ 8.73 (d, *J* = 2.5 Hz, 1H), 8.56 (d, *J* = 2.5 Hz, 1H), 1.39 (s, 18H); ESI-MS (m/z) 396.99 (MH)$^+$.

Step-3: 5-(Trifluoromethyl)-6-vinylpyridin-3-(di-tert-butyloxycarbonyl)amine: To a stirred solution of 6-chloro-5-(trifluoromethyl)pyridin-3-(di-tert-butyloxycarbonyl)amine (8.0 g, 20.16 mmol) in toluene (80 mL) were added, tributyl(vinyl)stannane (12.79 g, 40.3 mmol) and $PdCl_2(PPh_3)_2$ (1.415 g, 2.016 mmol) under nitrogen. The reaction mixture was heated at 120°C for 3h . After completion of the reaction, the reaction mixture was evaporated on rotary evaporated and the crude product was purified by by flash column chromatography (silica gel) to afford 6 g (77%) of the titled product as pale yellow solid. $^1$HNMR (400 MHz, DMSO-d$_6$) δ 8.77 (d, *J* = 2.4 Hz, 1H), 8.25 (d, *J* = 2.4 Hz, 1H), 7.11-6.97 (m, 1H), 6.58 (dd, *J* = 16.6, 2.2 Hz, 1H), 5.77 (dd, *J* = 10.6, 2.2 Hz, 1H), 1.39 (s, 18H).

Step-4: 5-(Di-tert-butyloxycarbonyl)amino-3-(trifluoromethyl)picolinaldehyde (5)

To a stirred solution of 5-(trifluoromethyl)-6-vinylpyridin-3-(di-tert-butyloxycarbonyl )amine (6.0 g, 15.45 mmol) in acetone: $CH_3CN$, $H_2O$(1:1:1) (180 mL), was added $NaIO_4$ (9.91 g, 46.3 mmol) and $OsO_4$ (0.39 g, 1.54 mmol). The reaction mixture was stirred at 25°C for 2h. After completion of the reaction, quenched with water (100 mL) and aqueous phase was extracted with ethyl acetate (50 mL×3), combined organic layer was dried over anhydrous sodium sulphate and filtered. The filtrate was rotary evaporated and residue was purified by flash column chromatography (silica gel) to afford 5.5 g (92%) of the titled product as a colorless gum. $^1$HNMR (400 MHz, DMSO-$d_6$) δ 10.08 (s, 1H), 9.06 (d, *J* = 2.2 Hz, 1H), 8.56 (d, *J* = 2.2 Hz, 1H), 1.40 (s, 18H); ESI-MS (m/z) 390.94 $(MH)^+$.

Step-5: (5-(Di-tert-butyloxycarbonyl)amino-3-(trifluoromethyl)pyridin-2-yl)methanol: To a solution of 5-(di-tert-butyloxycarbonyl)amino-3-(trifluoromethyl)picolinaldehyde (5.5 g, 14.09 mmol) in methanol (70 mL) was added in $NaBH_4$ (0.800 g, 21.13 mmol) at 0°C and the mixture was stirred for 0°C for 1 h. After completion of the reaction, quenched with water (100 mL) and aqueous phase was extracted with ethyl acetate (50 mL×3), combined organic layer was dried over anhydrous sodium sulphate and filtered. The filtrate was rotary evaporated and residue was purified by flash column chromatography (silica gel) to afford 4.5 g (81%) of the titled product as a colorless gum. $^1$HNMR (400 MHz, DMSO-$d_6$) δ 8.75 (d, *J* = 2.3 Hz, 1H), 8.24 (d, *J* = 2.3 Hz, 1H), 5.46 (t, *J* = 6.0 Hz, 1H), 4.69 (dd, *J* = 6.0, 1.3 Hz, 2H), 1.39 (s, 18H); ESI-MS (m/z) 393.04 $(MH)^+$.

Step-6: (5-Amino-3-(trifluoromethyl)pyridin-2-yl)methanol: To a solution of (5-(di-tert-butyloxycarbonyl)amino-3-(trifluoromethyl)pyridin-2-yl)methanol (600 mg, 1.52 mmol) in dichloromethane (10 mL) was added HCl (4M in dioxane, 6.09 mL, 24.36 mmol) and the mixture was stirred for 25°C for 16 h. After completion of the reaction, quenched with sat. $NaHCO_3$ (100 mL) and aqueous phase was extracted with ethyl acetate (50 mL×3), combined organic layer was dried over anhydrous sodium sulphate and filtered. The filtrate was rotary evaporated and residue was purified by flash column chromatography (silica gel) to afford 200 mg (85%) of the titled product. $^1$HNMR (400 MHz, DMSO-$d_6$) δ 8.11 (d, *J* = 2.7 Hz, 1H), 7.21 (d, *J* = 2.6 Hz, 1H), 5.78 (s, 2H), 5.01 (t, *J* = 5.7 Hz, 1H), 4.48 (dd, *J* = 5.8, 1.3 Hz, 2H); ESI-MS (m/z) 193.26 (MH)+.

Step-7: 6-(Methoxymethyl)-5-(trifluoromethyl)pyridin-3-amine: To a solution of (5-amino-3-(trifluoromethyl)pyridin-2-yl)methanol (192 mg, 1.00 mmol) in THF (10 mL) was added NaH(60% in mineral oil, 48 mg, 1.1 mmol) and the mixture was stirred for 0°C for 10 min. Iodomethane (0.068 ml, 1.093 mmol) was added to the reaction mixture and reaction was allowed to stir for 16 h. After completion, the reaction was quenched with sat. $NH_4Cl$ (10 mL) and aqueous phase was extracted with ethyl acetate (10 mL x 3), combined organic layer was dried over anhydrous sodium sulphate and filtered. The filtrate was rotary evaporated and residue was purified by flash column chromatography (silica gel) to afford 150 mg (73%) of the titled product. $^1$HNMR (400 MHz, DMSO-$d_6$) δ 8.09 (d, *J* = 2.6 Hz, 1H), 7.22 (d, *J* = 2.6 Hz, 1H), 5.89 (s, 2H), 4.40 (s, 2H), 3.24 (s, 3H); ESI-MS (m/z) 207.01$(MH)^+$.

**Example-96:** The following compounds were prepared by using the similar procedure described in example-83 from the corresponding intermediates:

1-(6-((S)-2-Aminopropoxy)-5-(trifluoromethyl)pyridin-3-yl)-3-(7-((S or R)-1-methoxyethyl)-2-methylthiazolo[5,4-b]pyridin-6-yl)urea hydrochloride (Compound 234) [Stereochemistry tentatively assigned, it could be either (S) or (R)].

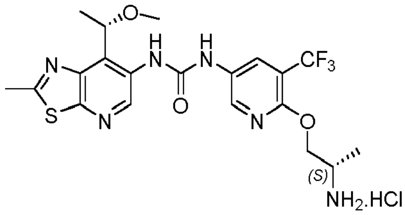

The compound 234 was prepared by using step-5 intermediate (peak-2 acid) of example-83. $^1$HNMR (400 MHz, DMSO-$d_6$) δ 10.25 (s, 1H), 9.09 (s, 1H), 8.64 (s, 1H), 8.50 (d, *J* = 2.6 Hz, 1H), 8.38 (d, *J* = 2.6 Hz, 1H), 8.16 (s, 3H), 5.49 (q, *J* = 6.7 Hz, 1H), 4.49 (dd, *J* = 11.1, 5.7 Hz, 1H), 4.42 (dd, *J* = 11.1, 5.7 Hz, 1H), 3.70-3.58 (m, 1H), 3.30 (s, 3H), 2.84 (s, 3H), 1.54 (d, *J* = 6.7 Hz, 3H), 1.31 (d, *J* = 6.6 Hz, 3H). ESI-MS (m/z) 485.4 $(MH)^+$ (free base);

1-(7-((S or R)-1-Methoxyethyl)-2-methylthiazolo[5,4-b]pyridin-6-yl)-3-(6-(((R)-1-methoxypropan-2-yl)(methyl)amino)-5-(trifluoromethyl)pyridin-3-yl)urea (Compound 235) [Stereochemistry tentatively assigned, it could be either (S) or (R)].

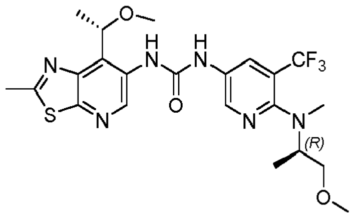

The compound 235 was prepared by using step-5 intermediate (peak-2 acid) of example-83. $^1$HNMR (400 MHz, DMSO-$d_6$) δ 10.00 (s, 1H), 9.11 (s, 1H), 8.55 (s, 1H), 8.50 (d, *J* = 2.5 Hz, 1H), 8.29 (d, *J* = 2.5 Hz, 1H), 5.53-5.46 (m, 1H), 3.80 (q, *J* = 6.6 Hz, 1H), 3.49 (dd, *J* = 9.6, 6.1 Hz, 1H), 3.33-3.30 (m, 1H), 3.29 (s, 3H), 3.19 (s, 3H), 2.84 (s, 3H), 2.73 (s, 3H), 1.54 (d, *J* = 6.6 Hz, 3H), 1.12 (d, *J* = 6.6 Hz, 3H); ESI-MS (m/z) 513.2 (MH)$^+$;

(S or R)-1-(7-(1-Methoxyethyl)-2-methylthiazolo[5,4-b]pyridin-6-yl)-3-(6-(thiazol-2-ylamino)-5-(trifluoromethyl)pyridin-3-yl)urea (Compound 236) [Stereochemistry tentatively assigned, it could be either (S) or (R)]

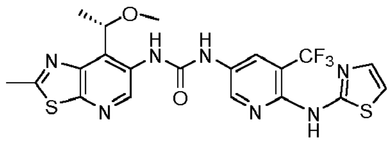

The compound 236 was prepared by using step-5 intermediate (peak-2 acid) of example-83. $^1$HNMR (400 MHz, DMSO-$d_6$) δ 12.07 (s, 1H), 9.90 (s, 1H), 9.13 (s, 1H), 8.63 (s, 1H), 8.54 (s, 1H), 8.22 (s, 1H), 7.19 (s, 1H), 6.66 (s, 1H), 5.50 (q, *J* = 6.7 Hz, 1H), 3.30 (s, 3H), 2.84 (s, 3H), 1.54 (d, *J* = 6.7 Hz, 3H); ESI-MS (m/z) 509.91 (MH)$^+$;

(S or R)-N-(5-(3-(7-(l-Methoxyethyl)-2-methylthiazolo[5,4-b]pyridin-6-yl)ureido)-3-(trifluoromethyl)pyridin-2-yl)acetamide (Compound 237) [Stereochemistry tentatively assigned, it could be either (S) or (R)]

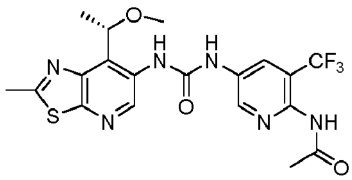

The compound 237 was prepared by using step-5 intermediate (peak-2 acid) of example-83. $^1$HNMR (400 MHz, DMSO-$d_6$) d 10.34 (s, 1H), 10.04 (s, 1H), 9.11 (s, 1H), 8.73 (d, *J* = 2.6 Hz, 1H), 8.66 (s, 1H), 8.46 (d, *J* = 2.7 Hz, 1H), 5.50 (q, *J* = 6.7 Hz, 1H), 3.31 (s, 3H), 2.85 (s, 3H), 2.01 (s, 3H), 1.55 (d, *J* = 6.7 Hz, 3H); ESI-MS (m/z) 469.30 (MH)$^+$;

(S or R)-1-(7-(1-methoxyethyl)-2-methylthiazolo[5,4-b]pyridin-6-yl)-3-(6-(methoxymethyl)-5-(trifluoromethyl)pyridin-3-yl)urea (Compound 238) [Stereochemistry tentatively assigned, it could be either (S) or (R)]

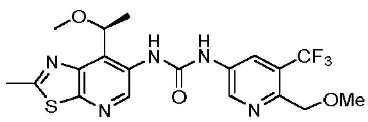

The compound 238 was prepared by using step-5 intermediate (peak-2 acid) of example-83. $^1$HNMR (400 MHz, DMSO-$d_6$) δ 10.37 (s, 1H), 9.11 (s, 1H), 8.82 (d, *J* = 2.4 Hz, 1H), 8.67 (s, 1H), 8.47 (d, *J* = 2.4 Hz, 1H), 5.50 (q, *J* = 6.7 Hz, 1H), 4.56 (s, 2H), 3.30 (s, 6H), 2.85 (s, 3H), 1.54 (d, *J* = 6.7 Hz, 3H); ESI-MS (m/z) 456.29 (MH)$^+$.

**Example-97:** Preparation of 6-(1H-tetrazol-1-yl)-5-(trifluoromethyl)pyridin-3-amine

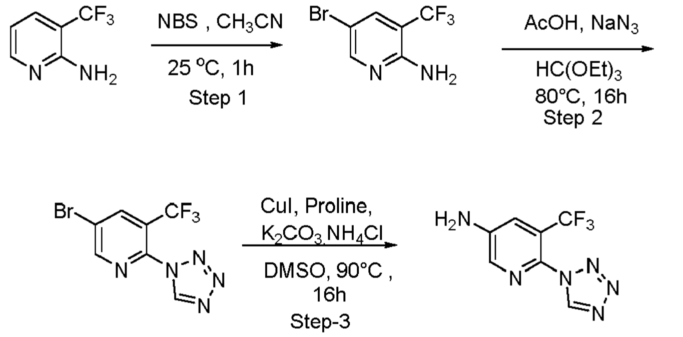


6-(1H-Tetrazol-1-yl)-5-(trifluoromethyl)pyridin-3-amine

Step-1: 5-Bromo-3-(trifluoromethyl)pyridin-2-amine: To a solution of 3-(trifluoromethyl)pyridin-2-amine (5.4 g, 33.3 mmol) in acetonitrile (100 mL) was added NBS (5.93 g, 33.3 mmol) at 0°C and reaction mixture was stirred at 25°C for 1 h. After completion of the reaction, reaction mixture was quenched with saturated sodium bicarbonate (25 mL) and extracted with EtOAc (25 mL×3). The combined organic phase was washed with brine (20 mL), dried over $Na_2SO_4$, filtered. The filtrate was rotary evaporated and residue was purified by flash column chromatography (silica gel) to afford 6.5 g (81%) of the titled product. $^1$HNMR (400 MHz, DMSO-$d_6$) δ 8.28 (d, *J* = 2.4 Hz, 1H), 7.91 (d, *J* = 2.4 Hz, 1H), 6.72 (s, 2H); ESI-MS (*m*/*z*) 241.08 (MH)+.

Step-2: 5-Bromo-2-(1H-tetrazol-1-yl)-3-(trifluoromethyl)pyridine: To a solution of 5-bromo-3-(trifluoromethyl)pyridin-2-amine (2.5 g, 10.37 mmol) in acetic acid (15 mL) were added, $NaN_3$ (0.776 g, 11.93 mmol) and triethyl orthoformate (1.90 mL, 11.41 mmol). Resulting reaction mixture was stirred at 80°C for 16h. The reaction was concentrated and residue was quenched with of saturated $NaHCO_3$( 20 mL) and extracted with EtOAc (25 mL×4). The combined organic phases were washed with brine, dried over $Na_2SO_4$ and filtered. The filtrate was rotary evaporated and residue was purified by flash column chromatography (silica gel) to afford 0.6 g (20%) of the titled product. $^1$HNMR (400 MHz, DMSO-$d_6$) δ 10.06 (d, *J* = 2.2 Hz, 1H), 9.21 (d, *J* = 2.3 Hz, 1H), 9.01 (d, *J* = 2.3 Hz, 1H); ESI-MS (m/z) 294.96 $(MH)^+$.

Step-3: 6-(1H-Tetrazol-1-yl)-5-(trifluoromethyl)pyridin-3-amine: To a stirred solution of 5-bromo-2-(1H-tetrazol-1-yl)-3-(trifluoromethyl)pyridine (0.400 g, 1.360 mmol) in DMSO (5 mL) was added, $K_2CO_3$ (0.564 g, 4.08 mmol), proline (0.063 g, 0.544 mmol), CuI (0.052 g, 0.272 mmol) and ammonium chloride (0.291 g, 5.44 mmol) at 25°C. Resulting reaction mixture was stirred at 90°C for 16h. The reaction mass diluted with water (20 mL) and extracted with ethyl acetate (25 mL×4). The combined organic phases were washed with brine, dried over $Na_2SO_4$, filtered and concentrated The residue was purified by flash column chromatography on silica gel using hexane/ethyl acetate (40:60) to afford 6-(1H-tetrazol-1-yl)-5-(trifluoromethyl)pyridin-3-amine (0.087 g, 0.378 mmol, 27.8%). $^1$HNMR (400 MHz, DMSO-$d_6$) δ 9.89 (s, 1H), 8.13 (d, *J* = 2.7 Hz, 1H), 7.51 (d, *J* = 2.7 Hz, 1H), 6.59 (s, 2H); ESI-MS (m/z) 231.20 $(MH)^+$.

**Example-98:** Preparation of (S or R)-1-(6-(1H-Tetrazol-1-yl)-5-(trifluoromethyl)pyridin-3-yl)-3-(7-(1-methoxyethyl)-2-methylthiazolo[5,4-b]pyridin-6-yl)urea (Compound 239) [Stereochemistry tentatively assigned, it could be either (S) or (R)].

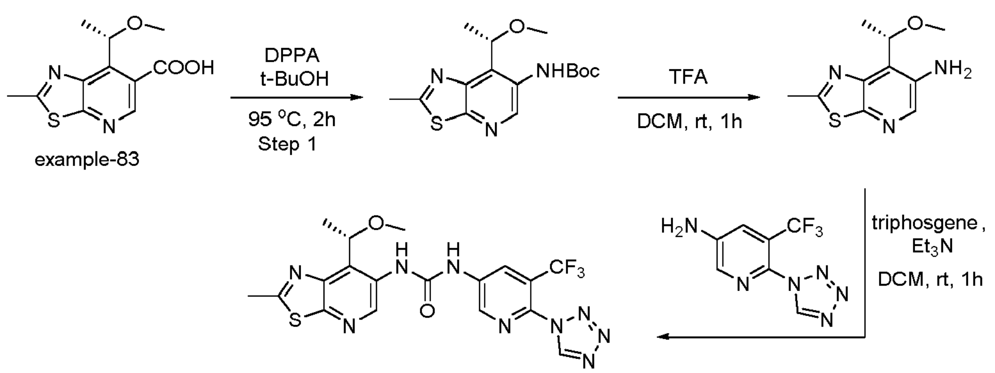


example-83

Compound 239

Step-1: tert-Butyl (S or R)-(7-(1-methoxyethyl)-2-methylthiazolo[5,4-b]pyridin-6-yl)carbamate: To a stirred solution of (S or R)-7-(1-methoxyethyl)-2-methylthiazolo[5,4-b]pyridine-6-carboxylic acid (6.0 g, 23.78 mmol) in t-BuOH (34.1 mL, 357 mmol) was added, DPPA (5.62 ml, 26.2 mmol) and $Et_3N$ (9.94 ml, 71.3 mmol) at 25°C. The resulting reaction mixture was stirred at 95°C for 2 h. After completion of the reaction, the reaction mixture was cooled to room temperature, quenched with water (100 mL) and aqueous phase was extracted with ethyl acetate (50 mL×3), combined organic layer was dried over anhydrous sodium sulphate and filtered. The filtrate was rotary evaporated and residue was purified by flash column chromatography (silica gel) to afford 5.5 g (71%) of the titled product as a colorless gum. $^1$HNMR (400 MHz, $CDCl_3$) δ 9.32 (s, 1H), 8.43 (s, 1H), 5.58 (q, *J* = 6.9 Hz, 1H), 3.40 (s, 3H), 2.84 (s, 3H), 1.59-1.50 (m, 12H); ESI-MS (m/z) 224.24 $(MH)^+$.

Step-2: (S or R)-7-(1-Methoxyethyl)-2-methylthiazolo[5,4-b]pyridin-6-amine: To a stirred solution of step 1 intermediate (4.2 g, 12.99 mmol) in DCM (20 mL) was added TFA (15.01 mL, 195 mmol) at 25°C and the resulting reaction mixture was stirred at 25°C for 1 h. After completion of the reaction, the reaction mixture was concentrated under vaccum, quenched with sat. $NaHCO_3$ (100 mL).The saqueous phase was extracted with ethyl acetate (50 mL×3), and combined organic layer was dried over anhydrous sodium sulphate and filtered. The filtrate was rotary evaporated and residue was purified by flash column chromatography (silica gel) to afford 3.3 g (68%) of the titled product as a colorless gum. $^1$HNMR (400 MHz, $CDCl_3$) δ 8.03 (s, 1H), 5.53 (q, *J* = 6.9 Hz, 1H), 3.38 (s, 3H), 2.82 (s, 3H), 1.59 (d, *J* = 6.9, Hz, 3H); ESI-MS (m/z) 223.92 $(MH)^+$.

Step-3: (S or R)-1-(6-(1H-Tetrazol-1-yl)-5-(trifluoromethyl)pyridin-3-yl)-3-(7-(1-methoxyethyl)-2-methylthiazolo[5,4-b]pyridin-6-yl)urea: The titled compound was prepared by reacting step-2 intermediate with 6-(1H-tetrazol-1-yl)-5-(trifluoromethyl)pyridin-3-amine by following the procedure described in example-71. $^1$HNMR (400 MHz, DMSO-$d_6$) δ 10.77 (s, 1H), 10.04 (s, 1H), 9.13 (s, 1H), 8.92 (d, *J* = 2.5 Hz, 1H), 8.82 (s, 1H), 8.78 (d, *J* = 2.5 Hz, 1H), 5.54 (q, *J* = 6.7 Hz, 1H), 3.33 (s, 3H), 2.86 (s, 3H), 1.57 (d, *J* = 6.7 Hz, 3H); ESI-MS (m/z) 480.3 $(MH)^+$.

**Example-99:** 7-(2-Cyclopropyl-1-methoxyethyl)-2-methylthiazolo[5,4-b]pyridin-6-amine

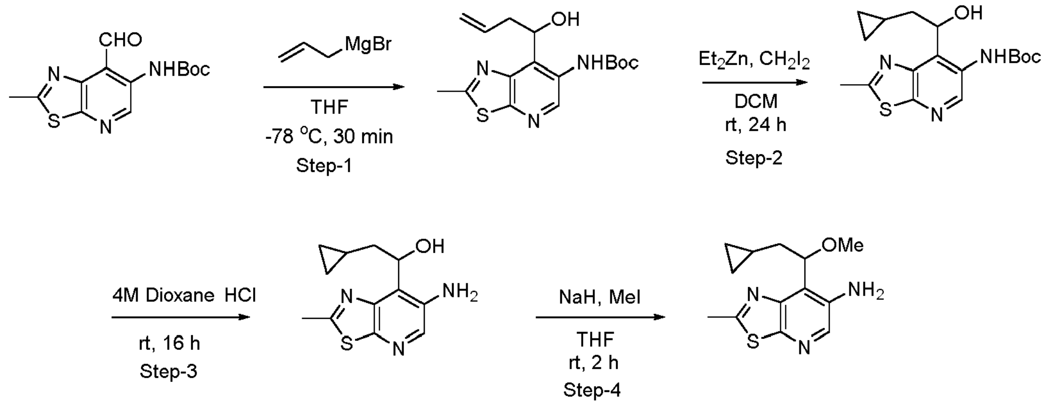


Step-1: *tert*-Butyl (7-(1-hydroxybut-3-en-1-yl)-2-methylthiazolo[5,4-b]pyridin-6-yl)carbamate: To a (-78°C) cooled and stirred solution of tert-butyl (7-formyl-2-methylthiazolo[5,4-b]pyridin-6-yl)carbamate (3.50 g, 11.93 mmol) in THF (20 mL) was added allylmagnesium bromide (26.2 mL, 26.2 mmol, 1M in THF). After stirring for 30 min at the same temperature, the reaction mixture was poured into ice cooled saturated aqueous ammonium chloride solution (20 mL) followed by ethyl acetate (50 mL). The layers were separated and the aqueous layer was extracted with ethyl acetate (2×50 mL). The combined organic layers were washed with water (30 mL) and brine (30 mL), dried ($Na_2SO_4$) and filtered. The filtrate was concentrated under vaccum and the crude product was purifed by flsah column chromatography (silica gel, 10% EtOAc in Hexane as eluent) to afford (2.20 g, 55.0%) of the desired product. $^1$HNMR (400 MHz, DMSO-$d_6$) δ 9.06 (s, 1H), 8.97 (s, 1H), 6.59 (d, *J* = 4.5 *Hz,* 1H), 5.92-5.62 (m, 2H), 5.07-4.87 (m, 2H), 2.83 (s, 3H), 2.68-2.52 (m, 2H), 1.49 (s, 9H); ESI-MS (m/z) 336.34 $(MH)^+$.

Step-2: tert-Butyl (7-(2-cyclopropyl-1-hydroxyethyl)-2-methylthiazolo[5,4-b]pyridin-6-yl)carbamate: To a (0°C) cooled and stirred solution of step-1 intermediate (2.0 g, 5.96 mmol) in DCM (20 mL) was added diethylzinc (59.6 mL, 59.6 mmol, 1M in hexane) followed by diiodomethane (4.81 mL, 59.6 mmol). The reaction was allowed to warm to RT and then stirred for 24 hrs. The reaction mixture was quenched with aqueous saturated ammonium chloride solution (20 mL) followed by ethyl acetate (50 mL). The layers were separated and the aqueous layer was extracted with ethyl acetate (2×50 mL). The combined organic layers were washed with brine (30 mL) dried ($Na_2SO_4$) and filtered. The filtrate was rotary evaporated and the crude product was purifed by flash column chromatography (silica gel) to afford (0.45 g, 21.60%) of the desired product. $^1$HNMR (400 MHz, DMSO-$d_6$) δ 9.16 (s, 1H), 8.99 (s, 1H), 6.53 (d, *J* = 4.4 *Hz,*

1H), 5.79-5.66 (m, 1H), 2.82 (s, 3H), 2.00-1.87 (m, 1H), 1.48 (s, 9H), 1.45-1.34 (m, 1H), 0.79-0.68 (m, 1H), 0.44-0.30 (m, 1H), 0.33-0.18 (m, 1H), 0.05 - - 0.17 (m, 2H); ESI-MS (m/z) 350.28 $(MH)^+$.

Step-3: 1-(6-Amino-2-methylthiazolo[5,4-b]pyridin-7-yl)-2-cyclopropylethanol: To a (0°C) cooled and stirred solution of step-2 intermediate (0.38 g, 1.087 mmol) in DCM (5.0 mL), was added hydrochloric acid (5.44 mL, 21.75 mmol, 4M in dioxane). The reaction was stirred at room temperature for 16 h and then quenched with saturated solution of sodium bicarbonate (3 mL) and extracted with ethyl acetate. The organic layer was dried ($Na_2SO_4$) and filtered. The filtrate was rotary evaporated and the crude product was purified by flash column chromatography (silica gel, 70% EtOAc in hexane as eluent) to (0.20 g, 74%) of the titled compound. ESI-MS (m/z) 250.14 (MH)+.

Step-4: 7-(2-Cyclopropyl-1-methoxyethyl)-2-methylthiazolo[5,4-b]pyridin-6-amine: To a (0°C) cooled and stirred solution of step-3 intermediate (0.22 g, 0.882 mmol) in THF (5 mL) was added NaH (0.039 g, 0.971 mmol) portionwise and then stirred for 15 min at the same temperature. A solution of iodomethane (0.066 mL, 1.059 mmol) in THF (1 mL) was then added to the above stirred reaction mixture and then continued to stir for another 3 h at 0°C. The reaction mass was diluted with ethyl acetate (5 mL) followed by water (2 mL). The layers were separated and the aqueous layer was extracted with ethyl acetate (2×5 mL). The combined organic layers were washed with brine (5 mL), dried ($Na_2SO_4$) and filtered. The filtrate was rotary evaporated and the crude mass was purified by flash column chromatography (silica gel, 70% EtOAc in hexane as eluent) to afford (0.08 g, 0.304 mmol, 34%) of the titled compound. $^1$HNMR (400 MHz, DMSO-$d_6$) δ 8.03 (s, 1H), 5.38 (s, 2H), 5.36-5.28 (m, 1H), 3.22 (s, 3H), 2.75 (s, 3H), 2.14-1.94 (m, 1H), 1.53-1.34 (m, 1H), 0.74-0.60 (m, 1H), 0.46-0.30 (m, 1H), 0.24-2.21 (m, 1H), 0.12 - -0.02 (m, 1H), -0.08 - -0.16 (m, 1H).

**Example-100:** The following compound was prepared by following the similar procedure described in example-66:
(±)-1-(6-(2H-1,2,3-Triazol-2-yl)-5-(trifluoromethyl)pyridin-3-yl)-3-(7-(2-cyclopropyl-1-methoxyethyl)-2-methylthiazolo[5,4-b]pyridin-6-yl)urea (Compound 240)

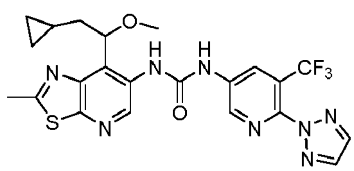

ESI-MS (m/z) 519.36 $(MH)^+$
Chiral separation of racemic compound 240 was carried out using chiral column and afforded the below isomers 240a and 240b.

1-(6-(2H-1,2,3- Triazol-2-yl)-5-(trifluoromethyl)pyridin-3-yl)-3-(7-(2-cyclopropyl-1-methoxyethyl)-2-methylthiazolo[5,4-b]pyridin-6-yl)urea (Compound 240a)
Chiral HPLC RT = 6.50 min
$^1$HNMR (400 MHz, DMSO-$d_6$) δ 10.67 (s, 1H, $D_2O$ exchangeable), 9.10 (s, 1H), 8.86 (d, *J*= 2.5 *Hz,* 1H), 8.76 (s, 1H, $D_2O$ exchangeable), 8.73 (d, *J*= 2.5 *Hz,* 1H), 8.18 (s, 2H), 5.48 (t, *J*= 7.0 *Hz,* 1H), 3.35 (s, 3H), 2.85 (s, 3H), 2.22-2.06 (m, 1H), 1.56-1.47 (m, 1H), 0.78-0.70 (m, 1H), 0.41-0.29 (m, 1H), 0.27-0.21 (m, 1H), 0.09-0.02 (m, 1H), -0.06 - -0.14 (m, 1H); ESI-MS (m/z) 519.06 $(MH)^+$;

1-(6-(2H-1,2,3-Triazol-2-yl)-5-(trifluoromethyl)pyridin-3-yl)-3-(7-(2-cyclopropyl-1-methoxyethyl)-2-methylthiazolo[5,4-b]pyridin-6-yl)urea (Compound 240b)
Chiral HPLC RT = 7.69 min
$^1$HNMR (400 MHz, DMSO-$d_6$) δ 10.67 (s, 1H, $D_2O$ exchangeable), 9.10 (s, 1H), 8.86 (d, *J* = 2.5 *Hz,* 1H), 8.76 (s, 1H, $D_2O$ exchangeable), 8.73 (d, *J*= 2.5 *Hz,* 1H), 8.18 (s, 2H), 5.48 (t, *J* = 7.0 *Hz,* 1H), 3.35 (s, 3H), 2.85 (s, 3H), 2.22-2.06 (m, 1H), 1.56-1.47 (m, 1H), 0.78-0.70 (m, 1H), 0.42-0.29 (m, 1H), 0.28-0.21 (m, 1H), 0.09-0.02 (m, 1H), -0.06 - -0.15 (m, 1H); ESI-MS (m/z) 519.07 $(MH)^+$.

**Example-101: MALT1 Biochemical assay**

The biochemical potency of the MALT1 inhibitors was tested by using a fluorescence based assay with full length MALT1 enzyme. The assay principle makes use of the preferential cleavage by MALT1 after the Arginine residue. Thus, the substrate used is a tetrapeptide (Ac-Leu-Arg-Ser-Arg-AMC; Catalogue # SMAMC013, SM Biochemicals) which is cleaved by active MALT1 releasing the AMC which is fluorescent. Upon addition of the MALT1 protease inhibitors, the proteolytic activity (and accordingly AMC fluorescence) is reduced in a dose dependent manner. The kinetic characterization of the enzymatic reaction was measured by determining the Michaelis constant, Km, of the reaction (ap-

proximately 130 μM). The assay buffer consisted of 50 mM MES, 150 mM NaCl, 0.1% w/v CHAPS, 1M Ammonium citrate and 10 mM DTT (pH = 7). The assay was established for the 384-well plate format using black microtiter square well plates (Optiplate 384-F, Perkin Elmer). The test compounds were dissolved in 100% DMSO at a stock concentration of 10 mM. Serial dilutions were made first in 100% DMSO. The final concentration of DMSO was 0.5% by wt.

For determining the extent of inhibition of MALT1 protease activity by MALT1 inhibitors, 10 μl of the test compound solutions were pre-incubated with 10 μl of MALT1 full length protein (100-300 ng protein / well) for 2 h at RT. This was followed by 10 μl of substrate addition at a final concentration of 100 μM for an additional 4-12 h. The increase in assay signal was linear over this period of time and proportional with increase in the enzyme content. The final concentrations of the test compounds typically ranged from 10000 nM to 0.03 nM in an alternate 3.16 and 3 serial dilutions. The positive control for the reaction contained enzyme and DMSO (without any test compound) and was considered to have 100% enzyme activity (0% inhibition) and the negative control containing only buffer and DMSO (without any enzyme) was considered to have no enzymatic activity (100% inhibition). The fluorescence was recorded in a Spectra Max plate reader, Molecular Devices, with a fluorescence excitation at 360 nm and emission recording at 460 nm. The fluorescence units were transformed to percentage inhibitions by using the positive and negative controls as references as per the following formula.

$$\%\ \text{enzyme inhibition} = \frac{[\text{Avg positive control RFU - Avg Test RFU}]}{[\text{ Avg positive control RFU}]} \text{x100}$$

Positive control = Reaction containing enzyme + substrate + DMSO

Negative control = Reaction containing substrate + DMSO but no enzyme

The $IC_{50}$ values of individual compounds were calculated with Non Linear Regression Analysis using Graph Pad Prism (Graph Pad software, Inc, USA).

Malt 1 inhibition $IC_{50}$ values of the compounds in accordance with embodiments of the invention are provided in Table 1 below: Compounds with $IC_{50}$ 1 nM to 50 nM are grouped under group A, compounds with $IC_{50}$ between 51 nM and 100 nM are grouped under group B, and compounds with $IC_{50}$ between 101 nM and 500 nM are grouped under group C.

**Table: 1**

| Group | Compound Nos. |
|---|---|
| A | 1, 2, 4, 9, 15, 16, 25, 26, 28, 31, 40, 49, 50, 51a, 51b, 52, 56b, 57b, 59, 71b, 74b, 76b, 78b, 79b, 80b, 81b, 84b, 85a, 88b, 90a, 90b, 93b, 94b, 95b, 96, 97, 98, 102, 107, 115, 116, 119, 125b, 126b, 131b, 142a, 142b, 143b, 144b, 145b, 146b, 147b, 151b, 152b, 153b, 154b, 155a, 155b, 156b, 157b, 158b, 159a, 160a, 160b, 161b, 162, 164b, 165b, 168, 169, 170, 176, 179, 191, 196b, 198b, 201b, 209b, 211, 213b, 223, 233b, 239, and 240b |
| B | 6, 18, 23, 29, 37, 39, 51, 73, 75b, 110, 112, 128b, 167b, 171, 172, 192, 193, 195, and 217b |
| C | 3, 5, 7, 8, 10, 17, and 204 |

**Example-102: NF-κB reporter assay**

The NF-κB reporter assay was performed to screen for MALT1 inhibition mediated reduction in the NF-κB transcriptional activity. For this purpose, MALT1 was stably overexpressed in HEK-293-NF-κB-Luc cell line. Cells were seeded in poly-D-lysine coated 96-well plates in a culture medium containing the selection markers (DMEM + 10% FBS + 50 μg/ml Hygromycin + 500 μg/ml Geniticin) and allowed to adhere overnight. On the following day, cells were treated with various concentrations of test compounds for 24 h. After 24 h of treatment with test compounds, media was removed from each well and Bright Glo™ (Promega, USA) substrate was added and incubated for further 10 min at ambient temperature. Luminescence was measured for detection of NF-κB reporter activity. RLUs (Relative Luminescence Units) were directly proportional to the NF-κB activity. % inhibition of NF-κB activity was calculated relative to the samples containing media with 0.1% DMSO alone as per the following formula

$$\% \text{ inhibition} = \frac{(\text{Avg. Vehicle Control RLU - Avg test RLU})}{\text{Avg. Vehicle control RLU}} \times 100$$

The NF-κB inhibition $IC_{50}$ values of the compounds of invention are provided in Table 2 below: Compounds with $IC_{50}$ 1 nM to 100 nM are grouped under group A, compounds with $IC_{50}$ between 101 nM and 500 nM are grouped under group B, and compounds with $IC_{50}$ between 501 nM and 1500 nM are grouped under group C.

**Table 2**

| Group | Compound Nos. |
|---|---|
| A | 1, 9, 35, 40, 42, 49, 53a, 57b, 69, 71b, 74b, 75b, 76b, 78b, 79b, 80a, 80b, 81a, 81b, 84b, 85a, 85b, 88a, 88b, 90a, 90b, 91a, 91b, 93a, 93b, 94b, 95a, 95b, 96, 98, 100, 102, 105, 107, 115, 117, 120b, 122b, 123b, 124b, 125b, 126b, 127a, 127b, 129b, 130b, 131b, 132b, 135a, 136b, 137a, 141a, 142a, 142b, 143b, 144b, 145b, 146a, 146b, 147b, 148b, 149a, 149b, 151b, 152b, 153b, 154a, 154b, 155a, 155b, 156a, 156b, 157a, 157b, 158b, 159a, 160b, 161, 161b, 162, 164, 164b, 165b, 166b, 167b, 177, 178, 179, 184, 191, 195, 196b, 198b, 200b, 201b, 202, 203b, 206b, 207a, 208b, 209b, 213b, 217b, 221, 223, 224b, 225b, 226a, 226b, 227b, 228b, 229b, 230b, 231b, 232b, 239, and 240b |
| B | 5, 7, 23, 26, 28, 29, 31, 34a, 37, 44, 50, 52, 59, 62, 63, 64, 67, 68, 73, 75a, 78a, 79a, 82b, 118b, 119, 122a, 124a, 125a, 131a, 140b, 151a, 159b, 160a, 161a, 162a, 163a, 164a, 169, 170, 171, 176, 188, 192, 196a, 200a, 204, 205b, 208a, 209a, and 233b |
| C | 2, 4, 12, 15, 25, 51, 55a, 58, 110, 133b, 162b, 168, 172, 173, 174, 175, 186, and 201a |

**Example-103: Anticancer assay (14 days)**

OCI-Ly-10 cells (UHN, Canada) seeded in culture media (IMDM + 20% FBS) in 96-well plates were treated with various concentrations of the test compounds. Cells were treated for a period of 14 days (13-15 days depending on the confluency of cells) with fresh treatments every $5^{th}$ day. After the first treatment, for all subsequent treatments, the cells were centrifuged, the spent media was removed and fresh media containing the test compound was added. Cell viability was assessed using CCK-8 kit (Dojindo Laboratories, China) as per manufacturer's instructions. Plates were read in colorimeter and absorbance was detected. (Detection at 450 nm; Background correction at 650 nm). % inhibition was calculated relative to the samples containing media with 0.1% DMSO alone as per the following formula

$$\% \text{ inhibition (Background subtracted)} = \frac{(\text{Avg. Vehicle Control OD - Avg. test OD})}{\text{Avg. Vehicle control RLU}} \times 100$$

Ly-10 (14d) inhibition $IC_{50}$ values of the compounds of the invention are provided in Table 3 below: Compounds with $IC_{50}$ 0.1 nM to 25 nM are grouped under group A, compounds with $IC_{50}$ between 26 nM and 100 nM are grouped under group B, compounds with $IC_{50}$ between 101 nM and 250 nM are grouped under group C, compounds with $IC_{50}$ between 251 nM and 500 nM are grouped under group D, and compounds with $IC_{50}$ between 501 nM and 1500 nM are grouped under group E.

**Table 3:**

| Group | Compound Nos. |
|---|---|
| A | 76b, 90b, 93b, 95b, 96, 131b, 142b, 143b, 144b, 145b, 146b, 147b, 151b, 155b, 156b, 158b, 160b, and 224b |
| B | 9, 35, 42, 43, 49, 56b, 57b, 59, 75b, 79b, 80a, 80b, 81b, 91b, 93a, 94b, 115, 125b, 128b, 153b, 155a, 156a, 157b, 159a, 159b, 161b, 164b, 165b, 196b, 201b, 203b, 221, 225b, 226a, 226b, and 227b |
| C | 1, 26, 34a, 44, 55a, 69, 76a, 79a, 85a, 90a, 107, 136b, 142a, 160a, 162b, 164, 205b, 206b, and 208b |
| D | 2, 85b, 95a, 119, 125a, 162a, 168, 217b, 223, and 227a |
| E | 28, 33, 45, 51b, 61, 75a, 161a, 162, 164a, and 192 |

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A compound of the general formula (I), a tautomer thereof, a stereoisomer thereof, a polymorph thereof, a solvate thereof, or a pharmaceutically acceptable salt thereof,

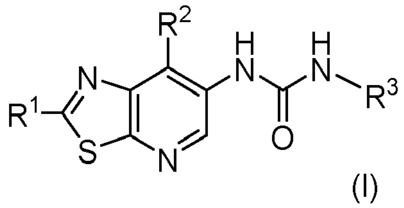

(I)

wherein,

$R^1$ is selected from hydrogen, halogen, cyano, substituted or unsubstituted alkyl, and cycloalkyl;
$R^2$ is selected from -

a) alkyl or alkyl substituted with 1 to 4 substituents independently selected from oxo (=O), halogen, cyano, cycloalkyl, substituted or unsubstituted aryl, heteroaryl, substituted or unsubstituted heterocyclyl, $-OR^4$, -C(=O)OH, $-SO_2$(alkyl), - C(=O)O(alkyl), $-NR^5R^{5a}$, $-NR^5C(=O)R^6$, $-C(=O)R^6$, and $-C(=O)NR^5R^{5a}$,
b) cycloalkyl or cycloalkyl substituted with 1 to 4 substituents independently selected from halogen, cyano, substituted or unsubstituted alkyl, $-OR^4$, -C(=O)OH, - C(=O)O(alkyl), $-C(=O)R^6$, and $-C(=O)NR^5R^{5a}$,
c) cycloalkenyl,
d) cyano,
e) substituted or unsubstituted aryl,
f) substituted or unsubstituted heteroaryl,
g) heterocyclyl or heterocyclyl substituted on either ring carbon atom or a ring nitrogen atom and when it is substituted on ring carbon atom it is substituted with 1 to 4 substituents independently selected from oxo (=O), halogen, cyano, substituted or unsubstituted alkyl, cycloalkyl, $-OR^4$, -C(=O)OH, -C(=O)O-alkyl, $-C(=O)NR^5N^{5a}$, - N(H)C(=O)(alkyl), $-N(H)R^5$, and $-N(alkyl)_2$, and when the heterocycle group is substituted

on a ring nitrogen, it is substituted with substituents independently selected from alkyl, cycloalkyl, aryl, heteroaryl, $-SO_2$(alkyl), -C(=O)$R^6$, C(=O)O(alkyl), -C(=O)N(H)$R^5$, and -C(=O)N(alkyl)$R^5$, and

h) $-NR^aR^b$, wherein, $R^a$ and $R^b$ are independent selected from hydrogen, cycloalkyl, and alkyl or alkyl substituted with 1 to 4 substituents independently selected from oxo (=O), halogen, cycloalkyl, $-OR^4$, and substituted or unsubstituted aryl;

$R^3$ is selected from -

a) heteroaryl or heteroaryl substituted with 1 to 4 substituents selected from halogen, cyano, $-COOR^{4b}$, $-OR^{4a}$, substituted or unsubstituted heteroaryl, substituted or unsubstituted alkyl, substituted or unsubstituted heterocyclyl, substituted or unsubstituted aryl, nitro, $-SO_2$alkyl, $-SO_2$NH(alkyl), $-SO_2NH_2$, - $SO_2NH(CF_3)$, $-SO_2N$(alkyl)$_2$, $-NHSO_2$(alkyl), $-COR^6$, -CON(H)OH, $-CONR^5R^{5a}$, - $N(R^5)COR^{5a}$, and $-NR^5R^{5a}$,

b) aryl or aryl substituted with 1 to 4 substituents selected from halogen, cyano, - $COOR^{4b}$, $-OR^{4a}$, substituted or unsubstituted alkyl, substituted or unsubstituted heterocyclyl, substituted or unsubstituted aryl, nitro, $-SO_2$alkyl, $-SO_2$NH(alkyl), - $SO_2NH_2$, $-SO_2NH(CF_3)$, $-SO_2N$(alkyl)$_2$, $-NHSO_2$(alkyl), $-COR^6$, $-CONR^5R^{5a}$, -CO(NH)OH, $-N(R^5)COR^{5a}$, $-NR^5R^{5a}$, and heteroaryl or heteroaryl substituted with 1 to 4 substituents selected from substituted or unsubstituted alkyl,

c) heterocyclyl or heterocyclyl substituted with 1 to 4 substituents selected from oxo (=O) and substituted or unsubstituted alkyl, and

d)

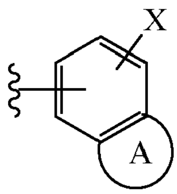
,

wherein, X is halogen and ring A is a heterocyclic ring containing heteroatom(s) selected from S, O, and N, which is optionally substituted with an oxo (=O) group;

$R^4$ is selected from hydrogen, cycloalkyl, and substituted or unsubstituted alkyl;
$R^{4a}$ is selected from

a) hydrogen, alkyl, and cycloalkyl, and

b) alkyl substituted with 1 to 4 substituents independently selected from halogen, - O-alkyl, $-NR^5R^{5a}$, and substituted or unsubstituted heterocyclyl;

$R^{4b}$ is selected from hydrogen and alkyl;
$R^5$ and $R^{5a}$ are each independently selected from

a) hydrogen, alkyl, and cycloalkyl,
b) alkyl substituted with -O-alkyl, $-NH_2$, and $-CONH_2$,
c) heteroaryl, and
d) heterocyclyl substituted with alkyl; and

$R^6$ is selected from alkyl, heterocyclyl, and cycloalkyl;
when an alkyl group is substituted, it is substituted with 1 to 4 substituents independently selected from oxo (=O), halogen, cyano, cycloalkyl, aryl, heteroaryl, heterocyclyl, $-OR^7$, -C(=O)OH, -C(=O)O(alkyl), $-NR^8R^{8a}$, $-NR^8C(=O)R^9$, and - $C(=O)NR^8R^{8a}$;
when the aryl group is substituted, it is substituted with 1 to 4 substituents independently selected from halogen, nitro, cyano, alkyl, perhaloalkyl, cycloalkyl, heterocyclyl, heteroaryl, $-OR^7$, $-NR^8R^{8a}$, $-NR^8C(=O)R^9$, $-C(=O)R^9$, $-C(=O)NR^8R^{8a}$, $-SO_2$-alkyl, -C(=O)OH, -C(=O)O-alkyl, and haloalkyl;
when the heteroaryl group is substituted, it is substituted with 1 to 4 substituents independently selected from halogen, nitro, cyano, alkyl, haloalkyl, perhaloalkyl, cycloalkyl, heterocyclyl, aryl, heteroaryl, $-OR^7$, $-NR^8R^{8a}$, $-NR^7C(=O)R^9$, $-C(=O)R^9$, - $C(=O)NR^8R^{8a}$, $-SO_2$-alkyl, -C(=O)OH, and -C(=O)O-alkyl;

when the heterocycle group is substituted, it is substituted either on a ring carbon atom or on a ring hetero atom, and when it is substituted on a ring carbon atom, it is substituted with 1 to 4 substituents independently selected from oxo (=O), halogen, cyano, alkyl, cycloalkyl, perhaloalkyl, -OR$^7$, -C(=O)NR$^8$R$^{8a}$, -C(=O)OH, -C(=O)O-alkyl, -N(H)C(=O)(alkyl), -N(H)R$^8$, and -N(alkyl)$_2$; and when the heterocycle group is substituted on a ring nitrogen, it is substituted with substituents independently selected from alkyl, cycloalkyl, aryl, heteroaryl, -SO$_2$(alkyl), -C(=O)R$^9$, and - C(=O)O(alkyl); when the heterocycle group is substituted on a ring sulfur, it is substituted with 1 or 2 oxo (=O) group(s);
R$^7$ is selected from hydrogen, alkyl, perhaloalkyl, and cycloalkyl;
R$^8$ and R$^{8a}$ are each independently selected from hydrogen, alkyl, and cycloalkyl; and
R$^9$ is selected from alkyl and cycloalkyl.

**2.** The compound of formula (I), a tautomer thereof, a stereoisomer thereof, a polymorph thereof, a solvate thereof, or a pharmaceutically acceptable salt thereof, as claimed in claim 1, wherein R$^1$ is selected from hydrogen and substituted or unsubstituted alkyl.

**3.** The compound of formula (I), a tautomer thereof, a stereoisomer thereof, a polymorph thereof, a solvate thereof, or a pharmaceutically acceptable salt thereof, as claimed in claim 1 or 2, wherein R$^1$ is selected from hydrogen, methyl, ethyl, and -CF$_3$.

**4.** The compound of formula (I), a tautomer thereof, a stereoisomer thereof, a polymorph thereof, a solvate thereof, or a pharmaceutically acceptable salt thereof, as claimed in any one of claims 1 to 3, wherein R$^2$ is selected from

a) alkyl or alkyl substituted with 1 to 4 substituents independently selected from halogen, cycloalkyl, substituted or unsubstituted heterocyclyl, -OR$^4$, -NR$^5$R$^{5a}$, and substituted or unsubstituted aryl,
b) cycloalkyl or cycloalkyl substituted with substituted or unsubstituted alkyl,
c) cycloalkenyl,
d) substituted or unsubstituted aryl,
e) substituted or unsubstituted heteroaryl,
f) heterocyclyl or heterocyclyl substituted on ring carbon atom with 1 to 2 substituents independently selected from halogen, -OR$^4$, and substituted or unsubstituted alkyl, and
g) -NR$^a$R$^b$, wherein R$^a$ and R$^b$ are independent selected from cycloalkyl and alkyl or alkyl substituted with 1 to 2 substituents independently selected from cycloalkyl, OR$^4$, and substituted or unsubstituted aryl.

**5.** The compound of formula (I), a tautomer thereof, a stereoisomer thereof, a polymorph thereof, a solvate thereof, or a pharmaceutically acceptable salt thereof, as claimed in any one of claims 1 to 4, wherein R$^2$ is selected from

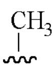 , 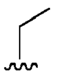 , 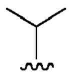 , 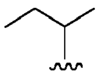 , 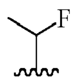 , 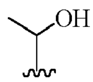 , 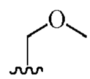 , 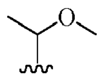 , 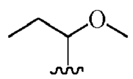 ,

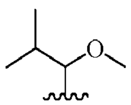 , 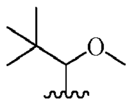 , 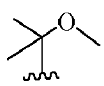 , 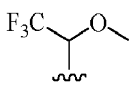 , 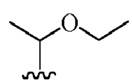 , 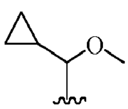 , 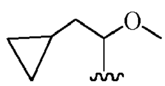 ,

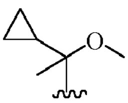 , 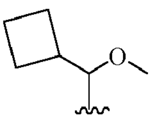 , 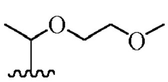 , 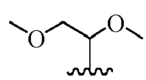 , 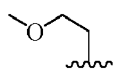 , 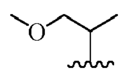 ,

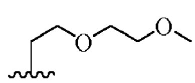 , 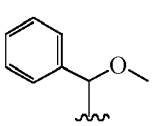 , 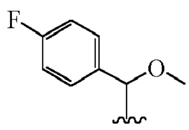 , 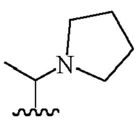 , 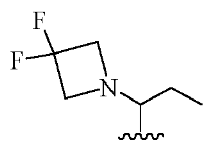 ,

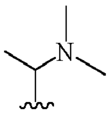 , 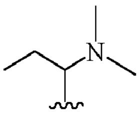 , 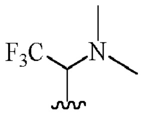 , 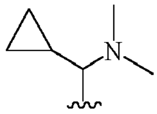 , 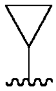 , 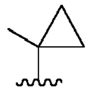 ,

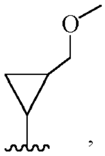 , 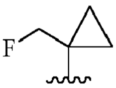 , 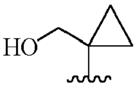 , 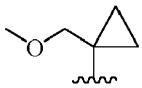 , 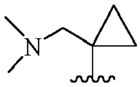 , 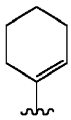 , 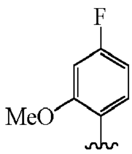 ,

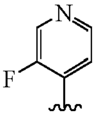 , 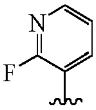 , 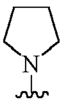 , 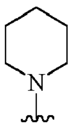 , 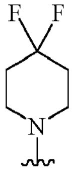 , 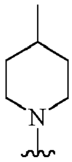 , 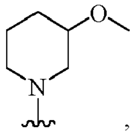 , 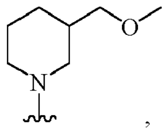 ,

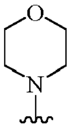 , 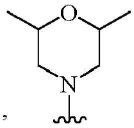 , 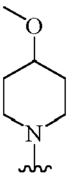 , 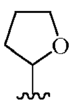 , 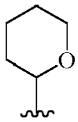 , 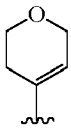 , 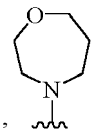 , 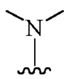 , 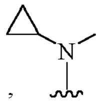 , 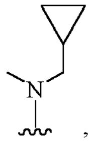 ,

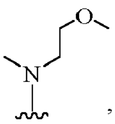 , 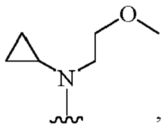 , 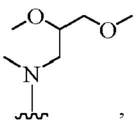 , 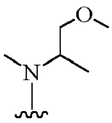 , 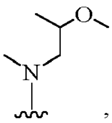 , 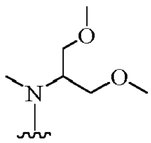 , and 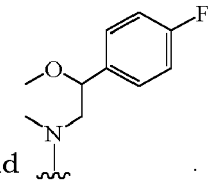 .

6. The compound of formula (I), a tautomer thereof, a stereoisomer thereof, a polymorph thereof, a solvate thereof, or a pharmaceutically acceptable salt thereof, as claimed in any one of claims 1 to 5, wherein $R^3$ is selected from

a) heteroaryl substituted with 1 to 3 substitutents selected from halogen, cyano, - $OR^{4a}$, substituted or unsubstituted heteroaryl, substituted or unsubstituted alkyl, and substituted or unsubstituted heterocyclyl,
b) aryl substituted with 1 to 3 substituents selected from halogen, cyano, $-OR^{4a}$, $COOR^{4b}$, substituted or unsubstituted alkyl, and heteroaryl or heteroaryl substituted with 1 to 4 substituents selected from substituted or unsubstituted alkyl,
c) heterocyclyl substituted with 1 to 3 substituents selected from oxo (=O) and substituted or unsubstituted alkyl, and
d)

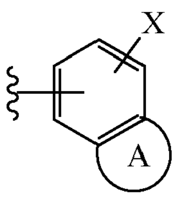 ,

wherein, X is chlorine and ring A is heterocyclic ring containing N, which is optionally substituted with an oxo (=O) group.

7. The compound of formula (I), a tautomer thereof, a stereoisomer thereof, a polymorph thereof, a solvate thereof,

or a pharmaceutically acceptable salt thereof, as claimed in any one of claims 1 to 6, wherein $R^3$ is selected from

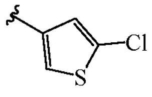 , 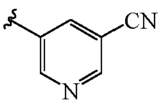 , 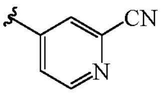 , 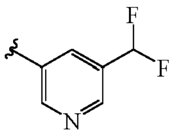 , 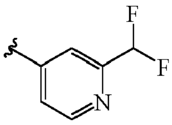 , 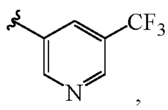 ,

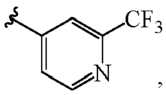 , 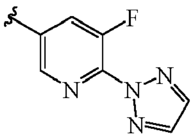 , 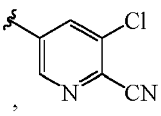 , 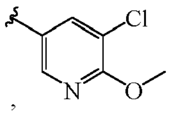 , 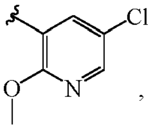 , 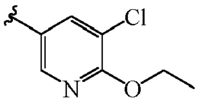 ,

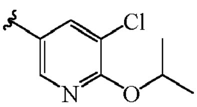 , 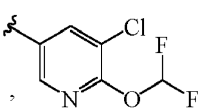 , 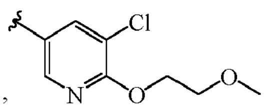 , 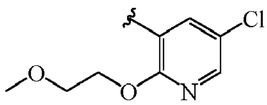 ,

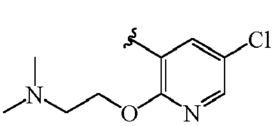 , 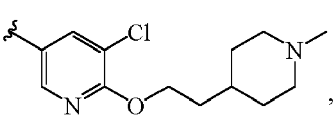 , 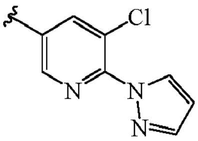 , 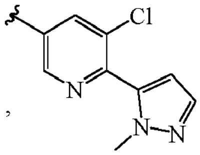 ,

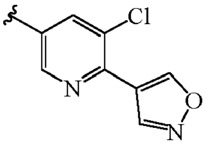 , 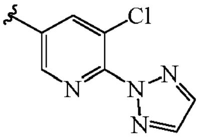 , 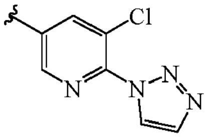 , 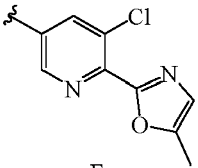 , 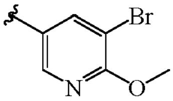 ,

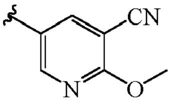 , 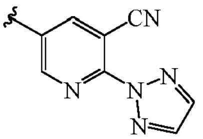 , 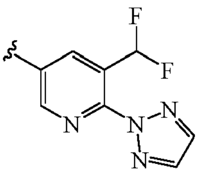 , 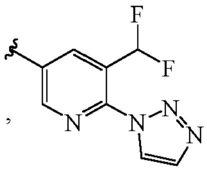 , 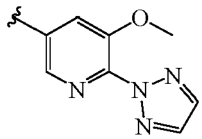 ,

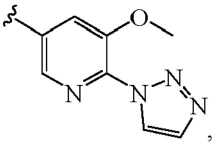 , 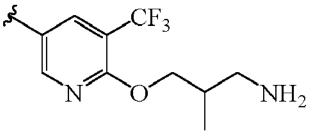 , 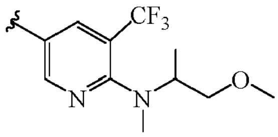 , 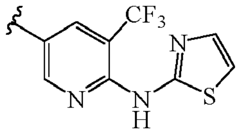 ,

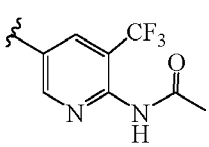 , 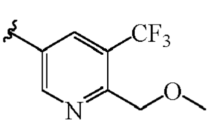 , 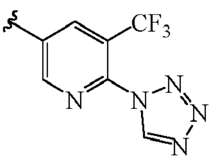 ,

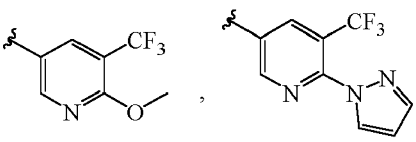 , 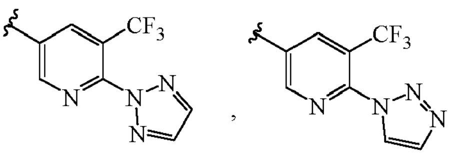 , 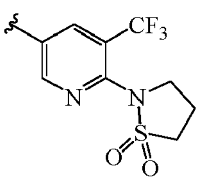 ,

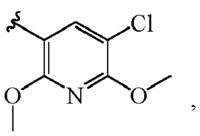 , 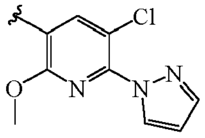 , 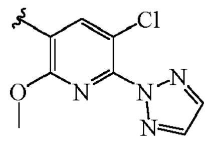 , 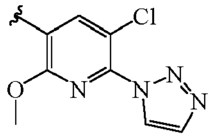 , 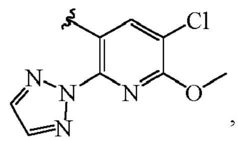 ,

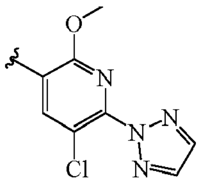 , 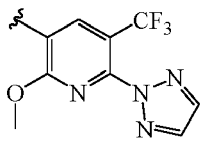 , 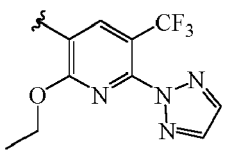 , 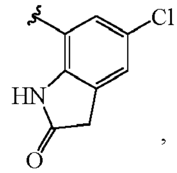 , 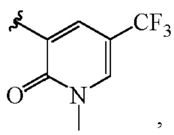 ,

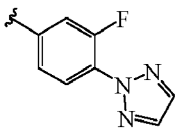 , 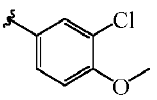 , 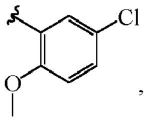 , 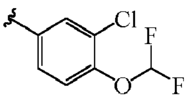 , 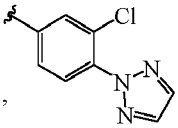 , 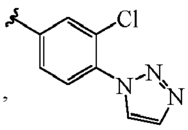 ,

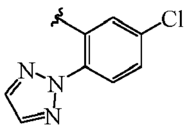 , 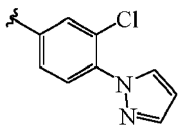 , 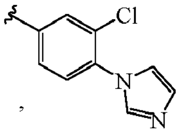 , 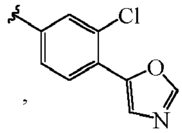 , 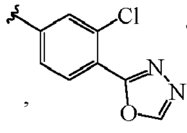 , 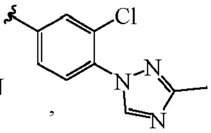 ,

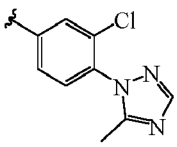 , 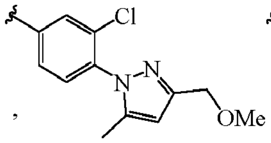 , 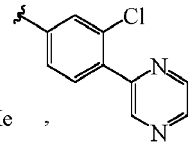 , 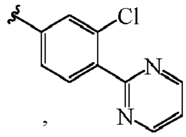 , 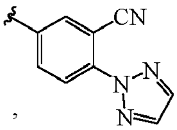 , 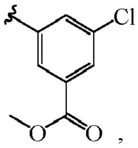 ,

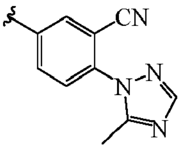 , 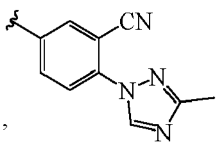 , 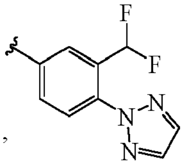 , 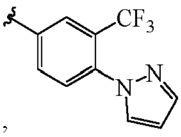 , 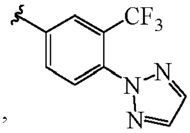 , 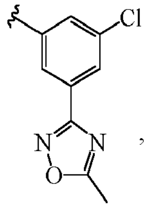 ,

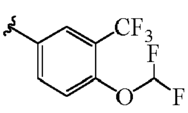 , 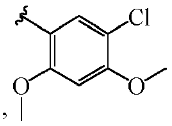 , 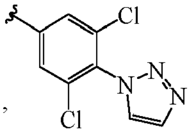 , 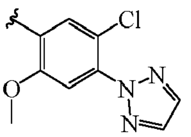 , 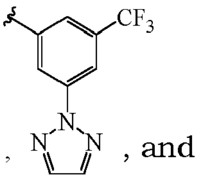 , and 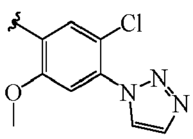 .

8. The compound of formula (I), a tautomer thereof, a stereoisomer thereof, a polymorph thereof, a solvate thereof, or a pharmaceutically acceptable salt thereof, as claimed in any one of claims 1 to 7, wherein $R^4$ is selected from hydrogen and substituted or unsubstituted alkyl.

9. The compound of formula (I), a tautomer thereof, a stereoisomer thereof, a polymorph thereof, a solvate thereof, or a pharmaceutically acceptable salt thereof, as claimed in any one of claims 1 to 8, wherein $R^{4a}$ is selected from alkyl or alkyl substituted with 1 to 2 substituents independently selected from halogen, -O-alkyl, -$NR^5R^{5a}$, and substituted or unsubstituted heterocyclyl.

10. The compound of formula (I), a tautomer thereof, a stereoisomer thereof, a polymorph thereof, a solvate thereof, or a pharmaceutically acceptable salt thereof, as claimed in any one of claims 1 to 9, wherein $R^{4b}$ is alkyl.

11. The compound of formula (I), a tautomer thereof, a stereoisomer thereof, a polymorph thereof, a solvate thereof,

or a pharmaceutically acceptable salt thereof, as claimed in any one of claims 1 to 10, wherein $R^5$ and $R^{5a}$ are each independently selected from alkyl.

**12.** The compound of formula (I), a tautomer thereof, a stereoisomer thereof, a polymorph thereof, a solvate thereof, or a pharmaceutically acceptable salt thereof, as claimed in any one of claims 1 to 11, wherein $R^1$ is selected from hydrogen and substituted or unsubstituted alkyl;

$R^2$ is selected from

a) alkyl or alkyl substituted with 1 to 4 substituents independently selected from halogen, cycloalkyl, heterocyclyl, -$OR^4$, -$NR^5R^{5a}$, and substituted or unsubstituted aryl,
b) cycloalkyl or cycloalkyl substituted with substituted or unsubstituted alkyl,
c) cycloalkenyl,
d) substituted or unsubstituted aryl,
e) substituted or unsubstituted heteroaryl,
f) heterocyclyl or heterocyclyl substituted on ring carbon atom with 1 to 2 substituents independently selected from halogen, -$OR^4$, and substituted or unsubstituted alkyl, and
g) -$NR^aR^b$, wherein $R^a$ and $R^b$ are independent selected from cycloalkyl and alkyl or alkyl substituted with 1 to 2 substituents independently selected from cycloalkyl, $OR^4$, and substituted or unsubstituted aryl; and

$R^3$ is selected from

a) heteroaryl substituted with 1 to 3 substitutents selected from halogen, cyano, - $OR^{4a}$, substituted or unsubstituted heteroaryl, substituted or unsubstituted alkyl, and substituted or unsubstituted heterocyclyl,
b) aryl substituted with 1 to 3 substituents selected from halogen, cyano, -$OR^{4a}$, $COOR^{4b}$, substituted or unsubstituted alkyl, and heteroaryl or heteroaryl substituted with 1 to 4 substituents selected from substituted or unsubstituted alkyl,
c) heterocyclyl substituted with 1 to 3 substituents selected from oxo (=O) and substituted or unsubstituted alkyl, and
d)

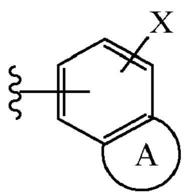,

wherein, X is chlorine and ring A is heterocyclic ring containing N, which is optionally substituted with an oxo (=O) group.

**13.** The compound of formula (I), a tautomer thereof, a stereoisomer thereof, a polymorph thereof, a solvate thereof, or a pharmaceutically acceptable salt thereof, as claimed in any one of claims 1 to 12, wherein:

$R^1$ is selected from hydrogen, methyl, ethyl, and -$CF_3$;
$R^2$ is selected from

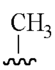, 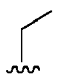, 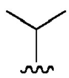, 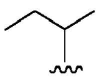, 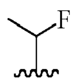, 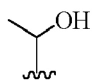, 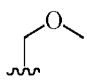, 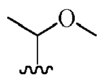, 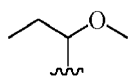,

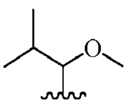, 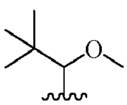, 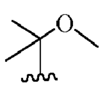, 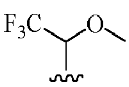, 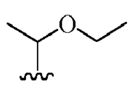, 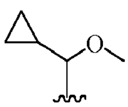, 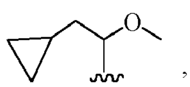,

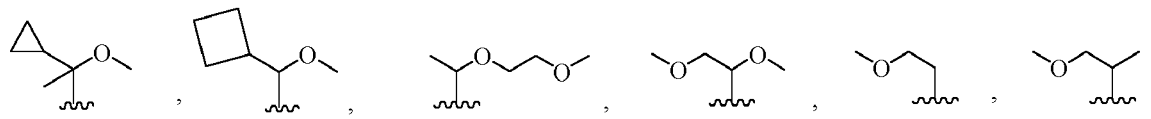
, , , , , ,

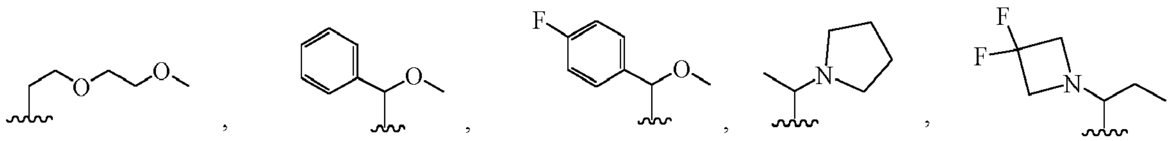
, , , , ,

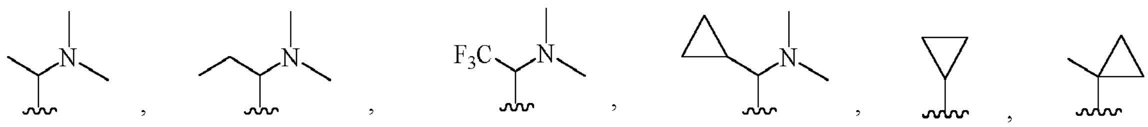
, , , , , ,

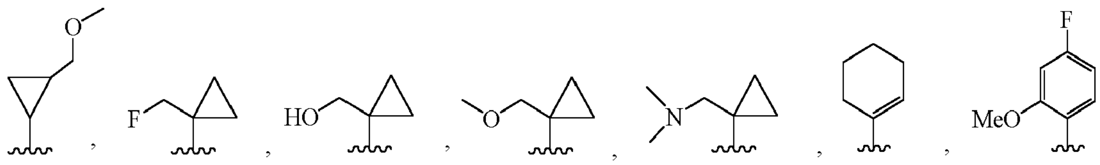
, , , , , , ,

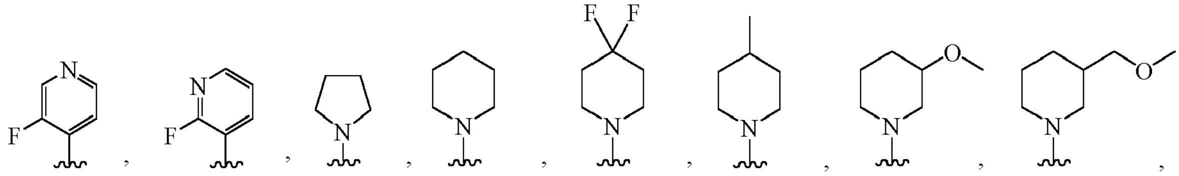
, , , , , , , ,

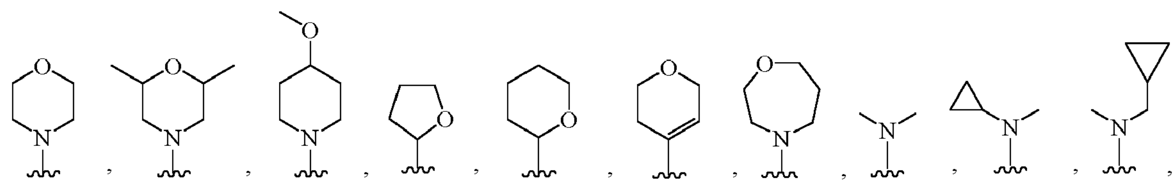
, , , , , , , , , ,

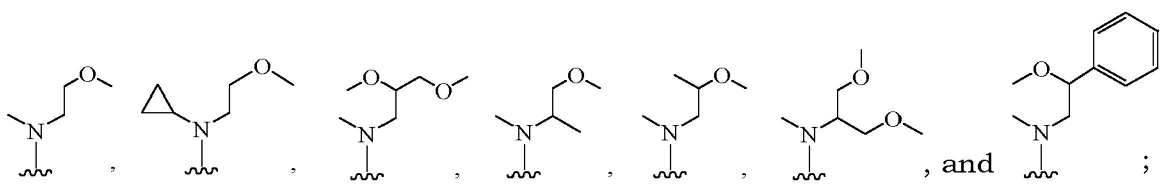
, , , , , , and ;

and
$R^3$ is selected from

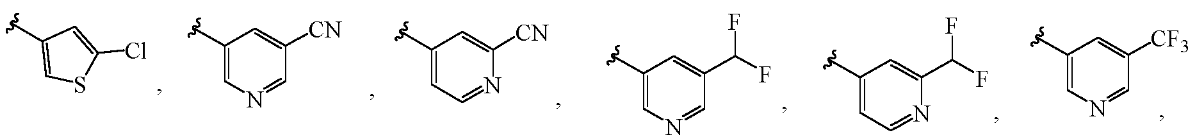
, , , , , ,

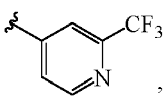 , 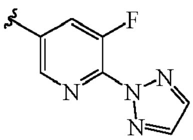 , 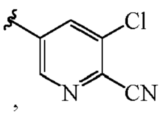 , 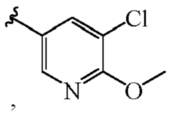 , 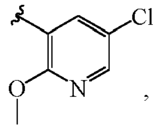 , 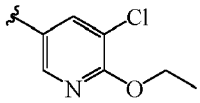 ,

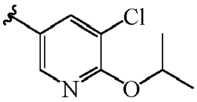 , 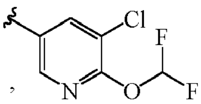 , 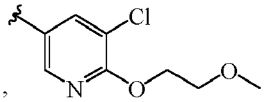 , 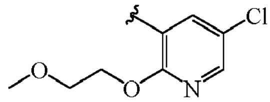 ,

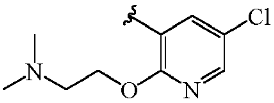 , 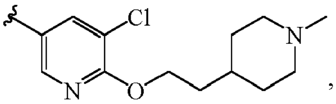 , 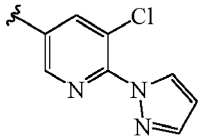 , 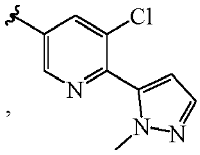 ,

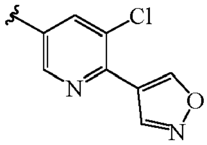 , 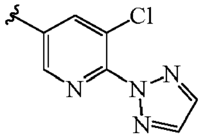 , 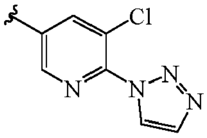 , 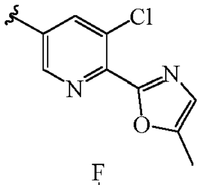 , 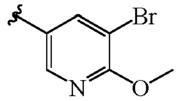 ,

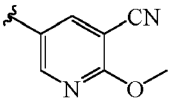 , 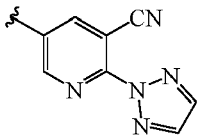 , 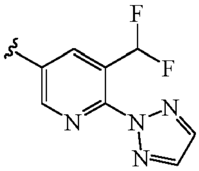 , 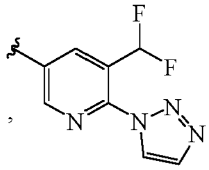 , 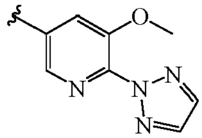 ,

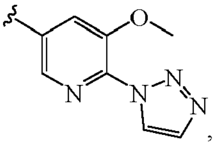 , 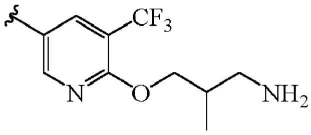 , 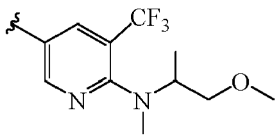 , 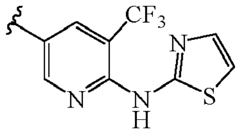 ,

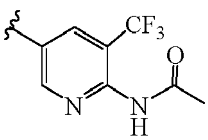 , 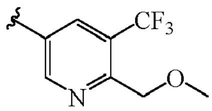 , 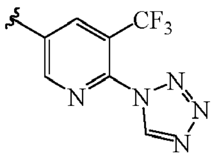 ,

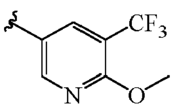 , 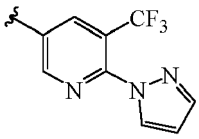 , 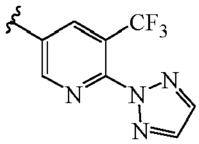 , 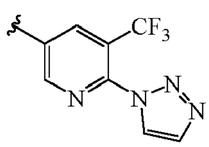 , 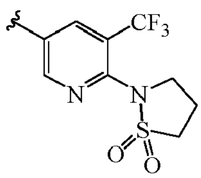 ,

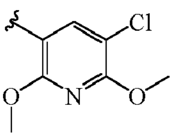 , 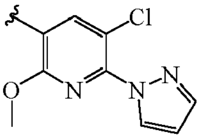 , 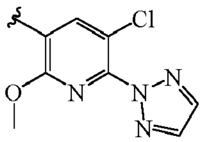 , 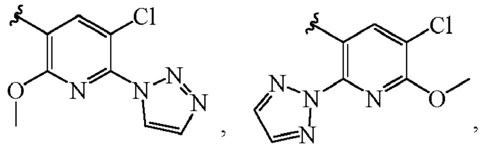 , ,

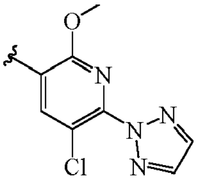, 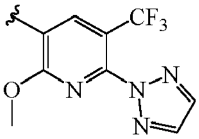, 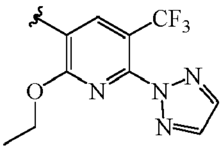, 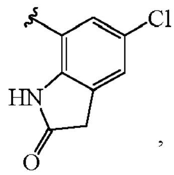, 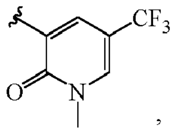,

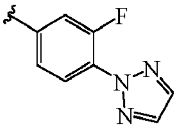, 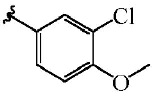, 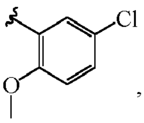, 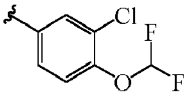, 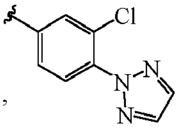, 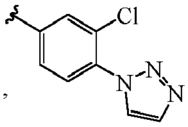,

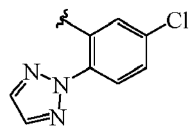, 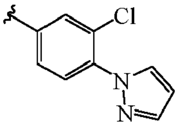, 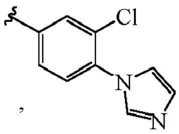, 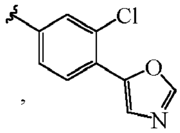, 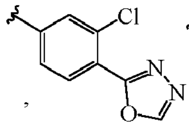, 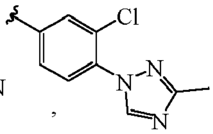,

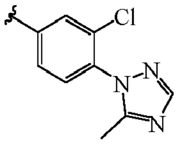, 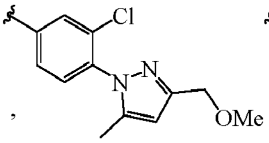, 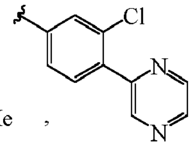, 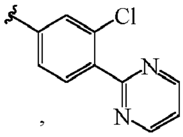, 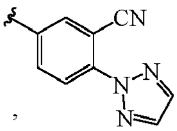, 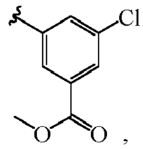,

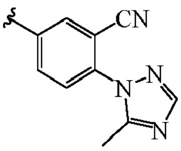, 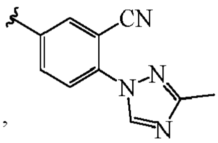, 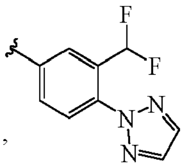, 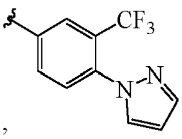, 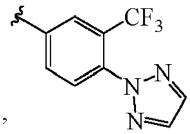, 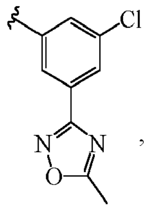,

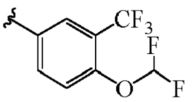, 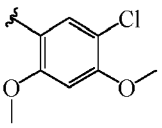, 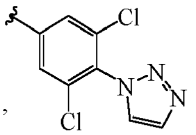, 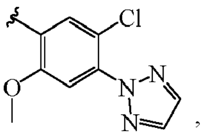, 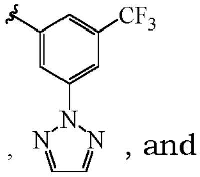, and 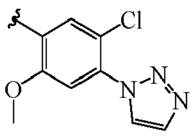.

**14.** The compound of formula (I), a tautomer thereof, a stereoisomer thereof, a polymorph thereof, a solvate thereof, or a pharmaceutically acceptable salt thereof, as claimed in any one of claims 1 to 13, wherein the compound is selected from:

1-(S-Chloro-6-methoxypyridin-3-yl)-3-(7-cyclopropyl-2-methylthiazolo [5,4-b]pyridin-6-yl)urea (Compound 1);
1-(3-Chloro-4-methoxyphenyl)-3-(7-cyclopropyl-2-methylthiazolo[5,4-b]pyridin-6-yl)urea (Compound 2);
1-(7-Cyclopropyl-2-methylthiazolo[5,4-b]pyridin-6-yl)-3-(2-(trifluoromethyl)pyridin-4-yl)urea (Compound 3);
1-(5-Chloro-6-ethoxypyridin-3-yl)-3-(7-cyclopropyl-2-methylthiazolo[5,4-b]pyridin-6-yl)urea (Compound 4);
1-(7-Cyclopropyl-2-methylthiazolo[5,4-b]pyridin-6-yl)-3-(1-methyl-2-oxo-5-(trifluoromethyl)-1,2-dihydropyridin-3-yl)urea (Compound 5);
1-(5-Chloro-6-isopropoxypyridin-3-yl)-3-(7-cyclopropyl-2 methylthiazolo[5,4-b]pyridin-6-yl)urea (Compound 6);
1-(7-Cyclopropyl-2-methylthiazolo[5,4-b]pyridin-6-yl)-3-(5-(trifluoromethyl)pyridin-3-yl)urea (Compound 7);
1-(7-Cyclopropyl-2-methylthiazolo[5,4-b]pyridin-6-yl)-3-(6-methoxy-5-(trifluoromethyl)pyridin-3-yl)urea (Compound 8);
1-(5-Chloro-6-(2H-1,2,3-triazol-2-yl)pyridin-3-yl)-3-(7-cyclopropyl-2-methylthiazolo[5,4-b]pyridin-6-yl)urea (Compound 9);
1-(5-Cyanopyridin-3-yl)-3-(7-cyclopropyl-2-methylthiazolo[5,4-b]pyridin-6-yl)urea (Compound 10);
1-(7-Cyclopropyl-2-methylthiazolo[5,4-b]pyridin-6-yl)-3-(5-(difluoromethyl)pyridin-3-yl)urea (Compound 11);
1-(2-Cyanopyridin-4-yl)-3-(7-cyclopropyl-2-methylthiazolo[5,4-b]pyridin-6-yl)urea (Compound 12);

1-(5-Chloro-6-methoxypyridin-3-yl)-3-(2,7-dimethylthiazolo[5,4-b]pyridin-6-yl)urea (Compound 13);
1-(3-Chloro-4-methoxyphenyl)-3-(2,7- dimethylthiazolo[5,4-b]pyridin-6-yl)urea (Compound 14);
1-(5-Chloro-6-methoxypyridin-3-yl)-3-(7-(4-fluoro-2-methoxyphenyl)-2-methylthiazolo[5,4-b]pyridin-6-yl)urea (Compound 15);
1-(5-Chloro-6-methoxypyridin-3-yl)-3-(7-(2-fluoropyridin-3-yl)-2-methylthiazolo[5,4-b]pyridin-6-yl)urea (Compound 16);
1-(3-Chloro-4-methoxyphenyl)-3-(7-(2-fluoropyridin-3-yl)-2-methylthiazolo[5,4-b]pyridin-6-yl)urea (Compound 17);
1-(5-Chloro-6-methoxypyridin-3-yl)-3-(7-(3-fluoropyridin-4-yl)-2-methylthiazolo[5,4-b]pyridin-6-yl)urea (Compound 18);
1-(6-(2H-1,2,3-Triazol-2-yl)-5-(trifluoromethyl)pyridin-3-yl)-3-(7-cyclopropyl-2-methylthiazolo[5,4-b]pyridin-6-yl)urea (Compound 19);
1-(5-Chloro-6-(difluoromethoxy)pyridin-3-yl)-3-(7-cyclopropyl-2-methylthiazolo[5,4-b]pyridin-6-yl)urea (Compound 20);
1-(5-Chloro-2-oxoindolin-7-yl)-3-(7-cyclopropyl-2-methylthiazolo[5,4-b]pyridin-6-yl)urea (Compound 21);
1-(7-Cyclopropyl-2-methylthiazolo[5,4-b]pyridin-6-yl)-3-(5-methoxy-6-(2H-1,2,3-triazol-2-yl)pyridin-3-yl)urea (Compound 22);
1-(7-Cyclopropyl-2-methylthiazolo[5,4-b]pyridin-6-yl)-3-(6-(1,1-dioxidoisothiazolidin-2-yl)-5-(trifluoromethyl)pyridin-3-yl)urea (Compound 23);
1-(7-Cyclopropyl-2-methylthiazolo[5,4-b]pyridin-6-yl)-3-(5-methoxy-6-(1H-1,2,3-triazol-1-yl)pyridin-3-yl)urea (Compound 24);
1-(3-Chloro-4-methoxyphenyl)-3-(7-ethyl-2-methylthiazolo[5,4-b]pyridin-6-yl)urea (Compound 25);
1-(5-Chloro-6-methoxypyridin-3-yl)-3-(7-ethyl-2-methylthiazolo[5,4-b]pyridin-6-yl)urea (Compound 26);
1-(5-Chloro-6-(2H-1,2,3-triazol-2-yl)pyridin-3-yl)-3-(7-(4,4-difluoropiperidin-1-yl)-2-methylthiazolo[5,4-b]pyridin-6-yl)urea (Compound 27);
1-(5-Chloro-6-(2H-1,2,3-triazol-2-yl)pyridin-3-yl)-3-(2-methyl-7-morpholinothiazolo[5,4-b]pyridin-6-yl)urea (Compound 28);
1-(5-Chloro-6-(2H-1,2,3-triazol-2-yl)pyridin-3-yl)-3-(7-(4-methoxypiperidin-1-yl)-2-methylthiazolo[5,4-b]pyridin-6-yl)urea (Compound 29);
1-(5-Chloro-6-methoxypyridin-3-yl)-3-(7-(4-methoxypiperidin-1-yl)-2-methylthiazolo[5,4-b]pyridin-6-yl)urea (Compound 30);
1-(5-Chloro-6-(2*H*-1,2,3-triazol-2-yl)pyridin-3-yl)-3-(7-cyclopropyl-2-ethylthiazolo[5,4-b]pyridin-6-yl)urea (Compound 31);
1-(5-Chloro-6-(2H-1,2,3-triazol-2-yl)pyridin-3-yl)-3-(7-(2-methoxyethyl)-2-methylthiazolo[5,4-b]pyridin-6-yl)urea (Compound 32);
1-(5-Chloro-6-methoxypyridin-3-yl)-3-(7-(2-methoxyethyl)-2-methylthiazolo[5,4-b]pyridin-6-yl)urea (Compound 33);
1-(5-Chloro-6-methoxypyridin-3-yl)-3-(7-(1,2-dimethoxyethyl)-2-methylthiazolo[5,4-b]pyridin-6-yl)urea (Compound 34);
1-(5-Chloro-6-(2H-1,2,3-triazol-2-yl)pyridin-3-yl)-3-(7-cyclopropylthiazolo[5,4-b]pyridin-6-yl)urea (Compound 35);
1-(5-Chloro-6-methoxypyridin-3-yl)-3-(7-cyclopropylthiazolo[5,4-b]pyridin-6-yl)urea (Compound 36);
1-(5-Chloro-6-(2H-1,2,3-triazol-2-yl)pyridin-3-yl)-3-(2-methyl-7-(4-methylpiperidin-1-yl)thiazolo[5,4-b]pyridin-6-yl)urea (Compound 37);
1-(5-Chloro-6-methoxypyridin-3-yl)-3-(7-(2,6-dimethylmorpholino)-2-methylthiazolo[5,4-b]pyridin-6-yl)urea (Compound 38);
1-(5-Chloro-6-(2H-1,2,3-triazol-2-yl)pyridin-3-yl)-3-(7-(2,6-dimethylmorpholino)-2-methylthiazolo[5,4-b]pyridin-6-yl)urea (Compound 39);
1-(5-Chloro-6-(2H-1,2,3-triazol-2-yl)pyridin-3-yl)-3-(2-methyl-7-(piperidin-1-yl)thiazolo[5,4-b]pyridin-6-yl)urea (Compound 40);
1-(5-Chloro-6-methoxypyridin-3-yl)-3-(7-((cyclopropylmethyl)(methyl)amino)-2-methylthiazolo[5,4-b]pyridin-6-yl)urea (Compound 41);
1-(5-Chloro-6-(2H -1,2,3-triazol-2-yl)pyridin-3-yl)-3-(7-((cyclopropylmethyl)(methyl)amino)-2-methylthiazolo[5,4-b]pyridin-6-yl)urea (Compound 42);
1-(5-Chloro-6-(2H-1,2,3-triazol-2-yl)pyridin-3-yl)-3-(7-((2,3-dimethoxypropyl)(methyl)amino)-2-methylthiazolo[5,4-b]pyridin-6-yl)urea (Compound 43);
1-(5-Chloro-6-(2H-1,2,3-triazol-2-yl)pyridin-3-yl)-3-(7-((2-methoxyethyl) (methyl) amino)-2 -methylthiazolo[5,4-b]pyridin-6 -yl)urea (Compound 44);

1-(5-Chloro-6-methoxypyridin-3-yl)-3-(7-((2-methoxyethyl)(methyl)amino)-2-methylthiazolo[5,4-b]pyridin-6-yl)urea (Compound 45);
1-(5-Chloro-6-(2H-1,2,3-triazol-2-yl)pyridin-3-yl)-3-(7-((1,3-dimethoxypropan-2-yl)(methyl)amino)-2-methylthiazolo[5,4-b]pyridin-6-yl)urea (Compound 46);
1-(5-Chloro-6-(2H-1,2,3-triazol-2-yl)pyridin-3-yl)-3-(7-((2-(4-fluorophenyl)-2-methoxyethyl)(methyl)amino)-2-methylthiazolo[5,4-b]pyridin-6-yl)urea (Compound 47);
1-(5-Chloro-6-methoxypyridin-3-yl)-3-(7-(cyclopropyl(2-methoxyethyl)amino)-2-methylthiazolo[5,4-b]pyridin-6-yl)urea (Compound 48);
1-(5-Chloro-6-(2H-1,2,3-triazol-2-yl)pyridin-3-yl)-3-(7-(cyclopropyl(2-methoxyethyl)amino)-2-methylthiazolo[5,4-b]pyridin-6-yl)urea (Compound 49);
1-(5-Chloro-6-(2H-1,2,3-triazol-2-yl)pyridin-3-yl)-3-(7-(3-(methoxymethyl)piperidin-1-yl)-2-methylthiazolo[5,4-b]pyridin-6-yl)urea (Compound 50);
1-(5-Chloro-6-methoxypyridin-3-yl)-3-(7-(3-(methoxymethyl)piperidin-1-yl)-2-methylthiazolo[5,4-b]pyridin-6-yl)urea (Compound 51);
1-(5-Chloro-6-(2H-1,2,3-triazol-2-yl)pyridin-3-yl)-3-(7-(3-methoxypiperidin-1-yl)-2-methylthiazolo[5,4-b]pyridin-6-yl)urea (Compound 52);
1-(5-Chloro-6-(2H-1,2,3-triazol-2-yl)pyridin-3-yl)-3-(7-((1-methoxypropan-2-yl)(methyl)amino)-2-methylthiazolo[5,4-b]pyridin-6-yl)urea (Compound 53);
1-(5-Chloro-6-methoxypyridin-3-yl)-3-(7-((1-methoxypropan-2-yl)(methyl)amino)-2-methylthiazolo[5,4-b]pyridin-6-yl)urea (Compound 54);
1-(5-Chloro-6-methoxypyridin-3-yl)-3-(7-((2-methoxypropyl)(methyl)amino)-2-methylthiazolo[5,4-b]pyridin-6-yl)urea (Compound 55);
1-(5-Chloro-6-(2H-1,2,3-triazol-2-yl)pyridin-3-yl)-3-(7-(1-methoxy-2,2-dimethylpropyl)-2-methylthiazolo[5,4-b]pyridin-6-yl)urea (Compound 56);
1-(6-(2H-1,2,3-Triazol-2-yl)-5-(trifluoromethyl)pyridin-3-yl)-3-(7-(1-methoxy-2,2-dimethylpropyl)-2-methylthiazolo[5,4-b]pyridin-6-yl)urea (Compound 57);
1-(5-Chloro-6-methoxypyridin-3-yl)-3-(7-(3,6-dihydro-2H-pyran-4-yl)-2-methylthiazolo[5,4-b]pyridin-6-yl)urea (Compound 58);
1-(5-Chloro-6-(2H-1,2,3-triazol-2-yl)pyridin-3-yl)-3-(7-(cyclohex-1-en-1-yl)-2-methylthiazolo[5,4-b]pyridin-6-yl)urea (Compound 59);
1-(5-Chloro-6-cyanopyridin-3-yl)-3-(7-cyclopropyl-2-methylthiazolo[5,4-b]pyridin-6-yl)urea (Compound 60);
1-(5-Chloro-6-(1H-1,2,3-triazol-1-yl)pyridin-3-yl)-3-(7-cyclopropyl-2-methylthiazolo[5,4-b]pyridin-6-yl)urea (Compound 61);
1-(5-Cyano-6-methoxypyridin-3-yl)-3-(7-cyclopropyl-2-methylthiazolo[5,4-b]pyridin-6-yl)urea (Compound 62);
1-(3-Chloro-4-(1,3,4-oxadiazol-2-yl)phenyl)-3-(7 -cyclopropyl-2-methylthiazolo[5,4-b]pyridin-6-yl)urea (Compound 63);
1-(3-Chloro-4-(2H-1,2,3-triazol-2-yl)phenyl)-3-(7-(1-(methoxymethyl)cyclopropyl)-2-methylthiazolo[5,4-b]pyridin-6-yl)urea (Compound 64);
1-(5-Chloro-6-methoxypyridin-3-yl)-3-(7-(1-(methoxymethyl)cyclopropyl)-2-methylthiazolo[5,4-b]pyridin-6-yl)urea (Compound 65);
1-(5-Chloro-6-methoxypyridin-3-yl)-3-(2-methyl-7-(1,4-oxazepan-4-yl)thiazolo[5,4-b]pyridin-6-yl)urea (Compound 66);
1-(5-Chloro-6-(2H-1,2,3-triazol-2-yl)pyridin-3-yl)-3-(2-methyl-7-(1,4-oxazepan-4-yl)thiazolo[5,4-b]pyridin-6-yl)urea (Compound 67);
1-(3-Chloro-4-(2H-1,2,3-triazol-2-yl)phenyl)-3-(7-(cyclopropyl(2-methoxyethyl)amino)-2-methylthiazolo[5,4-b]pyridin-6-yl)urea (Compound 68);
1-(6-(2H-1,2,3-Triazol-2-yl)-5-(trifluoromethyl)pyridin-3-yl)-3-(7-(cyclopropyl(2-methoxyethyl)amino)-2-methylthiazolo[5,4-b]pyridin-6-yl)urea (Compound 69);
1-(4-(2H-1,2,3-Triazol-2-yl)-3-(trifluoromethyl)phenyl)-3-(7-(cyclopropyl(2-methoxyethyl)amino)-2-methylthiazolo[5,4-b]pyridin-6-yl)urea (Compound 70);
1-(6-(2H-1,2,3-Triazol-2-yl)-5-(trifluoromethyl)pyridin-3-yl)-3-(7-(1-hydroxyethyl)-2-methylthiazolo[5,4-b]pyridin-6-yl)urea (Compound 71);
1-(6-(2H-1,2,3-Triazol-2-yl)-5-(trifluoromethyl)pyridin-3-yl)-3-(7-(1-fluoroethyl)-2-methylthiazolo[5,4-b]pyridin-6-yl)urea (Compound 72);
1-(5-Chloro-6-(2-(1-methylpiperidin-4-yl)ethoxy)pyridin-3-yl)-3-(7-cyclopropyl-2-methylthiazolo[5,4-b]pyridin-6-yl)urea (Compound 73);
1-(6-(2H-1,2,3-Triazol-2-yl)-5-(trifluoromethyl)pyridin-3-yl)-3-(7-(1-(dimethylamino)ethyl)-2-methylthiazolo[5,4-b]pyridin-6-yl)urea (Compound 74);

1-(6-(2H-1,2,3-Triazol-2-yl)-5-(trifluoromethyl)pyridin-3-yl)-3-(7-(1-(dimethylamino)propyl)-2-methylthiazolo[5,4-b]pyridin-6-yl)urea (Compound 75);
1-(6-(2H-1,2,3-Triazol-2-yl)-5-(trifluoromethyl)pyridin-3-yl)-3-(7-(cyclopropyl(dimethylamino)methyl)-2-methylthiazolo[5,4-b]pyridin-6-yl)urea (Compound 76);
1-(3-Chloro-4-(2H-1,2,3-triazol-2-yl)phenyl)-3-(2-methyl-7-(1-(pyrrolidin-1-yl)ethyl)thiazolo[5,4-b]pyridin-6-yl)urea (Compound 77);
1-(6-(2H-1,2,3-Triazol-2-yl)-5-(trifluoromethyl)pyridin-3-yl)-3-(7-(1-methoxy-2-methylpropyl)-2-methylthiazolo[5,4-b]pyridin-6-yl)urea (Compound 78);
1-(5-Chloro-6-(2H-1,2,3-triazol-2-yl)pyridin-3-yl)-3-(7-(1-methoxy-2-methylpropyl)-2-methylthiazolo[5,4-b]pyridin-6-yl)urea (Compound 79);
1-(7-(1-Methoxy-2-methylpropyl)-2-methylthiazolo[5,4-b]pyridin-6-yl)-3-(2-methoxy-6-(2H-1,2,3-triazol-2-yl)-5-(trifluoromethyl)pyridin-3-yl)urea (Compound 80);
1-(5-Chloro-2-methoxy-6-(2H-1,2,3-triazol-2-yl)pyridin-3-yl)-3-(7-(1-methoxy-2-methylpropyl)-2-methylthiazolo[5,4-b]pyridin-6-yl)urea (Compound 81);
1-(3-Chloro-4-(2H-1,2,3-triazol-2-yl)phenyl)-3-(7-(methoxy(phenyl)methyl)-2-methylthiazolo[5,4-b]pyridin-6-yl)urea (Compound 82);
1-(6-(2H-1,2,3-Triazol-2-yl)-5-(trifluoromethyl)pyridin-3-yl)-3-(7-(methoxy(phenyl)methyl)-2-methylthiazolo[5,4-b]pyridin-6-yl)urea (Compound 83);
1-(5-Chloro-6-(2H-1,2,3-triazol-2-yl)pyridin-3-yl)-3-(7-(methoxy(phenyl)methyl)-2-methylthiazolo[5,4-b]pyridin-6-yl)urea (Compound 84);
1-(6-(2H-1,2,3-Triazol-2-yl)-5-(trifluoromethyl)pyridin-3-yl)-3-(7-((4-fluorophenyl)(methoxy)methyl)-2-methylthiazolo[5,4-b]pyridin-6-yl)urea (Compound 85);
1-(4-(2H-1,2,3-Triazol-2-yl)-3-(trifluoromethyl)phenyl)-3-(7-((4-fluorophenyl)(methoxy)methyl)-2-methylthiazolo[5,4-b]pyridin-6-yl)urea (Compound 86);
1-(3-Chloro-4-(2H-1,2,3-triazol-2-yl)phenyl)-3-(7-((4-fluorophenyl)(methoxy)methyl)-2-methylthiazolo[5,4-b]pyridin-6-yl)urea (Compound 87);
1-(2-Methoxy-6-(2H-1,2,3-triazol-2-yl)-5-(trifluoromethyl)pyridin-3-yl)-3-(7-(1-methoxyethyl)-2-methylthiazolo[5,4-b]pyridin-6-yl)urea (Compound 88);
1-(5-chloro-2-(2H-1,2,3-triazol-2-yl)phenyl)-3-(7-(1-methoxyethyl)-2-methylthiazolo[5,4-b]pyridin-6-yl)urea (Compound 89);
1-(5-Chloro-2-methoxy-6-(2H-1,2,3-triazol-2-yl)pyridin-3-yl)-3-(7-(1-methoxyethyl)-2-methylthiazolo[5,4-b]pyridin-6-yl)urea (Compound 90);
1-(5-Chloro-2-methoxy-6-(1H-1,2,3-triazol-1-yl)pyridin-3-yl)-3-(7-(1-methoxyethyl)-2-methylthiazolo[5,4-b]pyridin-6-yl)urea (Compound 91);
1-(5-Chloro-6-methoxy-2-(2H-1,2,3-triazol-2-yl)pyridin-3-yl)-3-(7-(1-methoxyethyl)-2-methylthiazolo[5,4-b]pyridin-6-yl)urea (Compound 92);
1-(6-(2H-1,2,3-Triazol-2-yl)-5-(trifluoromethyl)pyridin-3-yl)-3-(7-(1-cyclopropyl-1-methoxyethyl)-2-methylthiazolo[5,4-b]pyridin-6-yl)urea (Compound 93);
1-(4-(2H-1,2,3-Triazol-2-yl)-3-(trifluoromethyl)phenyl)-3-(7-(1-cyclopropyl-1-methoxyethyl)-2-methylthiazolo[5,4-b]pyridin-6-yl)urea (Compound 94);
1-(6-(2H-1,2,3-Triazol-2-yl)-5-(trifluoromethyl)pyridin-3-yl)-3-(2-methyl-7-(2,2,2-trifluoro-1-methoxyethyl)thiazolo[5,4-b]pyridin-6-yl)urea (Compound 95);
1-(6-(2H-1,2,3-Triazol-2-yl)-5-(trifluoromethyl)pyridin-3-yl)-3-(7-(2-methoxypropan-2-yl)-2-methylthiazolo[5,4-b]pyridin-6-yl)urea (Compound 96);
1-(4-(2H-1,2,3-Triazol-2-yl)-3-(trifluoromethyl)phenyl)-3-(7-(2-methoxypropan-2-yl)-2-methylthiazolo[5,4-b]pyridin-6-yl)urea (Compound 97);
1-(3-Chloro-4-(2H-1,2,3-triazol-2-yl)phenyl)-3-(7-(2-methoxypropan-2-yl)-2-methylthiazolo[5,4-b]pyridin-6-yl)urea (Compound 98);
1-(4-(2H-1,2,3-Triazol-2-yl)-3-(trifluoromethyl)phenyl)-3-(7-(cyclopropyl(methyl)amino)-2-methylthiazolo[5,4-b]pyridin-6-yl)urea (Compound 99);
1-(6-(2H-1,2,3-Triazol-2-yl)-5-(trifluoromethyl)pyridin-3-yl)-3-(7-(cyclopropyl(methyl)amino)-2-methylthiazolo[5,4-b]pyridin-6-yl)urea (Compound 100);
1-(5-Chloro-6-(2H-1,2,3-triazol-2-yl)pyridin-3-yl)-3-(7-(1-(methoxymethyl)cyclopropyl)-2-methylthiazolo[5,4-b]pyridin-6-yl)urea (Compound 101);
1-(6-(2H-1,2,3-Triazol-2-yl)-5-(trifluoromethyl)pyridin-3-yl)-3-(7-(1-(methoxymethyl)cyclopropyl)-2-methylthiazolo[5,4-b]pyridin-6-yl)urea (Compound 102);
1-(5-Chloro-6-methoxypyridin-3-yl)-3-(7-cyclopropyl-2-(trifluoromethyl)thiazolo[5,4-b]pyridin-6-yl)urea (Compound 103);

1-(5-Chloro-6-(2H-1,2,3-triazol-2-yl)pyridin-3-yl)-3-(7-cyclopropyl-2-(trifluoromethyl)thiazolo[5,4-b]pyridin-6-yl)urea (Compound 104);
1-(5-Chloro-6-(2H-1,2,3-triazol-2-yl)pyridin-3-yl)-3-(7-(1-(hydroxymethyl)cyclopropyl)-2-methylthiazolo[5,4-b]pyridin-6-yl)urea (Compound 105);
1-(3-Chloro-4-(2H-1,2,3-triazol-2-yl)phenyl)-3-(7-(1-(hydroxymethyl)cyclopropyl)-2-methylthiazolo[5,4-b]pyridin-6-yl)urea (Compound 106);
1-(3-Chloro-4-(2H-1,2,3-triazol-2-yl)phenyl)-3-(7-(1-(fluoromethyl)cyclopropyl)-2-methylthiazolo[5,4-b]pyridin-6-yl)urea (Compound 107);
1-(5-Chloro-6-(2H-1,2,3-triazol-2-yl)pyridin-3-yl)-3-(7-(1-((dimethylamino)methyl)cyclopropyl)-2-methylthiazolo[5,4-b]pyridin-6-yl)urea (Compound 108);
1-(5-chloro-2,4-dimethoxyphenyl)-3-(7-(1-methoxyethyl)-2-methylthiazolo[5,4-b]pyridin-6-yl)urea (Compound 109);
1-(5-Chloro-6-methoxypyridin-3-yl)-3-(7-(dimethylamino)-2-methylthiazolo[5,4-b]pyridin-6-yl)urea (Compound 110);
1-(5-Chloro-6-methoxypyridin-3-yl)-3-(2-methyl-7-(pyrrolidin-1-yl)thiazolo[5,4-b]pyridin-6-yl)urea (Compound 111);
1-(5-Chloro-6-methoxypyridin-3-yl)-3-(2-methyl-7-morpholinothiazolo[5,4-b]pyridin-6-yl)urea (Compound 112);
1-(5-Chloro-6-methoxypyridin-3-yl)-3-(7-(4,4-difluoropiperidin-1-yl)-2-methylthiazolo[5,4-b]pyridin-6-yl)urea (Compound 113);
1-(7-Cyclopropyl-2-methylthiazolo[5,4-b]pyridin-6-yl)-3-(2-(difluoromethyl)pyridin-4-yl)urea (Compound 114);
1-(5-Chloro-6-(2H-1,2,3-triazol-2-yl)pyridin-3-yl)-3-(7-isopropyl-2-methylthiazolo[5,4-b]pyridin-6-yl)urea (Compound 115);
1-(5-Chloro-6-methoxypyridin-3-yl)-3-(7-cyclopropyl-2-ethylthiazolo[5,4-b]pyridin-6-yl)urea (Compound 116);
1-(5-Chloro-6-methoxypyridin-3-yl)-3-(2-methyl-7-(1-methylcyclopropyl)thiazolo[5,4-b]pyridin-6-yl)urea (Compound 117);
1-(3-chloro-4-methoxyphenyl)-3-(7-(1-methoxyethyl)-2-methylthiazolo[5,4-b]pyridin-6-yl)urea (Compound 118);
1-(5-Chloro-6-methoxypyridin-3-yl)-3-(7-isopropyl-2-methylthiazolo[5,4-b]pyridin-6-yl)urea (Compound 119);
1-(5-Chloro-2-methoxypyridin-3-yl)-3-(7-(1-methoxyethyl)-2-methylthiazolo[5,4-b]pyridin-6-yl)urea (Compound 120);
1-(5-Chloro-6-methoxypyridin-3-yl)-3-(7-(1-(2-methoxyethoxy)ethyl)-2-methylthiazolo[5,4-b]pyridin-6-yl)urea (Compound 121);
1-(5-Chloro-2,6-dimethoxypyridin-3-yl)-3-(7-(1-methoxyethyl)-2-methylthiazolo[5,4-b]pyridin-6-yl)urea (Compound 122);
1-(5-Chloro-6-(1H-1,2,3-triazol-1-yl)pyridin-3-yl)-3-(7-(1-methoxyethyl)-2-methylthiazolo[5,4-b]pyridin-6-yl)urea (Compound 123);
1-(5-Chloro-6-methoxypyridin-3-yl)-3-(7-(1-methoxy-2-methylpropyl)-2-methylthiazolo[5,4-b]pyridin-6-yl)urea (Compound 124);
1-(3-Chloro-4-(2H-1,2,3-triazol-2-yl)phenyl)-3-(7-(1-methoxy-2-methylpropyl)-2-methylthiazolo[5,4-b]pyridin-6-yl)urea (Compound 125);
1-(3-Chloro-4-(2H-1,2,3-triazol-2-yl)phenyl)-3-(7-(1-methoxypropyl)-2-methylthiazolo[5,4-b]pyridin-6-yl)urea (Compound 126);
1-(5-Chloro-6-methoxypyridin-3-yl)-3-(7-(1-methoxypropyl)-2-methylthiazolo[5,4-b]pyridin-6-yl)urea (Compound 127);
1-(5-Chloro-2-methoxyphenyl)-3-(7-(1-methoxyethyl)-2-methylthiazolo[5,4-b]pyridin-6-yl)urea (Compound 128);
1-(5-Cyanopyridin-3-yl)-3-(7-(1-methoxyethyl)-2-methylthiazolo[5,4-b]pyridin-6-yl)urea (Compound 129);
1-(7-(1-Methoxyethyl)-2-methylthiazolo[5,4-b]pyridin-6-yl)-3-(2-(trifluoromethyl)pyridin-4-yl)urea (Compound 130);
1-(5-Chloro-2-methoxy-4-(2H-1,2,3-triazol-2-yl)phenyl)-3-(7-(1-methoxyethyl)-2-methylthiazolo[5,4-b]pyridin-6-yl)urea (Compound 131);
1-(5-Chloro-2-methoxy-4-(1H-1,2,3-triazol-1-yl)phenyl)-3-(7-(1-methoxyethyl)-2-methylthiazolo[5,4-b]pyridin-6-yl)urea (Compound 132);
1-(7-(1-Methoxyethyl)-2-methylthiazolo[5,4-b]pyridin-6-yl)-3-(1-methyl-2-oxo-5-(trifluoromethyl)-1,2-dihydropyridin-3-yl)urea (Compound 133);
1-(3-Chloro-4-(2H-1,2,3-triazol-2-yl)phenyl)-3-(7-(1-methoxypropan-2-yl)-2-methylthiazolo[5,4-b]pyridin-6-yl)urea (Compound 134);
1-(5-Chloro-6-methoxypyridin-3-yl)-3-(2-methyl-7-(tetrahydrofuran-2-yl)thiazolo[5,4-b]pyridin-6-yl)urea (Compound 135);
1-(5-Chloro-6-(2H-1,2,3-triazol-2-yl)pyridin-3-yl)-3-(2-methyl-7-(tetrahydrofuran-2-yl)thiazolo[5,4-b]pyridin-6-yl)urea (Compound 136);
1-(3-Chloro-4-(2H-1,2,3-triazol-2-yl)phenyl)-3-(2-methyl-7-(tetrahydrofuran-2-yl)thiazolo[5,4-b]pyridin-6-yl)urea (Compound 137);
1-(3-Chloro-4-(2H-1,2,3-triazol-2-yl)phenyl)-3-(2-methyl-7-(tetrahydro-2H-pyran-2-yl)thiazolo[5,4-b]pyridin-6-yl)urea (Compound 138);
1-(5-Chloro-6-methoxypyridin-3-yl)-3-(2-methyl-7-(tetrahydro-2H-pyran-2-yl)thiazolo[5,4-b]pyridin-6-yl)urea (Compound 139);
1-(5-Chloro-6-(2H-1,2,3-triazol-2-yl)pyridin-3-yl)-3-(2-methyl-7-(tetrahydro-2H-pyran-2-yl)thiazolo[5,4-b]pyridin-6-yl)urea (Compound 140);
1-(6-(1H-1,2,3-Triazol-1-yl)-5-(trifluoromethyl)pyridin-3-yl)-3-(7-(1-methoxyethyl)-2-methylthiazolo[5,4-b]pyridin-6-yl)urea (Compound 141);
1-(6-(2H-1,2,3-Triazol-2-yl)-5-(trifluoromethyl)pyridin-3-yl)-3-(7-(1-methoxyethyl)-2-methylthiazolo[5,4-b]pyridin-6-yl)urea (Compound 142);
1-(4-(2H-1,2,3-Triazol-2-yl)-3-(trifluoromethyl)phenyl)-3-(7-(1-methoxyethyl)-2-methylthiazolo[5,4-b]pyridin-6-yl)urea (Compound 143);
1-(3-Chloro-4-(2H-1,2,3-triazol-2-yl)phenyl)-3-(7-(cyclopropyl(methoxy)methyl)-2-methylthiazolo[5,4-b]pyridin-6-yl)urea (Compound 144);
1-(5-Chloro-6-(2H-1,2,3-triazol-2-yl)pyridin-3-yl)-3-(7-(1-methoxypropyl)-2-methylthiazolo[5,4-b]pyridin-6-yl)urea (Compound 145);
1-(6-(2H-1,2,3-Triazol-2-yl)-5-(trifluoromethyl)pyridin-3-yl)-3-(7-(1-methoxypropyl)-2-methylthiazolo[5,4-b]pyridin-6-yl)urea (Compound 146);
1-(4-(2H-1,2,3-Triazol-2-yl)-3-(trifluoromethyl)phenyl)-3-(7-(1-methoxypropyl)-2-methylthiazolo[5,4-b]pyridin-6-yl)urea (Compound 147);
1-(5-Chloro-6-(5-methyloxazol-2-yl)pyridin-3-yl)-3-(7-(1-methoxyethyl)-2-methylthiazolo[5,4-b]pyridin-6-yl)urea (Compound 148);
1-(5-Chloro-6-(difluoromethoxy)pyridin-3-yl)-3-(7-(1-methoxyethyl)-2-methylthiazolo[5,4-b]pyridin-6-yl)urea (Compound 149);
1-(5-Chloro-6-methoxypyridin-3-yl)-3-(7-(cyclopropyl(methoxy)methyl)-2-methylthiazolo[5,4-b]pyridin-6-yl)urea (Compound 150);
1-(6-(2H-1,2,3-Triazol-2-yl)-5-(trifluoromethyl)pyridin-3-yl)-3-(7-(cyclopropyl(methoxy)methyl)-2-methylthiazolo[5,4-b]pyridin-6-yl)urea (Compound 151);
Methyl 3-chloro-5-(3-(7-(1-methoxyethyl)-2-methylthiazolo[5,4-b]pyridin-6-yl)ureido)benzoate (Compound 152);
1-(4-(2H-1,2,3-Triazol-2-yl)-3-(trifluoromethyl)phenyl)-3-(7-(cyclopropyl(methoxy)methyl)-2-methylthiazolo[5,4-b]pyridin-6-yl)urea (Compound 153);
1-(5-Chloro-6-(2H-1,2,3-triazol-2-yl)pyridin-3-yl)-3-(7-(cyclopropyl(methoxy)methyl)-2-methylthiazolo[5,4-b]pyridin-6-yl)urea (Compound 154);
1-(7-(Cyclopropyl(methoxy)methyl)-2-methylthiazolo[5,4-b]pyridin-6-yl)-3-(2-methoxy-6-(2H-1,2,3-triazol-2-yl)-5-(trifluoromethyl)pyridin-3-yl)urea (Compound 155);
1-(5-Chloro-2-methoxy-6-(2H-1,2,3-triazol-2-yl)pyridin-3-yl)-3-(7-(cyclopropyl(methoxy)methyl)-2-methylthiazolo[5,4-b]pyridin-6-yl)urea (Compound 156);
1-(7-(sec-Butyl)-2-methylthiazolo[5,4-b]pyridin-6-yl)-3-(5-chloro-6-(2H-1,2,3-triazol-2-yl)pyridin-3-yl)urea (Compound 157);
1-(3-Chloro-4-(2H-1,2,3-triazol-2-yl)phenyl)-3-(7-(1-methoxyethyl)-2-methylthiazolo[5,4-b]pyridin-6-yl)urea (Compound 158);
1-(5-Chloro-6-methoxypyridin-3-yl)-3-(7-(1-methoxyethyl)-2-methylthiazolo[5,4-b]pyridin-6-yl)urea (Compound 159);
1-(5-Chloro-6-(2H-1,2,3-triazol-2-yl)pyridin-3-yl)-3-(7-(1-methoxyethyl)-2-methylthiazolo[5,4-b]pyridin-6-yl)urea (Compound 160);
1-(3-Chloro-4-(2H-1,2,3-triazol-2-yl)phenyl)-3-(7-(1-ethoxyethyl)-2-methylthiazolo[5,4-b]pyridin-6-yl)urea (Compound 161);
1-(5-Chloro-6-methoxypyridin-3-yl)-3-(7-(1-ethoxyethyl)-2-methylthiazolo[5,4-b]pyridin-6-yl)urea (Compound 162);
1-(3-Chloro-4-methoxyphenyl)-3-(7-(1-ethoxyethyl)-2-methylthiazolo[5,4-b]pyridin-6-yl)urea (Compound 163);
1-(5-Chloro-6-(2H-1,2,3-triazol-2-yl)pyridin-3-yl)-3-(7-(1-ethoxyethyl)-2-methylthiazolo[5,4-b]pyridin-6-yl)urea (Compound 164);

1-(6-(2H-1,2,3-Triazol-2-yl)-5-(trifluoromethyl)pyridin-3-yl)-3-(7-(1-ethoxyethyl)-2-methylthiazolo[5,4-b]pyridin-6-yl)urea (Compound 165);
1-(5-Chloro-2-methoxy-6-(2H-1,2,3-triazol-2-yl)pyridin-3-yl)-3-(7-(1-ethoxyethyl)-2-methylthiazolo[5,4-b]pyridin-6-yl)urea (Compound 166);
1-(5-Chloro-2-methoxypyridin-3-yl)-3-(7-(1-ethoxyethyl)-2-methylthiazolo[5,4-b]pyridin-6-yl)urea (Compound 167);
1-(3-Chloro-4-(2H-1,2,3-triazol-2-yl)phenyl)-3-(7-cyclopropyl-2-methylthiazolo[5,4-b]pyridin-6-yl)urea (Compound 168);
1-(3-Chloro-4-(1H-1,2,3-triazol-1-yl)phenyl)-3-(7-cyclopropyl-2-methylthiazolo[5,4-b]pyridin-6-yl)urea (Compound 169);
1-(7-Cyclopropyl-2-methylthiazolo[5,4-b]pyridin-6-yl)-3-(3,5-dichloro-4-(1H-1,2,3-triazol-1-yl)phenyl)urea (Compound 170);
1-(3-Cyano-4-(3-methyl-1H-1,2,4-triazol-1-yl)phenyl)-3-(7-cyclopropyl-2-methylthiazolo[5,4-b]pyridin-6-yl)urea (Compound 171);
1-(3-Cyano-4-(5-methyl-1H-1,2,4-triazol-1-yl)phenyl)-3-(7-cyclopropyl-2-methylthiazolo[5,4-b]pyridin-6-yl)urea (Compound 172);
1-(3-Chloro-4-(3-methyl-1H-1,2,4-triazol-1-yl)phenyl)-3-(7-cyclopropyl-2-methylthiazolo[5,4-b]pyridin-6-yl)urea (Compound 173);
1-(3-Chloro-4-(5-methyl-1H-1,2,4-triazol-1-yl)phenyl)-3-(7-cyclopropyl-2-methylthiazolo[5,4-b]pyridin-6-yl)urea (Compound 174);
1-(5-Bromo-6-methoxypyridin-3-yl)-3-(7-cyclopropyl-2-methylthiazolo[5,4-b]pyridin-6-yl)urea (Compound 175);
1-(5-Chloro-6-(2H-1,2,3-triazol-2-yl)pyridin-3-yl)-3-(7-(methoxymethyl)-2-methylthiazolo[5,4-b]pyridin-6-yl)urea (Compound 176);
1-(6-(2H-1,2,3-triazol-2-yl)-5-(trifluoromethyl)pyridin-3-yl)-3-(2-methyl-7-(1-methylcyclopropyl)thiazolo[5,4-b]pyridin-6-yl)urea (Compound 177);
1-(5-Chloro-2-methoxy-6-(2H-1,2,3-triazol-2-yl)pyridin-3-yl)-3-(2-methyl-7-(1-methylcyclopropyl)thiazolo[5,4-b]pyridin-6-yl)urea (Compound 178);
1-(3-Chloro-4-(2H-1,2,3-triazol-2-yl)phenyl)-3-(2-methyl-7-(1-methylcyclopropyl)thiazolo[5,4-b]pyridin-6-yl)urea (Compound 179);
1-(2-Methoxy-6-(2H-1,2,3-triazol-2-yl)-5-(trifluoromethyl)pyridin-3-yl)-3-(2-methyl-7-(1-methylcyclopropyl)thiazolo[5,4-b]pyridin-6-yl)urea (Compound 180);
1-(5-Chloro-6-(1H-pyrazol-1-yl)pyridin-3-yl)-3-(7-cyclopropyl-2-methylthiazolo[5,4-b]pyridin-6-yl)urea (Compound 181);
1-(3-Chloro-4-(1H-pyrazol-1-yl)phenyl)-3-(7-cyclopropyl-2-methylthiazolo[5,4-b]pyridin-6-yl)urea (Compound 182);
1-(3-Chloro-4-(3-(methoxymethyl)-5-methyl-1H-pyrazol-1-yl)phenyl)-3-(7-cyclopropyl-2-methylthiazolo[5,4-b]pyridin-6-yl)urea (Compound 183);
1-(5-Chloro-2-methoxy-6-(2H-1,2,3-triazol-2-yl)pyridin-3-yl)-3-(7-isopropyl-2-methylthiazolo[5,4-b]pyridin-6-yl)urea (Compound 184);
1-(5-Chloro-6-(2H-1,2,3-triazol-2-yl)pyridin-3-yl)-3-(7-(2-(2-methoxyethoxy)ethyl)-2-methylthiazolo[5,4-b]pyridin-6-yl)urea (Compound 185);
1-(5-Chloro-2,6-dimethoxypyridin-3-yl)-3-(7-isopropyl-2-methylthiazolo[5,4-b]pyridin-6-yl)urea (Compound 186);
1-(5-Chloro-2-methoxypyridin-3-yl)-3-(7-isopropyl-2-methylthiazolo[5,4-b]pyridin-6-yl)urea (Compound 187);
1-(3-Chloro-4-(2H-1,2,3-triazol-2-yl)phenyl)-3-(7-isopropyl-2-methylthiazolo[5,4-b]pyridin-6-yl)urea (Compound 188);
1-(5-Chlorothiophen-3-yl)-3-(7-isopropyl-2-methylthiazolo[5,4-b]pyridin-6-yl)urea (Compound 189);
1-(5-Chlorothiophen-3-yl)-3-(7-cyclopropyl-2-methylthiazolo[5,4-b]pyridin-6-yl)urea (Compound 190);
1-(6-(2H-1,2,3-triazol-2-yl)-5-(trifluoromethyl)pyridin-3-yl)-3-(7-isopropyl-2-methylthiazolo[5,4-b]pyridin-6-yl)urea (Compound 191);
1-(5-Chloro-2-methoxypyridin-3-yl)-3-(7-cyclopropyl-2-methylthiazolo[5,4-b]pyridin-6-yl)urea (Compound 192);
1-(3-Chloro-4-(difluoromethoxy)phenyl)-3-(7-cyclopropyl-2-methylthiazolo[5,4-b]pyridin-6-yl)urea (Compound 193);
1-(5-Chloro-6-(1-methyl-1H-pyrazol-5-yl)pyridin-3-yl)-3-(7-cyclopropyl-2-methylthiazolo[5,4-b]pyridin-6-yl)urea (Compound 194);
1-(5-Chloro-2-(2-(dimethylamino)ethoxy)pyridin-3-yl)-3-(7-cyclopropyl-2-methylthiazolo[5,4-b]pyridin-6-yl)urea (Compound 195);
1-(5-Chloro-6-(1H-pyrazol-1-yl)pyridin-3-yl)-3-(7-(1-methoxyethyl)-2-methylthiazolo[5,4-b]pyridin-6-yl)urea

(Compound 196);
1-(5-Chloro-6-(isoxazol-4-yl)pyridin-3-yl)-3-(7-(1-methoxyethyl)-2-methylthiazolo[5,4-b]pyridin-6-yl)urea (Compound 197);
1-(3-Chloro-4-(1H-1,2,3-triazol-1-yl)phenyl)-3-(7-(1-methoxyethyl)-2-methylthiazolo[5,4-b]pyridin-6-yl)urea (Compound 198);
1-(3-Chloro-4-(pyrazin-2-yl)phenyl)-3-(7-(1-methoxyethyl)-2-methylthiazolo[5,4-b]pyridin-6-yl)urea (Compound 199);
1-(5-Cyano-6-(2H-1,2,3-triazol-2-yl)pyridin-3-yl)-3-(7-(1-methoxyethyl)-2-methylthiazolo[5,4-b]pyridin-6-yl)urea (Compound 200);
1-(3-Chloro-4-(1H-pyrazol-1-yl)phenyl)-3-(7-(1-methoxyethyl)-2-methylthiazolo[5,4-b]pyridin-6-yl)urea (Compound 201);
1-(3-Chloro-4-(pyrimidin-2-yl)phenyl)-3-(7-(1-methoxyethyl)-2-methylthiazolo[5,4-b]pyridin-6-yl)urea (Compound 202);
1-(3-Chloro-4-(1,3,4-oxadiazol-2-yl)phenyl)-3-(7-(1-methoxyethyl)-2-methylthiazolo[5,4-b]pyridin-6-yl)urea (Compound 203);
1-(3-Chloro-4-(oxazol-5-yl)phenyl)-3-(7-(1-methoxyethyl)-2-methylthiazolo[5,4-b]pyridin-6-yl)urea (Compound 204);
1-(5-(Difluoromethyl)-6-(2H-1,2,3-triazol-2-yl)pyridin-3-yl)-3-(7-(1-methoxyethyl)-2-methylthiazolo[5,4-b]pyridin-6-yl)urea (Compound 205);
1-(5-(Difluoromethyl)-6-(1H-1,2,3-triazol-1-yl)pyridin-3-yl)-3-(7-(1-methoxyethyl)-2-methylthiazolo[5,4-b]pyridin-6-yl)urea (Compound 206);
1-(3-(Difluoromethyl)-4-(2H-1,2,3-triazol-2-yl)phenyl)-3-(7-(1-methoxyethyl)-2-methylthiazolo[5,4-b]pyridin-6-yl)urea (Compound 207);
1-(3-Cyano-4-(2H-1,2,3-triazol-2-yl)phenyl)-3-(7-(1-methoxyethyl)-2-methylthiazolo[5,4-b]pyridin-6-yl)urea (Compound 208);
1-(5-Chloro-2-methoxy-6-(1H-pyrazol-1-yl)pyridin-3-yl)-3-(7-(1-methoxyethyl)-2-methylthiazolo[5,4-b]pyridin-6-yl)urea (Compound 209);
1-(4-(1H-Pyrazol-1-yl)-3-(trifluoromethyl)phenyl)-3-(7-(1-methoxyethyl)-2-methylthiazolo[5,4-b]pyridin-6-yl)urea (Compound 210);
1-(3-Fluoro-4-(2H-1,2,3-triazol-2-yl)phenyl)-3-(7-(1-methoxyethyl)-2-methylthiazolo[5,4-b]pyridin-6-yl)urea (Compound 211);
1-(5-Fluoro-6-(2H-1,2,3-triazol-2-yl)pyridin-3-yl)-3-(7-(1-methoxyethyl)-2-methylthiazolo[5,4-b]pyridin-6-yl)urea (Compound 212);
1-(6-(1H-Pyrazol-1-yl)-5-(trifluoromethyl)pyridin-3-yl)-3-(7-(1-methoxyethyl)-2-methylthiazolo[5,4-b]pyridin-6-yl)urea (Compound 213);
1-(4-(Difluoromethoxy)-3-(trifluoromethyl)phenyl)-3-(7-(1-methoxyethyl)-2-methylthiazolo[5,4-b]pyridin-6-yl)urea (Compound 214);
1-(3-Chloro-4-(1H-imidazol-1-yl)phenyl)-3-(7-(1-methoxyethyl)-2-methylthiazolo[5,4-b]pyridin-6-yl)urea (Compound 215);
1-(3-Chloro-5-(5-methyl-1,2,4-oxadiazol-3-yl)phenyl)-3-(7-(1-methoxyethyl)-2-methylthiazolo[5,4-b]pyridin-6-yl)urea (Compound 216);
1-(3-(2H-1,2,3-Triazol-2-yl)-5-(trifluoromethyl)phenyl)-3-(7-(1-methoxyethyl)-2-methylthiazolo[5,4-b]pyridin-6-yl)urea (Compound 217);
1-(5-Chloro-6-(2-methoxyethoxy)pyridin-3-yl)-3-(7-(1-methoxyethyl)-2-methylthiazolo[5,4-b]pyridin-6-yl)urea (Compound 218);
1-(5-Chloro-2-(2-methoxyethoxy)pyridin-3-yl)-3-(7-(1-methoxyethyl)-2-methylthiazolo[5,4-b]pyridin-6-yl)urea (Compound 219);
1-(5-chloro-6-(2H-1,2,3-triazol-2-yl)pyridin-3-yl)-3-(7-(2-(methoxymethyl)cyclopropyl)-2-methylthiazolo[5,4-b]pyridin-6-yl)urea (Compound 220);
1-(2-Ethoxy-6-(2H-1,2,3-triazol-2-yl)-5-(trifluoromethyl)pyridin-3-yl)-3-(7-(1-methoxyethyl)-2-methylthiazolo[5,4-b]pyridin-6-yl)urea (Compound 221);
1-(7-(1-Methoxyethyl)-2-methylthiazolo[5,4-b]pyridin-6-yl)-3-(5-(trifluoromethyl)pyridin-3-yl)urea (Compound 222);
1-(6-(2H-1,2,3-Triazol-2-yl)-5-(trifluoromethyl)pyridin-3-yl)-3-(7-(1-(hydroxymethyl)cyclopropyl)-2-methylthiazolo[5,4-b]pyridin-6-yl)urea (Compound 223);
1-(6-(2H-1,2,3-Triazol-2-yl)-5-(trifluoromethyl)pyridin-3-yl)-3-(7-(cyclobutyl(methoxy)methyl)-2-methylthiazolo[5,4-b]pyridin-6-yl)urea (Compound 224);
1-(5-Chloro-6-(2H-1,2,3-triazol-2-yl)pyridin-3-yl)-3-(7-(cyclobutyl(methoxy)methyl)-2-methylthiazolo[5,4-b]pyridin-6-yl)urea (Compound 225);
1-(7-(Cyclopropyl(methoxy)methyl)-2-methylthiazolo[5,4-b]pyridin-6-yl)-3-(2-ethoxy-6-(2H-1,2,3-triazol-2-yl)-5-(trifluoromethyl)pyridin-3-yl)urea (Compound 226);
1-(5-Chloro-6-(2H-1,2,3-triazol-2-yl)pyridin-3-yl)-3-(7-(1-(dimethylamino)propyl)-2-methylthiazolo[5,4-b]pyridin-6-yl)urea (Compound 227);
1-(5-Chloro-6-(2H-1,2,3-triazol-2-yl)pyridin-3-yl)-3-(7-(cyclopropyl(dimethylamino)methyl)-2-methylthiazolo[5,4-b]pyridin-6-yl)urea (Compound 228);
1-(6-(2H-1,2,3-triazol-2-yl)-5-(trifluoromethyl)pyridin-3-yl)-3-(7-(1-(3,3-difluoroazetidin-1-yl)propyl)-2-methylthiazolo[5,4-b]pyridin-6-yl)urea (compound 229);
1-(6-(2H-1,2,3-triazol-2-yl)-5-(trifluoromethyl)pyridin-3-yl)-3-(7-(1-(dimethylamino)-2,2,2-trifluoroethyl)-2-methylthiazolo[5,4-b]pyridin-6-yl)urea (Compound 230);
1-(5-Chloro-6-(2H-1,2,3-triazol-2-yl)pyridin-3-yl)-3-(7-(1-(dimethylamino)-2,2,2-trifluoroethyl)-2-methylthiazolo[5,4-b]pyridin-6-yl)urea (Compound 231);
1-(6-(2H-1,2,3-Triazol-2-yl)-5-(trifluoromethyl)pyridin-3-yl)-3-(2-ethyl-7-(1-methoxyethyl)thiazolo[5,4-b]pyridin-6-yl)urea (Compound 232);
1-(3-Chloro-4-(2H-1,2,3-triazol-2-yl)phenyl)-3-(2-ethyl-7-(1-methoxyethyl)thiazolo[5,4-b]pyridin-6-yl)urea (Compound 233);
1-(6-((S)-2-Aminopropoxy)-5-(trifluoromethyl)pyridin-3-yl)-3-(7-(1-methoxyethyl)-2-methylthiazolo[5,4-b]pyridin-6-yl)urea hydrochloride (Compound 234);
1-(7-(1-Methoxyethyl)-2-methylthiazolo[5,4-b]pyridin-6-yl)-3-(6-(((R)-1-methoxypropan-2-yl)(methyl)amino)-5-(trifluoromethyl)pyridin-3-yl)urea (Compound 235);
1-(7-(1-Methoxyethyl)-2-methylthiazolo[5,4-b]pyridin-6-yl)-3-(6-(thiazol-2-ylamino)-5-(trifluoromethyl)pyridin-3-yl)urea (Compound 236);
N-(5-(3-(7-(1-Methoxyethyl)-2-methylthiazolo[5,4-b]pyridin-6-yl)ureido)-3-(trifluoromethyl)pyridin-2-yl)acetamide (Compound 237);
1-(7-(1-methoxyethyl)-2-methylthiazolo[5,4-b]pyridin-6-yl)-3-(6-(methoxymethyl)-5-(trifluoromethyl)pyridin-3-yl)urea (Compound 238);
1-(6-(1H-Tetrazol-1-yl)-5-(trifluoromethyl)pyridin-3-yl)-3-(7-(1-methoxyethyl)-2-methylthiazolo[5,4-b]pyridin-6-yl)urea (Compound 239); and
1-(6-(2H-1,2,3-Triazol-2-yl)-5-(trifluoromethyl)pyridin-3-yl)-3-(7-(2-cyclopropyl-1-methoxyethyl)-2-methylthiazolo[5,4-b]pyridin-6-yl)urea (Compound 240).

**15.** A pharmaceutical composition comprising a compound of any one of claims 1 to 14, a tautomer thereof, a stereoisomer thereof, a polymorph thereof, a solvate thereof, or a pharmaceutically acceptable salt thereof, and a pharmaceutically acceptable carrier.

**16.** A compound according to any one of claims 1 to 14, or a tautomer thereof, a stereoisomer thereof, a polymorph thereof, a solvate thereof, or a pharmaceutically acceptably salt thereof, for use in a method for treating a disease or disorder mediated through MALT1 in a subject.

**17.** A compound for use according to claim 16, wherein the said disease or disorder is cancer, inflammation or inflammatory disease or disorder, or allergic or autoimmune disease or disorder.

**18.** A compound of use according to claim 17, wherein the said cancer is lymphoma or leukemia.

**19.** A compound of use according to claim 17, wherein the said cancer is ABC-DLBCL type of lymphomas, a subset of GCB-DLBCL type of lymphomas involving MALT1, MALT lymphomas, mantle cell lymphoma, marginal zone lymphoma, cutaneous T cell lymphomas, primary effusion lymphoma, pancreatic cancer, chronic lymphocytic leukemia with CARD 11 mutation, Hodgkin's and Non-Hodgkin's lymphomas, or a subset of acute myelogenous leukemia involving MALT1.

**20.** A compound of use according to claim 17, wherein the said cancer is germ cell tumors and neoplasm involving plasma cell, brain tumors including glioblastoma, hepatic adenomas, medulloblastoma, mesothelioma, different types of melanomas and multiple myeloma, clear cell carcinoma, or adenocarcinoma of lung, breast, bladder, skin, brain, colon, stomach, cervix, ovary, uterus, prostate, liver, and kidney.

**21.** A compound of use according to claim 17, wherein said inflammatory disease or disorder is psoriasis, multiple sclerosis, systemic lupus erythematosus, BENTA disease, ulcerative colitis, pancreatitis, rheumatic fever, or rheumatoid arthritis.

**22.** A compound of use according to claim 17, wherein said inflammatory disease or disorder is ankylosing spondylitis, inflammatory bowel disease, Crohn's disease, gastritis, celiac disease, gout, organ or transplant rejection, chronic allograft rejection, acute or chronic graft-versus-host disease, Behcet's disease, uveitis, dermatitis including atopic dermatitis, dermatomyositis, inflammation of skeletal muscles leading to polymyositis, myasthenia gravis, Grave's disease, Hashimoto thyroiditis, blistering disorders, vasculitis syndromes, Hennoch-Schonlein Purpura, or immune-complex vasculitides.

**23.** A compound of use according to claim 17, wherein the said allergic or autoimmune disease or disorder is Sjoren's syndrome, asthma, bronchitis, or chronic obstructive pulmonary disease.

**24.** A compound of use according to claim 17, wherein the said allergic or autoimmune disease or disorder is cystic fibrosis, respiratory diseases involving lungs leading to respiratory distress and failure.

**25.** A compound of use according to claim 24, wherein respiratory distress and failure means emphysema, pulmonary oedema, pulmonary embolism and primary pulmonary hypertension, and lung fibrosis due to Berylium poisoning.

Europäisches Patentamt
European Patent Office
Office européen des brevets

# EUROPEAN SEARCH REPORT

Application Number
EP 20 18 2522

| Category | Citation of document with indication, where appropriate, of relevant passages | Relevant to claim | CLASSIFICATION OF THE APPLICATION (IPC) |
|---|---|---|---|
| A,D | WO 2015/181747 A1 (NOVARTIS AG [CH]; PISSOT SOLDERMANN CAROLE [CH]; QUANCARD JEAN [CH]; S) 3 December 2015 (2015-12-03)<br>* abstract *<br>* examples 1-127 *<br>*assays *;<br>pages 134-139<br>* pages 140-143 *<br>* claims 1-15 *<br>----- | 1-25 | INV.<br>C07D513/04<br>A61K31/429<br>A61P35/00<br>A61P37/00<br>A61P17/00<br>A61P19/00 |
| A,D | WO 2014/086478 A1 (HELMHOLTZ ZENTRUM MÜNCHEN DEUTSCHES FORSCHUNGSZENTRUM FÜR GESUNDHEIT U) 12 June 2014 (2014-06-12)<br>* abstract *<br>* page 55, line 1 - page 67, line 12 *<br>* examples 1-24 *<br>* claims 1-16 *<br>----- | 1-25 | |
| A,D | WO 2013/017637 A1 (HELMHOLTZ ZENTRUM MUENCHEN [DE]; KRAPPMANN DANIEL [DE]; NAGEL DANIEL [)<br>7 February 2013 (2013-02-07)<br>* page 6, line 21 - page 9, line 10 *<br>* examples 1-8 *<br>* claims 1-10 *<br>* figures 1-13 *<br>----- | 1-25 | TECHNICAL FIELDS SEARCHED (IPC)<br>C07D<br>A61K<br>A61P |
| A,D | WO 2009/065897 A2 (VIB VZW [BE]; UNIV GENT [BE]; UNIV LEUVEN KATH [BE]; BEYAERT RUDI [BE]) 28 May 2009 (2009-05-28)<br>* abstract *<br>* compounds *;<br>pages 22-28<br>* claims 1-9 *<br>* figures 1-12 *<br>-----<br>-/-- | 1-25 | |

The present search report has been drawn up for all claims

1

| Place of search | Date of completion of the search | Examiner |
|---|---|---|
| Munich | 14 September 2020 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 18 2522

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | LORENA FONTAN ET AL: "MALT1 Small Molecule Inhibitors Specifically Suppress ABC-DLBCL In Vitro and In Vivo", CANCER CELL, vol. 22, no. 6, 11 December 2012 (2012-12-11), pages 812-824, XP055186560, ISSN: 1535-6108, DOI: 10.1016/j.ccr.2012.11.003<br>* abstract *<br>* pages 814-820; figures 1-7 *<br>----- | 1-25 | |
| E | WO 2018/165385 A1 (UNIV CORNELL [US]; CHILDRENS MEDICAL CENTER [US] ET AL.) 13 September 2018 (2018-09-13)<br>* abstract *<br>* paragraphs [0007], [0113] *<br>* paragraphs [0115] - [0119], [0127] - [0146] *<br>* paragraphs [0231] - [0239], [0265] - [0272] *<br>* compounds *; pages 9-10<br>* compounds *; pages 92-94<br>* examples 33-41 *<br>* claim 51 *<br>----- | 1,4-11, 15-25 | |

TECHNICAL FIELDS SEARCHED (IPC)

The present search report has been drawn up for all claims

1

| Place of search | Date of completion of the search | Examiner |
|---|---|---|
| Munich | 14 September 2020 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 18 2522

This annex lists the patent family members relating to the patent documents cited in the above-mentioned European search report. The members are as contained in the European Patent Office EDP file on
The European Patent Office is in no way liable for these particulars which are merely given for the purpose of information.

14-09-2020

| Patent document cited in search report | Publication date | Patent family member(s) | Publication date |
|---|---|---|---|
| WO 2015181747 A1 | 03-12-2015 | AR 100645 A1 | 19-10-2016 |
| | | AU 2015265478 A1 | 20-10-2016 |
| | | BR 112016024545 A2 | 15-08-2017 |
| | | CA 2945077 A1 | 03-12-2015 |
| | | CL 2016002942 A1 | 14-07-2017 |
| | | CN 106170489 A | 30-11-2016 |
| | | CR 20160548 A | 02-01-2017 |
| | | CU 20160181 A7 | 10-05-2017 |
| | | DK 3149001 T3 | 22-07-2019 |
| | | EA 201692105 A1 | 28-04-2017 |
| | | EP 3149001 A1 | 05-04-2017 |
| | | ES 2738695 T3 | 24-01-2020 |
| | | GT 201600249 A | 18-12-2018 |
| | | HR P20191261 T1 | 04-10-2019 |
| | | HU E044351 T2 | 28-10-2019 |
| | | JP 6545197 B2 | 17-07-2019 |
| | | JP 2017516778 A | 22-06-2017 |
| | | KR 20170007311 A | 18-01-2017 |
| | | LT 3149001 T | 12-08-2019 |
| | | PE 20170189 A1 | 15-03-2017 |
| | | PH 12016501862 A1 | 19-12-2016 |
| | | PL 3149001 T3 | 31-10-2019 |
| | | PT 3149001 T | 25-07-2019 |
| | | SG 11201607895P A | 29-12-2016 |
| | | SI 3149001 T1 | 30-08-2019 |
| | | SV 2016005328 A | 12-06-2018 |
| | | TR 201910730 T4 | 21-08-2019 |
| | | TW 201609740 A | 16-03-2016 |
| | | US 2017121335 A1 | 04-05-2017 |
| | | US 2018030061 A1 | 01-02-2018 |
| | | UY 36143 A | 08-01-2016 |
| | | WO 2015181747 A1 | 03-12-2015 |
| WO 2014086478 A1 | 12-06-2014 | NONE | |
| WO 2013017637 A1 | 07-02-2013 | AU 2012292016 A1 | 13-02-2014 |
| | | CA 2843338 A1 | 07-02-2013 |
| | | CN 103781481 A | 07-05-2014 |
| | | CN 109999041 A | 12-07-2019 |
| | | DK 2739285 T3 | 15-04-2019 |
| | | EP 2739285 A1 | 11-06-2014 |
| | | EP 3473254 A1 | 24-04-2019 |
| | | ES 2716276 T3 | 11-06-2019 |
| | | JP 6069321 B2 | 01-02-2017 |
| | | JP 2014521678 A | 28-08-2014 |
| | | PL 2739285 T3 | 31-07-2019 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 20 18 2522

This annex lists the patent family members relating to the patent documents cited in the above-mentioned European search report. The members are as contained in the European Patent Office EDP file on
The European Patent Office is in no way liable for these particulars which are merely given for the purpose of information.

14-09-2020

| Patent document cited in search report | Publication date | Patent family member(s) | Publication date |
|---|---|---|---|
| | | PT 2739285 T | 26-03-2019 |
| | | TR 201903280 T4 | 22-04-2019 |
| | | US 2014288060 A1 | 25-09-2014 |
| | | WO 2013017637 A1 | 07-02-2013 |
| WO 2009065897 A2 | 28-05-2009 | AT 553769 T | 15-05-2012 |
| | | AU 2008327884 A1 | 28-05-2009 |
| | | CA 2705694 A1 | 28-05-2009 |
| | | EP 2222326 A2 | 01-09-2010 |
| | | US 2011021548 A1 | 27-01-2011 |
| | | WO 2009065897 A2 | 28-05-2009 |
| WO 2018165385 A1 | 13-09-2018 | EP 3592731 A1 | 15-01-2020 |
| | | JP 2020511454 A | 16-04-2020 |
| | | WO 2018165385 A1 | 13-09-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- IN 201621026107
- IN 201621043859
- IN 201721009450
- WO 2009065897 A
- WO 2016193339 A1
- WO 2008146259 A
- WO 2013017637 A
- WO 2013053765 A
- WO 2014074815 A
- WO 2014086478 A
- WO 2014207067 A
- WO 2015110406 A
- WO 2015181747 A
- WO 2016193339 A
- WO 2017040304 A
- WO 2017057695 A
- WO 2017081641 A
- US 4235871 A
- US 4501728 A
- US 4837028 A
- US 5019369 A
- WO 2009125365 A


### Non-patent literature cited in the description


- **RULAND et al.** *Cell,* 2001, vol. 104, 33-42
- **RULAND et al.** *Immunity,* 2003, vol. 19, 749-58
- **RUEFLI-BRASSI et al.** *Science,* 2003, vol. 302, 1581-84
- **MCALLISTER-LUCAS et al.** *PNAS,* 2007, vol. 104, 139-44
- **FONTAN et al.** *Cancer Cell,* 2012, vol. 22, 812-24
- **NAGEL et al.** *Cancer Cell,* 2012, vol. 22, 825-37
- **FONTAN et al.** *Clin Cancer Res,* 2013, vol. 19, 6662-68
- **JIANG et al.** *Cancer Research,* 2011, vol. 71, 2183-92
- **PAN et al.** *Oncogene,* 2015, 1-10
- **PAN et al.** *Mol Cancer Res,* 2016, vol. 14, 93-102
- **PENAS et al.** *Blood,* 2010, vol. 115, 2214-19
- **RAHAL et al.** *Nature Medicine,* 2014, vol. 20, 87-95
- **REMSTEIN et al.** *Am J Pathol,* 2000, vol. 156, 1183-88
- **BAENS et al.** *Cancer Res,* 2006, vol. 66, 5270-77
- **GANAPATHI et al.** *Oncotarget,* 2016, 1-10
- **BENNETT et al.** *Am J of Surgical Pathology,* 2016, 1-7
- **QIN et al.** *Blood,* 2001, vol. 98, 2778-83
- **DOEBBELING et al.** *J of Exp and Clin Cancer Res,* 2010, vol. 29, 1-5
- **BONSIGNORE et al.** *Leukemia,* 2017, vol. 31, 614-24
- **LOWES et al.** *Ann Review Immunology,* 2014, vol. 32, 227-55
- **AFONINA et al.** *EMBO Reports,* 2016, 1-14
- **HOWES et al.** *Biochem J,* 2016, 1-23
- **JABARA et al.** *J Allergy Clin Immunology,* 2013, vol. 132, 151-58
- **MCGUIRE et al.** *J of Neuroinflammation,* 2014, vol. 11, 1-12
- **STREUBEL et al.** *Clin Cancer Research,* 2004, vol. 10, 476-80
- **SAGAERT et al.** *Modern Pathology,* 2006, vol. 19, 225-32
- **LIU et al.** *Oncotarget,* 2016, 1-14
- **SUZUKI et al.** *Blood,* 1999, vol. 94, 3270-71
- **AKAGI et al.** *Oncogene,* 1999, vol. 18, 5785-94
- **BERGE S. M. et al.** Pharmaceutical Salts, a review article. *Journal of Pharmaceutical sciences,* 1977, vol. 66, 1-19
- **P. HEINRICH STAHLAND ; CAMILLE G.WERMUTH.** Handbook of Pharmaceutical Salts-Properties, Selection, and Use. Wiley-VCH, 2002
- Remington's Pharmaceutical Sciences. Mack Publishing Company, 1990, 1445
- Journal of Pharmaceutical Science. 1977, vol. 66, 2-19
- Pharmaceutics and Pharmacy Practice. J.B. Lippincott Company, 1982, 238-250
- ASHP Handbook on Injectable Drugs. 1986, 622-630

- Remington's Pharmaceutical Science. Mack Publishing Company, 1985
- **SZOKA et al.** *Ann. Rev. Biophys. Bioeng.,* 1980, vol. 9, 467
- **WASSERMAN et al.** *Cancer,* 1975, vol. 36, 1258-1268
- **THOMSON PDR.** Physicians' Desk Reference. 2004